(19) Europäisches Patentamt
European Patent Office
Office européen des brevets

(11) EP 2 906 706 B1

(12) EUROPEAN PATENT SPECIFICATION

(45) Date of publication and mention of the grant of the patent:
**26.02.2020 Bulletin 2020/09**

(21) Application number: **13780295.5**

(22) Date of filing: **11.10.2013**

(51) Int Cl.:
***C12P 7/04*** (2006.01) ***C12P 7/16*** (2006.01)
***C12M 1/00*** (2006.01) ***B01D 21/26*** (2006.01)

(86) International application number:
**PCT/US2013/064535**

(87) International publication number:
**WO 2014/059273 (17.04.2014 Gazette 2014/16)**

# (54) PROCESSES AND SYSTEMS FOR THE PRODUCTION OF FERMENTATION PRODUCTS

VERFAHREN UND SYSTEME ZUR HERSTELLUNG VON FERMENTATIONSPRODUKTEN

PROCÉDÉS ET SYSTÈMES POUR LA PRODUCTION DE PRODUITS DE FERMENTATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**11.10.2012 US 201261712385 P**
**14.03.2013 US 201313828353**
**15.03.2013 US 201313836115**
**12.09.2013 US 201314024722**

(43) Date of publication of application:
**19.08.2015 Bulletin 2015/34**

(73) Proprietor: **Butamax Advanced Biofuels LLC**
**Wilmington, DE 19880-0356 (US)**

(72) Inventors:
- **BAZZANA, Stephan,e Francois**
  **Wilmington, Delaware 19805 (US)**
- **BERNFELD, Adam**
  **Newark, Delaware 19072 (US)**
- **BURLEW, Keith, H.**
  **Middletown, Delaware 19709 (US)**
- **COFFEY, Duncan**
  **Hockessin, Delaware 19707 (US)**
- **CRONIN, James, Timothy**
  **Townsend, Delaware 19734 (US)**
- **FUCHS, Benjamin**
  **Wilmington, Delaware 19803 (US)**
- **HALLAM, John, W.**
  **Beverley**
  **HU17 7JT (GB)**
- **JONES, Adam**
  **Wilmington, Delaware 19810 (US)**
- **LOWE, David, J.**
  **Wilmington, Delaware 19808 (US)**
- **ROESCH, Brian, Michael**
  **Middleton, Delaware 19709 (US)**
- **STOLARSKI, Mathias, E.**
  **Mt. Pleasant**
  **South Carolina 29466 (US)**
- **WOOD, James, Gregory**
  **Newark, Delaware 19711 (US)**
- **ZAHER, Joseph, J.**
  **Newark, Delaware 19711 (US)**

(74) Representative: **Dehns**
**St. Bride's House**
**10 Salisbury Square**
**London EC4Y 8JD (GB)**

(56) References cited:
**WO-A1-2011/159962**
**WO-A1-2011/159967**
**WO-A1-2013/166458**
**WO-A1-2014/018479**
**WO-A1-2014/159309**
**WO-A2-2011/160030**
**US-A1- 2012 322 117**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 906 706 B1

SEQUENCE LISTING

<110> Butamax Advanced Biofuels LLC

<120> Processes and Systems for the Production of Fermentation Products

<130> CL4723WOPCT3

<160> 128

<170> PatentIn version 3.5

<210> 1
<211> 570
<212> PRT
<213> Bacillus subtilis

<400> 1

```
Met Thr Lys Ala Thr Lys Glu Gln Lys Ser Leu Val Lys Asn Arg Gly
1               5                   10                  15

Ala Glu Leu Val Val Asp Cys Leu Val Glu Gln Gly Val Thr His Val
            20                  25                  30

Phe Gly Ile Pro Gly Ala Lys Ile Asp Ala Val Phe Asp Ala Leu Gln
        35                  40                  45

Asp Lys Gly Pro Glu Ile Ile Val Ala Arg His Glu Gln Asn Ala Ala
    50                  55                  60

Phe Met Ala Gln Ala Val Gly Arg Leu Thr Gly Lys Pro Gly Val Val
65                  70                  75                  80

Leu Val Thr Ser Gly Pro Gly Ala Ser Asn Leu Ala Thr Gly Leu Leu
                85                  90                  95

Thr Ala Asn Thr Glu Gly Asp Pro Val Val Ala Leu Ala Gly Asn Val
            100                 105                 110

Ile Arg Ala Asp Arg Leu Lys Arg Thr His Gln Ser Leu Asp Asn Ala
        115                 120                 125

Ala Leu Phe Gln Pro Ile Thr Lys Tyr Ser Val Glu Val Gln Asp Val
    130                 135                 140

Lys Asn Ile Pro Glu Ala Val Thr Asn Ala Phe Arg Ile Ala Ser Ala
145                 150                 155                 160

Gly Gln Ala Gly Ala Ala Phe Val Ser Phe Pro Gln Asp Val Val Asn
                165                 170                 175
```

```
Glu Val Thr Asn Thr Lys Asn Val Arg Ala Val Ala Ala Pro Lys Leu
            180                 185                 190

Gly Pro Ala Ala Asp Asp Ala Ile Ser Ala Ala Ile Ala Lys Ile Gln
        195                 200                 205

Thr Ala Lys Leu Pro Val Val Leu Val Gly Met Lys Gly Gly Arg Pro
    210                 215                 220

Glu Ala Ile Lys Ala Val Arg Lys Leu Leu Lys Lys Val Gln Leu Pro
225                 230                 235                 240

Phe Val Glu Thr Tyr Gln Ala Ala Gly Thr Leu Ser Arg Asp Leu Glu
                245                 250                 255

Asp Gln Tyr Phe Gly Arg Ile Gly Leu Phe Arg Asn Gln Pro Gly Asp
            260                 265                 270

Leu Leu Leu Glu Gln Ala Asp Val Val Leu Thr Ile Gly Tyr Asp Pro
        275                 280                 285

Ile Glu Tyr Asp Pro Lys Phe Trp Asn Ile Asn Gly Asp Arg Thr Ile
    290                 295                 300

Ile His Leu Asp Glu Ile Ile Ala Asp Ile Asp His Ala Tyr Gln Pro
305                 310                 315                 320

Asp Leu Glu Leu Ile Gly Asp Ile Pro Ser Thr Ile Asn His Ile Glu
                325                 330                 335

His Asp Ala Val Lys Val Glu Phe Ala Glu Arg Glu Gln Lys Ile Leu
            340                 345                 350

Ser Asp Leu Lys Gln Tyr Met His Glu Gly Glu Gln Val Pro Ala Asp
        355                 360                 365

Trp Lys Ser Asp Arg Ala His Pro Leu Glu Ile Val Lys Glu Leu Arg
    370                 375                 380

Asn Ala Val Asp Asp His Val Thr Val Thr Cys Asp Ile Gly Ser His
385                 390                 395                 400

Ala Ile Trp Met Ser Arg Tyr Phe Arg Ser Tyr Glu Pro Leu Thr Leu
                405                 410                 415

Met Ile Ser Asn Gly Met Gln Thr Leu Gly Val Ala Leu Pro Trp Ala
            420                 425                 430
```

Ile Gly Ala Ser Leu Val Lys Pro Gly Glu Lys Val Val Ser Val Ser
435 440 445

Gly Asp Gly Gly Phe Leu Phe Ser Ala Met Glu Leu Glu Thr Ala Val
450 455 460

Arg Leu Lys Ala Pro Ile Val His Ile Val Trp Asn Asp Ser Thr Tyr
465 470 475 480

Asp Met Val Ala Phe Gln Gln Leu Lys Lys Tyr Asn Arg Thr Ser Ala
485 490 495

Val Asp Phe Gly Asn Ile Asp Ile Val Lys Tyr Ala Glu Ser Phe Gly
500 505 510

Ala Thr Gly Leu Arg Val Glu Ser Pro Asp Gln Leu Ala Asp Val Leu
515 520 525

Arg Gln Gly Met Asn Ala Glu Gly Pro Val Ile Ile Asp Val Pro Val
530 535 540

Asp Tyr Ser Asp Asn Ile Asn Leu Ala Ser Asp Lys Leu Pro Lys Glu
545 550 555 560

Phe Gly Glu Leu Met Lys Thr Lys Ala Leu
565 570

<210> 2
<211> 1716
<212> DNA
<213> Bacillus subtilis

<400> 2

atgttgacaa aagcaacaaa agaacaaaaa tcccttgtga aaaacagagg ggcggagctt 60

gttgttgatt gcttagtgga gcaaggtgtc acacatgtat ttggcattcc aggtgcaaaa 120

attgatgcgg tatttgacgc tttacaagat aaaggacctg aaattatcgt tgcccggcac 180

gaacaaaacg cagcattcat ggcccaagca gtcggccgtt taactggaaa accgggagtc 240

gtgttagtca catcaggacc gggtgcctct aacttggcaa caggcctgct gacagcgaac 300

actgaaggag accctgtcgt tgcgcttgct ggaaacgtga tccgtgcaga tcgtttaaaa 360

cggacacatc aatctttgga taatgcggcg ctattccagc cgattacaaa atacagtgta 420

gaagttcaag atgtaaaaaa tataccggaa gctgttacaa atgcatttag gatagcgtca 480

gcagggcagg ctggggccgc ttttgtgagc tttccgcaag atgttgtgaa tgaagtcaca 540

aatacgaaaa acgtgcgtgc tgttgcagcg ccaaaactcg gtcctgcagc agatgatgca 600

```
atcagtgcgg ccatagcaaa aatccaaaca gcaaaacttc ctgtcgtttt ggtcggcatg      660
aaaggcggaa gaccggaagc aattaaagcg gttcgcaagc ttttgaaaaa ggttcagctt      720
ccatttgttg aaacatatca agctgccggt accctttcta gagatttaga ggatcaatat      780
tttggccgta tcggtttgtt ccgcaaccag cctggcgatt tactgctaga gcaggcagat      840
gttgttctga cgatcggcta tgacccgatt gaatatgatc cgaaattctg gaatatcaat      900
ggagaccgga caattatcca tttagacgag attatcgctg acattgatca tgcttaccag      960
cctgatcttg aattgatcgg tgacattccg tccacgatca atcatatcga acacgatgct     1020
gtgaaagtgg aatttgcaga gcgtgagcag aaaatccttt ctgatttaaa acaatatatg     1080
catgaaggtg agcaggtgcc tgcagattgg aaatcagaca gagcgcaccc tcttgaaatc     1140
gttaaagagt tgcgtaatgc agtcgatgat catgttacag taacttgcga tatcggttcg     1200
cacgccattt ggatgtcacg ttatttccgc agctacgagc cgttaacatt aatgatcagt     1260
aacggtatgc aaacactcgg cgttgcgctt ccttgggcaa tcggcgcttc attggtgaaa     1320
ccgggagaaa aagtggtttc tgtctctggt gacggcggtt tcttattctc agcaatggaa     1380
ttagagacag cagttcgact aaaagcacca attgtacaca ttgtatggaa cgacagcaca     1440
tatgacatgg ttgcattcca gcaattgaaa aaatataacc gtacatctgc ggtcgatttc     1500
ggaaatatcg atatcgtgaa atatgcggaa agcttcggag caactggctt gcgcgtagaa     1560
tcaccagacc agctggcaga tgttctgcgt caaggcatga acgctgaagg tcctgtcatc     1620
atcgatgtcc cggttgacta cagtgataac attaatttag caagtgacaa gcttccgaaa     1680
gaattcgggg aactcatgaa aacgaaagct ctctag                               1716
```

<210> 3
<211> 559
<212> PRT
<213> Klebsiella pneumoniae

<400> 3

```
Met Asp Lys Gln Tyr Pro Val Arg Gln Trp Ala His Gly Ala Asp Leu
1               5                   10                  15

Val Val Ser Gln Leu Glu Ala Gln Gly Val Arg Gln Val Phe Gly Ile
            20                  25                  30

Pro Gly Ala Lys Ile Asp Lys Val Phe Asp Ser Leu Leu Asp Ser Ser
        35                  40                  45

Ile Arg Ile Ile Pro Val Arg His Glu Ala Asn Ala Ala Phe Met Ala
    50                  55                  60

Ala Ala Val Gly Arg Ile Thr Gly Lys Ala Gly Val Ala Leu Val Thr
```

65 70 75 80

Ser Gly Pro Gly Cys Ser Asn Leu Ile Thr Gly Met Ala Thr Ala Asn
85 90 95

Ser Glu Gly Asp Pro Val Val Ala Leu Gly Gly Ala Val Lys Arg Ala
100 105 110

Asp Lys Ala Lys Gln Val His Gln Ser Met Asp Thr Val Ala Met Phe
115 120 125

Ser Pro Val Thr Lys Tyr Ala Ile Glu Val Thr Ala Pro Asp Ala Leu
130 135 140

Ala Glu Val Val Ser Asn Ala Phe Arg Ala Ala Glu Gln Gly Arg Pro
145 150 155 160

Gly Ser Ala Phe Val Ser Leu Pro Gln Asp Val Val Asp Gly Pro Val
165 170 175

Ser Gly Lys Val Leu Pro Ala Ser Gly Ala Pro Gln Met Gly Ala Ala
180 185 190

Pro Asp Asp Ala Ile Asp Gln Val Ala Lys Leu Ile Ala Gln Ala Lys
195 200 205

Asn Pro Ile Phe Leu Leu Gly Leu Met Ala Ser Gln Pro Glu Asn Ser
210 215 220

Lys Ala Leu Arg Arg Leu Leu Glu Thr Ser His Ile Pro Val Thr Ser
225 230 235 240

Thr Tyr Gln Ala Ala Gly Ala Val Asn Gln Asp Asn Phe Ser Arg Phe
245 250 255

Ala Gly Arg Val Gly Leu Phe Asn Asn Gln Ala Gly Asp Arg Leu Leu
260 265 270

Gln Leu Ala Asp Leu Val Ile Cys Ile Gly Tyr Ser Pro Val Glu Tyr
275 280 285

Glu Pro Ala Met Trp Asn Ser Gly Asn Ala Thr Leu Val His Ile Asp
290 295 300

Val Leu Pro Ala Tyr Glu Glu Arg Asn Tyr Thr Pro Asp Val Glu Leu
305 310 315 320

Val Gly Asp Ile Ala Gly Thr Leu Asn Lys Leu Ala Gln Asn Ile Asp
325 330 335

His Arg Leu Val Leu Ser Pro Gln Ala Ala Glu Ile Leu Arg Asp Arg
340 345 350

Gln His Gln Arg Glu Leu Leu Asp Arg Arg Gly Ala Gln Leu Asn Gln
355 360 365

Phe Ala Leu His Pro Leu Arg Ile Val Arg Ala Met Gln Asp Ile Val
370 375 380

Asn Ser Asp Val Thr Leu Thr Val Asp Met Gly Ser Phe His Ile Trp
385 390 395 400

Ile Ala Arg Tyr Leu Tyr Thr Phe Arg Ala Arg Gln Val Met Ile Ser
405 410 415

Asn Gly Gln Gln Thr Met Gly Val Ala Leu Pro Trp Ala Ile Gly Ala
420 425 430

Trp Leu Val Asn Pro Glu Arg Lys Val Val Ser Val Ser Gly Asp Gly
435 440 445

Gly Phe Leu Gln Ser Ser Met Glu Leu Glu Thr Ala Val Arg Leu Lys
450 455 460

Ala Asn Val Leu His Leu Ile Trp Val Asp Asn Gly Tyr Asn Met Val
465 470 475 480

Ala Ile Gln Glu Glu Lys Lys Tyr Gln Arg Leu Ser Gly Val Glu Phe
485 490 495

Gly Pro Met Asp Phe Lys Ala Tyr Ala Glu Ser Phe Gly Ala Lys Gly
500 505 510

Phe Ala Val Glu Ser Ala Glu Ala Leu Glu Pro Thr Leu Arg Ala Ala
515 520 525

Met Asp Val Asp Gly Pro Ala Val Val Ala Ile Pro Val Asp Tyr Arg
530 535 540

Asp Asn Pro Leu Leu Met Gly Gln Leu His Leu Ser Gln Ile Leu
545 550 555

<210> 4
<211> 2055

<212> DNA
<213> Klebsiella pneumoniae

<400> 4

tcgaccacgg ggtgctgacc ttcggcgaaa ttcacaagct gatgatcgac ctgcccgccg 60
acagcgcgtt cctgcaggct aatctgcatc ccgataatct cgatgccgcc atccgttccg 120
tagaaagtta aggggtcac atggacaaac agtatccggt acgccagtgg gcgcacggcg 180
ccgatctcgt cgtcagtcag ctggaagctc agggagtacg ccaggtgttc ggcatccccg 240
gcgccaaaat cgacaaggtc tttgattcac tgctggattc ctccattcgc attattccgg 300
tacgccacga agccaacgcc gcatttatgg ccgccgccgt cggacgcatt accggcaaag 360
cgggcgtggc gctggtcacc tccggtccgg gctgttccaa cctgatcacc ggcatggcca 420
ccgcgaacag cgaaggcgac ccggtggtgg ccctgggcgg cgcggtaaaa cgcgccgata 480
aagcgaagca ggtccaccag agtatggata cggtggcgat gttcagcccg gtcaccaaat 540
acgccatcga ggtgacggcg ccggatgcgc tggcggaagt ggtctccaac gccttccgcg 600
ccgccgagca gggccggccg ggcagcgcgt tcgttagcct gccgcaggat gtggtcgatg 660
gcccggtcag cggcaaagtg ctgccggcca gcggggcccc gcagatgggc gccgcgccgg 720
atgatgccat cgaccaggtg gcgaagctta tcgcccaggc gaagaacccg atcttcctgc 780
tcggcctgat ggccagccag ccggaaaaca gcaaggcgct gcgccgtttg ctggagacca 840
gccatattcc agtcaccagc acctatcagg ccgccggagc ggtgaatcag gataacttct 900
ctcgcttcgc cggccgggtt gggctgttta acaaccaggc cggggaccgt ctgctgcagc 960
tcgccgacct ggtgatctgc atcggctaca gcccggtgga atacgaaccg gcgatgtgga 1020
acagcggcaa cgcgacgctg gtgcacatcg acgtgctgcc cgcctatgaa gagcgcaact 1080
acaccccgga tgtcgagctg gtgggcgata tcgccggcac tctcaacaag ctggcgcaaa 1140
atatcgatca tcggctggtg ctctccccgc aggcggcgga gatcctccgc gaccgccagc 1200
accagcgcga gctgctggac cgccgcggcg cgcagctcaa ccagtttgcc ctgcatcccc 1260
tgcgcatcgt tcgcgccatg caggatatcg tcaacagcga cgtcacgttg accgtggaca 1320
tgggcagctt ccatatctgg attgcccgct acctgtacac gttccgcgcc cgtcaggtga 1380
tgatctccaa cggccagcag accatgggcg tcgccctgcc ctgggctatc ggcgcctggc 1440
tggtcaatcc tgagcgcaaa gtggtctccg tctccggcga cggcggcttc ctgcagtcga 1500
gcatggagct ggagaccgcc gtccgcctga aagccaacgt gctgcatctt atctgggtcg 1560
ataacggcta caacatggtc gctatccagg aagagaaaaa atatcagcgc ctgtccggcg 1620
tcgagtttgg gccgatggat tttaaagcct atgccgaatc cttcggcgcg aaagggtttg 1680
ccgtggaaag cgccgaggcg ctggagccga ccctgcgcgc ggcgatggac gtcgacggcc 1740
cggcggtagt ggccatcccg gtggattatc gcgataaccc gctgctgatg ggccagctgc 1800

```
atctgagtca gattctgtaa gtcatcacaa taaggaaaga aaaatgaaaa aagtcgcact      1860
tgttaccggc gccggccagg ggattggtaa agctatcgcc cttcgtctgg tgaaggatgg      1920
atttgccgtg gccattgccg attataacga cgccaccgcc aaagcggtcg cctccgaaat      1980
caaccaggcc ggcggccgcg ccatggcggt gaaagtggat gtttctgacc gcgaccaggt      2040
atttgccgcc gtcga                                                       2055
```

<210> 5
<211> 554
<212> PRT
<213> Lactococcus lactis

<400> 5

```
Met Ser Glu Lys Gln Phe Gly Ala Asn Leu Val Val Asp Ser Leu Ile
1               5                   10                  15

Asn His Lys Val Lys Tyr Val Phe Gly Ile Pro Gly Ala Lys Ile Asp
            20                  25                  30

Arg Val Phe Asp Leu Leu Glu Asn Glu Glu Gly Pro Gln Met Val Val
        35                  40                  45

Thr Arg His Glu Gln Gly Ala Ala Phe Met Ala Gln Ala Val Gly Arg
    50                  55                  60

Leu Thr Gly Glu Pro Gly Val Val Val Val Thr Ser Gly Pro Gly Val
65                  70                  75                  80

Ser Asn Leu Ala Thr Pro Leu Leu Thr Ala Thr Ser Glu Gly Asp Ala
                85                  90                  95

Ile Leu Ala Ile Gly Gly Gln Val Lys Arg Ser Asp Arg Leu Lys Arg
            100                 105                 110

Ala His Gln Ser Met Asp Asn Ala Gly Met Met Gln Ser Ala Thr Lys
        115                 120                 125

Tyr Ser Ala Glu Val Leu Asp Pro Asn Thr Leu Ser Glu Ser Ile Ala
    130                 135                 140

Asn Ala Tyr Arg Ile Ala Lys Ser Gly His Pro Gly Ala Thr Phe Leu
145                 150                 155                 160

Ser Ile Pro Gln Asp Val Thr Asp Ala Glu Val Ser Ile Lys Ala Ile
                165                 170                 175

Gln Pro Leu Ser Asp Pro Lys Met Gly Asn Ala Ser Ile Asp Asp Ile
```

180 185 190

Asn Tyr Leu Ala Gln Ala Ile Lys Asn Ala Val Leu Pro Val Ile Leu
195 200 205

Val Gly Ala Gly Ala Ser Asp Ala Lys Val Ala Ser Ser Leu Arg Asn
210 215 220

Leu Leu Thr His Val Asn Ile Pro Val Val Glu Thr Phe Gln Gly Ala
225 230 235 240

Gly Val Ile Ser His Asp Leu Glu His Thr Phe Tyr Gly Arg Ile Gly
245 250 255

Leu Phe Arg Asn Gln Pro Gly Asp Met Leu Leu Lys Arg Ser Asp Leu
260 265 270

Val Ile Ala Val Gly Tyr Asp Pro Ile Glu Tyr Glu Ala Arg Asn Trp
275 280 285

Asn Ala Glu Ile Asp Ser Arg Ile Ile Val Ile Asp Asn Ala Ile Ala
290 295 300

Glu Ile Asp Thr Tyr Tyr Gln Pro Glu Arg Glu Leu Ile Gly Asp Ile
305 310 315 320

Ala Ala Thr Leu Asp Asn Leu Leu Pro Ala Val Arg Gly Tyr Lys Ile
325 330 335

Pro Lys Gly Thr Lys Asp Tyr Leu Asp Gly Leu His Glu Val Ala Glu
340 345 350

Gln His Glu Phe Asp Thr Glu Asn Thr Glu Glu Gly Arg Met His Pro
355 360 365

Leu Asp Leu Val Ser Thr Phe Gln Glu Ile Val Lys Asp Asp Glu Thr
370 375 380

Val Thr Val Asp Val Gly Ser Leu Tyr Ile Trp Met Ala Arg His Phe
385 390 395 400

Lys Ser Tyr Glu Pro Arg His Leu Leu Phe Ser Asn Gly Met Gln Thr
405 410 415

Leu Gly Val Ala Leu Pro Trp Ala Ile Thr Ala Ala Leu Leu Arg Pro
420 425 430

```
Gly Lys Lys Val Tyr Ser His Ser Gly Asp Gly Gly Phe Leu Phe Thr
        435                 440                 445

Gly Gln Glu Leu Glu Thr Ala Val Arg Leu Asn Leu Pro Ile Val Gln
    450                 455                 460

Ile Ile Trp Asn Asp Gly His Tyr Asp Met Val Lys Phe Gln Glu Glu
465                 470                 475                 480

Met Lys Tyr Gly Arg Ser Ala Ala Val Asp Phe Gly Tyr Val Asp Tyr
                485                 490                 495

Val Lys Tyr Ala Glu Ala Met Arg Ala Lys Gly Tyr Arg Ala His Ser
            500                 505                 510

Lys Glu Glu Leu Ala Glu Ile Leu Lys Ser Ile Pro Asp Thr Thr Gly
        515                 520                 525

Pro Val Val Ile Asp Val Pro Leu Asp Tyr Ser Asp Asn Ile Lys Leu
    530                 535                 540

Ala Glu Lys Leu Leu Pro Glu Glu Phe Tyr
545                 550
```

<210> 6
<211> 3220
<212> DNA
<213> Lactococcus lactis

<400> 6

tagatccgga aacaactgat tacctgagtt aacttagcag aaattgcaga agataacggt 60
aatttggatg aagcattaaa ttacctttat caaattccgg tgaatgatga aaattatatt 120
gctgctttaa tcaaaattgc tgacttatat caatttgaag ttgattttga aacagcaatt 180
tctaagttag aagaagcaag agaattatcg gattctcctc tgattacttt tgctttggct 240
gagtcctact ttgaacaagg tgattattca gctgccatta ccgaatatgc aaaactttca 300
gaacgaaaaa ttttacatga aacaaaaatt tctatttatc aaagaattgg tgactcttat 360
gcccaattag gtaattttga gaatgccata tcatttcttg aaaaatcact tgaatttgat 420
gaaaaaccgg aaaccttgta taaaattgct cttctttatg gagaaactca taatgaaaca 480
agagccattg ctaatttcaa acggttagaa aaaatggatg ttgaattttt gaactatgaa 540
ttagcctatg cccaaaccct agaagctaat caagaattta aagctgcact agaaatggca 600
aagaaaggga tgaaaaaaaa tcctaatgcc gttcctctct tacacttcgc ttcaaaaatt 660
tgtttcaaac ttaaggacaa agctgcagca gaacgttatc tcgtggatgc tttaaattta 720
ccagaattac atgacgaaac agtctttttg cttgctaatt tatacttcaa cgaagaagat 780

tttgaagctg tcattaatct tgaagagctt ttagaagatg aacatttatt agctaaatgg 840

ctttttgcag gagcacataa agctttggaa aatgattctg aagcggctgc tttgtatgaa 900

gaactcattc aaaccaatct gtcagagaat ccagagtttt tagaagacta tattgatttt 960

cttaaagaaa ttggtcaaat ttctaaaaca gaaccaatta ttgaacaata tttggaactt 1020

gttccagatg atgaaaatat gagaaattta ctgacagact taaaaaataa ttactgacaa 1080

agctgtcagt aattattttt attgtaagct agaaaattca aaaacttgcg tcaaaataat 1140

tgtaaaaggt tctattatct gataaaatga ttgtgaagta atccaagaga ttatgaaata 1200

tgaattagaa caaatagagg taaaataaaa aatgtctgag aaacaatttg gggcgaactt 1260

ggttgtcgat agtttgatta accataaagt gaagtatgta tttgggattc caggagcaaa 1320

aattgaccgg gtttttgatt tattagaaaa tgaagaaggc cctcaaatgg tcgtgactcg 1380

tcatgagcaa ggagctgctt tcatggctca agctgtcggt cgtttaactg gcgaacctgg 1440

tgtagtagtt gttacgagtg ggcctggtgt atcaaacctt gcgactccgc ttttgaccgc 1500

gacatcagaa ggtgatgcta ttttggctat cggtggacaa gttaaacgaa gtgaccgtct 1560

taaacgtgcg caccaatcaa tggataatgc tggaatgatg caatcagcaa caaaatattc 1620

agcagaagtt cttgacccta atacactttc tgaatcaatt gccaacgctt atcgtattgc 1680

aaaatcagga catccaggtg caactttctt atcaatcccc caagatgtaa cggatgccga 1740

agtatcaatc aaagccattc aaccactttc agaccctaaa atggggaatg cctctattga 1800

tgacattaat tatttagcac aagcaattaa aaatgctgta ttgccagtaa ttttggttgg 1860

agctggtgct tcagatgcta aagtcgcttc atccttgcgt aatctattga ctcatgttaa 1920

tattcctgtc gttgaaacat tccaaggtgc aggggttatt tcacatgatt tagaacatac 1980

tttttatgga cgtatcggtc ttttccgcaa tcaaccaggc gatatgcttc tgaaacgttc 2040

tgaccttgtt attgctgttg gttatgaccc aattgaatat gaagctcgta actggaatgc 2100

agaaattgat agtcgaatta tcgttattga taatgccatt gctgaaattg atacttacta 2160

ccaaccagag cgtgaattaa ttggtgatat cgcagcaaca ttggataatc ttttaccagc 2220

tgttcgtggc tacaaaattc caaaaggaac aaaagattat ctcgatggcc ttcatgaagt 2280

tgctgagcaa cacgaatttg atactgaaaa tactgaagaa ggtagaatgc accctcttga 2340

tttggtcagc actttccaag aaatcgtcaa ggatgatgaa acagtaaccg ttgacgtagg 2400

ttcactctac atttggatgg cacgtcattt caaatcatac gaaccacgtc atctcctctt 2460

ctcaaacgga atgcaaacac tcggagttgc acttccttgg gcaattacag ccgcattgtt 2520

gcgcccaggt aaaaaagttt attcacactc tggtgatgga ggcttccttt tcacagggca 2580

agaattggaa acagctgtac gtttgaatct tccaatcgtt caaattatct ggaatgacgg 2640

ccattatgat atggttaaat tccaagaaga aatgaaatat ggtcgttcag cagccgttga 2700

ttttggctat gttgattacg taaaatatgc tgaagcaatg agagcaaaag gttaccgtgc 2760

acacagcaaa gaagaacttg ctgaaattct caaatcaatc ccagatacta ctggaccggt 2820

ggtaattgac gttcctttgg actattctga taacattaaa ttagcagaaa aattattgcc 2880

tgaagagttt tattgattac aatcaagcaa tttgtggcat aacaaaataa aagaagaagg 2940

ccttgaacac ctaagcgttc agggcctttt tttgtgaaat aaattagatg aaatttacaa 3000

tgagttttgt gaaactagct tctagtttgt gaaaaattgc ctataattgc cgaataaaaa 3060

tacccattta ccactccaag aggatgcttc aaattagcta aatacccgtt ttagaggatg 3120

cgtaaaaaca acaaaagagg atgagtatag aacgataaaa ctttttttatg ataggttgag 3180

agaattgaat ataaaatata ataagtagaa ggcagcaatt 3220

<210> 7
<211> 491
<212> PRT
<213> Escherichia coli

<400> 7

Met Ala Asn Tyr Phe Asn Thr Leu Asn Leu Arg Gln Gln Leu Ala Gln
1               5                   10                  15

Leu Gly Lys Cys Arg Phe Met Gly Arg Asp Glu Phe Ala Asp Gly Ala
            20                  25                  30

Ser Tyr Leu Gln Gly Lys Lys Val Val Ile Val Gly Cys Gly Ala Gln
        35                  40                  45

Gly Leu Asn Gln Gly Leu Asn Met Arg Asp Ser Gly Leu Asp Ile Ser
    50                  55                  60

Tyr Ala Leu Arg Lys Glu Ala Ile Ala Glu Lys Arg Ala Ser Trp Arg
65                  70                  75                  80

Lys Ala Thr Glu Asn Gly Phe Lys Val Gly Thr Tyr Glu Glu Leu Ile
                85                  90                  95

Pro Gln Ala Asp Leu Val Ile Asn Leu Thr Pro Asp Lys Gln His Ser
            100                 105                 110

Asp Val Val Arg Thr Val Gln Pro Leu Met Lys Asp Gly Ala Ala Leu
        115                 120                 125

Gly Tyr Ser His Gly Phe Asn Ile Val Glu Val Gly Glu Gln Ile Arg
    130                 135                 140

```
Lys Asp Ile Thr Val Val Met Val Ala Pro Lys Cys Pro Gly Thr Glu
145                 150                 155                 160

Val Arg Glu Glu Tyr Lys Arg Gly Phe Gly Val Pro Thr Leu Ile Ala
                165                 170                 175

Val His Pro Glu Asn Asp Pro Lys Gly Glu Gly Met Ala Ile Ala Lys
            180                 185                 190

Ala Trp Ala Ala Ala Thr Gly Gly His Arg Ala Gly Val Leu Glu Ser
        195                 200                 205

Ser Phe Val Ala Glu Val Lys Ser Asp Leu Met Gly Glu Gln Thr Ile
    210                 215                 220

Leu Cys Gly Met Leu Gln Ala Gly Ser Leu Leu Cys Phe Asp Lys Leu
225                 230                 235                 240

Val Glu Glu Gly Thr Asp Pro Ala Tyr Ala Glu Lys Leu Ile Gln Phe
                245                 250                 255

Gly Trp Glu Thr Ile Thr Glu Ala Leu Lys Gln Gly Gly Ile Thr Leu
            260                 265                 270

Met Met Asp Arg Leu Ser Asn Pro Ala Lys Leu Arg Ala Tyr Ala Leu
        275                 280                 285

Ser Glu Gln Leu Lys Glu Ile Met Ala Pro Leu Phe Gln Lys His Met
    290                 295                 300

Asp Asp Ile Ile Ser Gly Glu Phe Ser Ser Gly Met Met Ala Asp Trp
305                 310                 315                 320

Ala Asn Asp Asp Lys Lys Leu Leu Thr Trp Arg Glu Glu Thr Gly Lys
                325                 330                 335

Thr Ala Phe Glu Thr Ala Pro Gln Tyr Glu Gly Lys Ile Gly Glu Gln
            340                 345                 350

Glu Tyr Phe Asp Lys Gly Val Leu Met Ile Ala Met Val Lys Ala Gly
        355                 360                 365

Val Glu Leu Ala Phe Glu Thr Met Val Asp Ser Gly Ile Ile Glu Glu
    370                 375                 380

Ser Ala Tyr Tyr Glu Ser Leu His Glu Leu Pro Leu Ile Ala Asn Thr
385                 390                 395                 400
```

```
Ile Ala Arg Lys Arg Leu Tyr Glu Met Asn Val Val Ile Ser Asp Thr
                405                 410                 415

Ala Glu Tyr Gly Asn Tyr Leu Phe Ser Tyr Ala Cys Val Pro Leu Leu
            420                 425                 430

Lys Pro Phe Met Ala Glu Leu Gln Pro Gly Asp Leu Gly Lys Ala Ile
        435                 440                 445

Pro Glu Gly Ala Val Asp Asn Gly Gln Leu Arg Asp Val Asn Glu Ala
    450                 455                 460

Ile Arg Ser His Ala Ile Glu Gln Val Gly Lys Lys Leu Arg Gly Tyr
465                 470                 475                 480

Met Thr Asp Met Lys Arg Ile Ala Val Ala Gly
                485                 490
```

<210> 8
<211> 1476
<212> DNA
<213> Escherichia coli

<400> 8

```
atggctaact acttcaatac actgaatctg cgccagcagc tggcacagct gggcaaatgt      60
cgctttatgg gccgcgatga attcgccgat ggcgcgagct accttcaggg taaaaaagta     120
gtcatcgtcg gctgtggcgc acagggtctg aaccagggcc tgaacatgcg tgattctggt     180
ctcgatatct cctacgctct gcgtaaagaa gcgattgccg agaagcgcgc gtcctggcgt     240
aaagcgaccg aaaatggttt taaagtgggt acttacgaag aactgatccc acaggcggat     300
ctggtgatta acctgacgcc ggacaagcag cactctgatg tagtgcgcac cgtacagcca     360
ctgatgaaag acggcgcggc gctgggctac tcgcacggtt tcaacatcgt cgaagtgggc     420
gagcagatcc gtaaagatat caccgtagtg atggttgcgc cgaaatgccc aggcaccgaa     480
gtgcgtgaag agtacaaacg tgggttcggc gtaccgacgc tgattgccgt tcacccggaa     540
aacgatccga aaggcgaagg catggcgatt gccaaagcct gggcggctgc aaccggtggt     600
caccgtgcgg gtgtgctgga atcgtccttc gttgcggaag tgaaatctga cctgatgggc     660
gagcaaacca tcctgtgcgg tatgttgcag gctggctctc tgctgtgctt cgacaagctg     720
gtggaagaag gtaccgatcc agcatacgca gaaaaactga ttcagttcgg ttgggaaacc     780
atcaccgaag cactgaaaca gggcggcatc accctgatga tggaccgtct ctctaacccg     840
gcgaaactgc gtgcttatgc gctttctgaa cagctgaaag agatcatggc acccctgttc     900
cagaaacata tggacgacat catctccggc gaattctctt ccggtatgat ggcggactgg     960

gccaacgatg ataagaaact gctgacctgg cgtgaagaga ccggcaaaac cgcgtttgaa    1020
accgcgccgc agtatgaagg caaaatcggc gagcaggagt acttcgataa aggcgtactg    1080
atgattgcga tggtgaaagc gggcgttgaa ctggcgttcg aaaccatggt cgattccggc    1140
atcattgaag agtctgcata ttatgaatca ctgcacgagc tgccgctgat tgccaacacc    1200
atcgcccgta agcgtctgta cgaaatgaac gtggttatct ctgataccgc tgagtacggt    1260
aactatctgt tctcttacgc ttgtgtgccg ttgctgaaac cgtttatggc agagctgcaa    1320
ccgggcgacc tgggtaaagc tattccggaa ggcgcggtag ataacgggca actgcgtgat    1380
gtgaacgaag cgattcgcag ccatgcgatt gagcaggtag gtaagaaact gcgcggctat    1440
atgacagata tgaaacgtat tgctgttgcg ggttaa                              1476
```

<210> 9
<211> 395
<212> PRT
<213> Saccharomyces cerevisiae

<400> 9

```
Met Leu Arg Thr Gln Ala Ala Arg Leu Ile Cys Asn Ser Arg Val Ile
1               5                   10                  15

Thr Ala Lys Arg Thr Phe Ala Leu Ala Thr Arg Ala Ala Ala Tyr Ser
            20                  25                  30

Arg Pro Ala Ala Arg Phe Val Lys Pro Met Ile Thr Thr Arg Gly Leu
        35                  40                  45

Lys Gln Ile Asn Phe Gly Gly Thr Val Glu Thr Val Tyr Glu Arg Ala
    50                  55                  60

Asp Trp Pro Arg Glu Lys Leu Leu Asp Tyr Phe Lys Asn Asp Thr Phe
65                  70                  75                  80

Ala Leu Ile Gly Tyr Gly Ser Gln Gly Tyr Gly Gln Gly Leu Asn Leu
                85                  90                  95

Arg Asp Asn Gly Leu Asn Val Ile Ile Gly Val Arg Lys Asp Gly Ala
            100                 105                 110

Ser Trp Lys Ala Ala Ile Glu Asp Gly Trp Val Pro Gly Lys Asn Leu
        115                 120                 125

Phe Thr Val Glu Asp Ala Ile Lys Arg Gly Ser Tyr Val Met Asn Leu
    130                 135                 140
```

```
Leu Ser Asp Ala Ala Gln Ser Glu Thr Trp Pro Ala Ile Lys Pro Leu
145                 150                 155                 160

Leu Thr Lys Gly Lys Thr Leu Tyr Phe Ser His Gly Phe Ser Pro Val
                165                 170                 175

Phe Lys Asp Leu Thr His Val Glu Pro Pro Lys Asp Leu Asp Val Ile
            180                 185                 190

Leu Val Ala Pro Lys Gly Ser Gly Arg Thr Val Arg Ser Leu Phe Lys
        195                 200                 205

Glu Gly Arg Gly Ile Asn Ser Ser Tyr Ala Val Trp Asn Asp Val Thr
    210                 215                 220

Gly Lys Ala His Glu Lys Ala Gln Ala Leu Ala Val Ala Ile Gly Ser
225                 230                 235                 240

Gly Tyr Val Tyr Gln Thr Thr Phe Glu Arg Glu Val Asn Ser Asp Leu
                245                 250                 255

Tyr Gly Glu Arg Gly Cys Leu Met Gly Gly Ile His Gly Met Phe Leu
            260                 265                 270

Ala Gln Tyr Asp Val Leu Arg Glu Asn Gly His Ser Pro Ser Glu Ala
        275                 280                 285

Phe Asn Glu Thr Val Glu Glu Ala Thr Gln Ser Leu Tyr Pro Leu Ile
    290                 295                 300

Gly Lys Tyr Gly Met Asp Tyr Met Tyr Asp Ala Cys Ser Thr Thr Ala
305                 310                 315                 320

Arg Arg Gly Ala Leu Asp Trp Tyr Pro Ile Phe Lys Asn Ala Leu Lys
                325                 330                 335

Pro Val Phe Gln Asp Leu Tyr Glu Ser Thr Lys Asn Gly Thr Glu Thr
            340                 345                 350

Lys Arg Ser Leu Glu Phe Asn Ser Gln Pro Asp Tyr Arg Glu Lys Leu
        355                 360                 365

Glu Lys Glu Leu Asp Thr Ile Arg Asn Met Glu Ile Trp Lys Val Gly
    370                 375                 380

Lys Glu Val Arg Lys Leu Arg Pro Glu Asn Gln
385                 390                 395
```

<210> 10
<211> 1188
<212> DNA
<213> Saccharomyces cerevisiae

<400> 10

```
atgttgagaa ctcaagccgc cagattgatc tgcaactccc gtgtcatcac tgctaagaga      60
acctttgctt tggccacccg tgctgctgct tacagcagac cagctgcccg tttcgttaag     120
ccaatgatca ctacccgtgg tttgaagcaa atcaacttcg gtggtactgt tgaaaccgtc     180
tacgaaagag ctgactggcc aagagaaaag ttgttggact acttcaagaa cgacactttt     240
gctttgatcg gttacggttc ccaaggttac ggtcaaggtt tgaacttgag agacaacggt     300
ttgaacgtta tcattggtgt ccgtaaagat ggtgcttctt ggaaggctgc catcgaagac     360
ggttgggttc caggcaagaa cttgttcact gttgaagatg ctatcaagag aggtagttac     420
gttatgaact tgttgtccga tgccgctcaa tcagaaacct ggcctgctat caagccattg     480
ttgaccaagg gtaagacttt gtacttctcc cacggtttct ccccagtctt caaggacttg     540
actcacgttg aaccaccaaa ggacttagat gttatcttgg ttgctccaaa gggttccggt     600
agaactgtca gatctttgtt caaggaaggt cgtggtatta actcttctta cgccgtctgg     660
aacgatgtca ccggtaaggc tcacgaaaag gcccaagctt tggccgttgc cattggttcc     720
ggttacgttt accaaaccac tttcgaaaga gaagtcaact ctgacttgta cggtgaaaga     780
ggttgtttaa tgggtggtat ccacggtatg ttcttggctc aatacgacgt cttgagagaa     840
aacggtcact ccccatctga agctttcaac gaaaccgtcg aagaagctac ccaatctcta     900
tacccattga tcggtaagta cggtatggat tacatgtacg atgcttgttc caccaccgcc     960
agaagaggtg ctttggactg gtacccaatc ttcaagaatg ctttgaagcc tgttttccaa    1020
gacttgtacg aatctaccaa gaacggtacc gaaaccaaga gatctttgga attcaactct    1080
caacctgact acagagaaaa gctagaaaag gaattagaca ccatcagaaa catggaaatc    1140
tggaaggttg gtaaggaagt cagaaagttg agaccagaaa accaataa                 1188
```

<210> 11
<211> 330
<212> PRT
<213> Methanococcus maripaludis

<400> 11

```
Met Lys Val Phe Tyr Asp Ser Asp Phe Lys Leu Asp Ala Leu Lys Glu
1               5                   10                  15

Lys Thr Ile Ala Val Ile Gly Tyr Gly Ser Gln Gly Arg Ala Gln Ser
            20                  25                  30
```

```
Leu Asn Met Lys Asp Ser Gly Leu Asn Val Val Val Gly Leu Arg Lys
        35                  40                  45

Asn Gly Ala Ser Trp Asn Asn Ala Lys Ala Asp Gly His Asn Val Met
    50                  55                  60

Thr Ile Glu Glu Ala Ala Glu Lys Ala Asp Ile Ile His Ile Leu Ile
65                  70                  75                  80

Pro Asp Glu Leu Gln Ala Glu Val Tyr Glu Ser Gln Ile Lys Pro Tyr
                85                  90                  95

Leu Lys Glu Gly Lys Thr Leu Ser Phe Ser His Gly Phe Asn Ile His
            100                 105                 110

Tyr Gly Phe Ile Val Pro Pro Lys Gly Val Asn Val Val Leu Val Ala
        115                 120                 125

Pro Lys Ser Pro Gly Lys Met Val Arg Arg Thr Tyr Glu Glu Gly Phe
    130                 135                 140

Gly Val Pro Gly Leu Ile Cys Ile Glu Ile Asp Ala Thr Asn Asn Ala
145                 150                 155                 160

Phe Asp Ile Val Ser Ala Met Ala Lys Gly Ile Gly Leu Ser Arg Ala
                165                 170                 175

Gly Val Ile Gln Thr Thr Phe Lys Glu Glu Thr Glu Thr Asp Leu Phe
            180                 185                 190

Gly Glu Gln Ala Val Leu Cys Gly Gly Val Thr Glu Leu Ile Lys Ala
        195                 200                 205

Gly Phe Glu Thr Leu Val Glu Ala Gly Tyr Ala Pro Glu Met Ala Tyr
    210                 215                 220

Phe Glu Thr Cys His Glu Leu Lys Leu Ile Val Asp Leu Ile Tyr Gln
225                 230                 235                 240

Lys Gly Phe Lys Asn Met Trp Asn Asp Val Ser Asn Thr Ala Glu Tyr
                245                 250                 255

Gly Gly Leu Thr Arg Arg Ser Arg Ile Val Thr Ala Asp Ser Lys Ala
            260                 265                 270

Ala Met Lys Glu Ile Leu Arg Glu Ile Gln Asp Gly Arg Phe Thr Lys
        275                 280                 285
```

Glu Phe Leu Leu Glu Lys Gln Val Ser Tyr Ala His Leu Lys Ser Met
290 295 300

Arg Arg Leu Glu Gly Asp Leu Gln Ile Glu Glu Val Gly Ala Lys Leu
305 310 315 320

Arg Lys Met Cys Gly Leu Glu Lys Glu Glu
325 330

<210> 12
<211> 993
<212> DNA
<213> Methanococcus maripaludis

<400> 12

atgaaggtat tctatgactc agattttaaa ttagatgctt taaaagaaaa aacaattgca 60
gtaatcggtt atggaagtca aggtagggca cagtccttaa acatgaaaga cagcggatta 120
aacgttgttg ttggtttaag aaaaaacggt gcttcatgga acaacgctaa agcagacggt 180
cacaatgtaa tgaccattga agaagctgct gaaaaagcgg acatcatcca catcttaata 240
cctgatgaat tacaggcaga agtttatgaa agccagataa aaccatacct aaaagaagga 300
aaaacactaa gcttttcaca tggttttaac atccactatg gattcattgt tccaccaaaa 360
ggagttaacg tggttttagt tgctccaaaa tcacctggaa aaatggttag aagaacatac 420
gaagaaggtt tcggtgttcc aggtttaatc tgtattgaaa ttgatgcaac aaacaacgca 480
tttgatattg tttcagcaat ggcaaaagga atcggtttat caagagctgg agttatccag 540
acaactttca aagaagaaac agaaactgac cttttcggtg aacaagctgt tttatgcggt 600
ggagttaccg aattaatcaa ggcaggattt gaaacactcg ttgaagcagg atacgcacca 660
gaaatggcat actttgaaac ctgccacgaa ttgaaattaa tcgttgactt aatctaccaa 720
aaaggattca aaaacatgtg gaacgatgta agtaacactg cagaatacgg cggacttaca 780
agaagaagca gaatcgttac agctgattca aaagctgcaa tgaaagaaat cttaagagaa 840
atccaagatg gaagattcac aaaagaattc cttctcgaaa aacaggtaag ctatgctcat 900
ttaaaatcaa tgagaagact cgaaggagac ttacaaatcg aagaagtcgg cgcaaaatta 960
agaaaaatgt gcggtcttga aaaagaagaa taa 993

<210> 13
<211> 342
<212> PRT
<213> Bacillus subtilis

<400> 13

```
Met Val Lys Val Tyr Tyr Asn Gly Asp Ile Lys Glu Asn Val Leu Ala
1               5                   10                  15
```

Gly Lys Thr Val Ala Val Ile Gly Tyr Gly Ser Gln Gly His Ala His
20 25 30

Ala Leu Asn Leu Lys Glu Ser Gly Val Asp Val Ile Val Gly Val Arg
35 40 45

Gln Gly Lys Ser Phe Thr Gln Ala Gln Glu Asp Gly His Lys Val Phe
50 55 60

Ser Val Lys Glu Ala Ala Ala Gln Ala Glu Ile Ile Met Val Leu Leu
65 70 75 80

Pro Asp Glu Gln Gln Gln Lys Val Tyr Glu Ala Glu Ile Lys Asp Glu
85 90 95

Leu Thr Ala Gly Lys Ser Leu Val Phe Ala His Gly Phe Asn Val His
100 105 110

Phe His Gln Ile Val Pro Pro Ala Asp Val Asp Val Phe Leu Val Ala
115 120 125

Pro Lys Gly Pro Gly His Leu Val Arg Arg Thr Tyr Glu Gln Gly Ala
130 135 140

Gly Val Pro Ala Leu Phe Ala Ile Tyr Gln Asp Val Thr Gly Glu Ala
145 150 155 160

Arg Asp Lys Ala Leu Ala Tyr Ala Lys Gly Ile Gly Gly Ala Arg Ala
165 170 175

Gly Val Leu Glu Thr Thr Phe Lys Glu Glu Thr Glu Thr Asp Leu Phe
180 185 190

Gly Glu Gln Ala Val Leu Cys Gly Gly Leu Ser Ala Leu Val Lys Ala
195 200 205

Gly Phe Glu Thr Leu Thr Glu Ala Gly Tyr Gln Pro Glu Leu Ala Tyr
210 215 220

Phe Glu Cys Leu His Glu Leu Lys Leu Ile Val Asp Leu Met Tyr Glu
225 230 235 240

Glu Gly Leu Ala Gly Met Arg Tyr Ser Ile Ser Asp Thr Ala Gln Trp
245 250 255

Gly Asp Phe Val Ser Gly Pro Arg Val Val Asp Ala Lys Val Lys Glu

260 265 270

Ser Met Lys Glu Val Leu Lys Asp Ile Gln Asn Gly Thr Phe Ala Lys
275 280 285

Glu Trp Ile Val Glu Asn Gln Val Asn Arg Pro Arg Phe Asn Ala Ile
290 295 300

Asn Ala Ser Glu Asn Glu His Gln Ile Glu Val Val Gly Arg Lys Leu
305 310 315 320

Arg Glu Met Met Pro Phe Val Lys Gln Gly Lys Lys Lys Glu Ala Val
325 330 335

Val Ser Val Ala Gln Asn
340

<210> 14
<211> 1476
<212> DNA
<213> Bacillus subtilis

<400> 14

atggctaact acttcaatac actgaatctg cgccagcagc tggcacagct gggcaaatgt 60

cgctttatgg gccgcgatga attcgccgat ggcgcgagct accttcaggg taaaaaagta 120

gtcatcgtcg gctgtggcgc acagggtctg aaccagggcc tgaacatgcg tgattctggt 180

ctcgatatct cctacgctct gcgtaaagaa gcgattgccg agaagcgcgc gtcctggcgt 240

aaagcgaccg aaaatggttt taaagtgggt acttacgaag aactgatccc acaggcggat 300

ctggtgatta acctgacgcc ggacaagcag cactctgatg tagtgcgcac cgtacagcca 360

ctgatgaaag acggcgcggc gctgggctac tcgcacggtt tcaacatcgt cgaagtgggc 420

gagcagatcc gtaaagatat caccgtagtg atggttgcgc cgaaatgccc aggcaccgaa 480

gtgcgtgaag agtacaaacg tgggttcggc gtaccgacgc tgattgccgt tcacccggaa 540

aacgatccga aaggcgaagg catggcgatt gccaaagcct gggcggctgc aaccggtggt 600

caccgtgcgg gtgtgctgga atcgtccttc gttgcggaag tgaaatctga cctgatgggc 660

gagcaaacca tcctgtgcgg tatgttgcag gctggctctc tgctgtgctt cgacaagctg 720

gtggaagaag gtaccgatcc agcatacgca gaaaaactga ttcagttcgg ttgggaaacc 780

atcaccgaag cactgaaaca gggcggcatc accctgatga tggaccgtct ctctaacccg 840

gcgaaactgc gtgcttatgc gctttctgaa cagctgaaag agatcatggc acccctgttc 900

cagaaacata tggacgacat catctccggc gaattctctt ccggtatgat ggcggactgg 960

gccaacgatg ataagaaact gctgacctgg cgtgaagaga ccggcaaaac cgcgtttgaa 1020

accgcgccgc agtatgaagg caaaatcggc gagcaggagt acttcgataa aggcgtactg 1080

atgattgcga tggtgaaagc gggcgttgaa ctggcgttcg aaaccatggt cgattccggc 1140

atcattgaag agtctgcata ttatgaatca ctgcacgagc tgccgctgat tgccaacacc 1200

atcgcccgta agcgtctgta cgaaatgaac gtggttatct ctgataccgc tgagtacggt 1260

aactatctgt tctcttacgc ttgtgtgccg ttgctgaaac cgtttatggc agagctgcaa 1320

ccgggcgacc tgggtaaagc tattccggaa ggcgcggtag ataacgggca actgcgtgat 1380

gtgaacgaag cgattcgcag ccatgcgatt gagcaggtag gtaagaaact gcgcggctat 1440

atgacagata tgaaacgtat tgctgttgcg ggttaa 1476

<210> 15
<211> 343
<212> PRT
<213> Anaerostipes caccae

<400> 15

```
Met Glu Glu Cys Lys Met Ala Lys Ile Tyr Tyr Gln Glu Asp Cys Asn
1               5                   10                  15

Leu Ser Leu Leu Asp Gly Lys Thr Ile Ala Val Ile Gly Tyr Gly Ser
            20                  25                  30

Gln Gly His Ala His Ala Leu Asn Ala Lys Glu Ser Gly Cys Asn Val
        35                  40                  45

Ile Ile Gly Leu Tyr Glu Gly Ala Lys Glu Trp Lys Arg Ala Glu Glu
    50                  55                  60

Gln Gly Phe Glu Val Tyr Thr Ala Ala Glu Ala Ala Lys Lys Ala Asp
65                  70                  75                  80

Ile Ile Met Ile Leu Ile Asn Asp Glu Lys Gln Ala Thr Met Tyr Lys
                85                  90                  95

Asn Asp Ile Glu Pro Asn Leu Glu Ala Gly Asn Met Leu Met Phe Ala
            100                 105                 110

His Gly Phe Asn Ile His Phe Gly Cys Ile Val Pro Pro Lys Asp Val
        115                 120                 125

Asp Val Thr Met Ile Ala Pro Lys Gly Pro Gly His Thr Val Arg Ser
    130                 135                 140

Glu Tyr Glu Glu Gly Lys Gly Val Pro Cys Leu Val Ala Val Glu Gln
145                 150                 155                 160
```

Asp Ala Thr Gly Lys Ala Leu Asp Met Ala Leu Ala Tyr Ala Leu Ala
165 170 175

Ile Gly Gly Ala Arg Ala Gly Val Leu Glu Thr Thr Phe Arg Thr Glu
180 185 190

Thr Glu Thr Asp Leu Phe Gly Glu Gln Ala Val Leu Cys Gly Gly Val
195 200 205

Cys Ala Leu Met Gln Ala Gly Phe Glu Thr Leu Val Glu Ala Gly Tyr
210 215 220

Asp Pro Arg Asn Ala Tyr Phe Glu Cys Ile His Glu Met Lys Leu Ile
225 230 235 240

Val Asp Leu Ile Tyr Gln Ser Gly Phe Ser Gly Met Arg Tyr Ser Ile
245 250 255

Ser Asn Thr Ala Glu Tyr Gly Asp Tyr Ile Thr Gly Pro Lys Ile Ile
260 265 270

Thr Glu Asp Thr Lys Lys Ala Met Lys Lys Ile Leu Ser Asp Ile Gln
275 280 285

Asp Gly Thr Phe Ala Lys Asp Phe Leu Val Asp Met Ser Asp Ala Gly
290 295 300

Ser Gln Val His Phe Lys Ala Met Arg Lys Leu Ala Ser Glu His Pro
305 310 315 320

Ala Glu Val Val Gly Glu Glu Ile Arg Ser Leu Tyr Ser Trp Ser Asp
325 330 335

Glu Asp Lys Leu Ile Asn Asn
340

<210> 16
<211> 343
<212> PRT
<213> Anaerostipes caccae

<400> 16

Met Glu Glu Cys Lys Met Ala Lys Ile Tyr Tyr Gln Glu Asp Cys Asn
1 5 10 15

Leu Ser Leu Leu Asp Gly Lys Thr Ile Ala Val Ile Gly Tyr Gly Ser
20 25 30

```
Gln Gly His Ala His Ala Leu Asn Ala Lys Glu Ser Gly Cys Asn Val
        35                  40                  45

Ile Ile Gly Leu Tyr Glu Gly Ala Lys Asp Trp Lys Arg Ala Glu Glu
    50                  55                  60

Gln Gly Phe Glu Val Tyr Thr Ala Ala Glu Ala Ala Lys Lys Ala Asp
65                  70                  75                  80

Ile Ile Met Ile Leu Ile Asn Asp Glu Lys Gln Ala Thr Met Tyr Lys
                85                  90                  95

Asn Asp Ile Glu Pro Asn Leu Glu Ala Gly Asn Met Leu Met Phe Ala
            100                 105                 110

His Gly Phe Asn Ile His Phe Gly Cys Ile Val Pro Pro Lys Asp Val
        115                 120                 125

Asp Val Thr Met Ile Ala Pro Lys Gly Pro Gly His Thr Val Arg Ser
    130                 135                 140

Glu Tyr Glu Glu Gly Lys Gly Val Pro Cys Leu Val Ala Val Glu Gln
145                 150                 155                 160

Asp Ala Thr Gly Lys Ala Leu Asp Met Ala Leu Ala Tyr Ala Leu Ala
                165                 170                 175

Ile Gly Gly Ala Arg Ala Gly Val Leu Glu Thr Thr Phe Arg Thr Glu
            180                 185                 190

Thr Glu Thr Asp Leu Phe Gly Glu Gln Ala Val Leu Cys Gly Gly Val
        195                 200                 205

Cys Ala Leu Met Gln Ala Gly Phe Glu Thr Leu Val Glu Ala Gly Tyr
    210                 215                 220

Asp Pro Arg Asn Ala Tyr Phe Glu Cys Ile His Glu Met Lys Leu Ile
225                 230                 235                 240

Val Asp Leu Ile Tyr Gln Ser Gly Phe Ser Gly Met Arg Tyr Ser Ile
                245                 250                 255

Ser Asn Thr Ala Glu Tyr Gly Asp Tyr Ile Thr Gly Pro Lys Ile Ile
            260                 265                 270

Thr Glu Asp Thr Lys Lys Ala Met Lys Lys Ile Leu Ser Asp Ile Gln
        275                 280                 285
```

Asp Gly Thr Phe Ala Lys Asp Phe Leu Val Asp Met Ser Asp Ala Gly
290 295 300

Ser Gln Val His Phe Lys Ala Met Arg Lys Leu Ala Ser Glu His Pro
305 310 315 320

Ala Glu Val Val Gly Glu Glu Ile Arg Ser Leu Tyr Ser Trp Ser Asp
325 330 335

Glu Asp Lys Leu Ile Asn Asn
340

<210> 17
<211> 616
<212> PRT
<213> Escherichia coli

<400> 17

Met Pro Lys Tyr Arg Ser Ala Thr Thr Thr His Gly Arg Asn Met Ala
1 5 10 15

Gly Ala Arg Ala Leu Trp Arg Ala Thr Gly Met Thr Asp Ala Asp Phe
20 25 30

Gly Lys Pro Ile Ile Ala Val Val Asn Ser Phe Thr Gln Phe Val Pro
35 40 45

Gly His Val His Leu Arg Asp Leu Gly Lys Leu Val Ala Glu Gln Ile
50 55 60

Glu Ala Ala Gly Gly Val Ala Lys Glu Phe Asn Thr Ile Ala Val Asp
65 70 75 80

Asp Gly Ile Ala Met Gly His Gly Gly Met Leu Tyr Ser Leu Pro Ser
85 90 95

Arg Glu Leu Ile Ala Asp Ser Val Glu Tyr Met Val Asn Ala His Cys
100 105 110

Ala Asp Ala Met Val Cys Ile Ser Asn Cys Asp Lys Ile Thr Pro Gly
115 120 125

Met Leu Met Ala Ser Leu Arg Leu Asn Ile Pro Val Ile Phe Val Ser
130 135 140

Gly Gly Pro Met Glu Ala Gly Lys Thr Lys Leu Ser Asp Gln Ile Ile
145 150 155 160

```
Lys Leu Asp Leu Val Asp Ala Met Ile Gln Gly Ala Asp Pro Lys Val
                165                 170                 175

Ser Asp Ser Gln Ser Asp Gln Val Glu Arg Ser Ala Cys Pro Thr Cys
            180                 185                 190

Gly Ser Cys Ser Gly Met Phe Thr Ala Asn Ser Met Asn Cys Leu Thr
        195                 200                 205

Glu Ala Leu Gly Leu Ser Gln Pro Gly Asn Gly Ser Leu Leu Ala Thr
    210                 215                 220

His Ala Asp Arg Lys Gln Leu Phe Leu Asn Ala Gly Lys Arg Ile Val
225                 230                 235                 240

Glu Leu Thr Lys Arg Tyr Tyr Glu Gln Asn Asp Glu Ser Ala Leu Pro
                245                 250                 255

Arg Asn Ile Ala Ser Lys Ala Ala Phe Glu Asn Ala Met Thr Leu Asp
            260                 265                 270

Ile Ala Met Gly Gly Ser Thr Asn Thr Val Leu His Leu Leu Ala Ala
        275                 280                 285

Ala Gln Glu Ala Glu Ile Asp Phe Thr Met Ser Asp Ile Asp Lys Leu
    290                 295                 300

Ser Arg Lys Val Pro Gln Leu Cys Lys Val Ala Pro Ser Thr Gln Lys
305                 310                 315                 320

Tyr His Met Glu Asp Val His Arg Ala Gly Gly Val Ile Gly Ile Leu
                325                 330                 335

Gly Glu Leu Asp Arg Ala Gly Leu Leu Asn Arg Asp Val Lys Asn Val
            340                 345                 350

Leu Gly Leu Thr Leu Pro Gln Thr Leu Glu Gln Tyr Asp Val Met Leu
        355                 360                 365

Thr Gln Asp Asp Ala Val Lys Asn Met Phe Arg Ala Gly Pro Ala Gly
    370                 375                 380

Ile Arg Thr Thr Gln Ala Phe Ser Gln Asp Cys Arg Trp Asp Thr Leu
385                 390                 395                 400

Asp Asp Asp Arg Ala Asn Gly Cys Ile Arg Ser Leu Glu His Ala Tyr
```

```
                405                 410                 415

Ser Lys Asp Gly Gly Leu Ala Val Leu Tyr Gly Asn Phe Ala Glu Asn
            420                 425                 430

Gly Cys Ile Val Lys Thr Ala Gly Val Asp Asp Ser Ile Leu Lys Phe
        435                 440                 445

Thr Gly Pro Ala Lys Val Tyr Glu Ser Gln Asp Asp Ala Val Glu Ala
    450                 455                 460

Ile Leu Gly Gly Lys Val Val Ala Gly Asp Val Val Val Ile Arg Tyr
465                 470                 475                 480

Glu Gly Pro Lys Gly Gly Pro Gly Met Gln Glu Met Leu Tyr Pro Thr
                485                 490                 495

Ser Phe Leu Lys Ser Met Gly Leu Gly Lys Ala Cys Ala Leu Ile Thr
            500                 505                 510

Asp Gly Arg Phe Ser Gly Gly Thr Ser Gly Leu Ser Ile Gly His Val
        515                 520                 525

Ser Pro Glu Ala Ala Ser Gly Gly Ser Ile Gly Leu Ile Glu Asp Gly
    530                 535                 540

Asp Leu Ile Ala Ile Asp Ile Pro Asn Arg Gly Ile Gln Leu Gln Val
545                 550                 555                 560

Ser Asp Ala Glu Leu Ala Ala Arg Arg Glu Ala Gln Asp Ala Arg Gly
                565                 570                 575

Asp Lys Ala Trp Thr Pro Lys Asn Arg Glu Arg Gln Val Ser Phe Ala
            580                 585                 590

Leu Arg Ala Tyr Ala Ser Leu Ala Thr Ser Ala Asp Lys Gly Ala Val
        595                 600                 605

Arg Asp Lys Ser Lys Leu Gly Gly
    610                 615
```

<210> 18
<211> 1851
<212> DNA
<213> Escherichia coli

<400> 18

atgcctaagt accgttccgc caccaccact catggtcgta atatggcggg tgctcgtgcg 60

```
ctgtggcgcg ccaccggaat gaccgacgcc gatttcggta agccgattat cgcggttgtg      120
aactcgttca cccaatttgt accgggtcac gtccatctgc gcgatctcgg taaactggtc      180
gccgaacaaa ttgaagcggc tggcggcgtt gccaaagagt tcaacaccat tgcggtggat      240
gatgggattg ccatgggcca cggggggatg ctttattcac tgccatctcg cgaactgatc      300
gctgattccg ttgagtatat ggtcaacgcc cactgcgccg acgccatggt ctgcatctct      360
aactgcgaca aaatcacccc ggggatgctg atggcttccc tgcgcctgaa tattccggtg      420
atctttgttt ccggcggccc gatggaggcc gggaaaacca aactttccga tcagatcatc      480
aagctcgatc tggttgatgc gatgatccag ggcgcagacc cgaaagtatc tgactcccag      540
agcgatcagg ttgaacgttc cgcgtgtccg acctgcggtt cctgctccgg gatgtttacc      600
gctaactcaa tgaactgcct gaccgaagcg ctgggcctgt cgcagccggg caacggctcg      660
ctgctggcaa cccacgccga ccgtaagcag ctgttcctta atgctggtaa acgcattgtt      720
gaattgacca aacgttatta cgagcaaaac gacgaaagtg cactgccgcg taatatcgcc      780
agtaaggcgg cgtttgaaaa cgccatgacg ctggatatcg cgatgggtgg atcgactaac      840
accgtacttc acctgctggc ggcggcgcag gaagcggaaa tcgacttcac catgagtgat      900
atcgataagc tttcccgcaa ggttccacag ctgtgtaaag ttgcgccgag cacccagaaa      960
taccatatgg aagatgttca ccgtgctggt ggtgttatcg gtattctcgg cgaactggat     1020
cgcgcggggt tactgaaccg tgatgtgaaa aacgtacttg gcctgacgtt gccgcaaacg     1080
ctggaacaat acgacgttat gctgacccag gatgacgcgg taaaaaatat gttccgcgca     1140
ggtcctgcag gcattcgtac cacacaggca ttctcgcaag attgccgttg ggatacgctg     1200
gacgacgatc gcgccaatgg ctgtatccgc tcgctggaac acgcctacag caaagacggc     1260
ggcctggcgg tgctctacgg taactttgcg gaaaacggct gcatcgtgaa aacggcaggc     1320
gtcgatgaca gcatcctcaa attcaccggc ccggcgaaag tgtacgaaag ccaggacgat     1380
gcggtagaag cgattctcgg cggtaaagtt gtcgccggag atgtggtagt aattcgctat     1440
gaaggcccga aaggcggtcc ggggatgcag gaaatgctct acccaaccag cttcctgaaa     1500
tcaatgggtc tcggcaaagc ctgtgcgctg atcaccgacg gtcgtttctc tggtggcacc     1560
tctggtcttt ccatcggcca cgtctcaccg gaagcggcaa gcggcggcag cattggcctg     1620
attgaagatg gtgacctgat cgctatcgac atcccgaacc gtggcattca gttacaggta     1680
agcgatgccg aactggcggc gcgtcgtgaa gcgcaggacg ctcgaggtga caaagcctgg     1740
acgccgaaaa atcgtgaacg tcaggtctcc tttgccctgc gtgcttatgc cagcctggca     1800
accagcgccg acaaaggcgc ggtgcgcgat aaatcgaaac tgggggggtta a              1851
```

<210> 19

<211> 585
<212> PRT
<213> Saccharomyces cerevisiae

<400> 19

```
Met Gly Leu Leu Thr Lys Val Ala Thr Ser Arg Gln Phe Ser Thr Thr
1               5                   10                  15

Arg Cys Val Ala Lys Lys Leu Asn Lys Tyr Ser Tyr Ile Ile Thr Glu
            20                  25                  30

Pro Lys Gly Gln Gly Ala Ser Gln Ala Met Leu Tyr Ala Thr Gly Phe
        35                  40                  45

Lys Lys Glu Asp Phe Lys Lys Pro Gln Val Gly Val Gly Ser Cys Trp
    50                  55                  60

Trp Ser Gly Asn Pro Cys Asn Met His Leu Leu Asp Leu Asn Asn Arg
65                  70                  75                  80

Cys Ser Gln Ser Ile Glu Lys Ala Gly Leu Lys Ala Met Gln Phe Asn
                85                  90                  95

Thr Ile Gly Val Ser Asp Gly Ile Ser Met Gly Thr Lys Gly Met Arg
            100                 105                 110

Tyr Ser Leu Gln Ser Arg Glu Ile Ile Ala Asp Ser Phe Glu Thr Ile
        115                 120                 125

Met Met Ala Gln His Tyr Asp Ala Asn Ile Ala Ile Pro Ser Cys Asp
    130                 135                 140

Lys Asn Met Pro Gly Val Met Met Ala Met Gly Arg His Asn Arg Pro
145                 150                 155                 160

Ser Ile Met Val Tyr Gly Gly Thr Ile Leu Pro Gly His Pro Thr Cys
                165                 170                 175

Gly Ser Ser Lys Ile Ser Lys Asn Ile Asp Ile Val Ser Ala Phe Gln
            180                 185                 190

Ser Tyr Gly Glu Tyr Ile Ser Lys Gln Phe Thr Glu Glu Glu Arg Glu
        195                 200                 205

Asp Val Val Glu His Ala Cys Pro Gly Pro Gly Ser Cys Gly Gly Met
    210                 215                 220

Tyr Thr Ala Asn Thr Met Ala Ser Ala Ala Glu Val Leu Gly Leu Thr
225                 230                 235                 240
```

```
Ile Pro Asn Ser Ser Ser Phe Pro Ala Val Ser Lys Glu Lys Leu Ala
                245                 250                 255

Glu Cys Asp Asn Ile Gly Glu Tyr Ile Lys Lys Thr Met Glu Leu Gly
            260                 265                 270

Ile Leu Pro Arg Asp Ile Leu Thr Lys Glu Ala Phe Glu Asn Ala Ile
        275                 280                 285

Thr Tyr Val Val Ala Thr Gly Gly Ser Thr Asn Ala Val Leu His Leu
    290                 295                 300

Val Ala Val Ala His Ser Ala Gly Val Lys Leu Ser Pro Asp Asp Phe
305                 310                 315                 320

Gln Arg Ile Ser Asp Thr Thr Pro Leu Ile Gly Asp Phe Lys Pro Ser
                325                 330                 335

Gly Lys Tyr Val Met Ala Asp Leu Ile Asn Val Gly Gly Thr Gln Ser
            340                 345                 350

Val Ile Lys Tyr Leu Tyr Glu Asn Asn Met Leu His Gly Asn Thr Met
        355                 360                 365

Thr Val Thr Gly Asp Thr Leu Ala Glu Arg Ala Lys Lys Ala Pro Ser
    370                 375                 380

Leu Pro Glu Gly Gln Glu Ile Ile Lys Pro Leu Ser His Pro Ile Lys
385                 390                 395                 400

Ala Asn Gly His Leu Gln Ile Leu Tyr Gly Ser Leu Ala Pro Gly Gly
                405                 410                 415

Ala Val Gly Lys Ile Thr Gly Lys Glu Gly Thr Tyr Phe Lys Gly Arg
            420                 425                 430

Ala Arg Val Phe Glu Glu Glu Gly Ala Phe Ile Glu Ala Leu Glu Arg
        435                 440                 445

Gly Glu Ile Lys Lys Gly Glu Lys Thr Val Val Val Ile Arg Tyr Glu
    450                 455                 460

Gly Pro Arg Gly Ala Pro Gly Met Pro Glu Met Leu Lys Pro Ser Ser
465                 470                 475                 480

Ala Leu Met Gly Tyr Gly Leu Gly Lys Asp Val Ala Leu Leu Thr Asp
```

```
                485                 490                 495
Gly Arg Phe Ser Gly Gly Ser His Gly Phe Leu Ile Gly His Ile Val
            500                 505                 510
Pro Glu Ala Ala Glu Gly Gly Pro Ile Gly Leu Val Arg Asp Gly Asp
        515                 520                 525
Glu Ile Ile Ile Asp Ala Asp Asn Asn Lys Ile Asp Leu Leu Val Ser
    530                 535                 540
Asp Lys Glu Met Ala Gln Arg Lys Gln Ser Trp Val Ala Pro Pro Pro
545                 550                 555                 560
Arg Tyr Thr Arg Gly Thr Leu Ser Lys Tyr Ala Lys Leu Val Ser Asn
                565                 570                 575
Ala Ser Asn Gly Cys Val Leu Asp Ala
            580                 585
```

<210> 20
<211> 1131
<212> DNA
<213> Saccharomyces cerevisiae

<400> 20

```
atgaccttgg caccccctaga cgcctccaaa gttaagataa ctaccacaca acatgcatct      60
aagccaaaac cgaacagtga gttagtgttt ggcaagagct tcacggacca catgttaact      120
gcggaatgga cagctgaaaa agggtggggt accccagaga ttaaacctta tcaaaatctg      180
tctttagacc cttccgcggt ggttttccat tatgcttttg agctattcga agggatgaag      240
gcttacagaa cggtggacaa caaaattaca atgtttcgtc cagatatgaa tatgaagcgc      300
atgaataagt ctgctcagag aatctgtttg ccaacgttcg acccagaaga gttgattacc      360
ctaattggga aactgatcca gcaagataag tgcttagttc ctgaaggaaa aggttactct      420
ttatatatca ggcctacatt aatcggcact acggccggtt taggggtttc cacgcctgat      480
agagccttgc tatatgtcat ttgctgccct gtgggtcctt attacaaaac tggatttaag      540
gcggtcagac tggaagccac tgattatgcc acaagagctt ggccaggagg ctgtggtgac      600
aagaaactag gtgcaaacta cgccccctgc gtcctgccac aattgcaagc tgcttcaagg      660
ggttaccaac aaaatttatg gctatttggt ccaaataaca acattactga agtcggcacc      720
atgaatgctt ttttcgtgtt taaagatagt aaaacgggca agaaggaact agttactgct      780
ccactagacg gtaccatttt ggaaggtgtt actagggatt ccattttaaa tcttgctaaa      840
gaaagactcg aaccaagtga atggaccatt agtgaacgct acttcactat aggcgaagtt      900
actgagagat ccaagaacgg tgaactactt gaagcctttg gttctggtac tgctgcgatt      960
gtttctccca ttaaggaaat cggctggaaa ggcgaacaaa ttaatattcc gttgttgccc     1020
ggcgaacaaa ccggtccatt ggccaaagaa gttgcacaat ggattaatgg aatccaatat     1080
ggcgagactg agcatggcaa ttggtcaagg gttgttactg atttgaactg a              1131
```

<210> 21
<211> 550
<212> PRT
<213> Methanococcus maripaludis

<400> 21

```
Met Ile Ser Asp Asn Val Lys Lys Gly Val Ile Arg Thr Pro Asn Arg
1               5                   10                  15

Ala Leu Leu Lys Ala Cys Gly Tyr Thr Asp Glu Asp Met Glu Lys Pro
            20                  25                  30

Phe Ile Gly Ile Val Asn Ser Phe Thr Glu Val Val Pro Gly His Ile
        35                  40                  45

His Leu Arg Thr Leu Ser Glu Ala Ala Lys His Gly Val Tyr Ala Asn
    50                  55                  60

Gly Gly Thr Pro Phe Glu Phe Asn Thr Ile Gly Ile Cys Asp Gly Ile
65                  70                  75                  80

Ala Met Gly His Glu Gly Met Lys Tyr Ser Leu Pro Ser Arg Glu Ile
                85                  90                  95

Ile Ala Asp Ala Val Glu Ser Met Ala Arg Ala His Gly Phe Asp Gly
            100                 105                 110

Leu Val Leu Ile Pro Thr Cys Asp Lys Ile Val Pro Gly Met Ile Met
        115                 120                 125

Gly Ala Leu Arg Leu Asn Ile Pro Phe Ile Val Val Thr Gly Gly Pro
    130                 135                 140

Met Leu Pro Gly Glu Phe Gln Gly Lys Lys Tyr Glu Leu Ile Ser Leu
145                 150                 155                 160

Phe Glu Gly Val Gly Glu Tyr Gln Val Gly Lys Ile Thr Glu Glu Glu
                165                 170                 175

Leu Lys Cys Ile Glu Asp Cys Ala Cys Ser Gly Ala Gly Ser Cys Ala
            180                 185                 190
```

```
Gly Leu Tyr Thr Ala Asn Ser Met Ala Cys Leu Thr Glu Ala Leu Gly
        195                 200                 205

Leu Ser Leu Pro Met Cys Ala Thr Thr His Ala Val Asp Ala Gln Lys
    210                 215                 220

Val Arg Leu Ala Lys Lys Ser Gly Ser Lys Ile Val Asp Met Val Lys
225                 230                 235                 240

Glu Asp Leu Lys Pro Thr Asp Ile Leu Thr Lys Glu Ala Phe Glu Asn
                245                 250                 255

Ala Ile Leu Val Asp Leu Ala Leu Gly Gly Ser Thr Asn Thr Thr Leu
            260                 265                 270

His Ile Pro Ala Ile Ala Asn Glu Ile Glu Asn Lys Phe Ile Thr Leu
        275                 280                 285

Asp Asp Phe Asp Arg Leu Ser Asp Glu Val Pro His Ile Ala Ser Ile
    290                 295                 300

Lys Pro Gly Gly Glu His Tyr Met Ile Asp Leu His Asn Ala Gly Gly
305                 310                 315                 320

Ile Pro Ala Val Leu Asn Val Leu Lys Glu Lys Ile Arg Asp Thr Lys
                325                 330                 335

Thr Val Asp Gly Arg Ser Ile Leu Glu Ile Ala Glu Ser Val Lys Tyr
            340                 345                 350

Ile Asn Tyr Asp Val Ile Arg Lys Val Glu Ala Pro Val His Glu Thr
        355                 360                 365

Ala Gly Leu Arg Val Leu Lys Gly Asn Leu Ala Pro Asn Gly Cys Val
    370                 375                 380

Val Lys Ile Gly Ala Val His Pro Lys Met Tyr Lys His Asp Gly Pro
385                 390                 395                 400

Ala Lys Val Tyr Asn Ser Glu Asp Glu Ala Ile Ser Ala Ile Leu Gly
                405                 410                 415

Gly Lys Ile Val Glu Gly Asp Val Ile Val Ile Arg Tyr Glu Gly Pro
            420                 425                 430

Ser Gly Gly Pro Gly Met Arg Glu Met Leu Ser Pro Thr Ser Ala Ile
        435                 440                 445
```

Cys Gly Met Gly Leu Asp Asp Ser Val Ala Leu Ile Thr Asp Gly Arg
450 455 460

Phe Ser Gly Gly Ser Arg Gly Pro Cys Ile Gly His Val Ser Pro Glu
465 470 475 480

Ala Ala Ala Gly Gly Val Ile Ala Ala Ile Glu Asn Gly Asp Ile Ile
485 490 495

Lys Ile Asp Met Ile Glu Lys Glu Ile Asn Val Asp Leu Asp Glu Ser
500 505 510

Val Ile Lys Glu Arg Leu Ser Lys Leu Gly Glu Phe Glu Pro Lys Ile
515 520 525

Lys Lys Gly Tyr Leu Ser Arg Tyr Ser Lys Leu Val Ser Ser Ala Asp
530 535 540

Glu Gly Ala Val Leu Lys
545 550

<210> 22
<211> 1653
<212> DNA
<213> Methanococcus maripaludis

<400> 22

```
atgataagtg ataacgtcaa aaagggagtt ataagaactc caaaccgagc tcttttaaag    60
gcttgcggat atacagacga agacatggaa aaaccattta ttggaattgt aaacagcttt   120
acagaagttg ttcccggcca cattcactta agaacattat cagaagcggc taaacatggt   180
gtttatgcaa acggtggaac accatttgaa tttaatacca ttggaatttg cgacggtatt   240
gcaatgggcc acgaaggtat gaaatactct ttaccttcaa gagaaattat tgcagacgct   300
gttgaatcaa tggcaagagc acatggattt gatggtcttg ttttaattcc tacgtgtgat   360
aaaatcgttc ctggaatgat aatgggtgct ttaagactaa acattccatt tattgtagtt   420
actggaggac caatgcttcc cggagaattc caaggtaaaa aatacgaact tatcagcctt   480
tttgaaggtg tcggagaata ccaagttgga aaaattactg aagaagagtt aaagtgcatt   540
gaagactgtg catgttcagg tgctggaagt tgtgcagggc tttacactgc aaacagtatg   600
gcctgcctta cagaagcttt gggactctct cttccaatgt gtgcaacaac gcatgcagtt   660
gatgcccaaa aagttaggct tgctaaaaaa agtggctcaa aaattgttga tatggtaaaa   720
gaagacctaa aaccaacaga catattaaca aaagaagctt ttgaaaatgc tattttagtt   780
gaccttgcac ttggtggatc aacaaacaca acattacaca ttcctgcaat tgcaaatgaa   840

attgaaaata aattcataac tctcgatgac tttgacaggt taagcgatga agttccacac   900
attgcatcaa tcaaaccagg tggagaacac tacatgattg atttacacaa tgctggaggt   960
attcctgcgg tattgaacgt tttaaaagaa aaaattagag atacaaaaac agttgatgga  1020
agaagcattt tggaaatcgc agaatctgtt aaatacataa attacgacgt tataagaaaa  1080
gtggaagctc cggttcacga aactgctggt ttaagggttt taaagggaaa tcttgctcca  1140
aacggttgcg ttgtaaaaat cggtgcagta catccgaaaa tgtacaaaca cgatggacct  1200
gcaaaagttt acaattccga agatgaagca atttctgcga tacttggcgg aaaaattgta  1260
gaaggggacg ttatagtaat cagatacgaa ggaccatcag gaggccctgg aatgagagaa  1320
atgctctccc caacttcagc aatctgtgga atgggtcttg atgacagcgt tgcattgatt  1380
actgatggaa gattcagtgg tggaagtagg ggcccatgta tcggacacgt ttctccagaa  1440
gctgcagctg gcggagtaat tgctgcaatt gaaaacgggg atatcatcaa aatcgacatg  1500
attgaaaaag aaataaatgt tgatttagat gaatcagtca ttaaagaaag actctcaaaa  1560
ctgggagaat ttgagcctaa aatcaaaaaa ggctatttat caagatactc aaaacttgtc  1620
tcatctgctg acgaaggggc agttttaaaa taa                                1653
```

<210> 23
<211> 558
<212> PRT
<213> Bacillus subtilis

<400> 23

```
Met Ala Glu Leu Arg Ser Asn Met Ile Thr Gln Gly Ile Asp Arg Ala
1               5                   10                  15

Pro His Arg Ser Leu Leu Arg Ala Ala Gly Val Lys Glu Glu Asp Phe
            20                  25                  30

Gly Lys Pro Phe Ile Ala Val Cys Asn Ser Tyr Ile Asp Ile Val Pro
        35                  40                  45

Gly His Val His Leu Gln Glu Phe Gly Lys Ile Val Lys Glu Ala Ile
    50                  55                  60

Arg Glu Ala Gly Gly Val Pro Phe Glu Phe Asn Thr Ile Gly Val Asp
65                  70                  75                  80

Asp Gly Ile Ala Met Gly His Ile Gly Met Arg Tyr Ser Leu Pro Ser
                85                  90                  95

Arg Glu Ile Ile Ala Asp Ser Val Glu Thr Val Val Ser Ala His Trp
            100                 105                 110
```

```
Phe Asp Gly Met Val Cys Ile Pro Asn Cys Asp Lys Ile Thr Pro Gly
        115                 120                 125

Met Leu Met Ala Ala Met Arg Ile Asn Ile Pro Thr Ile Phe Val Ser
    130                 135                 140

Gly Gly Pro Met Ala Ala Gly Arg Thr Ser Asp Gly Arg Lys Ile Ser
145                 150                 155                 160

Leu Ser Ser Val Phe Glu Gly Val Gly Ala Tyr Gln Ala Gly Lys Ile
                165                 170                 175

Asn Glu Asn Glu Leu Gln Glu Leu Glu Gln Phe Gly Cys Pro Thr Cys
            180                 185                 190

Gly Ser Cys Ser Gly Met Phe Thr Ala Asn Ser Met Asn Cys Leu Ser
        195                 200                 205

Glu Ala Leu Gly Leu Ala Leu Pro Gly Asn Gly Thr Ile Leu Ala Thr
    210                 215                 220

Ser Pro Glu Arg Lys Glu Phe Val Arg Lys Ser Ala Ala Gln Leu Met
225                 230                 235                 240

Glu Thr Ile Arg Lys Asp Ile Lys Pro Arg Asp Ile Val Thr Val Lys
                245                 250                 255

Ala Ile Asp Asn Ala Phe Ala Leu Asp Met Ala Leu Gly Gly Ser Thr
            260                 265                 270

Asn Thr Val Leu His Thr Leu Ala Leu Ala Asn Glu Ala Gly Val Glu
        275                 280                 285

Tyr Ser Leu Glu Arg Ile Asn Glu Val Ala Glu Arg Val Pro His Leu
    290                 295                 300

Ala Lys Leu Ala Pro Ala Ser Asp Val Phe Ile Glu Asp Leu His Glu
305                 310                 315                 320

Ala Gly Gly Val Ser Ala Ala Leu Asn Glu Leu Ser Lys Lys Glu Gly
                325                 330                 335

Ala Leu His Leu Asp Ala Leu Thr Val Thr Gly Lys Thr Leu Gly Glu
            340                 345                 350

Thr Ile Ala Gly His Glu Val Lys Asp Tyr Asp Val Ile His Pro Leu
```

355 360 365

Asp Gln Pro Phe Thr Glu Lys Gly Gly Leu Ala Val Leu Phe Gly Asn
370 375 380

Leu Ala Pro Asp Gly Ala Ile Ile Lys Thr Gly Gly Val Gln Asn Gly
385 390 395 400

Ile Thr Arg His Glu Gly Pro Ala Val Val Phe Asp Ser Gln Asp Glu
405 410 415

Ala Leu Asp Gly Ile Ile Asn Arg Lys Val Lys Glu Gly Asp Val Val
420 425 430

Ile Ile Arg Tyr Glu Gly Pro Lys Gly Gly Pro Gly Met Pro Glu Met
435 440 445

Leu Ala Pro Thr Ser Gln Ile Val Gly Met Gly Leu Gly Pro Lys Val
450 455 460

Ala Leu Ile Thr Asp Gly Arg Phe Ser Gly Ala Ser Arg Gly Leu Ser
465 470 475 480

Ile Gly His Val Ser Pro Glu Ala Ala Glu Gly Gly Pro Leu Ala Phe
485 490 495

Val Glu Asn Gly Asp His Ile Ile Val Asp Ile Glu Lys Arg Ile Leu
500 505 510

Asp Val Gln Val Pro Glu Glu Glu Trp Glu Lys Arg Lys Ala Asn Trp
515 520 525

Lys Gly Phe Glu Pro Lys Val Lys Thr Gly Tyr Leu Ala Arg Tyr Ser
530 535 540

Lys Leu Val Thr Ser Ala Asn Thr Gly Gly Ile Met Lys Ile
545 550 555

<210> 24
<211> 1677
<212> DNA
<213> Bacillus subtilis

<400> 24

atggcagaat tacgcagtaa tatgatcaca caaggaatcg atagagctcc gcaccgcagt 60
ttgcttcgtg cagcaggggt aaaagaagag gatttcggca agccgtttat tgcggtgtgt 120
aattcataca ttgatatcgt tcccggtcat gttcacttgc aggagtttgg gaaaatcgta 180
aaagaagcaa tcagagaagc aggggggcgtt ccgtttgaat ttaataccat tggggtagat 240
gatggcatcg caatggggca tatcggtatg agatattcgc tgccaagccg tgaaattatc 300
gcagactctg tggaaacggt tgtatccgca cactggtttg acggaatggt ctgtattccg 360
aactgcgaca aaatcacacc gggaatgctt atggcggcaa tgcgcatcaa cattccgacg 420
atttttgtca gcggcggacc gatggcggca ggaagaacaa gttacgggcg aaaaatctcc 480
ctttcctcag tattcgaagg ggtaggcgcc taccaagcag ggaaaatcaa cgaaaacgag 540
cttcaagaac tagagcagtt cggatgccca acgtgcgggt cttgctcagg catgtttacg 600
gcgaactcaa tgaactgtct gtcagaagca cttggtcttg ctttgccggg taatggaacc 660
attctggcaa catctccgga acgcaaagag tttgtgagaa aatcggctgc gcaattaatg 720
gaaacgattc gcaaagatat caaaccgcgt gatattgtta cagtaaaagc gattgataac 780
gcgtttgcac tcgatatggc gctcggaggt tctacaaata ccgttcttca taccttgcc 840
cttgcaaacg aagccggcgt tgaatactct ttagaacgca ttaacgaagt cgctgagcgc 900
gtgccgcact tggctaagct ggcgcctgca tcggatgtgt ttattgaaga tcttcacgaa 960
gcgggcggcg tttcagcggc tctgaatgag ctttcgaaga aagaaggagc gcttcattta 1020
gatgcgctga ctgttacagg aaaaactctt ggagaaacca ttgccggaca tgaagtaaag 1080
gattatgacg tcattcaccc gctggatcaa ccattcactg aaaagggagg ccttgctgtt 1140
ttattcggta atctagctcc ggacggcgct atcattaaaa caggcggcgt acagaatggg 1200
attacaagac acgaagggcc ggctgtcgta ttcgattctc aggacgaggc gcttgacggc 1260
attatcaacc gaaaagtaaa agaaggcgac gttgtcatca tcagatacga agggccaaaa 1320
ggcggacctg gcatgccgga aatgctggcg ccaacatccc aaatcgttgg aatgggactc 1380
gggccaaaag tggcattgat tacggacgga cgtttttccg gagcctcccg tggcctctca 1440
atcggccacg tatcacctga ggccgctgag ggcgggccgc ttgcctttgt tgaaaacgga 1500
gaccatatta tcgttgatat tgaaaaacgc atcttggatg tacaagtgcc agaagaagag 1560
tgggaaaaac gaaaagcgaa ctggaaaggt tttgaaccga aagtgaaaac cggctacctg 1620
gcacgttatt ctaaacttgt gacaagtgcc aacaccggcg gtattatgaa aatctag 1677

<210> 25
<211> 547
<212> PRT
<213> Lactococcus lactis

<400> 25

```
Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
```

```
            20                  25                  30

Asp Gln Ile Ile Ser Arg Glu Asp Met Lys Trp Ile Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Ile
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Asp Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Tyr
    130                 135                 140

Glu Ile Asp Arg Val Leu Ser Gln Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ala Leu Ser Leu Glu Lys Glu Ser Ser Thr Thr Asn Thr Thr Glu Gln
            180                 185                 190

Val Ile Leu Ser Lys Ile Glu Glu Ser Leu Lys Asn Ala Gln Lys Pro
        195                 200                 205

Val Val Ile Ala Gly His Glu Val Ile Ser Phe Gly Leu Glu Lys Thr
    210                 215                 220

Val Thr Gln Phe Val Ser Glu Thr Lys Leu Pro Ile Thr Thr Leu Asn
225                 230                 235                 240

Phe Gly Lys Ser Ala Val Asp Glu Ser Leu Pro Ser Phe Leu Gly Ile
                245                 250                 255

Tyr Asn Gly Lys Leu Ser Glu Ile Ser Leu Lys Asn Phe Val Glu Ser
            260                 265                 270
```

```
Ala Asp Phe Ile Leu Met Leu Gly Val Lys Leu Thr Asp Ser Ser Thr
        275                 280                 285

Gly Ala Phe Thr His His Leu Asp Glu Asn Lys Met Ile Ser Leu Asn
    290                 295                 300

Ile Asp Glu Gly Ile Ile Phe Asn Lys Val Val Glu Asp Phe Asp Phe
305                 310                 315                 320

Arg Ala Val Val Ser Ser Leu Ser Glu Leu Lys Gly Ile Glu Tyr Glu
                325                 330                 335

Gly Gln Tyr Ile Asp Lys Gln Tyr Glu Glu Phe Ile Pro Ser Ser Ala
            340                 345                 350

Pro Leu Ser Gln Asp Arg Leu Trp Gln Ala Val Glu Ser Leu Thr Gln
        355                 360                 365

Ser Asn Glu Thr Ile Val Ala Glu Gln Gly Thr Ser Phe Phe Gly Ala
    370                 375                 380

Ser Thr Ile Phe Leu Lys Ser Asn Ser Arg Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ser Ile Arg Glu Lys Leu Asn
        435                 440                 445

Pro Ile Cys Phe Ile Ile Asn Asn Asp Gly Tyr Thr Val Glu Arg Glu
    450                 455                 460

Ile His Gly Pro Thr Gln Ser Tyr Asn Asp Ile Pro Met Trp Asn Tyr
465                 470                 475                 480

Ser Lys Leu Pro Glu Thr Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Val Asn Arg Met Tyr Trp Ile Glu Leu Val Leu Glu Lys
        515                 520                 525
```

```
Glu Asp Ala Pro Lys Leu Leu Lys Lys Met Gly Lys Leu Phe Ala Glu
    530                 535                 540

Gln Asn Lys
545
```

<210> 26
<211> 1828
<212> DNA
<213> Lactococcus lactis

<400> 26

```
tttaaataag tcaatatcgt tgacttattt agaagaaaga gttattcttt aaatgtcaag      60
ttagttgact aaattaaata taaaatatgg aggaatgtga tgtatacagt aggagattac     120
ctgttagacc gattacacga gttgggaatt gaagaaattt ttggagttcc tggtgactat     180
aacttacaat ttttagatca aattatttca cgcgaagata tgaaatggat tggaaatgct     240
aatgaattaa atgcttctta tatggctgat ggttatgctc gtactaaaaa agctgccgca     300
tttctcacca catttggagt cggcgaattg agtgcgatca atggactggc aggaagttat     360
gccgaaaatt taccagtagt agaaattgtt ggttcaccaa cttcaaaagt acaaaatgac     420
ggaaaatttg tccatcatac actagcagat ggtgatttta aacactttat gaagatgcat     480
gaacctgtta cagcagcgcg gactttactg acagcagaaa atgccacata tgaaattgac     540
cgagtacttt ctcaattact aaaagaaaga aaaccagtct atattaactt accagtcgat     600
gttgctgcag caaaagcaga gaagcctgca ttatctttag aaaaagaaag ctctacaaca     660
aatacaactg aacaagtgat tttgagtaag attgaagaaa gtttgaaaaa tgcccaaaaa     720
ccagtagtga ttgcaggaca cgaagtaatt agttttggtt tagaaaaaac ggtaactcag     780
tttgtttcag aaacaaaact accgattacg acactaaatt ttggtaaaag tgctgttgat     840
gaatctttgc cctcattttt aggaatatat aacgggaaac tttcagaaat cagtcttaaa     900
aattttgtgg agtccgcaga ctttatccta atgcttggag tgaagcttac ggactcctca     960
acaggtgcat tcacacatca tttagatgaa aataaaatga tttcactaaa catagatgaa    1020
ggaataattt tcaataaagt ggtagaagat tttgatttta gagcagtggt ttcttcttta    1080
tcagaattaa aaggaataga atatgaagga caatatattg ataagcaata tgaagaattt    1140
attccatcaa gtgctccctt atcacaagac cgtctatggc aggcagttga aagtttgact    1200
caaagcaatg aaacaatcgt tgctgaacaa ggaacctcat tttttggagc ttcaacaatt    1260
ttcttaaaat caaatagtcg ttttattgga caacctttat ggggttctat tggatatact    1320
tttccagcgg ctttaggaag ccaaattgcg gataaagaga gcagacacct tttatttatt    1380
ggtgatggtt cacttcaact taccgtacaa gaattaggac tatcaatcag agaaaaactc    1440
```

```
aatccaattt gttttatcat aaataatgat ggttatacag ttgaaagaga aatccacgga     1500
cctactcaaa gttataacga cattccaatg tggaattact cgaaattacc agaaacattt     1560
ggagcaacag aagatcgtgt agtatcaaaa attgttagaa cagagaatga atttgtgtct     1620
gtcatgaaag aagcccaagc agatgtcaat agaatgtatt ggatagaact agttttggaa     1680
aaagaagatg cgccaaaatt actgaaaaaa atgggtaaat tatttgctga gcaaaataaa     1740
tagatatcaa cggatgatga aaagtaaaat agacaaagtc caataatttt ataaaaagta     1800
aaaacattag gattttccta atgttttt                                        1828
```

<210> 27
<211> 548
<212> PRT
<213> Lactococcus lactis

<400> 27

```
Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser His Lys Asp Met Lys Trp Val Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Val
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Glu Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Val
    130                 135                 140

Glu Ile Asp Arg Val Leu Ser Ala Leu Leu Lys Glu Arg Lys Pro Val
145                 150                 155                 160

Tyr Ile Asn Leu Pro Val Asp Val Ala Ala Ala Lys Ala Glu Lys Pro
```

```
                165                 170                 175

Ser Leu Pro Leu Lys Lys Glu Asn Ser Thr Ser Asn Thr Ser Asp Gln
            180                 185                 190

Glu Ile Leu Asn Lys Ile Gln Glu Ser Leu Lys Asn Ala Lys Lys Pro
        195                 200                 205

Ile Val Ile Thr Gly His Glu Ile Ile Ser Phe Gly Leu Glu Lys Thr
    210                 215                 220

Val Thr Gln Phe Ile Ser Lys Thr Lys Leu Pro Ile Thr Thr Leu Asn
225                 230                 235                 240

Phe Gly Lys Ser Ser Val Asp Glu Ala Leu Pro Ser Phe Leu Gly Ile
                245                 250                 255

Tyr Asn Gly Thr Leu Ser Glu Pro Asn Leu Lys Glu Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asn Glu Asn Lys Met Ile Ser Leu Asn
    290                 295                 300

Ile Asp Glu Gly Lys Ile Phe Asn Glu Arg Ile Gln Asn Phe Asp Phe
305                 310                 315                 320

Glu Ser Leu Ile Ser Ser Leu Leu Asp Leu Ser Glu Ile Glu Tyr Lys
                325                 330                 335

Gly Lys Tyr Ile Asp Lys Lys Gln Glu Asp Phe Val Pro Ser Asn Ala
            340                 345                 350

Leu Leu Ser Gln Asp Arg Leu Trp Gln Ala Val Glu Asn Leu Thr Gln
        355                 360                 365

Ser Asn Glu Thr Ile Val Ala Glu Gln Gly Thr Ser Phe Phe Gly Ala
    370                 375                 380

Ser Ser Ile Phe Leu Lys Ser Lys Ser His Phe Ile Gly Gln Pro Leu
385                 390                 395                 400

Trp Gly Ser Ile Gly Tyr Thr Phe Pro Ala Ala Leu Gly Ser Gln Ile
                405                 410                 415
```

```
Ala Asp Lys Glu Ser Arg His Leu Leu Phe Ile Gly Asp Gly Ser Leu
            420                 425                 430

Gln Leu Thr Val Gln Glu Leu Gly Leu Ala Ile Arg Glu Lys Ile Asn
        435                 440                 445

Pro Ile Cys Phe Ile Ile Asn Asn Asp Gly Tyr Thr Val Glu Arg Glu
    450                 455                 460

Ile His Gly Pro Asn Gln Ser Tyr Asn Asp Ile Pro Met Trp Asn Tyr
465                 470                 475                 480

Ser Lys Leu Pro Glu Ser Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Pro Asn Arg Met Tyr Trp Ile Glu Leu Ile Leu Ala Lys
        515                 520                 525

Glu Gly Ala Pro Lys Val Leu Lys Lys Met Gly Lys Leu Phe Ala Glu
    530                 535                 540

Gln Asn Lys Ser
545
```

<210> 28
<211> 1954
<212> DNA
<213> Lactococcus lactis

<400> 28

```
ctagagtttt ctttagtcat aattcactcc ttttattagt ctattatact tgataattca      60
aataagtcaa tatcgttgac ttatttaaag aaaagcgtta ttctataaat gtcaagttga     120
ttgaccaata tataataaaa tatggaggaa tgcgatgtat acagtaggag attacctatt     180
agaccgatta cacgagttag gaattgaaga aatttttgga gtccctggag actataactt     240
acaattttta gatcaaatta tttcccacaa ggatatgaaa tgggtcggaa atgctaatga     300
attaaatgct tcatatatgg ctgatggcta tgctcgtact aaaaaagctg ccgcatttct     360
tacaaccttt ggagtaggtg aattgagtgc agttaatgga ttagcaggaa gttacgccga     420
aaatttacca gtagtagaaa tagtgggatc acctacatca aaagttcaaa atgaaggaaa     480
atttgttcat catacgctgg ctgacggtga ttttaaacac tttatgaaaa tgcacgaacc     540
tgttacagca gctcgaactt tactgacagc agaaaatgca accgttgaaa ttgaccgagt     600
actttctgca ctattaaaag aaagaaaacc tgtctatatc aacttaccag ttgatgttgc     660
```

tgctgcaaaa gcagagaaac cctcactccc tttgaaaaag gaaaactcaa cttcaaatac 720

aagtgaccaa gaaattttga acaaaattca agaaagcttg aaaaatgcca aaaaaccaat 780

cgtgattaca ggacatgaaa taattagttt tggcttagaa aaaacagtca ctcaatttat 840

ttcaaagaca aaactaccta ttacgacatt aaactttggt aaaagttcag ttgatgaagc 900

cctcccttca tttttaggaa tctataatgg tacactctca gagcctaatc ttaaagaatt 960

cgtggaatca gccgacttca tcttgatgct tggagttaaa ctcacagact cttcaacagg 1020

agccttcact catcatttaa atgaaaataa aatgatttca ctgaatatag atgaaggaaa 1080

aatatttaac gaaagaatcc aaaattttga ttttgaatcc ctcatctcct ctctcttaga 1140

cctaagcgaa atagaataca aaggaaaata tatcgataaa aagcaagaag actttgttcc 1200

atcaaatgcg cttttatcac aagaccgcct atggcaagca gttgaaaacc taactcaaag 1260

caatgaaaca atcgttgctg aacaagggac atcattcttt ggcgcttcat caattttctt 1320

aaaatcaaag agtcatttta ttggtcaacc cttatgggga tcaattggat atacattccc 1380

agcagcatta ggaagccaaa ttgcagataa agaaagcaga caccttttat ttattggtga 1440

tggttcactt caacttacag tgcaagaatt aggattagca atcagagaaa aaattaatcc 1500

aatttgcttt attatcaata atgatggtta tacagtcgaa agagaaattc atggaccaaa 1560

tcaaagctac aatgatattc caatgtggaa ttactcaaaa ttaccagaat cgtttggagc 1620

aacagaagat cgagtagtct caaaaatcgt tagaactgaa aatgaatttg tgtctgtcat 1680

gaaagaagct caagcagatc caaatagaat gtactggatt gagttaattt tggcaaaaga 1740

aggtgcacca aaagtactga aaaaaatggg caaactattt gctgaacaaa ataaatcata 1800

atttataaat agtaaaaaac attaggaaat acctaatgtt tttttgttga ctaaatcaat 1860

ccctctttat atagaaaacc ttagtttctc aaagacaact taattaagcc tgccaaattg 1920

gaactcgcaa aatgtaatct atcctctgct ccta 1954

<210> 29
<211> 550
<212> PRT
<213> Salmonella typhimurium

<400> 29

```
Met Gln Asn Pro Tyr Thr Val Ala Asp Tyr Leu Leu Asp Arg Leu Ala
1               5                   10                  15

Gly Cys Gly Ile Gly His Leu Phe Gly Val Pro Gly Asp Tyr Asn Leu
            20                  25                  30

Gln Phe Leu Asp His Val Ile Asp His Pro Thr Leu Arg Trp Val Gly
        35                  40                  45
```

```
Cys Ala Asn Glu Leu Asn Ala Ala Tyr Ala Ala Asp Gly Tyr Ala Arg
    50                  55                  60

Met Ser Gly Ala Gly Ala Leu Leu Thr Thr Phe Gly Val Gly Glu Leu
65                  70                  75                  80

Ser Ala Ile Asn Gly Ile Ala Gly Ser Tyr Ala Glu Tyr Val Pro Val
                85                  90                  95

Leu His Ile Val Gly Ala Pro Cys Ser Ala Ala Gln Gln Arg Gly Glu
            100                 105                 110

Leu Met His His Thr Leu Gly Asp Gly Asp Phe Arg His Phe Tyr Arg
        115                 120                 125

Met Ser Gln Ala Ile Ser Ala Ala Ser Ala Ile Leu Asp Glu Gln Asn
    130                 135                 140

Ala Cys Phe Glu Ile Asp Arg Val Leu Gly Glu Met Leu Ala Ala Arg
145                 150                 155                 160

Arg Pro Gly Tyr Ile Met Leu Pro Ala Asp Val Ala Lys Lys Thr Ala
                165                 170                 175

Ile Pro Pro Thr Gln Ala Leu Ala Leu Pro Val His Glu Ala Gln Ser
            180                 185                 190

Gly Val Glu Thr Ala Phe Arg Tyr His Ala Arg Gln Cys Leu Met Asn
        195                 200                 205

Ser Arg Arg Ile Ala Leu Leu Ala Asp Phe Leu Ala Gly Arg Phe Gly
    210                 215                 220

Leu Arg Pro Leu Leu Gln Arg Trp Met Ala Glu Thr Pro Ile Ala His
225                 230                 235                 240

Ala Thr Leu Leu Met Gly Lys Gly Leu Phe Asp Glu Gln His Pro Asn
                245                 250                 255

Phe Val Gly Thr Tyr Ser Ala Gly Ala Ser Ser Lys Glu Val Arg Gln
            260                 265                 270

Ala Ile Glu Asp Ala Asp Arg Val Ile Cys Val Gly Thr Arg Phe Val
        275                 280                 285

Asp Thr Leu Thr Ala Gly Phe Thr Gln Gln Leu Pro Ala Glu Arg Thr
```

290 295 300

Leu Glu Ile Gln Pro Tyr Ala Ser Arg Ile Gly Glu Thr Trp Phe Asn
305 310 315 320

Leu Pro Met Ala Gln Ala Val Ser Thr Leu Arg Glu Leu Cys Leu Glu
325 330 335

Cys Ala Phe Ala Pro Pro Pro Thr Arg Ser Ala Gly Gln Pro Val Arg
340 345 350

Ile Asp Lys Gly Glu Leu Thr Gln Glu Ser Phe Trp Gln Thr Leu Gln
355 360 365

Gln Tyr Leu Lys Pro Gly Asp Ile Ile Leu Val Asp Gln Gly Thr Ala
370 375 380

Ala Phe Gly Ala Ala Ala Leu Ser Leu Pro Asp Gly Ala Glu Val Val
385 390 395 400

Leu Gln Pro Leu Trp Gly Ser Ile Gly Tyr Ser Leu Pro Ala Ala Phe
405 410 415

Gly Ala Gln Thr Ala Cys Pro Asp Arg Arg Val Ile Leu Ile Ile Gly
420 425 430

Asp Gly Ala Ala Gln Leu Thr Ile Gln Glu Met Gly Ser Met Leu Arg
435 440 445

Asp Gly Gln Ala Pro Val Ile Leu Leu Leu Asn Asn Asp Gly Tyr Thr
450 455 460

Val Glu Arg Ala Ile His Gly Ala Ala Gln Arg Tyr Asn Asp Ile Ala
465 470 475 480

Ser Trp Asn Trp Thr Gln Ile Pro Pro Ala Leu Asn Ala Ala Gln Gln
485 490 495

Ala Glu Cys Trp Arg Val Thr Gln Ala Ile Gln Leu Ala Glu Val Leu
500 505 510

Glu Arg Leu Ala Arg Pro Gln Arg Leu Ser Phe Ile Glu Val Met Leu
515 520 525

Pro Lys Ala Asp Leu Pro Glu Leu Leu Arg Thr Val Thr Arg Ala Leu
530 535 540

Glu Ala Arg Asn Gly Gly
545 550

<210> 30
<211> 1653
<212> DNA
<213> Salmonella typhimurium

<400> 30

ttatccccg ttgcgggctt ccagcgcccg ggtcacggta cgcagtaatt ccggcagatc 60
ggcttttggc aacatcactt caataaatga cagacgttgt gggcgcgcca accgttcgag 120
gacctctgcc agttggatag cctgcgtcac ccgccagcac tccgcctgtt gcgccgcgtt 180
tagcgccggt ggtatctgcg tccagttcca gctcgcgatg tcgttatacc gctgggccgc 240
gccgtgaatg gcgcgctcta cggtatagcc gtcattgttg agcagcagga tgaccggcgc 300
ctgcccgtcg cgtaacatcg agcccatctc ctgaatcgtg agctgcgccg cgccatcgcc 360
gataatcaga atcacccgcc gatcgggaca ggcggtttgc gcgccaaacg cggcgggcaa 420
ggaatagccg atagaccccc acagcggctg taacacaact tccgcgccgt caggaagcga 480
cagcgcggca gcgccaaaag ctgctgtccc ctggtcgaca aggataatat ctccgggttt 540
gagatactgc tgtaaggttt gccagaagct ttcctgggtc agttctcctt tatcaatccg 600
cactggctgt ccggcggaac gcgtcggcgg cggcgcaaaa gcgcattcca ggcacagttc 660
gcgcagcgta gacaccgcct gcgccatcgg gaggttgaac caggtttcgc cgatgcgcga 720
cgcgtaaggc tgaatctcca gcgtgcgttc cgccggtaat tgttgggtaa atccggccgt 780
aagggtatcg acaaaacggg tgccgacgca gataacccta tcggcgtcct ctatggcctg 840
acgcacttct ttgctgctgg cgccagcgct ataggtgcca acgaagttcg ggtgctgttc 900
atcaaaaagc cccttcccca tcagtagtgt cgcatgagcg atgggcgttt ccgccatcca 960
gcgctgcaac agtggtcgta aaccaaaacg cccggcaaga aagtcggcca atagcgcaat 1020
gcgccgactg ttcatcaggc actgacgggc gtgataacga aaggccgtct ccacgccgct 1080
ttgcgcttca tgcacgggca acgccagcgc ctgcgtaggt gggatggccg ttttttttcgc 1140
cacatcggcg ggcaacatga tgtatcctgg cctgcgtgcg gcaagcattt cacccaacac 1200
gcggtcaatc tcgaaacagg cgttctgttc atctaatatt gcgctggcag cggatatcgc 1260
ctgactcatg cgataaaaat gacgaaaatc gccgtcaccg agggtatggt gcatcaattc 1320
gccacgctgc tgcgcagcgc tacagggcgc gccgacgata tgcaagaccg ggacatattc 1380
cgcgtaactg cccgcgatac cgttaatagc gctaagttct cccacgccaa aggtggtgag 1440
tagcgctcca gcgcccgaca tgcgcgcata gccgtccgcg gcataagcgg cgttcagctc 1500
attggcgcat cccacccaac gcagggtcgg gtggtcaatc acatggtcaa gaaactgcaa 1560
gttataatcg cccggtacgc caaaaagatg gccaatgccg catcctgcca gtctgtccag 1620

caaatagtcg gccacggtat aggggttttg cat 1653

<210> 31
<211> 554
<212> PRT
<213> Clostridium acetobutylicum

<400> 31

```
Met Lys Ser Glu Tyr Thr Ile Gly Arg Tyr Leu Leu Asp Arg Leu Ser
1               5                   10                  15

Glu Leu Gly Ile Arg His Ile Phe Gly Val Pro Gly Asp Tyr Asn Leu
            20                  25                  30

Ser Phe Leu Asp Tyr Ile Met Glu Tyr Lys Gly Ile Asp Trp Val Gly
        35                  40                  45

Asn Cys Asn Glu Leu Asn Ala Gly Tyr Ala Ala Asp Gly Tyr Ala Arg
    50                  55                  60

Ile Asn Gly Ile Gly Ala Ile Leu Thr Thr Phe Gly Val Gly Glu Leu
65                  70                  75                  80

Ser Ala Ile Asn Ala Ile Ala Gly Ala Tyr Ala Glu Gln Val Pro Val
                85                  90                  95

Val Lys Ile Thr Gly Ile Pro Thr Ala Lys Val Arg Asp Asn Gly Leu
            100                 105                 110

Tyr Val His His Thr Leu Gly Asp Gly Arg Phe Asp His Phe Phe Glu
        115                 120                 125

Met Phe Arg Glu Val Thr Val Ala Glu Ala Leu Leu Ser Glu Glu Asn
    130                 135                 140

Ala Ala Gln Glu Ile Asp Arg Val Leu Ile Ser Cys Trp Arg Gln Lys
145                 150                 155                 160

Arg Pro Val Leu Ile Asn Leu Pro Ile Asp Val Tyr Asp Lys Pro Ile
                165                 170                 175

Asn Lys Pro Leu Lys Pro Leu Leu Asp Tyr Thr Ile Ser Ser Asn Lys
            180                 185                 190

Glu Ala Ala Cys Glu Phe Val Thr Glu Ile Val Pro Ile Ile Asn Arg
        195                 200                 205
```

```
Ala Lys Lys Pro Val Ile Leu Ala Asp Tyr Gly Val Tyr Arg Tyr Gln
    210                 215                 220

Val Gln His Val Leu Lys Asn Leu Ala Glu Lys Thr Gly Phe Pro Val
225                 230                 235                 240

Ala Thr Leu Ser Met Gly Lys Gly Val Phe Asn Glu Ala His Pro Gln
                245                 250                 255

Phe Ile Gly Val Tyr Asn Gly Asp Val Ser Ser Pro Tyr Leu Arg Gln
            260                 265                 270

Arg Val Asp Glu Ala Asp Cys Ile Ile Ser Val Gly Val Lys Leu Thr
        275                 280                 285

Asp Ser Thr Thr Gly Gly Phe Ser His Gly Phe Ser Lys Arg Asn Val
    290                 295                 300

Ile His Ile Asp Pro Phe Ser Ile Lys Ala Lys Gly Lys Lys Tyr Ala
305                 310                 315                 320

Pro Ile Thr Met Lys Asp Ala Leu Thr Glu Leu Thr Ser Lys Ile Glu
                325                 330                 335

His Arg Asn Phe Glu Asp Leu Asp Ile Lys Pro Tyr Lys Ser Asp Asn
            340                 345                 350

Gln Lys Tyr Phe Ala Lys Glu Lys Pro Ile Thr Gln Lys Arg Phe Phe
        355                 360                 365

Glu Arg Ile Ala His Phe Ile Lys Glu Lys Asp Val Leu Leu Ala Glu
    370                 375                 380

Gln Gly Thr Cys Phe Phe Gly Ala Ser Thr Ile Gln Leu Pro Lys Asp
385                 390                 395                 400

Ala Thr Phe Ile Gly Gln Pro Leu Trp Gly Ser Ile Gly Tyr Thr Leu
                405                 410                 415

Pro Ala Leu Leu Gly Ser Gln Leu Ala Asp Gln Lys Arg Arg Asn Ile
            420                 425                 430

Leu Leu Ile Gly Asp Gly Ala Phe Gln Met Thr Ala Gln Glu Ile Ser
        435                 440                 445

Thr Met Leu Arg Leu Gln Ile Lys Pro Ile Ile Phe Leu Ile Asn Asn
    450                 455                 460
```

```
Asp Gly Tyr Thr Ile Glu Arg Ala Ile His Gly Arg Glu Gln Val Tyr
465                 470                 475                 480

Asn Asn Ile Gln Met Trp Arg Tyr His Asn Val Pro Lys Val Leu Gly
                485                 490                 495

Pro Lys Glu Cys Ser Leu Thr Phe Lys Val Gln Ser Glu Thr Glu Leu
            500                 505                 510

Glu Lys Ala Leu Leu Val Ala Asp Lys Asp Cys Glu His Leu Ile Phe
        515                 520                 525

Ile Glu Val Val Met Asp Arg Tyr Asp Lys Pro Glu Pro Leu Glu Arg
    530                 535                 540

Leu Ser Lys Arg Phe Ala Asn Gln Asn Asn
545                 550
```

<210> 32
<211> 1665
<212> DNA
<213> Clostridium acetobutylicum

<400> 32

```
ttgaagagtg aatacacaat tggaagatat ttgttagacc gtttatcaga gttgggtatt      60
cggcatatct ttggtgtacc tggagattac aatctatcct ttttagacta tataatggag     120
tacaaaggga tagattgggt tggaaattgc aatgaattga atgctgggta tgctgctgat     180
ggatatgcaa gaataaatgg aattggagcc atacttacaa catttggtgt tggagaatta     240
agtgccatta acgcaattgc tggggcatac gctgagcaag ttccagttgt taaaattaca     300
ggtatcccca cagcaaaagt tagggacaat ggattatatg tacaccacac attaggtgac     360
ggaaggtttg atcacttttt tgaaatgttt agagaagtaa cagttgctga ggcattacta     420
agcgaagaaa atgcagcaca agaaattgat cgtgttctta tttcatgctg gagacaaaaa     480
cgtcctgttc ttataaattt accgattgat gtatatgata aaccaattaa caaaccatta     540
aagccattac tcgattatac tatttcaagt aacaaagagg ctgcatgtga atttgttaca     600
gaaatagtac ctataataaa tagggcaaaa aagcctgtta ttcttgcaga ttatggagta     660
tatcgttacc aagttcaaca tgtgcttaaa aacttggccg aaaaaaccgg atttcctgtg     720
gctacactaa gtatgggaaa aggtgttttc aatgaagcac accctcaatt tattggtgtt     780
tataatggtg atgtaagttc tccttattta aggcagcgag ttgatgaagc agactgcatt     840
attagcgttg gtgtaaaatt gacggattca accacagggg gattttctca tggattttct     900
aaaaggaatg taattcacat tgatcctttt tcaataaagg caaaaggtaa aaaatatgca     960

cctattacga tgaaagatgc tttaacagaa ttaacaagta aaattgagca tagaaacttt    1020
gaggatttag atataaagcc ttacaaatca gataatcaaa agtattttgc aaaagagaag    1080
ccaattacac aaaaacgttt ttttgagcgt attgctcact ttataaaaga aaaagatgta    1140
ttattagcag aacagggtac atgctttttt ggtgcgtcaa ccatacaact acccaaagat    1200
gcaactttta ttggtcaacc tttatgggga tctattggat acacacttcc tgctttatta    1260
ggttcacaat tagctgatca aaaaaggcgt aatattcttt taattgggga tggtgcattt    1320
caaatgacag cacaagaaat ttcaacaatg cttcgtttac aaatcaaacc tattattttt    1380
ttaattaata acgatggtta tacaattgaa cgtgctattc atggtagaga acaagtatat    1440
aacaatattc aaatgtggcg atatcataat gttccaaagg ttttaggtcc taaagaatgc    1500
agcttaacct ttaaagtaca aagtgaaact gaacttgaaa aggctctttt agtggcagat    1560
aaggattgtg aacatttgat ttttatagaa gttgttatgg atcgttatga taaacccgag    1620
cctttagaac gtctttcgaa acgttttgca aatcaaaata attag                    1665
```

<210> 33
<211> 1641
<212> DNA
<213> Clostridium acetobutylicum

<400> 33

atgaaacaac gtatcgggca atacttgatc gatgccctac acgttaatgg tgtcgataag 60
atctttggag tcccaggtga tttcacttta gcctttttgg acgatatcat aagacatgac 120
aacgtggaat gggtgggaaa tactaatgag ttgaacgccg cttacgccgc tgatggttac 180
gctagagtta atggattagc cgctgtatct accacttttg gggttggcga gttatctgct 240
gtgaatggta ttgctggaag ttacgcagag cgtgttcctg taatcaaaat ctcaggcggt 300
ccttcatcag ttgctcaaca agagggtaga tatgtccacc attcattggg tgaaggaatc 360
tttgattcat attcaaagat gtacgctcac ataaccgcaa caactacaat cttatccgtt 420
gacaacgcag tcgacgaaat tgatagagtt attcattgtg ctttgaagga aaagaggcca 480
gtgcatattc atttgcctat tgacgtagcc ttaactgaga ttgaaatccc tcatgcacca 540
aaagtttaca cacacgaatc ccagaacgtc gatgcttaca ttcaagctgt tgagaaaaag 600
ttaatgtctg caaaacaacc agtaatcata gcaggtcatg aaatcaattc attcaagttg 660
cacgaacaac tggaacagtt tgtcaatcag acaaacatcc ctgttgcaca actttccttg 720
ggtaagtctg ctttcaatga agagaatgaa cattaccttg gtatctacga tggcaaaatc 780
gcaaaggaaa atgtgagaga gtacgtcgac aatgctgatg tcatattgaa cataggtgcc 840
aaactgactg attctgctac agctggattt tcctacaagt tcgatacaaa caacataatc 900
tacattaacc ataatgactt caaagctgaa gatgtgattt ctgataatgt ttcactgatt 960

gatcttgtga atggcctgaa ttctattgac tatagaaatg aaacacacta cccatcttat 1020
caaagatctg atatgaaata cgaattgaat gacgcaccac ttacacaatc taactatttc 1080
aaaatgatga acgcttttct agaaaaagat gacatcctac tagctgaaca aggtacatcc 1140
tttttcggcg catatgactt atccctatac aagggaaatc agtttatcgg tcagccttta 1200
tgggggtcaa tagggtatac ttttccatct ttactaggaa gtcaactagc agacatgcat 1260
aggagaaaca ttttgcttat aggcgatggt agtttacaac ttactgttca agccctaagt 1320
acaatgatta gaaaggatat caaaccaatc attttcgtta tcaataacga cggttacacc 1380
gtcgaaagac ttatccacgg catggaagag ccatacaatg atatccaaat gtggaactac 1440
aagcaattgc cagaagtatt tggtggaaaa gatactgtaa aagttcatga tgctaaaacc 1500
tccaacgaac tgaaaactgt aatggattct gttaaagcag acaaagatca catgcatttc 1560
attgaagtgc atatggcagt agaggacgcc ccaaagaagt tgattgatat agctaaagcc 1620
tttagtgatg ctaacaagta a 1641

<210> 34
<211> 1647

<212> DNA
<213> Listeria grayi

<400> 34

```
atgtacaccg tcggccaata cttagtagac cgcttagaag agatcggcat cgataaggtt      60
tttggtgtcc cgggtgacta caacctgacc tttttggact acatccagaa ccacgaaggt     120
ctgagctggc aaggtaatac gaatgaactg aatgccgcgt acgcagctga tggctatgct     180
cgtgaacgcg gtgttagcgc tttggtcacg accttcggcg ttggtgagct gtccgcaatc     240
aatggcaccg caggtagctt cgcggagcaa gttccggtga ttcatatcgt gggcagcccg     300
accatgaatg ttcagagcaa caagaaactg gttcatcaca gcctgggtat gggcaacttt     360
cacaacttca gcgagatggc gaaagaagtc accgccgcaa ccacgatgct gacggaagag     420
aatgcggcgt cggagattga tcgtgttctg gaaaccgccc tgctggagaa acgcccagtg     480
tacatcaatc tgccgatcga cattgctcac aaggcgatcg tcaagccggc gaaagccctg     540
caaaccgaga agagctctgg cgagcgtgag gcacaactgg cggagatcat tctgagccat     600
ctggagaagg ctgcacagcc gattgtgatt gcgggtcacg agatcgcgcg cttccagatc     660
cgtgagcgtt tcgagaattg gattaatcaa acgaaactgc cggtgaccaa tctggcctac     720
ggcaagggta gcttcaacga agaaaacgag catttcattg gtacctatta tcctgcattt     780
agcgataaga acgtgctgga ctacgtggat aactccgact ttgtcctgca ctttggtggt     840
aaaatcattg ataacagcac ctccagcttc tcccaaggct tcaaaaccga gaacaccctg     900
actgcggcga acgatatcat tatgctgccg gacggtagca cgtattctgg tattagcctg     960

aatggcctgc tggccgagct ggaaaaactg aatttcacgt ttgccgacac cgcagcaaag    1020
caggcggagt tggcggtgtt tgagccgcag gctgaaaccc cgttgaaaca ggaccgtttt    1080
caccaggcgg tgatgaattt tctgcaagct gacgatgtcc tggttacgga acagggcacc    1140
tcttcttttg gcttgatgct ggcgcctctg aaaaagggta tgaacttgat ctcgcaaacg    1200
ctgtggggta gcattggtta cacgttgccg gcgatgattg gtagccaaat tgcggcaccg    1260
gagcgtcgtc atatcctgag cattggtgat ggtagctttc agctgactgc gcaggaaatg    1320
agcaccattt tccgtgagaa actgacccca gtcatcttca tcattaacaa tgatggctat    1380
accgttgagc gtgcgatcca tggcgaagat gaaagctata acgacattcc gacgtggaac    1440
ttgcaactgg tggcggaaac cttcggtggt gacgccgaaa ccgtcgacac tcacaatgtg    1500
ttcacggaga ctgatttcgc caacaccctg gcggcaattg acgcgacgcc gcagaaagca    1560
cacgttgtgg aagttcacat ggaacaaatg gatatgccgg agagcctgcg ccagatcggt    1620
ctggcactgt ccaagcagaa tagctaa                                        1647
```

<210> 35
<211> 312
<212> PRT
<213> Saccharomyces cerevisiae

<400> 35

```
Met Pro Ala Thr Leu Lys Asn Ser Ser Ala Thr Leu Lys Leu Asn Thr
1               5                   10                  15

Gly Ala Ser Ile Pro Val Leu Gly Phe Gly Thr Trp Arg Ser Val Asp
            20                  25                  30

Asn Asn Gly Tyr His Ser Val Ile Ala Ala Leu Lys Ala Gly Tyr Arg
        35                  40                  45

His Ile Asp Ala Ala Ala Ile Tyr Leu Asn Glu Glu Glu Val Gly Arg
    50                  55                  60

Ala Ile Lys Asp Ser Gly Val Pro Arg Glu Glu Ile Phe Ile Thr Thr
65                  70                  75                  80

Lys Leu Trp Gly Thr Glu Gln Arg Asp Pro Glu Ala Ala Leu Asn Lys
                85                  90                  95

Ser Leu Lys Arg Leu Gly Leu Asp Tyr Val Asp Leu Tyr Leu Met His
            100                 105                 110

Trp Pro Val Pro Leu Lys Thr Asp Arg Val Thr Asp Gly Asn Val Leu
        115                 120                 125
```

Cys Ile Pro Thr Leu Glu Asp Gly Thr Val Asp Ile Asp Thr Lys Glu
130 135 140

Trp Asn Phe Ile Lys Thr Trp Glu Leu Met Gln Glu Leu Pro Lys Thr
145 150 155 160

Gly Lys Thr Lys Ala Val Gly Val Ser Asn Phe Ser Ile Asn Asn Ile
165 170 175

Lys Glu Leu Leu Glu Ser Pro Asn Asn Lys Val Val Pro Ala Thr Asn
180 185 190

Gln Ile Glu Ile His Pro Leu Leu Pro Gln Asp Glu Leu Ile Ala Phe
195 200 205

Cys Lys Glu Lys Gly Ile Val Val Glu Ala Tyr Ser Pro Phe Gly Ser
210 215 220

Ala Asn Ala Pro Leu Leu Lys Glu Gln Ala Ile Ile Asp Met Ala Lys
225 230 235 240

Lys His Gly Val Glu Pro Ala Gln Leu Ile Ile Ser Trp Ser Ile Gln
245 250 255

Arg Gly Tyr Val Val Leu Ala Lys Ser Val Asn Pro Glu Arg Ile Val
260 265 270

Ser Asn Phe Lys Ile Phe Thr Leu Pro Glu Asp Asp Phe Lys Thr Ile
275 280 285

Ser Asn Leu Ser Lys Val His Gly Thr Lys Arg Val Val Asp Met Lys
290 295 300

Trp Gly Ser Phe Pro Ile Phe Gln
305 310

<210> 36
<211> 939
<212> DNA
<213> Saccharomyces cerevisiae

<400> 36

atgcctgcta cgttaaagaa ttcttctgct acattaaaac taaatactgg tgcctccatt 60

ccagtgttgg gtttcggcac ttggcgttcc gttgacaata acggttacca ttctgtaatt 120

gcagctttga aagctggata cagacacatt gatgctgcgg ctatctattt gaatgaagaa 180

gaagttggca gggctattaa agattccgga gtccctcgtg aggaaatttt tattactact 240

aagctttggg gtacggaaca acgtgatccg gaagctgctc taaacaagtc tttgaaaaga 300

ctaggcttgg attatgttga cctatatctg atgcattggc cagtgccttt gaaaaccgac 360

agagttactg atggtaacgt tctgtgcatt ccaacattag aagatggcac tgttgacatc 420

gatactaagg aatggaattt tatcaagacg tgggagttga tgcaagagtt gccaaagacg 480

ggcaaaacta aagccgttgg tgtctctaat ttttctatta acaacattaa agaattatta 540

gaatctccaa ataacaaggt ggtaccagct actaatcaaa ttgaaattca tccattgcta 600

ccacaagacg aattgattgc cttttgtaag gaaaagggta ttgttgttga agcctactca 660

ccatttggga gtgctaatgc tcctttacta aaagagcaag caattattga tatggctaaa 720

aagcacggcg ttgagccagc acagcttatt atcagttgga gtattcaaag aggctacgtt 780

gttctggcca aatcggttaa tcctgaaaga attgtatcca attttaagat tttcactctg 840

cctgaggatg atttcaagac tattagtaac ctatccaaag tgcatggtac aaagagagtc 900

gttgatatga agtggggatc cttcccaatt ttccaatga 939

<210> 37
<211> 360
<212> PRT
<213> Saccharomyces cerevisiae

<400> 37

```
Met Ser Tyr Pro Glu Lys Phe Glu Gly Ile Ala Ile Gln Ser His Glu
1               5                   10                  15

Asp Trp Lys Asn Pro Lys Lys Thr Lys Tyr Asp Pro Lys Pro Phe Tyr
            20                  25                  30

Asp His Asp Ile Asp Ile Lys Ile Glu Ala Cys Gly Val Cys Gly Ser
        35                  40                  45

Asp Ile His Cys Ala Ala Gly His Trp Gly Asn Met Lys Met Pro Leu
    50                  55                  60

Val Val Gly His Glu Ile Val Gly Lys Val Val Lys Leu Gly Pro Lys
65                  70                  75                  80

Ser Asn Ser Gly Leu Lys Val Gly Gln Arg Val Gly Val Gly Ala Gln
                85                  90                  95

Val Phe Ser Cys Leu Glu Cys Asp Arg Cys Lys Asn Asp Asn Glu Pro
            100                 105                 110

Tyr Cys Thr Lys Phe Val Thr Thr Tyr Ser Gln Pro Tyr Glu Asp Gly
        115                 120                 125
```

```
Tyr Val Ser Gln Gly Gly Tyr Ala Asn Tyr Val Arg Val His Glu His
    130                 135                 140

Phe Val Val Pro Ile Pro Glu Asn Ile Pro Ser His Leu Ala Ala Pro
145                 150                 155                 160

Leu Leu Cys Gly Gly Leu Thr Val Tyr Ser Pro Leu Val Arg Asn Gly
                165                 170                 175

Cys Gly Pro Gly Lys Lys Val Gly Ile Val Gly Leu Gly Gly Ile Gly
            180                 185                 190

Ser Met Gly Thr Leu Ile Ser Lys Ala Met Gly Ala Glu Thr Tyr Val
        195                 200                 205

Ile Ser Arg Ser Ser Arg Lys Arg Glu Asp Ala Met Lys Met Gly Ala
    210                 215                 220

Asp His Tyr Ile Ala Thr Leu Glu Glu Gly Asp Trp Gly Glu Lys Tyr
225                 230                 235                 240

Phe Asp Thr Phe Asp Leu Ile Val Val Cys Ala Ser Ser Leu Thr Asp
                245                 250                 255

Ile Asp Phe Asn Ile Met Pro Lys Ala Met Lys Val Gly Gly Arg Ile
            260                 265                 270

Val Ser Ile Ser Ile Pro Glu Gln His Glu Met Leu Ser Leu Lys Pro
        275                 280                 285

Tyr Gly Leu Lys Ala Val Ser Ile Ser Tyr Ser Ala Leu Gly Ser Ile
    290                 295                 300

Lys Glu Leu Asn Gln Leu Leu Lys Leu Val Ser Glu Lys Asp Ile Lys
305                 310                 315                 320

Ile Trp Val Glu Thr Leu Pro Val Gly Glu Ala Gly Val His Glu Ala
                325                 330                 335

Phe Glu Arg Met Glu Lys Gly Asp Val Arg Tyr Arg Phe Thr Leu Val
            340                 345                 350

Gly Tyr Asp Lys Glu Phe Ser Asp
        355                 360
```

<210> 38
<211> 1083

<212> DNA
<213> Saccharomyces cerevisiae

<400> 38

```
ctagtctgaa aattctttgt cgtagccgac taaggtaaat ctatatctaa cgtcaccctt      60
ttccatcctt tcgaaggctt catggacgcc ggcttcacca acaggtaatg tttccaccca     120
aattttgata tctttttcag agactaattt caagagttgg ttcaattctt tgatggaacc     180
taaagcactg taagaaatgg agacagcctt taagccatat ggctttagcg ataacatttc     240
gtgttgttct ggtatagaga ttgagacaat tctaccacca accttcatag cctttggcat     300
aatgttgaag tcaatgtcgg taagggagga agcacagact acaatcaggt cgaaggtgtc     360
aaagtacttt tcaccccaat caccttcttc taatgtagca atgtagtgat cggcgcccat     420
cttcattgca tcttctcttt ttctcgaaga acgagaaata acatacgtct ctgcccccat     480
ggctttggaa atcaatgtac ccatactgcc gataccacca agaccaacta taccaacttt     540
tttacctgga ccgcaaccgt tacgaaccaa tggagagtac acagtcaaac caccacataa     600
tagtggagca gccaaatgtg atggaatatt ctctgggata ggcaccacaa aatgttcatg     660
aactctgacg tagtttgcat agccaccctg cgacacatag ccgtcttcat aaggctgact     720
gtatgtggta acaaacttgg tgcagtatgg ttcattatca ttcttacaac ggtcacattc     780
caagcatgaa aagacttgag cacctacacc aacacgttga ccgactttca acccactgtt     840
tgacttgggc cctagcttga caactttacc aacgatttca tgaccaacga ctagcggcat     900
cttcatattg ccccaatgac cagctgcaca atgaatatca ctaccgcaga caccacatgc     960
ttcgatctta atgtcaatgt catgatcgta aaatggtttt gggtcatact ttgtcttctt    1020
tgggtttttc caatcttcgt gtgattgaat agcgatacct tcaaatttct caggataaga    1080
cat                                                                  1083
```

<210> 39
<211> 387
<212> PRT
<213> Escherichia coli

<400> 39

```
Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
```

```
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Ala Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Asp Thr Lys Arg Ala Lys Leu
    290                 295                 300
```

Leu Gln Tyr Ala Glu Arg Val Trp Asn Ile Thr Glu Gly Ser Asp Asp
305 310 315 320

Glu Arg Ile Asp Ala Ala Ile Ala Ala Thr Arg Asn Phe Phe Glu Gln
325 330 335

Leu Gly Val Pro Thr His Leu Ser Asp Tyr Gly Leu Asp Gly Ser Ser
340 345 350

Ile Pro Ala Leu Leu Lys Lys Leu Glu Glu His Gly Met Thr Gln Leu
355 360 365

Gly Glu Asn His Asp Ile Thr Leu Asp Val Ser Arg Arg Ile Tyr Glu
370 375 380

Ala Ala Arg
385

<210> 40
<211> 387
<212> PRT
<213> Escherichia coli

<400> 40

Met Asn Asn Phe Asn Leu His Thr Pro Thr Arg Ile Leu Phe Gly Lys
1 5 10 15

Gly Ala Ile Ala Gly Leu Arg Glu Gln Ile Pro His Asp Ala Arg Val
20 25 30

Leu Ile Thr Tyr Gly Gly Gly Ser Val Lys Lys Thr Gly Val Leu Asp
35 40 45

Gln Val Leu Asp Ala Leu Lys Gly Met Asp Val Leu Glu Phe Gly Gly
50 55 60

Ile Glu Pro Asn Pro Ala Tyr Glu Thr Leu Met Asn Ala Val Lys Leu
65 70 75 80

Val Arg Glu Gln Lys Val Thr Phe Leu Leu Ala Val Gly Gly Gly Ser
85 90 95

Val Leu Asp Gly Thr Lys Phe Ile Ala Ala Ala Ala Asn Tyr Pro Glu
100 105 110

Asn Ile Asp Pro Trp His Ile Leu Gln Thr Gly Gly Lys Glu Ile Lys
115 120 125

```
Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Ala Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Asp Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380
```

Ala Ala Arg
385

<210> 41
<211> 389
<212> PRT
<213> Clostridium acetobutylicum

<400> 41

Met Leu Ser Phe Asp Tyr Ser Ile Pro Thr Lys Val Phe Phe Gly Lys
1 5 10 15

Gly Lys Ile Asp Val Ile Gly Glu Glu Ile Lys Lys Tyr Gly Ser Arg
20 25 30

Val Leu Ile Val Tyr Gly Gly Gly Ser Ile Lys Arg Asn Gly Ile Tyr
35 40 45

Asp Arg Ala Thr Ala Ile Leu Lys Glu Asn Asn Ile Ala Phe Tyr Glu
50 55 60

Leu Ser Gly Val Glu Pro Asn Pro Arg Ile Thr Thr Val Lys Lys Gly
65 70 75 80

Ile Glu Ile Cys Arg Glu Asn Asn Val Asp Leu Val Leu Ala Ile Gly
85 90 95

Gly Gly Ser Ala Ile Asp Cys Ser Lys Val Ile Ala Ala Gly Val Tyr
100 105 110

Tyr Asp Gly Asp Thr Trp Asp Met Val Lys Asp Pro Ser Lys Ile Thr
115 120 125

Lys Val Leu Pro Ile Ala Ser Ile Leu Thr Leu Ser Ala Thr Gly Ser
130 135 140

Glu Met Asp Gln Ile Ala Val Ile Ser Asn Met Glu Thr Asn Glu Lys
145 150 155 160

Leu Gly Val Gly His Asp Asp Met Arg Pro Lys Phe Ser Val Leu Asp
165 170 175

Pro Thr Tyr Thr Phe Thr Val Pro Lys Asn Gln Thr Ala Ala Gly Thr
180 185 190

Ala Asp Ile Met Ser His Thr Phe Glu Ser Tyr Phe Ser Gly Val Glu
195 200 205

Gly Ala Tyr Val Gln Asp Gly Ile Ala Glu Ala Ile Leu Arg Thr Cys
    210                 215                 220

Ile Lys Tyr Gly Lys Ile Ala Met Glu Lys Thr Asp Asp Tyr Glu Ala
225                 230                 235                 240

Arg Ala Asn Leu Met Trp Ala Ser Ser Leu Ala Ile Asn Gly Leu Leu
                245                 250                 255

Ser Leu Gly Lys Asp Arg Lys Trp Ser Cys His Pro Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asp Asp Thr Leu His Lys
    290                 295                 300

Phe Val Ser Tyr Gly Ile Asn Val Trp Gly Ile Asp Lys Asn Lys Asp
305                 310                 315                 320

Asn Tyr Glu Ile Ala Arg Glu Ala Ile Lys Asn Thr Arg Glu Tyr Phe
                325                 330                 335

Asn Ser Leu Gly Ile Pro Ser Lys Leu Arg Glu Val Gly Ile Gly Lys
            340                 345                 350

Asp Lys Leu Glu Leu Met Ala Lys Gln Ala Val Arg Asn Ser Gly Gly
        355                 360                 365

Thr Ile Gly Ser Leu Arg Pro Ile Asn Ala Glu Asp Val Leu Glu Ile
    370                 375                 380

Phe Lys Lys Ser Tyr
385

<210> 42
<211> 1170
<212> DNA
<213> Clostridium acetobutylicum

<400> 42

ttaataagat tttttaaata tctcaagaac atcctctgca tttattggtc ttaaacttcc 60

tattgttcct ccagaatttc taacagcttg ctttgccatt agttctagtt tatcttttcc 120

tattccaact tctctaagct ttgaaggaat acccaatgaa ttaaagtatt ctctcgtatt 180

tttaatagcc tctcgtgcta tttcatagtt atctttgttc ttgtctattc cccaaacatt 240

```
tattccataa gaaacaaatt tatgaagtgt atcgtcattt agaatatatt ccatccaatt      300
aggtgttaaa attgcaagtc ctacaccatg tgttatatca taatatgcac ttaactcgtg      360
ttccatagga tgacaactcc attttctatc cttaccaagt gataatagac catttatagc      420
taaacttgaa gcccacatca aattagctct agcctcgtaa tcatcagtct tctccattgc      480
tatttttcca tactttatac atgttcttaa gattgcttct gctataccgt cctgcacata      540
agcaccttca acaccactaa agtaagattc aaaggtgtga ctcataatgt cagctgttcc      600
cgctgctgtt tgatttttag gtactgtaaa agtatatgta ggatctaaca ctgaaaattt      660
aggtctcata tcatcatgtc ctactccaag cttttcatta gtctccatat ttgaaattac      720
tgcaatttga tccatttcag accctgttgc tgaaagagta agtatacttg caattggaag      780
aactttagtt attttagatg gatctttaac catgtcccat gtatcgccat cataataaac      840
tccagctgca attaccttag aacagtctat tgcacttcct ccccctattg ctaatactaa      900
atccacatta ttttctctac atatttctat gccttttttt actgttgtta tcctaggatt      960
tggctctact cctgaaagtt catagaaagc tatattgttt tcttttaata tagctgttgc     1020
tctatcatat ataccgttcc tttttatact tcctccgcca taaactataa gcactcttga     1080
gccatatttc ttaatttctt ctccaattac gtctattttt ccttttccaa aaaaaacttt     1140
agttggtatt gaataatcaa aacttagcat                                      1170
```

<210> 43
<211> 390
<212> PRT
<213> Clostridium acetobutylicum

<400> 43

```
Met Val Asp Phe Glu Tyr Ser Ile Pro Thr Arg Ile Phe Phe Gly Lys
1               5                   10                  15

Asp Lys Ile Asn Val Leu Gly Arg Glu Leu Lys Lys Tyr Gly Ser Lys
            20                  25                  30

Val Leu Ile Val Tyr Gly Gly Gly Ser Ile Lys Arg Asn Gly Ile Tyr
        35                  40                  45

Asp Lys Ala Val Ser Ile Leu Glu Lys Asn Ser Ile Lys Phe Tyr Glu
    50                  55                  60

Leu Ala Gly Val Glu Pro Asn Pro Arg Val Thr Thr Val Glu Lys Gly
65                  70                  75                  80

Val Lys Ile Cys Arg Glu Asn Gly Val Glu Val Val Leu Ala Ile Gly
                85                  90                  95
```

```
Gly Gly Ser Ala Ile Asp Cys Ala Lys Val Ile Ala Ala Ala Cys Glu
            100                 105                 110

Tyr Asp Gly Asn Pro Trp Asp Ile Val Leu Asp Gly Ser Lys Ile Lys
        115                 120                 125

Arg Val Leu Pro Ile Ala Ser Ile Leu Thr Ile Ala Ala Thr Gly Ser
    130                 135                 140

Glu Met Asp Thr Trp Ala Val Ile Asn Asn Met Asp Thr Asn Glu Lys
145                 150                 155                 160

Leu Ile Ala Ala His Pro Asp Met Ala Pro Lys Phe Ser Ile Leu Asp
                165                 170                 175

Pro Thr Tyr Thr Tyr Thr Val Pro Thr Asn Gln Thr Ala Ala Gly Thr
            180                 185                 190

Ala Asp Ile Met Ser His Ile Phe Glu Val Tyr Phe Ser Asn Thr Lys
        195                 200                 205

Thr Ala Tyr Leu Gln Asp Arg Met Ala Glu Ala Leu Leu Arg Thr Cys
    210                 215                 220

Ile Lys Tyr Gly Gly Ile Ala Leu Glu Lys Pro Asp Asp Tyr Glu Ala
225                 230                 235                 240

Arg Ala Asn Leu Met Trp Ala Ser Ser Leu Ala Ile Asn Gly Leu Leu
                245                 250                 255

Thr Tyr Gly Lys Asp Thr Asn Trp Ser Val His Leu Met Glu His Glu
            260                 265                 270

Leu Ser Ala Tyr Tyr Asp Ile Thr His Gly Val Gly Leu Ala Ile Leu
        275                 280                 285

Thr Pro Asn Trp Met Glu Tyr Ile Leu Asn Asn Asp Thr Val Tyr Lys
    290                 295                 300

Phe Val Glu Tyr Gly Val Asn Val Trp Gly Ile Asp Lys Glu Lys Asn
305                 310                 315                 320

His Tyr Asp Ile Ala His Gln Ala Ile Gln Lys Thr Arg Asp Tyr Phe
                325                 330                 335

Val Asn Val Leu Gly Leu Pro Ser Arg Leu Arg Asp Val Gly Ile Glu
            340                 345                 350
```

```
Glu Glu Lys Leu Asp Ile Met Ala Lys Glu Ser Val Lys Leu Thr Gly
        355                 360                 365

Gly Thr Ile Gly Asn Leu Arg Pro Val Asn Ala Ser Glu Val Leu Gln
    370                 375                 380

Ile Phe Lys Lys Ser Val
385                 390
```

<210> 44
<211> 1173
<212> DNA
<213> Clostridium acetobutylicum

<400> 44

```
gtggttgatt tcgaatattc aataccaact agaatttttt tcggtaaaga taagataaat      60
gtacttggaa gagagcttaa aaaatatggt tctaaagtgc ttatagttta tggtggagga     120
agtataaaga gaaatggaat atatgataaa gctgtaagta tacttgaaaa aaacagtatt     180
aaattttatg aacttgcagg agtagagcca aatccaagag taactacagt tgaaaaagga     240
gttaaaatat gtagagaaaa tggagttgaa gtagtactag ctataggtgg aggaagtgca     300
atagattgcg caaaggttat agcagcagca tgtgaatatg atggaaatcc atgggatatt     360
gtgttagatg gctcaaaaat aaaaagggtg cttcctatag ctagtatatt aaccattgct     420
gcaacaggat cagaaatgga tacgtgggca gtaataaata atatggatac aaacgaaaaa     480
ctaattgcgg cacatccaga tatggctcct aagttttcta tattagatcc aacgtatacg     540
tataccgtac ctaccaatca aacagcagca ggaacagctg atattatgag tcatatattt     600
gaggtgtatt ttagtaatac aaaaacagca tatttgcagg atagaatggc agaagcgtta     660
ttaagaactt gtattaaata tggaggaata gctcttgaga agccggatga ttatgaggca     720
agagccaatc taatgtgggc ttcaagtctt gcgataaatg gacttttaac atatggtaaa     780
gacactaatt ggagtgtaca cttaatggaa catgaattaa gtgcttatta cgacataaca     840
cacggcgtag ggcttgcaat tttaacacct aattggatgg agtatatttt aaataatgat     900
acagtgtaca agtttgttga atatggtgta aatgtttggg gaatagacaa agaaaaaaat     960
cactatgaca tagcacatca agcaatacaa aaaacaagag attactttgt aaatgtacta    1020
ggtttaccat ctagactgag agatgttgga attgaagaag aaaaattgga cataatggca    1080
aaggaatcag taaagcttac aggaggaacc ataggaaacc taagaccagt aaacgcctcc    1140
gaagtcctac aaatattcaa aaaatctgtg taa                                 1173
```

<210> 45
<211> 330

<212> PRT
<213> Bacillus subtilis

<400> 45

```
Met Ser Thr Asn Arg His Gln Ala Leu Gly Leu Thr Asp Gln Glu Ala
1               5                   10                  15

Val Asp Met Tyr Arg Thr Met Leu Leu Ala Arg Lys Ile Asp Glu Arg
            20                  25                  30

Met Trp Leu Leu Asn Arg Ser Gly Lys Ile Pro Phe Val Ile Ser Cys
        35                  40                  45

Gln Gly Gln Glu Ala Ala Gln Val Gly Ala Ala Phe Ala Leu Asp Arg
    50                  55                  60

Glu Met Asp Tyr Val Leu Pro Tyr Tyr Arg Asp Met Gly Val Val Leu
65                  70                  75                  80

Ala Phe Gly Met Thr Ala Lys Asp Leu Met Met Ser Gly Phe Ala Lys
                85                  90                  95

Ala Ala Asp Pro Asn Ser Gly Gly Arg Gln Met Pro Gly His Phe Gly
            100                 105                 110

Gln Lys Lys Asn Arg Ile Val Thr Gly Ser Ser Pro Val Thr Thr Gln
        115                 120                 125

Val Pro His Ala Val Gly Ile Ala Leu Ala Gly Arg Met Glu Lys Lys
    130                 135                 140

Asp Ile Ala Ala Phe Val Thr Phe Gly Glu Gly Ser Ser Asn Gln Gly
145                 150                 155                 160

Asp Phe His Glu Gly Ala Asn Phe Ala Ala Val His Lys Leu Pro Val
                165                 170                 175

Ile Phe Met Cys Glu Asn Asn Lys Tyr Ala Ile Ser Val Pro Tyr Asp
            180                 185                 190

Lys Gln Val Ala Cys Glu Asn Ile Ser Asp Arg Ala Ile Gly Tyr Gly
        195                 200                 205

Met Pro Gly Val Thr Val Asn Gly Asn Asp Pro Leu Glu Val Tyr Gln
    210                 215                 220

Ala Val Lys Glu Ala Arg Glu Arg Ala Arg Arg Gly Glu Gly Pro Thr
225                 230                 235                 240
```

Leu Ile Glu Thr Ile Ser Tyr Arg Leu Thr Pro His Ser Ser Asp Asp
245 250 255

Asp Asp Ser Ser Tyr Arg Gly Arg Glu Glu Val Glu Glu Ala Lys Lys
260 265 270

Ser Asp Pro Leu Leu Thr Tyr Gln Ala Tyr Leu Lys Glu Thr Gly Leu
275 280 285

Leu Ser Asp Glu Ile Glu Gln Thr Met Leu Asp Glu Ile Met Ala Ile
290 295 300

Val Asn Glu Ala Thr Asp Glu Ala Glu Asn Ala Pro Tyr Ala Ala Pro
305 310 315 320

Glu Ser Ala Leu Asp Tyr Val Tyr Ala Lys
325 330

<210> 46
<211> 993
<212> DNA
<213> Bacillus subtilis

<400> 46

```
atgagtacaa accgacatca agcactaggg ctgactgatc aggaagccgt tgatatgtat        60
agaaccatgc tgttagcaag aaaaatcgat gaaagaatgt ggctgttaaa ccgttctggc       120
aaaattccat ttgtaatctc ttgtcaagga caggaagcag cacaggtagg agcggctttc       180
gcacttgacc gtgaaatgga ttatgtattg ccgtactaca gagacatggg tgtcgtgctc       240
gcgtttggca tgacagcaaa ggacttaatg atgtccgggt ttgcaaaagc agcagatccg       300
aactcaggag gccgccagat gccgggacat ttcggacaaa agaaaaaccg cattgtgacg       360
ggatcatctc cggttacaac gcaagtgccg cacgcagtcg gtattgcgct tgcgggacgt       420
atggagaaaa aggatatcgc agcctttgtt acattcgggg aagggtcttc aaaccaaggc       480
gatttccatg aaggggcaaa ctttgccgct gtccataagc tgccggttat tttcatgtgt       540
gaaaacaaca aatacgcaat ctcagtgcct tacgataagc aagtcgcatg tgagaacatt       600
tccgaccgtg ccataggcta tgggatgcct ggcgtaactg tgaatggaaa tgatccgctg       660
gaagtttatc aagcggttaa agaagcacgc gaaagggcac gcagaggaga aggcccgaca       720
ttaattgaaa cgatttctta ccgccttaca ccacattcca gtgatgacga tgacagcagc       780
tacagaggcc gtgaagaagt agaggaagcg aaaaaaagtg atcccctgct tacttatcaa       840
gcttacttaa aggaaacagg cctgctgtcc gatgagatag aacaaaccat gctggatgaa       900
attatggcaa tcgtaaatga agcgacggat gaagcggaga acgccccata tgcagctcct       960

gagtcagcgc ttgattatgt ttatgcgaag tag                                    993
```

<210> 47
<211> 327
<212> PRT
<213> Bacillus subtilis

<400> 47

```
Met Ser Val Met Ser Tyr Ile Asp Ala Ile Asn Leu Ala Met Lys Glu
1               5                   10                  15

Glu Met Glu Arg Asp Ser Arg Val Phe Val Leu Gly Glu Asp Val Gly
            20                  25                  30

Arg Lys Gly Gly Val Phe Lys Ala Thr Ala Gly Leu Tyr Glu Gln Phe
        35                  40                  45

Gly Glu Glu Arg Val Met Asp Thr Pro Leu Ala Glu Ser Ala Ile Ala
    50                  55                  60

Gly Val Gly Ile Gly Ala Ala Met Tyr Gly Met Arg Pro Ile Ala Glu
65                  70                  75                  80

Met Gln Phe Ala Asp Phe Ile Met Pro Ala Val Asn Gln Ile Ile Ser
                85                  90                  95

Glu Ala Ala Lys Ile Arg Tyr Arg Ser Asn Asn Asp Trp Ser Cys Pro
            100                 105                 110

Ile Val Val Arg Ala Pro Tyr Gly Gly Gly Val His Gly Ala Leu Tyr
        115                 120                 125

His Ser Gln Ser Val Glu Ala Ile Phe Ala Asn Gln Pro Gly Leu Lys
    130                 135                 140

Ile Val Met Pro Ser Thr Pro Tyr Asp Ala Lys Gly Leu Leu Lys Ala
145                 150                 155                 160

Ala Val Arg Asp Glu Asp Pro Val Leu Phe Phe Glu His Lys Arg Ala
                165                 170                 175

Tyr Arg Leu Ile Lys Gly Glu Val Pro Ala Asp Asp Tyr Val Leu Pro
            180                 185                 190

Ile Gly Lys Ala Asp Val Lys Arg Glu Gly Asp Asp Ile Thr Val Ile
        195                 200                 205
```

Thr Tyr Gly Leu Cys Val His Phe Ala Leu Gln Ala Ala Glu Arg Leu
    210                 215                 220

Glu Lys Asp Gly Ile Ser Ala His Val Val Asp Leu Arg Thr Val Tyr
225                 230                 235                 240

Pro Leu Asp Lys Glu Ala Ile Ile Glu Ala Ala Ser Lys Thr Gly Lys
                245                 250                 255

Val Leu Leu Val Thr Glu Asp Thr Lys Glu Gly Ser Ile Met Ser Glu
            260                 265                 270

Val Ala Ala Ile Ile Ser Glu His Cys Leu Phe Asp Leu Asp Ala Pro
        275                 280                 285

Ile Lys Arg Leu Ala Gly Pro Asp Ile Pro Ala Met Pro Tyr Ala Pro
    290                 295                 300

Thr Met Glu Lys Tyr Phe Met Val Asn Pro Asp Lys Val Glu Ala Ala
305                 310                 315                 320

Met Arg Glu Leu Ala Glu Phe
                325

<210> 48
<211> 984
<212> DNA
<213> Bacillus subtilis

<400> 48

```
atgtcagtaa tgtcatatat tgatgcaatc aatttggcga tgaaagaaga aatggaacga        60
gattctcgcg ttttcgtcct tggggaagat gtaggaagaa aaggcggtgt gtttaaagcg       120
acagcgggac tctatgaaca atttggggaa gagcgcgtta tggatacgcc gcttgctgaa       180
tctgcaatcg caggagtcgg tatcggagcg gcaatgtacg gaatgagacc gattgctgaa       240
atgcagtttg ctgatttcat tatgccggca gtcaaccaaa ttatttctga agcggctaaa       300
atccgctacc gcagcaacaa tgactggagc tgtccgattg tcgtcagagc gccatacggc       360
ggaggcgtgc acggagccct gtatcattct caatcagtcg aagcaatttt cgccaaccag       420
cccggactga aaattgtcat gccatcaaca ccatatgacg cgaaagggct cttaaaagcc       480
gcagttcgtg acgaagaccc cgtgctgttt tttgagcaca agcgggcata ccgtctgata       540
aagggcgagg ttccggctga tgattatgtc ctgccaatcg gcaaggcgga cgtaaaaagg       600
gaaggcgacg acatcacagt gatcacatac ggcctgtgtg tccacttcgc cttacaagct       660
gcagaacgtc tcgaaaaaga tggcatttca gcgcatgtgg tggatttaag aacagtttac       720
ccgcttgata aagaagccat catcgaagct gcgtccaaaa ctggaaaggt tcttttggtc       780
acagaagata caaaagaagg cagcatcatg agcgaagtag ccgcaattat atccgagcat       840
tgtctgttcg acttagacgc gccgatcaaa cggcttgcag gtcctgatat tccggctatg       900
ccttatgcgc cgacaatgga aaaatacttt atggtcaacc ctgataaagt ggaagcggcg       960
atgagagaat tagcggagtt ttaa                                              984
```

<210> 49
<211> 424
<212> PRT
<213> Bacillus subtilis

<400> 49

```
Met Ala Ile Glu Gln Met Thr Met Pro Gln Leu Gly Glu Ser Val Thr
1               5                   10                  15

Glu Gly Thr Ile Ser Lys Trp Leu Val Ala Pro Gly Asp Lys Val Asn
            20                  25                  30

Lys Tyr Asp Pro Ile Ala Glu Val Met Thr Asp Lys Val Asn Ala Glu
        35                  40                  45

Val Pro Ser Ser Phe Thr Gly Thr Ile Thr Glu Leu Val Gly Glu Glu
    50                  55                  60

Gly Gln Thr Leu Gln Val Gly Glu Met Ile Cys Lys Ile Glu Thr Glu
65                  70                  75                  80

Gly Ala Asn Pro Ala Glu Gln Lys Gln Glu Gln Pro Ala Ala Ser Glu
                85                  90                  95

Ala Ala Glu Asn Pro Val Ala Lys Ser Ala Gly Ala Ala Asp Gln Pro
            100                 105                 110

Asn Lys Lys Arg Tyr Ser Pro Ala Val Leu Arg Leu Ala Gly Glu His
        115                 120                 125

Gly Ile Asp Leu Asp Gln Val Thr Gly Thr Gly Ala Gly Gly Arg Ile
    130                 135                 140

Thr Arg Lys Asp Ile Gln Arg Leu Ile Glu Thr Gly Gly Val Gln Glu
145                 150                 155                 160

Gln Asn Pro Glu Glu Leu Lys Thr Ala Ala Pro Ala Pro Lys Ser Ala
                165                 170                 175

Ser Lys Pro Glu Pro Lys Glu Glu Thr Ser Tyr Pro Ala Ser Ala Ala
            180                 185                 190
```

```
Gly Asp Lys Glu Ile Pro Val Thr Gly Val Arg Lys Ala Ile Ala Ser
        195                 200                 205

Asn Met Lys Arg Ser Lys Thr Glu Ile Pro His Ala Trp Thr Met Met
    210                 215                 220

Glu Val Asp Val Thr Asn Met Val Ala Tyr Arg Asn Ser Ile Lys Asp
225                 230                 235                 240

Ser Phe Lys Lys Thr Glu Gly Phe Asn Leu Thr Phe Phe Ala Phe Phe
                245                 250                 255

Val Lys Ala Val Ala Gln Ala Leu Lys Glu Phe Pro Gln Met Asn Ser
            260                 265                 270

Met Trp Ala Gly Asp Lys Ile Ile Gln Lys Lys Asp Ile Asn Ile Ser
        275                 280                 285

Ile Ala Val Ala Thr Glu Asp Ser Leu Phe Val Pro Val Ile Lys Asn
    290                 295                 300

Ala Asp Glu Lys Thr Ile Lys Gly Ile Ala Lys Asp Ile Thr Gly Leu
305                 310                 315                 320

Ala Lys Lys Val Arg Asp Gly Lys Leu Thr Ala Asp Asp Met Gln Gly
                325                 330                 335

Gly Thr Phe Thr Val Asn Asn Thr Gly Ser Phe Gly Ser Val Gln Ser
            340                 345                 350

Met Gly Ile Ile Asn Tyr Pro Gln Ala Ala Ile Leu Gln Val Glu Ser
        355                 360                 365

Ile Val Lys Arg Pro Val Val Met Asp Asn Gly Met Ile Ala Val Arg
    370                 375                 380

Asp Met Val Asn Leu Cys Leu Ser Leu Asp His Arg Val Leu Asp Gly
385                 390                 395                 400

Leu Val Cys Gly Arg Phe Leu Gly Arg Val Lys Gln Ile Leu Glu Ser
                405                 410                 415

Ile Asp Glu Lys Thr Ser Val Tyr
            420
```

<210> 50
<211> 1275

<212> DNA
<213> Bacillus subtilis

<400> 50

```
atggcaattg aacaaatgac gatgccgcag cttggagaaa gcgtaacaga ggggacgatc      60
agcaaatggc ttgtcgcccc cggtgataaa gtgaacaaat acgatccgat cgcggaagtc     120
atgacagata aggtaaatgc agaggttccg tcttctttta ctggtacgat aacagagctt     180
gtgggagaag aaggccaaac cctgcaagtc ggagaaatga tttgcaaaat tgaaacagaa     240
ggcgcgaatc cggctgaaca aaaacaagaa cagccagcag catcagaagc cgctgagaac     300
cctgttgcaa aaagtgctgg agcagccgat cagcccaata aaaagcgcta ctcgccagct     360
gttctccgtt tggccggaga gcacggcatt gacctcgatc aagtgacagg aactggtgcc     420
ggcgggcgca tcacacgaaa agatattcag cgcttaattg aaacaggcgg cgtgcaagaa     480
cagaatcctg aggagctgaa aacagcagct cctgcaccga agtctgcatc aaaacctgag     540
ccaaaagaag agacgtcata tcctgcgtct gcagccggtg ataaagaaat ccctgtcaca     600
ggtgtaagaa aagcaattgc ttccaatatg aagcgaagca aaacagaaat tccgcatgct     660
tggacgatga tggaagtcga cgtcacaaat atggttgcat atcgcaacag tataaaagat     720
tcttttaaga agacagaagg ctttaattta acgttcttcg cctttttgt aaaagcggtc      780
gctcaggcgt taaaagaatt cccgcaaatg aatagcatgt gggcgggga caaaattatt      840
cagaaaaagg atatcaatat ttcaattgca gttgccacag aggattcttt atttgttccg     900
gtgattaaaa acgctgatga aaaaacaatt aaaggcattg cgaaagacat taccggccta     960
gctaaaaaag taagagacgg aaaactcact gcagatgaca tgcagggagg cacgtttacc    1020
gtcaacaaca caggttcgtt cgggtctgtt cagtcgatgg gcattatcaa ctaccctcag    1080
gctgcgattc ttcaagtaga atccatcgtc aaacgcccgg ttgtcatgga caatggcatg    1140
attgctgtca gagacatggt taatctgtgc ctgtcattag atcacagagt gcttgacggt    1200
ctcgtgtgcg gacgattcct cggacgagtg aaacaaattt tagaatcgat tgacgagaag    1260
acatctgttt actaa                                                     1275
```

<210> 51
<211> 474
<212> PRT
<213> Bacillus subtilis

<400> 51

```
Met Ala Thr Glu Tyr Asp Val Val Ile Leu Gly Gly Gly Thr Gly Gly
1               5                   10                  15

Tyr Val Ala Ala Ile Arg Ala Ala Gln Leu Gly Leu Lys Thr Ala Val
            20                  25                  30
```

```
Val Glu Lys Glu Lys Leu Gly Gly Thr Cys Leu His Lys Gly Cys Ile
        35                  40                  45

Pro Ser Lys Ala Leu Leu Arg Ser Ala Glu Val Tyr Arg Thr Ala Arg
    50                  55                  60

Glu Ala Asp Gln Phe Gly Val Glu Thr Ala Gly Val Ser Leu Asn Phe
65                  70                  75                  80

Glu Lys Val Gln Gln Arg Lys Gln Ala Val Val Asp Lys Leu Ala Ala
                85                  90                  95

Gly Val Asn His Leu Met Lys Lys Gly Lys Ile Asp Val Tyr Thr Gly
            100                 105                 110

Tyr Gly Arg Ile Leu Gly Pro Ser Ile Phe Ser Pro Leu Pro Gly Thr
        115                 120                 125

Ile Ser Val Glu Arg Gly Asn Gly Glu Glu Asn Asp Met Leu Ile Pro
    130                 135                 140

Lys Gln Val Ile Ile Ala Thr Gly Ser Arg Pro Arg Met Leu Pro Gly
145                 150                 155                 160

Leu Glu Val Asp Gly Lys Ser Val Leu Thr Ser Asp Glu Ala Leu Gln
                165                 170                 175

Met Glu Glu Leu Pro Gln Ser Ile Ile Ile Val Gly Gly Gly Val Ile
            180                 185                 190

Gly Ile Glu Trp Ala Ser Met Leu His Asp Phe Gly Val Lys Val Thr
        195                 200                 205

Val Ile Glu Tyr Ala Asp Arg Ile Leu Pro Thr Glu Asp Leu Glu Ile
    210                 215                 220

Ser Lys Glu Met Glu Ser Leu Leu Lys Lys Lys Gly Ile Gln Phe Ile
225                 230                 235                 240

Thr Gly Ala Lys Val Leu Pro Asp Thr Met Thr Lys Thr Ser Asp Asp
                245                 250                 255

Ile Ser Ile Gln Ala Glu Lys Asp Gly Glu Thr Val Thr Tyr Ser Ala
            260                 265                 270

Glu Lys Met Leu Val Ser Ile Gly Arg Gln Ala Asn Ile Glu Gly Ile
```

```
        275                 280                 285

Gly Leu Glu Asn Thr Asp Ile Val Thr Glu Asn Gly Met Ile Ser Val
    290                 295                 300

Asn Glu Ser Cys Gln Thr Lys Glu Ser His Ile Tyr Ala Ile Gly Asp
305                 310                 315                 320

Val Ile Gly Gly Leu Gln Leu Ala His Val Ala Ser His Glu Gly Ile
                325                 330                 335

Ile Ala Val Glu His Phe Ala Gly Leu Asn Pro His Pro Leu Asp Pro
            340                 345                 350

Thr Leu Val Pro Lys Cys Ile Tyr Ser Ser Pro Glu Ala Ala Ser Val
        355                 360                 365

Gly Leu Thr Glu Asp Glu Ala Lys Ala Asn Gly His Asn Val Lys Ile
    370                 375                 380

Gly Lys Phe Pro Phe Met Ala Ile Gly Lys Ala Leu Val Tyr Gly Glu
385                 390                 395                 400

Ser Asp Gly Phe Val Lys Ile Val Ala Asp Arg Asp Thr Asp Asp Ile
                405                 410                 415

Leu Gly Val His Met Ile Gly Pro His Val Thr Asp Met Ile Ser Glu
            420                 425                 430

Ala Gly Leu Ala Lys Val Leu Asp Ala Thr Pro Trp Glu Val Gly Gln
        435                 440                 445

Thr Ile His Pro His Pro Thr Leu Ser Glu Ala Ile Gly Glu Ala Ala
    450                 455                 460

Leu Ala Ala Asp Gly Lys Ala Ile His Phe
465                 470
```

<210> 52
<211> 1425
<212> DNA
<213> Bacillus subtilis

<400> 52

atggcaactg agtatgacgt agtcattctg ggcggcggta ccggcggtta tgttgcggcc 60
atcagagccg ctcagctcgg cttaaaaaca gccgttgtgg aaaaggaaaa actcggggga 120
acatgtctgc ataaaggctg tatcccgagt aaagcgctgc ttagaagcgc agaggtatac 180
cggacagctc gtgaagccga tcaattcgga gtggaaacgg ctggcgtgtc cctcaacttt 240
gaaaaagtgc agcagcgtaa gcaagccgtt gttgataagc ttgcagcggg tgtaaatcat 300
ttaatgaaaa aaggaaaaat tgacgtgtac accggatatg gacgtatcct tggaccgtca 360
atcttctctc cgctgccggg aacaatttct gttgagcggg gaaatggcga agaaaatgac 420
atgctgatcc cgaaacaagt gatcattgca acaggatcaa gaccgagaat gcttccgggt 480
cttgaagtgg acggtaagtc tgtactgact tcagatgagg cgctccaaat ggaggagctg 540
ccacagtcaa tcatcattgt cggcggaggg gttatcggta tcgaatgggc gtctatgctt 600
catgattttg gcgttaaggt aacggttatt gaatacgcgg atcgcatatt gccgactgaa 660
gatctagaga tttcaaaaga aatggaaagt cttcttaaga aaaaaggcat ccagttcata 720
acaggggcaa aagtgctgcc tgacacaatg acaaaaacat cagacgatat cagcatacaa 780
gcggaaaaag acggagaaac cgttacctat tctgctgaga aaatgcttgt ttccatcggc 840
agacaggcaa atatcgaagg catcggccta gagaacaccg atattgttac tgaaaatggc 900
atgatttcag tcaatgaaag ctgccaaacg aaggaatctc atatttatgc aatcggagac 960
gtaatcggtg gcctgcagtt agctcacgtt gcttcacatg agggaattat tgctgttgag 1020
cattttgcag gtctcaatcc gcatccgctt gatccgacgc ttgtgccgaa gtgcatttac 1080
tcaagccctg aagctgccag tgtcggctta accgaagacg aagcaaaggc gaacgggcat 1140
aatgtcaaaa tcggcaagtt cccatttatg gcgattggaa aagcgcttgt atacggtgaa 1200
agcgacggtt ttgtcaaaat cgtggctgac cgagatacag atgatattct cggcgttcat 1260
atgattggcc cgcatgtcac cgacatgatt tctgaagcgg gtcttgccaa agtgctggac 1320
gcaacaccgt gggaggtcgg gcaaacgatt cacccgcatc caacgctttc tgaagcaatt 1380
ggagaagctg cgcttgccgc agatggcaaa gccattcatt tttaa 1425

<210> 53
<211> 410
<212> PRT
<213> Pseudomonas putida

<400> 53

```
Met Asn Glu Tyr Ala Pro Leu Arg Leu His Val Pro Glu Pro Thr Gly
1               5                   10                  15

Arg Pro Gly Cys Gln Thr Asp Phe Ser Tyr Leu Arg Leu Asn Asp Ala
            20                  25                  30

Gly Gln Ala Arg Lys Pro Pro Val Asp Val Asp Ala Ala Asp Thr Ala
        35                  40                  45

Asp Leu Ser Tyr Ser Leu Val Arg Val Leu Asp Glu Gln Gly Asp Ala
```

50 55 60

Gln Gly Pro Trp Ala Glu Asp Ile Asp Pro Gln Ile Leu Arg Gln Gly
65 70 75 80

Met Arg Ala Met Leu Lys Thr Arg Ile Phe Asp Ser Arg Met Val Val
85 90 95

Ala Gln Arg Gln Lys Lys Met Ser Phe Tyr Met Gln Ser Leu Gly Glu
100 105 110

Glu Ala Ile Gly Ser Gly Gln Ala Leu Ala Leu Asn Arg Thr Asp Met
115 120 125

Cys Phe Pro Thr Tyr Arg Gln Gln Ser Ile Leu Met Ala Arg Asp Val
130 135 140

Ser Leu Val Glu Met Ile Cys Gln Leu Leu Ser Asn Glu Arg Asp Pro
145 150 155 160

Leu Lys Gly Arg Gln Leu Pro Ile Met Tyr Ser Val Arg Glu Ala Gly
165 170 175

Phe Phe Thr Ile Ser Gly Asn Leu Ala Thr Gln Phe Val Gln Ala Val
180 185 190

Gly Trp Ala Met Ala Ser Ala Ile Lys Gly Asp Thr Lys Ile Ala Ser
195 200 205

Ala Trp Ile Gly Asp Gly Ala Thr Ala Glu Ser Asp Phe His Thr Ala
210 215 220

Leu Thr Phe Ala His Val Tyr Arg Ala Pro Val Ile Leu Asn Val Val
225 230 235 240

Asn Asn Gln Trp Ala Ile Ser Thr Phe Gln Ala Ile Ala Gly Gly Glu
245 250 255

Ser Thr Thr Phe Ala Gly Arg Gly Val Gly Cys Gly Ile Ala Ser Leu
260 265 270

Arg Val Asp Gly Asn Asp Phe Val Ala Val Tyr Ala Ala Ser Arg Trp
275 280 285

Ala Ala Glu Arg Ala Arg Arg Gly Leu Gly Pro Ser Leu Ile Glu Trp
290 295 300

Val Thr Tyr Arg Ala Gly Pro His Ser Thr Ser Asp Asp Pro Ser Lys
305 310 315 320

Tyr Arg Pro Ala Asp Asp Trp Ser His Phe Pro Leu Gly Asp Pro Ile
325 330 335

Ala Arg Leu Lys Gln His Leu Ile Lys Ile Gly His Trp Ser Glu Glu
340 345 350

Glu His Gln Ala Thr Thr Ala Glu Phe Glu Ala Ala Val Ile Ala Ala
355 360 365

Gln Lys Glu Ala Glu Gln Tyr Gly Thr Leu Ala Asn Gly His Ile Pro
370 375 380

Ser Ala Ala Ser Met Phe Glu Asp Val Tyr Lys Glu Met Pro Asp His
385 390 395 400

Leu Arg Arg Gln Arg Gln Glu Leu Gly Val
405 410

<210> 54
<211> 6643
<212> DNA
<213> Pseudomonas putida

<400> 54

gcatgcctgc aggccgccga tgaaatggtg gaaggtatcg gtaggctggc cctgctcatc 60

gctgaacacg ttacgcccgc tgccggtatc gaccaggctc tggtgaatat gcatggaact 120

gccaggcgtg cgcgccagcg gtttggccat gcacaccacg gtcagcccgt gcttgagtgc 180

cacttccttg agcaggtgtt tgaacaggaa ggtctggtcg gccagcagca gcgggtcgcc 240

atgtagcaag ttgatctcga actggctgac gcccatttcg tgcatgaagg tgtcgcgcgg 300

caggccgagc gcggccatgc actggtacac ctcattgaag aacgggcgca ggccgttgtt 360

ggaactgaca ctgaacgccg aatggcccag ctcgcggcgg ccgtcggtgc ccagcggtgg 420

ctggaacggc tgctgcgggt cactgttggg ggcaaacacg aagaactcaa gctcggtcgc 480

cactaccggt gccagaccca acgctgcgta gcgggcgatc acggccttca gctggccccg 540

ggtggacagt gccgagggcc ggccatccag ttcattggca tcgcagatgg ccagggcgcg 600

accgtcatcg ctccagggca agcgatgaac ctggctgggt tccgctacca acgccaggtc 660

gccgtcgtcg cagccgtaga atttcgccgg cgggtagccg cccatgatgc attgcagcag 720

caccccacgg gccatctgca ggcggcggcc ttcgagaaag ccttcggcgg tcatcacctt 780

gccgcgtggg acgccgttga ggtcgggggt gacgcattcg atttcatcga tgccctggag 840

ctgagcgatg ctcatgacgc ttgtccttgt tgttgtaggc tgacaacaac ataggctggg 900

ggtgtttaaa atatcaagca gcctctcgaa cgcctggggc ctcttctatt cgcgcaaggt 960
catgccattg gccggcaacg gcaaggctgt cttgtagcgc acctgtttca aggcaaaact 1020
cgagcggata ttcgccacac ccggcaaccg ggtcaggtaa tcgagaaacc gctccagcgc 1080
ctggatactc ggcagcagta cccgcaacag gtagtccggg tcgcccgtca tcaggtagca 1140
ctccatcacc tcgggccgtt cggcaatttc ttcctcgaag cggtgcagcg actgctctac 1200
ctgtttttcc aggctgacat ggatgaacac attcacatcc agccccaacg cctcgggcga 1260
caacaaggtc acctgctggc ggatcacccc cagttcttcc atggcccgca cccggttgaa 1320
acagggcgtg ggcgacaggt tgaccgagcg tgccagctcg gcgttggtga tgcgggcgtt 1380
ttcctgcagg ctgttgagaa tgccgatatc ggtacgatcg agtttgcgca tgagacaaaa 1440
tcaccggttt tttgtgttta tgcggaatgt ttatctgccc cgctcggcaa aggcaatcaa 1500
cttgagagaa aaattctcct gccggaccac taagatgtag ggacgctga cttaccagtc 1560
acaagccggt actcagcggc ggccgcttca gagctcacaa aaacaaatac ccgagcgagc 1620
gtaaaaagca tgaacgagta cgccccccctg cgtttgcatg tgcccgagcc caccggccgg 1680
ccaggctgcc agaccgattt ttcctacctg cgcctgaacg atgcaggtca agcccgtaaa 1740
ccccctgtcg atgtcgacgc tgccgacacc gccgacctgt cctacagcct ggtccgcgtg 1800
ctcgacgagc aaggcgacgc ccaaggcccg tgggctgaag acatcgaccc gcagatcctg 1860
cgccaaggca tgcgcgccat gctcaagacg cggatcttcg acagccgcat ggtggttgcc 1920
cagcgccaga agaagatgtc cttctacatg cagagcctgg gcgaagaagc catcggcagc 1980
ggccaggcgc tggcgcttaa ccgcaccgac atgtgcttcc ccacctaccg tcagcaaagc 2040
atcctgatgg cccgcgacgt gtcgctggtg gagatgatct gccagttgct gtccaacgaa 2100
cgcgaccccc tcaagggccg ccagctgccg atcatgtact cggtacgcga ggccggcttc 2160
ttcaccatca gcggcaacct ggcgacccag ttcgtgcagg cggtcggctg ggccatggcc 2220
tcggcgatca agggcgatac caagattgcc tcggcctgga tcggcgacgg cgccactgcc 2280
gaatcggact tccacaccgc cctcaccttt gcccacgttt accgcgcccc ggtgatcctc 2340
aacgtggtca acaaccagtg ggccatctca accttccagg ccatcgccgg tggcgagtcg 2400
accaccttcg ccggccgtgg cgtgggctgc ggcatcgctt cgctgcgggt ggacggcaac 2460
gacttcgtcg ccgtttacgc cgcttcgcgc tgggctgccg aacgtgcccg ccgtggtttg 2520
ggcccgagcc tgatcgagtg ggtcacctac cgtgccggcc cgcactcgac ctcggacgac 2580
ccgtccaagt accgccctgc cgatgactgg agccacttcc cgctgggtga cccgatcgcc 2640
cgcctgaagc agcacctgat caagatcggc cactggtccg aagaagaaca ccaggccacc 2700
acggccgagt tcgaagcggc cgtgattgct gcgcaaaaag aagccgagca gtacggcacc 2760

ctggccaacg gtcacatccc gagcgccgcc tcgatgttcg aggacgtgta caaggagatg 2820
cccgaccacc tgcgccgcca acgccaggaa ctgggggttt gagatgaacg accacaacaa 2880
cagcatcaac ccggaaaccg ccatggccac cactaccatg accatgatcc aggccctgcg 2940
ctcggccatg gatgtcatgc ttgagcgcga cgacaatgtg gtggtgtacg gccaggacgt 3000
cggctacttc ggcggcgtgt tccgctgcac cgaaggcctg cagaccaagt acggcaagtc 3060
ccgcgtgttc gacgcgccca tctctgaaag cggcatcgtc ggcaccgccg tgggcatggg 3120
tgcctacggc ctgcgcccgg tggtggaaat ccagttcgct gactacttct acccggcctc 3180
cgaccagatc gtttctgaaa tggcccgcct gcgctaccgt tcggccggcg agttcatcgc 3240
cccgctgacc ctgcgtatgc cctgcggtgg cggtatctat ggcggccaga cacacagcca 3300
gagcccggaa gcgatgttca ctcaggtgtg cggcctgcgc accgtaatgc catccaaccc 3360
gtacgacgcc aaaggcctgc tgattgcctc gatcgaatgc gacgacccgg tgatcttcct 3420
ggagcccaag cgcctgtaca acggcccgtt cgacggccac catgaccgcc cggttacgcc 3480
gtggtcgaaa cacccgcaca gcgccgtgcc cgatggctac tacaccgtgc cactggacaa 3540
ggccgccatc acccgccccg gcaatgacgt gagcgtgctc acctatggca ccaccgtgta 3600
cgtggcccag gtggccgccg aagaaagtgg cgtggatgcc gaagtgatcg acctgcgcag 3660
cctgtggccg ctagacctgg acaccatcgt cgagtcggtg aaaaagaccg gccgttgcgt 3720
ggtagtacac gaggccaccc gtacttgtgg ctttggcgca gaactggtgt cgctggtgca 3780
ggagcactgc ttccaccacc tggaggcgcc gatcgagcgc gtcaccggtt gggacacccc 3840
ctaccctcac gcgcaggaat gggcttactt cccagggcct tcgcgggtag gtgcggcatt 3900
gaaaaaggtc atggaggtct gaatgggcac gcacgtcatc aagatgccgg acattggcga 3960
aggcatcgcg caggtcgaat tggtggaatg gttcgtcaag gtgggcgaca tcatcgccga 4020
ggaccaagtg gtagccgacg tcatgaccga caaggccacc gtggaaatcc cgtcgccggt 4080
cagcggcaag gtgctggccc tgggtggcca gccaggtgaa gtgatggcgg tcggcagtga 4140
gctgatccgc atcgaagtgg aaggcagcgg caaccatgtg gatgtgccgc aagccaagcc 4200
ggccgaagtg cctgcggcac cggtagccgc taaacctgaa ccacagaaag acgttaaacc 4260
ggcggcgtac caggcgtcag ccagccacga ggcagcgccc atcgtgccgc gccagccggg 4320
cgacaagccg ctggcctcgc cggcggtgcg caaacgcgcc ctcgatgccg gcatcgaatt 4380
gcgttatgtg cacggcagcg gcccggccgg gcgcatcctg cacgaagacc tcgacgcgtt 4440
catgagcaaa ccgcaaagcg ctgccgggca aacccccaat ggctatgcca ggcgcaccga 4500
cagcgagcag gtgccggtga tcggcctgcg ccgcaagatc gcccagcgca tgcaggacgc 4560
caagcgccgg gtcgcgcact tcagctatgt ggaagaaatc gacgtcaccg ccctggaagc 4620
cctgcgccag cagctcaaca gcaagcacgg cgacagccgc ggcaagctga cactgctgcc 4680

gttcctggtg cgcgccctgg tcgtggcact gcgtgacttc ccgcagataa acgccaccta 4740
cgatgacgaa gcgcagatca tcacccgcca tggcgcggtg catgtgggca tcgccaccca 4800
aggtgacaac ggcctgatgg tacccgtgct gcgccacgcc gaagcgggca gcctgtgggc 4860
caatgccggt gagatttcac gcctggccaa cgctgcgcgc aacaacaagg ccagccgcga 4920
agagctgtcc ggttcgacca ttaccctgac cagcctcggc gccctgggcg gcatcgtcag 4980
cacgccggtg gtcaacaccc cggaagtggc gatcgtcggt gtcaaccgca tggttgagcg 5040
gcccgtggtg atcgacggcc agatcgtcgt gcgcaagatg atgaacctgt ccagctcgtt 5100
cgaccaccgc gtggtcgatg gcatggacgc cgccctgttc atccaggccg tgcgtggcct 5160
gctcgaacaa cccgcctgcc tgttcgtgga gtgagcatgc aacagactat ccagacaacc 5220
ctgttgatca tcggcggcgg ccctggcggc tatgtggcgg ccatccgcgc cgggcaactg 5280
ggcatcccta ccgtgctggt ggaaggccag gcgctgggcg gtacctgcct gaacatcggc 5340
tgcattccgt ccaaggcgct gatccatgtg gccgagcagt tccaccaggc ctcgcgcttt 5400
accgaaccct cgccgctggg catcagcgtg gcttcgccac gcctggacat cggccagagc 5460
gtggcctgga aagacggcat cgtcgatcgc ctgaccactg gtgtcgccgc cctgctgaaa 5520
aagcacgggg tgaaggtggt gcacggctgg gccaaggtgc ttgatggcaa gcaggtcgag 5580
gtggatggcc agcgcatcca gtgcgagcac ctgttgctgg ccacgggctc cagcagtgtc 5640
gaactgccga tgctgccgtt gggtgggccg gtgatttcct cgaccgaggc cctggcaccg 5700
aaagccctgc cgcaacacct ggtggtggtg ggcggtggct acatcggcct ggagctgggt 5760
atcgcctacc gcaagctcgg cgcgcaggtc agcgtggtgg aagcgcgcga gcgcatcctg 5820
ccgacttacg acagcgaact gaccgccccg gtggccgagt cgctgaaaaa gctgggtatc 5880
gccctgcacc ttggccacag cgtcgaaggt tacgaaaatg gctgcctgct ggccaacgat 5940
ggcaagggcg gacaactgcg cctggaagcc gaccgggtgc tggtggccgt gggccgccgc 6000
ccacgcacca agggcttcaa cctggaatgc ctggacctga agatgaatgg tgccgcgatt 6060
gccatcgacg agcgctgcca gaccagcatg cacaacgtct gggccatcgg cgacgtggcc 6120
ggcgaaccga tgctggcgca ccgggccatg gcccagggcg agatggtggc cgagatcatc 6180
gccggcaagg cacgccgctt cgaacccgct gcgatagccg ccgtgtgctt caccgacccg 6240
gaagtggtcg tggtcggcaa gacgccggaa caggccagtc agcaaggcct ggactgcatc 6300
gtcgcgcagt tcccgttcgc cgccaacggc cgggccatga gcctggagtc gaaaagcggt 6360
ttcgtgcgcg tggtcgcgcg gcgtgacaac cacctgatcc tgggctggca agcggttggc 6420
gtggcggttt ccgagctgtc cacggcgttt gcccagtcgc tggagatggg tgcctgcctg 6480
gaggatgtgg ccggtaccat ccatgcccac ccgaccctgg gtgaagcggt acaggaagcg 6540

gcactgcgtg ccctgggcca cgccctgcat atctgacact gaagcggccg aggccgattt 6600

ggcccgccgc gccgagaggc gctgcgggtc ttttttatac ctg 6643

<210> 55
<211> 352
<212> PRT
<213> Pseudomonas putida

<400> 55

```
Met Asn Asp His Asn Asn Ser Ile Asn Pro Glu Thr Ala Met Ala Thr
1               5                   10                  15

Thr Thr Met Thr Met Ile Gln Ala Leu Arg Ser Ala Met Asp Val Met
            20                  25                  30

Leu Glu Arg Asp Asp Asn Val Val Val Tyr Gly Gln Asp Val Gly Tyr
        35                  40                  45

Phe Gly Gly Val Phe Arg Cys Thr Glu Gly Leu Gln Thr Lys Tyr Gly
    50                  55                  60

Lys Ser Arg Val Phe Asp Ala Pro Ile Ser Glu Ser Gly Ile Val Gly
65                  70                  75                  80

Thr Ala Val Gly Met Gly Ala Tyr Gly Leu Arg Pro Val Val Glu Ile
                85                  90                  95

Gln Phe Ala Asp Tyr Phe Tyr Pro Ala Ser Asp Gln Ile Val Ser Glu
            100                 105                 110

Met Ala Arg Leu Arg Tyr Arg Ser Ala Gly Glu Phe Ile Ala Pro Leu
        115                 120                 125

Thr Leu Arg Met Pro Cys Gly Gly Gly Ile Tyr Gly Gly Gln Thr His
    130                 135                 140

Ser Gln Ser Pro Glu Ala Met Phe Thr Gln Val Cys Gly Leu Arg Thr
145                 150                 155                 160

Val Met Pro Ser Asn Pro Tyr Asp Ala Lys Gly Leu Leu Ile Ala Ser
                165                 170                 175

Ile Glu Cys Asp Asp Pro Val Ile Phe Leu Glu Pro Lys Arg Leu Tyr
            180                 185                 190

Asn Gly Pro Phe Asp Gly His His Asp Arg Pro Val Thr Pro Trp Ser
        195                 200                 205
```

Lys His Pro His Ser Ala Val Pro Asp Gly Tyr Tyr Thr Val Pro Leu
210 215 220

Asp Lys Ala Ala Ile Thr Arg Pro Gly Asn Asp Val Ser Val Leu Thr
225 230 235 240

Tyr Gly Thr Thr Val Tyr Val Ala Gln Val Ala Ala Glu Glu Ser Gly
245 250 255

Val Asp Ala Glu Val Ile Asp Leu Arg Ser Leu Trp Pro Leu Asp Leu
260 265 270

Asp Thr Ile Val Glu Ser Val Lys Lys Thr Gly Arg Cys Val Val Val
275 280 285

His Glu Ala Thr Arg Thr Cys Gly Phe Gly Ala Glu Leu Val Ser Leu
290 295 300

Val Gln Glu His Cys Phe His His Leu Glu Ala Pro Ile Glu Arg Val
305 310 315 320

Thr Gly Trp Asp Thr Pro Tyr Pro His Ala Gln Glu Trp Ala Tyr Phe
325 330 335

Pro Gly Pro Ser Arg Val Gly Ala Ala Leu Lys Lys Val Met Glu Val
340 345 350

<210> 56
<211> 6643
<212> DNA
<213> Pseudomonas putida

<400> 56

gcatgcctgc aggccgccga tgaaatggtg gaaggtatcg gtaggctggc cctgctcatc 60

gctgaacacg ttacgcccgc tgccggtatc gaccaggctc tggtgaatat gcatggaact 120

gccaggcgtg cgcgccagcg gtttggccat gcacaccacg gtcagcccgt gcttgagtgc 180

cacttccttg agcaggtgtt tgaacaggaa ggtctggtcg gccagcagca gcgggtcgcc 240

atgtagcaag ttgatctcga actggctgac gcccatttcg tgcatgaagg tgtcgcgcgg 300

caggccgagc gcggccatgc actggtacac ctcattgaag aacgggcgca ggccgttgtt 360

ggaactgaca ctgaacgccg aatggcccag ctcgcggcgg ccgtcggtgc ccagcggtgg 420

ctggaacggc tgctgcgggt cactgttggg ggcaaacacg aagaactcaa gctcggtcgc 480

cactaccggt gccagaccca acgctgcgta gcgggcgatc acggccttca gctggccccg 540

ggtggacagt gccgagggcc ggccatccag ttcattggca tcgcagatgg ccagggcgcg 600

accgtcatcg ctccagggca agcgatgaac ctggctgggt tccgctacca acgccaggtc 660

gccgtcgtcg cagccgtaga atttcgccgg cgggtagccg cccatgatgc attgcagcag 720

caccccacgg gccatctgca ggcggcggcc ttcgagaaag ccttcggcgg tcatcacctt 780

gccgcgtggg acgccgttga ggtcgggggt gacgcattcg atttcatcga tgccctggag 840

ctgagcgatg ctcatgacgc ttgtccttgt tgttgtaggc tgacaacaac ataggctggg 900

ggtgtttaaa atatcaagca gcctctcgaa cgcctggggc ctcttctatt cgcgcaaggt 960

catgccattg gccggcaacg gcaaggctgt cttgtagcgc acctgtttca aggcaaaact 1020

cgagcggata ttcgccacac ccggcaaccg ggtcaggtaa tcgagaaacc gctccagcgc 1080

ctggatactc ggcagcagta cccgcaacag gtagtccggg tcgcccgtca tcaggtagca 1140

ctccatcacc tcgggccgtt cggcaatttc ttcctcgaag cggtgcagcg actgctctac 1200

ctgtttttcc aggctgacat ggatgaacac attcacatcc agccccaacg cctcgggcga 1260

caacaaggtc acctgctggc ggatcacccc cagttcttcc atggcccgca cccggttgaa 1320

acagggcgtg ggcgacaggt tgaccgagcg tgccagctcg gcgttggtga tgcgggcgtt 1380

ttcctgcagg ctgttgagaa tgccgatatc ggtacgatcg agtttgcgca tgagacaaaa 1440

tcaccggttt tttgtgttta tgcggaatgt ttatctgccc cgctcggcaa aggcaatcaa 1500

cttgagagaa aaattctcct gccggaccac taagatgtag ggacgctga cttaccagtc 1560

acaagccggt actcagcggc ggccgcttca gagctcacaa aaacaaatac ccgagcgagc 1620

gtaaaaagca tgaacgagta cgcccccctg cgtttgcatg tgcccgagcc caccggccgg 1680

ccaggctgcc agaccgattt ttcctacctg cgcctgaacg atgcaggtca agcccgtaaa 1740

cccccctgtcg atgtcgacgc tgccgacacc gccgacctgt cctacagcct ggtccgcgtg 1800

ctcgacgagc aaggcgacgc ccaaggcccg tgggctgaag acatcgaccc gcagatcctg 1860

cgccaaggca tgcgcgccat gctcaagacg cggatcttcg acagccgcat ggtggttgcc 1920

cagcgccaga agaagatgtc cttctacatg cagagcctgg gcgaagaagc catcggcagc 1980

ggccaggcgc tggcgcttaa ccgcaccgac atgtgcttcc ccacctaccg tcagcaaagc 2040

atcctgatgg cccgcgacgt gtcgctggtg gagatgatct gccagttgct gtccaacgaa 2100

cgcgaccccc tcaagggccg ccagctgccg atcatgtact cggtacgcga ggccggcttc 2160

ttcaccatca gcggcaacct ggcgacccag ttcgtgcagg cggtcggctg ggccatggcc 2220

tcggcgatca agggcgatac caagattgcc tcggcctgga tcggcgacgg cgccactgcc 2280

gaatcggact tccacaccgc cctcaccttt gcccacgttt accgcgcccc ggtgatcctc 2340

aacgtggtca acaaccagtg ggccatctca accttccagg ccatcgccgg tggcgagtcg 2400

accaccttcg ccggccgtgg cgtgggctgc ggcatcgctt cgctgcgggt ggacggcaac 2460

gacttcgtcg ccgtttacgc cgcttcgcgc tgggctgccg aacgtgcccg ccgtggtttg 2520

ggcccgagcc tgatcgagtg ggtcacctac cgtgccggcc cgcactcgac ctcggacgac 2580

ccgtccaagt accgccctgc cgatgactgg agccacttcc cgctgggtga cccgatcgcc 2640

cgcctgaagc agcacctgat caagatcggc cactggtccg aagaagaaca ccaggccacc 2700

acggccgagt tcgaagcggc cgtgattgct gcgcaaaaag aagccgagca gtacggcacc 2760

ctggccaacg gtcacatccc gagcgccgcc tcgatgttcg aggacgtgta caaggagatg 2820

cccgaccacc tgcgccgcca acgccaggaa ctgggggttt gagatgaacg accacaacaa 2880

cagcatcaac ccggaaaccg ccatggccac cactaccatg accatgatcc aggccctgcg 2940

ctcggccatg gatgtcatgc ttgagcgcga cgacaatgtg gtggtgtacg gccaggacgt 3000

cggctacttc ggcggcgtgt tccgctgcac cgaaggcctg cagaccaagt acggcaagtc 3060

ccgcgtgttc gacgcgccca tctctgaaag cggcatcgtc ggcaccgccg tgggcatggg 3120

tgcctacggc ctgcgcccgg tggtggaaat ccagttcgct gactacttct acccggcctc 3180

cgaccagatc gtttctgaaa tggcccgcct gcgctaccgt tcggccggcg agttcatcgc 3240

cccgctgacc ctgcgtatgc cctgcggtgg cggtatctat ggcggccaga cacacagcca 3300

gagcccggaa gcgatgttca ctcaggtgtg cggcctgcgc accgtaatgc catccaaccc 3360

gtacgacgcc aaaggcctgc tgattgcctc gatcgaatgc gacgacccgg tgatcttcct 3420

ggagcccaag cgcctgtaca acggcccgtt cgacggccac catgaccgcc cggttacgcc 3480

gtggtcgaaa cacccgcaca gcgccgtgcc cgatggctac tacaccgtgc cactggacaa 3540

ggccgccatc acccgccccg gcaatgacgt gagcgtgctc acctatggca ccaccgtgta 3600

cgtggcccag gtggccgccg aagaaagtgg cgtggatgcc gaagtgatcg acctgcgcag 3660

cctgtggccg ctagacctgg acaccatcgt cgagtcggtg aaaaagaccg gccgttgcgt 3720

ggtagtacac gaggccaccc gtacttgtgg ctttggcgca gaactggtgt cgctggtgca 3780

ggagcactgc ttccaccacc tggaggcgcc gatcgagcgc gtcaccggtt gggacacccc 3840

ctaccctcac gcgcaggaat gggcttactt cccagggcct tcgcgggtag gtgcggcatt 3900

gaaaaaggtc atggaggtct gaatgggcac gcacgtcatc aagatgccgg acattggcga 3960

aggcatcgcg caggtcgaat tggtggaatg gttcgtcaag gtgggcgaca tcatcgccga 4020

ggaccaagtg gtagccgacg tcatgaccga caaggccacc gtggaaatcc cgtcgccggt 4080

cagcggcaag gtgctggccc tgggtggcca gccaggtgaa gtgatggcgg tcggcagtga 4140

gctgatccgc atcgaagtgg aaggcagcgg caaccatgtg gatgtgccgc aagccaagcc 4200

ggccgaagtg cctgcggcac cggtagccgc taaacctgaa ccacagaaag acgttaaacc 4260

ggcggcgtac caggcgtcag ccagccacga ggcagcgccc atcgtgccgc gccagccggg 4320

cgacaagccg ctggcctcgc cggcggtgcg caaacgcgcc ctcgatgccg gcatcgaatt 4380

gcgttatgtg cacggcagcg gcccggccgg gcgcatcctg cacgaagacc tcgacgcgtt 4440
catgagcaaa ccgcaaagcg ctgccgggca aaccccccaat ggctatgcca ggcgcaccga 4500
cagcgagcag gtgccggtga tcggcctgcg ccgcaagatc gcccagcgca tgcaggacgc 4560
caagcgccgg gtcgcgcact tcagctatgt ggaagaaatc gacgtcaccg ccctggaagc 4620
cctgcgccag cagctcaaca gcaagcacgg cgacagccgc ggcaagctga cactgctgcc 4680
gttcctggtg cgcgccctgg tcgtggcact gcgtgacttc ccgcagataa acgccaccta 4740
cgatgacgaa gcgcagatca tcacccgcca tggcgcggtg catgtgggca tcgccaccca 4800
aggtgacaac ggcctgatgg tacccgtgct gcgccacgcc gaagcgggca gcctgtgggc 4860
caatgccggt gagatttcac gcctggccaa cgctgcgcgc aacaacaagg ccagccgcga 4920
agagctgtcc ggttcgacca ttaccctgac cagcctcggc gccctgggcg gcatcgtcag 4980
cacgccggtg gtcaacaccc cggaagtggc gatcgtcggt gtcaaccgca tggttgagcg 5040
gcccgtggtg atcgacggcc agatcgtcgt gcgcaagatg atgaacctgt ccagctcgtt 5100
cgaccaccgc gtggtcgatg gcatggacgc cgccctgttc atccaggccg tgcgtggcct 5160
gctcgaacaa cccgcctgcc tgttcgtgga gtgagcatgc aacagactat ccagacaacc 5220
ctgttgatca tcggcggcgg ccctggcggc tatgtggcgg ccatccgcgc cgggcaactg 5280
ggcatcccta ccgtgctggt ggaaggccag gcgctgggcg gtacctgcct gaacatcggc 5340
tgcattccgt ccaaggcgct gatccatgtg gccgagcagt tccaccaggc ctcgcgcttt 5400
accgaaccct cgccgctggg catcagcgtg gcttcgccac gcctggacat cggccagagc 5460
gtggcctgga aagacggcat cgtcgatcgc ctgaccactg gtgtcgccgc cctgctgaaa 5520
aagcacgggg tgaaggtggt gcacggctgg gccaaggtgc ttgatggcaa gcaggtcgag 5580
gtggatggcc agcgcatcca gtgcgagcac ctgttgctgg ccacgggctc cagcagtgtc 5640
gaactgccga tgctgccgtt gggtgggccg gtgatttcct cgaccgaggc cctggcaccg 5700
aaagccctgc cgcaacacct ggtggtggtg ggcggtggct acatcggcct ggagctgggt 5760
atcgcctacc gcaagctcgg cgcgcaggtc agcgtggtgg aagcgcgcga gcgcatcctg 5820
ccgacttacg acagcgaact gaccgccccg gtggccgagt cgctgaaaaa gctgggtatc 5880
gccctgcacc ttggccacag cgtcgaaggt tacgaaaatg gctgcctgct ggccaacgat 5940
ggcaagggcg gacaactgcg cctggaagcc gaccgggtgc tggtggccgt gggccgccgc 6000
ccacgcacca agggcttcaa cctggaatgc ctggacctga agatgaatgg tgccgcgatt 6060
gccatcgacg agcgctgcca gaccagcatg cacaacgtct gggccatcgg cgacgtggcc 6120
ggcgaaccga tgctggcgca ccgggccatg gcccagggcg agatggtggc cgagatcatc 6180
gccggcaagg cacgccgctt cgaacccgct gcgatagccg ccgtgtgctt caccgacccg 6240
gaagtggtcg tggtcggcaa gacgccggaa caggccagtc agcaaggcct ggactgcatc 6300

gtcgcgcagt tcccgttcgc cgccaacggc cgggccatga gcctggagtc gaaaagcggt 6360

ttcgtgcgcg tggtcgcgcg gcgtgacaac cacctgatcc tgggctggca agcggttggc 6420

gtggcggttt ccgagctgtc cacggcgttt gcccagtcgc tggagatggg tgcctgcctg 6480

gaggatgtgg ccggtaccat ccatgcccac ccgaccctgg gtgaagcggt acaggaagcg 6540

gcactgcgtg ccctgggcca cgccctgcat atctgacact gaagcggccg aggccgattt 6600

ggcccgccgc gccgagaggc gctgcgggtc ttttttatac ctg 6643

<210> 57
<211> 423
<212> PRT
<213> Pseudomonas putida

<400> 57

```
Met Gly Thr His Val Ile Lys Met Pro Asp Ile Gly Glu Gly Ile Ala
1               5                   10                  15

Gln Val Glu Leu Val Glu Trp Phe Val Lys Val Gly Asp Ile Ile Ala
            20                  25                  30

Glu Asp Gln Val Val Ala Asp Val Met Thr Asp Lys Ala Thr Val Glu
        35                  40                  45

Ile Pro Ser Pro Val Ser Gly Lys Val Leu Ala Leu Gly Gly Gln Pro
    50                  55                  60

Gly Glu Val Met Ala Val Gly Ser Glu Leu Ile Arg Ile Glu Val Glu
65                  70                  75                  80

Gly Ser Gly Asn His Val Asp Val Pro Gln Ala Lys Pro Ala Glu Val
                85                  90                  95

Pro Ala Ala Pro Val Ala Ala Lys Pro Glu Pro Gln Lys Asp Val Lys
            100                 105                 110

Pro Ala Ala Tyr Gln Ala Ser Ala Ser His Glu Ala Ala Pro Ile Val
        115                 120                 125

Pro Arg Gln Pro Gly Asp Lys Pro Leu Ala Ser Pro Ala Val Arg Lys
    130                 135                 140

Arg Ala Leu Asp Ala Gly Ile Glu Leu Arg Tyr Val His Gly Ser Gly
145                 150                 155                 160

Pro Ala Gly Arg Ile Leu His Glu Asp Leu Asp Ala Phe Met Ser Lys
                165                 170                 175
```

```
Pro Gln Ser Ala Ala Gly Gln Thr Pro Asn Gly Tyr Ala Arg Arg Thr
            180                 185                 190

Asp Ser Glu Gln Val Pro Val Ile Gly Leu Arg Arg Lys Ile Ala Gln
        195                 200                 205

Arg Met Gln Asp Ala Lys Arg Arg Val Ala His Phe Ser Tyr Val Glu
    210                 215                 220

Glu Ile Asp Val Thr Ala Leu Glu Ala Leu Arg Gln Gln Leu Asn Ser
225                 230                 235                 240

Lys His Gly Asp Ser Arg Gly Lys Leu Thr Leu Leu Pro Phe Leu Val
                245                 250                 255

Arg Ala Leu Val Val Ala Leu Arg Asp Phe Pro Gln Ile Asn Ala Thr
            260                 265                 270

Tyr Asp Asp Glu Ala Gln Ile Ile Thr Arg His Gly Ala Val His Val
        275                 280                 285

Gly Ile Ala Thr Gln Gly Asp Asn Gly Leu Met Val Pro Val Leu Arg
    290                 295                 300

His Ala Glu Ala Gly Ser Leu Trp Ala Asn Ala Gly Glu Ile Ser Arg
305                 310                 315                 320

Leu Ala Asn Ala Ala Arg Asn Asn Lys Ala Ser Arg Glu Glu Leu Ser
                325                 330                 335

Gly Ser Thr Ile Thr Leu Thr Ser Leu Gly Ala Leu Gly Gly Ile Val
            340                 345                 350

Ser Thr Pro Val Val Asn Thr Pro Glu Val Ala Ile Val Gly Val Asn
        355                 360                 365

Arg Met Val Glu Arg Pro Val Val Ile Asp Gly Gln Ile Val Val Arg
    370                 375                 380

Lys Met Met Asn Leu Ser Ser Ser Phe Asp His Arg Val Val Asp Gly
385                 390                 395                 400

Met Asp Ala Ala Leu Phe Ile Gln Ala Val Arg Gly Leu Leu Glu Gln
                405                 410                 415

Pro Ala Cys Leu Phe Val Glu
```

420

<210> 58
<211> 6643
<212> DNA
<213> Pseudomonas putida

<400> 58

gcatgcctgc aggccgccga tgaaatggtg gaaggtatcg gtaggctggc cctgctcatc 60
gctgaacacg ttacgcccgc tgccggtatc gaccaggctc tggtgaatat gcatggaact 120
gccaggcgtg cgcgccagcg gtttggccat gcacaccacg gtcagcccgt gcttgagtgc 180
cacttccttg agcaggtgtt tgaacaggaa ggtctggtcg gccagcagca gcgggtcgcc 240
atgtagcaag ttgatctcga actggctgac gcccatttcg tgcatgaagg tgtcgcgcgg 300
caggccgagc gcggccatgc actggtacac ctcattgaag aacgggcgca ggccgttgtt 360
ggaactgaca ctgaacgccg aatggcccag ctcgcggcgg ccgtcggtgc ccagcggtgg 420
ctggaacggc tgctgcgggt cactgttggg ggcaaacacg aagaactcaa gctcggtcgc 480
cactaccggt gccagaccca acgctgcgta gcgggcgatc acggccttca gctggccccg 540
ggtggacagt gccgagggcc ggccatccag ttcattggca tcgcagatgg ccagggcgcg 600
accgtcatcg ctccagggca agcgatgaac ctggctgggt tccgctacca acgccaggtc 660
gccgtcgtcg cagccgtaga atttcgccgg cgggtagccg cccatgatgc attgcagcag 720
caccccacgg gccatctgca ggcggcggcc ttcgagaaag ccttcggcgg tcatcacctt 780
gccgcgtggg acgccgttga ggtcgggggt gacgcattcg atttcatcga tgccctggag 840
ctgagcgatg ctcatgacgc ttgtccttgt tgttgtaggc tgacaacaac ataggctggg 900
ggtgtttaaa atatcaagca gcctctcgaa cgcctggggc ctcttctatt cgcgcaaggt 960
catgccattg gccggcaacg gcaaggctgt cttgtagcgc acctgtttca aggcaaaact 1020
cgagcggata ttcgccacac ccggcaaccg ggtcaggtaa tcgagaaacc gctccagcgc 1080
ctggatactc ggcagcagta cccgcaacag gtagtccggg tcgcccgtca tcaggtagca 1140
ctccatcacc tcgggccgtt cggcaatttc ttcctcgaag cggtgcagcg actgctctac 1200
ctgtttttcc aggctgacat ggatgaacac attcacatcc agccccaacg cctcgggcga 1260
caacaaggtc acctgctggc ggatcacccc cagttcttcc atggcccgca cccggttgaa 1320
acagggcgtg ggcgacaggt tgaccgagcg tgccagctcg gcgttggtga tgcgggcgtt 1380
ttcctgcagg ctgttgagaa tgccgatatc ggtacgatcg agtttgcgca tgagacaaaa 1440
tcaccggttt tttgtgttta tgcggaatgt ttatctgccc cgctcggcaa aggcaatcaa 1500
cttgagagaa aaattctcct gccggaccac taagatgtag ggacgctgga cttaccagtc 1560
acaagccggt actcagcggc ggccgcttca gagctcacaa aaacaaatac ccgagcgagc 1620

gtaaaaagca tgaacgagta cgcccccctg cgtttgcatg tgcccgagcc caccggccgg 1680

ccaggctgcc agaccgattt ttcctacctg cgcctgaacg atgcaggtca agcccgtaaa 1740

ccccctgtcg atgtcgacgc tgccgacacc gccgacctgt cctacagcct ggtccgcgtg 1800

ctcgacgagc aaggcgacgc ccaaggcccg tgggctgaag acatcgaccc gcagatcctg 1860

cgccaaggca tgcgcgccat gctcaagacg cggatcttcg acagccgcat ggtggttgcc 1920

cagcgccaga agaagatgtc cttctacatg cagagcctgg gcgaagaagc catcggcagc 1980

ggccaggcgc tggcgcttaa ccgcaccgac atgtgcttcc ccacctaccg tcagcaaagc 2040

atcctgatgg cccgcgacgt gtcgctggtg gagatgatct gccagttgct gtccaacgaa 2100

cgcgaccccc tcaagggccg ccagctgccg atcatgtact cggtacgcga ggccggcttc 2160

ttcaccatca gcggcaacct ggcgacccag ttcgtgcagg cggtcggctg ggccatggcc 2220

tcggcgatca agggcgatac caagattgcc tcggcctgga tcggcgacgg cgccactgcc 2280

gaatcggact tccacaccgc cctcaccttt gcccacgttt accgcgcccc ggtgatcctc 2340

aacgtggtca acaaccagtg ggccatctca accttccagg ccatcgccgg tggcgagtcg 2400

accaccttcg ccggccgtgg cgtgggctgc ggcatcgctt cgctgcgggt ggacggcaac 2460

gacttcgtcg ccgtttacgc cgcttcgcgc tgggctgccg aacgtgcccg ccgtggtttg 2520

ggcccgagcc tgatcgagtg ggtcacctac cgtgccggcc cgcactcgac ctcggacgac 2580

ccgtccaagt accgccctgc cgatgactgg agccacttcc cgctgggtga cccgatcgcc 2640

cgcctgaagc agcacctgat caagatcggc cactggtccg aagaagaaca ccaggccacc 2700

acggccgagt tcgaagcggc cgtgattgct gcgcaaaaag aagccgagca gtacggcacc 2760

ctggccaacg gtcacatccc gagcgccgcc tcgatgttcg aggacgtgta caaggagatg 2820

cccgaccacc tgcgccgcca acgccaggaa ctgggggttt gagatgaacg accacaacaa 2880

cagcatcaac ccggaaaccg ccatggccac cactaccatg accatgatcc aggccctgcg 2940

ctcggccatg gatgtcatgc ttgagcgcga cgacaatgtg gtggtgtacg gccaggacgt 3000

cggctacttc ggcggcgtgt tccgctgcac cgaaggcctg cagaccaagt acggcaagtc 3060

ccgcgtgttc gacgcgccca tctctgaaag cggcatcgtc ggcaccgccg tgggcatggg 3120

tgcctacggc ctgcgcccgg tggtggaaat ccagttcgct gactacttct acccggcctc 3180

cgaccagatc gtttctgaaa tggcccgcct gcgctaccgt tcggccggcg agttcatcgc 3240

cccgctgacc ctgcgtatgc cctgcggtgg cggtatctat ggcggccaga cacacagcca 3300

gagcccggaa gcgatgttca ctcaggtgtg cggcctgcgc accgtaatgc catccaaccc 3360

gtacgacgcc aaaggcctgc tgattgcctc gatcgaatgc gacgacccgg tgatcttcct 3420

ggagcccaag cgcctgtaca acggcccgtt cgacggccac catgaccgcc cggttacgcc 3480

gtggtcgaaa cacccgcaca gcgccgtgcc cgatggctac tacaccgtgc cactggacaa 3540

ggccgccatc acccgccccg gcaatgacgt gagcgtgctc acctatggca ccaccgtgta 3600
cgtggcccag gtggccgccg aagaaagtgg cgtggatgcc gaagtgatcg acctgcgcag 3660
cctgtggccg ctagacctgg acaccatcgt cgagtcggtg aaaaagaccg gccgttgcgt 3720
ggtagtacac gaggccaccc gtacttgtgg ctttggcgca gaactggtgt cgctggtgca 3780
ggagcactgc ttccaccacc tggaggcgcc gatcgagcgc gtcaccggtt gggacacccc 3840
ctaccctcac gcgcaggaat gggcttactt cccagggcct tcgcgggtag gtgcggcatt 3900
gaaaaaggtc atggaggtct gaatgggcac gcacgtcatc aagatgccgg acattggcga 3960
aggcatcgcg caggtcgaat tggtggaatg gttcgtcaag gtgggcgaca tcatcgccga 4020
ggaccaagtg gtagccgacg tcatgaccga caaggccacc gtggaaatcc cgtcgccggt 4080
cagcggcaag gtgctggccc tgggtggcca gccaggtgaa gtgatggcgg tcggcagtga 4140
gctgatccgc atcgaagtgg aaggcagcgg caaccatgtg gatgtgccgc aagccaagcc 4200
ggccgaagtg cctgcggcac cggtagccgc taaacctgaa ccacagaaag acgttaaacc 4260
ggcggcgtac caggcgtcag ccagccacga ggcagcgccc atcgtgccgc gccagccggg 4320
cgacaagccg ctggcctcgc cggcggtgcg caaacgcgcc ctcgatgccg gcatcgaatt 4380
gcgttatgtg cacggcagcg gcccggccgg gcgcatcctg cacgaagacc tcgacgcgtt 4440
catgagcaaa ccgcaaagcg ctgccgggca aaccccccaat ggctatgcca ggcgcaccga 4500
cagcgagcag gtgccggtga tcggcctgcg ccgcaagatc gcccagcgca tgcaggacgc 4560
caagcgccgg gtcgcgcact tcagctatgt ggaagaaatc gacgtcaccg ccctggaagc 4620
cctgcgccag cagctcaaca gcaagcacgg cgacagccgc ggcaagctga cactgctgcc 4680
gttcctggtg cgcgccctgg tcgtggcact gcgtgacttc ccgcagataa acgccaccta 4740
cgatgacgaa gcgcagatca tcacccgcca tggcgcggtg catgtgggca tcgccaccca 4800
aggtgacaac ggcctgatgg tacccgtgct gcgccacgcc gaagcgggca gcctgtgggc 4860
caatgccggt gagatttcac gcctggccaa cgctgcgcgc aacaacaagg ccagccgcga 4920
agagctgtcc ggttcgacca ttaccctgac cagcctcggc gccctgggcg gcatcgtcag 4980
cacgccggtg gtcaacaccc cggaagtggc gatcgtcggt gtcaaccgca tggttgagcg 5040
gcccgtggtg atcgacggcc agatcgtcgt gcgcaagatg atgaacctgt ccagctcgtt 5100
cgaccaccgc gtggtcgatg gcatggacgc cgccctgttc atccaggccg tgcgtggcct 5160
gctcgaacaa cccgcctgcc tgttcgtgga gtgagcatgc aacagactat ccagacaacc 5220
ctgttgatca tcggcggcgg ccctggcggc tatgtggcgg ccatccgcgc cgggcaactg 5280
ggcatcccta ccgtgctggt ggaaggccag gcgctgggcg gtacctgcct gaacatcggc 5340
tgcattccgt ccaaggcgct gatccatgtg gccgagcagt tccaccaggc ctcgcgcttt 5400

```
accgaaccct cgccgctggg catcagcgtg gcttcgccac gcctggacat cggccagagc     5460
gtggcctgga aagacggcat cgtcgatcgc ctgaccactg gtgtcgccgc cctgctgaaa     5520
aagcacgggg tgaaggtggt gcacggctgg gccaaggtgc ttgatggcaa gcaggtcgag     5580
gtggatggcc agcgcatcca gtgcgagcac ctgttgctgg ccacgggctc cagcagtgtc     5640
gaactgccga tgctgccgtt gggtgggccg gtgatttcct cgaccgaggc cctggcaccg     5700
aaagccctgc cgcaacacct ggtggtggtg ggcggtggct acatcggcct ggagctgggt     5760
atcgcctacc gcaagctcgg cgcgcaggtc agcgtggtgg aagcgcgcga gcgcatcctg     5820
ccgacttacg acagcgaact gaccgccccg gtggccgagt cgctgaaaaa gctgggtatc     5880
gccctgcacc ttggccacag cgtcgaaggt tacgaaaatg gctgcctgct ggccaacgat     5940
ggcaagggcg gacaactgcg cctggaagcc gaccgggtgc tggtggccgt gggccgccgc     6000
ccacgcacca agggcttcaa cctggaatgc ctggacctga agatgaatgg tgccgcgatt     6060
gccatcgacg agcgctgcca gaccagcatg cacaacgtct gggccatcgg cgacgtggcc     6120
ggcgaaccga tgctggcgca ccgggccatg gcccagggcg agatggtggc cgagatcatc     6180
gccggcaagg cacgccgctt cgaacccgct gcgatagccg ccgtgtgctt caccgacccg     6240
gaagtggtcg tggtcggcaa gacgccggaa caggccagtc agcaaggcct ggactgcatc     6300
gtcgcgcagt tcccgttcgc cgccaacggc cgggccatga gcctggagtc gaaaagcggt     6360
ttcgtgcgcg tggtcgcgcg gcgtgacaac cacctgatcc tgggctggca agcggttggc     6420
gtggcggttt ccgagctgtc cacggcgttt gcccagtcgc tggagatggg tgcctgcctg     6480
gaggatgtgg ccggtaccat ccatgcccac ccgaccctgg gtgaagcggt acaggaagcg     6540
gcactgcgtg ccctgggcca cgccctgcat atctgacact gaagcggccg aggccgattt     6600
ggcccgccgc gccgagaggc gctgcgggtc ttttttatac ctg                       6643
```

<210> 59
<211> 459
<212> PRT
<213> Pseudomonas putida

<400> 59

Met Gln Gln Thr Ile Gln Thr Thr Leu Leu Ile Ile Gly Gly Gly Pro
1               5                   10                  15

Gly Gly Tyr Val Ala Ala Ile Arg Ala Gly Gln Leu Gly Ile Pro Thr
            20                  25                  30

Val Leu Val Glu Gly Gln Ala Leu Gly Gly Thr Cys Leu Asn Ile Gly
        35                  40                  45

Cys Ile Pro Ser Lys Ala Leu Ile His Val Ala Glu Gln Phe His Gln

50 55 60

Ala Ser Arg Phe Thr Glu Pro Ser Pro Leu Gly Ile Ser Val Ala Ser
65 70 75 80

Pro Arg Leu Asp Ile Gly Gln Ser Val Ala Trp Lys Asp Gly Ile Val
85 90 95

Asp Arg Leu Thr Thr Gly Val Ala Ala Leu Leu Lys Lys His Gly Val
100 105 110

Lys Val Val His Gly Trp Ala Lys Val Leu Asp Gly Lys Gln Val Glu
115 120 125

Val Asp Gly Gln Arg Ile Gln Cys Glu His Leu Leu Leu Ala Thr Gly
130 135 140

Ser Ser Ser Val Glu Leu Pro Met Leu Pro Leu Gly Gly Pro Val Ile
145 150 155 160

Ser Ser Thr Glu Ala Leu Ala Pro Lys Ala Leu Pro Gln His Leu Val
165 170 175

Val Val Gly Gly Gly Tyr Ile Gly Leu Glu Leu Gly Ile Ala Tyr Arg
180 185 190

Lys Leu Gly Ala Gln Val Ser Val Val Glu Ala Arg Glu Arg Ile Leu
195 200 205

Pro Thr Tyr Asp Ser Glu Leu Thr Ala Pro Val Ala Glu Ser Leu Lys
210 215 220

Lys Leu Gly Ile Ala Leu His Leu Gly His Ser Val Glu Gly Tyr Glu
225 230 235 240

Asn Gly Cys Leu Leu Ala Asn Asp Gly Lys Gly Gly Gln Leu Arg Leu
245 250 255

Glu Ala Asp Arg Val Leu Val Ala Val Gly Arg Arg Pro Arg Thr Lys
260 265 270

Gly Phe Asn Leu Glu Cys Leu Asp Leu Lys Met Asn Gly Ala Ala Ile
275 280 285

Ala Ile Asp Glu Arg Cys Gln Thr Ser Met His Asn Val Trp Ala Ile
290 295 300

Gly Asp Val Ala Gly Glu Pro Met Leu Ala His Arg Ala Met Ala Gln
305 310 315 320

Gly Glu Met Val Ala Glu Ile Ile Ala Gly Lys Ala Arg Arg Phe Glu
325 330 335

Pro Ala Ala Ile Ala Ala Val Cys Phe Thr Asp Pro Glu Val Val Val
340 345 350

Val Gly Lys Thr Pro Glu Gln Ala Ser Gln Gln Gly Leu Asp Cys Ile
355 360 365

Val Ala Gln Phe Pro Phe Ala Ala Asn Gly Arg Ala Met Ser Leu Glu
370 375 380

Ser Lys Ser Gly Phe Val Arg Val Val Ala Arg Arg Asp Asn His Leu
385 390 395 400

Ile Leu Gly Trp Gln Ala Val Gly Val Ala Val Ser Glu Leu Ser Thr
405 410 415

Ala Phe Ala Gln Ser Leu Glu Met Gly Ala Cys Leu Glu Asp Val Ala
420 425 430

Gly Thr Ile His Ala His Pro Thr Leu Gly Glu Ala Val Gln Glu Ala
435 440 445

Ala Leu Arg Ala Leu Gly His Ala Leu His Ile
450 455

<210> 60
<211> 6643
<212> DNA
<213> Pseudomonas putida

<400> 60

```
gcatgcctgc aggccgccga tgaaatggtg gaaggtatcg gtaggctggc cctgctcatc      60
gctgaacacg ttacgcccgc tgccggtatc gaccaggctc tggtgaatat gcatggaact     120
gccaggcgtg cgcgccagcg gtttggccat gcacaccacg gtcagcccgt gcttgagtgc     180
cacttccttg agcaggtgtt tgaacaggaa ggtctggtcg gccagcagca gcgggtcgcc     240
atgtagcaag ttgatctcga actggctgac gcccatttcg tgcatgaagg tgtcgcgcgg     300
caggccgagc gcggccatgc actggtacac ctcattgaag aacgggcgca ggccgttgtt     360
ggaactgaca ctgaacgccg aatggcccag ctcgcggcgg ccgtcggtgc ccagcggtgg     420
ctggaacggc tgctgcgggt cactgttggg ggcaaacacg aagaactcaa gctcggtcgc     480
cactaccggt gccagaccca acgctgcgta gcgggcgatc acggccttca gctggccccg     540
```

ggtggacagt gccgagggcc ggccatccag ttcattggca tcgcagatgg ccagggcgcg 600
accgtcatcg ctccagggca agcgatgaac ctggctgggt tccgctacca acgccaggtc 660
gccgtcgtcg cagccgtaga atttcgccgg cgggtagccg cccatgatgc attgcagcag 720
caccccacgg gccatctgca ggcggcggcc ttcgagaaag ccttcggcgg tcatcacctt 780
gccgcgtggg acgccgttga ggtcgggggt gacgcattcg atttcatcga tgccctggag 840
ctgagcgatg ctcatgacgc ttgtccttgt tgttgtaggc tgacaacaac ataggctggg 900
ggtgtttaaa atatcaagca gcctctcgaa cgcctggggc ctcttctatt cgcgcaaggt 960
catgccattg gccggcaacg gcaaggctgt cttgtagcgc acctgtttca aggcaaaact 1020
cgagcggata ttcgccacac ccggcaaccg ggtcaggtaa tcgagaaacc gctccagcgc 1080
ctggatactc ggcagcagta cccgcaacag gtagtccggg tcgcccgtca tcaggtagca 1140
ctccatcacc tcgggccgtt cggcaatttc ttcctcgaag cggtgcagcg actgctctac 1200
ctgtttttcc aggctgacat ggatgaacac attcacatcc agccccaacg cctcgggcga 1260
caacaaggtc acctgctggc ggatcacccc cagttcttcc atggcccgca cccggttgaa 1320
acagggcgtg ggcgacaggt tgaccgagcg tgccagctcg gcgttggtga tgcgggcgtt 1380
ttcctgcagg ctgttgagaa tgccgatatc ggtacgatcg agtttgcgca tgagacaaaa 1440
tcaccggttt tttgtgttta tgcggaatgt ttatctgccc cgctcggcaa aggcaatcaa 1500
cttgagagaa aaattctcct gccggaccac taagatgtag ggacgctga cttaccagtc 1560
acaagccggt actcagcggc ggccgcttca gagctcacaa aaacaaatac ccgagcgagc 1620
gtaaaaagca tgaacgagta cgcccccctg cgtttgcatg tgcccgagcc caccggccgg 1680
ccaggctgcc agaccgattt ttcctacctg cgcctgaacg atgcaggtca agcccgtaaa 1740
ccccctgtcg atgtcgacgc tgccgacacc gccgacctgt cctacagcct ggtccgcgtg 1800
ctcgacgagc aaggcgacgc ccaaggcccg tgggctgaag acatcgaccc gcagatcctg 1860
cgccaaggca tgcgcgccat gctcaagacg cggatcttcg acagccgcat ggtggttgcc 1920
cagcgccaga agaagatgtc cttctacatg cagagcctgg gcgaagaagc catcggcagc 1980
ggccaggcgc tggcgcttaa ccgcaccgac atgtgcttcc ccacctaccg tcagcaaagc 2040
atcctgatgg cccgcgacgt gtcgctggtg gagatgatct gccagttgct gtccaacgaa 2100
cgcgaccccc tcaagggccg ccagctgccg atcatgtact cggtacgcga ggccggcttc 2160
ttcaccatca gcggcaacct ggcgacccag ttcgtgcagg cggtcggctg ggccatggcc 2220
tcggcgatca agggcgatac caagattgcc tcggcctgga tcggcgacgg cgccactgcc 2280
gaatcggact tccacaccgc cctcaccttt gcccacgttt accgcgcccc ggtgatcctc 2340
aacgtggtca acaaccagtg ggccatctca accttccagg ccatcgccgg tggcgagtcg 2400

accaccttcg ccggccgtgg cgtgggctgc ggcatcgctt cgctgcgggt ggacggcaac 2460
gacttcgtcg ccgtttacgc cgcttcgcgc tgggctgccg aacgtgcccg ccgtggtttg 2520
ggcccgagcc tgatcgagtg ggtcacctac cgtgccggcc cgcactcgac ctcggacgac 2580
ccgtccaagt accgccctgc cgatgactgg agccacttcc cgctgggtga cccgatcgcc 2640
cgcctgaagc agcacctgat caagatcggc cactggtccg aagaagaaca ccaggccacc 2700
acggccgagt tcgaagcggc cgtgattgct gcgcaaaaag aagccgagca gtacggcacc 2760
ctggccaacg gtcacatccc gagcgccgcc tcgatgttcg aggacgtgta caaggagatg 2820
cccgaccacc tgcgccgcca acgccaggaa ctgggggttt gagatgaacg accacaacaa 2880
cagcatcaac ccggaaaccg ccatggccac cactaccatg accatgatcc aggccctgcg 2940
ctcggccatg gatgtcatgc ttgagcgcga cgacaatgtg gtggtgtacg gccaggacgt 3000
cggctacttc ggcggcgtgt tccgctgcac cgaaggcctg cagaccaagt acggcaagtc 3060
ccgcgtgttc gacgcgccca tctctgaaag cggcatcgtc ggcaccgccg tgggcatggg 3120
tgcctacggc ctgcgcccgg tggtggaaat ccagttcgct gactacttct acccggcctc 3180
cgaccagatc gtttctgaaa tggcccgcct gcgctaccgt tcggccggcg agttcatcgc 3240
cccgctgacc ctgcgtatgc cctgcggtgg cggtatctat ggcggccaga cacacagcca 3300
gagcccggaa gcgatgttca ctcaggtgtg cggcctgcgc accgtaatgc catccaaccc 3360
gtacgacgcc aaaggcctgc tgattgcctc gatcgaatgc gacgacccgg tgatcttcct 3420
ggagcccaag cgcctgtaca acggcccgtt cgacggccac catgaccgcc cggttacgcc 3480
gtggtcgaaa cacccgcaca gcgccgtgcc cgatggctac tacaccgtgc cactggacaa 3540
ggccgccatc acccgccccg gcaatgacgt gagcgtgctc acctatggca ccaccgtgta 3600
cgtggcccag gtggccgccg aagaaagtgg cgtggatgcc gaagtgatcg acctgcgcag 3660
cctgtggccg ctagacctgg acaccatcgt cgagtcggtg aaaaagaccg gccgttgcgt 3720
ggtagtacac gaggccaccc gtacttgtgg ctttggcgca gaactggtgt cgctggtgca 3780
ggagcactgc ttccaccacc tggaggcgcc gatcgagcgc gtcaccggtt gggacacccc 3840
ctaccctcac gcgcaggaat gggcttactt cccagggcct tcgcgggtag gtgcggcatt 3900
gaaaaaggtc atggaggtct gaatgggcac gcacgtcatc aagatgccgg acattggcga 3960
aggcatcgcg caggtcgaat tggtggaatg gttcgtcaag gtgggcgaca tcatcgccga 4020
ggaccaagtg gtagccgacg tcatgaccga caaggccacc gtggaaatcc cgtcgccggt 4080
cagcggcaag gtgctggccc tgggtggcca gccaggtgaa gtgatggcgg tcggcagtga 4140
gctgatccgc atcgaagtgg aaggcagcgg caaccatgtg gatgtgccgc aagccaagcc 4200
ggccgaagtg cctgcggcac cggtagccgc taaacctgaa ccacagaaag acgttaaacc 4260
ggcggcgtac caggcgtcag ccagccacga ggcagcgccc atcgtgccgc gccagccggg 4320

cgacaagccg ctggcctcgc cggcggtgcg caaacgcgcc ctcgatgccg gcatcgaatt 4380

gcgttatgtg cacggcagcg gcccggccgg gcgcatcctg cacgaagacc tcgacgcgtt 4440

catgagcaaa ccgcaaagcg ctgccgggca aaccccccaat ggctatgcca ggcgcaccga 4500

cagcgagcag gtgccggtga tcggcctgcg ccgcaagatc gcccagcgca tgcaggacgc 4560

caagcgccgg gtcgcgcact tcagctatgt ggaagaaatc gacgtcaccg ccctggaagc 4620

cctgcgccag cagctcaaca gcaagcacgg cgacagccgc ggcaagctga cactgctgcc 4680

gttcctggtg cgcgccctgg tcgtggcact gcgtgacttc ccgcagataa acgccaccta 4740

cgatgacgaa gcgcagatca tcacccgcca tggcgcggtg catgtgggca tcgccaccca 4800

aggtgacaac ggcctgatgg tacccgtgct gcgccacgcc gaagcgggca gcctgtgggc 4860

caatgccggt gagatttcac gcctggccaa cgctgcgcgc aacaacaagg ccagccgcga 4920

agagctgtcc ggttcgacca ttaccctgac cagcctcggc gccctgggcg gcatcgtcag 4980

cacgccggtg gtcaacaccc cggaagtggc gatcgtcggt gtcaaccgca tggttgagcg 5040

gcccgtggtg atcgacggcc agatcgtcgt gcgcaagatg atgaacctgt ccagctcgtt 5100

cgaccaccgc gtggtcgatg gcatggacgc cgccctgttc atccaggccg tgcgtggcct 5160

gctcgaacaa cccgcctgcc tgttcgtgga gtgagcatgc aacagactat ccagacaacc 5220

ctgttgatca tcggcggcgg ccctggcggc tatgtggcgg ccatccgcgc cgggcaactg 5280

ggcatcccta ccgtgctggt ggaaggccag gcgctgggcg gtacctgcct gaacatcggc 5340

tgcattccgt ccaaggcgct gatccatgtg gccgagcagt tccaccaggc ctcgcgcttt 5400

accgaaccct cgccgctggg catcagcgtg gcttcgccac gcctggacat cggccagagc 5460

gtggcctgga aagacggcat cgtcgatcgc ctgaccactg gtgtcgccgc cctgctgaaa 5520

aagcacgggg tgaaggtggt gcacggctgg gccaaggtgc ttgatggcaa gcaggtcgag 5580

gtggatggcc agcgcatcca gtgcgagcac ctgttgctgg ccacgggctc cagcagtgtc 5640

gaactgccga tgctgccgtt gggtgggccg gtgatttcct cgaccgaggc cctggcaccg 5700

aaagccctgc cgcaacacct ggtggtggtg ggcggtggct acatcggcct ggagctgggt 5760

atcgcctacc gcaagctcgg cgcgcaggtc agcgtggtgg aagcgcgcga gcgcatcctg 5820

ccgacttacg acagcgaact gaccgccccg gtggccgagt cgctgaaaaa gctgggtatc 5880

gccctgcacc ttggccacag cgtcgaaggt tacgaaaatg gctgcctgct ggccaacgat 5940

ggcaagggcg gacaactgcg cctggaagcc gaccgggtgc tggtggccgt gggccgccgc 6000

ccacgcacca agggcttcaa cctggaatgc ctggacctga agatgaatgg tgccgcgatt 6060

gccatcgacg agcgctgcca gaccagcatg cacaacgtct gggccatcgg cgacgtggcc 6120

ggcgaaccga tgctggcgca ccgggccatg gcccagggcg agatggtggc cgagatcatc 6180

```
gccggcaagg cacgccgctt cgaacccgct gcgatagccg ccgtgtgctt caccgacccg     6240

gaagtggtcg tggtcggcaa gacgccggaa caggccagtc agcaaggcct ggactgcatc     6300

gtcgcgcagt tcccgttcgc cgccaacggc cgggccatga gcctggagtc gaaaagcggt     6360

ttcgtgcgcg tggtcgcgcg gcgtgacaac cacctgatcc tgggctggca agcggttggc     6420

gtggcggttt ccgagctgtc cacggcgttt gcccagtcgc tggagatggg tgcctgcctg     6480

gaggatgtgg ccggtaccat ccatgcccac ccgaccctgg gtgaagcggt acaggaagcg     6540

gcactgcgtg ccctgggcca cgccctgcat atctgacact gaagcggccg aggccgattt     6600

ggcccgccgc gccgagaggc gctgcgggtc tttttatac ctg                        6643
```

<210> 61
<211> 468
<212> PRT
<213> Clostridium beijerinckii

<400> 61

```
Met Asn Lys Asp Thr Leu Ile Pro Thr Thr Lys Asp Leu Lys Leu Lys
1               5                   10                  15

Thr Asn Val Glu Asn Ile Asn Leu Lys Asn Tyr Lys Asp Asn Ser Ser
            20                  25                  30

Cys Phe Gly Val Phe Glu Asn Val Glu Asn Ala Ile Asn Ser Ala Val
        35                  40                  45

His Ala Gln Lys Ile Leu Ser Leu His Tyr Thr Lys Glu Gln Arg Glu
    50                  55                  60

Lys Ile Ile Thr Glu Ile Arg Lys Ala Ala Leu Glu Asn Lys Glu Val
65                  70                  75                  80

Leu Ala Thr Met Ile Leu Glu Glu Thr His Met Gly Arg Tyr Glu Asp
                85                  90                  95

Lys Ile Leu Lys His Glu Leu Val Ala Lys Tyr Thr Pro Gly Thr Glu
            100                 105                 110

Asp Leu Thr Thr Thr Ala Trp Ser Gly Asp Asn Gly Leu Thr Val Val
        115                 120                 125

Glu Met Ser Pro Tyr Gly Val Ile Gly Ala Ile Thr Pro Ser Thr Asn
    130                 135                 140

Pro Thr Glu Thr Val Ile Cys Asn Ser Ile Gly Met Ile Ala Ala Gly
145                 150                 155                 160
```

```
Asn Ala Val Val Phe Asn Gly His Pro Gly Ala Lys Lys Cys Val Ala
                165                 170                 175

Phe Ala Ile Glu Met Ile Asn Lys Ala Ile Ile Ser Cys Gly Gly Pro
            180                 185                 190

Glu Asn Leu Val Thr Thr Ile Lys Asn Pro Thr Met Glu Ser Leu Asp
        195                 200                 205

Ala Ile Ile Lys His Pro Leu Ile Lys Leu Leu Cys Gly Thr Gly Gly
    210                 215                 220

Pro Gly Met Val Lys Thr Leu Leu Asn Ser Gly Lys Lys Ala Ile Gly
225                 230                 235                 240

Ala Gly Ala Gly Asn Pro Pro Val Ile Val Asp Asp Thr Ala Asp Ile
                245                 250                 255

Glu Lys Ala Gly Lys Ser Ile Ile Glu Gly Cys Ser Phe Asp Asn Asn
            260                 265                 270

Leu Pro Cys Ile Ala Glu Lys Glu Val Phe Val Phe Glu Asn Val Ala
        275                 280                 285

Asp Asp Leu Ile Ser Asn Met Leu Lys Asn Asn Ala Val Ile Ile Asn
    290                 295                 300

Glu Asp Gln Val Ser Lys Leu Ile Asp Leu Val Leu Gln Lys Asn Asn
305                 310                 315                 320

Glu Thr Gln Glu Tyr Phe Ile Asn Lys Lys Trp Val Gly Lys Asp Ala
                325                 330                 335

Lys Leu Phe Ser Asp Glu Ile Asp Val Glu Ser Pro Ser Asn Ile Lys
            340                 345                 350

Cys Ile Val Cys Glu Val Asn Ala Asn His Pro Phe Val Met Thr Glu
        355                 360                 365

Leu Met Met Pro Ile Leu Pro Ile Val Arg Val Lys Asp Ile Asp Glu
    370                 375                 380

Ala Val Lys Tyr Thr Lys Ile Ala Glu Gln Asn Arg Lys His Ser Ala
385                 390                 395                 400

Tyr Ile Tyr Ser Lys Asn Ile Asp Asn Leu Asn Arg Phe Glu Arg Glu
                405                 410                 415
```

Ile Asp Thr Thr Ile Phe Val Lys Asn Ala Lys Ser Phe Ala Gly Val
420 425 430

Gly Tyr Glu Ala Glu Gly Phe Thr Thr Phe Thr Ile Ala Gly Ser Thr
435 440 445

Gly Glu Gly Ile Thr Ser Ala Arg Asn Phe Thr Arg Gln Arg Arg Cys
450 455 460

Val Leu Ala Gly
465

<210> 62
<211> 6558
<212> DNA
<213> Clostridium beijerinckii

<400> 62

```
aagcttaaaa tatccatagg ctattgttaa taagactata gcgcttaata ctctaagcgc      60
accatctaaa aaattataca taggaattgg ataaactcca atcttctcca taatactttt     120
cataggtgaa attgatatta taattaaggc tgcataaagc aaactcatat ctccaataaa     180
tgtcactatc ataggtaatt tccttttcat gttcacatac tcccccgtat tctataataa     240
tttacaataa acttccatca caaataaatt ataacatata ttgtaaatat agttttatta     300
ttcgcatatt tatagataaa caatatataa cttagactta tgcaataacc taccgaaagt     360
aaaaacattg ttatttcaca ggactatgaa aatttcgctg aaagtactaa attgcaggtt     420
gcaccactaa tgcttgctcc caatttcatt gtgacaagca ttagttgaac aacctacaat     480
taagagcctt taacagctca tttccaatgc ctgctacata aaaatgtttc tactttctaa     540
tatggtattt acttcaaagt gtaaatcaaa ttcttaagtt gtttatctat atagggttcc     600
atattgataa caatacatat ctactgtttc aatttcatga ataccagcga tattgaaatt     660
ttgtaagttt gaatatcatt gtgaaaccct atatatcaaa tacaatctca aaattataca     720
aaaagatccc aacttcacaa tatgaatttg agatcttcta tcttaacttc ttcaatattt     780
ttaagattat ttatactgcg tgcaaatctt ctataagtat caattatcag ctatgtacat     840
tgatagtgga cttgtaatct gaacagctta tatatttaca cttttaagtt ctcttccact     900
aacccctgct ttgaaacttc tcataaacaa atcgaaagta cctttaatta gctgtgcatt     960
ctcaaactca gaatttaact taagtacttc aattgccgct tcgatagtat agagctctcc    1020
ttctgaagca ccttttctta aagtatattc tgatttatta attggattta atgaaattct    1080
tggaagcttc tttaagtagt cgctctttct tagtatcttc tctgcttctt tccatgtgcc    1140
atctaagata ataaatgctg gaatttttttc tgaatcttta catttagact ttctttctag    1200
```

aggttcatca tcatccatag gaaataatac acgtatttca taatcatcgc tattaatata 1260
ttcaattaat ttttcaggag tctttactct ctcccaaaga attaactcag ttgattctgg 1320
attcaccaat ttcaataatc tagcggtatt tgaaggccta ctaaattctc tttctgttga 1380
taatatcaat atctttgctt ttgtctctat tttaggcaca atatcgcaga tacaatttat 1440
tattggcaac ccacatttat tgcagctctc atataactta gtaatttgct taactttaaa 1500
ttcagactcc attttacctc cattattagt tggttagtgt gtcatatctt cttgctatta 1560
ctaactgatt ataacatatg tattcaatat atcactccta gttttcaaag cactggcaat 1620
acgaattaca aattaatttc tggatttatg tcagtatttc attaataaaa ggtcggactt 1680
ttaagatact tgttttagct attgatcata tttattaaag actatgcatt taatgtataa 1740
ttataatgaa tattatcaat aatatttatt ttatattaca atcttacagt ctttattcta 1800
aatttcactc aaataccaaa cgagctttat tcataaacaa tatataacaa taattccaaa 1860
ataatacgat attttatctg taacagccat ataaaaaaaa tatcatatag tcttgtcatt 1920
tgataacgtt ttgtcttcct tatatttact ttttcggttt aataggttga ttctgtaaat 1980
tttagtgata acatatattt gatgacatta aaaatttaat atttcatata aatttttaat 2040
gtctattaat ttttaaatca caaggaggaa tagttcatga ataaagacac actaatacct 2100
acaactaaag atttaaaatt aaaaacaaat gttgaaaaca ttaatttaaa gaactacaag 2160
gataattctt catgtttcgg agtattcgaa aatgttgaaa atgctataaa cagcgctgta 2220
cacgcgcaaa agatattatc ccttcattat acaaaagaac aaagagaaaa aatcataact 2280
gagataagaa aggccgcatt agaaaataaa gaggttttag ctaccatgat tctggaagaa 2340
acacatatgg gaaggtatga agataaaata ttaaagcatg aattagtagc taaatatact 2400
cctggtacag aagatttaac tactactgct tggtcaggtg ataatggtct tacagttgta 2460
gaaatgtctc catatggcgt tataggtgca ataactcctt ctacgaatcc aactgaaact 2520
gtaatatgta atagcatcgg catgatagct gctggaaatg ctgtagtatt taacggacac 2580
ccaggcgcta aaaaatgtgt tgcttttgct attgaaatga taaataaagc aattatttca 2640
tgtggcggtc ctgagaattt agtaacaact ataaaaaatc caactatgga atccctagat 2700
gcaattatta agcatccttt aataaaactt ctttgcggaa ctggaggtcc aggaatggta 2760
aaaaccctct taaattctgg caagaaagct ataggtgctg gtgctggaaa tccaccagtt 2820
attgtagatg ataccgctga tatagaaaag gctggtaaga gtatcattga aggctgttct 2880
tttgataata atttaccttg tattgcagaa aaagaagtat ttgtttttga gaatgttgca 2940
gatgatttaa tatctaacat gctaaaaaat aatgctgtaa ttataaatga agatcaagta 3000
tcaaaattaa tagatttagt attacaaaaa aataatgaaa ctcaagaata ctttataaac 3060

aaaaaatggg taggaaaaga tgcaaaatta ttctcagatg aaatagatgt tgagtctcct 3120

tcaaatatta aatgcatagt ctgcgaagta aatgcaaatc atccatttgt catgacagaa 3180

ctcatgatgc caatattacc aattgtaaga gttaaagata tagatgaagc tgttaaatat 3240

acaaagatag cagaacaaaa tagaaaacat agtgcctata tttattctaa aaatatagac 3300

aacctaaata gatttgaaag agaaattgat actactattt ttgtaaagaa tgctaaatct 3360

tttgctggtg ttggttatga agctgaagga tttacaactt tcactattgc tggatctact 3420

ggtgaaggca taacctctgc aagaaatttt acaagacaaa gaagatgtgt acttgccggc 3480

taacttcttg ctaaatttat acatttattc acataacttt aatatgcaat gttcccacaa 3540

aatattaaaa actatttaga agggagatat taaatgaata aattagtaaa attaacagat 3600

ttaaagcgca ttttcaaaga tggtatgaca attatggttg gggggttttt agattgtgga 3660

actcctgaaa atattataga tatgctagtt gatttaaata taaaaaatct gactattata 3720

agcaatgata cagcttttcc taataaagga ataggaaaac ttattgtaaa tggtcaagtt 3780

tctaaagtaa ttgcttcaca tattggaact aatcctgaaa ctgggaaaaa aatgagctct 3840

ggtgaactta aagttgagct ttctccacaa ggaacactga tcgaaagaat tcgtgcagct 3900

ggatctggac tcggaggtgt attaactcca accggacttg ggactatcgt tgaagaaggt 3960

aagaaaaaag ttactatcgg tggcaaagaa tatctattag aacttccttt atccgctgat 4020

gtttcattaa taaaaggtag cattgtagat gaatttggaa ataccttcta tagagctgct 4080

actaaaaatt tcaatccata tatggcaatg gctgcaaaaa cagttatagt tgaagcagaa 4140

aatttagtta aatgtgaaga tttaaaaaga gatgccataa tgactcctgg cgtattagta 4200

gattatatcg ttaaggaggc ggcttaattg attgtagata aagttttagc aaaagagata 4260

attgccaaaa gagttgcaaa agaactaaaa aaaggccaac tcgtaaacct tggaatagga 4320

cttccaactt tagtagctaa ttatgtgcca aaagaaatga acattacttt cgaatcagaa 4380

aatggcatgg ttggcatggc acaaatggcc tcatcaggtg aaaatgaccc agatataata 4440

aatgctggtg gggaatatgt aacattatta cctcaaggtg catttttttga tagttcaacg 4500

tcttttgcac taataagagg aggacatgtt gatgttgctg ttcttggtgc tctagaagtt 4560

gatgaagaag gtaatttagc taactggatt gttccaaata aaattgtccc aggtatggga 4620

ggcgccatgg atttggcaat aggcgcaaaa aaaataatag tggcaatgca acatacagga 4680

aaaggtaaac ctaaaatcgt aaaaaaatgt actctcccac ttactgctaa ggctcaggta 4740

gatttaattg ttacagaact ttgtgtaatt gatgtaacaa atgatggttt acttttcaga 4800

gaaattcata aagatacaac tattgatgaa ataaaatttt taacagatgc agatttaatt 4860

attcccgaca acttaaaaat tatggatatc taaatcattc tattttaaat atataacttt 4920

aaaaatctta tgtattaaaa actaagaaaa gaggttgatt attttatgtt agaaagtgaa 4980

gtatctaaac aaattacaac tccacttgct gctccagcgt ttcctagagg accatataga 5040
tttcacaata gagaatatct aaacattatt tatcgaactg atttagatgc tcttcgaaaa 5100
atagtaccag agccacttga attagatgga gcatatgtta ggtttgagat gatggctatg 5160
cctgatacaa ccggactagg ctcatatact gagtgtggtc aagccattcc agtaaaatat 5220
aatgaggtta aaggtgacta cttgcatatg atgtacctag ataatgaacc tgctattgct 5280
gttggaagag aaagcagtgc ttatcccaaa aagttcggct atccaaagct atttgttgat 5340
tcagacgccc tagttggcgc ccttaagtat ggtgcattac cggtagttac tgcgacgatg 5400
ggatataagc atgagcccct agatcttaaa gaagcctata ctcaaattgc aagacccaat 5460
ttcatgctaa aaatcattca aggttatgat ggtaagccaa gaatttgtga actcatctgt 5520
gcagaaaata ctgatataac tatccacggt gcttggactg gaagtgcacg cctacaatta 5580
tttagccatg cactagctcc tcttgctgat ttacctgtat tagagatcgt atcagcatct 5640
catatcctaa cagatttaac tcttggaaca cctaaggttg tacatgatta tctttcagta 5700
aaataaaagc aatatagaat aaccactaca aaagtagtgg ttattctata ttttaaatca 5760
aactgtaaaa cttaagtttt atagtaccta ataatatttt actaccagca ttagattagt 5820
taaaatacaa agtttgtggt aaaagtattt tagattgcat aatagccttc tatactttta 5880
acaatataac caattgctca ccatctgctt agaatatgct tctttaagct ctaaaataca 5940
tataaaaaag taggaatttc ttattaaaat tcctacttat attatatata aatttaatcg 6000
ttaggtttta ttcgcattgt tcctctttaa tttatctctt ataacatttt attataattg 6060
ttcatataat taattcaata tactattata tattttcaag cattaataat tattcagcat 6120
ctgtcattac atatgcttcc atactttgac ttcttattaa atcatagcta atccatccat 6180
agccattgat tccccagtct ttaccccatg aatttattat ttttacagct tttttactat 6240
catcataacc aactacgcaa actgcatgac cacctctatt ttctccatca atctggtcat 6300
aaattggatt atcagaattt aaattatcaa aatctggata tactgatatt ccaataacta 6360
ctggatttcc agctgctatt tgtgccttta ttgcattata gtcaccatct ggaagttgac 6420
tccaactttt tgctttatat ttggctgcat tagccttttg ttcatctgta ggtgtaacct 6480
cccaactata ttcactacca tcataaggca tatcagataa tgtagtacaa ccttgttctt 6540
ctaataattt aaatgcat 6558

<210> 63
<211> 862
<212> PRT
<213> Clostridium acetobutylicum

<400> 63

```
Met Lys Val Thr Thr Val Lys Glu Leu Asp Glu Lys Leu Lys Val Ile
1               5                   10                  15

Lys Glu Ala Gln Lys Lys Phe Ser Cys Tyr Ser Gln Glu Met Val Asp
            20                  25                  30

Glu Ile Phe Arg Asn Ala Ala Met Ala Ala Ile Asp Ala Arg Ile Glu
        35                  40                  45

Leu Ala Lys Ala Ala Val Leu Glu Thr Gly Met Gly Leu Val Glu Asp
    50                  55                  60

Lys Val Ile Lys Asn His Phe Ala Gly Glu Tyr Ile Tyr Asn Lys Tyr
65                  70                  75                  80

Lys Asp Glu Lys Thr Cys Gly Ile Ile Glu Arg Asn Glu Pro Tyr Gly
                85                  90                  95

Ile Thr Lys Ile Ala Glu Pro Ile Gly Val Val Ala Ala Ile Ile Pro
            100                 105                 110

Val Thr Asn Pro Thr Ser Thr Thr Ile Phe Lys Ser Leu Ile Ser Leu
        115                 120                 125

Lys Thr Arg Asn Gly Ile Phe Phe Ser Pro His Pro Arg Ala Lys Lys
    130                 135                 140

Ser Thr Ile Leu Ala Ala Lys Thr Ile Leu Asp Ala Ala Val Lys Ser
145                 150                 155                 160

Gly Ala Pro Glu Asn Ile Ile Gly Trp Ile Asp Glu Pro Ser Ile Glu
                165                 170                 175

Leu Thr Gln Tyr Leu Met Gln Lys Ala Asp Ile Thr Leu Ala Thr Gly
            180                 185                 190

Gly Pro Ser Leu Val Lys Ser Ala Tyr Ser Ser Gly Lys Pro Ala Ile
        195                 200                 205

Gly Val Gly Pro Gly Asn Thr Pro Val Ile Ile Asp Glu Ser Ala His
    210                 215                 220

Ile Lys Met Ala Val Ser Ser Ile Ile Leu Ser Lys Thr Tyr Asp Asn
225                 230                 235                 240

Gly Val Ile Cys Ala Ser Glu Gln Ser Val Ile Val Leu Lys Ser Ile
                245                 250                 255
```

```
Tyr Asn Lys Val Lys Asp Glu Phe Gln Glu Arg Gly Ala Tyr Ile Ile
            260                 265                 270

Lys Lys Asn Glu Leu Asp Lys Val Arg Glu Val Ile Phe Lys Asp Gly
        275                 280                 285

Ser Val Asn Pro Lys Ile Val Gly Gln Ser Ala Tyr Thr Ile Ala Ala
    290                 295                 300

Met Ala Gly Ile Lys Val Pro Lys Thr Thr Arg Ile Leu Ile Gly Glu
305                 310                 315                 320

Val Thr Ser Leu Gly Glu Glu Glu Pro Phe Ala His Glu Lys Leu Ser
                325                 330                 335

Pro Val Leu Ala Met Tyr Glu Ala Asp Asn Phe Asp Asp Ala Leu Lys
            340                 345                 350

Lys Ala Val Thr Leu Ile Asn Leu Gly Gly Leu Gly His Thr Ser Gly
        355                 360                 365

Ile Tyr Ala Asp Glu Ile Lys Ala Arg Asp Lys Ile Asp Arg Phe Ser
    370                 375                 380

Ser Ala Met Lys Thr Val Arg Thr Phe Val Asn Ile Pro Thr Ser Gln
385                 390                 395                 400

Gly Ala Ser Gly Asp Leu Tyr Asn Phe Arg Ile Pro Pro Ser Phe Thr
                405                 410                 415

Leu Gly Cys Gly Phe Trp Gly Gly Asn Ser Val Ser Glu Asn Val Gly
            420                 425                 430

Pro Lys His Leu Leu Asn Ile Lys Thr Val Ala Glu Arg Arg Glu Asn
        435                 440                 445

Met Leu Trp Phe Arg Val Pro His Lys Val Tyr Phe Lys Phe Gly Cys
    450                 455                 460

Leu Gln Phe Ala Leu Lys Asp Leu Lys Asp Leu Lys Lys Lys Arg Ala
465                 470                 475                 480

Phe Ile Val Thr Asp Ser Asp Pro Tyr Asn Leu Asn Tyr Val Asp Ser
                485                 490                 495

Ile Ile Lys Ile Leu Glu His Leu Asp Ile Asp Phe Lys Val Phe Asn
            500                 505                 510
```

Lys Val Gly Arg Glu Ala Asp Leu Lys Thr Ile Lys Lys Ala Thr Glu
515 520 525

Glu Met Ser Ser Phe Met Pro Asp Thr Ile Ile Ala Leu Gly Gly Thr
530 535 540

Pro Glu Met Ser Ser Ala Lys Leu Met Trp Val Leu Tyr Glu His Pro
545 550 555 560

Glu Val Lys Phe Glu Asp Leu Ala Ile Lys Phe Met Asp Ile Arg Lys
565 570 575

Arg Ile Tyr Thr Phe Pro Lys Leu Gly Lys Lys Ala Met Leu Val Ala
580 585 590

Ile Thr Thr Ser Ala Gly Ser Gly Ser Glu Val Thr Pro Phe Ala Leu
595 600 605

Val Thr Asp Asn Asn Thr Gly Asn Lys Tyr Met Leu Ala Asp Tyr Glu
610 615 620

Met Thr Pro Asn Met Ala Ile Val Asp Ala Glu Leu Met Met Lys Met
625 630 635 640

Pro Lys Gly Leu Thr Ala Tyr Ser Gly Ile Asp Ala Leu Val Asn Ser
645 650 655

Ile Glu Ala Tyr Thr Ser Val Tyr Ala Ser Glu Tyr Thr Asn Gly Leu
660 665 670

Ala Leu Glu Ala Ile Arg Leu Ile Phe Lys Tyr Leu Pro Glu Ala Tyr
675 680 685

Lys Asn Gly Arg Thr Asn Glu Lys Ala Arg Glu Lys Met Ala His Ala
690 695 700

Ser Thr Met Ala Gly Met Ala Ser Ala Asn Ala Phe Leu Gly Leu Cys
705 710 715 720

His Ser Met Ala Ile Lys Leu Ser Ser Glu His Asn Ile Pro Ser Gly
725 730 735

Ile Ala Asn Ala Leu Leu Ile Glu Glu Val Ile Lys Phe Asn Ala Val
740 745 750

Asp Asn Pro Val Lys Gln Ala Pro Cys Pro Gln Tyr Lys Tyr Pro Asn

755 760 765

Thr Ile Phe Arg Tyr Ala Arg Ile Ala Asp Tyr Ile Lys Leu Gly Gly
770 775 780

Asn Thr Asp Glu Glu Lys Val Asp Leu Leu Ile Asn Lys Ile His Glu
785 790 795 800

Leu Lys Lys Ala Leu Asn Ile Pro Thr Ser Ile Lys Asp Ala Gly Val
805 810 815

Leu Glu Glu Asn Phe Tyr Ser Ser Leu Asp Arg Ile Ser Glu Leu Ala
820 825 830

Leu Asp Asp Gln Cys Thr Gly Ala Asn Pro Arg Phe Pro Leu Thr Ser
835 840 845

Glu Ile Lys Glu Met Tyr Ile Asn Cys Phe Lys Lys Gln Pro
850 855 860

<210> 64
<211> 1665
<212> DNA
<213> Clostridium acetobutylicum

<400> 64

```
ttgaagagtg aatacacaat tggaagatat ttgttagacc gtttatcaga gttgggtatt      60
cggcatatct ttggtgtacc tggagattac aatctatcct tttagacta tataatggag      120
tacaaaggga tagattgggt tggaaattgc aatgaattga atgctgggta tgctgctgat     180
ggatatgcaa gaataaatgg aattggagcc atacttacaa catttggtgt tggagaatta     240
agtgccatta acgcaattgc tggggcatac gctgagcaag ttccagttgt taaaattaca     300
ggtatcccca cagcaaaagt tagggacaat ggattatatg tacaccacac attaggtgac     360
ggaaggtttg atcacttttt tgaaatgttt agagaagtaa cagttgctga ggcattacta     420
agcgaagaaa atgcagcaca agaaattgat cgtgttctta tttcatgctg gagacaaaaa     480
cgtcctgttc ttataaattt accgattgat gtatatgata aaccaattaa caaaccatta     540
aagccattac tcgattatac tatttcaagt aacaaagagg ctgcatgtga atttgttaca     600
gaaatagtac ctataataaa tagggcaaaa aagcctgtta ttcttgcaga ttatggagta     660
tatcgttacc aagttcaaca tgtgcttaaa aacttggccg aaaaaaccgg atttcctgtg     720
gctacactaa gtatgggaaa aggtgttttc aatgaagcac accctcaatt tattggtgtt     780
tataatggtg atgtaagttc tccttattta aggcagcgag ttgatgaagc agactgcatt     840
attagcgttg gtgtaaaatt gacggattca accacagggg gattttctca tggattttct     900

aaaaggaatg taattcacat tgatcctttt tcaataaagg caaaaggtaa aaaatatgca     960
cctattacga tgaaagatgc tttaacagaa ttaacaagta aaattgagca tagaaacttt    1020
gaggatttag atataaagcc ttacaaatca gataatcaaa agtattttgc aaaagagaag    1080
ccaattacac aaaaacgttt ttttgagcgt attgctcact ttataaaaga aaaagatgta    1140
ttattagcag aacagggtac atgctttttt ggtgcgtcaa ccatacaact acccaaagat    1200
gcaactttta ttggtcaacc tttatgggga tctattggat acacacttcc tgctttatta    1260
ggttcacaat tagctgatca aaaaaggcgt aatattcttt taattgggga tggtgcattt    1320
caaatgacag cacaagaaat ttcaacaatg cttcgtttac aaatcaaacc tattattttt    1380
ttaattaata acgatggtta tacaattgaa cgtgctattc atggtagaga acaagtatat    1440
aacaatattc aaatgtggcg atatcataat gttccaaagg ttttaggtcc taaagaatgc    1500
agcttaacct ttaaagtaca aagtgaaact gaacttgaaa aggctctttt agtggcagat    1560
aaggattgtg aacatttgat ttttatagaa gttgttatgg atcgttatga taaacccgag    1620
cctttagaac gtctttcgaa acgttttgca aatcaaaata attag                    1665
```

<210> 65
<211> 858
<212> PRT
<213> Clostridium acetobutylicum

<400> 65

```
Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
```

```
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365
```

```
Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620
```

Leu Thr Pro Asn Met Ala Ile Ile Asp Thr Glu Leu Met Leu Asn Met
625 630 635 640

Pro Arg Lys Leu Thr Ala Ala Thr Gly Ile Asp Ala Leu Val His Ala
645 650 655

Ile Glu Ala Tyr Val Ser Val Met Ala Thr Asp Tyr Thr Asp Glu Leu
660 665 670

Ala Leu Arg Ala Ile Lys Met Ile Phe Lys Tyr Leu Pro Arg Ala Tyr
675 680 685

Lys Asn Gly Thr Asn Asp Ile Glu Ala Arg Glu Lys Met Ala His Ala
690 695 700

Ser Asn Ile Ala Gly Met Ala Phe Ala Asn Ala Phe Leu Gly Val Cys
705 710 715 720

His Ser Met Ala His Lys Leu Gly Ala Met His His Val Pro His Gly
725 730 735

Ile Ala Cys Ala Val Leu Ile Glu Glu Val Ile Lys Tyr Asn Ala Thr
740 745 750

Asp Cys Pro Thr Lys Gln Thr Ala Phe Pro Gln Tyr Lys Ser Pro Asn
755 760 765

Ala Lys Arg Lys Tyr Ala Glu Ile Ala Glu Tyr Leu Asn Leu Lys Gly
770 775 780

Thr Ser Asp Thr Glu Lys Val Thr Ala Leu Ile Glu Ala Ile Ser Lys
785 790 795 800

Leu Lys Ile Asp Leu Ser Ile Pro Gln Asn Ile Ser Ala Ala Gly Ile
805 810 815

Asn Lys Lys Asp Phe Tyr Asn Thr Leu Asp Lys Met Ser Glu Leu Ala
820 825 830

Phe Asp Asp Gln Cys Thr Thr Ala Asn Pro Arg Tyr Pro Leu Ile Ser
835 840 845

Glu Leu Lys Asp Ile Tyr Ile Lys Ser Phe
850 855

<210> 66
<211> 2589

<212> DNA
<213> Clostridium acetobutylicum

<400> 66

atgaaagtca caacagtaaa ggaattagat gaaaaactca aggtaattaa agaagctcaa 60
aaaaaattct cttgttactc gcaagaaatg gttgatgaaa tctttagaaa tgcagcaatg 120
gcagcaatcg acgcaaggat agagctagca aaagcagctg ttttggaaac cggtatgggc 180
ttagttgaag acaaggttat aaaaaatcat tttgcaggcg aatacatcta taacaaatat 240
aaggatgaaa aaacctgcgg tataattgaa cgaaatgaac cctacggaat tacaaaaata 300
gcagaaccta taggagttgt agctgctata atccctgtaa caaaccccac atcaacaaca 360
atatttaaat ccttaatatc ccttaaaact agaaatggaa ttttctttc gcctcaccca 420
agggcaaaaa aatccacaat actagcagct aaaacaatac ttgatgcagc cgttaagagt 480
ggtgccccgg aaaatataat aggttggata gatgaacctt caattgaact aactcaatat 540
ttaatgcaaa aagcagatat aacccttgca actggtggtc cctcactagt taaatctgct 600
tattcttccg gaaaaccagc aataggtgtt ggtccgggta acaccccagt aataattgat 660
gaatctgctc atataaaaat ggcagtaagt tcaattatat tatccaaaac ctatgataat 720
ggtgttatat gtgcttctga acaatctgta atagtcttaa aatccatata taacaaggta 780
aaagatgagt tccaagaaag aggagcttat ataataaaga aaaacgaatt ggataaagtc 840
cgtgaagtga tttttaaaga tggatccgta aaccctaaaa tagtcggaca gtcagcttat 900
actatagcag ctatggctgg cataaaagta cctaaaacca caagaatatt aataggagaa 960
gttacctcct taggtgaaga agaacctttt gcccacgaaa aactatctcc tgttttggct 1020
atgtatgagg ctgacaattt tgatgatgct ttaaaaaaag cagtaactct aataaactta 1080
ggaggcctcg gccatacctc aggaatatat gcagatgaaa taaaagcacg agataaaata 1140
gatagattta gtagtgccat gaaaaccgta agaacctttg taaatatccc aacctcacaa 1200
ggtgcaagtg gagatctata taattttaga ataccacctt ctttcacgct tggctgcgga 1260
ttttggggag gaaattctgt ttccgagaat gttggtccaa aacatctttt gaatattaaa 1320
accgtagctg aaaggagaga aaacatgctt tggtttagag ttccacataa agtatatttt 1380
aagttcggtt gtcttcaatt tgctttaaaa gatttaaaag atctaaagaa aaaaagagcc 1440
tttatagtta ctgatagtga cccctataat ttaaactatg ttgattcaat aataaaaata 1500
cttgagcacc tagatattga ttttaaagta tttaataagg ttggaagaga agctgatctt 1560
aaaaccataa aaaaagcaac tgaagaaatg tcctccttta tgccagacac tataatagct 1620
ttaggtggta cccctgaaat gagctctgca aagctaatgt gggtactata tgaacatcca 1680
gaagtaaaat ttgaagatct tgcaataaaa tttatggaca taagaaagag aatatatact 1740
ttcccaaaac tcggtaaaaa ggctatgtta gttgcaatta caacttctgc tggttccggt 1800

```
tctgaggtta ctccttttgc tttagtaact gacaataaca ctggaaataa gtacatgtta     1860
gcagattatg aaatgacacc aaatatggca attgtagatg cagaacttat gatgaaaatg     1920
ccaaagggat taaccgctta ttcaggtata gatgcactag taaatagtat agaagcatac     1980
acatccgtat atgcttcaga atacacaaac ggactagcac tagaggcaat acgattaata     2040
tttaaatatt tgcctgaggc ttacaaaaac ggaagaacca atgaaaaagc aagagagaaa     2100
atggctcacg cttcaactat ggcaggtatg gcatccgcta atgcatttct aggtctatgt     2160
cattccatgg caataaaatt aagttcagaa cacaatattc ctagtggcat tgccaatgca     2220
ttactaatag aagaagtaat aaaatttaac gcagttgata atcctgtaaa acaagcccct     2280
tgcccacaat ataagtatcc aaacaccata tttagatatg ctcgaattgc agattatata     2340
aagcttggag gaaatactga tgaggaaaag gtagatctct taattaacaa aatacatgaa     2400
ctaaaaaaag ctttaaatat accaacttca ataaaggatg caggtgtttt ggaggaaaac     2460
ttctattcct cccttgatag aatatctgaa cttgcactag atgatcaatg cacaggcgct     2520
aatcctagat ttcctcttac aagtgagata aaagaaatgt atataaattg ttttaaaaaa     2580
caaccttaa                                                             2589
```

<210> 67
<211> 307
<212> PRT
<213> Pseudomonas putida

<400> 67

```
Met Ser Lys Lys Leu Lys Ala Ala Ile Ile Gly Pro Gly Asn Ile Gly
1               5                   10                  15

Thr Asp Leu Val Met Lys Met Leu Arg Ser Glu Trp Ile Glu Pro Val
            20                  25                  30

Trp Met Val Gly Ile Asp Pro Asn Ser Asp Gly Leu Lys Arg Ala Arg
        35                  40                  45

Asp Phe Gly Met Lys Thr Thr Ala Glu Gly Val Asp Gly Leu Leu Pro
    50                  55                  60

His Val Leu Asp Asp Asp Ile Arg Ile Ala Phe Asp Ala Thr Ser Ala
65                  70                  75                  80

Tyr Val His Ala Glu Asn Ser Arg Lys Leu Asn Ala Leu Gly Val Leu
                85                  90                  95

Met Val Asp Leu Thr Pro Ala Ala Ile Gly Pro Tyr Cys Val Pro Pro
            100                 105                 110
```

```
Val Asn Leu Lys Gln His Val Gly Arg Leu Glu Met Asn Val Asn Met
        115                 120                 125

Val Thr Cys Gly Gly Gln Ala Thr Ile Pro Met Val Ala Ala Val Ser
    130                 135                 140

Arg Val Gln Pro Val Ala Tyr Ala Glu Ile Val Ala Thr Val Ser Ser
145                 150                 155                 160

Arg Ser Val Gly Pro Gly Thr Arg Lys Asn Ile Asp Glu Phe Thr Arg
                165                 170                 175

Thr Thr Ala Gly Ala Ile Glu Gln Val Gly Gly Ala Arg Glu Gly Lys
            180                 185                 190

Ala Ile Ile Val Ile Asn Pro Ala Glu Pro Pro Leu Met Met Arg Asp
        195                 200                 205

Thr Ile His Cys Leu Thr Asp Ser Glu Pro Asp Gln Ala Ala Ile Thr
    210                 215                 220

Ala Ser Val His Ala Met Ile Ala Glu Val Gln Lys Tyr Val Pro Gly
225                 230                 235                 240

Tyr Arg Leu Lys Asn Gly Pro Val Phe Asp Gly Asn Arg Val Ser Ile
                245                 250                 255

Phe Met Glu Val Glu Gly Leu Gly Asp Tyr Leu Pro Lys Tyr Ala Gly
            260                 265                 270

Asn Leu Asp Ile Met Thr Ala Ala Ala Leu Arg Thr Gly Glu Met Phe
        275                 280                 285

Ala Glu Glu Ile Ala Ala Gly Thr Ile Gln Leu Pro Arg Arg Asp Ile
    290                 295                 300

Ala Leu Ala
305
```

<210> 68
<211> 2180
<212> DNA
<213> Pseudomonas putida

<400> 68

ggtacccctg gagccggtca aggccggcga cttcatgcgc gtcgagatcg gcggcatcgg 60

cagcgcctcc gtgcgcttca cctgatcgaa cagaggacaa acccatgagc aagaaactca 120

aggcggccat cataggcccc ggcaatatcg gtaccgatct ggtgatgaag atgctccgtt 180

ccgagtggat tgagccggtg tggatggtcg gcatcgaccc caactccgac ggcctcaaac 240

gcgcccgcga tttcggcatg aagaccacag ccgaaggcgt cgacggcctg ctcccgcacg 300

tgctggacga cgacatccgc atcgccttcg acgccacctc ggcctatgtg catgccgaga 360

atagccgcaa gctcaacgcg cttggcgtgc tgatggtcga cctgaccccg gcggccatcg 420

gccccctactg cgtgccgccg gtcaacctca agcagcatgt cggccgcctg gaaatgaacg 480

tcaacatggt cacctgcggc ggccaggcca ccatccccat ggtcgccgcg gtgtcccgcg 540

tgcagccggt ggcctacgcc gagatcgtcg ccaccgtctc ctcgcgctcg gtcggcccgg 600

gcacgcgcaa gaacatcgac gagttcaccc gcaccaccgc cggcgccatc gagcaggtcg 660

gcggcgccag ggaaggcaag gcgatcatcg tcatcaaccc ggccgagccg ccgctgatga 720

tgcgcgacac catccactgc ctgaccgaca gcgagccgga ccaggctgcg atcaccgctt 780

cggttcacgc gatgatcgcc gaggtgcaga aatacgtgcc cggctaccgc ctgaagaacg 840

gcccggtgtt cgacggcaac cgcgtgtcga tcttcatgga agtcgaaggc ctgggcgact 900

acctgcccaa gtacgccggc aacctcgaca tcatgaccgc cgccgcgctg cgtaccggcg 960

agatgttcgc cgaggaaatc gccgccggca ccattcaact gccgcgtcgc gacatcgcgc 1020

tggcttgagg agtagcacca tgaatttgca cggcaagagc gtcatcctgc acgacatgag 1080

cctgcgcgac ggcatgcacg ccaagcgcca ccagatcagc ctggagcaga tggtcgcggt 1140

cgccaccggc ctcgatcaag ccggtatgcc gctgatcgag atcacccacg gcgacggcct 1200

cggcggtcgt tcgatcaact acggcttccc ggcccacagt gacgaggagt acctgcgcgc 1260

ggtgatcccg cagctcaagc aggccaaagt ctcggcgctg ctgctgcccg gcatcggcac 1320

cgtcgaccac ctgaagatgg ccctggactg cggcgtctcg actattcgcg tggccaccca 1380

ctgtaccgag gcggatgtct ccgagcagca catcggcatg gcgcgcaagc tgggggtcga 1440

caccgtcggc ttcctgatga tggcgcacat gatcagcgcc gagaaagtcc tggagcaggc 1500

caagctgatg gaaagctatg gtgccaactg catctactgc accgactcgg ccggctacat 1560

gctgcctgat gaagtcagcg agaaaatcgg cctcctgcgc gccgagctga acccggccac 1620

cgaagtcggc ttccacggcc accacaacat gggcatggct atcgccaact cgctggccgc 1680

catcgaagcc ggtgccgcgc gcatcgacgg ctcggtcgcc ggcctcggcg ccggtgccgg 1740

caacaccccg ctggaagtgt tcgtcgcagt gtgcaaacgc atgggcgtgg agaccggcat 1800

cgacctgtac aagatcatgg acgtggccga ggacctggtg gtgccgatga tggatcagcc 1860

gatccgcgtc gaccgcgacg ccctgaccct gggctacgcc ggggtgtaca gctcgttcct 1920

gctgttcgcc cagcgcgccg agaagaaata tggcgtgtcg gcccgcgaca tcctggtcga 1980

actgggccgg cgcggcaccg tcggtggcca ggaagacatg atcgaagacc tcgccctgga 2040

catggcccgg gcccgtcagc agcagaaggt gagcgcatga accgtaccct gacccgcgaa 2100

caggtgctgg ccctggccga gcacatcgaa aacgccgagc tgaatgtcca cgacatcggc 2160

aaggtgacca acgattttcc 2180

<210> 69
<211> 307
<212> PRT
<213> Thermus thermophilus

<400> 69

Met Ser Glu Arg Val Lys Val Ala Ile Leu Gly Ser Gly Asn Ile Gly
1 5 10 15

Thr Asp Leu Met Tyr Lys Leu Leu Lys Asn Pro Gly His Met Glu Leu
20 25 30

Val Ala Val Val Gly Ile Asp Pro Lys Ser Glu Gly Leu Ala Arg Ala
35 40 45

Arg Ala Leu Gly Leu Glu Ala Ser His Glu Gly Ile Ala Tyr Ile Leu
50 55 60

Glu Arg Pro Glu Ile Lys Ile Val Phe Asp Ala Thr Ser Ala Lys Ala
65 70 75 80

His Val Arg His Ala Lys Leu Leu Arg Glu Ala Gly Lys Ile Ala Ile
85 90 95

Asp Leu Thr Pro Ala Ala Arg Gly Pro Tyr Val Val Pro Pro Val Asn
100 105 110

Leu Lys Glu His Leu Asp Lys Asp Asn Val Asn Leu Ile Thr Cys Gly
115 120 125

Gly Gln Ala Thr Ile Pro Leu Val Tyr Ala Val His Arg Val Ala Pro
130 135 140

Val Leu Tyr Ala Glu Met Val Ser Thr Val Ala Ser Arg Ser Ala Gly
145 150 155 160

Pro Gly Thr Arg Gln Asn Ile Asp Glu Phe Thr Phe Thr Thr Ala Arg
165 170 175

Gly Leu Glu Ala Ile Gly Gly Ala Lys Lys Gly Lys Ala Ile Ile Ile
180 185 190

Leu Asn Pro Ala Glu Pro Pro Ile Leu Met Thr Asn Thr Val Arg Cys
195 200 205

Ile Pro Glu Asp Glu Gly Phe Asp Arg Glu Ala Val Val Ala Ser Val
210 215 220

Arg Ala Met Glu Arg Glu Val Gln Ala Tyr Val Pro Gly Tyr Arg Leu
225 230 235 240

Lys Ala Asp Pro Val Phe Glu Arg Leu Pro Thr Pro Trp Gly Glu Arg
245 250 255

Thr Val Val Ser Met Leu Leu Glu Val Glu Gly Ala Gly Asp Tyr Leu
260 265 270

Pro Lys Tyr Ala Gly Asn Leu Asp Ile Met Thr Ala Ser Ala Arg Arg
275 280 285

Val Gly Glu Val Phe Ala Gln His Leu Leu Gly Lys Pro Val Glu Glu
290 295 300

Val Val Ala
305

<210> 70
<211> 924
<212> DNA
<213> Thermus thermophilus

<400> 70

```
atgtccgaaa gggttaaggt agccatcctg ggctccggca acatcgggac ggacctgatg        60
tacaagctcc tgaagaaccc gggccacatg gagcttgtgg cggtggtggg gatagacccc       120
aagtccgagg gcctggcccg ggcgcgggcc ttagggttag aggcgagcca cgaagggatc       180
gcctacatcc tggagaggcc ggagatcaag atcgtctttg acgccaccag cgccaaggcc       240
cacgtgcgcc acgccaagct cctgagggag gcggggaaga tcgccataga cctcacgccg       300
gcggcccggg gcccttacgt ggtgcccccg gtgaacctga aggaacacct ggacaaggac       360
aacgtgaacc tcatcacctg cgggggggcag gccaccatcc ccctggtcta cgcggtgcac      420
cgggtggccc ccgtgctcta cgcggagatg gtctccacgg tggcctcccg ctccgcgggc       480
cccggcaccc ggcagaacat cgacgagttc accttcacca ccgcccgggg cctggaggcc       540
atcggggggg ccaagaaggg gaaggccatc atcatcctga acccggcgga accccccatc       600
ctcatgacca acaccgtgcg ctgcatcccc gaggacgagg gctttgaccg ggaggccgtg       660
gtggcgagcg tccgggccat ggagcgggag gtccaggcct acgtgcccgg ctaccgcctg       720
aaggcggacc cggtgtttga gaggcttccc accccctggg gggagcgcac cgtggtctcc       780
atgctcctgg aggtggaggg ggcgggggac tatttgccca aatacgccgg caacctggac       840
atcatgacgg cttctgcccg gagggtgggg gaggtcttcg cccagcacct cctggggaag       900
cccgtggagg aggtggtggc gtga                                              924
```

<210> 71
<211> 417
<212> PRT
<213> Escherichia coli

<400> 71

```
Met Thr Phe Ser Leu Phe Gly Asp Lys Phe Thr Arg His Ser Gly Ile
1               5                   10                  15

Thr Leu Leu Met Glu Asp Leu Asn Asp Gly Leu Arg Thr Pro Gly Ala
            20                  25                  30

Ile Met Leu Gly Gly Gly Asn Pro Ala Gln Ile Pro Glu Met Gln Asp
        35                  40                  45

Tyr Phe Gln Thr Leu Leu Thr Asp Met Leu Glu Ser Gly Lys Ala Thr
    50                  55                  60

Asp Ala Leu Cys Asn Tyr Asp Gly Pro Gln Gly Lys Thr Glu Leu Leu
65                  70                  75                  80

Thr Leu Leu Ala Gly Met Leu Arg Glu Lys Leu Gly Trp Asp Ile Glu
                85                  90                  95

Pro Gln Asn Ile Ala Leu Thr Asn Gly Ser Gln Ser Ala Phe Phe Tyr
            100                 105                 110

Leu Phe Asn Leu Phe Ala Gly Arg Arg Ala Asp Gly Arg Val Lys Lys
        115                 120                 125

Val Leu Phe Pro Leu Ala Pro Glu Tyr Ile Gly Tyr Ala Asp Ala Gly
    130                 135                 140

Leu Glu Glu Asp Leu Phe Val Ser Ala Arg Pro Asn Ile Glu Leu Leu
145                 150                 155                 160

Pro Glu Gly Gln Phe Lys Tyr His Val Asp Phe Glu His Leu His Ile
                165                 170                 175

Gly Glu Glu Thr Gly Met Ile Cys Val Ser Arg Pro Thr Asn Pro Thr
            180                 185                 190
```

Gly Asn Val Ile Thr Asp Glu Glu Leu Leu Lys Leu Asp Ala Leu Ala
195 200 205

Asn Gln His Gly Ile Pro Leu Val Ile Asp Asn Ala Tyr Gly Val Pro
210 215 220

Phe Pro Gly Ile Ile Phe Ser Glu Ala Arg Pro Leu Trp Asn Pro Asn
225 230 235 240

Ile Val Leu Cys Met Ser Leu Ser Lys Leu Gly Leu Pro Gly Ser Arg
245 250 255

Cys Gly Ile Ile Ile Ala Asn Glu Lys Ile Ile Thr Ala Ile Thr Asn
260 265 270

Met Asn Gly Ile Ile Ser Leu Ala Pro Gly Gly Ile Gly Pro Ala Met
275 280 285

Met Cys Glu Met Ile Lys Arg Asn Asp Leu Leu Arg Leu Ser Glu Thr
290 295 300

Val Ile Lys Pro Phe Tyr Tyr Gln Arg Val Gln Glu Thr Ile Ala Ile
305 310 315 320

Ile Arg Arg Tyr Leu Pro Glu Asn Arg Cys Leu Ile His Lys Pro Glu
325 330 335

Gly Ala Ile Phe Leu Trp Leu Trp Phe Lys Asp Leu Pro Ile Thr Thr
340 345 350

Lys Gln Leu Tyr Gln Arg Leu Lys Ala Arg Gly Val Leu Met Val Pro
355 360 365

Gly His Asn Phe Phe Pro Gly Leu Asp Lys Pro Trp Pro His Thr His
370 375 380

Gln Cys Met Arg Met Asn Tyr Val Pro Glu Pro Glu Lys Ile Glu Ala
385 390 395 400

Gly Val Lys Ile Leu Ala Glu Glu Ile Glu Arg Ala Trp Ala Glu Ser
405 410 415

His

<210> 72
<211> 417
<212> PRT

<213> Escherichia coli

<400> 72

```
Met Thr Phe Ser Leu Phe Gly Asp Lys Phe Thr Arg His Ser Gly Ile
1               5                   10                  15

Thr Leu Leu Met Glu Asp Leu Asn Asp Gly Leu Arg Thr Pro Gly Ala
            20                  25                  30

Ile Met Leu Gly Gly Gly Asn Pro Ala Gln Ile Pro Glu Met Gln Asp
        35                  40                  45

Tyr Phe Gln Thr Leu Leu Thr Asp Met Leu Glu Ser Gly Lys Ala Thr
    50                  55                  60

Asp Ala Leu Cys Asn Tyr Asp Gly Pro Gln Gly Lys Thr Glu Leu Leu
65                  70                  75                  80

Thr Leu Leu Ala Gly Met Leu Arg Glu Lys Leu Gly Trp Asp Ile Glu
                85                  90                  95

Pro Gln Asn Ile Ala Leu Thr Asn Gly Ser Gln Ser Ala Phe Phe Tyr
            100                 105                 110

Leu Phe Asn Leu Phe Ala Gly Arg Arg Ala Asp Gly Arg Val Lys Lys
        115                 120                 125

Val Leu Phe Pro Leu Ala Pro Glu Tyr Ile Gly Tyr Ala Asp Ala Gly
    130                 135                 140

Leu Glu Glu Asp Leu Phe Val Ser Ala Arg Pro Asn Ile Glu Leu Leu
145                 150                 155                 160

Pro Glu Gly Gln Phe Lys Tyr His Val Asp Phe Glu His Leu His Ile
                165                 170                 175

Gly Glu Glu Thr Gly Met Ile Cys Val Ser Arg Pro Thr Asn Pro Thr
            180                 185                 190

Gly Asn Val Ile Thr Asp Glu Glu Leu Leu Lys Leu Asp Ala Leu Ala
        195                 200                 205

Asn Gln His Gly Ile Pro Leu Val Ile Asp Asn Ala Tyr Gly Val Pro
    210                 215                 220

Phe Pro Gly Ile Ile Phe Ser Glu Ala Arg Pro Leu Trp Asn Pro Asn
225                 230                 235                 240
```

```
Ile Val Leu Cys Met Ser Leu Ser Lys Leu Gly Leu Pro Gly Ser Arg
                245                 250                 255

Cys Gly Ile Ile Ile Ala Asn Glu Lys Ile Ile Thr Ala Ile Thr Asn
            260                 265                 270

Met Asn Gly Ile Ile Ser Leu Ala Pro Gly Gly Ile Gly Pro Ala Met
        275                 280                 285

Met Cys Glu Met Ile Lys Arg Asn Asp Leu Leu Arg Leu Ser Glu Thr
    290                 295                 300

Val Ile Lys Pro Phe Tyr Tyr Gln Arg Val Gln Glu Thr Ile Ala Ile
305                 310                 315                 320

Ile Arg Arg Tyr Leu Pro Glu Asn Arg Cys Leu Ile His Lys Pro Glu
                325                 330                 335

Gly Ala Ile Phe Leu Trp Leu Trp Phe Lys Asp Leu Pro Ile Thr Thr
            340                 345                 350

Lys Gln Leu Tyr Gln Arg Leu Lys Ala Arg Gly Val Leu Met Val Pro
        355                 360                 365

Gly His Asn Phe Phe Pro Gly Leu Asp Lys Pro Trp Pro His Thr His
    370                 375                 380

Gln Cys Met Arg Met Asn Tyr Val Pro Glu Pro Glu Lys Ile Glu Ala
385                 390                 395                 400

Gly Val Lys Ile Leu Ala Glu Glu Ile Glu Arg Ala Trp Ala Glu Ser
                405                 410                 415

His
```

<210> 73
<211> 425
<212> PRT
<213> Bacillus licheniformis

<400> 73

```
Met Lys Pro Pro Leu Ser Lys Ile Gly Glu Lys Met Ile Glu Lys Thr
1               5                   10                  15

Gly Val Arg Ala Val Met Ser Asp Ile Gln Glu Val Leu Ala Gly Gly
            20                  25                  30
```

Glu Arg Ser Tyr Ile Asn Leu Ser Ala Gly Asn Pro Met Ile Leu Pro
35 40 45

Gly Val Ser Ala Met Trp Lys Ser Ala Leu Ala Asp Leu Leu Asp Asp
50 55 60

Asp Arg Phe Ser Ser Val Ile Gly Gln Tyr Gly Ser Ser Tyr Gly Thr
65 70 75 80

Asp Glu Leu Ile Ala Ser Val Val Arg Phe Phe Ser Glu Arg Tyr Ser
85 90 95

Ala Gly Ile Arg Lys Glu Asn Val Leu Ile Thr Ala Gly Ser Gln Gln
100 105 110

Leu Phe Phe Leu Ala Ile Asn Ser Phe Cys Gly Met Gly Ser Gly Ser
115 120 125

Val Met Lys Lys Ala Leu Ile Pro Met Leu Pro Asp Tyr Ser Gly Tyr
130 135 140

Ser Gly Ala Ala Leu Glu Arg Glu Met Ile Glu Gly Ile Pro Pro Leu
145 150 155 160

Ile Ser Lys Leu Asp Asp His Thr Phe Arg Tyr Glu Leu Asp Arg Lys
165 170 175

Gly Phe Leu Glu Arg Met Arg Ile Gly Ala Val Leu Leu Ser Arg Pro
180 185 190

Asn Asn Pro Cys Gly Asn Ile Leu Pro Lys Glu Asp Val Ala Phe Ile
195 200 205

Ser Asp Ala Cys Arg Glu Ala Asn Val Pro Leu Phe Ile Asp Ser Ala
210 215 220

Tyr Ala Pro Pro Phe Pro Ala Ile His Phe Ile Asp Met Glu Pro Ile
225 230 235 240

Phe Asn Glu Gln Ile Ile His Cys Met Ser Leu Ser Lys Ala Gly Leu
245 250 255

Pro Gly Glu Arg Ile Gly Ile Ala Ile Gly Pro Ser Arg Tyr Ile Gln
260 265 270

Ala Met Glu Ala Phe Gln Ser Asn Ala Ala Ile His Ser Ser Arg Leu

```
            275                 280                 285

Gly Gln Tyr Met Ala Ala Ser Val Leu Asn Asp Gly Arg Leu Ala Asp
    290                 295                 300

Val Ser Leu Asn Glu Val Arg Pro Tyr Tyr Arg Asn Lys Phe Met Leu
305                 310                 315                 320

Leu Lys Glu Thr Leu Leu Cys Lys Met Pro Glu Asp Ile Lys Trp Tyr
                325                 330                 335

Leu His Gln Gly Glu Gly Ser Leu Phe Gly Trp Leu Trp Phe Glu Asp
            340                 345                 350

Leu Pro Val Thr Asp Ala Ala Leu Tyr Glu Tyr Met Lys Ala Asp Gly
        355                 360                 365

Val Ile Ile Val Pro Gly Ser Ser Phe Phe His Arg Gln Ser Arg Arg
    370                 375                 380

Leu Ala His Ser His Gln Cys Ile Arg Ile Ser Leu Thr Ala Ala Asp
385                 390                 395                 400

Glu Asp Ile Ile Arg Gly Ile Asp Val Leu Ala Lys Ile Ala Lys Gly
                405                 410                 415

Val Tyr Glu Lys Gln Val Glu Tyr Leu
            420                 425
```

<210> 74
<211> 1278
<212> DNA
<213> Bacillus licheniformis

<400> 74

ttataagtat tcaacctgtt tctcatatac acccttcgca attttagcta aaacatcgat 60
tccccttata atatcttcat ccgccgcggt taggctgatt cgtatacact ggtgtgaatg 120
cgccaggcgc cgggattgac ggtgaaagaa agatgatccg ggaacgataa tgactccatc 180
cgctttcata tactcataca gcgctgcatc ggtcaccggc aggtcttcaa accacagcca 240
tccgaaaagc gatccttccc cttgatgcag ataccatttg atgtcttcag gcatcttgca 300
taaaagcgtt tccttgagca gcatgaattt attgcggtaa tatggcctga cttcattcag 360
cgacacgtcg gcgaggcgcc cgtcattcaa tactgatgca gccatatact gccccagcct 420
tgaagaatgg atcgccgcat tcgactgaaa agcttccatt gcctgaatat accgggacgg 480
cccgatggcg attccgatcc tttcgccagg caggccggct tttgaaaggc tcatacagtg 540

aatgatctgc tcgttgaaaa tcggttccat gtcgataaag tgaatcgccg gaaaaggcgg 600
agcatatgcg gaatcaatga acagcggaac attcgcttct cggcatgcgt ctgaaatgaa 660
tgctacatct tctttaggca agatgtttcc gcaaggattg ttcgggcgcg atagcaagac 720
agcaccgatg cgcatcctct ctaaaaaccc cttacggtcg agctcatatc gaaacgtatg 780
atcatccaat ttcgatatga gcggagggat cccctcaatc atctcccgct ccagtgccgc 840
cccgctgtat cccgaatagt caggcagcat cgggatcaag gctttttca tcacagatcc 900
gcttcccatt ccgcaaaacg aattgatcgc cagaaaaaac agctgctggc ttccggctgt 960
aatcaacacg ttctcttttc gaatgccggc gctataccgc tctgaaaaga agcggacaac 1020
acttgcaatc agttcatcgg ttccatagct cgatccgtat tggccgatca ccgaagaaaa 1080
cctgtcatcg tcaaggagat cggcaagagc cgacttccac atggctgaca cgccgggcaa 1140
aatcatcgga ttgcccgcac ttaaattaat gtatgaccgt tcaccgccgg ccaggacttc 1200
ctgaatatcg ctcatcacag ccctgacccc tgttttctca atcattttct ctccgatttt 1260
gcttaatggc ggcttcac 1278

<210> 75
<211> 309
<212> PRT
<213> Escherichia coli

<400> 75

```
Met Thr Thr Lys Lys Ala Asp Tyr Ile Trp Phe Asn Gly Glu Met Val
1               5                   10                  15

Arg Trp Glu Asp Ala Lys Val His Val Met Ser His Ala Leu His Tyr
            20                  25                  30

Gly Thr Ser Val Phe Glu Gly Ile Arg Cys Tyr Asp Ser His Lys Gly
        35                  40                  45

Pro Val Val Phe Arg His Arg Glu His Met Gln Arg Leu His Asp Ser
    50                  55                  60

Ala Lys Ile Tyr Arg Phe Pro Val Ser Gln Ser Ile Asp Glu Leu Met
65                  70                  75                  80

Glu Ala Cys Arg Asp Val Ile Arg Lys Asn Asn Leu Thr Ser Ala Tyr
                85                  90                  95

Ile Arg Pro Leu Ile Phe Val Gly Asp Val Gly Met Gly Val Asn Pro
            100                 105                 110

Pro Ala Gly Tyr Ser Thr Asp Val Ile Ile Ala Ala Phe Pro Trp Gly
```

115 120 125

Ala Tyr Leu Gly Ala Glu Ala Leu Glu Gln Gly Ile Asp Ala Met Val
130 135 140

Ser Ser Trp Asn Arg Ala Ala Pro Asn Thr Ile Pro Thr Ala Ala Lys
145 150 155 160

Ala Gly Gly Asn Tyr Leu Ser Ser Leu Leu Val Gly Ser Glu Ala Arg
165 170 175

Arg His Gly Tyr Gln Glu Gly Ile Ala Leu Asp Val Asn Gly Tyr Ile
180 185 190

Ser Glu Gly Ala Gly Glu Asn Leu Phe Glu Val Lys Asp Gly Val Leu
195 200 205

Phe Thr Pro Pro Phe Thr Ser Ser Ala Leu Pro Gly Ile Thr Arg Asp
210 215 220

Ala Ile Ile Lys Leu Ala Lys Glu Leu Gly Ile Glu Val Arg Glu Gln
225 230 235 240

Val Leu Ser Arg Glu Ser Leu Tyr Leu Ala Asp Glu Val Phe Met Ser
245 250 255

Gly Thr Ala Ala Glu Ile Thr Pro Val Arg Ser Val Asp Gly Ile Gln
260 265 270

Val Gly Glu Gly Arg Cys Gly Pro Val Thr Lys Arg Ile Gln Gln Ala
275 280 285

Phe Phe Gly Leu Phe Thr Gly Glu Thr Glu Asp Lys Trp Gly Trp Leu
290 295 300

Asp Gln Val Asn Gln
305

<210> 76
<211> 1476
<212> DNA
<213> Escherichia coli

<400> 76

```
atggctaact acttcaatac actgaatctg cgccagcagc tggcacagct gggcaaatgt      60
cgctttatgg gccgcgatga attcgccgat ggcgcgagct accttcaggg taaaaaagta     120
gtcatcgtcg gctgtggcgc acagggtctg aaccagggcc tgaacatgcg tgattctggt     180
ctcgatatct cctacgctct gcgtaaagaa gcgattgccg agaagcgcgc gtcctggcgt     240
aaagcgaccg aaaatggttt taaagtgggt acttacgaag aactgatccc acaggcggat     300
ctggtgatta acctgacgcc ggacaagcag cactctgatg tagtgcgcac cgtacagcca     360
ctgatgaaag acggcgcggc gctgggctac tcgcacggtt tcaacatcgt cgaagtgggc     420
gagcagatcc gtaaagatat caccgtagtg atggttgcgc cgaaatgccc aggcaccgaa     480
gtgcgtgaag agtacaaacg tgggttcggc gtaccgacgc tgattgccgt tcacccggaa     540
aacgatccga aaggcgaagg catggcgatt gccaaagcct gggcggctgc aaccggtggt     600
caccgtgcgg gtgtgctgga atcgtccttc gttgcggaag tgaaatctga cctgatgggc     660
gagcaaacca tcctgtgcgg tatgttgcag gctggctctc tgctgtgctt cgacaagctg     720
gtggaagaag gtaccgatcc agcatacgca gaaaaactga ttcagttcgg ttgggaaacc     780
atcaccgaag cactgaaaca gggcggcatc accctgatga tggaccgtct ctctaacccg     840
gcgaaactgc gtgcttatgc gctttctgaa cagctgaaag agatcatggc acccctgttc     900
cagaaacata tggacgacat catctccggc gaattctctt ccggtatgat ggcggactgg     960
gccaacgatg ataagaaact gctgacctgg cgtgaagaga ccggcaaaac cgcgtttgaa    1020
accgcgccgc agtatgaagg caaaatcggc gagcaggagt acttcgataa aggcgtactg    1080
atgattgcga tggtgaaagc gggcgttgaa ctggcgttcg aaaccatggt cgattccggc    1140
atcattgaag agtctgcata ttatgaatca ctgcacgagc tgccgctgat tgccaacacc    1200
atcgcccgta agcgtctgta cgaaatgaac gtggttatct ctgataccgc tgagtacggt    1260
aactatctgt tctcttacgc ttgtgtgccg ttgctgaaac cgtttatggc agagctgcaa    1320
ccgggcgacc tgggtaaagc tattccggaa ggcgcggtag ataacgggca actgcgtgat    1380
gtgaacgaag cgattcgcag ccatgcgatt gagcaggtag gtaagaaact gcgcggctat    1440
atgacagata tgaaacgtat tgctgttgcg ggttaa                              1476
```

<210> 77
<211> 376
<212> PRT
<213> Saccharomyces cerevisiae

<400> 77

```
Met Thr Leu Ala Pro Leu Asp Ala Ser Lys Val Lys Ile Thr Thr Thr
1               5                   10                  15

Gln His Ala Ser Lys Pro Lys Pro Asn Ser Glu Leu Val Phe Gly Lys
            20                  25                  30

Ser Phe Thr Asp His Met Leu Thr Ala Glu Trp Thr Ala Glu Lys Gly
        35                  40                  45
```

```
Trp Gly Thr Pro Glu Ile Lys Pro Tyr Gln Asn Leu Ser Leu Asp Pro
    50                  55                  60

Ser Ala Val Val Phe His Tyr Ala Phe Glu Leu Phe Glu Gly Met Lys
65                  70                  75                  80

Ala Tyr Arg Thr Val Asp Asn Lys Ile Thr Met Phe Arg Pro Asp Met
                85                  90                  95

Asn Met Lys Arg Met Asn Lys Ser Ala Gln Arg Ile Cys Leu Pro Thr
            100                 105                 110

Phe Asp Pro Glu Glu Leu Ile Thr Leu Ile Gly Lys Leu Ile Gln Gln
        115                 120                 125

Asp Lys Cys Leu Val Pro Glu Gly Lys Gly Tyr Ser Leu Tyr Ile Arg
    130                 135                 140

Pro Thr Leu Ile Gly Thr Thr Ala Gly Leu Gly Val Ser Thr Pro Asp
145                 150                 155                 160

Arg Ala Leu Leu Tyr Val Ile Cys Cys Pro Val Gly Pro Tyr Tyr Lys
                165                 170                 175

Thr Gly Phe Lys Ala Val Arg Leu Glu Ala Thr Asp Tyr Ala Thr Arg
            180                 185                 190

Ala Trp Pro Gly Gly Cys Gly Asp Lys Lys Leu Gly Ala Asn Tyr Ala
        195                 200                 205

Pro Cys Val Leu Pro Gln Leu Gln Ala Ala Ser Arg Gly Tyr Gln Gln
    210                 215                 220

Asn Leu Trp Leu Phe Gly Pro Asn Asn Asn Ile Thr Glu Val Gly Thr
225                 230                 235                 240

Met Asn Ala Phe Phe Val Phe Lys Asp Ser Lys Thr Gly Lys Lys Glu
                245                 250                 255

Leu Val Thr Ala Pro Leu Asp Gly Thr Ile Leu Glu Gly Val Thr Arg
            260                 265                 270

Asp Ser Ile Leu Asn Leu Ala Lys Glu Arg Leu Glu Pro Ser Glu Trp
        275                 280                 285

Thr Ile Ser Glu Arg Tyr Phe Thr Ile Gly Glu Val Thr Glu Arg Ser
    290                 295                 300
```

Lys Asn Gly Glu Leu Leu Glu Ala Phe Gly Ser Gly Thr Ala Ala Ile
305 310 315 320

Val Ser Pro Ile Lys Glu Ile Gly Trp Lys Gly Glu Gln Ile Asn Ile
325 330 335

Pro Leu Leu Pro Gly Glu Gln Thr Gly Pro Leu Ala Lys Glu Val Ala
340 345 350

Gln Trp Ile Asn Gly Ile Gln Tyr Gly Glu Thr Glu His Gly Asn Trp
355 360 365

Ser Arg Val Val Thr Asp Leu Asn
370 375

<210> 78
<211> 376
<212> PRT
<213> Saccharomyces cerevisiae

<400> 78

Met Thr Leu Ala Pro Leu Asp Ala Ser Lys Val Lys Ile Thr Thr Thr
1 5 10 15

Gln His Ala Ser Lys Pro Lys Pro Asn Ser Glu Leu Val Phe Gly Lys
20 25 30

Ser Phe Thr Asp His Met Leu Thr Ala Glu Trp Thr Ala Glu Lys Gly
35 40 45

Trp Gly Thr Pro Glu Ile Lys Pro Tyr Gln Asn Leu Ser Leu Asp Pro
50 55 60

Ser Ala Val Val Phe His Tyr Ala Phe Glu Leu Phe Glu Gly Met Lys
65 70 75 80

Ala Tyr Arg Thr Val Asp Asn Lys Ile Thr Met Phe Arg Pro Asp Met
85 90 95

Asn Met Lys Arg Met Asn Lys Ser Ala Gln Arg Ile Cys Leu Pro Thr
100 105 110

Phe Asp Pro Glu Glu Leu Ile Thr Leu Ile Gly Lys Leu Ile Gln Gln
115 120 125

Asp Lys Cys Leu Val Pro Glu Gly Lys Gly Tyr Ser Leu Tyr Ile Arg
130 135 140

Pro Thr Leu Ile Gly Thr Thr Ala Gly Leu Gly Val Ser Thr Pro Asp
145 150 155 160

Arg Ala Leu Leu Tyr Val Ile Cys Cys Pro Val Gly Pro Tyr Tyr Lys
165 170 175

Thr Gly Phe Lys Ala Val Arg Leu Glu Ala Thr Asp Tyr Ala Thr Arg
180 185 190

Ala Trp Pro Gly Gly Cys Gly Asp Lys Lys Leu Gly Ala Asn Tyr Ala
195 200 205

Pro Cys Val Leu Pro Gln Leu Gln Ala Ala Ser Arg Gly Tyr Gln Gln
210 215 220

Asn Leu Trp Leu Phe Gly Pro Asn Asn Asn Ile Thr Glu Val Gly Thr
225 230 235 240

Met Asn Ala Phe Phe Val Phe Lys Asp Ser Lys Thr Gly Lys Lys Glu
245 250 255

Leu Val Thr Ala Pro Leu Asp Gly Thr Ile Leu Glu Gly Val Thr Arg
260 265 270

Asp Ser Ile Leu Asn Leu Ala Lys Glu Arg Leu Glu Pro Ser Glu Trp
275 280 285

Thr Ile Ser Glu Arg Tyr Phe Thr Ile Gly Glu Val Thr Glu Arg Ser
290 295 300

Lys Asn Gly Glu Leu Leu Glu Ala Phe Gly Ser Gly Thr Ala Ala Ile
305 310 315 320

Val Ser Pro Ile Lys Glu Ile Gly Trp Lys Gly Glu Gln Ile Asn Ile
325 330 335

Pro Leu Leu Pro Gly Glu Gln Thr Gly Pro Leu Ala Lys Glu Val Ala
340 345 350

Gln Trp Ile Asn Gly Ile Gln Tyr Gly Glu Thr Glu His Gly Asn Trp
355 360 365

Ser Arg Val Val Thr Asp Leu Asn
370 375

<210> 79
<211> 330

<212> PRT
<213> Methanobacterium thermoautotrophicum

<400> 79

```
Met Arg Leu Trp Arg Ala Leu Tyr Arg Pro Pro Thr Ile Thr Tyr Pro
1               5                   10                  15

Ser Lys Ser Pro Glu Val Ile Ile Met Ser Cys Glu Ala Ser Gly Lys
            20                  25                  30

Ile Trp Leu Asn Gly Glu Met Val Glu Trp Glu Glu Ala Thr Val His
        35                  40                  45

Val Leu Ser His Val Val His Tyr Gly Ser Ser Val Phe Glu Gly Ile
    50                  55                  60

Arg Cys Tyr Arg Asn Ser Lys Gly Ser Ala Ile Phe Arg Leu Arg Glu
65                  70                  75                  80

His Val Lys Arg Leu Phe Asp Ser Ala Lys Ile Tyr Arg Met Asp Ile
                85                  90                  95

Pro Tyr Thr Gln Glu Gln Ile Cys Asp Ala Ile Val Glu Thr Val Arg
            100                 105                 110

Glu Asn Gly Leu Glu Glu Cys Tyr Ile Arg Pro Val Val Phe Arg Gly
        115                 120                 125

Tyr Gly Glu Met Gly Val His Pro Val Asn Cys Pro Val Asp Val Ala
    130                 135                 140

Val Ala Ala Trp Glu Trp Gly Ala Tyr Leu Gly Ala Glu Ala Leu Glu
145                 150                 155                 160

Val Gly Val Asp Ala Gly Val Ser Thr Trp Arg Arg Met Ala Pro Asn
                165                 170                 175

Thr Met Pro Asn Met Ala Lys Ala Gly Gly Asn Tyr Leu Asn Ser Gln
            180                 185                 190

Leu Ala Lys Met Glu Ala Val Arg His Gly Tyr Asp Glu Ala Ile Met
        195                 200                 205

Leu Asp Tyr His Gly Tyr Ile Ser Glu Gly Ser Gly Glu Asn Ile Phe
    210                 215                 220

Leu Val Ser Glu Gly Glu Ile Tyr Thr Pro Pro Val Ser Ser Ser Leu
```

```
225                 230                 235                 240

Leu Arg Gly Ile Thr Arg Asp Ser Val Ile Lys Ile Ala Arg Thr Glu
                245                 250                 255

Gly Val Thr Val His Glu Glu Pro Ile Thr Arg Glu Met Leu Tyr Ile
            260                 265                 270

Ala Asp Glu Ala Phe Phe Thr Gly Thr Ala Ala Glu Ile Thr Pro Ile
        275                 280                 285

Arg Ser Val Asp Gly Ile Glu Ile Gly Ala Gly Arg Arg Gly Pro Val
    290                 295                 300

Thr Lys Leu Leu Gln Asp Glu Phe Phe Arg Ile Ile Arg Ala Glu Thr
305                 310                 315                 320

Glu Asp Ser Phe Gly Trp Leu Thr Tyr Ile
                325                 330
```

<210> 80
<211> 993
<212> DNA
<213> Methanobacterium thermoautotrophicum

<400> 80

```
tcagatgtag gtgagccatc cgaagctgtc ctctgtctct gccctgatta tcctgaagaa        60
ctcatcctgc agcagctttg taacgggacc ccttcgcccg gcacctatct ctataccatc       120
aactgatctg atgggtgtta tctctgcggc tgtacctgtg aagaaggcct catctgcgat       180
gtagagcatc tccctggtta tgggttcctc atgcacggta acaccctcgg tcctggctat       240
ctttattacg gagtcccttg ttatcccccct cagaagggat gatgaaacag gggggtgta       300
aatttcaccc tcactgacga ggaatatgtt ctccccgcta ccctcactta tgtagccatg       360
gtagtccagc attatggcct catcatagcc gtgtctcaca gcctccatct tggcaagctg       420
tgagttgagg tagttaccgc cggcctttgc catgttgggc attgtgtttg gtgccatcct       480
ccgccaggtt gaaacaccag catcgacacc aacctcaagg gcctctgcac ccagataggc       540
cccccattcc caggcagcca cagcgacgtc cactgggcag ttcaccgggt gaacacccat       600
ctcaccgtat cccctgaata ccacgggtct tatatagcac tcctcaagtc cgttctccct       660
gacggtctca actatggcat cacatatctg ctcctgggtg tagggtatgt ccatccggta       720
tatctttgca gaatcaaaaa ggcgtttaac atgctcccgc aaacggaaga tggctgaccc       780
cttactgttc ctgtagcacc ttattccctc aaagacagat gatccataat gcacaacatg       840
tgagagtacg tggacggtgg cttcttccca ttcaaccatt tcaccgttta accatatctt       900

tccactggct tcgcatgaca tgataataac ctcaggtgat ttactaggat aggttatggt       960
tggaggccta tataatgctc tccataaccg caa                                    993
```

<210> 81
<211> 364
<212> PRT
<213> Streptomyces coelicolor

<400> 81

```
Met Thr Asp Val Asn Gly Ala Pro Ala Asp Val Leu His Thr Leu Phe
1               5                   10                  15

His Ser Asp Gln Gly Gly His Glu Gln Val Val Leu Cys Gln Asp Arg
            20                  25                  30

Ala Ser Gly Leu Lys Ala Val Ile Ala Leu His Ser Thr Ala Leu Gly
        35                  40                  45

Pro Ala Leu Gly Gly Thr Arg Phe Tyr Pro Tyr Ala Ser Glu Ala Glu
    50                  55                  60

Ala Val Ala Asp Ala Leu Asn Leu Ala Arg Gly Met Ser Tyr Lys Asn
65                  70                  75                  80

Ala Met Ala Gly Leu Asp His Gly Gly Gly Lys Ala Val Ile Ile Gly
                85                  90                  95

Asp Pro Glu Gln Ile Lys Ser Glu Glu Leu Leu Leu Ala Tyr Gly Arg
            100                 105                 110

Phe Val Ala Ser Leu Gly Gly Arg Tyr Val Thr Ala Cys Asp Val Gly
        115                 120                 125

Thr Tyr Val Ala Asp Met Asp Val Val Ala Arg Glu Cys Arg Trp Thr
    130                 135                 140

Thr Gly Arg Ser Pro Glu Asn Gly Gly Ala Gly Asp Ser Ser Val Leu
145                 150                 155                 160

Thr Ser Phe Gly Val Tyr Gln Gly Met Arg Ala Ala Ala Gln His Leu
                165                 170                 175

Trp Gly Asp Pro Thr Leu Arg Asp Arg Thr Val Gly Ile Ala Gly Val
            180                 185                 190

Gly Lys Val Gly His His Leu Val Glu His Leu Leu Ala Glu Gly Ala
        195                 200                 205
```

His Val Val Val Thr Asp Val Arg Lys Asp Val Val Arg Gly Ile Thr
210 215 220

Glu Arg His Pro Ser Val Val Ala Val Ala Asp Thr Asp Ala Leu Ile
225 230 235 240

Arg Val Glu Asn Leu Asp Ile Tyr Ala Pro Cys Ala Leu Gly Gly Ala
245 250 255

Leu Asn Asp Asp Thr Val Pro Val Leu Thr Ala Lys Val Val Cys Gly
260 265 270

Ala Ala Asn Asn Gln Leu Ala His Pro Gly Val Glu Lys Asp Leu Ala
275 280 285

Asp Arg Gly Ile Leu Tyr Ala Pro Asp Tyr Val Val Asn Ala Gly Gly
290 295 300

Val Ile Gln Val Ala Asp Glu Leu His Gly Phe Asp Phe Asp Arg Cys
305 310 315 320

Lys Ala Lys Ala Ser Lys Ile Tyr Asp Thr Thr Leu Ala Ile Phe Ala
325 330 335

Arg Ala Lys Glu Asp Gly Ile Pro Pro Ala Ala Ala Ala Asp Arg Ile
340 345 350

Ala Glu Gln Arg Met Ala Glu Ala Arg Pro Arg Pro
355 360

<210> 82
<211> 1095
<212> DNA
<213> Streptomyces coelicolor

<400> 82

```
tcacggccgg ggacgggcct ccgccatccg ctgctcggcg atccggtcgg ccgccgcggc      60
cggcggaata ccgtcctcct tcgcacgtgc gaatatggcc agcgtggtgt cgtagatctt     120
cgaggccttc gccttgcacc ggtcgaagtc gaacccgtgc agctcgtcgg cgacctggat     180
gacaccgccg gcgttcacca catagtccgg cgcgtagagg atcccgcggt cggcgaggtc     240
cttctcgacg cccgggtggg cgagctggtt gttggccgcg ccgcacacca ccttggcggt     300
cagcaccggc acggtgtcgt cgttcagcgc gccgccgagc gcgcagggcg cgtagatgtc     360
caggttctcc acccggatca gcgcgtcggt gtcggcgacg gcgaccaccg acgggtgccg     420
ctccgtgatc ccgcgcacca cgtccttgcg cacgtccgtg acgacgacgt gggcgccctc     480
ggcgagcagg tgctcgacca ggtggtggcc gaccttgccg acgcccgcga tgccgacggt     540
gcggtcgcgc agcgtcgggt cgccccacag gtgctgggcg gcggcccgca tgccctggta     600
gacgccgaag gaggtgagca cggaggagtc gcccgcgccg ccgttctccg gggaacgccc     660
ggtcgtccag cggcactcgc gggccacgac gtccatgtcg gcgacgtagg tgccgacgtc     720
gcacgcggtg acgtagcggc cgcccagcga ggcgacgaac cggccgtagg cgaggagcag     780
ctcctcgctc ttgatctgct ccggatcgcc gatgatcacg gccttgccgc caccgtggtc     840
cagaccggcc atggcgttct tgtacgacat cccgcgggcg aggttcagcg cgtcggcgac     900
ggcctccgcc tcgctcgcgt acgggtagaa gcgggtaccg ccgagcgccg ggcccagggc     960
ggtggagtgg agggcgatca cggccttgag gccgctggca cggtcctggc agagcacgac    1020
ttgctcatgt ccccccctgat ccgagtggaa cagggtgtgc agtacatcag caggtgcgcc   1080
gtttacgtcg gtcac                                                    1095
```

<210> 83
<211> 364
<212> PRT
<213> Bacillus subtilis

<400> 83

Met Glu Leu Phe Lys Tyr Met Glu Lys Tyr Asp Tyr Glu Gln Leu Val
1               5                   10                  15

Phe Cys Gln Asp Glu Gln Ser Gly Leu Lys Ala Ile Ile Ala Ile His
            20                  25                  30

Asp Thr Thr Leu Gly Pro Ala Leu Gly Gly Thr Arg Met Trp Thr Tyr
        35                  40                  45

Glu Asn Glu Glu Ala Ala Ile Glu Asp Ala Leu Arg Leu Ala Arg Gly
    50                  55                  60

Met Thr Tyr Lys Asn Ala Ala Ala Gly Leu Asn Leu Gly Gly Gly Lys
65                  70                  75                  80

Thr Val Ile Ile Gly Asp Pro Arg Lys Asp Lys Asn Glu Glu Met Phe
                85                  90                  95

Arg Ala Phe Gly Arg Tyr Ile Gln Gly Leu Asn Gly Arg Tyr Ile Thr
            100                 105                 110

Ala Glu Asp Val Gly Thr Thr Val Glu Asp Met Asp Ile Ile His Asp
        115                 120                 125

Glu Thr Asp Tyr Val Thr Gly Ile Ser Pro Ala Phe Gly Ser Ser Gly

130 135 140

Asn Pro Ser Pro Val Thr Ala Tyr Gly Val Tyr Arg Gly Met Lys Ala
145 150 155 160

Ala Ala Lys Ala Ala Phe Gly Thr Asp Ser Leu Glu Gly Lys Thr Ile
165 170 175

Ala Val Gln Gly Val Gly Asn Val Ala Tyr Asn Leu Cys Arg His Leu
180 185 190

His Glu Glu Gly Ala Asn Leu Ile Val Thr Asp Ile Asn Lys Gln Ser
195 200 205

Val Gln Arg Ala Val Glu Asp Phe Gly Ala Arg Ala Val Asp Pro Asp
210 215 220

Asp Ile Tyr Ser Gln Asp Cys Asp Ile Tyr Ala Pro Cys Ala Leu Gly
225 230 235 240

Ala Thr Ile Asn Asp Asp Thr Ile Lys Gln Leu Lys Ala Lys Val Ile
245 250 255

Ala Gly Ala Ala Asn Asn Gln Leu Lys Glu Thr Arg His Gly Asp Gln
260 265 270

Ile His Glu Met Gly Ile Val Tyr Ala Pro Asp Tyr Val Ile Asn Ala
275 280 285

Gly Gly Val Ile Asn Val Ala Asp Glu Leu Tyr Gly Tyr Asn Ala Glu
290 295 300

Arg Ala Leu Lys Lys Val Glu Gly Ile Tyr Gly Asn Ile Glu Arg Val
305 310 315 320

Leu Glu Ile Ser Gln Arg Asp Gly Ile Pro Ala Tyr Leu Ala Ala Asp
325 330 335

Arg Leu Ala Glu Glu Arg Ile Glu Arg Met Arg Arg Ser Arg Ser Gln
340 345 350

Phe Leu Gln Asn Gly His Ser Val Leu Ser Arg Arg
355 360

<210> 84
<211> 364
<212> PRT

<213> Bacillus subtilis

<400> 84

```
Met Glu Leu Phe Lys Tyr Met Glu Lys Tyr Asp Tyr Glu Gln Leu Val
1               5                   10                  15

Phe Cys Gln Asp Glu Gln Ser Gly Leu Lys Ala Ile Ile Ala Ile His
            20                  25                  30

Asp Thr Thr Leu Gly Pro Ala Leu Gly Gly Thr Arg Met Trp Thr Tyr
        35                  40                  45

Glu Asn Glu Glu Ala Ala Ile Glu Asp Ala Leu Arg Leu Ala Arg Gly
    50                  55                  60

Met Thr Tyr Lys Asn Ala Ala Ala Gly Leu Asn Leu Gly Gly Gly Lys
65                  70                  75                  80

Thr Val Ile Ile Gly Asp Pro Arg Lys Asp Lys Asn Glu Glu Met Phe
                85                  90                  95

Arg Ala Phe Gly Arg Tyr Ile Gln Gly Leu Asn Gly Arg Tyr Ile Thr
            100                 105                 110

Ala Glu Asp Val Gly Thr Thr Val Glu Asp Met Asp Ile Ile His Asp
        115                 120                 125

Glu Thr Asp Tyr Val Thr Gly Ile Ser Pro Ala Phe Gly Ser Ser Gly
    130                 135                 140

Asn Pro Ser Pro Val Thr Ala Tyr Gly Val Tyr Arg Gly Met Lys Ala
145                 150                 155                 160

Ala Ala Lys Ala Ala Phe Gly Thr Asp Ser Leu Glu Gly Lys Thr Ile
                165                 170                 175

Ala Val Gln Gly Val Gly Asn Val Ala Tyr Asn Leu Cys Arg His Leu
            180                 185                 190

His Glu Glu Gly Ala Asn Leu Ile Val Thr Asp Ile Asn Lys Gln Ser
        195                 200                 205

Val Gln Arg Ala Val Glu Asp Phe Gly Ala Arg Ala Val Asp Pro Asp
    210                 215                 220

Asp Ile Tyr Ser Gln Asp Cys Asp Ile Tyr Ala Pro Cys Ala Leu Gly
225                 230                 235                 240
```

Ala Thr Ile Asn Asp Asp Thr Ile Lys Gln Leu Lys Ala Lys Val Ile
245 250 255

Ala Gly Ala Ala Asn Asn Gln Leu Lys Glu Thr Arg His Gly Asp Gln
260 265 270

Ile His Glu Met Gly Ile Val Tyr Ala Pro Asp Tyr Val Ile Asn Ala
275 280 285

Gly Gly Val Ile Asn Val Ala Asp Glu Leu Tyr Gly Tyr Asn Ala Glu
290 295 300

Arg Ala Leu Lys Lys Val Glu Gly Ile Tyr Gly Asn Ile Glu Arg Val
305 310 315 320

Leu Glu Ile Ser Gln Arg Asp Gly Ile Pro Ala Tyr Leu Ala Ala Asp
325 330 335

Arg Leu Ala Glu Glu Arg Ile Glu Arg Met Arg Arg Ser Arg Ser Gln
340 345 350

Phe Leu Gln Asn Gly His Ser Val Leu Ser Arg Arg
355 360

<210> 85
<211> 594
<212> PRT
<213> Streptomyces viridifaciens

<400> 85

Met Ser Thr Ser Ser Ala Ser Ser Gly Pro Asp Leu Pro Phe Gly Pro
1 5 10 15

Glu Asp Thr Pro Trp Gln Lys Ala Phe Ser Arg Leu Arg Ala Val Asp
20 25 30

Gly Val Pro Arg Val Thr Ala Pro Ser Ser Asp Pro Arg Glu Val Tyr
35 40 45

Met Asp Ile Pro Glu Ile Pro Phe Ser Lys Val Gln Ile Pro Pro Asp
50 55 60

Gly Met Asp Glu Gln Gln Tyr Ala Glu Ala Glu Ser Leu Phe Arg Arg
65 70 75 80

Tyr Val Asp Ala Gln Thr Arg Asn Phe Ala Gly Tyr Gln Val Thr Ser
85 90 95

```
Asp Leu Asp Tyr Gln His Leu Ser His Tyr Leu Asn Arg His Leu Asn
            100                 105                 110

Asn Val Gly Asp Pro Tyr Glu Ser Ser Ser Tyr Thr Leu Asn Ser Lys
        115                 120                 125

Val Leu Glu Arg Ala Val Leu Asp Tyr Phe Ala Ser Leu Trp Asn Ala
    130                 135                 140

Lys Trp Pro His Asp Ala Ser Asp Pro Glu Thr Tyr Trp Gly Tyr Val
145                 150                 155                 160

Leu Thr Met Gly Ser Ser Glu Gly Asn Leu Tyr Gly Leu Trp Asn Ala
                165                 170                 175

Arg Asp Tyr Leu Ser Gly Lys Leu Leu Arg Arg Gln His Arg Glu Ala
            180                 185                 190

Gly Gly Asp Lys Ala Ser Val Val Tyr Thr Gln Ala Leu Arg His Glu
        195                 200                 205

Gly Gln Ser Pro His Ala Tyr Glu Pro Val Ala Phe Phe Ser Gln Asp
    210                 215                 220

Thr His Tyr Ser Leu Thr Lys Ala Val Arg Val Leu Gly Ile Asp Thr
225                 230                 235                 240

Phe His Ser Ile Gly Ser Ser Arg Tyr Pro Asp Glu Asn Pro Leu Gly
                245                 250                 255

Pro Gly Thr Pro Trp Pro Thr Glu Val Pro Ser Val Asp Gly Ala Ile
            260                 265                 270

Asp Val Asp Lys Leu Ala Ser Leu Val Arg Phe Phe Ala Ser Lys Gly
        275                 280                 285

Tyr Pro Ile Leu Val Ser Leu Asn Tyr Gly Ser Thr Phe Lys Gly Ala
    290                 295                 300

Tyr Asp Asp Val Pro Ala Val Ala Gln Ala Val Arg Asp Ile Cys Thr
305                 310                 315                 320

Glu Tyr Gly Leu Asp Arg Arg Arg Val Tyr His Asp Arg Ser Lys Asp
                325                 330                 335

Ser Asp Phe Asp Glu Arg Ser Gly Phe Trp Ile His Ile Asp Ala Ala
            340                 345                 350
```

Leu Gly Ala Gly Tyr Ala Pro Tyr Leu Gln Met Ala Arg Asp Ala Gly
355 360 365

Met Val Glu Glu Ala Pro Pro Val Phe Asp Phe Arg Leu Pro Glu Val
370 375 380

His Ser Leu Thr Met Ser Gly His Lys Trp Met Gly Thr Pro Trp Ala
385 390 395 400

Cys Gly Val Tyr Met Thr Arg Thr Gly Leu Gln Met Thr Pro Pro Lys
405 410 415

Ser Ser Glu Tyr Ile Gly Ala Ala Asp Thr Thr Phe Ala Gly Ser Arg
420 425 430

Asn Gly Phe Ser Ser Leu Leu Leu Trp Asp Tyr Leu Ser Arg His Ser
435 440 445

Tyr Asp Asp Leu Val Arg Leu Ala Ala Asp Cys Asp Arg Leu Ala Gly
450 455 460

Tyr Ala His Asp Arg Leu Leu Thr Leu Gln Asp Lys Leu Gly Met Asp
465 470 475 480

Leu Trp Val Ala Arg Ser Pro Gln Ser Leu Thr Val Arg Phe Arg Gln
485 490 495

Pro Cys Ala Asp Ile Val Arg Lys Tyr Ser Leu Ser Cys Glu Thr Val
500 505 510

Tyr Glu Asp Asn Glu Gln Arg Thr Tyr Val His Leu Tyr Ala Val Pro
515 520 525

His Leu Thr Arg Glu Leu Val Asp Glu Leu Val Arg Asp Leu Arg Gln
530 535 540

Pro Gly Ala Phe Thr Asn Ala Gly Ala Leu Glu Gly Glu Ala Trp Ala
545 550 555 560

Gly Val Ile Asp Ala Leu Gly Arg Pro Asp Pro Asp Gly Thr Tyr Ala
565 570 575

Gly Ala Leu Ser Ala Pro Ala Ser Gly Pro Arg Ser Glu Asp Gly Gly
580 585 590

Gly Ser

<210> 86
<211> 1785
<212> DNA
<213> Streptomyces viridifaciens

<400> 86

gtgtcaactt cctccgcttc ttccgggccg gacctcccct tcgggcccga ggacacgcca 60

tggcagaagg ccttcagcag gctgcgggcg gtggatggcg tgccgcgcgt caccgcgccg 120

tccagtgatc cgcgtgaggt ctacatggac atcccggaga tccccttctc caaggtccag 180

atcccccgg acggaatgga cgagcagcag tacgcagagg ccgagagcct cttccgccgc 240

tacgtagacg cccagacccg caacttcgcg ggataccagg tcaccagcga cctcgactac 300

cagcacctca gtcactatct caaccggcat ctgaacaacg tcggcgatcc ctatgagtcc 360

agctcctaca cgctgaactc caaggtcctt gagcgagccg ttctcgacta cttcgcctcc 420

ctgtggaacg ccaagtggcc ccatgacgca agcgatccgg aaacgtactg gggttacgtg 480

ctgaccatgg gctccagcga aggcaacctg tacgggttgt ggaacgcacg ggactatctg 540

tcgggcaagc tgctgcggcg ccagcaccgg gaggccggcg gcgacaaggc ctcggtcgtc 600

tacacgcaag cgctgcgaca cgaagggcag agtccgcatg cctacgagcc ggtggcgttc 660

ttctcgcagg acacgcacta ctcgctcacg aaggccgtgc gggttctggg catcgacacc 720

ttccacagca tcggcagcag tcggtatccg gacgagaacc cgctgggccc cggcactccg 780

tggccgaccg aagtgccctc ggttgacggt gccatcgatg tcgacaaact cgcctcgttg 840

gtccgcttct tcgccagcaa gggctacccg atactggtca gcctcaacta cgggtcaacg 900

ttcaagggcg cctacgacga cgtcccggcc gtggcacagg ccgtgcggga catctgcacg 960

gaatacggtc tggatcggcg gcgggtatac cacgaccgca gtaaggacag tgacttcgac 1020

gagcgcagcg gcttctggat ccacatcgat gccgccctgg gggcgggcta cgctccctac 1080

ctgcagatgg cccgggatgc cggcatggtc gaggaggcgc cgcccgtttt cgacttccgg 1140

ctcccggagg tgcactcgct gaccatgagc ggccacaagt ggatgggaac accgtgggca 1200

tgcggtgtct acatgacacg gaccgggctg cagatgaccc cgccgaagtc gtccgagtac 1260

atcggggcgg ccgacaccac cttcgcgggc tcccgcaacg gcttctcgtc actgctgctg 1320

tgggactacc tgtcccggca ttcgtatgac gatctggtgc gcctggccgc cgactgcgac 1380

cggctggccg gctacgccca cgaccggttg ctgaccttgc aggacaaact cggcatggat 1440

ctgtgggtcg cccgcagccc gcagtccctc acggtgcgct tccgtcagcc atgtgcagac 1500

atcgtccgca agtactcgct gtcgtgtgag acggtctacg aagacaacga gcaacggacc 1560

tacgtacatc tctacgccgt tccccacctc actcgggaac tcgtggatga gctcgtgcgc 1620

gatctgcgcc agcccggagc cttcaccaac gctggtgcac tggaggggga ggcctgggcc 1680

ggggtgatcg atgccctcgg ccgcccggac cccgacggaa cctatgccgg cgccttgagc 1740

gctccggctt ccggcccccg ctccgaggac ggcggcggga gctga 1785

<210> 87
<211> 440
<212> PRT
<213> Alcaligenes denitrificans

<400> 87

```
Met Ser Ala Ala Lys Leu Pro Asp Leu Ser His Leu Trp Met Pro Phe
1               5                   10                  15

Thr Ala Asn Arg Gln Phe Lys Ala Asn Pro Arg Leu Leu Ala Ser Ala
            20                  25                  30

Lys Gly Met Tyr Tyr Thr Ser Phe Asp Gly Arg Gln Ile Leu Asp Gly
        35                  40                  45

Thr Ala Gly Leu Trp Cys Val Asn Ala Gly His Cys Arg Glu Glu Ile
    50                  55                  60

Val Ser Ala Ile Ala Ser Gln Ala Gly Val Met Asp Tyr Ala Pro Gly
65                  70                  75                  80

Phe Gln Leu Gly His Pro Leu Ala Phe Glu Ala Ala Thr Ala Val Ala
                85                  90                  95

Gly Leu Met Pro Gln Gly Leu Asp Arg Val Phe Phe Thr Asn Ser Gly
            100                 105                 110

Ser Glu Ser Val Asp Thr Ala Leu Lys Ile Ala Leu Ala Tyr His Arg
        115                 120                 125

Ala Arg Gly Glu Ala Gln Arg Thr Arg Leu Ile Gly Arg Glu Arg Gly
    130                 135                 140

Tyr His Gly Val Gly Phe Gly Gly Ile Ser Val Gly Gly Ile Ser Pro
145                 150                 155                 160

Asn Arg Lys Thr Phe Ser Gly Ala Leu Leu Pro Ala Val Asp His Leu
                165                 170                 175

Pro His Thr His Ser Leu Glu His Asn Ala Phe Thr Arg Gly Gln Pro
            180                 185                 190

Glu Trp Gly Ala His Leu Ala Asp Glu Leu Glu Arg Ile Ile Ala Leu
        195                 200                 205
```

```
His Asp Ala Ser Thr Ile Ala Ala Val Ile Val Glu Pro Met Ala Gly
    210                 215                 220

Ser Thr Gly Val Leu Val Pro Pro Lys Gly Tyr Leu Glu Lys Leu Arg
225                 230                 235                 240

Glu Ile Thr Ala Arg His Gly Ile Leu Leu Ile Phe Asp Glu Val Ile
                245                 250                 255

Thr Ala Tyr Gly Arg Leu Gly Glu Ala Thr Ala Ala Ala Tyr Phe Gly
            260                 265                 270

Val Thr Pro Asp Leu Ile Thr Met Ala Lys Gly Val Ser Asn Ala Ala
        275                 280                 285

Val Pro Ala Gly Ala Val Ala Val Arg Arg Glu Val His Asp Ala Ile
    290                 295                 300

Val Asn Gly Pro Gln Gly Gly Ile Glu Phe Phe His Gly Tyr Thr Tyr
305                 310                 315                 320

Ser Ala His Pro Leu Ala Ala Ala Ala Val Leu Ala Thr Leu Asp Ile
                325                 330                 335

Tyr Arg Arg Glu Asp Leu Phe Ala Arg Ala Arg Lys Leu Ser Ala Ala
            340                 345                 350

Phe Glu Glu Ala Ala His Ser Leu Lys Gly Ala Pro His Val Ile Asp
        355                 360                 365

Val Arg Asn Ile Gly Leu Val Ala Gly Ile Glu Leu Ser Pro Arg Glu
    370                 375                 380

Gly Ala Pro Gly Ala Arg Ala Ala Glu Ala Phe Gln Lys Cys Phe Asp
385                 390                 395                 400

Thr Gly Leu Met Val Arg Tyr Thr Gly Asp Ile Leu Ala Val Ser Pro
                405                 410                 415

Pro Leu Ile Val Asp Glu Asn Gln Ile Gly Gln Ile Phe Glu Gly Ile
            420                 425                 430

Gly Lys Val Leu Lys Glu Val Ala
        435                 440
```

<210> 88
<211> 1947

<212> DNA
<213> Alcaligenes denitrificans

<400> 88

ttcgatggcg cgctgcacgg cggccaccag ctgctccacc aggggtgggc gcctgcccgc 60
gcgcgcggtc gggctggaaa tcgatcatgg atgaatctat acagttgtca tgattgcaac 120
tatacagtta gcccgttttg cggcaattgt atattttcat tcgctcgtgg acgtccgaga 180
atcggtttga tcgcgccgcc cgcccctttc cgcgcagcgg cgtttctttt cctccggagt 240
ctccccatga gcgctgccaa actgcccgac ctgtcccacc tctggatgcc ctttaccgcc 300
aaccggcagt tcaaggcgaa cccccgcctg ctggcctcgg ccaagggcat gtactacacg 360
tctttcgacg gccgccagat cctggacggc acggccggcc tgtggtgcgt gaacgccggc 420
cactgccgcg aagaaatcgt ctccgccatc gccagccagg ccggcgtcat ggactacgcg 480
ccggggttcc agctcggcca cccgctggcc ttcgaggccg ccaccgccgt ggccggcctg 540
atgccgcagg gcctggaccg cgtgttcttc accaattcgg gctccgaatc ggtggacacc 600
gcgctgaaga tcgccctggc ctaccaccgc gcgcgcggcg aggcgcagcg cacccgcctc 660
atcgggcgcg agcgcggcta ccacggcgtg ggcttcggcg gcatttccgt gggcggcatc 720
tcgcccaacc gcaagacctt ctccggcgcg ctgctgccgg ccgtggacca cctgccgcac 780
acccacagcc tggaacacaa cgccttcacg cgcggccagc ccgagtgggg cgcgcacctg 840
gccgacgagt tggaacgcat catcgccctg cacgacgcct ccaccatcgc ggccgtgatc 900
gtcgagccca tggccggctc caccggcgtg ctcgtcccgc ccaagggcta tctcgaaaaa 960
ctgcgcgaaa tcaccgcccg ccacggcatt ctgctgatct tcgacgaagt catcaccgcg 1020
tacggccgcc tgggcgaggc caccgccgcg gcctatttcg gcgtaacgcc cgacctcatc 1080
accatggcca agggcgtgag caacgccgcc gttccggccg gcgccgtcgc ggtgcgccgc 1140
gaagtgcatg acgccatcgt caacggaccg caaggcggca tcgagttctt ccacggctac 1200
acctactcgg cccacccgct ggccgccgcc gccgtgctcg ccacgctgga catctaccgc 1260
cgcgaagacc tgttcgcccg cgcccgcaag ctgtcggccg cgttcgagga agccgcccac 1320
agcctcaagg gcgcgccgca cgtcatcgac gtgcgcaaca tcggcctggt ggccggcatc 1380
gagctgtcgc cgcgcgaagg cgccccgggc gcgcgcgccg ccgaagcctt ccagaaatgc 1440
ttcgacaccg gcctcatggt gcgctacacg ggcgacatcc tcgcggtgtc gcctccgctc 1500
atcgtcgacg aaaaccagat cggccagatc ttcgagggca tcggcaaggt gctcaaggaa 1560
gtggcttagg gtgaacacgc cctgagccgg ccccggcagg aaacgcgccg ccgcgcggcg 1620
gcgcgtccat cgaactcccg catcgagctt ttgcattcat gaagaaaatc acgcatttca 1680
tcaacggcca gccccacgaa ggccgcagca accgctacac cgagggcttc aacccggcca 1740

cgggcgagtc gtctcctcga tctgcctggg cggggccgaa gaagtggacc tggccgtggc 1800

ggccgcccgc gcggcctttc ccgcctggtc cgaaacgccg gcgctcaagc gcgcgcgcgt 1860

gctgttcaac ttcaaggcgc tgctggacaa gcaccaggac gagctggccg cgctcatcac 1920

gcgcgagcac ggcaaggtgt tttccga 1947

<210> 89
<211> 443
<212> PRT
<213> Ralstonia eutropha

<400> 89

Met Asp Ala Ala Lys Thr Val Ile Pro Asp Leu Asp Ala Leu Trp Met
1 5 10 15

Pro Phe Thr Ala Asn Arg Gln Tyr Lys Ala Ala Pro Arg Leu Leu Ala
20 25 30

Ser Ala Ser Gly Met Tyr Tyr Thr Thr His Asp Gly Arg Gln Ile Leu
35 40 45

Asp Gly Cys Ala Gly Leu Trp Cys Val Ala Ala Gly His Cys Arg Lys
50 55 60

Glu Ile Ala Glu Ala Val Ala Arg Gln Ala Ala Thr Leu Asp Tyr Ala
65 70 75 80

Pro Pro Phe Gln Met Gly His Pro Leu Ser Phe Glu Ala Ala Thr Lys
85 90 95

Val Ala Ala Ile Met Pro Gln Gly Leu Asp Arg Ile Phe Phe Thr Asn
100 105 110

Ser Gly Ser Glu Ser Val Asp Thr Ala Leu Lys Ile Ala Leu Ala Tyr
115 120 125

His Arg Ala Arg Gly Glu Gly Gln Arg Thr Arg Phe Ile Gly Arg Glu
130 135 140

Arg Gly Tyr His Gly Val Gly Phe Gly Gly Met Ala Val Gly Gly Ile
145 150 155 160

Gly Pro Asn Arg Lys Ala Phe Ser Ala Asn Leu Met Pro Gly Thr Asp
165 170 175

His Leu Pro Ala Thr Leu Asn Ile Ala Glu Ala Ala Phe Ser Lys Gly
180 185 190

```
Gln Pro Thr Trp Gly Ala His Leu Ala Asp Glu Leu Glu Arg Ile Val
        195                 200                 205

Ala Leu His Asp Pro Ser Thr Ile Ala Ala Val Ile Val Glu Pro Leu
    210                 215                 220

Ala Gly Ser Ala Gly Val Leu Val Pro Pro Val Gly Tyr Leu Asp Lys
225                 230                 235                 240

Leu Arg Glu Ile Thr Thr Lys His Gly Ile Leu Leu Ile Phe Asp Glu
                245                 250                 255

Val Ile Thr Ala Phe Gly Arg Leu Gly Thr Ala Thr Ala Ala Glu Arg
            260                 265                 270

Phe Lys Val Thr Pro Asp Leu Ile Thr Met Ala Lys Ala Ile Asn Asn
        275                 280                 285

Ala Ala Val Pro Met Gly Ala Val Ala Val Arg Arg Glu Val His Asp
    290                 295                 300

Thr Val Val Asn Ser Ala Ala Pro Gly Ala Ile Glu Leu Ala His Gly
305                 310                 315                 320

Tyr Thr Tyr Ser Gly His Pro Leu Ala Ala Ala Ala Ala Ile Ala Thr
                325                 330                 335

Leu Asp Leu Tyr Gln Arg Glu Asn Leu Phe Gly Arg Ala Ala Glu Leu
            340                 345                 350

Ser Pro Val Phe Glu Ala Ala Val His Ser Val Arg Ser Ala Pro His
        355                 360                 365

Val Lys Asp Ile Arg Asn Leu Gly Met Val Ala Gly Ile Glu Leu Glu
    370                 375                 380

Pro Arg Pro Gly Gln Pro Gly Ala Arg Ala Tyr Glu Ala Phe Leu Lys
385                 390                 395                 400

Cys Leu Glu Arg Gly Val Leu Val Arg Tyr Thr Gly Asp Ile Leu Ala
                405                 410                 415

Phe Ser Pro Pro Leu Ile Ile Ser Glu Ala Gln Ile Ala Glu Leu Phe
            420                 425                 430

Asp Thr Val Lys Gln Ala Leu Gln Glu Val Gln
        435                 440
```

<210> 90
<211> 1341
<212> DNA
<213> Ralstonia eutropha

<400> 90

```
atggccgact cacccaacaa cctcgctcac gaacatcctt cacttgaaca ctattggatg      60
ccttttaccg ccaatcgcca attcaaagcg agccctcgtt tactcgccca agctgaaggt     120
atgtattaca cagatatcaa tggcaacaag gtattagact ctacagcggg cttatggtgt     180
tgtaatgctg gccatggtcg ccgtgagatc agtgaagccg tcagcaaaca aattcggcag     240
atggattacg ctccctcctt ccaaatgggc catcccatcg cttttgaact ggccgaacgt     300
ttaaccgaac tcagcccaga aggactcaac aaagtattct ttaccaactc aggctctgag     360
tcggttgata ccgcgctaaa aatggctctt tgctaccata gagccaatgg ccaagcgtca     420
cgcacccgct ttattggccg tgaaatgggt taccatggcg taggatttgg tgggatctcg     480
gtgggtggtt taagcaataa ccgtaaagcc ttcagcggcc agctattgca aggcgtggat     540
cacctgcccc acaccttaga cattcaacat gccgccttta gtcgtggctt accgagcctc     600
ggtgctgaaa aagctgaggt attagaacaa ttagtcacac tccatggcgc cgaaaatatt     660
gccgccgtta ttgttgaacc catgtcaggt tctgcagggg taattttacc acctcaaggc     720
tacttaaaac gcttacgtga aatcactaaa aaacacggca tcttattgat tttcgatgaa     780
gtcattaccg catttggccg tgtaggtgca gcattcgcca gccaacgttg gggcgttatt     840
ccagacataa tcaccacggc taaagccatt aataatggcg ccatccccat gggcgcagtg     900
tttgtacagg attatatcca cgatacttgc atgcaagggc caaccgaact gattgaattt     960
ttccacggtt atacctattc gggccaccca gtcgccgcag cagcagcact cgccacgctc    1020
tccatctacc aaaacgagca actgtttgag cgcagttttg agcttgagcg gtatttcgaa    1080
gaagccgttc atagcctcaa agggttaccg aatgtgattg atattcgcaa caccggatta    1140
gtcgcgggtt tccagctagc accgaatagc caaggtgttg gtaaacgcgg atacagcgtg    1200
ttcgagcatt gtttccatca aggcacactc gtgcgggcaa cgggcgatat tatcgccatg    1260
tccccaccac tcattgttga gaaacatcag attgaccaaa tggtaaatag ccttagcgat    1320
gcaattcacg ccgttggatg a                                              1341
```

<210> 91
<211> **446**
<212> PRT
<213> Shewanella oneidensis

<400> 91

Met Ala Asp Ser Pro Asn Asn Leu Ala His Glu His Pro Ser Leu Glu

1 5 10 15

His Tyr Trp Met Pro Phe Thr Ala Asn Arg Gln Phe Lys Ala Ser Pro
20 25 30

Arg Leu Leu Ala Gln Ala Glu Gly Met Tyr Tyr Thr Asp Ile Asn Gly
35 40 45

Asn Lys Val Leu Asp Ser Thr Ala Gly Leu Trp Cys Cys Asn Ala Gly
50 55 60

His Gly Arg Arg Glu Ile Ser Glu Ala Val Ser Lys Gln Ile Arg Gln
65 70 75 80

Met Asp Tyr Ala Pro Ser Phe Gln Met Gly His Pro Ile Ala Phe Glu
85 90 95

Leu Ala Glu Arg Leu Thr Glu Leu Ser Pro Glu Gly Leu Asn Lys Val
100 105 110

Phe Phe Thr Asn Ser Gly Ser Glu Ser Val Asp Thr Ala Leu Lys Met
115 120 125

Ala Leu Cys Tyr His Arg Ala Asn Gly Gln Ala Ser Arg Thr Arg Phe
130 135 140

Ile Gly Arg Glu Met Gly Tyr His Gly Val Gly Phe Gly Gly Ile Ser
145 150 155 160

Val Gly Gly Leu Ser Asn Asn Arg Lys Ala Phe Ser Gly Gln Leu Leu
165 170 175

Gln Gly Val Asp His Leu Pro His Thr Leu Asp Ile Gln His Ala Ala
180 185 190

Phe Ser Arg Gly Leu Pro Ser Leu Gly Ala Glu Lys Ala Glu Val Leu
195 200 205

Glu Gln Leu Val Thr Leu His Gly Ala Glu Asn Ile Ala Ala Val Ile
210 215 220

Val Glu Pro Met Ser Gly Ser Ala Gly Val Ile Leu Pro Pro Gln Gly
225 230 235 240

Tyr Leu Lys Arg Leu Arg Glu Ile Thr Lys Lys His Gly Ile Leu Leu
245 250 255

Ile Phe Asp Glu Val Ile Thr Ala Phe Gly Arg Val Gly Ala Ala Phe
260 265 270

Ala Ser Gln Arg Trp Gly Val Ile Pro Asp Ile Ile Thr Thr Ala Lys
275 280 285

Ala Ile Asn Asn Gly Ala Ile Pro Met Gly Ala Val Phe Val Gln Asp
290 295 300

Tyr Ile His Asp Thr Cys Met Gln Gly Pro Thr Glu Leu Ile Glu Phe
305 310 315 320

Phe His Gly Tyr Thr Tyr Ser Gly His Pro Val Ala Ala Ala Ala Ala
325 330 335

Leu Ala Thr Leu Ser Ile Tyr Gln Asn Glu Gln Leu Phe Glu Arg Ser
340 345 350

Phe Glu Leu Glu Arg Tyr Phe Glu Glu Ala Val His Ser Leu Lys Gly
355 360 365

Leu Pro Asn Val Ile Asp Ile Arg Asn Thr Gly Leu Val Ala Gly Phe
370 375 380

Gln Leu Ala Pro Asn Ser Gln Gly Val Gly Lys Arg Gly Tyr Ser Val
385 390 395 400

Phe Glu His Cys Phe His Gln Gly Thr Leu Val Arg Ala Thr Gly Asp
405 410 415

Ile Ile Ala Met Ser Pro Pro Leu Ile Val Glu Lys His Gln Ile Asp
420 425 430

Gln Met Val Asn Ser Leu Ser Asp Ala Ile His Ala Val Gly
435 440 445

<210> 92
<211> 1341
<212> DNA
<213> Shewanella oneidensis

<400> 92

```
atggccgact cacccaacaa cctcgctcac gaacatcctt cacttgaaca ctattggatg      60
ccttttaccg ccaatcgcca attcaaagcg agccctcgtt tactcgccca agctgaaggt     120
atgtattaca cagatatcaa tggcaacaag gtattagact ctacagcggg cttatggtgt     180
tgtaatgctg gccatggtcg ccgtgagatc agtgaagccg tcagcaaaca aattcggcag     240
atggattacg ctccctcctt ccaaatgggc catcccatcg cttttgaact ggccgaacgt     300
ttaaccgaac tcagcccaga aggactcaac aaagtattct ttaccaactc aggctctgag     360
tcggttgata ccgcgctaaa aatggctctt tgctaccata gagccaatgg ccaagcgtca     420
cgcacccgct ttattggccg tgaaatgggt taccatggcg taggatttgg tgggatctcg     480
gtgggtggtt taagcaataa ccgtaaagcc ttcagcggcc agctattgca aggcgtggat     540
cacctgcccc acaccttaga cattcaacat gccgccttta gtcgtggctt accgagcctc     600
ggtgctgaaa aagctgaggt attagaacaa ttagtcacac tccatggcgc cgaaaatatt     660
gccgccgtta ttgttgaacc catgtcaggt tctgcagggg taattttacc acctcaaggc     720
tacttaaaac gcttacgtga aatcactaaa aaacacggca tcttattgat tttcgatgaa     780
gtcattaccg catttggccg tgtaggtgca gcattcgcca gccaacgttg gggcgttatt     840
ccagacataa tcaccacggc taaagccatt aataatggcg ccatccccat gggcgcagtg     900
tttgtacagg attatatcca cgatacttgc atgcaagggc caaccgaact gattgaattt     960
ttccacggtt atacctattc gggccaccca gtcgccgcag cagcagcact cgccacgctc    1020
tccatctacc aaaacgagca actgtttgag cgcagttttg agcttgagcg gtatttcgaa    1080
gaagccgttc atagcctcaa agggttaccg aatgtgattg atattcgcaa caccggatta    1140
gtcgcgggtt tccagctagc accgaatagc caaggtgttg gtaaacgcgg atacagcgtg    1200
ttcgagcatt gtttccatca aggcacactc gtgcgggcaa cgggcgatat tatcgccatg    1260
tccccaccac tcattgttga gaaacatcag attgaccaaa tggtaaatag ccttagcgat    1320
gcaattcacg ccgttggatg a                                               1341
```

<210> 93
<211> 448
<212> PRT
<213> Pseudomonas putida

<400> 93

```
Met Asn Met Pro Glu Thr Gly Pro Ala Gly Ile Ala Ser Gln Leu Lys
1               5                   10                  15

Leu Asp Ala His Trp Met Pro Tyr Thr Ala Asn Arg Asn Phe Gln Arg
            20                  25                  30

Asp Pro Arg Leu Ile Val Ala Ala Glu Gly Asn Tyr Leu Val Asp Asp
        35                  40                  45

His Gly Arg Lys Ile Phe Asp Ala Leu Ser Gly Leu Trp Thr Cys Gly
    50                  55                  60

Ala Gly His Thr Arg Lys Glu Ile Ala Asp Ala Val Thr Arg Gln Leu
65                  70                  75                  80
```

```
Ser Thr Leu Asp Tyr Ser Pro Ala Phe Gln Phe Gly His Pro Leu Ser
                85                  90                  95

Phe Gln Leu Ala Glu Lys Ile Ala Glu Leu Val Pro Gly Asn Leu Asn
            100                 105                 110

His Val Phe Tyr Thr Asn Ser Gly Ser Glu Cys Ala Asp Thr Ala Leu
        115                 120                 125

Lys Met Val Arg Ala Tyr Trp Arg Leu Lys Gly Gln Ala Thr Lys Thr
    130                 135                 140

Lys Ile Ile Gly Arg Ala Arg Gly Tyr His Gly Val Asn Ile Ala Gly
145                 150                 155                 160

Thr Ser Leu Gly Gly Val Asn Gly Asn Arg Lys Met Phe Gly Gln Leu
                165                 170                 175

Leu Asp Val Asp His Leu Pro His Thr Val Leu Pro Val Asn Ala Phe
            180                 185                 190

Ser Lys Gly Leu Pro Glu Glu Gly Gly Ile Ala Leu Ala Asp Glu Met
        195                 200                 205

Leu Lys Leu Ile Glu Leu His Asp Ala Ser Asn Ile Ala Ala Val Ile
    210                 215                 220

Val Glu Pro Leu Ala Gly Ser Ala Gly Val Leu Pro Pro Pro Lys Gly
225                 230                 235                 240

Tyr Leu Lys Arg Leu Arg Glu Ile Cys Thr Gln His Asn Ile Leu Leu
                245                 250                 255

Ile Phe Asp Glu Val Ile Thr Gly Phe Gly Arg Met Gly Ala Met Thr
            260                 265                 270

Gly Ser Glu Ala Phe Gly Val Thr Pro Asp Leu Met Cys Ile Ala Lys
        275                 280                 285

Gln Val Thr Asn Gly Ala Ile Pro Met Gly Ala Val Ile Ala Ser Ser
    290                 295                 300

Glu Ile Tyr Gln Thr Phe Met Asn Gln Pro Thr Pro Glu Tyr Ala Val
305                 310                 315                 320

Glu Phe Pro His Gly Tyr Thr Tyr Ser Ala His Pro Val Ala Cys Ala
```

325 330 335

Ala Gly Leu Ala Ala Leu Asp Leu Leu Gln Lys Glu Asn Leu Val Gln
340 345 350

Ser Ala Ala Glu Leu Ala Pro His Phe Glu Lys Leu Leu His Gly Val
355 360 365

Lys Gly Thr Lys Asn Ile Val Asp Ile Arg Asn Tyr Gly Leu Ala Gly
370 375 380

Ala Ile Gln Ile Ala Ala Arg Asp Gly Asp Ala Ile Val Arg Pro Tyr
385 390 395 400

Glu Ala Ala Met Lys Leu Trp Lys Ala Gly Phe Tyr Val Arg Phe Gly
405 410 415

Gly Asp Thr Leu Gln Phe Gly Pro Thr Phe Asn Thr Lys Pro Gln Glu
420 425 430

Leu Asp Arg Leu Phe Asp Ala Val Gly Glu Thr Leu Asn Leu Ile Asp
435 440 445

<210> 94
<211> 930
<212> DNA
<213> Pseudomonas putida

<400> 94

```
atgaccacga agaaagctga ttacatttgg ttcaatgggg agatggttcg ctgggaagac        60
gcgaaggtgc atgtgatgtc gcacgcgctg cactatggca cttcggtttt tgaaggcatc       120
cgttgctacg actcgcacaa aggaccggtt gtattccgcc atcgtgagca tatgcagcgt       180
ctgcatgact ccgccaaaat ctatcgcttc ccggtttcgc agagcattga tgagctgatg       240
gaagcttgtc gtgacgtgat ccgcaaaaac aatctcacca gcgcctatat ccgtccgctg       300
atcttcgtcg gtgatgttgg catgggagta aacccgccag cgggatactc aaccgacgtg       360
attatcgctg ctttcccgtg gggagcgtat ctgggcgcag aagcgctgga gcaggggatc       420
gatgcgatgg tttcctcctg gaaccgcgca gcaccaaaca ccatcccgac ggcggcaaaa       480
gccggtggta actacctctc ttccctgctg gtgggtagcg aagcgcgccg ccacggttat       540
caggaaggta tcgcgctgga tgtgaacggt tatatctctg aaggcgcagg cgaaaacctg       600
tttgaagtga aagatggtgt gctgttcacc ccaccgttca cctcctccgc gctgccgggt       660
attacccgtg atgccatcat caaactggcg aaagagctgg gaattgaagt acgtgagcag       720
gtgctgtcgc gcgaatccct gtacctggcg gatgaagtgt ttatgtccgg tacggcggca       780
gaaatcacgc cagtgcgcag cgtagacggt attcaggttg gcgaaggccg ttgtggcccg       840
gttaccaaac gcattcagca agccttcttc ggcctcttca ctggcgaaac cgaagataaa       900
tggggctggt tagatcaagt taatcaataa                                        930
```

<210> 95
<211> 566
<212> PRT
<213> Streptomyces cinnamonensis

<400> 95

Met Asp Ala Asp Ala Ile Glu Glu Gly Arg Arg Arg Trp Gln Ala Arg
1               5                   10                  15

Tyr Asp Lys Ala Arg Lys Arg Asp Ala Asp Phe Thr Thr Leu Ser Gly
            20                  25                  30

Asp Pro Val Asp Pro Val Tyr Gly Pro Arg Pro Gly Asp Thr Tyr Asp
        35                  40                  45

Gly Phe Glu Arg Ile Gly Trp Pro Gly Glu Tyr Pro Phe Thr Arg Gly
    50                  55                  60

Leu Tyr Ala Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Met Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Pro Arg Ser Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ala Val Leu Asn Gly Thr Leu Gln Thr Asp Ile Phe
            180                 185                 190

Lys Glu Tyr Ile Ala Gln Lys Glu Trp Leu Phe Gln Pro Glu Pro His

195 200 205

Leu Arg Leu Ile Gly Asp Leu Met Glu His Cys Ala Arg Asp Ile Pro
210 215 220

Ala Tyr Lys Pro Leu Ser Val Ser Gly Tyr His Ile Arg Glu Ala Gly
225 230 235 240

Ala Thr Ala Ala Gln Glu Leu Ala Tyr Thr Leu Ala Asp Gly Phe Gly
245 250 255

Tyr Val Glu Leu Gly Leu Ser Arg Gly Leu Asp Val Asp Val Phe Ala
260 265 270

Pro Gly Leu Ser Phe Phe Phe Asp Ala His Val Asp Phe Phe Glu Glu
275 280 285

Ile Ala Lys Phe Arg Ala Ala Arg Arg Ile Trp Ala Arg Trp Leu Arg
290 295 300

Asp Glu Tyr Gly Ala Lys Thr Glu Lys Ala Gln Trp Leu Arg Phe His
305 310 315 320

Thr Gln Thr Ala Gly Val Ser Leu Thr Ala Gln Gln Pro Tyr Asn Asn
325 330 335

Val Val Arg Thr Ala Val Glu Ala Leu Ala Ala Val Leu Gly Gly Thr
340 345 350

Asn Ser Leu His Thr Asn Ala Leu Asp Glu Thr Leu Ala Leu Pro Ser
355 360 365

Glu Gln Ala Ala Glu Ile Ala Leu Arg Thr Gln Gln Val Leu Met Glu
370 375 380

Glu Thr Gly Val Ala Asn Val Ala Asp Pro Leu Gly Gly Ser Trp Tyr
385 390 395 400

Ile Glu Gln Leu Thr Asp Arg Ile Glu Ala Asp Ala Glu Lys Ile Phe
405 410 415

Glu Gln Ile Arg Glu Arg Gly Arg Arg Ala Cys Pro Asp Gly Gln His
420 425 430

Pro Ile Gly Pro Ile Thr Ser Gly Ile Leu Arg Gly Ile Glu Asp Gly
435 440 445

```
Trp Phe Thr Gly Glu Ile Ala Glu Ser Ala Phe Gln Tyr Gln Arg Ser
    450                 455                 460

Leu Glu Lys Gly Asp Lys Arg Val Val Gly Val Asn Cys Leu Glu Gly
465                 470                 475                 480

Ser Val Thr Gly Asp Leu Glu Ile Leu Arg Val Ser His Glu Val Glu
                485                 490                 495

Arg Glu Gln Val Arg Glu Leu Ala Gly Arg Lys Gly Arg Arg Asp Asp
            500                 505                 510

Ala Arg Val Arg Ala Ser Leu Asp Ala Met Leu Ala Ala Ala Arg Asp
        515                 520                 525

Gly Ser Asn Met Ile Ala Pro Met Leu Glu Ala Val Arg Ala Glu Ala
    530                 535                 540

Thr Leu Gly Glu Ile Cys Gly Val Leu Arg Asp Glu Trp Gly Val Tyr
545                 550                 555                 560

Val Glu Pro Pro Gly Phe
                565
```

<210> 96
<211> 4362
<212> DNA
<213> Streptomyces cinnamonensis

<400> 96

```
tgaggcgctg gatcgcctcg gagagcagct ggtaacggtc cgcgtggtac tcggccgggg      60
tgcagccgtc cacgatgtgc gggatcgcgt cgggctcgag gatcaccagg gcgggggcgt     120
cgccgatcgc gtcggcgaac gtgtccaccc agctccggta ggcctccgca ctggccgcgc     180
cgcccgcgga gtgctgaccg cagtcgcggt gcgggatgtt gtacgcgacg agtacggcgg     240
tgcggtcctc cttgaccgcg ccccgcgtcg ccttcgcgac gtcgggcgcc ggatcgtccc     300
cggccggcca cacggccatg gcccgttcgg agatgcgcct gagcgtctcg gcgtcctcgg     360
cgcggccctg ttcctcccac tgcctgacct ggcgcgcggc ggggctgtcg gggtcgaccc     420
agaaggtgcc ggcgggggc ccggcgctcg cggtggcggg cttgcgcacg gccgcctcct     480
ccttcgtgcc gtcggacccc gggtctgagg aggagcagcc tgccgggagc ccgagggcgg     540
cgagggccgc gagtgccgtg aacgtgcgga gcagccggtg catccagccc ccttgggcga     600
tggtgacagt gacggtcagt cagcccggca atcgttacat aaaggactat tcaagctctt     660
gtgccacacc gcctccggtg ccgagcgcga acccggcggg caccagagcc ccgccgcggc     720
cgcggagccg tacgtacgac cgaattgcga gacggggctg accaccatat gaccggcggg     780
```

taaggtcgat gccgtgccga agccgctcag cctccccttc gatcccatcg cccgcgccga 840
cgagctctgg aagcagcgct ggggatcggt cccggccatg ggcgcgatca cctcgatcat 900
gcgggcgcac cagatcctgc tcgccgaggt cgacgcggtc gtcaagccgt acggactgac 960
cttcgcgcgc tacgaggcgc tggtgctcct caccttctcg caggccggcg agttgccgat 1020
gtcgaagatc ggcgagcggc tcatggtgca cccgacctcg gtcacgaaca ccgtggaccg 1080
cctggtgaag tccggcctgg tcgacaagcg cccgaacccc aacgacggcc gcggcacgct 1140
cgcctccatc acggagaagg gccgcgaggt cgtcgaggcg gccacccgcg agctgatggc 1200
gatggacttc gggctcgggg tgtacgacgc ggaggagtgc ggggagatct tcgcgatgct 1260
gcggcccctg cgggtggcgg cgcgcgattt cgaggagcag tagggcccgc ccggtgagaa 1320
gtgggatcgg gtcgtcccgg tacgggcggg ggcggcgaag atcgcgtgaa aagggcggtt 1380
acgctcgtag ccatgaaacg cagcgtgctg acccgctacc gggtgatggc ctacgtcacc 1440
gccgtcatgc tcctcatcct gtgcgcctgc atggtggcca agtacggctt cgacaagggc 1500
gagggtctga ccctcgtcgt gtcgcaggtg cacggcgtgc tctacatcat ctacctgatc 1560
ttcgccttcg acctgggctc caaggcgaag tggccgttcg gcaagctgct ctgggtgctg 1620
gtctcgggca cgatcccgac cgccgccttc ttcgtcgagc gcaaggtcgc ccgtgacgtc 1680
gagccgctga tcgccgacgg ctccccggtc accgcgaagg cgtaacccgc accgccacgg 1740
acaggtccgt ggcggttggc catcgacttt tactaggacg tcctagtaaa ttcgatggta 1800
tggacgctga cgcgatcgag gaaggccgcc gacgctggca ggcccgttac gacaaggccc 1860
gcaagcgcga cgcggacttc accacgctct ccggggaccc cgtcgacccc gtctacggcc 1920
cccggcccgg ggacacgtac gacgggttcg agcggatcgg ctggccgggg gagtacccct 1980
tcacccgcgg gctctacgcc accgggtacc gcggccgcac ctggaccatc cgccagttcg 2040
ccggcttcgg caacgccgag cagacgaacg agcgctacaa gatgatcctg gccaacggcg 2100
gcggcggcct ctccgtcgcc ttcgacatgc cgaccctcat gggccgcgac tccgacgacc 2160
cgcgctcgct cggcgaggtc ggccactgcg gtgtcgccat cgactccgcc gccgacatgg 2220
aggtcctctt caaggacatc ccgctcggcg acgtcacgac gtccatgacc atcagcgggc 2280
ccgccgtgcc cgtcttctgc atgtacctcg tcgcggccga gcgccagggc gtcgacccgg 2340
ccgtcctcaa cggcacgctg cagaccgaca tcttcaagga gtacatcgcc cagaaggagt 2400
ggctcttcca gcccgagccg cacctgcgcc tcatcggcga cctgatggag cactgcgcgc 2460
gcgacatccc cgcgtacaag ccgctctcgg tctccggcta ccacatccgc gaggccgggg 2520
cgacggccgc gcaggagctc gcgtacaccc tcgcggacgg cttcgggtac gtggaactgg 2580
gcctctcgcg cggcctggac gtggacgtct tcgcgcccgg cctctccttc ttcttcgacg 2640

cgcacgtcga cttcttcgag gagatcgcga agttccgcgc cgcacgccgc atctgggcgc 2700
gctggctccg ggacgagtac ggagcgaaga ccgagaaggc acagtggctg cgcttccaca 2760
cgcagaccgc gggggtctcg ctcacggccc agcagccgta caacaacgtg gtgcggacgg 2820
cggtggaggc cctcgccgcg gtgctcggcg gcacgaactc cctgcacacc aacgctctcg 2880
acgagaccct tgccctcccc agcgagcagg ccgcggagat cgcgctgcgc acccagcagg 2940
tgctgatgga ggagaccggc gtcgccaacg tcgcggaccc gctgggcggc tcctggtaca 3000
tcgagcagct caccgaccgc atcgaggccg acgccgagaa gatcttcgag cagatcaggg 3060
agcgggggcg gcgggcctgc cccgacgggc agcacccgat cgggccgatc acctccggca 3120
tcctgcgcgg catcgaggac ggctggttca ccggcgagat cgccgagtcc gccttccagt 3180
accagcggtc cctggagaag ggcgacaagc gggtcgtcgg cgtcaactgc ctcgaaggct 3240
ccgtcaccgg cgacctggag atcctgcgcg tcagccacga ggtcgagcgc gagcaggtgc 3300
gggagcttgc ggggcgcaag gggcggcgtg acgatgcgcg ggtgcgggcc tcgctcgacg 3360
cgatgctcgc cgctgcgcgg gacgggtcga acatgattgc cccccatgctg gaggcggtgc 3420
gggccgaggc gaccctcggg gagatctgcg gggtgcttcg cgatgagtgg ggggtctacg 3480
tggagccgcc cgggttctga gggcgcgctc cctttgcctg cgggtctgct gtggctggtc 3540
gcgcagttcc ccgcacccct gaaagacccc ggcgctttcc cttcctggct cgcctcgtcg 3600
ctgtctgcgg ggccgtgggg gctggtcgcg cagttccccg cgcccctgcc cgcacctgcg 3660
ccccgccgcc tgcatgccgc ccccaccctg acgggggcgt tcggggccca ccctgacggg 3720
tgcggtcggg gcgtgccggg gtcttttagg ggcgcgggga actgcgcgag caacccccac 3780
ccacccgcag gtgcacgcgg agcggcggac gccccgcaga cgggggcaaa acgggcggag 3840
tgcccccgcc cgccgggcgg cgcgaattcg taggtttaag gggcaggggt cagggcaggc 3900
gccgagccgg tcaaccgccc ccgtcccagg agaccccgtg acctcgaccg gccacgcccg 3960
caccgccgcc atcgccatcg gagccgccac cgccaccgtc ctcggcgcgc tgctggtcgg 4020
cggctccggc gaggtgagtg cgagcccgcc gcccgagccc aaggtccagg acgacttcga 4080
ctccctcggc cccgaggtgc gcgccgcgaa gctctccgac gggcggacgg cccactactc 4140
ggacacgggc gacaaggacg gcaagccggc cctgttcatc ggcggcaccg gcacgagcgc 4200
ccgcgcctcc cacatgaccg acttcttccg ctcgacgcgc gaggacctgg gcctgcgcct 4260
catctccgtg gagcgcaacg gcttcggcga caccgcgttc gacgagaagc tgggcaccgc 4320
cgacttcgcg aaggacgccc tcgaagtcct cgaccggctc gg 4362

<210> 97
<211> 136
<212> PRT

<213> Streptomyces cinnamonensis

<400> 97

```
Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Val Val Asp Thr Ala Ile Gln Glu Asp Ala Asp Ala Ile Gly Leu
    50                  55                  60

Ser Ile Leu Ser Gly Ala His Asn Thr Leu Phe Ala Arg Val Leu Glu
65                  70                  75                  80

Leu Leu Lys Glu Arg Asp Ala Glu Asp Ile Lys Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Thr Ser Ile Val Glu Trp Val
        115                 120                 125

Arg Gly Asn Val Arg Gln Ala Val
    130                 135
```

<210> 98
<211> 1643
<212> DNA
<213> Streptomyces cinnamonensis

<400> 98

gtcgacctcc cgtttggcgc acggaaggga ggctctgtcc cccgtgtgcc ctagggggag 60
tcgtggtcga ggagtcggct gtgcgatggc gatcccggcc accgccctgc ggtgactccg 120
tgcccccgtt gcatcgccga tgcgcggtgt caccacgccg tgcggctgcc ggcgcggtgg 180
cccggcgtct cgttgcggct cccctcgcgc ctggtccgga tgcggagcgt gaacccctgg 240
gttacggacg ggcgcgcagc gaacgtgtcc cacgtgtgat ttccccctcg ctctccaccg 300
cgaaactgcc gcttgcgcga tgctggggat aacgttcgtt cacttccccg gccggtgcgg 360
tgcggggtat ctgtgccggg acagactttg tcggtacgga tatcggtaca tggaggcagt 420
gatgggtgtg gcagccgggc cgatccgcgt ggtggtcgcc aagccggggc tcgacgggca 480
cgatcgcggg gccaaggtga tcgcgcgggc gttgcgtgac gcgggtatgg aggtcatcta 540

caccgggctg caccagacgc ccgagcaggt ggtggacacc gcgatccagg aggacgccga 600
cgcgatcggc ctctccatcc tctccggagc gcacaacacg ctgttcgcgc gcgtgttgga 660
gctcttgaag gagcgggacg cggaggacat caaggtgttt ggtggcggca tcatcccgga 720
ggcggacatc gcgccgctga aggagaaggg cgtcgcggag atcttcacgc ccggggccac 780
caccacgtcg atcgtggagt gggttcgggg gaacgtgcga caggccgtct gaggcattcc 840
ccgtcgcccg tctgccgtgg tcggcgtcat atcggcggac atcgtctcgg tggacgtcat 900
ggcggcgggg ggagttcgtc gcgtatcgcc gcgcggaggc gcagggtggt gaccaggcgc 960
tggaacgctt ccgaccagta gctgcccgcg ccgggtgacg cgtcctccgc ttcgtcgggg 1020
accgcggtga gcgcttccag gcggaccgcc tcggccgggt ccagacagcg ttccgccagg 1080
cccatcactc cgctgaagct ccatgggtaa ctgcccgcgt cgcgcgcgat gttcagggcg 1140
tccaccacgg cccggccgag agggccggcc cagggcaccg cgcagacgcc gagcagttgg 1200
aacgcctccg acaggccgtg tgccgctatg aaccccgcca cccagtccgc gcgctcggcg 1260
gcaggcatgg aggcgagcag tttggcccgc tcggcgaggg acacggcgcc aggccccgcc 1320
gcgtcgggtg aggcgggggc gccgagcagc gctctggacc aggcgacgtc acgctggcgt 1380
acggccgcgc ggcaccatgc ggcgtgcagt tcgccccgcc agtcgtcggc caccgggagc 1440
gccacgatct ccgccggggt gcggttgccg agccggggcg gccaggtggc gagcggggcc 1500
gattccacga gctggccgag ccaccaggag cgctcgcccc ggccggtggg gggcttcggg 1560
acgacgccgt cccgctccat gcccgcgtcg cactcgtgcg gcgcctcgac ggtgagggtc 1620
ggcgtgctcg atgtgtggtc gac 1643

<210> 99
<211> 566
<212> PRT
<213> Streptomyces coelicolor

<400> 99

```
Met Asp Ala His Ala Ile Glu Glu Gly Arg Leu Arg Trp Gln Ala Arg
1               5                   10                  15

Tyr Asp Ala Ala Arg Lys Arg Asp Ala Asp Phe Thr Thr Leu Ser Gly
            20                  25                  30

Asp Pro Val Glu Pro Val Tyr Gly Pro Arg Pro Gly Asp Glu Tyr Glu
        35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
```

65 70 75 80

Ala Gly Phe Gly Asn Ala Glu Gln Thr Asn Glu Arg Tyr Lys Met Ile
85 90 95

Leu Arg Asn Gly Gly Gly Gly Leu Ser Val Ala Phe Asp Met Pro Thr
100 105 110

Leu Met Gly Arg Asp Ser Asp Asp Pro Arg Ser Leu Gly Glu Val Gly
115 120 125

His Cys Gly Val Ala Ile Asp Ser Ala Ala Asp Met Glu Val Leu Phe
130 135 140

Lys Asp Ile Pro Leu Gly Asp Val Thr Thr Ser Met Thr Ile Ser Gly
145 150 155 160

Pro Ala Val Pro Val Phe Cys Met Tyr Leu Val Ala Ala Glu Arg Gln
165 170 175

Gly Val Asp Ala Ser Val Leu Asn Gly Thr Leu Gln Thr Asp Ile Phe
180 185 190

Lys Glu Tyr Ile Ala Gln Lys Glu Trp Leu Phe Gln Pro Glu Pro His
195 200 205

Leu Arg Leu Ile Gly Asp Leu Met Glu Tyr Cys Ala Ala Gly Ile Pro
210 215 220

Ala Tyr Lys Pro Leu Ser Val Ser Gly Tyr His Ile Arg Glu Ala Gly
225 230 235 240

Ala Thr Ala Ala Gln Glu Leu Ala Tyr Thr Leu Ala Asp Gly Phe Gly
245 250 255

Tyr Val Glu Leu Gly Leu Ser Arg Gly Leu Asp Val Asp Val Phe Ala
260 265 270

Pro Gly Leu Ser Phe Phe Phe Asp Ala His Leu Asp Phe Phe Glu Glu
275 280 285

Ile Ala Lys Phe Arg Ala Ala Arg Arg Ile Trp Ala Arg Trp Met Arg
290 295 300

Asp Val Tyr Gly Ala Arg Thr Asp Lys Ala Gln Trp Leu Arg Phe His
305 310 315 320

```
Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Phe
385                 390                 395                 400

Ile Glu Gln Leu Thr Asp Arg Ile Glu Ala Asp Ala Glu Lys Ile Phe
                405                 410                 415

Glu Gln Ile Lys Glu Arg Gly Leu Arg Ala His Pro Asp Gly Gln His
            420                 425                 430

Pro Val Gly Pro Ile Thr Ser Gly Leu Leu Arg Gly Ile Glu Asp Gly
        435                 440                 445

Trp Phe Thr Gly Glu Ile Ala Glu Ser Ala Phe Arg Tyr Gln Gln Ser
    450                 455                 460

Leu Glu Lys Asp Asp Lys Lys Val Val Gly Val Asn Val His Thr Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Asp Ala Arg Asp Asp
            500                 505                 510

Ala Ala Val Arg Gly Ala Leu Asp Ala Met Leu Ala Ala Ala Arg Ser
        515                 520                 525

Gly Gly Asn Met Ile Gly Pro Met Leu Asp Ala Val Arg Ala Glu Ala
    530                 535                 540

Thr Leu Gly Glu Ile Cys Gly Val Leu Arg Asp Glu Trp Gly Val Tyr
545                 550                 555                 560

Thr Glu Pro Ala Gly Phe
                565
```

<210> 100
<211> 1701
<212> DNA
<213> Streptomyces coelicolor

<400> 100

atggacgctc atgccataga ggagggccgc cttcgctggc aggcccggta cgacgcggcg 60
cgcaagcgcg acgcggactt caccacgctc tccggagacc ccgtggagcc ggtgtacggg 120
ccccgccccg gggacgagta cgagggcttc gagcggatcg gctggccggg cgagtacccc 180
ttcacccgcg gcctgtatcc gaccgggtac cggggcgta cgtggaccat ccggcagttc 240
gccgggttcg gcaacgccga gcagaccaac gagcgctaca agatgatcct ccgcaacggc 300
ggcggcgggc tctcggtcgc cttcgacatg ccgaccctga tgggccgcga ctccgacgac 360
ccgcgctcgc tgggcgaggt cgggcactgc ggggtggcca tcgactcggc cgccgacatg 420
gaagtgctgt tcaaggacat cccgctcggg gacgtgacga cctccatgac gatcagcggg 480
cccgccgtgc ccgtgttctg catgtacctc gtcgccgccg agcgccaggg cgtcgacgca 540
tccgtgctca acggcacgct gcagaccgac atcttcaagg agtacatcgc ccagaaggag 600
tggctcttcc agcccgagcc ccacctccgg ctcatcggcg acctcatgga gtactgcgcg 660
gccggcatcc ccgcctacaa gccgctctcc gtctccggct accacatccg cgaggcgggc 720
gcgacggccg cgcaggagct ggcgtacacg ctcgccgacg gcttcggata cgtggagctg 780
ggcctcagcc gcgggctcga cgtggacgtc ttcgcgcccg gcctctcctt cttcttcgac 840
gcgcacctcg acttcttcga ggagatcgcc aagttccgcg cggcccgcag gatctgggcc 900
cgctggatgc gcgacgtgta cggcgcgcgg accgacaagg cccagtggct gcggttccac 960
acccagaccg ccggagtctc gctcaccgcg cagcagccgt acaacaacgt cgtacgcacc 1020
gcggtggagg cgctggcggc cgtgctcggc ggcaccaact ccctgcacac caacgcgctc 1080
gacgagaccc tcgccctgcc cagcgagcag gccgccgaga tcgccctgcg cacccagcag 1140
gtgctgatgg aggagaccgg cgtcgccaac gtcgccgacc cgctgggcgg ttcctggttc 1200
atcgagcagc tgaccgaccg catcgaggcc gacgccgaga agatcttcga gcagatcaag 1260
gagcgggggc tgcgcgccca ccccgacggg cagcaccccg tcggaccgat cacctccggc 1320
ctgctgcgcg gcatcgagga cggctggttc accggcgaga tcgccgagtc cgccttccgc 1380
taccagcagt ccttggagaa ggacgacaag aaggtggtcg gcgtcaacgt ccacaccggc 1440
tccgtcaccg gcgacctgga gatcctgcgg gtcagccacg aggtcgagcg cgagcaggtg 1500
cgggtcctgg gcgagcgcaa ggacgcccgg gacgacgccg ccgtgcgcgg cgccctggac 1560
gccatgctgg ccgcggcccg ctccggcggc aacatgatcg ggccgatgct ggacgcggtg 1620
cgcgcggagg cgacgctggg cgagatctgc ggtgtgctgc gcgacgagtg gggggtgtac 1680

acggaaccgg cggggttctg a 1701

<210> 101
<211> 138

<212> PRT
<213> Streptomyces coelicolor

<400> 101

```
Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Asp Thr Ala Ile Gln Glu Asp Ala Asp Ala Ile Gly Leu
    50                  55                  60

Ser Ile Leu Ser Gly Ala His Asn Thr Leu Phe Ala Ala Val Ile Glu
65                  70                  75                  80

Leu Leu Arg Glu Arg Asp Ala Ala Asp Ile Leu Val Phe Gly Gly Gly
                85                  90                  95

Ile Ile Pro Glu Ala Asp Ile Ala Pro Leu Lys Glu Lys Gly Val Ala
            100                 105                 110

Glu Ile Phe Thr Pro Gly Ala Thr Thr Ala Ser Ile Val Asp Trp Val
        115                 120                 125

Arg Ala Asn Val Arg Glu Pro Ala Gly Ala
    130                 135
```

<210> 102
<211> 417
<212> DNA
<213> Streptomyces coelicolor

<400> 102

```
atgggtgtgg cagccggtcc gatccgcgtg gtggtggcca agccggggct cgacggccac      60
gatcgcgggg ccaaggtgat cgcgagggcc ctgcgtgacg ccggtatgga ggtgatctac     120
accgggctcc accagacgcc cgagcagatc gtcgacaccg cgatccagga ggacgccgac     180
gcgatcgggc tgtccatcct ctccggtgcg cacaacacgc tcttcgccgc cgtgatcgag     240
ctgctccggg agcgggacgc cgcggacatc ctggtcttcg gcggcgggat catccccgag     300
gcggacatcg ccccgctgaa ggagaagggc gtcgcggaga tcttcacgcc cggcgccacc     360
```

acggcgtcca tcgtggactg ggtccgggcg aacgtgcggg agcccgcggg agcatag 417

<210> 103
<211> 566
<212> PRT
<213> Streptomyces avermitilis

<400> 103

```
Met Asp Ala Asp Ala Ile Glu Glu Gly Arg Arg Arg Trp Gln Ala Arg
1               5                   10                  15

Tyr Asp Ala Ser Arg Lys Arg Glu Ala Asp Phe Thr Thr Leu Ser Gly
            20                  25                  30

Asp Pro Val Glu Pro Ala Tyr Gly Pro Arg Pro Gly Asp Ala Tyr Glu
        35                  40                  45

Gly Phe Glu Arg Ile Gly Trp Pro Gly Glu Tyr Pro Phe Thr Arg Gly
    50                  55                  60

Leu Tyr Pro Thr Gly Tyr Arg Gly Arg Thr Trp Thr Ile Arg Gln Phe
65                  70                  75                  80

Ala Gly Phe Gly Asn Ala Glu Gln Thr Asn Glu Arg Tyr Lys Lys Ile
                85                  90                  95

Leu Ala Asn Gly Gly Gly Gly Leu Ser Val Ala Phe Asp Met Pro Thr
            100                 105                 110

Leu Met Gly Arg Asp Ser Asp Asp Arg Arg Ala Leu Gly Glu Val Gly
        115                 120                 125

His Cys Gly Val Ala Ile Asp Ser Ala Ala Asp Met Glu Val Leu Phe
    130                 135                 140

Lys Asp Ile Pro Leu Gly Asp Val Thr Thr Ser Met Thr Ile Ser Gly
145                 150                 155                 160

Pro Ala Val Pro Val Phe Cys Met Tyr Leu Val Ala Ala Glu Arg Gln
                165                 170                 175

Gly Val Asp Pro Ser Val Leu Asn Gly Thr Leu Gln Thr Asp Ile Phe
            180                 185                 190

Lys Glu Tyr Ile Ala Gln Lys Glu Trp Leu Phe Gln Pro Glu Pro His
        195                 200                 205
```

```
Leu Arg Leu Ile Gly Asp Leu Met Glu His Cys Ala Ser Lys Ile Pro
    210                 215                 220

Ala Tyr Lys Pro Leu Ser Val Ser Gly Tyr His Ile Arg Glu Ala Gly
225                 230                 235                 240

Ala Thr Ala Ala Gln Glu Leu Ala Tyr Thr Leu Ala Asp Gly Phe Gly
                245                 250                 255

Tyr Val Glu Leu Gly Leu Ser Arg Gly Leu Asp Val Asp Val Phe Ala
            260                 265                 270

Pro Gly Leu Ser Phe Phe Phe Asp Ala His Val Asp Phe Phe Glu Glu
        275                 280                 285

Ile Ala Lys Phe Arg Ala Ala Arg Arg Ile Trp Ala Arg Trp Leu Arg
    290                 295                 300

Asp Val Tyr Gly Ala Lys Ser Glu Lys Ala Gln Trp Leu Arg Phe His
305                 310                 315                 320

Thr Gln Thr Ala Gly Val Ser Leu Thr Ala Gln Gln Pro Tyr Asn Asn
                325                 330                 335

Val Val Arg Thr Ala Val Glu Ala Leu Ala Ala Val Leu Gly Gly Thr
            340                 345                 350

Asn Ser Leu His Thr Asn Ala Leu Asp Glu Thr Leu Ala Leu Pro Ser
        355                 360                 365

Glu Gln Ala Ala Glu Ile Ala Leu Arg Thr Gln Gln Val Leu Met Glu
    370                 375                 380

Glu Thr Gly Val Ala Asn Val Ala Asp Pro Leu Gly Gly Ser Trp Tyr
385                 390                 395                 400

Val Glu Gln Leu Thr Asp Arg Ile Glu Ala Asp Ala Glu Lys Ile Phe
                405                 410                 415

Glu Gln Ile Arg Glu Arg Gly Leu Arg Ala His Pro Asp Gly Arg His
            420                 425                 430

Pro Ile Gly Pro Ile Thr Ser Gly Ile Leu Arg Gly Ile Glu Asp Gly
        435                 440                 445

Trp Phe Thr Gly Glu Ile Ala Glu Ser Ala Phe Gln Tyr Gln Gln Ala
    450                 455                 460
```

```
Leu Glu Lys Gly Asp Lys Arg Val Val Gly Val Asn Val His His Gly
465                 470                 475                 480

Ser Val Thr Gly Asp Leu Glu Ile Leu Arg Val Ser His Glu Val Glu
                485                 490                 495

Arg Glu Gln Val Arg Val Leu Gly Glu Arg Lys Ser Gly Arg Asp Asp
            500                 505                 510

Thr Ala Val Thr Ala Ala Leu Asp Ala Met Leu Ala Ala Ala Arg Asp
        515                 520                 525

Gly Ser Asn Met Ile Ala Pro Met Leu Asp Ala Val Arg Ala Glu Ala
    530                 535                 540

Thr Leu Gly Glu Ile Cys Asp Val Leu Arg Glu Glu Trp Gly Val Tyr
545                 550                 555                 560

Thr Glu Pro Ala Gly Phe
                565
```

<210> 104
<211> 1701
<212> DNA
<213> Streptomyces avermitilis

<400> 104

tcagaaaccg gcgggctccg tgtagacccc ccactcctcc cggaggacat cgcagatctc 60
gcccagcgtg gcctccgcgc ggaccgcgtc cagcatcggg gcgatcatgt tcgacccgtc 120
gcgcgcggcg gcgagcatcg cgtccagggc cgcggttacg gccgtgtcgt cgcgccccga 180
cttccgctcg cccagcaccc gcacctgctc gcgctccacc tcgtggctga cgcgcaggat 240
ctccaggtcg cccgtcacgg acccgtggtg gacgttgacg ccgacgaccc gcttgtcgcc 300
cttctccagc gcctgctggt actggaaggc cgactcggcg atctccccgg tgaaccagcc 360
gtcctcgatg ccgcgcagga tgccggaggt gatgggcccg atcgggtgcc gcccgtccgg 420
gtgggcccgc agcccgcgct ccctgatctg ttcgaagatc ttctcggcgt cggcctcgat 480
ccggtcggtc agctgctcca cgtaccagga accgcccagc ggatcggcca cgttggcgac 540
gcccgtctcc tccatcagca cctgctgggt gcgcagggcg atctcggccg cctgctcgga 600
cggcagggcg agggtctcgt cgagggcgtt ggtgtgcagc gagttcgtcc cgccgagcac 660
cgcggcgagg gcctccacgg ccgtccgtac gacgttgttg tacggctgct gcgcggtgag 720
cgagacgccc gcggtctggg tgtggaagcg cagccactgc gccttctccg acttcgcccc 780
gtacacgtcc cgcagccagc gcgcccagat gcgccgcgcc gcacggaact tggcgatctc 840

ctcgaagaag tcgacgtgcg cgtcgaagaa gaaggagagc ccgggcgcga acacgtccac 900
gtccaggccg cggctcagcc ccagctccac gtatccgaaa ccgtcggcga gggtgtacgc 960
cagctcctgg gcggccgtgg caccggcctc ccggatgtgg tacccggaga cggacagcgg 1020
cttgtacgcg gggatcttcg aggcgcagtg ctccatcagg tcgccgatga gccgcagatg 1080
gggctcgggc tggaagagcc actccttctg cgcgatgtac tccttgaaga tgtcggtctg 1140
gagggtgccg ttgaggacgg aggggtcgac gccctgccgc tcggccgcga ccaggtacat 1200
gcagaagacg ggcacggcgg gcccgctgat cgtcatcgac gtcgtcacgt cacccagcgg 1260
gatgtccttg aacaggacct ccatgtcggc cgccgagtcg atcgcgaccc cgcagtgccc 1320
gacctcgccg agcgcgcggc ggtcgtcgga gtcgcgcccc atgagcgtcg gcatgtcgaa 1380
ggccacggac agcccaccgc cgccgttggc gaggatcttc ttgtagcgct cgttggtctg 1440
ctcggcgttg ccgaacccgg cgaactgccg gatggtccag gtccggcccc ggtagccggt 1500
cggatacaga ccgcgcgtga aggggtactc acccggccag ccgatccgct cgaaaccctc 1560
gtacgcgtcc ccgggccggg gcccgtacgc cggctccacg ggatcgccgg agagcgtggt 1620
gaaatcggcc tcgcgcttgc gtgaggcgtc gtagcgggcc tgccagcgtc ggcggccttc 1680
ctcgatggcg tcagcgtcca t 1701

<210> 105
<211> 138

<212> PRT
<213> Streptomyces avermitilis

<400> 105

```
Met Gly Val Ala Ala Gly Pro Ile Arg Val Val Val Ala Lys Pro Gly
1               5                   10                  15

Leu Asp Gly His Asp Arg Gly Ala Lys Val Ile Ala Arg Ala Leu Arg
            20                  25                  30

Asp Ala Gly Met Glu Val Ile Tyr Thr Gly Leu His Gln Thr Pro Glu
        35                  40                  45

Gln Ile Val Gly Thr Ala Ile Gln Glu Asp Ala Asp Ala Ile Gly Leu
    50                  55                  60

Ser Ile Leu Ser Gly Ala His Asn Thr Leu Phe Ala Ala Val Ile Asp
65                  70                  75                  80

Leu Leu Lys Glu Arg Asp Ala Glu Asp Ile Lys Val Phe Gly Gly Gly
                85                  90                  95

Ile Ile Pro Glu Ala Asp Ile Ala Pro Leu Lys Glu Lys Gly Val Ala

            100                 105                 110

Glu Ile Phe Thr Pro Gly Ala Thr Thr Ala Ser Ile Val Glu Trp Val
        115                 120                 125

Arg Ala Asn Val Arg Gln Pro Ala Gly Ala
    130                 135
```

<210> 106
<211> 1701
<212> DNA
<213> Streptomyces avermitilis

<400> 106

tcagaaaccg gcgggctccg tgtagacccc ccactcctcc cggaggacat cgcagatctc 60
gcccagcgtg gcctccgcgc ggaccgcgtc cagcatcggg gcgatcatgt tcgacccgtc 120
gcgcgcggcg gcgagcatcg cgtccagggc cgcggttacg gccgtgtcgt cgcgccccga 180
cttccgctcg cccagcaccc gcacctgctc gcgctccacc tcgtggctga cgcgcaggat 240
ctccaggtcg cccgtcacgg acccgtggtg gacgttgacg ccgacgaccc gcttgtcgcc 300
cttctccagc gcctgctggt actggaaggc cgactcggcg atctccccgg tgaaccagcc 360
gtcctcgatg ccgcgcagga tgccggaggt gatgggcccg atcgggtgcc gcccgtccgg 420
gtgggcccgc agcccgcgct ccctgatctg ttcgaagatc ttctcggcgt cggcctcgat 480
ccggtcggtc agctgctcca cgtaccagga accgcccagc ggatcggcca cgttggcgac 540
gcccgtctcc tccatcagca cctgctgggt gcgcagggcg atctcggccg cctgctcgga 600
cggcagggcg agggtctcgt cgagggcgtt ggtgtgcagc gagttcgtcc cgccgagcac 660
cgcggcgagg gcctccacgg ccgtccgtac gacgttgttg tacggctgct gcgcggtgag 720
cgagacgccc gcggtctggg tgtggaagcg cagccactgc gccttctccg acttcgcccc 780
gtacacgtcc cgcagccagc gcgcccagat gcgccgcgcc gcacggaact tggcgatctc 840
ctcgaagaag tcgacgtgcg cgtcgaagaa gaaggagagc ccgggcgcga acacgtccac 900
gtccaggccg cggctcagcc ccagctccac gtatccgaaa ccgtcggcga gggtgtacgc 960
cagctcctgg gcggccgtgg caccggcctc ccggatgtgg tacccggaga cggacagcgg 1020
cttgtacgcg gggatcttcg aggcgcagtg ctccatcagg tcgccgatga gccgcagatg 1080
gggctcgggc tggaagagcc actccttctg cgcgatgtac tccttgaaga tgtcggtctg 1140
gagggtgccg ttgaggacgg aggggtcgac gccctgccgc tcggccgcga ccaggtacat 1200
gcagaagacg ggcacggcgg gcccgctgat cgtcatcgac gtcgtcacgt cacccagcgg 1260
gatgtccttg aacaggacct ccatgtcggc cgccgagtcg atcgcgaccc cgcagtgccc 1320
gacctcgccg agcgcgcggc ggtcgtcgga gtcgcgcccc atgagcgtcg gcatgtcgaa 1380
ggccacggac agcccaccgc cgccgttggc gaggatcttc ttgtagcgct cgttggtctg 1440
ctcggcgttg ccgaacccgg cgaactgccg gatggtccag gtccggcccc ggtagccggt 1500
cggatacaga ccgcgcgtga aggggtactc acccggccag ccgatccgct cgaaaccctc 1560
gtacgcgtcc ccgggccggg gcccgtacgc cggctccacg ggatcgccgg agagcgtggt 1620
gaaatcggcc tcgcgcttgc gtgaggcgtc gtagcgggcc tgccagcgtc ggcggccttc 1680
ctcgatggcg tcagcgtcca t 1701

<210> 107
<211> 139

<212> PRT
<213> Saccharomyces cerevisiae

<400> 107

```
Met Ser Glu Ile Thr Leu Gly Lys Tyr Leu Phe Glu Arg Leu Lys Gln
1               5                   10                  15

Val Asn Val Asn Thr Val Phe Gly Leu Pro Gly Asp Phe Asn Leu Ser
            20                  25                  30

Leu Leu Asp Lys Ile Tyr Glu Val Glu Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ala Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Ile
    130                 135
```

<210> 108
<211> 1689
<212> DNA
<213> Saccharomyces cerevisiae

<400> 108

```
atgtctgaaa ttactttggg taaatatttg ttcgaaagat taaagcaagt caacgttaac        60
```

```
accgttttcg gtttgccagg tgacttcaac ttgtccttgt tggacaagat ctacgaagtt      120
gaaggtatga gatgggctgg taacgccaac gaattgaacg ctgcttacgc cgctgatggt      180
tacgctcgta tcaagggtat gtcttgtatc atcaccacct tcggtgtcgg tgaattgtct      240
gctttgaacg gtattgccgg ttcttacgct gaacacgtcg gtgttttgca cgttgttggt      300
gtcccatcca tctctgctca agctaagcaa ttgttgttgc accacacctt gggtaacggt      360
gacttcactg ttttccacag aatgtctgcc aacatttctg aaaccactgc tatgatcact      420
gacattgcta ccgccccagc tgaaattgac agatgtatca gaaccactta cgtcacccaa      480
agaccagtct acttaggttt gccagctaac ttggtcgact tgaacgtccc agctaagttg      540
ttgcaaactc caattgacat gtctttgaag ccaaacgatg ctgaatccga aaaggaagtc      600
attgacacca tcttggcttt ggtcaaggat gctaagaacc cagttatctt ggctgatgct      660
tgttgttcca gacacgacgt caaggctgaa actaagaagt tgattgactt gactcaattc      720
ccagctttcg tcaccccaat gggtaagggt tccattgacg aacaacaccc aagatacggt      780
ggtgtttacg tcggtacctt gtccaagcca gaagttaagg aagccgttga atctgctgac      840
ttgattttgt ctgtcggtgc tttgttgtct gatttcaaca ccggttcttt ctcttactct      900
tacaagacca agaacattgt cgaattccac tccgaccaca tgaagatcag aaacgccact      960
ttcccaggtg tccaaatgaa attcgttttg caaaagttgt tgaccactat tgctgacgcc     1020
gctaagggtt acaagccagt tgctgtccca gctagaactc cagctaacgc tgctgtccca     1080
gcttctaccc cattgaagca agaatggatg tggaaccaat tgggtaactt cttgcaagaa     1140
ggtgatgttg tcattgctga aaccggtacc tccgctttcg gtatcaacca aaccactttc     1200
ccaaacaaca cctacggtat ctctcaagtc ttatggggtt ccattggttt caccactggt     1260
gctaccttgg gtgctgcttt cgctgctgaa gaaattgatc caaagaagag agttatctta     1320
ttcattggtg acggttcttt gcaattgact gttcaagaaa tctccaccat gatcagatgg     1380
ggcttgaagc catacttgtt cgtcttgaac aacgatggtt acaccattga aaagttgatt     1440
cacggtccaa aggctcaata caacgaaatt caaggttggg accacctatc cttgttgcca     1500
actttcggtg ctaaggacta tgaaacccac agagtcgcta ccaccggtga atgggacaag     1560
ttgacccaag acaagtcttt caacgacaac tctaagatca gaatgattga aatcatgttg     1620
ccagtcttcg atgctccaca aaacttggtt gaacaagcta agttgactgc tgctaccaac     1680
gctaagcaa                                                             1689
```

<210> 109
<211> 563
<212> PRT
<213> Saccharomyces cerevisiae

<400> 109

```
Met Ser Glu Ile Thr Leu Gly Lys Tyr Leu Phe Glu Arg Leu Ser Gln
1               5                   10                  15

Val Asn Cys Asn Thr Val Phe Gly Leu Pro Gly Asp Phe Asn Leu Ser
            20                  25                  30

Leu Leu Asp Lys Leu Tyr Glu Val Lys Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ser Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Ile Thr Asp Ile Ala Asn
    130                 135                 140

Ala Pro Ala Glu Ile Asp Arg Cys Ile Arg Thr Thr Tyr Thr Thr Gln
145                 150                 155                 160

Arg Pro Val Tyr Leu Gly Leu Pro Ala Asn Leu Val Asp Leu Asn Val
                165                 170                 175

Pro Ala Lys Leu Leu Glu Thr Pro Ile Asp Leu Ser Leu Lys Pro Asn
            180                 185                 190

Asp Ala Glu Ala Glu Ala Glu Val Val Arg Thr Val Val Glu Leu Ile
        195                 200                 205

Lys Asp Ala Lys Asn Pro Val Ile Leu Ala Asp Ala Cys Ala Ser Arg
    210                 215                 220

His Asp Val Lys Ala Glu Thr Lys Lys Leu Met Asp Leu Thr Gln Phe
225                 230                 235                 240

Pro Val Tyr Val Thr Pro Met Gly Lys Gly Ala Ile Asp Glu Gln His
```

245 250 255

Pro Arg Tyr Gly Gly Val Tyr Val Gly Thr Leu Ser Arg Pro Glu Val
260 265 270

Lys Lys Ala Val Glu Ser Ala Asp Leu Ile Leu Ser Ile Gly Ala Leu
275 280 285

Leu Ser Asp Phe Asn Thr Gly Ser Phe Ser Tyr Ser Tyr Lys Thr Lys
290 295 300

Asn Ile Val Glu Phe His Ser Asp His Ile Lys Ile Arg Asn Ala Thr
305 310 315 320

Phe Pro Gly Val Gln Met Lys Phe Ala Leu Gln Lys Leu Leu Asp Ala
325 330 335

Ile Pro Glu Val Val Lys Asp Tyr Lys Pro Val Ala Val Pro Ala Arg
340 345 350

Val Pro Ile Thr Lys Ser Thr Pro Ala Asn Thr Pro Met Lys Gln Glu
355 360 365

Trp Met Trp Asn His Leu Gly Asn Phe Leu Arg Glu Gly Asp Ile Val
370 375 380

Ile Ala Glu Thr Gly Thr Ser Ala Phe Gly Ile Asn Gln Thr Thr Phe
385 390 395 400

Pro Thr Asp Val Tyr Ala Ile Val Gln Val Leu Trp Gly Ser Ile Gly
405 410 415

Phe Thr Val Gly Ala Leu Leu Gly Ala Thr Met Ala Ala Glu Glu Leu
420 425 430

Asp Pro Lys Lys Arg Val Ile Leu Phe Ile Gly Asp Gly Ser Leu Gln
435 440 445

Leu Thr Val Gln Glu Ile Ser Thr Met Ile Arg Trp Gly Leu Lys Pro
450 455 460

Tyr Ile Phe Val Leu Asn Asn Asn Gly Tyr Thr Ile Glu Lys Leu Ile
465 470 475 480

His Gly Pro His Ala Glu Tyr Asn Glu Ile Gln Gly Trp Asp His Leu
485 490 495

```
Ala Leu Leu Pro Thr Phe Gly Ala Arg Asn Tyr Glu Thr His Arg Val
            500                 505                 510

Ala Thr Thr Gly Glu Trp Glu Lys Leu Thr Gln Asp Lys Asp Phe Gln
        515                 520                 525

Asp Asn Ser Lys Ile Arg Met Ile Glu Val Met Leu Pro Val Phe Asp
    530                 535                 540

Ala Pro Gln Asn Leu Val Lys Gln Ala Gln Leu Thr Ala Ala Thr Asn
545                 550                 555                 560

Ala Lys Gln
```

<210> 110
<211> 1689
<212> DNA
<213> Saccharomyces cerevisiae

<400> 110

```
atgtctgaaa taaccttagg taaatattta tttgaaagat tgagccaagt caactgtaac        60
accgtcttcg gtttgccagg tgactttaac ttgtctcttt tggataagct ttatgaagtc       120
aaaggtatga gatgggctgg taacgctaac gaattgaacg ctgcctatgc tgctgatggt       180
tacgctcgta tcaagggtat gtcctgtatt attaccacct tcggtgttgg tgaattgtct       240
gctttgaatg gtattgccgg ttcttacgct gaacatgtcg gtgttttgca cgttgttggt       300
gttccatcca tctcttctca agctaagcaa ttgttgttgc atcatacctt gggtaacggt       360
gacttcactg ttttccacag aatgtctgcc aacatttctg aaaccactgc catgatcact       420
gatattgcta acgctccagc tgaaattgac agatgtatca gaaccaccta cactacccaa       480
agaccagtct acttgggttt gccagctaac ttggttgact tgaacgtccc agccaagtta       540
ttggaaactc caattgactt gtctttgaag ccaaacgacg ctgaagctga agctgaagtt       600
gttagaactg ttgttgaatt gatcaaggat gctaagaacc cagttatctt ggctgatgct       660
tgtgcttcta gacatgatgt caaggctgaa actaagaagt tgatggactt gactcaattc       720
ccagtttacg tcaccccaat gggtaagggt gctattgacg aacaacaccc aagatacggt       780
ggtgtttacg ttggtacctt gtctagacca gaagttaaga aggctgtaga atctgctgat       840
ttgatattgt ctatcggtgc tttgttgtct gatttcaata ccggttcttt ctcttactcc       900
tacaagacca aaaatatcgt tgaattccac tctgaccaca tcaagatcag aaacgccacc       960
ttcccaggtg ttcaaatgaa atttgccttg caaaaattgt tggatgctat tccagaagtc      1020
gtcaaggact acaaacctgt tgctgtccca gctagagttc caattaccaa gtctactcca      1080
gctaacactc caatgaagca agaatggatg tggaaccatt tgggtaactt cttgagagaa      1140
ggtgatattg ttattgctga aaccggtact tccgccttcg gtattaacca aactactttc      1200
ccaacagatg tatacgctat cgtccaagtc ttgtggggtt ccattggttt cacagtcggc      1260
gctctattgg gtgctactat ggccgctgaa gaacttgatc caaagaagag agttatttta      1320
ttcattggtg acggttctct acaattgact gttcaagaaa tctctaccat gattagatgg      1380
ggtttgaagc catacatttt tgtcttgaat aacaacggtt acaccattga aaaattgatt      1440
cacggtcctc atgccgaata taatgaaatt caaggttggg accacttggc cttattgcca      1500
acttttggtg ctagaaacta cgaaacccac agagttgcta ccactggtga atgggaaaag      1560
ttgactcaag acaaggactt ccaagacaac tctaagatta gaatgattga agttatgttg      1620
ccagtctttg atgctccaca aaacttggtt aaacaagctc aattgactgc cgctactaac      1680
gctaaacaa                                                              1689
```

<210> 111
<211> 533
<212> PRT

<213> Saccharomyces cerevisiae

<400> 111

```
Met Ser Glu Ile Thr Leu Gly Lys Tyr Leu Phe Glu Arg Leu Lys Gln
1               5                   10                  15

Val Asn Val Asn Thr Ile Phe Gly Leu Pro Gly Asp Phe Asn Leu Ser
            20                  25                  30

Leu Leu Asp Lys Ile Tyr Glu Val Asp Gly Leu Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Leu Ser Val Leu Val Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Ile Ser Ala Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ser Met Ile Thr Asp Ile Ala Thr
    130                 135                 140
```

```
Ala Pro Ser Glu Ile Asp Arg Leu Ile Arg Thr Thr Phe Ile Thr Gln
145                 150                 155                 160

Arg Pro Ser Tyr Leu Gly Leu Pro Ala Asn Leu Val Asp Leu Lys Val
                165                 170                 175

Pro Gly Ser Leu Leu Glu Lys Pro Ile Asp Leu Ser Leu Lys Pro Asn
            180                 185                 190

Asp Pro Glu Ala Glu Lys Glu Val Ile Asp Thr Val Leu Glu Leu Ile
        195                 200                 205

Gln Asn Ser Lys Asn Pro Val Ile Leu Ser Asp Ala Cys Ala Ser Arg
    210                 215                 220

His Asn Val Lys Lys Glu Thr Gln Lys Leu Ile Asp Leu Thr Gln Phe
225                 230                 235                 240

Pro Ala Phe Val Thr Pro Leu Gly Lys Gly Ser Ile Asp Glu Gln His
                245                 250                 255

Pro Arg Tyr Gly Gly Val Tyr Val Gly Thr Leu Ser Lys Gln Asp Val
            260                 265                 270

Lys Gln Ala Val Glu Ser Ala Asp Leu Ile Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Val Val Glu Phe His Ser Asp Tyr Val Lys Val Lys Asn Ala Thr
305                 310                 315                 320

Phe Leu Gly Val Gln Met Lys Phe Ala Leu Gln Asn Leu Leu Lys Val
                325                 330                 335

Ile Pro Asp Val Val Lys Gly Tyr Lys Ser Val Pro Val Pro Thr Lys
            340                 345                 350

Thr Pro Ala Asn Lys Gly Val Pro Ala Ser Thr Pro Leu Lys Gln Glu
        355                 360                 365

Trp Leu Trp Asn Glu Leu Ser Lys Phe Leu Gln Glu Gly Asp Val Ile
    370                 375                 380

Ile Ser Glu Thr Gly Thr Ser Ala Phe Gly Ile Asn Gln Thr Ile Phe
```

385 390 395 400

Pro Lys Asp Ala Tyr Gly Ile Ser Gln Val Leu Trp Gly Ser Ile Gly
405 410 415

Phe Thr Thr Gly Ala Thr Leu Gly Ala Ala Phe Ala Ala Glu Glu Ile
420 425 430

Asp Pro Asn Lys Arg Val Ile Leu Phe Ile Gly Asp Gly Ser Leu Gln
435 440 445

Leu Thr Val Gln Glu Ile Ser Thr Met Ile Arg Trp Gly Leu Lys Pro
450 455 460

Tyr Leu Phe Val Leu Asn Asn Asp Gly Tyr Thr Ile Glu Lys Leu Ile
465 470 475 480

His Gly Pro His Ala Glu Tyr Asn Glu Ile Gln Thr Trp Asp His Leu
485 490 495

Ala Leu Leu Pro Ala Phe Gly Ala Lys Lys Tyr Glu Asn His Lys Ile
500 505 510

Ala Thr Thr Gly Glu Trp Asp Ala Leu Thr Thr Asp Ser Glu Phe Gln
515 520 525

Lys Asn Ser Val Ile
530

<210> 112
<211> 1599
<212> DNA
<213> Saccharomyces cerevisiae

<400> 112

```
atgtctgaaa ttactcttgg aaaatactta tttgaaagat tgaagcaagt taatgttaac        60
accatttttg ggctaccagg cgacttcaac ttgtccctat tggacaagat ttacgaggta       120
gatggattga gatgggctgg taatgcaaat gagctgaacg ccgcctatgc cgccgatggt       180
tacgcacgca tcaagggttt atctgtgctg gtaactactt ttggcgtagg tgaattatcc       240
gccttgaatg gtattgcagg atcgtatgca gaacacgtcg gtgtactgca tgttgttggt       300
gtcccctcta tctccgctca ggctaagcaa ttgttgttgc atcatacctt gggtaacggt       360
gattttaccg tttttcacag aatgtccgcc aatatctcag aaactacatc aatgattaca       420
gacattgcta cagccccttc agaaatcgat aggttgatca ggacaacatt tataacacaa       480
aggcctagct acttggggtt gccagcgaat ttggtagatc taaaggttcc tggttctctt       540
ttggaaaaac cgattgatct atcattaaaa cctaacgatc ccgaagctga aaaggaagtt       600
attgataccg tactagaatt gatccagaat tcgaaaaacc ctgttatact atcggatgcc       660
tgtgcttcta ggcacaacgt taaaaaagaa acccagaagt taattgattt gacgcaattc       720
ccagcttttg tgacacctct aggtaaaggg tcaatagatg aacagcatcc cagatatggc       780
ggtgtttatg tgggaacgct gtccaaacaa gacgtgaaac aggccgttga gtcggctgat       840
ttgatccttt cggtcggtgc tttgctctct gattttaaca caggttcgtt ttcctactcc       900
tacaagacta aaaatgtagt ggagtttcat tccgattacg taaaggtgaa gaacgctacg       960
ttcctcggtg tacaaatgaa atttgcacta caaaacttac tgaaggttat tcccgatgtt      1020
gttaagggct acaagagcgt tcccgtacca accaaaactc ccgcaaacaa aggtgtacct      1080
gctagcacgc ccttgaaaca agagtggttg tggaacgaat tgtccaaatt cttgcaagaa      1140
ggtgatgtta tcatttccga gaccggcacg tctgccttcg gtatcaatca aactatcttt      1200
cctaaggacg cctacggtat ctcgcaggtg ttgtgggggt ccatcggttt tacaacagga      1260
gcaactttag gtgctgcctt tgccgctgag gagattgacc ccaacaagag agtcatctta      1320
ttcataggtg acgggtcttt gcagttaacc gtccaagaaa tctccaccat gatcagatgg      1380
gggttaaagc cgtatctttt tgtccttaac aacgacggct acactatcga aaagctgatt      1440
catgggcctc acgcagagta caacgaaatc cagacctggg atcacctcgc cctgttgccc      1500
gcatttggtg cgaaaaagta cgaaaatcac aagatcgcca ctacgggtga gtgggatgcc      1560
ttaaccactg attcagagtt ccagaaaaac tcggtgatc                             1599
```

<210> 113
<211> 564
<212> PRT
<213> Candida glabrata

<400> 113

```
Met Ser Glu Ile Thr Leu Gly Arg Tyr Leu Phe Glu Arg Leu Asn Gln
1               5                   10                  15

Val Asp Val Lys Thr Ile Phe Gly Leu Pro Gly Asp Phe Asn Leu Ser
            20                  25                  30

Leu Leu Asp Lys Ile Tyr Glu Val Glu Gly Met Arg Trp Ala Gly Asn
        35                  40                  45

Ala Asn Glu Leu Asn Ala Ala Tyr Ala Ala Asp Gly Tyr Ala Arg Ile
    50                  55                  60

Lys Gly Met Ser Cys Ile Ile Thr Thr Phe Gly Val Gly Glu Leu Ser
65                  70                  75                  80
```

```
Ala Leu Asn Gly Ile Ala Gly Ser Tyr Ala Glu His Val Gly Val Leu
                85                  90                  95

His Val Val Gly Val Pro Ser Ile Ser Ser Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Val Thr Asp Ile Ala Thr
    130                 135                 140

Ala Pro Ala Glu Ile Asp Arg Cys Ile Arg Thr Thr Tyr Ile Thr Gln
145                 150                 155                 160

Arg Pro Val Tyr Leu Gly Leu Pro Ala Asn Leu Val Asp Leu Lys Val
                165                 170                 175

Pro Ala Lys Leu Leu Glu Thr Pro Ile Asp Leu Ser Leu Lys Pro Asn
            180                 185                 190

Asp Pro Glu Ala Glu Thr Glu Val Val Asp Thr Val Leu Glu Leu Ile
        195                 200                 205

Lys Ala Ala Lys Asn Pro Val Ile Leu Ala Asp Ala Cys Ala Ser Arg
    210                 215                 220

His Asp Val Lys Ala Glu Thr Lys Lys Leu Ile Asp Ala Thr Gln Phe
225                 230                 235                 240

Pro Ser Phe Val Thr Pro Met Gly Lys Gly Ser Ile Asp Glu Gln His
                245                 250                 255

Pro Arg Phe Gly Gly Val Tyr Val Gly Thr Leu Ser Arg Pro Glu Val
            260                 265                 270

Lys Glu Ala Val Glu Ser Ala Asp Leu Ile Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp Tyr Ile Lys Ile Arg Asn Ala Thr
305                 310                 315                 320

Phe Pro Gly Val Gln Met Lys Phe Ala Leu Gln Lys Leu Leu Asn Ala
                325                 330                 335
```

Val Pro Glu Ala Ile Lys Gly Tyr Lys Pro Val Pro Val Pro Ala Arg
340 345 350

Val Pro Glu Asn Lys Ser Cys Asp Pro Ala Thr Pro Leu Lys Gln Glu
355 360 365

Trp Met Trp Asn Gln Val Ser Lys Phe Leu Gln Glu Gly Asp Val Val
370 375 380

Ile Thr Glu Thr Gly Thr Ser Ala Phe Gly Ile Asn Gln Thr Pro Phe
385 390 395 400

Pro Asn Asn Ala Tyr Gly Ile Ser Gln Val Leu Trp Gly Ser Ile Gly
405 410 415

Phe Thr Thr Gly Ala Cys Leu Gly Ala Ala Phe Ala Ala Glu Glu Ile
420 425 430

Asp Pro Lys Lys Arg Val Ile Leu Phe Ile Gly Asp Gly Ser Leu Gln
435 440 445

Leu Thr Val Gln Glu Ile Ser Thr Met Ile Arg Trp Gly Leu Lys Pro
450 455 460

Tyr Leu Phe Val Leu Asn Asn Asp Gly Tyr Thr Ile Glu Arg Leu Ile
465 470 475 480

His Gly Glu Lys Ala Gly Tyr Asn Asp Ile Gln Asn Trp Asp His Leu
485 490 495

Ala Leu Leu Pro Thr Phe Gly Ala Lys Asp Tyr Glu Asn His Arg Val
500 505 510

Ala Thr Thr Gly Glu Trp Asp Lys Leu Thr Gln Asp Lys Glu Phe Asn
515 520 525

Lys Asn Ser Lys Ile Arg Met Ile Glu Val Met Leu Pro Val Met Asp
530 535 540

Ala Pro Thr Ser Leu Ile Glu Gln Ala Lys Leu Thr Ala Ser Ile Asn
545 550 555 560

Ala Lys Gln Glu

<210> 114
<211> 1692
<212> DNA

<213> Candida glabrata

<400> 114

```
atgtctgaga ttactttggg tagatacttg ttcgagagat tgaaccaagt cgacgttaag      60
accatcttcg gtttgccagg tgacttcaac ttgtccctat tggacaagat ctacgaagtt     120
gaaggtatga gatgggctgg taacgctaac gaattgaacg ctgcttacgc tgctgacggt     180
tacgctagaa tcaagggtat gtcctgtatc atcaccacct tcggtgtcgg tgaattgtct     240
gccttgaacg gtattgccgg ttcttacgct gaacacgtcg gtgtcttgca cgtcgtcggt     300
gtcccatcca tctcctctca agctaagcaa ttgttgttgc accacacctt gggtaacggt     360
gacttcactg tcttccacag aatgtccgct aacatctctg agaccaccgc tatggtcact     420
gacatcgcta ccgctccagc tgagatcgac agatgtatca gaaccaccta catcacccaa     480
agaccagtct acttgggtct accagctaac ttggtcgacc taaaggtccc agccaagctt     540
ttggaaaccc caattgactt gtccttgaag ccaaacgacc cagaagccga aactgaagtc     600
gttgacaccg tcttggaatt gatcaaggct gctaagaacc cagttatctt ggctgatgct     660
tgtgcttcca gacacgacgt caaggctgaa accaagaagt tgattgacgc cactcaattc     720
ccatccttcg ttaccccaat gggtaagggt tccatcgacg aacaacaccc aagattcggt     780
ggtgtctacg tcggtacctt gtccagacca gaagttaagg aagctgttga atccgctgac     840
ttgatcttgt ctgtcggtgc tttgttgtcc gatttcaaca ctggttcttt ctcttactct     900
tacaagacca agaacatcgt cgaattccac tctgactaca tcaagatcag aaacgctacc     960
ttcccaggtg tccaaatgaa gttcgctttg caaaagttgt tgaacgccgt cccagaagct    1020
atcaagggtt acaagccagt ccctgtccca gctagagtcc cagaaaacaa gtcctgtgac    1080
ccagctaccc cattgaagca agaatggatg tggaaccaag tttccaagtt cttgcaagaa    1140
ggtgatgttg ttatcactga aaccggtacc tccgcttttg gtatcaacca aaccccattc    1200
ccaaacaacg cttacggtat ctcccaagtt ctatggggtt ccatcggttt caccaccggt    1260
gcttgtttgg gtgccgcttt cgctgctgaa gaaatcgacc caaagaagag agttatcttg    1320
ttcattggtg acggttcttt gcaattgact gtccaagaaa tctccaccat gatcagatgg    1380
ggcttgaagc catacttgtt cgtcttgaac aacgacggtt acaccatcga aagattgatt    1440
cacggtgaaa aggctggtta caacgacatc caaaactggg accacttggc tctattgcca    1500
accttcggtg ctaaggacta cgaaaaccac agagtcgcca ccaccggtga atgggacaag    1560
ttgacccaag acaaggaatt caacaagaac tccaagatca gaatgatcga agttatgttg    1620
ccagttatgg acgctccaac ttccttgatt gaacaagcta agttgaccgc ttccatcaac    1680
gctaagcaag aa                                                        1692
```

<210> 115
<211> 596
<212> PRT
<213> Pichia stipitis

<400> 115

```
Met Ala Glu Val Ser Leu Gly Arg Tyr Leu Phe Glu Arg Leu Tyr Gln
1               5                   10                  15

Leu Gln Val Gln Thr Ile Phe Gly Val Pro Gly Asp Phe Asn Leu Ser
            20                  25                  30

Leu Leu Asp Lys Ile Tyr Glu Val Glu Asp Ala His Gly Lys Asn Ser
        35                  40                  45

Phe Arg Trp Ala Gly Asn Ala Asn Glu Leu Asn Ala Ser Tyr Ala Ala
    50                  55                  60

Asp Gly Tyr Ser Arg Val Lys Arg Leu Gly Cys Leu Val Thr Thr Phe
65                  70                  75                  80

Gly Val Gly Glu Leu Ser Ala Leu Asn Gly Ile Ala Gly Ser Tyr Ala
                85                  90                  95

Glu His Val Gly Leu Leu His Val Val Gly Val Pro Ser Ile Ser Ser
            100                 105                 110

Gln Ala Lys Gln Leu Leu Leu His His Thr Leu Gly Asn Gly Asp Phe
        115                 120                 125

Thr Val Phe His Arg Met Ser Asn Asn Ile Ser Gln Thr Thr Ala Phe
    130                 135                 140

Ile Ser Asp Ile Asn Ser Ala Pro Ala Glu Ile Asp Arg Cys Ile Arg
145                 150                 155                 160

Glu Ala Tyr Val Lys Gln Arg Pro Val Tyr Ile Gly Leu Pro Ala Asn
                165                 170                 175

Leu Val Asp Leu Asn Val Pro Ala Ser Leu Leu Glu Ser Pro Ile Asn
            180                 185                 190

Leu Ser Leu Glu Lys Asn Asp Pro Glu Ala Gln Asp Glu Val Ile Asp
        195                 200                 205

Ser Val Leu Asp Leu Ile Lys Lys Ser Ser Asn Pro Ile Ile Leu Val
    210                 215                 220
```

```
Asp Ala Cys Ala Ser Arg His Asp Cys Lys Ala Glu Val Thr Gln Leu
225                 230                 235                 240

Ile Glu Gln Thr Gln Phe Pro Val Phe Val Thr Pro Met Gly Lys Gly
                245                 250                 255

Thr Val Asp Glu Gly Gly Val Asp Gly Glu Leu Leu Glu Asp Asp Pro
            260                 265                 270

His Leu Ile Ala Lys Val Ala Ala Arg Leu Ser Ala Gly Lys Asn Ala
        275                 280                 285

Ala Ser Arg Phe Gly Gly Val Tyr Val Gly Thr Leu Ser Lys Pro Glu
    290                 295                 300

Val Lys Asp Ala Val Glu Ser Ala Asp Leu Ile Leu Ser Val Gly Ala
305                 310                 315                 320

Leu Leu Ser Asp Phe Asn Thr Gly Ser Phe Ser Tyr Ser Tyr Arg Thr
                325                 330                 335

Lys Asn Ile Val Glu Phe His Ser Asp Tyr Thr Lys Ile Arg Gln Ala
            340                 345                 350

Thr Phe Pro Gly Val Gln Met Lys Glu Ala Leu Gln Glu Leu Asn Lys
        355                 360                 365

Lys Val Ser Ser Ala Ala Ser His Tyr Glu Val Lys Pro Val Pro Lys
    370                 375                 380

Ile Lys Leu Ala Asn Thr Pro Ala Thr Arg Glu Val Lys Leu Thr Gln
385                 390                 395                 400

Glu Trp Leu Trp Thr Arg Val Ser Ser Trp Phe Arg Glu Gly Asp Ile
                405                 410                 415

Ile Ile Thr Glu Thr Gly Thr Ser Ser Phe Gly Ile Val Gln Ser Arg
            420                 425                 430

Phe Pro Asn Asn Thr Ile Gly Ile Ser Gln Val Leu Trp Gly Ser Ile
        435                 440                 445

Gly Phe Ser Val Gly Ala Thr Leu Gly Ala Ala Met Ala Ala Gln Glu
    450                 455                 460

Leu Asp Pro Asn Lys Arg Thr Ile Leu Phe Val Gly Asp Gly Ser Leu
465                 470                 475                 480
```

```
Gln Leu Thr Val Gln Glu Ile Ser Thr Ile Ile Arg Trp Gly Thr Thr
                485                 490                 495

Pro Tyr Leu Phe Val Leu Asn Asn Asp Gly Tyr Thr Ile Glu Arg Leu
            500                 505                 510

Ile His Gly Val Asn Ala Ser Tyr Asn Asp Ile Gln Pro Trp Gln Asn
        515                 520                 525

Leu Glu Ile Leu Pro Thr Phe Ser Ala Lys Asn Tyr Asp Ala Val Arg
    530                 535                 540

Ile Ser Asn Ile Gly Glu Ala Glu Asp Ile Leu Lys Asp Lys Glu Phe
545                 550                 555                 560

Gly Lys Asn Ser Lys Ile Arg Leu Ile Glu Val Met Leu Pro Arg Leu
                565                 570                 575

Asp Ala Pro Ser Asn Leu Ala Lys Gln Ala Ala Ile Thr Ala Ala Thr
            580                 585                 590

Asn Ala Glu Ala
        595
```

<210> 116
<211> 1788
<212> DNA
<213> Pichia stipitis

<400> 116

atggctgaag tctcattagg aagatatctc ttcgagagat tgtaccaatt gcaagtgcag 60
accatcttcg gtgtccctgg tgatttcaac ttgtcgcttt tggacaagat ctacgaagtg 120
gaagatgccc atggcaagaa ttcgtttaga tgggctggta atgccaacga attgaatgca 180
tcgtacgctg ctgacggtta ctcgagagtc aagcgtttag ggtgtttggt cactaccttt 240
ggtgtcggtg aattgtctgc tttgaatggt attgccggtt cttatgccga acatgttggt 300
ttgcttcatg tcgtaggtgt tccatcgatt tcctcgcaag ctaagcaatt gttacttcac 360
cacactttgg gtaatggtga tttcactgtt ttccatagaa tgtccaacaa catttctcag 420
accacagcct ttatctccga tatcaactcg gctccagctg aaattgatag atgtatcaga 480
gaggcctacg tcaaacaaag accagtttat atcgggttac cagctaactt agttgatttg 540
aatgttccgg cctctttgct tgagtctcca atcaacttgt cgttggaaaa gaacgaccca 600
gaggctcaag atgaagtcat tgactctgtc ttagacttga tcaaaaagtc gctgaaccca 660
atcatcttgg tcgatgcctg tgcctcgaga catgactgta aggctgaagt tactcagttg 720
attgaacaaa cccaattccc agtatttgtc actccaatgg gtaaaggtac cgttgatgag 780
ggtggtgtag acggagaatt gttagaagat gatcctcatt tgattgccaa ggtcgctgct 840
aggttgtctg ctggcaagaa cgctgcctct agattcggag gtgtttatgt cggaaccttg 900
tcgaagcccg aagtcaagga cgctgtagag agtgcagatt tgattttgtc tgtcggtgcc 960
cttttgtctg atttcaacac tggttcattt tcctactcct acagaaccaa gaacatcgtc 1020
gaattccatt ctgattacac taagattaga caagccactt tcccaggtgt gcagatgaag 1080
gaagccttgc aagaattgaa caagaaagtt tcatctgctg ctagtcacta tgaagtcaag 1140
cctgtgccca agatcaagtt ggccaataca ccagccacca gagaagtcaa gttaactcag 1200
gaatggttgt ggaccagagt gtcttcgtgg ttcagagaag gtgatattat tatcaccgaa 1260
accggtacat cctccttcgg tatagttcaa tccagattcc caaacaacac catcggtatc 1320
tcccaagtat tgtggggttc tattggtttc tctgttggtg ccactttggg tgctgccatg 1380
gctgcccaag aactcgaccc taacaagaga accatcttgt ttgttggaga tggttctttg 1440
caattgaccg ttcaggaaat ctccaccata atcagatggg gtaccacacc ttacctttc 1500
gtgttgaaca atgacggtta caccatcgag cgtttgatcc acggtgtaaa tgcctcatat 1560
aatgacatcc aaccatggca aaacttggaa atcttgccta ctttctcggc caagaactac 1620
gacgctgtga gaatctccaa catcggagaa gcagaagata tcttgaaaga caaggaattc 1680
ggaaagaact ccaagattag attgatagaa gtcatgttac caagattgga tgcaccatct 1740
aaccttgcca aacaagctgc cattacagct gccaccaacg ccgaagct 1788

<210> 117

<211> 569
<212> PRT
<213> Pichia stipitis

<400> 117

```
Met Val Ser Thr Tyr Pro Glu Ser Glu Val Thr Leu Gly Arg Tyr Leu
1               5                   10                  15

Phe Glu Arg Leu His Gln Leu Lys Val Asp Thr Ile Phe Gly Leu Pro
            20                  25                  30

Gly Asp Phe Asn Leu Ser Leu Leu Asp Lys Val Tyr Glu Val Pro Asp
        35                  40                  45

Met Arg Trp Ala Gly Asn Ala Asn Glu Leu Asn Ala Ala Tyr Ala Ala
    50                  55                  60

Asp Gly Tyr Ser Arg Ile Lys Gly Leu Ser Cys Leu Val Thr Thr Phe
65                  70                  75                  80
```

```
Gly Val Gly Glu Leu Ser Ala Leu Asn Gly Val Gly Gly Ala Tyr Ala
                85                  90                  95

Glu His Val Gly Leu Leu His Val Val Gly Val Pro Ser Ile Ser Ser
            100                 105                 110

Gln Ala Lys Gln Leu Leu Leu His His Thr Leu Gly Asn Gly Asp Phe
        115                 120                 125

Thr Val Phe His Arg Met Ser Asn Ser Ile Ser Gln Thr Thr Ala Phe
    130                 135                 140

Leu Ser Asp Ile Ser Ile Ala Pro Gly Gln Ile Asp Arg Cys Ile Arg
145                 150                 155                 160

Glu Ala Tyr Val His Gln Arg Pro Val Tyr Val Gly Leu Pro Ala Asn
                165                 170                 175

Met Val Asp Leu Lys Val Pro Ser Ser Leu Leu Glu Thr Pro Ile Asp
            180                 185                 190

Leu Lys Leu Lys Gln Asn Asp Pro Glu Ala Gln Glu Val Val Glu Thr
        195                 200                 205

Val Leu Lys Leu Val Ser Gln Ala Thr Asn Pro Ile Ile Leu Val Asp
    210                 215                 220

Ala Cys Ala Leu Arg His Asn Cys Lys Glu Glu Val Lys Gln Leu Val
225                 230                 235                 240

Asp Ala Thr Asn Phe Gln Val Phe Thr Thr Pro Met Gly Lys Ser Gly
                245                 250                 255

Ile Ser Glu Ser His Pro Arg Leu Gly Gly Val Tyr Val Gly Thr Met
            260                 265                 270

Ser Ser Pro Gln Val Lys Lys Ala Val Glu Asn Ala Asp Leu Ile Leu
        275                 280                 285

Ser Val Gly Ser Leu Leu Ser Asp Phe Asn Thr Gly Ser Phe Ser Tyr
    290                 295                 300

Ser Tyr Lys Thr Lys Asn Val Val Glu Phe His Ser Asp Tyr Met Lys
305                 310                 315                 320

Ile Arg Gln Ala Thr Phe Pro Gly Val Gln Met Lys Glu Ala Leu Gln
```

```
                325                 330                 335

Gln Leu Ile Lys Arg Val Ser Ser Tyr Ile Asn Pro Ser Tyr Ile Pro
            340                 345                 350

Thr Arg Val Pro Lys Arg Lys Gln Pro Leu Lys Ala Pro Ser Glu Ala
        355                 360                 365

Pro Leu Thr Gln Glu Tyr Leu Trp Ser Lys Val Ser Gly Trp Phe Arg
    370                 375                 380

Glu Gly Asp Ile Ile Val Thr Glu Thr Gly Thr Ser Ala Phe Gly Ile
385                 390                 395                 400

Ile Gln Ser His Phe Pro Ser Asn Thr Ile Gly Ile Ser Gln Val Leu
                405                 410                 415

Trp Gly Ser Ile Gly Phe Thr Val Gly Ala Thr Val Gly Ala Ala Met
            420                 425                 430

Ala Ala Gln Glu Ile Asp Pro Ser Arg Arg Val Ile Leu Phe Val Gly
        435                 440                 445

Asp Gly Ser Leu Gln Leu Thr Val Gln Glu Ile Ser Thr Leu Cys Lys
    450                 455                 460

Trp Asp Cys Asn Asn Thr Tyr Leu Tyr Val Leu Asn Asn Asp Gly Tyr
465                 470                 475                 480

Thr Ile Glu Arg Leu Ile His Gly Lys Ser Ala Ser Tyr Asn Asp Ile
                485                 490                 495

Gln Pro Trp Asn His Leu Ser Leu Leu Arg Leu Phe Asn Ala Lys Lys
            500                 505                 510

Tyr Gln Asn Val Arg Val Ser Thr Ala Gly Glu Leu Asp Ser Leu Phe
        515                 520                 525

Ser Asp Lys Lys Phe Ala Ser Pro Asp Arg Ile Arg Met Ile Glu Val
    530                 535                 540

Met Leu Ser Arg Leu Asp Ala Pro Ala Asn Leu Val Ala Gln Ala Lys
545                 550                 555                 560

Leu Ser Glu Arg Val Asn Leu Glu Asn
                565
```

<210> 118
<211> 1707
<212> DNA
<213> Pichia stipitis

<400> 118

```
atggtatcaa cctacccaga atcagaggtt actctaggaa ggtacctctt tgagcgactc      60
caccaattga aagtggacac cattttcggc ttgccgggtg acttcaacct ttccttattg     120
gacaaagtgt atgaagttcc ggatatgagg tgggctggaa atgccaacga attgaatgct     180
gcctatgctg ccgatggtta ctccagaata aagggattgt cttgcttggt cacaactttt     240
ggtgttggtg aattgtctgc tttaaacgga gttggtggtg cctatgctga acacgtagga     300
cttctacatg tcgttggagt tccatccata tcgtcacagg ctaaacagtt gttgctccac     360
catacettgg gtaatggtga cttcactgtt tttcacagaa tgtccaatag catttctcaa     420
actacagcat ttctctcaga tatctctatt gcaccaggtc aaatagatag atgcatcaga     480
gaagcatatg ttcatcagag accagtttat gttggtttac cggcaaatat ggttgatctc     540
aaggttcctt ctagtctctt agaaactcca attgatttga aattgaaaca aaatgatcct     600
gaagctcaag aagttgttga aacagtcctg aagttggtgt cccaagctac aaaccccatt     660
atcttggtag acgcttgtgc cctcagacac aattgcaaag aggaagtcaa acaattggtt     720
gatgccacta attttcaagt ctttacaact ccaatgggta aatctggtat ctccgaatct     780
catccaagat tgggcggtgt ctatgtcggg acaatgtcga gtcctcaagt caaaaaagcc     840
gttgaaaatg ccgatcttat actatctgtt ggttcgttgt tatcggactt caatacaggt     900
tcattttcat actcctacaa gacgaagaat gttgttgaat tccactctga ctatatgaaa     960
atcagacagg ccaccttccc aggagttcaa atgaaagaag ccttgcaaca gttgataaaa    1020
agggtctctt cttacatcaa tccaagctac attcctactc gagttcctaa aaggaaacag    1080
ccattgaaag ctccatcaga agctcctttg acccaagaat atttgtggtc taaagtatcc    1140
ggctggttta gagagggtga tattatcgta accgaaactg gtacatctgc tttcggaatt    1200
attcaatccc attttcccag caacactatc ggtatatccc aagtcttgtg ggctcaatt     1260
ggtttcacag taggtgcaac agttggtgct gccatggcag cccaggaaat cgaccctagc    1320
aggagagtaa ttttgttcgt cggtgatggt tcattgcagt tgacggttca ggaaatctct    1380
acgttgtgta aatgggattg taacaatact tatctttacg tgttgaacaa tgatggttac    1440
actatagaaa ggttgatcca cggcaaaagt gccagctaca acgatataca gccttggaac    1500
catttatcct tgcttcgctt attcaatgct aagaaatacc aaaatgtcag agtatcgact    1560
gctggagaat tggactcttt gttctctgat aagaaatttg cttctccaga taggataaga    1620
atgattgagg tgatgttatc gagattggat gcaccagcaa atcttgttgc tcaagcaaag    1680
ttgtctgaac gggtaaacct tgaaaat                                        1707
```

<210> 119
<211> 563
<212> PRT

<213> Kluyveromyces lactis

<400> 119

```
Met Ser Glu Ile Thr Leu Gly Arg Tyr Leu Phe Glu Arg Leu Lys Gln
1               5                   10                  15

Val Glu Val Gln Thr Ile Phe Gly Leu Pro Gly Asp Phe Asn Leu Ser
            20                  25                  30

Leu Leu Asp Asn Ile Tyr Glu Val Pro Gly Met Arg Trp Ala Gly Asn
        35                  40                  45

Ala Asn Glu Leu Asn Ala Ala Tyr Ala Ala Asp Gly Tyr Ala Arg Leu
    50                  55                  60

Lys Gly Met Ser Cys Ile Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Val Val Gly Val Pro Ser Val Ser Ser Gln Ala Lys Gln Leu Leu
            100                 105                 110

Leu His His Thr Leu Gly Asn Gly Asp Phe Thr Val Phe His Arg Met
        115                 120                 125

Ser Ser Asn Ile Ser Glu Thr Thr Ala Met Ile Thr Asp Ile Asn Thr
    130                 135                 140

Ala Pro Ala Glu Ile Asp Arg Cys Ile Arg Thr Thr Tyr Val Ser Gln
145                 150                 155                 160

Arg Pro Val Tyr Leu Gly Leu Pro Ala Asn Leu Val Asp Leu Thr Val
                165                 170                 175

Pro Ala Ser Leu Leu Asp Thr Pro Ile Asp Leu Ser Leu Lys Pro Asn
            180                 185                 190

Asp Pro Glu Ala Glu Glu Glu Val Ile Glu Asn Val Leu Gln Leu Ile
        195                 200                 205

Lys Glu Ala Lys Asn Pro Val Ile Leu Ala Asp Ala Cys Cys Ser Arg
    210                 215                 220
```

```
His Asp Ala Lys Ala Glu Thr Lys Lys Leu Ile Asp Leu Thr Gln Phe
225                 230                 235                 240

Pro Ala Phe Val Thr Pro Met Gly Lys Gly Ser Ile Asp Glu Lys His
                245                 250                 255

Pro Arg Phe Gly Gly Val Tyr Val Gly Thr Leu Ser Ser Pro Ala Val
            260                 265                 270

Lys Glu Ala Val Glu Ser Ala Asp Leu Val Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Ile Val Glu Phe His Ser Asp Tyr Thr Lys Ile Arg Ser Ala Thr
305                 310                 315                 320

Phe Pro Gly Val Gln Met Lys Phe Ala Leu Gln Lys Leu Leu Thr Lys
                325                 330                 335

Val Ala Asp Ala Ala Lys Gly Tyr Lys Pro Val Pro Val Pro Ser Glu
            340                 345                 350

Pro Glu His Asn Glu Ala Val Ala Asp Ser Thr Pro Leu Lys Gln Glu
        355                 360                 365

Trp Val Trp Thr Gln Val Gly Glu Phe Leu Arg Glu Gly Asp Val Val
    370                 375                 380

Ile Thr Glu Thr Gly Thr Ser Ala Phe Gly Ile Asn Gln Thr His Phe
385                 390                 395                 400

Pro Asn Asn Thr Tyr Gly Ile Ser Gln Val Leu Trp Gly Ser Ile Gly
                405                 410                 415

Phe Thr Thr Gly Ala Thr Leu Gly Ala Ala Phe Ala Ala Glu Glu Ile
            420                 425                 430

Asp Pro Lys Lys Arg Val Ile Leu Phe Ile Gly Asp Gly Ser Leu Gln
        435                 440                 445

Leu Thr Val Gln Glu Ile Ser Thr Met Ile Arg Trp Gly Leu Lys Pro
    450                 455                 460

Tyr Leu Phe Val Leu Asn Asn Asp Gly Tyr Thr Ile Glu Arg Leu Ile
```

465 470 475 480

His Gly Glu Thr Ala Gln Tyr Asn Cys Ile Gln Asn Trp Gln His Leu
485 490 495

Glu Leu Leu Pro Thr Phe Gly Ala Lys Asp Tyr Glu Ala Val Arg Val
500 505 510

Ser Thr Thr Gly Glu Trp Asn Lys Leu Thr Thr Asp Glu Lys Phe Gln
515 520 525

Asp Asn Thr Arg Ile Arg Leu Ile Glu Val Met Leu Pro Thr Met Asp
530 535 540

Ala Pro Ser Asn Leu Val Lys Gln Ala Gln Leu Thr Ala Ala Thr Asn
545 550 555 560

Ala Lys Asn

<210> 120
<211> 1689
<212> DNA
<213> Kluyveromyces lactis

<400> 120

atgtctgaaa ttacattagg tcgttacttg ttcgaaagat taaagcaagt cgaagttcaa 60
accatctttg gtctaccagg tgatttcaac ttgtccctat tggacaatat ctacgaagtc 120
ccaggtatga gatgggctgg taatgccaac gaattgaacg ctgcttacgc tgctgatggt 180
tacgccagat taaagggtat gtcctgtatc atcaccacct tcggtgtcgg tgaattgtct 240
gctttgaacg gtattgccgg ttcttacgct gaacacgttg gtgtcttgca cgttgtcggt 300
gttccatccg tctcttctca agctaagcaa ttgttgttgc accacacctt gggtaacggt 360
gacttcactg ttttccacag aatgtcctcc aacatttctg aaaccactgc tatgatcacc 420
gatatcaaca ctgccccagc tgaaatcgac agatgtatca gaaccactta cgtttcccaa 480
agaccagtct acttgggttt gccagctaac ttggtcgact tgactgtccc agcttctttg 540
ttggacactc caattgattt gagcttgaag ccaaatgacc cagaagccga agaagaagtc 600
atcgaaaacg tcttgcaact gatcaaggaa gctaagaacc cagttatctt ggctgatgct 660
tgttgttcca gacacgatgc caaggctgag accaagaagt tgatcgactt gactcaattc 720
ccagccttcg ttaccccaat gggtaagggt tccattgacg aaaagcaccc aagattcggt 780
ggtgtctacg tcggtaccct atcttctcca gctgtcaagg aagccgttga atctgctgac 840
ttggttctat cggtcggtgc tctattgtcc gatttcaaca ctggttcttt ctcttactct 900

tacaagacca agaacattgt cgaattccac tctgactaca ccaagatcag aagcgctacc 960
ttcccaggtg tccaaatgaa gttcgcttta caaaaattgt tgactaaggt tgccgatgct 1020
gctaagggtt acaagccagt tccagttcca tctgaaccag aacacaacga agctgtcgct 1080
gactccactc cattgaagca agaatgggtc tggactcaag tcggtgaatt cttgagagaa 1140
ggtgatgttg ttatcactga aaccggtacc tctgccttcg gtatcaacca aactcatttc 1200
ccaaacaaca catacggtat ctctcaagtt ttatggggtt ccattggttt caccactggt 1260
gctaccttgg gtgctgcctt cgctgccgaa gaaattgatc caaagaagag agttatctta 1320
ttcattggtg acggttcttt gcaattgact gttcaagaaa tctccaccat gatcagatgg 1380
ggcttgaagc catacttgtt cgtattgaac aacgacggtt acaccattga aagattgatt 1440
cacggtgaaa ccgctcaata caactgtatc caaaactggc aacacttgga attattgcca 1500
actttcggtg ccaaggacta cgaagctgtc agagtttcca ccactggtga atggaacaag 1560
ttgaccactg acgaaaagtt ccaagacaac accagaatca gattgatcga agttatgttg 1620
ccaactatgg atgctccatc taacttggtt aagcaagctc aattgactgc tgctaccaac 1680
gctaagaac 1689

<210> 121
<211> 571

<212> PRT
<213> Yarrowia lipolytica

<400> 121

```
Met Ser Asp Ser Glu Pro Gln Met Val Asp Leu Gly Asp Tyr Leu Phe
1               5                   10                  15

Ala Arg Phe Lys Gln Leu Gly Val Asp Ser Val Phe Gly Val Pro Gly
            20                  25                  30

Asp Phe Asn Leu Thr Leu Leu Asp His Val Tyr Asn Val Asp Met Arg
        35                  40                  45

Trp Val Gly Asn Thr Asn Glu Leu Asn Ala Gly Tyr Ser Ala Asp Gly
    50                  55                  60

Tyr Ser Arg Val Lys Arg Leu Ala Cys Leu Val Thr Thr Phe Gly Val
65                  70                  75                  80

Gly Glu Leu Ser Ala Val Ala Ala Val Ala Gly Ser Tyr Ala Glu His
                85                  90                  95

Val Gly Val Val His Val Val Gly Val Pro Ser Thr Ser Ala Glu Asn
            100                 105                 110
```

```
Lys His Leu Leu Leu His His Thr Leu Gly Asn Gly Asp Phe Arg Val
        115                 120                 125

Phe Ala Gln Met Ser Lys Leu Ile Ser Glu Tyr Thr His His Ile Glu
    130                 135                 140

Asp Pro Ser Glu Ala Ala Asp Val Ile Asp Thr Ala Ile Arg Ile Ala
145                 150                 155                 160

Tyr Thr His Gln Arg Pro Val Tyr Ile Ala Val Pro Ser Asn Phe Ser
                165                 170                 175

Glu Val Asp Ile Ala Asp Gln Ala Arg Leu Asp Thr Pro Leu Asp Leu
            180                 185                 190

Ser Leu Gln Pro Asn Asp Pro Glu Ser Gln Tyr Glu Val Ile Glu Glu
        195                 200                 205

Ile Cys Ser Arg Ile Lys Ala Ala Lys Lys Pro Val Ile Leu Val Asp
    210                 215                 220

Ala Cys Ala Ser Arg Tyr Arg Cys Val Asp Glu Thr Lys Glu Leu Ala
225                 230                 235                 240

Lys Ile Thr Asn Phe Ala Tyr Phe Val Thr Pro Met Gly Lys Gly Ser
                245                 250                 255

Val Asp Glu Asp Thr Asp Arg Tyr Gly Gly Thr Tyr Val Gly Ser Leu
            260                 265                 270

Thr Ala Pro Ala Thr Ala Glu Val Val Glu Thr Ala Asp Leu Ile Ile
        275                 280                 285

Ser Val Gly Ala Leu Leu Ser Asp Phe Asn Thr Gly Ser Phe Ser Tyr
    290                 295                 300

Ser Tyr Ser Thr Lys Asn Val Val Glu Leu His Ser Asp His Val Lys
305                 310                 315                 320

Ile Lys Ser Ala Thr Tyr Asn Asn Val Gly Met Lys Met Leu Phe Pro
                325                 330                 335

Pro Leu Leu Glu Ala Val Lys Lys Leu Val Ala Glu Thr Pro Asp Phe
            340                 345                 350

Ala Ser Lys Ala Leu Ala Val Pro Asp Thr Thr Pro Lys Ile Pro Glu
        355                 360                 365
```

```
Val Pro Asp Asp His Ile Thr Thr Gln Ala Trp Leu Trp Gln Arg Leu
    370                 375                 380

Ser Tyr Phe Leu Arg Pro Thr Asp Ile Val Val Thr Glu Thr Gly Thr
385                 390                 395                 400

Ser Ser Phe Gly Ile Ile Gln Thr Lys Phe Pro His Asn Val Arg Gly
                405                 410                 415

Ile Ser Gln Val Leu Trp Gly Ser Ile Gly Tyr Ser Val Gly Ala Ala
            420                 425                 430

Cys Gly Ala Ser Ile Ala Ala Gln Glu Ile Asp Pro Gln Gln Arg Val
        435                 440                 445

Ile Leu Phe Val Gly Asp Gly Ser Leu Gln Leu Thr Val Thr Glu Ile
    450                 455                 460

Ser Cys Met Ile Arg Asn Asn Val Lys Pro Tyr Ile Phe Val Leu Asn
465                 470                 475                 480

Asn Asp Gly Tyr Thr Ile Glu Arg Leu Ile His Gly Glu Asn Ala Ser
                485                 490                 495

Tyr Asn Asp Val His Met Trp Lys Tyr Ser Lys Ile Leu Asp Thr Phe
            500                 505                 510

Asn Ala Lys Ala His Glu Ser Ile Val Val Asn Thr Lys Gly Glu Met
        515                 520                 525

Asp Ala Leu Phe Asp Asn Glu Glu Phe Ala Lys Pro Asp Lys Ile Arg
    530                 535                 540

Leu Ile Glu Val Met Cys Asp Lys Met Asp Ala Pro Ala Ser Leu Ile
545                 550                 555                 560

Lys Gln Ala Glu Leu Ser Ala Lys Thr Asn Val
                565                 570
```

<210> 122
<211> 1713
<212> DNA
<213> Yarrowia lipolytica

<400> 122

```
atgagcgact ccgaacccca aatggtcgac ctgggcgact atctctttgc ccgattcaag      60
cagctaggcg tggactccgt ctttggagtg cccggcgact tcaacctcac cctgttggac     120
cacgtgtaca atgtcgacat gcggtgggtt gggaacacaa acgagctgaa tgccggctac     180
tcggccgacg gctactcccg ggtcaagcgg ctggcatgtc ttgtcaccac ctttggcgtg     240
ggagagctgt ctgccgtggc tgctgtggca ggctcgtacg ccgagcatgt gggcgtggtg     300
catgttgtgg gcgttcccag cacctctgct gagaacaagc atctgctgct gcaccacaca     360
ctcggtaacg gcgacttccg ggtctttgcc cagatgtcca aactcatctc cgagtacacc     420
caccatattg aggaccccag cgaggctgcc gacgtaatcg acaccgccat ccgaatcgcc     480
tacacccacc agcggcccgt ttacattgct gtgccctcca acttctccga ggtcgatatt     540
gccgaccagg ctagactgga tacccccctg gacctttcgc tgcagcccaa cgaccccgag     600
agccagtacg aggtgattga ggagatttgc tcgcgtatca aggccgccaa gaagcccgtg     660
attctcgtcg acgcctgcgc ttcgcgatac agatgtgtgg acgagaccaa ggagctggcc     720
aagatcacca actttgccta ctttgtcact cccatgggta agggttctgt ggacgaggat     780
actgaccggt acggaggaac atacgtcgga tcgctgactg ctcctgctac tgccgaggtg     840
gttgagacag ctgatctcat catctccgta ggagctcttc tgtcggactt caacaccggt     900
tccttctcgt actcctactc caccaaaaac gtggtggaat tgcattcgga ccacgtcaaa     960
atcaagtccg ccacctacaa caacgtcggc atgaaaatgc tgttcccgcc cctgctcgaa    1020
gccgtcaaga aactggttgc cgagacccct gactttgcat ccaaggctct ggctgttccc    1080
gacaccactc ccaagatccc cgaggtaccc gatgatcaca ttacgaccca ggcatggctg    1140
tggcagcgtc tcagttactt tctgaggccc accgacatcg tggtcaccga gaccggaacc    1200
tcgtcctttg gaatcatcca gaccaagttc cccacaacg tccgaggtat ctcgcaggtg     1260
ctgtggggct ctattggata ctcggtggga gcagcctgtg gagcctccat tgctgcacag    1320
gagattgacc cccagcagcg agtgattctg tttgtgggcg acggctctct tcagctgacg    1380
gtgaccgaga tctcgtgcat gatccgcaac aacgtcaagc cgtacatttt tgtgctcaac    1440
aacgacggct acaccatcga gaggctcatt cacggcgaaa acgcctcgta caacgatgtg    1500
cacatgtgga agtactccaa gattctcgac acgttcaacg ccaaggccca cgagtcgatt    1560
gtggtcaaca ccaagggcga gatggacgct ctgttcgaca acgaagagtt tgccaagccc    1620
gacaagatcc ggctcattga ggtcatgtgc gacaagatgg acgcgcctgc ctcgttgatc    1680
aagcaggctg agctctctgc caagaccaac gtt                                 1713
```

<210> 123
<211> 571

<212> PRT
<213> Schizosaccharomyces pombe

<400> 123

```
Met Ser Gly Asp Ile Leu Val Gly Glu Tyr Leu Phe Lys Arg Leu Glu
1               5                   10                  15

Gln Leu Gly Val Lys Ser Ile Leu Gly Val Pro Gly Asp Phe Asn Leu
            20                  25                  30

Ala Leu Leu Asp Leu Ile Glu Lys Val Gly Asp Glu Lys Phe Arg Trp
        35                  40                  45

Val Gly Asn Thr Asn Glu Leu Asn Gly Ala Tyr Ala Ala Asp Gly Tyr
    50                  55                  60

Ala Arg Val Asn Gly Leu Ser Ala Ile Val Thr Thr Phe Gly Val Gly
65                  70                  75                  80

Glu Leu Ser Ala Ile Asn Gly Val Ala Gly Ser Tyr Ala Glu His Val
                85                  90                  95

Pro Val Val His Ile Val Gly Met Pro Ser Thr Lys Val Gln Asp Thr
            100                 105                 110

Gly Ala Leu Leu His His Thr Leu Gly Asp Gly Asp Phe Arg Thr Phe
        115                 120                 125

Met Asp Met Phe Lys Lys Val Ser Ala Tyr Ser Ile Met Ile Asp Asn
    130                 135                 140

Gly Asn Asp Ala Ala Glu Lys Ile Asp Glu Ala Leu Ser Ile Cys Tyr
145                 150                 155                 160

Lys Lys Ala Arg Pro Val Tyr Ile Gly Ile Pro Ser Asp Ala Gly Tyr
                165                 170                 175

Phe Lys Ala Ser Ser Ser Asn Leu Gly Lys Arg Leu Lys Leu Glu Glu
            180                 185                 190

Asp Thr Asn Asp Pro Ala Val Glu Gln Glu Val Ile Asn His Ile Ser
        195                 200                 205

Glu Met Val Val Asn Ala Lys Lys Pro Val Ile Leu Ile Asp Ala Cys
    210                 215                 220

Ala Val Arg His Arg Val Val Pro Glu Val His Glu Leu Ile Lys Leu
225                 230                 235                 240

Thr His Phe Pro Thr Tyr Val Thr Pro Met Gly Lys Ser Ala Ile Asp
                245                 250                 255
```

Glu Thr Ser Gln Phe Phe Asp Gly Val Tyr Val Gly Ser Ile Ser Asp
260 265 270

Pro Glu Val Lys Asp Arg Ile Glu Ser Thr Asp Leu Leu Leu Ser Ile
275 280 285

Gly Ala Leu Lys Ser Asp Phe Asn Thr Gly Ser Phe Ser Tyr His Leu
290 295 300

Ser Gln Lys Asn Ala Val Glu Phe His Ser Asp His Met Arg Ile Arg
305 310 315 320

Tyr Ala Leu Tyr Pro Asn Val Ala Met Lys Tyr Ile Leu Arg Lys Leu
325 330 335

Leu Lys Val Leu Asp Ala Ser Met Cys His Ser Lys Ala Ala Pro Thr
340 345 350

Ile Gly Tyr Asn Ile Lys Pro Lys His Ala Glu Gly Tyr Ser Ser Asn
355 360 365

Glu Ile Thr His Cys Trp Phe Trp Pro Lys Phe Ser Glu Phe Leu Lys
370 375 380

Pro Arg Asp Val Leu Ile Thr Glu Thr Gly Thr Ala Asn Phe Gly Val
385 390 395 400

Leu Asp Cys Arg Phe Pro Lys Asp Val Thr Ala Ile Ser Gln Val Leu
405 410 415

Trp Gly Ser Ile Gly Tyr Ser Val Gly Ala Met Phe Gly Ala Val Leu
420 425 430

Ala Val His Asp Ser Lys Glu Pro Asp Arg Arg Thr Ile Leu Val Val
435 440 445

Gly Asp Gly Ser Leu Gln Leu Thr Ile Thr Glu Ile Ser Thr Cys Ile
450 455 460

Arg His Asn Leu Lys Pro Ile Ile Phe Ile Ile Asn Asn Asp Gly Tyr
465 470 475 480

Thr Ile Glu Arg Leu Ile His Gly Leu His Ala Ser Tyr Asn Glu Ile
485 490 495

Asn Thr Lys Trp Gly Tyr Gln Gln Ile Pro Lys Phe Phe Gly Ala Ala
500 505 510

```
Glu Asn His Phe Arg Thr Tyr Cys Val Lys Thr Pro Thr Asp Val Glu
        515                     520                     525

Lys Leu Phe Ser Asp Lys Glu Phe Ala Asn Ala Asp Val Ile Gln Val
    530                     535                     540

Val Glu Leu Val Met Pro Met Leu Asp Ala Pro Arg Val Leu Val Glu
545                 550                     555                 560

Gln Ala Lys Leu Thr Ser Lys Ile Asn Lys Gln
                565                     570
```

<210> 124
<211> 1713
<212> DNA
<213> Schizosaccharomyces pombe

<400> 124

atgagtgggg atattttagt cggtgaatat ctattcaaaa ggcttgaaca attaggggtc 60
aagtccattc ttggtgttcc aggagatttc aatttagctc tacttgactt aattgagaaa 120
gttggagatg agaaatttcg ttgggttggc aataccaatg agttgaatgg tgcttatgcc 180
gctgatggtt atgctcgtgt taatggtctt tcagccattg ttacaacgtt cggcgtggga 240
gagctttccg ctattaatgg agtggcaggt tcttatgcgg agcatgtccc agtagttcat 300
attgttggaa tgccttccac aaaggtgcaa gatactggag ctttgcttca tcatacttta 360
ggagatggag actttcgcac tttcatggat atgtttaaga aagtttctgc ctacagtata 420
atgatcgata acggaaacga tgcagctgaa aagatcgatg aagccttgtc gatttgttat 480
aaaaaggcta ggcctgttta cattggtatt ccttctgatg ctggctactt caaagcatct 540
tcatcaaatc ttgggaaaag actaaagctc gaggaggata ctaacgatcc agcagttgag 600
caagaagtca tcaatcatat ctcggaaatg gttgtcaatg caaagaaacc agtgatttta 660
attgacgctt gtgctgtaag acatcgtgtc gttccagaag tacatgagct gattaaattg 720
acccatttcc ctacatatgt aactcccatg ggtaaatctg caattgacga aacttcgcaa 780
ttttttgacg gcgtttatgt tggttcaatt tcagatcctg aagttaaaga cagaattgaa 840
tccactgatc tgttgctatc catcggtgct ctcaaatcag actttaacac gggttccttc 900
tcttaccacc tcagccaaaa gaatgccgtt gagtttcatt cagaccacat gcgcattcga 960
tatgctcttt atccaaatgt agccatgaag tatattcttc gcaaactgtt gaaagtactt 1020
gatgcttcta tgtgtcattc caaggctgct cctaccattg gctacaacat caagcctaag 1080
catgcggaag gatattcttc caacgagatt actcattgct ggttttggcc taaatttagt 1140
gaatttttga agccccgaga tgttttgatc accgagactg gaactgcaaa ctttggtgtc 1200

cttgattgca ggtttccaaa ggatgtaaca gccatttccc aggtattatg gggatctatt 1260
ggatactccg ttggtgcaat gtttggtgct gttttggccg tccacgattc taaagagccc 1320
gatcgtcgta ccattcttgt agtaggtgat ggatccttac aactgacgat tacagagatt 1380
tcaacctgca ttcgccataa cctcaaacca attattttca taattaacaa cgacggttac 1440
accattgagc gtttaattca tggtttgcat gctagctata acgaaattaa cactaaatgg 1500
ggctaccaac agattcccaa gtttttcgga gctgctgaaa accacttccg cacttactgt 1560
gttaaaactc ctactgacgt tgaaaagttg tttagcgaca aggagtttgc aaatgcagat 1620
gtcattcaag tagttgagct tgtaatgcct atgttggatg cacctcgtgt cctagttgag 1680
caagccaagt tgacgtctaa gatcaataag caa 1713

<210> 125
<211> 563

<212> PRT
<213> Zygosaccharomyces rouxii

<400> 125

```
Met Ser Glu Ile Thr Leu Gly Arg Tyr Leu Phe Glu Arg Leu Lys Gln
1               5                   10                  15

Val Asp Thr Asn Thr Ile Phe Gly Val Pro Gly Asp Phe Asn Leu Ser
            20                  25                  30

Leu Leu Asp Lys Val Tyr Glu Val Gln Gly Leu Arg Trp Ala Gly Asn
        35                  40                  45

Ala Asn Glu Leu Asn Ala Ala Tyr Ala Ala Asp Gly Tyr Ala Arg Val
    50                  55                  60

Lys Gly Leu Ala Ala Leu Ile Thr Thr Phe Gly Val Gly Glu Leu Ser
65                  70                  75                  80

Ala Leu Asn Gly Ile Ala Gly Ser Tyr Ala Glu His Val Gly Val Leu
                85                  90                  95

His Ile Val Gly Val Pro Ser Val Ser Ser Gln Ala Lys Gln Leu Leu
            100                 105                 110

Leu His His Thr Leu Gly Asn Gly Asp Phe Thr Val Phe His Arg Met
        115                 120                 125

Ser Ala Asn Ile Ser Glu Thr Thr Ala Met Leu Thr Asp Ile Thr Ala
    130                 135                 140
```

```
Ala Pro Ala Glu Ile Asp Arg Cys Ile Arg Val Ala Tyr Val Asn Gln
145                 150                 155                 160

Arg Pro Val Tyr Leu Gly Leu Pro Ala Asn Leu Val Asp Gln Lys Val
                165                 170                 175

Pro Ala Ser Leu Leu Asn Thr Pro Ile Asp Leu Ser Leu Lys Glu Asn
            180                 185                 190

Asp Pro Glu Ala Glu Thr Glu Val Val Asp Thr Val Leu Glu Leu Ile
        195                 200                 205

Lys Glu Ala Lys Asn Pro Val Ile Leu Ala Asp Ala Cys Cys Ser Arg
    210                 215                 220

His Asp Val Lys Ala Glu Thr Lys Lys Leu Ile Asp Leu Thr Gln Phe
225                 230                 235                 240

Pro Ser Phe Val Thr Pro Met Gly Lys Gly Ser Ile Asp Glu Gln Asn
                245                 250                 255

Pro Arg Phe Gly Gly Val Tyr Val Gly Thr Leu Ser Ser Pro Glu Val
            260                 265                 270

Lys Glu Ala Val Glu Ser Ala Asp Leu Val Leu Ser Val Gly Ala Leu
        275                 280                 285

Leu Ser Asp Phe Asn Thr Gly Ser Phe Ser Tyr Ser Tyr Lys Thr Lys
    290                 295                 300

Asn Val Val Glu Phe His Ser Asp His Ile Lys Ile Arg Asn Ala Thr
305                 310                 315                 320

Phe Pro Gly Val Gln Met Lys Phe Val Leu Lys Lys Leu Leu Gln Ala
                325                 330                 335

Val Pro Glu Ala Val Lys Asn Tyr Lys Pro Gly Pro Val Pro Ala Pro
            340                 345                 350

Pro Ser Pro Asn Ala Glu Val Ala Asp Ser Thr Thr Leu Lys Gln Glu
        355                 360                 365

Trp Leu Trp Arg Gln Val Gly Ser Phe Leu Arg Glu Gly Asp Val Val
    370                 375                 380

Ile Thr Glu Thr Gly Thr Ser Ala Phe Gly Ile Asn Gln Thr His Phe
385                 390                 395                 400
```

Pro Asn Gln Thr Tyr Gly Ile Ser Gln Val Leu Trp Gly Ser Ile Gly
405 410 415

Tyr Thr Thr Gly Ser Thr Leu Gly Ala Ala Phe Ala Ala Glu Glu Ile
420 425 430

Asp Pro Lys Lys Arg Val Ile Leu Phe Ile Gly Asp Gly Ser Leu Gln
435 440 445

Leu Thr Val Gln Glu Ile Ser Thr Met Ile Arg Trp Gly Leu Lys Pro
450 455 460

Tyr Leu Phe Val Leu Asn Asn Asp Gly Tyr Thr Ile Glu Arg Leu Ile
465 470 475 480

His Gly Glu Thr Ala Glu Tyr Asn Cys Ile Gln Pro Trp Lys His Leu
485 490 495

Glu Leu Leu Asn Thr Phe Gly Ala Lys Asp Tyr Glu Asn His Arg Val
500 505 510

Ser Thr Val Gly Glu Trp Asn Lys Leu Thr Gln Asp Pro Lys Phe Asn
515 520 525

Glu Asn Ser Arg Ile Arg Met Ile Glu Val Met Leu Glu Val Met Asp
530 535 540

Ala Pro Ser Ser Leu Val Ala Gln Ala Gln Leu Thr Ala Ala Thr Asn
545 550 555 560

Ala Lys Gln

<210> 126
<211> 1689
<212> DNA
<213> Zygosaccharomyces rouxii

<400> 126

atgtctgaaa ttactctagg tcgttacttg ttcgaaagat taaagcaagt tgacactaac 60

accatcttcg gtgttccagg tgacttcaac ttgtccttgt tggacaaggt ctacgaagtg 120

caaggtctaa gatgggctgg taacgctaac gaattgaacg ctgcctacgc tgctgacggt 180

tacgccagag ttaagggttt ggctgctttg atcaccacct tcggtgtcgg tgaattgtct 240

gctttgaacg gtattgcagg ttcttacgct gaacacgttg gtgttttgca cattgttggt 300

gttccatctg tctcttctca agctaagcaa ttgttgttgc accacacctt gggtaacggt 360

gacttcactg ttttccacag aatgtccgcc aacatctctg aaaccaccgc tatgttgacc 420

gacatcactg ctgctccagc tgaaattgac cgttgcatca gagttgctta cgtcaaccaa 480

agaccagtct acttgggtct accagctaac ttggttgacc aaaaggtccc agcttctttg 540

ttgaacactc caattgatct atctctaaag gagaacgacc cagaagctga aaccgaagtt 600

gttgacaccg ttttggaatt gatcaaggaa gctaagaacc cagttatctt ggctgatgct 660

tgctgctcca gacacgacgt caaggctgaa accaagaagt tgatcgactt gactcaattc 720

ccatctttcg ttactcctat gggtaagggt tccatcgacg aacaaaaccc aagattcggt 780

ggtgtctacg tcggtactct atccagccca gaagttaagg aagctgttga atctgctgac 840

ttggttctat ctgtcggtgc tctattgtcc gatttcaaca ctggttcttt ctcttactct 900

tacaagacca agaacgttgt tgaattccac tctgaccaca tcaagatcag aaacgctacc 960

ttcccaggtg ttcaaatgaa attcgttttg aagaaactat tgcaagctgt cccagaagct 1020

gtcaagaact acaagccagg tccagtccca gctccgccat ctccaaacgc tgaagttgct 1080

gactctacca ccttgaagca agaatggtta tggagacaag tcggtagctt cttgagagaa 1140

ggtgatgttg ttattaccga aactggtacc tctgctttcg gtatcaacca aactcacttc 1200

cctaaccaaa cttacggtat ctctcaagtc ttgtggggtt ctattggtta caccactggt 1260

tccactttgg gtgctgcctt cgctgctgaa gaaattgacc ctaagaagag agttatcttg 1320

ttcattggtg acggttctct acaattgacc gttcaagaaa tctccaccat gatcagatgg 1380

ggtctaaagc catacttgtt cgttttgaac aacgatggtt acaccattga aagattgatt 1440

cacggtgaaa ccgctgaata caactgtatc caaccatgga agcacttgga attgttgaac 1500

accttcggtg ccaaggacta cgaaaaccac agagtctcca ctgtcggtga atggaacaag 1560

ttgactcaag atccaaaatt caacgaaaac tctagaatta gaatgatcga agttatgctt 1620

gaagtcatgg acgctccatc ttctttggtc gctcaagctc aattgaccgc tgctactaac 1680

gctaagcaa 1689

<210> 127
<211> 267
<212> PRT
<213> Saccharomyces cerevisiae

<400> 127

Met Ser Gln Gly Arg Lys Ala Ala Glu Arg Leu Ala Lys Lys Thr Val
1               5                   10                  15

Leu Ile Thr Gly Ala Ser Ala Gly Ile Gly Lys Ala Thr Ala Leu Glu
            20                  25                  30

Tyr Leu Glu Ala Ser Asn Gly Asp Met Lys Leu Ile Leu Ala Ala Arg

```
            35                  40                  45

        Arg Leu Glu Lys Leu Glu Glu Leu Lys Lys Thr Ile Asp Gln Glu Phe
            50                  55                  60

        Pro Asn Ala Lys Val His Val Ala Gln Leu Asp Ile Thr Gln Ala Glu
        65                  70                  75                  80

        Lys Ile Lys Pro Phe Ile Glu Asn Leu Pro Gln Glu Phe Lys Asp Ile
                        85                  90                  95

        Asp Ile Leu Val Asn Asn Ala Gly Lys Ala Leu Gly Ser Asp Arg Val
                    100                 105                 110

        Gly Gln Ile Ala Thr Glu Asp Ile Gln Asp Val Phe Asp Thr Asn Val
                115                 120                 125

        Thr Ala Leu Ile Asn Ile Thr Gln Ala Val Leu Pro Ile Phe Gln Ala
            130                 135                 140

        Lys Asn Ser Gly Asp Ile Val Asn Leu Gly Ser Ile Ala Gly Arg Asp
        145                 150                 155                 160

        Ala Tyr Pro Thr Gly Ser Ile Tyr Cys Ala Ser Lys Phe Ala Val Gly
                        165                 170                 175

        Ala Phe Thr Asp Ser Leu Arg Lys Glu Leu Ile Asn Thr Lys Ile Arg
                    180                 185                 190

        Val Ile Leu Ile Ala Pro Gly Leu Val Glu Thr Glu Phe Ser Leu Val
                195                 200                 205

        Arg Tyr Arg Gly Asn Glu Glu Gln Ala Lys Asn Val Tyr Lys Asp Thr
            210                 215                 220

        Thr Pro Leu Met Ala Asp Asp Val Ala Asp Leu Ile Val Tyr Ala Thr
        225                 230                 235                 240

        Ser Arg Lys Gln Asn Thr Val Ile Ala Asp Thr Leu Ile Phe Pro Thr
                        245                 250                 255

        Asn Gln Ala Ser Pro His His Ile Phe Arg Gly
                    260                 265
```

<210> 128
<211> 804
<212> DNA

<213> Saccharomyces cerevisiae

<400> 128

```
atgtcccaag gtagaaaagc tgcagaaaga ttggctaaga agactgtcct cattacaggt      60
gcatctgctg gtattggtaa ggcgaccgca ttagagtact tggaggcatc caatggtgat     120
atgaaactga tcttggctgc tagaagatta gaaaagctcg aggaattgaa gaagaccatt     180
gatcaagagt ttccaaacgc aaaagttcat gtggcccagc tggatatcac tcaagcagaa     240
aaaatcaagc ccttcattga aaacttgcca caagagttca aggatattga cattctggtg     300
aacaatgccg gaaaggctct tggcagtgac cgtgtgggcc agatcgcaac ggaggatatc     360
caggacgtgt ttgacaccaa cgtcacggct ttaatcaata tcacacaagc tgtactgccc     420
atattccaag ccaagaattc aggagatatt gtaaatttgg gttcaatcgc tggcagagac     480
gcatacccaa caggttctat ctattgtgcc tctaagtttg ccgtgggggc gttcactgat     540
agtttgagaa aggagctcat caacactaaa attagagtca ttctaattgc accagggcta     600
gtcgagactg aattttcact agttagatac agaggtaacg aggaacaagc caagaatgtt     660
tacaaggata ctaccccatt gatggctgat gacgtggctg atctgatcgt ctatgcaact     720
tccagaaaac aaaatactgt aattgcagac actttaatct ttccaacaaa ccaagcgtca     780
cctcatcata tcttccgtgg ataa                                            804
```

## Description

This application claims the benefit of U.S. Provisional Application No. 61/712,385, filed October 11, 2012; U.S. Patent Application No. 13/828,353, filed March 14, 2013; U.S. Patent Application No. 13/836,115, filed March 15, 2013; and U.S. Patent Application No. 14/024,722, filed on September 12, 2013.

The Sequence Listing associated with this application is filed in electronic form via EFS-Web.

FIELD OF THE INVENTION

The present invention relates to processes and systems for the production of fermentation products such as alcohols. The present invention also provides processes for separating feed stream components for improved biomass processing productivity.

BACKGROUND OF THE INVENTION

Alcohols have a variety of industrial and scientific applications such as fuels, reagents, and solvents. For example, butanol is an important industrial chemical with a variety of applications including use as a fuel additive, as a feedstock chemical in the plastics industry, and as a food-grade extractant in the food and flavor industry. Accordingly, there is a high demand for alcohols such as butanol as well as for efficient and environmentally friendly production methods including, for example, the use of biomass as feedstock for these methods.

Production of alcohols by fermentation is one such environmentally friendly production method. Some microorganisms that produce alcohols in high yields also have low toxicity thresholds, such that the alcohol needs to be removed from the fermentor as it is being produced. One method, in situ product removal (ISPR), may be used to remove alcohol from the fermentor as it is produced, thereby allowing the microorganism to produce alcohol at high yields. An example of ISPR that has been described in the art is liquid-liquid extraction (see, e.g., U.S. Patent Application Publication No. 2009/0305370). In order to be technically and economically viable, liquid-liquid extraction requires contact between the extractant and the fermentation broth for efficient mass transfer; phase separation of the extractant from the fermentation broth; efficient recovery and recycle of the extractant; and minimal degradation and/or contamination of the extractant over a long-term operation.

When the aqueous stream entering the fermentor contains undissolved solids from feedstock, the undissolved solids may interfere with liquid-liquid extraction and the extraction method may not be technically and economically viable, for example, leading to increases in capital and operating costs. The presence of undissolved solids during extractive fermentation may lower the mass transfer coefficient, impede phase separation, result in the accumulation of oil from the undissolved solids in the extractant leading to reduced extraction efficiency over time, increase the loss of extractant because it becomes trapped in solids and ultimately removed as Dried Distillers Grains with Solubles (DDGS), slow the disengagement of extractant droplets from the fermentation broth, and/or result in a lower fermentor volume efficiency. Thus, solids removal provides an efficient means to produce and recover an alcohol from a fermentation process.

In addition to solids removal, removal of oil from feedstock may also provide beneficial effects on the production of alcohols as well as commercial benefits. For example, some oils such as corn oil and soybean oil may be used as feedstock for biodiesel and thus, provide an additional revenue stream for alcohol producers. In addition, removing oil can result in energy savings for the production plant due to more efficient fermentation, less fouling due to the removal of the oil, increased fermentor volume efficiency, and decreased energy requirements, for example, the energy needed to dry distillers grains.

There is a continuing need to develop more efficient processes and systems for producing alcohols such as ethanol and butanol. The present invention satisfies this need and provides processes and systems for producing alcohols including processes and systems for separating feed stream components prior to the fermentation and controlling the amount of undissolved solids and/or oil in the fermentation process.

WO2011/160030 discloses a method and system for efficiently producing a fermentative product alcohol. WO 2011/159967 discloses a method for producing product alcohol in which lipids in a biomass are converted into an extractant that can be used in in-situ product removal.

SUMMARY OF THE INVENTION

The present invention relates to processes and systems for separating feed stream components and controlling the amount of undissolved solids and/or oil in a fermentor feed stream in the production of fermentation products. The separated components provide a mechanism for increasing biomass processing productivity, including improving alcohol fermentation co-product profiles. By separating the feed streams into certain components including (1) an aqueous stream comprising fermentable carbon sources, (2) a feed stream comprising oil, and (3) a feed stream comprising undissolved solids, these components may be recombined in a controlled manner, or removed from the system for other uses. This provides a mechanism to develop co-product compositions to meet the needs of different markets, such as animal feed markets requiring higher protein and/or higher fat feeds.

The present invention also relates to processes and systems for removing oil from a fermentor feed stream in the production of fermentation products. In some embodiments, undissolved solids and oil may be removed from a fermentor feed stream.

The present disclosure is directed to a method for producing fermentation products such as product alcohols comprising: providing a feedstock slurry comprising fermentable carbon source, undissolved solids, and oil; separating a portion of the undissolved solids and oil from the feedstock slurry whereby an aqueous solution comprising fermentable carbon source, a wet cake comprising solids, and an oil stream are formed; and adding the aqueous solution to a fermentation broth comprising microorganisms whereby a fermentation product is produced. In some aspects, the method may further comprise the step of recovering the oil stream. In some aspects, the method may further comprise the step of washing the wet cake wherein a second aqueous solution comprising carbohydrate is generated. In some aspects, the method may further comprise the step of adding the second aqueous solution to the fermentation broth. In some aspects, the second aqueous solution may be added to feedstock slurry. In some aspects, the second aqueous solution may be added to liquefaction and/or feedstock preparation. In some aspects, the aqueous solution may contain no more than 5% by weight of undissolved solids.

In some aspects, the oil may be corn oil and may comprise one or more of triglycerides, fatty acids, diglycerides, monoglycerides, and phospholipids. In some aspects, the method may further comprise the step of combining a portion of the wet cake and a portion of oil to produce a wet cake comprising triglycerides, fatty acids, diglycerides, monoglycerides, and phospholipids.

In some aspects, the method may further comprise the step of combining the aqueous solution with a portion of the wet cake to produce a mixture of the aqueous solution and wet cake and adding the mixture to the fermentation broth. In some aspects, separating the feedstock slurry may be a single step process. In some aspects, the undissolved solids and oil may be separated from feedstock slurry by decanter bowl centrifugation, three-phase centrifugation, disk stack centrifugation, filtering centrifugation, decanter centrifugation, filtration, microfiltration, vacuum filtration, beltfilter, pressure filtration, membrane filtration, crossflow filtration, drum filter, filtration using a screen, screen separation, rotary screen, grating, porous grating, flotation, hydrocyclone, filter press, screwpress, gravity settler, vortex separator, or combination thereof. In some aspects, the undissolved solids and oil may be separated from feedstock slurry using two or more separation means such as decanter bowl centrifugation, three-phase centrifugation, disk stack centrifugation, filtering centrifugation, decanter centrifugation, filtration, microfiltration, vacuum filtration, beltfilter, pressure filtration, membrane filtration, crossflow filtration, drum filter, filtration using a screen, screen separation, rotary screen, grating, porous grating, flotation, hydrocyclone, filter press, screwpress, gravity settler, vortex separator, or combination thereof. In some aspects, the undissolved solids and oil may be separated from feedstock slurry using two or three separation means.

In some aspects, the two separation means may be three-phase centrifuges. In some aspects, the two separation means may be a three-phase centrifuge and a disk stack centrifuge. In some aspects, the two separation means may be a three-phase centrifuge and a decanter centrifuge. In some aspects, the two separation means may be centrifugation and filtration. In some aspects, the two separation means may be a three-phase centrifuge and crossflow filtration. In some aspects, the two separation means may be a three-phase centrifuge and a drum filter. In some aspects, the two separation means may be a three-phase centrifuge and a rotary screen.

In some aspects, the three separation means may be three-phase centrifuges. In some aspects, the three separation means may be a three-phase centrifuge, and two disk stack centrifuges. In some aspects, the three separation means may be a three-phase centrifuge, and two decanter centrifuges. In some aspects, the three separation means may be a combination of centrifugation and filtration. In some aspects, the three separation means may be a three-phase centrifuge, a rotary screen, and a disk stack centrifuge. In some aspectss, the three separation means may be a three-phase centrifuge, crossflow filtration, and a decanter centrifuge. In some aspects, the three separation means may be a three-phase centrifuge, a rotary screen, and a disk stack centrifuge. In some aspects, the three separation means may be a three-phase centrifuge, crossflow filtration, and a decanter centrifuge.

In some aspects, one or more control parameters of the separation means or devices may be adjusted to improve separation of the feedstock slurry. In some aspects, the one or more control parameters may be differential speed, bowl speed, flow rate, impeller position, weir position, scroll pitch, residence time, discharge volume, or combinations thereof. In some aspects, real-time measurements may be used to monitor separation of the feedstock slurry. In some aspects, separation may be monitored by Fourier transform infrared spectroscopy, near-infrared spectroscopy, Raman spectroscopy, high pressure liquid chromatography, viscometers, densitometers, tensiometers, droplet size analyzers, particle analyzers, or combinations thereof.

In some aspects, the fermentation product may be a product alcohol. In some aspects, the product alcohol may be ethanol, propanol, butanol, pentanol, hexanol, and/or fusel alcohols. In some aspects, the microorganism may comprise a butanol biosynthetic pathway. In some aspects, the butanol biosynthetic pathway may be a 1-butanol biosynthetic pathway, a 2-butanol biosynthetic pathway, or an isobutanol biosynthetic pathway.

The present disclosure is also directed to a method comprising providing a feedstock slurry comprising fermentable carbon source and undissolved solids; separating at least a portion of the undissolved solids from the feedstock slurry whereby an aqueous solution comprising fermentable carbon source and a wet cake comprising solids are generated; contacting the wet cake with a liquid to form a wet cake mixture; and separating at least a portion of undissolved solids from the wet cake mixture whereby a second aqueous solution comprising fermentable carbon source and a second wet cake comprising solids are generated. In some aspects, the liquid may be fresh water, backset, cook water, process water, lutter water, evaporation water, or combinations thereof. In some embodiments, the steps contacting and separating steps may be repeated. In some aspects, the steps contacting and separating steps may be repeated two times. In some aspects, the steps contacting and separating steps may be repeated three times. In some aspects, the steps contacting and separating steps may be repeated four or more times. In some aspects, the second aqueous solution may be added to feedstock slurry. In some aspects, the second aqueous solution may be added to liquefaction and/or feedstock preparation.

The present disclosure is directed to a method comprising: providing a feedstock slurry comprising fermentable carbon source, undissolved solids, and oil; separating at least a portion of the oil and undissolved solids from the feedstock slurry whereby an aqueous solution comprising fermentable carbon source, an oil stream; and a wet cake comprising solids are generated; and contacting the wet cake with a liquid to form a wet cake mixture; and separating at least a portion of undissolved solids and oil from the wet cake mixture whereby a second aqueous solution comprising fermentable carbon source, a second oil stream; and a second wet cake comprising solids are generated. In some aspects, separating the feedstock slurry may be a single step process. In some aspects, the undissolved solids and oil may be separated from feedstock slurry by decanter bowl centrifugation, three-phase centrifugation, disk stack centrifugation, filtering centrifugation, decanter centrifugation, filtration, micro filtration vacuum filtration, beltfilter, pressure filtration, membrane filtration, crossflow filtration, drum filter, filtration using a screen, screen separation, rotary screen, grating, porous grating, flotation, hydrocyclone, filter press, screwpress, gravity settler, vortex separator, or combinations thereof. In some aspects, one or more control parameters of the separation device may be adjusted to improve separation of the feedstock slurry. In some aspects, the one or more control parameters may be differential speed, bowl speed, flow rate, impeller position, weir position, scroll pitch, residence time, discharge volume, or combinations thereof. In some aspects, real-time measurements may be used to monitor separation of the feedstock slurry. In some aspects, separation may be monitored by Fourier transform infrared spectroscopy, near-infrared spectroscopy, Raman spectroscopy, high pressure liquid chromatography, viscometers, densitometers, tensiometers, droplet size analyzers, particle analyzers, or combinations thereof.

The present invention is directed to a method comprising providing a feedstock slurry comprising fermentable carbon source, undissolved solids, and oil; separating the feedstock slurry whereby (i) a first aqueous solution comprising a fermentable carbon source, (ii) a first wet cake comprising solids, and (iii) a stream comprising oil, solids, and an aqueous stream comprising a fermentable carbon source are formed; and adding the first aqueous solution to a fermentation broth comprising microorganisms whereby a fermentation product is produced. The method further comprises separating the stream comprising oil, solids, and aqueous stream comprising a fermentable carbon source whereby (i) a second aqueous solution comprising a fermentable carbon source, (ii) a second wet cake comprising solids, and (iii) an oil stream are formed. The second aqueous solution is added to liquefaction and/or feedstock preparation. In some aspects, the second aqueous solution may be added to fermentation broth. In some aspects, the first and second aqueous solutions may be combined prior to the addition to the fermentation broth. In some embodiments, the second aqueous solution may further comprise oil. In some embodiments, the oil of the second aqueous solution or portion thereof may be treated to generate an extractant. In some embodiments, the oil may be treated chemically or enzymatically. In some embodiments, separation may be a single step process. In some embodiments, separation may be performed by decanter bowl centrifugation, three-phase centrifugation, disk stack centrifugation, filtering centrifugation, decanter centrifugation, filtration, microfiltration, membrane filtration, vacuum filtration, beltfilter, pressure filtration, crossflow filtration, drum filter, filtration using a screen, screen separation, rotary screen, grating, porous grating, flotation, hydrocyclone, filter press, screwpress, gravity settler, vortex separator, or combination thereof.

The present disclosure is directed to a method comprising providing a feedstock slurry comprising a fermentable carbon source, undissolved solids, and oil; separating the feedstock slurry whereby (i) a first aqueous solution comprising a fermentable carbon source and solids, (ii) a first wet cake comprising solids, and (iii) a first oil stream are formed; and adding oil to the first aqueous solution whereby an oil layer comprising solids and a second aqueous solution comprising a fermentable carbon source are formed. In some aspects, the oil layer comprising solids may be separated forming (i) a second oil stream, (ii) a second wet cake comprising solids, and (iii) a third aqueous solution comprising a fermentable carbon source. In some aspects, the second aqueous solution and the third aqueous solution may be added to a fermentation broth comprising microorganisms whereby a fermentation product is produced. In some aspects, the second aqueous solution and the third aqueous solution may further comprise oil. In some aspects, the second aqueous solution and the third aqueous solution may be combined and the oil of the combined second aqueous solution and the third aqueous solution or portions thereof may be treated to generate an extractant. In some aspects, the oil may be treated chemically or enzymatically. In some aspects, separation may be a single step process. In some aspects, separation may be performed by decanter bowl centrifugation, three-phase centrifugation, disk stack centrifugation, filtering centrifugation, decanter centrifugation, filtration, microfiltration, membrane filtration, vacuum filtration, beltfilter, pressure filtration, crossflow filtration, drum filter, filtration using a screen, screen separation, rotary screen, grating, porous grating, flotation, hydrocyclone, filter press, screwpress, gravity settler, vortex separator, or combination thereof.

The present disclosure is also directed to a system comprising one or more liquefaction units configured to liquefy a feedstock to create a feedstock slurry, the liquefaction unit comprising: an inlet for receiving the feedstock; and an outlet for discharging a feedstock slurry, wherein the feedstock slurry comprises fermentable carbon source, oil, and undissolved solids; and one or more separation units configured to remove the oil and undissolved solids from the feedstock slurry to create an aqueous solution comprising the fermentable carbon source, an oil stream, and a wet cake comprising the portion of the undissolved solids, the one or more separation units comprising: an inlet for receiving the feedstock slurry; a first outlet for discharging the aqueous solution; a second outlet for discharging the wet cake; and a third outlet for discharging the oil. In some aspects, the system may further comprise one or more wash systems configured to recover the fermentable carbon source from the wet cake comprising: one or more mixing units; and one or more separation units. In some aspects, the one or more separation units may be decanter bowl centrifugation, three-phase centrifugation, disk stack centrifugation, filtering centrifugation, decanter centrifugation, filtration, microfiltration, vacuum filtration, beltfilter, pressure filtration, membrane filtration, crossflow filtration, drum filter, filtration using a screen, screen separation, rotary screen, grating, porous grating, flotation, hydrocyclone, filter press, screwpress, gravity settler, vortex separator, or combinations thereof. In some aspects, the system may further comprise one or more fermentors configured to ferment the aqueous solution to produce one or fermentation products, the fermentors comprising: an inlet for receiving the aqueous solution and/or wet cake; and an outlet for discharging fermentation broth comprising one or fermentation products. In some aspects, the system may further comprise on-line measurement devices. In some aspects, the on-line measurement devices may be particle size analyzers, Fourier transform infrared spectroscopes, near-infrared spectroscopes, Raman spectroscopes, high pressure liquid chromatography, viscometers, densitometers, tensiometers, droplet size analyzers, pH meters, dissolved oxygen probes, or combinations thereof.

## DESCRIPTION OF THE DRAWINGS

The accompanying drawings, which are incorporated herein and form a part of the specification, illustrate the present invention and, together with the description, further serve to explain the principles of the invention and to enable a person skilled in the pertinent art to make and use the invention.

Figure 1 schematically illustrates an exemplary process and system of the present disclosure, in which undissolved solids are removed by separation after liquefaction and before fermentation.

Figure 2 schematically illustrates an exemplary alternative process and system of the present disclosure, in which feedstock is milled.

Figure 3 schematically illustrates another exemplary alternative process and system of the present disclosure, in which undissolved solids and oil are removed by separation.

Figures 4A and 4B schematically illustrate another exemplary alternative process and system of the present disclosure, in which the wet cake is subjected to one or more wash cycles.

Figures 5A and 5B schematically illustrate another exemplary alternative process and system of the present disclosure, in which undissolved solids and oil are removed by separation and wet cake is subjected to one or more wash cycles.

Figure 6 schematically illustrates another exemplary alternative process and system of the present disclosure, in which the aqueous solution and wet cake are combined and conducted to fermentation.

Figure 7 schematically illustrates another exemplary alternative process and system of the present disclosure, in which the aqueous solution is saccharified prior to fermentation.

Figure 8 schematically illustrates another exemplary alternative process and system of the present disclosure, in which the feedstock slurry is saccharified prior to separation.

Figures 9A-9D and 9F schematically illustrate exemplary alternative processes and systems of the present disclosure, in which additional separation units are utilized to remove undissolved solids and oil. Figure 9E schematically illustrates an exemplary processes and system of the present invention, in which additional separation units are utilized to remove undissolved solids and oil.

Figure 10 schematically illustrates an exemplary fermentation process of the present disclosure utilizing on-line, in-line, at-line, and/or real-time measurements for monitoring fermentation processes.

Figure 11 schematically illustrates an exemplary fermentation process of the present invention including downstream processing.

Figure 12 illustrates the effect of the presence of undissolved corn mash solids on the overall volumetric mass transfer coefficient, $k_La$, for the transfer of *i*-BuOH from an aqueous solution of liquefied corn starch to a dispersion of oleyl alcohol droplets flowing up through a bubble column when a nozzle with an inner diameter of 2.03 mm is used to disperse the oleyl alcohol.

Figure 13 illustrates the effect of the presence of undissolved corn mash solids on the overall volumetric mass transfer coefficient, $k_La$, for the transfer of *i*-BuOH from an aqueous solution of liquefied corn starch to a dispersion of oleyl alcohol droplets flowing up through a bubble column when a nozzle with an inner diameter of 0.76 mm is used to disperse the oleyl alcohol.

Figure 14 illustrates the position of the liquid-liquid interface in the fermentation sample tubes as a function of (gravity) settling time. Phase separation data shown for run times: 5.3, 29.3, 53.3, and 70.3 hr run time. Sample data from extractive-fermentation where solids were removed from the mash feed, and oleyl alcohol was the solvent.

Figure 15 illustrates the position of the liquid-liquid interface of the final fermentation broth as a function of (gravity) settling time. Data from extractive-fermentation where solids were removed from the mash feed, and oleyl alcohol was the solvent.

Figure 16 illustrates the concentration of glucose in the aqueous phase of the slurries as a function of time for Batch 1 and Batch 2.

Figure 17 illustrates concentration of glucose in the aqueous phase of the slurries as a function of time for Batch 3 and Batch 4.

Figure 18 illustrates the effect of enzyme loading and +/- a high temperature stage was applied at some time during the liquefaction on starch conversion.

Figures 19A to 19E illustrate the effect of three-phase centrifuge conditions on separation of feedstock slurry.

DESCRIPTION OF THE INVENTION

Unless defined otherwise, all technical and scientific terms used herein have the same meaning as commonly understood by one of ordinary skill in the art to which this invention belongs. In case of conflict, the present application including the definitions will control. Also, unless otherwise required by context, singular terms shall include pluralities and plural terms shall include the singular.

In order to further define this invention, the following terms and definitions are herein provided.

As used herein, the terms "comprises," "comprising," "includes," "including," "has," "having," "contains," or "containing," or any other variation thereof, will be understood to imply the inclusion of a stated integer or group of integers but not the exclusion of any other integer or group of integers. For example, a composition, a mixture, a process, a method, an article, or an apparatus that comprises a list of elements is not necessarily limited to only those elements but can include other elements not expressly listed or inherent to such composition, mixture, process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be nonrestrictive regarding the number of instances, *i.e.,* occurrences of the element or component. Therefore "a" or "an" should be read to include one or at least one, and the singular word form of the element or component also includes the plural unless the number is obviously meant to be singular.

The term "invention" or "present invention" as used herein is a non-limiting term and is not intended to refer to any single embodiment of the particular invention but encompasses all possible embodiments as described in the application.

As used herein, the term "about" modifying the quantity of an ingredient or reactant of the invention employed refers to variation in the numerical quantity that can occur, for example, through typical measuring and liquid handling procedures used for making concentrates or solutions in the real world; through inadvertent error in these procedures; through differences in the manufacture, source, or purity of the ingredients employed to make the compositions or to carry out the methods; and the like. The term "about" also encompasses amounts that differ due to different equilibrium conditions for a composition resulting from a particular initial mixture. Whether or not modified by the term "about," the claims include equivalents to the quantities. In one embodiment, the term "about" means within 10% of the reported numerical value, alternatively within 5% of the reported numerical value.

"Biomass" as used herein refers to a natural product containing hydrolyzable polysaccharides that provide fermentable sugars including any sugars and starch derived from natural resources such as corn, sugar cane (or cane), wheat, cellulosic or lignocellulosic material, and materials comprising cellulose, hemicellulose, lignin, starch, oligosaccharides, disaccharides, and/or monosaccharides, and mixtures thereof. Biomass may also comprise additional components such as protein and/or lipids. Biomass may be derived from a single source or biomass may comprise a mixture derived from more than one source. For example, biomass may comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, and wood and forestry waste (e.g., forest thinnings). Examples of biomass include, but are not limited to, corn grain, corn cobs, crop residues such as corn husks, corn stover, grasses, wheat, rye, wheat straw, spelt, triticale, barley, barley straw, oats, hay, rice, rice straw, switchgrass, potato, sweet potato, cassava, Jerusalem artichoke, sugar cane bagasse, sorghum, sugar cane, sugar beet, fodder beet, soy, palm, coconut, rapeseed, safflower, sunflower, millet, eucalyptus, miscanthus, waste paper, components obtained from milling of grains, trees, branches, roots, leaves, wood chips, sawdust, shrubs and bushes, vegetables, fruits, flowers, animal manure, and mixtures thereof. Mash, juice, molasses, or hydrolysate may be formed from biomass by any method known in the art for processing biomass for purposes of fermentation such as milling, treating (e.g., enzymatic, chemical), and/or liquefying. Treated biomass may comprise fermentable sugar and/or water. Cellulosic and/or lignocellulosic biomass may be processed to obtain a hydrolysate containing fermentable sugars by any method known to one skilled in the art. For example, a low ammonia pretreatment is disclosed in U.S. Patent Application Publication No. 2007/0031918A1. Enzymatic saccharification of cellulosic and/or lignocellulosic biomass typically makes use of enzyme mixtures for hydrolysis of cellulose and hemicellulose to produce a hydrolysate containing sugars including glucose, xylose, and arabinose. Saccharification enzymes suitable for cellulosic and/or lignocellulosic biomass are reviewed in Lynd, et al. (Microbiol. Mol. Biol. Rev. 66:506-577, 2002).

"Fermentable carbon source" or "fermentable carbon substrate" as used herein refers to a carbon source capable of being metabolized by microorganisms. Suitable fermentable carbon sources include, but are not limited to, monosaccharides such as glucose or fructose; disaccharides such as lactose or sucrose; oligosaccharides; polysaccharides such as starch or cellulose; one carbon substrates; and mixtures thereof.

"Fermentable sugar" as used herein refers to one or more sugars capable of being metabolized by microorganisms for the production of fermentation products such as alcohols.

"Feedstock" as used herein refers to a feed in a fermentation process. The feed may comprise a fermentable carbon source and may comprise undissolved solids and/or oil. Where applicable, the feed may comprise a fermentable carbon source before or after the fermentable carbon source has been liberated from starch or obtained from the hydrolysis of complex sugars by further processing such as by liquefaction, saccharification, or other process. Feedstock includes or may be derived from biomass. Suitable feedstocks include, but are not limited to, rye, wheat, corn, corn mash, sugar cane, cane mash, barley, cellulosic material, lignocellulosic material, or mixtures thereof. Where reference is made to "feedstock oil," it will be appreciated that the term encompasses the oil produced from a given feedstock.

"Fermentation broth" as used herein refers to a mixture of water, fermentable carbon sources (e.g., sugars, starch), dissolved solids, optionally microorganisms producing fermentation products, optionally fermentation products (e.g., product alcohols), optionally undissolved solids, and other constituents of the material held in the fermentor in which fermentation product is being made by the metabolism of fermentable carbon sources by the microorganisms to form fermentation products, water, and carbon dioxide ($CO_2$). From time to time as used herein, the term "fermentation medium" and "fermented mixture" may be used synonymously with "fermentation broth."

"Fermentor" or "fermentation vessel" as used herein refers to a vessel, unit, or tank in which the fermentation reaction is carried out whereby fermentation product (e.g., product alcohols such as ethanol or butanol) is made from fermentable carbon sources. Fermentor may also refer to a vessel, unit, or tank in which growth of microorganism occurs. In some instances, both microbial growth and fermentation may occur in a fermentor. The term "fermentor" may be used synonymously herein with "fermentation vessel."

"Saccharification vessel" as used herein refers to a vessel, unit, or tank in which saccharification (i.e., the hydrolysis of oligosaccharides to monosaccharides) is carried out. Where fermentation and saccharification occur simultaneously, the saccharification vessel and the fermentor may be the same vessel.

"Saccharification enzyme" as used herein refers to one or more enzymes that are capable of hydrolyzing polysaccharides and/or oligosaccharides, for example, alpha-1,4-glucosidic bonds of glycogen, or starch. Saccharification enzymes may include enzymes capable of hydrolyzing cellulosic or lignocellulosic materials as well.

"Liquefaction vessel" as used herein refers to a vessel, unit, or tank in which liquefaction is carried out. Liquefaction is a process in which starch is hydrolyzed, for example, by an enzymatic process to obtain oligosaccharides. In embodiments where the feedstock is corn, oligosaccharides are hydrolyzed from the corn starch content during liquefaction.

"Sugar" as used herein refers to oligosaccharides, disaccharides, monosaccharides, and/or mixtures thereof. The term "saccharide" may also include carbohydrates such as starches, dextrans, glycogens, cellulose, pentosans, as well as sugars.

"Undissolved solids" as used herein refers to non-fermentable portions of feedstock which are not dissolved in the liquid or aqueous phase, for example, germ, fiber, and gluten. The non-fermentable portions of feedstock include the portion of feedstock that remains as solids and can absorb liquid from the fermentation broth. From time to time as used herein, the term "undissolved solids" may be used synonymously with "solids" or "suspended solids."

"Extractant" as used herein refers to a solvent used to extract a fermentation product. From time to time as used herein, the term "extractant" may be used synonymously with "solvent."

"In Situ Product Removal" (ISPR) as used herein refers to the selective removal of a product from a biological process such as fermentation to control the product concentration in the biological process as the product is produced.

"Product alcohol" as used herein refers to any alcohol that may be produced by a microorganism in a fermentation process that utilizes biomass as a fermentable carbon source. Product alcohols include, but are not limited to, $C_1$ to $C_8$ alkyl alcohols. In some embodiments, the product alcohols are $C_2$ to $C_8$ alkyl alcohols. In other embodiments, the product alcohols are $C_2$ to $C_5$ alkyl alcohols. It will be appreciated that $C_1$ to $C_8$ alkyl alcohols include, but are not limited to, methanol, ethanol, propanol, butanol, pentanol, hexanol, and isomers thereof. Likewise, $C_2$ to $C_8$ alkyl alcohols include, but are not limited to, ethanol, propanol, butanol, pentanol, hexanol, and isomers thereof. The term "alcohol" may also be used herein with reference to a product alcohol.

"Butanol" as used herein refers to butanol isomers: 1-butanol (1-BuOH), 2-butanol (2-BuOH), tertiary-butanol (tert-BuOH), and/or isobutanol (iBuOH, *i*-BuOH, or I-BUOH), either individually or as mixtures thereof.

"Propanol" as used herein refers to the propanol isomers: isopropanol or 1-propanol, either individually or as mixtures thereof.

"Pentanol" as used herein refers to the pentanol isomers: 1-pentanol, 3-methyl-1-butanol, 2-methyl-1-butanol, 2,2-dimethyl-1-propanol, 3-pentanol, 2-pentanol, 3-methyl-2-butanol, or 2-methyl-2-butanol, either individually or as mixtures thereof.

"Effective titer" as used herein refers to the total amount of a particular fermentation product (e.g., product alcohol) produced by fermentation. In some embodiments wherein the fermentation product is a product alcohol, effective titer may refer to the alcohol equivalent of an alcohol ester produced by alcohol esterification per liter of fermentation medium.

"Water-immiscible" or "insoluble" as used herein refers to a chemical component such as an extractant or solvent, which is incapable of mixing with an aqueous solution such as a fermentation broth, in such a manner as to form one liquid phase.

"Aqueous phase" as used herein refers to the aqueous phase of at least a biphasic mixture obtained by contacting a fermentation broth with an extractant, for example, a water-immiscible organic extractant. In an embodiment of a process described herein that includes fermentative extraction, the term "fermentation broth" then may refer to the aqueous phase in biphasic fermentative extraction.

"Aqueous phase titer" as used herein refers to the concentration of a fermentation product (e.g., product alcohol) in the aqueous phase.

"Organic phase" as used herein refers to the non-aqueous phase of at least a biphasic mixture obtained by contacting a fermentation broth with an extractant, for example, a water-immiscible organic extractant.

"Portion" as used herein with reference to a process stream refers to any fractional part of the stream which retains the composition of the stream, including the entire stream, as well as any component or components of the stream, including all components of the stream.

"By-product" or "co-product" as used herein refers to a product produced during the production of another product. In some instances, the term "co-product" may be used synonymously with the term "by-product." Co-products include for example, oil recovered from the feedstock slurry, wet cake, and DDGS. Co-products may also include modification of the oil, wet cake, and DDGS for the purposes of improving value and/or for the manufacture of other products, such as biodiesel from the oil.

"Distillers co-products" as used herein refers to by-products from a product alcohol production process that can be isolated before or during fermentation. Distillers co-products include non-fermentable products remaining after product alcohol is removed from a fermented mash and solids isolated from a mash. As used herein, distillers co-products may be used in a variety of animal feed and non-animal feed applications. Examples of distillers co-products include, but are not limited to, fatty acids from oil hydrolysis, lipids from evaporation of thin stillage, syrup, distillers grains, distillers grains and solubles, solids from mash before fermentation, solids from whole stillage after fermentation, biodiesel, and acyl glycerides.

"Distillers co-products for animal feed" as used herein refers to distillers co-products that are suitable for use in or as animal feed. Examples of distillers co-products for animal feed include, but are not limited to, fatty acids from oil hydrolysis, lipids from evaporation of thin stillage, syrup, distillers grains, distillers grains and solubles, solids from mash before fermentation, and solids from whole stillage after fermentation.

"Distillers grains" or "DG" as used herein refer to the non-fermentable products remaining after product alcohol is removed from a fermented mash. Distillers grains that are dried are known as "distillers dried grains" or "DDG." Distillers grains that are not dried are known as "wet distillers grains" or "WDG."

"Distillers grains and solubles" or "DGS" as used herein refer to the non-fermentable products remaining after product alcohol is removed from a fermented mash, that have been blended with solubles. Distillers grains and solubles that are dried are known as "distillers dried grains and solubles" or "DDGS." Distillers grains and solubles that are not dried are known as "wet distillers grains and solubles" or "WDGS."

"Dried Distillers Grains with Solubles" (DDGS) as used herein refer to a co-product or by-product from a fermentation of a feedstock or biomass (e.g., fermentation of grain or grain mixture that produces a product alcohol). In some embodiments, DDGS may also refer to an animal feed produced from a process of making a product alcohol.

"Lipid" as used herein refers to any of a heterogeneous group of fats and fat-like substances including fatty acids, neutral fats, waxes, and steroids, which are water-insoluble and soluble in nonpolar solvents. Examples of lipids include monoglycerides, diglycerides, triglycerides, and phospholipids.

"Lipids from evaporation" as used herein in reference to a process stream refer to a lipid by-product produced by evaporation and centrifugation of thin stillage following fermentation in a product alcohol production process.

"Syrup" or "condensed distillers solubles" (CDS) as used herein in reference to a process stream refers to a by-product produced by evaporation of thin stillage following fermentation in a product alcohol production process.

"Process stream" as used herein refers to any by-product or co-product formed by a fermentation product production process. Examples of process streams include, but are not limited to, COFA, lipids from evaporation, syrup, DG, DDG, WDG, DGS, DDGS, and WDGS. Another example of a process stream is solids removed (e.g., by centrifugation) from a mash before fermentation in a fermentation product production process (e.g., the solids removed from a corn mash before fermentation). These solids may be referred to as "wet cake" when they have not been dried, and may be referred to as "dry cake" when they have been dried. Another example of a process stream is solids removed (e.g., by centrifugation) from whole stillage following fermentation in a fermentation product production process. These solids may be referred to as "WS wet cake" when they have not been dried, and may be referred to as "WS dry cake" when they have been dried. Process stream may also refer to any stream of the fermentation process such as feedstock slurry, aqueous solutions and streams, extractant, product stream, and the like. From time to time as used herein, the term "process stream" may be used synonymously with "stream."

The present invention provides processes and methods for producing fermentation products such as product alcohols using fermentation. Other fermentation products that may be produced using the processes and methods described herein include propanediol, butanediol, acetone; acids such as lactic acid, acetic acid, butyric acid, and propionic acid; gases such as hydrogen methane, and carbon dioxide; amino acids; vitamins such as biotin, vitamin B2 (riboflavin), vitamin B12 (e.g., cobalamin), ascorbic acid (e.g., vitamin C), vitamin E (e.g., a-tocopherol), and vitamin K (e.g., menaquinone); antibiotics such as erythromycin, penicillin, streptomycin, and tetracycline; and other products such as citric acid, invertase, sorbitol, pectinase, and xylitol.

As an example of the processes and methods provided herein, in an aspect, a feedstock may be liquefied to create a feedstock slurry which comprises a fermentable carbon source (e.g., soluble sugar) and undissolved solids. In some instances, the terms "feedstock slurry" and "mash" may be used interchangeably. In some embodiments, the feedstock slurry comprises soluble sugar, undissolved solids, and oil. If the feedstock slurry is fed directly to a fermentor, the undissolved solids and/or oil may interfere with efficient removal and recovery of the fermentation product such as a product alcohol. For example, if liquid-liquid extraction is utilized to extract product alcohol from fermentation broth, the presence of undissolved solids may cause system inefficiencies including, but not limited to, decreasing the mass transfer rate of the product alcohol to the extractant by interfering with the contact between the extractant and the fermentation broth; creating an emulsion in the fermentor and thereby interfering with phase separation of the extractant and the fermentation broth; slowing disengagement of the extractant from the fermentation broth; reducing the efficiency of recovering and recycling the extractant because at least a portion of the extractant and product alcohol becomes "trapped" in the solids; shortening the life cycle of the extractant by contamination with oil; and lowering fermentor volume efficiency because there are solids taking up volume in the fermentor. These effects can result in higher capital and operating costs. In addition, the extractant "trapped" in undissolved solids used to generate Distillers Dried Grains with Solubles (DDGS), may detract from DDGS value and qualification for sale as animal feed. Therefore, in order to avoid and/or minimize these problems, at least a portion of the undissolved solids may be removed from the feedstock slurry prior to the addition of the feedstock slurry to the fermentor. Extraction activity and the efficiency of product alcohol production can be increased when extraction is performed on fermentation broth containing an aqueous solution where undissolved solids have been removed relative to extraction performed on fermentation broth containing an aqueous solution where undissolved solids have not been removed.

The processes and systems of the present invention will be described with reference to the Figures. In some aspects, as shown, for example, in Figure 1, the system includes liquefaction 10 configured to liquefy a feedstock to create a feedstock slurry.

For example, feedstock 12 may be introduced to an inlet in liquefaction 10. Feedstock 12 may be any suitable biomass material that contains a fermentable carbon source such as starch including, but not limited to, barley, oat, rye, sorghum, wheat, triticale, spelt, millet, cane, corn, or combinations thereof. Water may also be introduced to liquefaction 10.

The process of liquefying feedstock 12 involves hydrolysis of feedstock 12 generating water-soluble sugars.

Any known liquefying processes utilized by the industry may be used including, but not limited to, an acid process, an enzyme process, or an acid-enzyme process. Such processes may be used alone or in combination. In some aspects, the enzyme process may be utilized and an appropriate enzyme 14, for example, alpha-amylase, is introduced to an inlet in liquefaction 10. Examples of alpha-amylases that may be used in the processes and systems of the present invention are described in U.S. Patent No. 7,541,026; U.S. Patent Application Publication No. 2009/0209026; U.S. Patent Application Publication No. 2009/0238923; U.S. Patent Application Publication No. 2009/0252828; U.S. Patent Application Publication No. 2009/0314286; U.S. Patent Application Publication No. 2010/02278970; U.S. Patent Application Publication No. 2010/0048446; and U.S. Patent Application Publication No. 2010/0021587.

In some aspects, enzymes for liquefaction and/or saccharification may be produced by the microorganism (e.g., microorganism 32). Examples of microorganisms producing such enzymes are described in U.S. Patent No. 7,498,159; U.S. Patent Application Publication No. 2012/0003701; U.S. Patent Application Publication No. 2012/0129229; PCT International Publication No. WO 2010/096562; and PCT International Publication No. WO 2011/153516.

The process of liquefying feedstock 12 produces feedstock slurry 16 that comprises a fermentable carbon source and undissolved solids. In some aspects, feedstock slurry 16 may comprise a fermentable carbon source, oil, and undissolved solids. The undissolved solids are non-fermentable portions of feedstock 12. In some embodiments, feedstock 12 may be corn, such as dry milled, unfractionated corn kernels, and the undissolved solids may comprise germ, fiber, and gluten. In some aspects, feedstock 12 is corn or corn kernels and feedstock slurry 16 is corn mash slurry. Feedstock slurry 16 may be discharged from an outlet of liquefaction 10 and conducted to separation 20.

In some aspects, nutrients such as amino acids, nitrogen, minerals, trace elements, and/or vitamins may be added to feedstock slurry 16 or fermentation 30. For example, one or more of the following: biotin, pantothenate, folic acid, niacin, aminobenzoic acid, pyridoxine, riboflavin, thiamine, vitamin A, vitamin C, vitamin D, vitamin E, vitamin K, inositol, potassium (e.g., potassium phosphate), boric acid, calcium (e.g., calcium chloride), chromium, copper (e.g., copper sulfate), iodide (e.g., potassium iodide), iron (e.g., ferric chloride), lithium, magnesium (e.g., magnesium sulfate, magnesium chloride), manganese (e.g., manganese sulfate), molybdenum, phosphorus, potassium, sodium chloride, vanadium, zinc (e.g., zinc sulfate), yeast extract, soy peptone, and the like may be added to feedstock slurry 16 or fermentation 30. Examples of amino acids include essential amino acids such as histidine, isoleucine, leucine, lysine, methionine, phenylalanine, threonine, tryptophan, and valine as well as other amine acids such as alanine, arginine, aspartic acid, asparagine, cysteine, glutamic acid, glutamine, glycine, hydroxylysine, hydroxyproline, ornithine, proline, serine, and tyrosine.

Separation 20 via an inlet may be configured to remove undissolved solids from feedstock slurry 16. Separation 20 may also be configured to remove oil, or to remove both oil and undissolved solids. Separation 20 may be any device capable of separating solids and liquids. For example, separation 20 may be any conventional centrifuge utilized in the industry, including, for example, a decanter bowl centrifuge, three-phase centrifuge, disk stack centrifuge, filtering centrifuge, or decanter centrifuge. In some aspects, separation may be accomplished by filtration, microfiltration, vacuum filtration, beltfilter, membrane filtration, crossflow filtration, drum filter, pressure filtration, filtration using a screen, screen separation, rotary screen, grates or grating, porous grating, flotation, hydrocyclone, filter press, screwpress, gravity settler, vortex separator, or any method or separation device that may be used to separate solids and liquids.

Feedstock slurry 16, conducted to separation 20, may be separated to form a liquid phase or aqueous solution 22 (also known as thin mash) and a solid phase, solid stream, or wet cake 24. Aqueous solution 22 may comprise sugar, for example, in the form of oligosaccharides, and water. In some aspects, aqueous solution 22 may comprise at least 10% by weight oligosaccharides, at least 20% by weight of oligosaccharides, or at least 30% by weight of oligosaccharides. In some aspects, aqueous solution 22 may be discharged from an outlet located near the top of separation 20. In some aspects, aqueous solution 22 may have a viscosity of less than 20 centipoise (cP). In some aspects, aqueous solution 22 may comprise less than 20 g/L of monomeric glucose, less than 10 g/L of monomeric glucose, or less than 5 g/L of monomeric glucose. Suitable methodology to determine the amount of monomeric glucose is well known in the art such as high performance liquid chromatography (HPLC).

Wet cake 24 may be discharged from separation 20. In some aspects, wet cake 24 may be discharged from an outlet located near the bottom of separation 20. Wet cake 24 may comprise undissolved solids. In some aspects, wet cake 24 may also comprise a portion of sugar and water. Wet cake 24 may be washed with additional water using separation 20 once aqueous solution 22 has been discharged from separation 20. In some aspects, wet cake 24 may be washed with additional water using additional separation devices. Washing wet cake 24 will recover the sugar or sugar source (e.g., oligosaccharides) present in the wet cake, and the recovered sugar and water may be recycled to liquefaction 10. After washing, wet cake 24 may be processed to form DDGS using any suitable known process. The formation of DDGS from wet cake 24 has several benefits. For example, since the undissolved solids are not added to the fermentor, the undissolved solids are not subjected to the conditions of the fermentor and therefore, the undissolved solids do not contact the microorganisms present in the fermentor, and fermentation product such as product alcohol or other components such as extractant are not trapped in the undissolved solids. These effects provide benefits to subsequent processing and use of DDGS, for example, as animal feed because the DDGS would not contain microorganism or other components (e.g., product alcohol, extractant) of the fermentation broth.

In some aspects, undissolved solids may be separated from feedstock slurry to form two product streams, for example, an aqueous solution of oligosaccharides which contains a lower concentration of solids as compared to the feedstock slurry, and a wet cake which contains a higher concentration of solids as compared to the feedstock slurry. In addition, a third stream containing oil may be generated. As such, a number of product streams may be generated by using different separation techniques or a combination thereof. As an example, feedstock slurry 16 may be separated using a three-phase centrifuge. A three-phase centrifuge allows for three-phase separation yielding two liquid phases (e.g., aqueous stream and oil stream) and a solid stream (e.g., solids or wet cake) (see, e.g., Flottweg Tricanter®, Flottweg AG, Vilsibiburg, Germany). The two liquid phases may be separated and decanted, for example, from a bowl via two discharge systems to prevent cross-contamination and the solids stream may be removed via a separate discharge system.

In some aspects using corn as feedstock 12, a three-phase centrifuge may be used to remove solids and corn oil simultaneously from feedstock slurry 16 (e.g., liquefied corn mash). The solids are the undissolved solids remaining after the starch is hydrolyzed to soluble oligosaccharides during liquefaction, and the corn oil is free oil that is released from the germ during grinding and/or liquefaction. In some aspects, the three-phase centrifuge may have one feed stream and three outlet streams. The feed stream may consist of liquefied corn mash produced during liquefaction. The mash may consist of an aqueous solution of liquefied starch (e.g., oligosaccharides); undissolved solids which comprise insoluble, non-starch components from the corn; and corn oil which comprise glycerides and free fatty acids. The three outlet streams from the three-phase centrifuge may be a wet cake (e.g., wet cake 24) which contains the undissolved solids from the feedstock slurry; a heavy centrate stream which contains the liquefied starch from the feedstock slurry; and a light centrate stream which contains the corn oil from the feedstock slurry. In some aspects, the light centrate stream (e.g., oil 26) may be conducted to a storage tank or any vessel that is suitable for oil storage. In some aspects, the oil may be sold as a co-product, converted to another co-product, or used in processing such as the case in converting corn oil to corn oil fatty acids. In some aspects, the heavy centrate stream (e.g., aqueous solution 22) may be used for fermentation. In some aspects, the wet cake may be washed with process recycle water, such as evaporator condensate and/or backset as described herein, to recover the soluble starch in the liquid phase of the cake.

In some aspects, wet cake 24 is a composition formed from feedstock slurry 16, and may comprise at least 50% by weight of the undissolved solids present in the feedstock slurry, at least 55% by weight of the undissolved solids present in the feedstock slurry, at least 60% by weight of the undissolved solids present in the feedstock slurry, at least 65% by weight of the undissolved solids present in the feedstock slurry, at least 70% by weight of the undissolved solids present in the feedstock slurry, at least 75% by weight of the undissolved solids present in the feedstock slurry, at least 80% by weight of the undissolved solids present in the feedstock slurry, at least 85% by weight of the undissolved solids present in the feedstock slurry, at least 90% by weight of the undissolved solids present in the feedstock slurry, at least 95% by weight of the undissolved solids present in the feedstock slurry, or at least 99% by weight of the undissolved solids present in the feedstock slurry.

In some aspects, aqueous solution 22, formed from feedstock slurry 16, may comprise no more than 50% by weight of the undissolved solids present in the feedstock slurry, no more than 45% by weight of the undissolved solids present in the feedstock slurry, no more than 40% by weight of the undissolved solids present in the feedstock slurry, no more than 35% by weight of the undissolved solids present in the feedstock slurry, no more than 30% by weight of the undissolved solids present in the feedstock slurry, no more than 25% by weight of the undissolved solids present in the feedstock slurry, no more than 20% by weight of the undissolved solids present in the feedstock slurry, no more than 15% by weight of the undissolved solids present in the feedstock slurry, no more than 10% by weight of the undissolved solids present in the feedstock slurry, no more than 5% by weight of the undissolved solids present in the feedstock slurry, or 1% by weight of the undissolved solids present in the feedstock slurry.

Fermentation 30 configured to ferment aqueous solution 22 to produce a fermentation product such as a product alcohol has an inlet for receiving aqueous solution 22. Fermentation 30 may include fermentation broth. In some aspects, microorganism 32 may be bacteria, cyanobacteria, filamentous fungi, or yeasts, and may be introduced to fermentation 30 to be included in the fermentation broth. In some aspects, microorganism 32 may be bacteria such as *Escherichia coli.* In some aspects, microorganism 32 may be *Saccharomyces cerevisiae.* In some aspects, microorganism 32 consumes the sugar in aqueous solution 22 and produces a product alcohol such as ethanol or butanol. In some aspects, microorganism 32 may be a recombinant microorganism.

In some aspects, microorganism 32 may be engineered to contain a biosynthetic pathway. In some aspects, the biosynthetic pathway may be a butanol biosynthetic pathway. In some aspects, the butanol biosynthetic pathway may be a 1-butanol biosynthetic pathway, a 2-butanol biosynthetic pathway, or an isobutanol biosynthetic pathway. In some aspects, the biosynthetic pathway converts pyruvate to a fermentation product. In some aspects, the biosynthetic pathway converts pyruvate as well as amino acids to a fermentation product. In some aspects, the biosynthetic pathway comprises at least one heterologous polynucleotide encoding a polypeptide which catalyzes a substrate to product conversion of the biosynthetic pathway. In some aspects, each substrate to product conversion of the biosynthetic pathway is catalyzed by a polypeptide encoded by a heterologous polynucleotide. Examples of the production of a product alcohol by a microorganism comprising a biosynthetic pathway are disclosed, for example, in U.S. Patent No. 7,851,188, and U.S. Patent Application Publication Nos. 2007/0092957; 2007/0259410; 2007/0292927; 2008/0182308; 2008/0274525; 2009/0155870; 2009/0305363; and 2009/0305370.

In some aspects, microorganism 32 may also be immobilized, such as by adsorption, covalent bonding, crosslinking, entrapment, and encapsulation. Methods for encapsulating cells are known in the art such as methods described in U.S. Patent Application Publication No. 2011/0306116.

In some aspects of the processes and systems described herein, in situ product removal (ISPR) may be utilized to remove a fermentation product such as a product alcohol from fermentation broth. In some aspects, ISPR may be conducted in fermentation 30 as the fermentation product is produced by the microorganism, or external to fermentation 30, using, for example, liquid-liquid extraction. Methods for producing and recovering product alcohols from a fermentation broth using extractive fermentation are described in U.S. Patent Application Publication No. 2009/0305370; U.S. Patent Application Publication No. 2010/0221802; U.S. Patent Application Publication No. 2011/0097773; U.S. Patent Application Publication No. 2011/0312044; U.S. Patent Application Publication No. 2011/0312043; and U.S. Patent Application Publication No. 2012/0156738.

In some aspects, fermentation 30 may have an inlet for receiving extractant 34. In some aspects, extractant 34 may be added to the fermentation broth external to fermentation 30. In some aspects, extractant 34 may be added to an external extractor or external extraction loop. Alternative means of additions of extraction 34 to fermentation 30 or external to fermentation 30 are represented by the dotted lines. In some aspects, extractant 34 may be immiscible organic solvents. In some aspects, extractant 34 may be water-immiscible organic solvents. In some aspects, extractant 34 may be an organic extractant selected from the group consisting of saturated, monounsaturated, polyunsaturated compounds, and mixtures thereof. In some aspects, extractant 34 may be selected from the group consisting of $C_{12}$ to $C_{22}$ fatty alcohols, $C_{12}$ to $C_{22}$ fatty acids, esters of $C_{12}$ to $C_{22}$ fatty acids, $C_{12}$ to $C_{22}$ fatty aldehydes, $C_{12}$ to $C_{22}$ fatty amides, and mixtures thereof. In some aspects, extractant 34 may also be selected from the group consisting of $C_4$ to $C_{22}$ fatty alcohols, $C_4$ to $C_{28}$ fatty acids, esters of $C_4$ to $C_{28}$ fatty acids, $C_4$ to $C_{22}$ fatty aldehydes, and mixtures thereof. In some aspects, extractant 34 may include a first extractant selected from $C_{12}$ to $C_{22}$ fatty alcohols, $C_{12}$ to $C_{22}$ fatty acids, esters of $C_{12}$ to $C_{22}$ fatty acids, $C_{12}$ to $C_{22}$ fatty aldehydes, $C_{12}$ to $C_{22}$ fatty amides, and mixtures thereof; and a second extractant selected from $C_7$ to $C_{11}$ fatty alcohols, $C_7$ to $C_{11}$ fatty acids, esters of $C_7$ to $C_{11}$ fatty acids, $C_7$ to $C_{11}$ fatty aldehydes, and mixtures thereof In some aspects, extractant 34 may be carboxylic acids. In some aspects, extractant 34 may be corn oil fatty acids (COFA) or soybean oil fatty acids (SOFA). In some aspects, extractant 34 may be an organic extractant such as oleyl alcohol, behenyl alcohol, cetyl alcohol, lauryl alcohol, myristyl alcohol, stearyl alcohol, oleic acid, lauric acid, linoleic acid, linolenic acid, myristic acid, stearic acid, octanoic acid, decanoic acid, undecanoic acid, methyl myristate, methyl oleate, 1-nonanol, 1-decanol, 2-undecanol, 1-nonanal, 1-undecanol, undecanal, lauric aldehyde, 2-methylundecanal, oleamide, linoleamide, palmitamide, stearylamide, 2-ethyl-1-hexanol, 2-hexyl-1-decanol, 2-octyl-1-dodecanol, and mixtures thereof. For the processes and systems described herein and as illustrated in the figures, extractant may be added to the fermentor or an external extractor.

In some aspects, the extractant may be selected based upon certain properties. For example, the extractant may have a high $K_d$. $K_d$ refers to the partition coefficient of the fermentation product (e.g., product alcohol) between the extractant phase (e.g., organic phase) and aqueous phase. In some embodiments, the extractant may have a high selectivity. For example, selectivity refers to the relative amounts of product alcohol to water taken up by the extractant.

In some aspects, the extractant may be biocompatible. In some aspects, biocompatible refers to a measure of the ability of a microorganism to utilize fermentable carbon sources in the presence of an extractant. In some aspects, the extractant may be a mixture of biocompatible and non-biocompatible extractants. In some aspects, a non-biocompatible extractant refers to an extractant that interferes with the ability of a microorganism to utilize fermentable carbon sources. For example, in the presence of a non-biocompatible extractant, the microorganism does not utilize fermentable carbon sources at a rate greater than 50% of the rate when the extractant is not present. In some aspects, in the presence of a non-biocompatible extractant, the microorganism does not utilize fermentable carbon sources at a rate greater than 25% of the rate when the extractant is not present. Examples of mixtures of biocompatible and non-biocompatible extractants include, but are not limited to, oleyl alcohol and nonanol, oleyl alcohol and 1-undecanol, oleyl alcohol and 2-undecanol, oleyl alcohol and 1-nonanal, oleyl alcohol and decanol, and oleyl alcohol and dodecanol. Additional examples of biocompatible and non-biocompatible extractants are described in U.S. Patent Application Publication No. 2011/0097773.

Extractant 34 contacts the fermentation broth forming stream 36 comprising a biphasic mixture (i.e., aqueous phase and organic phase). In the case that the fermentation product is a product alcohol, product alcohol present in the fermentation broth is transferred to extractant 34 forming extractant rich with product alcohol (e.g., organic phase). In some aspects, stream 36 may be discharged through an outlet in fermentation 30. Product alcohol may be separated from the extractant in stream 36 using conventional techniques. Feed stream may be added to fermentation 30. Fermentation 30 can be any suitable fermentor known in the art.

In some aspects, where extractant 34 is not added to the fermentation broth, stream 36 comprises fermentation broth and product alcohol. Stream 36 or a portion thereof comprising product alcohol and fermentation broth may be discharged from fermentation 30 and further processed for recovery of product alcohol. In some aspects, the processed fermentation broth may be recycled to fermentation 30.

In some aspects, simultaneous saccharification and fermentation (SSF) may occur in fermentation 30. Any known saccharification process utilized by the industry may be used including, but not limited to, an acid process, an enzyme process, or an acid-enzyme process. In some aspects, enzyme 38 such as glucoamylase, may be introduced to hydrolyze sugars (e.g., oligosaccharides) in feedstock slurry 16 or aqueous solution 22 to form monosaccharides. Examples of glucoamylases that may be used in the processes and systems of the present invention are described in U.S. Patent No. 7,413,887; U.S. Patent No. 7,723,079; U.S. Patent Application Publication No. 2009/0275080; U.S. Patent Application Publication No. 2010/0267114; U.S. Patent Application Publication No. 2011/0014681; U.S. Patent Application Publication No. 2011/0020899. In some aspects, the glucoamylase may be expressed by a recombinant microorganism that also produces the fermentation product (e.g., product alcohol).

In some aspects, enzymes such as glucoamylases may be added to liquefaction. The addition of enzymes such as glucoamylases to liquefaction may reduce the viscosity of the feedstock slurry or liquefied mash, and may improve separation efficiency. In some aspects, any enzyme capable of reducing the viscosity of the feedstock slurry may be used (e.g., Viscozyme®, Sigma-Aldrich, St. Louis, MO). Viscosity of the feedstock may be measured by any method known in the art, including the method described in Example 22.

In some aspects, stream 35 may be discharged from an outlet in fermentation 30. The discharged stream 35 may include microorganism 32 such as yeast. Microorganism 32 may be separated from the stream 35, for example, by centrifugation (not shown). Microorganism 32 may then be recycled to fermentation 30 which over time can increase the production rate of product alcohol, thereby resulting in an increase in the efficiency of product alcohol production.

When a portion of stream 35 exits fermentation 30, stream 35 may include no more than 50% by weight of the undissolved solids present in the feedstock slurry, no more than 45% by weight of the undissolved solids present in the feedstock slurry, no more than 40% by weight of the undissolved solids present in the feedstock slurry, no more than 35% by weight of the undissolved solids present in the feedstock slurry, no more than 30% by weight of the undissolved solids present in the feedstock slurry, no more than 25% by weight of the undissolved solids present in the feedstock slurry, no more than 20% by weight of the undissolved solids present in the feedstock slurry, no more than 15% by weight of the undissolved solids present in the feedstock slurry, no more than 10% by weight of the undissolved solids present in the feedstock slurry, no more than 5% by weight of the undissolved solids present in the feedstock slurry, or no more than 1% by weight of the undissolved solids present in the feedstock slurry.

In some aspects, as shown, for example, in Figure 2, the processes and systems of the present invention may include mill 40 configured to dry mill feedstock 12. Feedstock 12 may enter mill 40 through an inlet, and mill 40 may mill or grind feedstock 12. In some aspects, feedstock 12 may be unfractionated. In some aspects, feedstock 12 may be unfractionated corn kernels. Mill 40 may be any suitable known mill, for example, a hammer mill. Dry milled feedstock 44 is discharged from mill 40 through an outlet and enters liquefaction 10. The remainder of Figure 2 is similar to Figure 1, and therefore will not be described in detail again. In other aspects, the feedstock may be fractionated and/or wet milled as is known in the industry as an alternative to being unfractionated and/or dry milled.

Wet milling is a multi-step process that separates biomass into several components such as germ, pericarp fiber, starch, and gluten in order to capture value from each co-product separately. Using corn as a feedstock, this process produces several co-products: starch, gluten feed, gluten meal, and corn oil streams. These streams may be recombined and processed to produce customized products for the feed industry. As an example of a wet milling process, feedstock (e.g., corn) may be conducted to steeping tanks where it is soaked, for example, in a sodium dioxide solution for 30-50 hours at 120-130°F (50-55°C). Nutrients released into the water may be collected and evaporated to produce condensed fermented extractives (or steep liquor). Germ may be removed from the soaked feedstock and further processed to recover oil and germ meal. After removal of the germ, the remaining portion of feedstock may be processed to remove bran and to produce a starch and gluten slurry. The slurry may be further processed to separate the starch and gluten protein which may be dried to form gluten meal. The starch stream may be further processed via fermentation to produce a fermentation product (e.g., product alcohol) or may be utilized by the food, paper, or textile industries. For example, the starch stream may be used to produce sweeteners. The gluten meal and gluten feed stream which both contain protein, fat, and fiber, may be used in feeds for dairy and beef cattle, poultry, swine, livestock, equine, aquaculture, and domestic pets. Gluten feed may also be used as a carrier for added micronutrients. Gluten meal also contains methionine and xanthophylls which may be used a pigment ingredient in, for example, poultry feeds (e.g., xanthophylls provide yellow pigmentation for egg yolks). Condensed fermented extractives which contain protein, growth factors, B vitamins, and minerals may be used as a high energy liquid feed ingredient. Condensed extractives may also be used as a pellet binder. This process provides a purified starch stream; however, it is costly and includes the separation of the biomass into its non-starch components which may not be necessary for fermentation production.

Fractionation removes fiber and germ, which contains a majority of the lipids (e.g., oil) present in ground whole corn resulting in a fractionated corn that has a higher starch (endosperm) content. In some aspects, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or more of the oil may be removed from the germ by fractionation. In some aspects, fractionation may reduce the undissolved solids content of the feedstock to at least 0.5%, at least 1%, at least 2%, at least 3%, at least 4%, or at least 5% of the feedstock.

Dry fractionation does not separate the germ from fiber and therefore, it is less expensive than wet milling. The benefits of fractionation may include, for example, improved yield of product, increased volume (i.e., space) in the fermentor, smaller column diameters, lower enzyme loadings and increased efficiency for saccharification, improved oil removal, decreased equipment clogging due to the removal of oil, fewer cleaning shutdowns, increased protein levels in DDGS, reduced drying time for DDGS, and reduced energy consumption. However, fractionation does not remove the entirety of the fiber or germ, and does not result in total elimination of solids. Furthermore, there is some loss of starch in fractionation.

Dry milling may also be utilized for feedstock processing. Feedstock may be milled, for example, using a hammermill to generate a meal that may then be mixed with water to form a slurry. The slurry may be subjected to liquefaction by the addition of enzymes such as amylases to hydrolyze starch to sugars, forming a mash. The mash may be heated ("cooked") to inactivate the enzyme and then cooled for addition to fermentation. Cooled mash, microorganism, and enzyme such as glucoamylase may be added to fermentation for the production of fermentation product (e.g., product alcohol). Following fermentation, the fermentation broth may be conducted to distillation for recovery of the fermentation product. If the undissolved solids have not been removed, the bottoms stream of the distillation column is whole stillage containing unfermented solids (e.g., distillers grain solids), dissolved materials, and water which may be collected for further processing. For example, the whole stillage may be separated into solids (e.g., wet cake) and thin stillage. Separation may be accomplished by a number of means including, but not limited to, centrifugation, filtration, screen separation, hydrocyclone, or any other means or separation device for separating liquids from solids. Thin stillage may be conducted to evaporation forming condensed distillers solubles (CDS) or syrup. Thin stillage may comprise soluble nutrients, small grain solids (or fine particles), and microorganisms. The solids (e.g., wet cake) may be combined with syrup and then dried to form DDGS. Syrup contains protein, fat, and fiber as well as vitamins and minerals such as phosphorus and potassium; and may be added to animal feeds for its nutritional value and palatability. DDGS contains protein, fat, and fiber; and provides a source of bypass proteins. DDGS may be used in animal feeds for dairy and beef cattle, poultry, swine, livestock, equine, aquaculture, and domestic pets.

In some aspects, as shown, for example, in Figure 3, the processes and systems of the present invention may include discharging oil 26 from an outlet of separation 20. Figure 3 is similar to Figure 1, except for oil stream 26 exiting separation 20, and therefore will not be described in detail again.

Feedstock slurry 16, conducted to separation 20, may be separated into a first liquid phase or aqueous solution 22 containing a fermentable sugar, a second liquid phase containing oil 26, and a solid phase or wet cake 24 containing undissolved solids. Any suitable separation device can be used to discharge aqueous solution 22 (or aqueous stream), wet cake 24 (or solid stream), and oil 26 (or oil stream), for example, a three-phase centrifuge. In some aspects, feedstock 12 is corn and oil 26 is corn oil (e.g., free corn oil). The term free corn oil as used herein means corn oil that is freed from the corn germ. In some aspects, oil 26 may be conducted to a storage tank or any vessel that is suitable for oil storage. In some aspects, a portion of oil from feedstock 12 such as corn oil when the feedstock is corn, remains in wet cake 24.

In some aspects, when oil 26 is removed via separation 20, the fermentation broth in fermentation 30 includes a reduced amount of oil. In some aspects after oil removal, the feedstock slurry may comprise 1 wt%, 2 wt%, 3 wt%, or 4 wt% oil.

In some aspects, the process and system of Figure 2 may be modified to include discharge of an oil stream from separation 20 as described herein in connection to the process and system of Figure 3.

As illustrated in Figure 4A, if oil is not discharged separately, it may be removed with wet cake 24. When wet cake 24 is separated via separation 20, in some aspects, a portion of the oil from feedstock 12, such as corn oil when the feedstock is corn, remains in wet cake 24. Wet cake 24 may be conducted to mix 60 and combined with water or other solvents (i.e., re-slurried) forming wet cake mixture 65. In some aspects, the water may be fresh water, backset, cook water, process water, lutter water, evaporation water, or any water source available in the fermentation processing facility, or any combination thereof. Wet cake mixture 65 may be conducted to separation 70 producing wash centrate 75 comprising fermentable sugars recovered from wet cake 24, and wet cake 74. Wash centrate 75 may be recycled to liquefaction 10. The remainder of Figure 4A is similar to Figure 1, and therefore will not be described in detail again.

In some aspects, separation 70 may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation may be a single step process.

In some aspects, wet cake may be subjected to one or more wash cycles or wash systems. For example, wet cake 74 may be further processed by conducting wet cake 74 to a second wash system. In some aspects, wet cake 74 may be conducted to a second mix 60' forming wet cake mixture 65'. Wet cake mixture 65' may be conducted to a second separation 70' producing wash centrate 75' and wet cake 74'. Wash centrate 75' may be recycled to liquefaction 10 and/or wash centrate 75' may be combined with wash centrate 75 and the combined wash centrates may be recycled to liquefaction 10. Wet cake 74' may be combined with wet cake 74 for further processing as described herein. In some aspects, separation 70' may be any separation device capable of separating solids and liquids including, for example, decanter bowl centrifugation, three-phase centrifugation, disk stack centrifugation, filtering centrifugation, decanter centrifugation, filtration, micro filtration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation may be a single step process. In some aspects, the wet cake may be subjected to one, two, three, four, five, or more wash cycles or wash systems.

Wet cake 74 may be combined with solubles and then dried to form DDGS through any suitable known process. The formation of the DDGS from wet cake 74 has several benefits. For example, since the undissolved solids are not added to the fermentor, the undissolved solids are not subjected to the conditions of the fermentor and therefore, the undissolved solids do not contact the microorganisms present in the fermentor, and fermentation product such as product alcohol or other components such as extractant are not trapped in the undissolved solids. These effects provide benefits to subsequent processing and use of DDGS, for example, as animal feed because the DDGS would not contain microorganism or other components (e.g., product alcohol, extractant) of the fermentation broth.

As shown in Figure 4A, oil is not discharged separately from the wet cake, but rather oil is included as part of the wet cake and is ultimately present in the DDGS. If corn is utilized as feedstock, corn oil contains triglycerides, diglycerides, monoglycerides, fatty acids, and phospholipids, which provide a source of metabolizable energy for animals. The presence of oil in the wet cake and ultimately DDGS may provide a desirable animal feed, for example, a high fat content animal feed.

In some aspects, oil may be separated from the DDGS and converted to an ISPR extractant for subsequent use in the same or different fermentation processes. Methods for deriving extractants from biomass are described in U.S. Patent Application Publication No. 2011/0312043, U.S. Patent Application Publication No. 2011/0312044, U.S. Patent Application Publication No. 2012/0156738, and PCT International Publication No. WO 2011/159998. Oil may be separated from DDGS using any suitable known process including, for example, a solvent extraction process. In one aspect of the invention, DDGS may be added to an extraction vessel and washed with a solvent such as hexane to remove oil. Other solvents that may be utilized include, for example, isobutanol, isohexane, ethanol, butanol, isododecane, hexadecane, isohexadecane, heptane, triisobutylene, diisobutylene, tetraisobutylene, isooctane, petroleum distillates such as petroleum ether, or mixtures thereof. After oil extraction, DDGS may be treated to remove any residual solvent. For example, DDGS may be heated to vaporize any residual solvent using any method known in the art. Following solvent removal, DDGS may be subjected to a drying process to remove any residual water. The processed DDGS may be used as a feed supplement for animals such as dairy and beef cattle, poultry, swine, livestock, equine, aquaculture, and domestic pets.

After extraction from DDGS, the resulting oil and solvent mixture may be collected for separation of oil from the solvent. In one aspect, the oil/solvent mixture may be processed by evaporation whereby the solvent is evaporated and may be collected and recycled. The recovered oil may be converted to an ISPR extractant for subsequent use in the same or different fermentation processes.

In some aspects, oil may also be separated from the feedstock or feedstock slurry using a solvent extraction process. For example, the feedstock or feedstock slurry may be added to an extraction vessel and washed with a solvent to remove oil. Solvents that may be utilized include, for example, isobutanol, isohexane, ethanol, butanol, isododecane, hexadecane, isohexadecane, heptane, triisobutylene, diisobutylene, tetraisobutylene, isooctane, petroleum distillates such as petroleum ether, or mixtures thereof. After oil extraction, the feedstock or feedstock slurry may be separated generating a wet cake and an aqueous solution. By removing oil from the feedstock or feedstock slurry, separation of solids and aqueous solution may be improved. For example, the amount of solids in the aqueous solution may be decreased.

In an aspect of the process shown in Figure 4A, wash centrate 75 or a portion thereof (shown as dotted lines) may be recycled or combined with other streams as illustrated in Figure 4B. For example, wash centrate 75 may be combined with other streams as a means to reduce the amount of total suspended solids (TSS) in mash. As shown in Figure 4B, wash centrate 75 or a portion thereof may be combined with feedstock 12 in slurry mixer 15 producing feedstock stream 17 comprising a fermentable carbon source and undissolved solids including TSS. Feedstock stream 17 may be conducted to liquefaction 10 configured to liquefy a feedstock stream, producing feedstock slurry 16 which may be further processed as described herein. In another aspect, wash centrate 75 or a portion thereof may be combined with feedstock slurry 16. By diluting feedstock slurry 16 with wash centrate 75, the amount of TSS in feedstock slurry 16 may be further reduced. In some aspects, the amount of TSS in feedstock slurry 16 may be reduced by 1 wt%, 2 wt%, 3 wt%, 4 wt%, 5 wt%, 6 wt%, 7 wt%, 8 wt%, 9 wt%, 10 wt%, or more as compared to the amount of TSS (wt%) in mash (e.g., feedstock stream 17).

As described herein, wet cake may be subjected to one or more wash cycles or wash systems. For example, wet cake 74 may be further processed by conducting wet cake 74 to a second wash system. In some aspects, wet cake 74 may be conducted to a second mix 60' forming wet cake mixture 65'. Wet cake mixture 65' may be conducted to a second separation 70' producing wash centrate 75' and wet cake 74'. Wash centrate 75' or a portion thereof may be recycled to slurry mixer 15 and/or wash centrate 75' or a portion thereof may be combined with wash centrate 75 and the combined wash centrates may be recycled to slurry mixer 15. In some aspects, wash centrate 75' or a portion thereof may be combined with feedstock slurry 16 and/or wash centrate 75' or a portion thereof may be combined with wash centrate 75 and the combined wash centrates may in turn, be combined with feedstock slurry 16.

Removal of the oil component of the feedstock is advantageous to production because oil present in the fermentor may be hydrolyzed to fatty acids and glycerin. Glycerin can accumulate in water and reduce the amount of water that is available for recycling throughout the fermentation system. Thus, removal of the oil component of feedstock increases the efficiency of production by increasing the amount of water that can be recycled through the system. In addition, removing oil can result in energy savings for the production plant due to more efficient fermentation, less fouling due to the removal of the oil, increased fermentor volume efficiency, and decreased energy requirements, for example, the energy needed to dry distillers grains.

As illustrated in Figure 5A, oil may be removed at various points during the processes described herein. Feedstock slurry 16 may be separated, for example, using a three-phase centrifuge, into a first liquid phase or aqueous solution 22 (or aqueous stream), a second liquid phase comprising oil 26 (or oil stream), and a solid phase or wet cake 24 (or solid stream). Wet cake 24 may be further processed to recover fermentable sugars and oil. Wet cake 24 may be conducted to mix 60 and combined with water or other solvents forming wet cake mixture 65. In some aspects, water may be backset, cook water, process water, lutter water, water collected from evaporation, or any water source available in the fermentation processing facility, or any combination thereof. Wet cake mixture 65 may be conducted to separation 70 (e.g., three-phase centrifuge) producing wash centrate 75 comprising fermentable sugars, oil 76, and wet cake 74. In some aspects, separation may be a single step process. Wash centrate 75 may be recycled to liquefaction 10. Oil 76 and oil 26 may be combined and further processed for the manufacture of various consumer products. In some aspects, the oil (e.g., oil 26, oil 76) may be further processed to generate extractant. For example, the oil may be treated chemically or enzymatically to generate extractant. In some aspects where the oil is corn oil, the corn oil may be treated chemically or enzymatically to generated fatty acids (e.g., corn oil fatty acids) that may be used as extractant. In some aspects where the oil is treated enzymatically, the enzymatic reaction may be subjected to a treatment (e.g., heat) post conversion to deactivate the enzyme. In some aspects, the oil may be enzymatically treated utilizing enzymes such as esterases, lipases, phospholipases, lysophospholipases, or combinations thereof. In some aspects, the oil may be chemically treated with ammonium hydroxide, anhydrous ammonia, ammonium acetate, hydrogen peroxide, toluene, glacial acetic acid, or combinations thereof. Methods for deriving extractants from biomass are described in U.S. Patent Application Publication No. 2011/0312043 and U.S. Patent Application Publication No. 2011/0312044. In some aspects where the oil is corn oil, the feedstock slurry may comprise at least 1 wt%, at least 2 wt%, at least 3 wt%, at least 4 wt% corn oil, or at least 5 wt% corn oil. The remainder of Figure 5A is similar to Figure 1, and therefore will not be described in detail again.

As described herein, wet cake may be subjected to one or more wash cycles or wash systems. In some aspects, wet cake 74 may be conducted to a second mix 60' forming wet cake mixture 65'. Wet cake mixture 65' may be conducted to a second separation 70' producing wash centrate 75', oil 76', and wet cake 74'. Wash centrate 75' may be recycled to liquefaction 10 and/or wash centrate 75' may be combined with wash centrate 75 and the combined wash centrates may be recycled to liquefaction 10. Wet cake 74' may be combined with wet cake 74 for further processing as described herein. Oil 76, oil 76', and oil 26 may be combined and further processed for the manufacture of various consumer products. In some aspects, oil (e.g., oil 26, oil 76, oil 76') may be further processed to generate extractant as described herein. Methods for deriving extractants from biomass are described in U.S. Patent Application Publication No. 2011/0312043 and U.S. Patent Application Publication No. 2011/0312044.

In an aspect of the process shown in Figure 5A, wash centrate 75 or a portion thereof (shown as dotted lines) may be recycled or combined with other streams as illustrated in Figure 5B. For example, wash centrate 75 may be combined with other streams as a means to reduce the amount of total suspended solids (TSS) in mash. As shown in Figure 5B, wash centrate 75 or a portion thereof may be combined with feedstock 12 in slurry mixer 15 producing feedstock stream 17 comprising a fermentable carbon source and undissolved solids including TSS. Feedstock stream 17 may be conducted to liquefaction 10 configured to liquefy a feedstock stream, producing feedstock slurry 16 which may be further processed as described herein. In another aspect, wash centrate 75 or a portion thereof may be combined with feedstock slurry 16. By diluting feedstock slurry 16 with wash centrate 75, the amount of TSS in feedstock slurry 16 may be further reduced. In some aspects, the amount of TSS in feedstock slurry 16 may be reduced by 1 wt%, 2 wt%, 3 wt%, 4 wt%, 5 wt%, 6 wt%, 7 wt%, 8 wt%, 9 wt%, 10 wt%, or more as compared to the amount of TSS (wt%) in mash (e.g., feedstock stream 17).

As described herein, wet cake may be subjected to one or more wash cycles or wash systems. For example, wet cake 74 may be further processed by conducting wet cake 74 to a second wash system. In some aspects, wet cake 74 may be conducted to a second mix 60' forming wet cake mixture 65'. Wet cake mixture 65' may be conducted to a second separation 70' producing wash centrate 75' and wet cake 74'. Wash centrate 75' or a portion thereof may be recycled to slurry mixer 15 and/or wash centrate 75' or a portion thereof may be combined with wash centrate 75 and the combined wash centrates may be recycled to slurry mixer 15. In some aspects, wash centrate 75' or a portion thereof may be combined with feedstock slurry 16 and/or wash centrate 75' or a portion thereof may be combined with wash centrate 75 and the combined wash centrates may in turn, be combined with feedstock slurry 16.

As illustrated in Figure 6, aqueous solution 22 and wet cake 24 may be combined, cooled, and conducted to fermentation 30. Feedstock slurry 16 may be separated, for example, using a three-phase centrifuge, into a first liquid phase or aqueous solution 22, a second liquid phase comprising oil 26, and a solid phase or wet cake 24. In some aspects, oil 26 (or oil stream) may be conducted to a storage tank or any vessel that is suitable for oil storage. Aqueous solution 22 (or aqueous stream) and wet cake 24 (solid stream) may be conducted to mix 80 and re-slurried forming aqueous solution/wet cake mixture 82. Mixture 82 may be conducted to cooler 90 producing cooled mixture 92 which may be conducted to fermentation 30. In some aspects, when oil 26 is removed via separation 20 from feedstock 12, mixtures 82 and 92 include a reduced amount of corn oil. The remainder of Figure 6 is similar to Figure 1, and therefore will not be described in detail again.

In some aspects, as shown, for example, in Figures 7 and 8, saccharification may occur in a separate saccharification system 50 which is located between separation 20 and fermentation 30 (Figure 7) or between liquefaction 10 and separation 20 (Figure 8). Figures 7 and 8 are similar to Figure 1 except for the inclusion of a separate saccharification 50 and fermentation 30 does not receive enzyme 38. In some aspects, enzyme 38 may also be added to fermentation 30.

Any known saccharification processes utilized by the industry may be used including, but not limited to, an acid process, an enzyme process, or an acid-enzyme process. Saccharification 50 may be conducted in any suitable saccharification vessel. In some aspects, enzyme 38 such as glucoamylase, may be introduced to hydrolyze sugars (e.g., oligosaccharides) in feedstock slurry 16 or aqueous solution 22 to form monosaccharides. For example, in Figure 7, oligosaccharides present in aqueous solution 22 discharged from separation 20 and conducted to saccharification 50 through an inlet are hydrolyzed to monosaccharides. Aqueous solution 52 containing monosaccharides is discharged from saccharification 50 through an outlet and conducted to fermentation 30. Alternatively, as shown in Figure 8, oligosaccharides present in feedstock slurry 16 discharged from liquefaction 10 and conducted to saccharification 50 through an inlet are hydrolyzed to monosaccharides. Feedstock slurry 54 containing monosaccharides is discharged from saccharification 50 through an outlet and conducted to separation 20. In some aspects of the processes illustrated in Figures 7 and 8, feedstock slurry 16 and feedstock slurry 54 may be separated via separation 20 to form a first liquid phase or aqueous solution 22, a second liquid phase comprising oil, and a solid phase or wet cake 24. Aqueous solution 22, wet cake 24, and the oil stream may be processed as described herein.

In some aspects, the system and processes of Figures 1-6 may be modified to include a separate saccharification system as described herein in connection to the systems and processes of Figures 7 and 8.

In some aspects, as shown, for example, in Figures 9A - 9F, the systems and processes of the present invention may include a series of two or more separation devices and/or wash systems. Figures 9A - 9F are similar to Figure 1, except for the addition of separation systems and/or wash systems, and therefore will not be described in detail again.

Referring to Figure 9A, aqueous solution 22 discharged from separation 20 may be conducted to separation 20'. Separation 20' may be identical or different to separation 20, and may operate in the same manner or different manner. Separation 20' may remove undissolved solids and oil not separated from aqueous solution 22 to generate (i) aqueous solution 22' similar to aqueous solution 22, but containing reduced amounts of undissolved solids and oil in comparison to aqueous solution 22, (ii) wet cake 24' similar to wet cake 24, and (iii) oil 26' similar to oil 26. Aqueous solution 22' may then be introduced to fermentation 30. In some aspects, there may be one or more additional separation devices following separation 20'. In some aspects, separation may be a single step process.

In some aspects, separation 20 and separation 20' may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof.

In some aspects, stream 35 may be discharged from an outlet in fermentation 30. The absence or minimization of the undissolved solids exiting fermentation 30 via stream 35 has several additional benefits. For example, the need for units and operations in downstream processing may be decreased or eliminated, for example, beer columns or distillation columns, thereby resulting in an increased efficiency for production. Also, some or all of the centrifuges used to process whole stillage may be eliminated as a result of less undissolved solids in the stream exiting the fermentor.

In some aspects, the systems and processes of the present invention may include a series of two, three, four, or more separation devices and/or wash systems.

Referring to Figure 9B, feedstock slurry 16 may be conducted to separation 20 and may be separated into a first liquid phase or aqueous solution 22 containing a fermentable sugar, a solid phase or wet cake 24 containing undissolved solids, and optionally a second liquid phase containing oil 26. Wet cake 24 may be conducted to mix 60 and combined with water or other solvents forming wet cake mixture 65. Aqueous solution 22 may be conducted to separation 20' and may be separated into aqueous solution 22' containing a fermentable sugar and wet cake 24' containing undissolved solids. In some aspects, a second liquid phase containing oil 26' may be formed via separation 20'. Aqueous solution 22' may be conducted to fermentation 30. Wet cake 24' may be conducted to mix 60' and combined with water or other solvents forming wet cake mixture 65'. In some aspects, the water combined with wet cake 24 and wet cake 24' may be fresh water, backset, cook water, process water, lutter water, evaporation water, or any water source available in the fermentation processing facility, or any combination thereof. Wet cake mixture 65 and wet cake mixture 65' may be combined, and may be conducted to separation 70 producing wash centrate 75 comprising fermentable sugars, and wet cake 74. Wash centrate 75 may be recycled to the fermentation process, for example, liquefaction 10 or combined with feedstock slurry 16. In aspects where oil 26 and/or 26' are separated, oil may be returned to the fermentation process or may be conducted to a storage tank or any vessel that is suitable for oil storage. In some aspects, oil may be separated via separation 20 and/or separation 20'.

Separation 20 and separation 20' may be identical or may be different, and may operate in the same manner or different manner. In some aspects, separation may be a single step process. In some aspects, separation 20 and separation 20' may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation 20 and separation 20' may be centrifugation. In some aspects, separation 20 and separation 20' may be three-phase centrifuges. In some aspects, separation 20 may be a three-phase centrifuge and separation 20' may be a disk stack centrifuge. In some aspects, separation 20 may be a three-phase centrifuge and separation 20' may be a decanter centrifuge. In some aspects, separation 20 may be centrifugation and separation 20' may be filtration. In some aspects, separation 20 may be a three-phase centrifuge and separation 20' may be crossflow filtration. In some aspects, separation 20 may be a three-phase centrifuge and separation 20' may be a drum filter. In some aspects, separation 20 may be a three-phase centrifuge and separation 20' may be a rotary screen. In some aspects, separation 20 may be filtration and separation 20' may be centrifugation. In some aspects, separation 20 may be a rotary screen and separation 20' may be a three-phase centrifuge.

In some aspects, there may be three or more separation devices and/or wash systems. For example, aqueous stream 22' may be conducted to separation 20" and may be separated into aqueous solution 22" containing a fermentable sugar and wet cake 24" containing undissolved solids. In some aspects, a second liquid phase containing oil 26" may be formed via separation 20". Aqueous solution 22" may be conducted to fermentation 30. Wet cake 24" may be conducted to mix 60" and combined with water or other solvents forming wet cake mixture 65". In some aspects, the water combined with wet cake 24" may be fresh water, backset, cook water, process water, lutter water, evaporation water, or any water source available in the fermentation processing facility, or any combination thereof. Wet cake mixture 65" may be combined with wet cake mixture 65 and wet cake mixture 65', and the combined wet cake mixture may be conducted to separation 70 producing wash centrate 75 comprising fermentable sugars, and wet cake 74. Wash centrate 75 may be recycled to the fermentation process, for example, liquefaction 10 or combined with feedstock slurry 16. In some aspects, oil may be separated via separation 20, separation 20', and/or separation 20". In aspects where oil 26, oil 26' and/or 26" are separated, oil may be returned to the fermentation process or may be conducted to a storage tank or any vessel that is suitable for oil storage.

Separation 20" may be identical or may be different to separation 20 and separation 20', and may operate in the same manner or different manner. In some aspects, separation may be a single step process. In some aspects, separation 20" may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation 20, separation 20', and separation 20" may be centrifugation. In some aspects, separation 20, separation 20', and separation 20" may be three-phase centrifuges. In some aspects, separation 20 may be a three-phase centrifuge, and separation 20' and separation 20" may be disk stack centrifuges. In some aspects, separation 20 may be a three-phase centrifuge, and separation 20' and separation 20" may be decanter centrifuges. In some aspects, separation 20, separation 20', and separation 20" may be a combination of centrifugation and filtration. In some aspects, separation 20 may be a three-phase centrifuge, separation 20’ may be a rotary screen, and separation 20" may be a disk stack centrifuge. In some aspects, separation 20 may be a three-phase centrifuge, separation 20’ may be a disk stack centrifuge, and separation 20" may be a rotary screen. In some aspects, separation 20 may be a rotary screen, separation 20’ may be a three-phase centrifuge, and separation 20" may be a disk stack centrifuge. In some aspects, separation 20, separation 20’, and separation 20" may be a three-phase centrifuge, crossflow filtration, and a decanter centrifuge. In some aspects, separation 20, separation 20’, and separation 20" may be a three-phase centrifuge, a rotary screen, and a disk stack centrifuge. In some aspects, separation 20, separation 20’, and separation 20" may be a three-phase centrifuge, crossflow filtration, and a decanter centrifuge.

Separation 70 may be any separation device capable of separating solids and liquids including, for example, decanter bowl centrifugation, three-phase centrifugation, disk stack centrifugation, filtering centrifugation, decanter centrifugation, filtration, micro filtration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof.

In some aspects, mix 60, mix 60’, and mix 60" may comprise one or more agitator devices. In some aspects, the agitator devices may be blade turbines. In some aspects, the blade turbines may be pitch blade turbines. In some aspects, the blade turbines may be flat blade turbines. In some aspects, mix 60, mix 60’, and mix 60" may comprise one or more pitch blade turbines and/or one or more flat blade turbines. In some aspects, mix 60, mix 60’, and mix 60" may comprise clean-in-place capability.

Figure 9C illustrates another aspect of the processes and systems described herein. Feedstock slurry 16 may be conducted to separation 20 and may be separated into a first liquid phase or aqueous solution 22 containing a fermentable sugar, a solid phase or wet cake 24 containing undissolved solids, and optionally a second liquid phase containing oil 26. Aqueous solution 22 may be conducted to separation 20’ and may be separated into aqueous solution 22’ containing a fermentable sugar and wet cake 24’ containing undissolved solids. In some aspects, a second liquid phase containing oil 26’ may be formed via separation 20’. Aqueous solution 22’ may then be conducted to separation 20" and may be separated into aqueous solution 22" containing a fermentable sugar and wet cake 24" containing undissolved solids. In some aspects, a second liquid phase containing oil 26" may be formed via separation 20". Aqueous solution 22" may be conducted to fermentation 30.

Wet cake 24, wet cake 24’, and wet cake 24" may be combined, and the combined wet cakes may be conducted to mix 60 and combined with water or other solvents forming wet cake mixture 65. In some aspects, the water may be fresh water, backset, cook water, process water, lutter water, evaporation water, or any water source available in the fermentation processing facility, or any combination thereof. Wet cake mixture 65 may be conducted to separation 70 producing wash centrate 75 comprising fermentable sugars, and wet cake 74. Wash centrate 75 may be recycled to the fermentation process, for example, liquefaction 10 or combined with feedstock slurry 16. In some aspects, oil may be separated via separation 20, separation 20’, and/or separation 20". In aspects where oil 26, oil 26’ and/or oil 26" are separated, oil may be returned to the fermentation process or may be conducted to a storage tank or any vessel that is suitable for oil storage.

Separation 20, separation 20’, and separation 20" may be identical or may be different, and may operate in the same manner or different manner. In some aspects, separation may be a single step process. In some aspects, separation 20, separation 20’, and separation 20" may be any separation device capable of separating solids and liquids including, for example, decanter bowl centrifugation, three-phase centrifugation, disk stack centrifugation, filtering centrifugation, decanter centrifugation, filtration, micro filtration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation 20, separation 20’, and separation 20" may be centrifugation. In some aspects, separation 20, separation 20’, and separation 20" may be three-phase centrifuges. In some aspects, separation 20 may be a three-phase centrifuge, and separation 20’ and separation 20" may be disk stack centrifuges. In some aspects, separation 20 may be a three-phase centrifuge, and separation 20’ and separation 20" may be decanter centrifuges. In some aspects, separation 20, separation 20’, and separation 20" may be a combination of centrifugation and filtration. In some aspects, separation 20 may be a three-phase centrifuge, separation 20’ may be a rotary screen, and separation 20" may be a disk stack centrifuge. In some aspects, separation 20 may be a three-phase centrifuge, separation 20’ may be a disk stack centrifuge, and separation 20" may be a rotary screen. In some aspects, separation 20 may be a rotary screen, separation 20’ may be a three-phase centrifuge, and separation 20" may be a disk stack centrifuge. In some aspects, separation 20, separation 20’, and separation 20" may be a three-phase centrifuge, crossflow filtration, and a decanter centrifuge. In some aspects, separation 20, separation 20’, and separation 20" may be a three-phase centrifuge, a rotary screen, and a disk stack centrifuge. In some aspects, separation 20, separation 20’, and separation 20" may be a three-phase centrifuge, crossflow filtration, and a decanter centrifuge.

In some aspects, mix 60 may comprise one or more agitator devices. In some aspects, the agitator devices may be blade turbines. In some aspects, the blade turbines may be pitch blade turbines. In some aspects, the blade turbines may be flat blade turbines. In some aspects, mix 60 may comprise one or more pitch blade turbines and/or one or more flat blade turbines. In some aspects, mix 60 may comprise clean-in-place capability.

Separation 70 may be any separation device capable of separating solids and liquids including, for example, decanter bowl centrifugation, three-phase centrifugation, disk stack centrifugation, filtering centrifugation, decanter centrifugation, filtration, micro filtration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation may be a single step process.

In another aspect of Figure 9C, there may be two separation devices and/or wash systems. Feedstock slurry 16 may be conducted to separation 20 and may be separated into a first liquid phase or aqueous solution 22 containing a fermentable sugar, a solid phase or wet cake 24 containing undissolved solids, and optionally a second liquid phase containing oil 26. Aqueous solution 22 may be conducted to separation 20' and may be separated into aqueous solution 22' containing a fermentable sugar and wet cake 24' containing undissolved solids. In some aspects, a second liquid phase containing oil 26' may be formed via separation 20'. Aqueous solution 22' may then be conducted to fermentation 30. Wet cake 24 and wet cake 24' may be combined, and the combined wet cakes may be conducted to mix 60 and combined with water or other solvents forming wet cake mixture 65. In some aspects, the water may be fresh water, backset, cook water, process water, lutter water, evaporation water, or any water source available in the fermentation processing facility, or any combination thereof. Wet cake mixture 65 may be conducted to separation 70 producing wash centrate 75 comprising fermentable sugars, and wet cake 74. Wash centrate 75 may be recycled to the fermentation process, for example, liquefaction 10 or combined with feedstock slurry 16. In some aspects, oil may be separated via separation 20 and separation 20'. In aspects where oil 26 and oil 26' are separated, oil may be returned to the fermentation process or may be conducted to a storage tank or any vessel that is suitable for oil storage. Separation 20 and separation 20' may be identical or may be different, and may operate in the same manner or different manner. Separation 20 and separation 20' may be any separation device capable of separating solids and liquids as described herein.

In another aspect, referring to Figure 9D, aqueous solution 22 discharged from separation 20 may be conducted to separation 20'. Separation 20' may be identical or different to separation 20. Separation 20' may operate in a manner which could include separation additive 28. Separation additive 28 may aid in the removal of oil or solids. In some aspects, separation additive 28 may be an extractant or flocculant. Separation 20' may remove undissolved solids and oil not separated from aqueous solution 22 to generate (i) aqueous solution 22' similar to aqueous solution 22, but containing reduced amounts of undissolved solids and oil in comparison to aqueous solution 22, and (ii) stream 23. In some aspects, stream 23 may be similar to a combined stream of oil 26 and wet cake 24, and may contain separation additive 28. Stream 23 may be conducted to separation 20" and may generate a stream that contains separation additive 28' and wet cake 24'. Separation 20" may be identical or different to separation 20 and separation 20'. Aqueous solution 22' may be introduced to fermentation 30.

In some aspects, separation 20, separation 20', and separation 20" may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation may be a single step process.

Referring to Figure 9E, feedstock slurry 16 may be discharged from liquefaction 10 and conducted to separation 20. Feedstock slurry 16 is separated to generate streams: (i) aqueous solution 22, (ii) wet cake 24, and (iii) stream 25 comprising oil, solids, and an aqueous stream comprising a fermentable carbon source. In some embodiments, the solids of stream 25 may be light solids. In some embodiments, light solids may be solids that are less dense than water but more dense than oil. In some embodiments, light solids may be coated in oil, resulting in solids that are less dense than water. In some embodiments, solids may have lipophilic and/or hydrophilic properties. In some embodiments, the solids of stream 25 may comprise one or more of the following: germ, fiber, starch, and gluten. In some embodiments, the solids of stream 25 may comprise fine particles. In some embodiments, the solids of stream 25 may comprise germ, gluten, and fiber. Aqueous solution 22 comprising a fermentable carbon source is conducted to fermentation 30 for production of a fermentation product as described herein. In some embodiments, wet cake 24 may be further processed as described herein, for example, processed to form DDGS.

Stream 25 discharged from separation 20 is conducted to separation 20'. Separation 20' may be identical or different to separation 20. In some embodiments, separation 20 and separation 20' may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some embodiments, separation may be a single step process.

Stream 25 is separated by separation 20' to generate streams: (i) aqueous solution 22', (ii) wet cake 24', and (iii) oil 26. In some embodiments, aqueous solution 22' may be combined with aqueous solution 22, and the combined aqueous solution may be conducted to fermentation 30. In some embodiments, the amount of solids in aqueous solution 22' is reduced compared to the amount of solids in aqueous solution 22. In some embodiments, aqueous solution 22' may comprise oil and the oil in aqueous solution 22' may be further processed to generate an extractant. For example, aqueous solution 22' may be treated chemically or enzymatically to generate an extractant. In some embodiments, aqueous solution 22' may be treated chemically or enzymatically to generate fatty acids (e.g., corn oil fatty acids) that may be used as an extractant. In some embodiments where aqueous solution 22' is treated enzymatically, the enzymatic reaction may be subjected to a treatment (e.g., heat) post conversion to deactivate the enzyme. Converting the oil in aqueous solution 22' which has a relatively small stream volume to fatty acids may reduce the capital cost of generating extractant via enzymatic or chemical conversion. Methods for deriving extractants from biomass are described in U.S. Patent Application Publication No. 2011/0312043 and U.S. Patent Application Publication No. 2011/0312044.

In some embodiments, wet cake 24' may be combined with wet cake 24, and the combined wet cake may be further processed as described herein. In some embodiments, wet cake 24' may comprise oil and this oil-rich wet cake may be combined with wet cake 24 to produce a wet cake with increased fat content (e.g., increased triglyceride content) which would provide a metabolizable energy source for animal feed. In some embodiments, this wet cake with increased fat content may be combined with syrup to generate a high triglyceride, high protein, low carbohydrate DDGS. In some embodiments, wet cake 24' comprising oil may be further processed as described herein, for example, to produce DDGS.

In some embodiments, oil 26 may be conducted to a storage tank or any vessel that is suitable for oil storage. In some embodiments, oil 26 or a portion thereof may be combined with feedstock slurry 16 (dotted line, Figure 9E). The addition of oil to the feedstock slurry may improve solids removal via stream 25 by increasing the amount of solids captured.

In some embodiments, oil 26 may be further processed to generate extractant as described herein. Converting oil 26, which has a relatively small stream volume and would have a reduced flow rate compared to feedstock slurry 16, may reduce the capital cost as well as energy requirements of generating extractant via enzymatic or chemical conversion. In some embodiments, the flow rate of oil 26 may be 1% to 10% of the flow rate of feedstock slurry 16. Methods for deriving extractants from biomass are described in U.S. Patent Application Publication No. 2011/0312043 and U.S. Patent Application Publication No. 2011/0312044.

In some embodiments, stream 25 may be generated by adjusting one or more parameters of the separation device. For example, stream 25 may be generated by adjusting the weir (or dip weir) of a centrifuge such as a decanter centrifuge or three-phase centrifuge.

As described herein, solids may interfere with liquid-liquid extraction and therefore, utilizing an extraction method may not be technically or economically viable. During the extraction process, a rag layer may form at the interface of the aqueous and organic phases, and the rag layer, composed of solids (e.g., light solids), can accumulate and possibly interfere with phase separation. To mitigate the formation of the rag layer, removal of solids via stream 25 prior to fermentation may reduce or eliminate the formation of the rag layer and thereby improve downstream processing of the fermentation broth and recovery of fermentation products.

In another aspect to mitigate the formation of the rag layer, oil may be added to the aqueous solution as a means to selectively capture solids that form the rag layer. Referring to Figure 9F, feedstock slurry 16 may be discharged from liquefaction 10 and conducted to separation 20. Feedstock slurry 16 may be separated to generate streams: (i) aqueous solution 22, (ii) wet cake 24, and (iii) oil 26. In some aspects, wet cake 24 may be further processed as described herein, for example, processed to form DDGS. In some aspects, oil may be added to aqueous solution 22 and the resulting mixture may be conducted to vessel 80 where the mixture settles or separates forming (i) oil layer comprising solids 86 and (ii) aqueous solution 22'. In some aspects, aqueous solution 22' may be conducted to fermentation 30 for production of a fermentation product as described herein. In some aspects, the amount of solids in aqueous solution 22' is reduced compared to the amount of solids in aqueous solution 22. In some aspects, solids-rich oil layer 86 may be conducted to separation 20' and may be separated to generate streams: (i) aqueous solution 22", (ii) wet cake 24', and (iii) oil 26' (a solids-lean oil). In some aspects, solids-rich oil layer 86 may be removed (e.g., skimmed from the mixture) and filtered to remove the solids from the oil layer. In some aspects, the oil added to aqueous solution 22 may be the oil 26 separated from feedstock slurry 16, oil 26', an external oil source, or combinations thereof.

In some aspects, wet cake 24' may be combined with wet cake 24, and the combined wet cake may be further processed as described herein. In some aspects, wet cake 24' may comprise oil and this oil-rich wet cake may be combined with wet cake 24 to produce a wet cake with increased fat content and may be further processed as described herein. In some aspects, wet cake 24' comprising oil may be further processed as described herein.

In some aspects, aqueous solution 22" may be combined with aqueous solution 22', and the combined aqueous solution may be conducted to fermentation 30. The amount of solids in this combined aqueous solution would be reduced compared to aqueous solution 22, and with reduced solids, the formation of the rag layer would be mitigated. In some aspects, aqueous solution 22' and aqueous solution 22" may comprise oil and the oil may be further processed to generate an extractant. For example, aqueous solution 22' and aqueous solution 22" may be combined and may be treated chemically or enzymatically (dotted line in Figure 9F) to generate an extractant as described herein. Methods for deriving extractants from biomass are described in U.S. Patent Application Publication No. 2011/0312043 and U.S. Patent Application Publication No. 2011/0312044.

In some aspects, separation 20 and separation 20' may be any separation device capable of separating solids and liquids including, for example, decanter bowl centrifugation, three-phase centrifugation, disk stack centrifugation, filtering centrifugation, decanter centrifugation, filtration, microfiltration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. In some aspects, separation may be a single step process. In some aspects, vessel 80 may be a static mixer, mixer-settler, decanter, gravity settler, or combinations thereof. In some aspects, the process may be maintained at temperatures to minimize contamination (e.g., 70-110°C).

In some aspects, the systems and processes described herein may further comprise a liquefaction conditioning tank. In some aspects, following liquefaction, the feedstock slurry (or liquefied mash) or a portion thereof may be conducted to a liquefaction conditioning tank for further conditioning. For example, the pH of the feedstock slurry may be adjusted. In some aspects, the pH of the feedstock slurry may be adjusted by the addition of an acid such as sulfuric acid. In some aspects, enzymes may be added to the feedstock slurry. In some aspects, the enzymes may be amylases and/or glucoamylases. Examples of amylases that may be used in the processes and systems of the present invention are described in U.S. Patent No. 7,541,026; U.S. Patent Application Publication No. 2009/0209026; U.S. Patent Application Publication No. 2009/0238923; U.S. Patent Application Publication No. 2009/0252828; U.S. Patent Application Publication No. 2009/0314286; U.S. Patent Application Publication No. 2010/02278970; U.S. Patent Application Publication No. 2010/0048446; and U.S. Patent Application Publication No. 2010/0021587. Examples of glucoamylases that may be used in the processes and systems of the present invention are described in U.S. Patent No. 7,413,887; U.S. Patent No. 7,723,079; U.S. Patent Application Publication No. 2009/0275080; U.S. Patent Application Publication No. 2010/0267114; U.S. Patent Application Publication No. 2011/0014681; U.S. Patent Application Publication No. 2011/0020899. In some aspects, the feedstock slurry may be heated or cooled.

In some aspects, as shown, for example, in Figure 10, the systems and processes of the present invention may include means for on-line, in-line, at-line, and/or real-time measurements (circles represent measurement devices and dotted lines represent feedback loops). Figure 10 is similar to Figure 5A, except for the addition of measurement devices for on-line, in-line, at-line, and/or real-time measurements, and therefore will not be described in detail again.

The processes described herein may be integrated fermentation processes using on-line, in-line, at-line, and/or real-time measurements, for example, of concentrations and other physical properties of the various streams generated during fermentation (e.g., feedstock slurry, aqueous solution, oil stream, wet cake, wet cake mixtures, wash centrate, etc.). These measurements may be used, for example, in feed-back loops to adjust and control the conditions of the fermentation and/or the conditions of the fermentors, liquefaction units, saccharification units, separation units, and mixing units. In some aspects, the concentration of fermentation products and/or other metabolites and substrates in the fermentation broth may be measured using any suitable measurement device for on-line, in-line, at-line, and/or real-time measurements. In some aspects, the measurement device may be one or more of the following: Fourier transform infrared spectroscope (FTIR), near-infrared spectroscope (NIR), Raman spectroscope, high pressure liquid chromatography (HPLC), viscometer, densitometer, tensiometer, droplet size analyzer, particle analyzers, pH meter, dissolved oxygen (DO) probe, and the like. In some aspects, off-gas venting from the fermentor may be analyzed, for example, by an in-line mass spectrometer. Measuring off-gas venting from the fermentor may be used as a means to identify species or reaction products present in the fermentation reaction. The concentration of fermentation products and other metabolites and substrates may also be measured using the techniques and devices described herein.

In some aspects, measured inputs may be sent to a controller and/or control system, and conditions within the fermentor (temperature, pH, nutrients, enzyme and/or substrate concentration), liquefaction units, saccharification units, separation units, and mixing units may be varied to maintain a concentration or concentration profile of the various streams. By utilizing such a control system, process parameters may be maintained in such a way to improve overall plant productivity and economic goals. In some aspects, real-time control of fermentation may be achieved by variation of concentrations of components (e.g., biomass, sugars, enzymes, nutrients, microorganisms, and the like) in the fermentors, liquefaction units, saccharification units, separation units, and mixing units. In some aspects, automated systems may be used to adjust separation and mixing conditions, flow rates to and from the separation and mixing operations, solids and oil removal, and sugar and starch recovery.

During the fermentation processes, it is possible for units of operation to perform at sub-optimal levels over time. It may be necessary to adjust flow rates, mixing rates, equipment settings, and the like for operations such as liquefaction, saccharification, and separation in order to maintain overall plant productivity. The processes and systems described herein may be integrated using on-line, in-line, at-line, and/or real-time measurements for monitoring the concentrations and other physical properties of fermentation streams such as feedstock slurry 16, aqueous solution 22, oil 26, and wet cake 24. These measurements may be used, for example, in feed-back loops to adjust and control the conditions of fermentation, separation of feedstock slurry, and wash cycle performance. By utilizing on-line, in-line, at-line, and/or real-time measurements, immediate feedback and adjustments of process conditions may be made, resulting in an overall improved fermentation process. For example, the amount of fermentable carbon source (e.g., starch, sugars), oil, and solids may be monitored in feedstock slurry 16, aqueous solution 22, and streams 65 and 75 using, for example, FTIR or NIR. By monitoring these parameters, enzyme concentrations and residence time for liquefaction and saccharification may be adjusted to improve preparation of feedstock slurry 16 and separation and mixing conditions may be adjusted to, for example, increase the amount of fermentable carbon source, oil, and/or solids in aqueous solution 22 and streams 65 and 75.

As another example, washing wet cake 24 allows for recovery of sugars, starch, and oil in the wet cake, minimizing the yield loss of these fermentable carbon sources and oil. By monitoring moisture, sugar, starch, and oil content of streams 24, 65, 74, and 75, the washing performance may be adjusted to improve sugar, starch, and oil recovery. For example, real-time measurement of sugar, starch, and oil content of these streams may be performed by FTIR and NIR, and these measurements allow for immediate feedback and adjustment of mixing (60) and separation (20, 70) conditions. Differential speed, feed rate, bowl speed, scroll differential speed, impeller position, weir position, scroll pitch, residence time, and discharge volume of a separation device may be adjusted to modify the moisture, sugar, starch, and oil content of streams 24, 65, 74, and 75. Mixing (60) conditions such as pump rate or agitator speed may also be adjusted to modify the moisture, sugar, starch, and oil content of streams 24, 65, 74, and 75. In addition, the wash ratio (e.g., ratio between water and wet cake) may also be adjusted to modify the moisture, sugar, starch, and oil content of streams 24, 65, and 74. FTIR measurements and droplet imaging may be used to monitor the water content in oil streams (26, 76). In addition, color and turbidity of oil streams (26, 76) may be monitored to assess the quality of the oil. This real-time measurement would allow for adjustment to separation (20, 70) conditions resulting in a cleaner oil stream (e.g., less water).

In another aspect of the processes and systems described herein, moisture content of wet cake 24 may be monitored using real-time measurements. Real-time measurement of moisture content of the wet cake may be performed by NIR, and these measurements allow for immediate feedback and adjustment of separation (20, 70) conditions. By decreasing the water content of the wet cake, less energy is needed to dry the wet cake and therefore, overall energy usage may be improved for the production process. In addition, a lower water content of the wet cake may result in improved starch recovery.

As another example of process control strategy using real-time measurements, solids in aqueous solution 22 and oil 26, 76 may be measured using particle size analysis such as a process particle analyzer (JM Canty, Inc., Buffalo, NY), focused beam reflectance measurement (FBRM®), or particle vision and measurement (PVM®) technologies (Mettler-Toledo, LLC, Columbus OH). By monitoring solids in real time, process steps may be adjusted to improve solids removal and thereby, minimize the amount of solids in the aqueous solution and oil streams, maximize the recovery of solids, and improve the overall fermentation process including downstream processing.

The processes and systems disclosed in Figures 1-10 include removing undissolved solids and/or oil from feedstock slurry 16 and as a result, improving the processing productivity and cost effectiveness. The improved productivity can include increased efficiency of fermentation product production and/or increased extraction activity relative to processes and systems that do not remove undissolved solids and/or oil prior to fermentation.

An exemplary fermentation process of the present invention including downstream processing is described in Figure 11. Some processes and streams in Figure 11 have been identified using the same name and numbering as used in Figures 1-10 and represent the same or similar processes and streams as described in Figures 1-10.

Feedstock 12 may be processed and undissolved solids and/or oil separated (100) as described herein with reference to Figures 1-10. Briefly, feedstock 12 may be liquefied to generate feedstock slurry comprising undissolved solids, fermentable carbon sources, and oil. For example, milled grain and one or more enzymes may be combined to generate a feedstock slurry. This feedstock slurry may be heated (or cooked), liquefied, and/or flashed with flash vapor producing a "cooked" feedstock slurry or mash. In some embodiments, the feedstock slurry may be heated to at least 100°C. In some embodiments, the feedstock slurry may be heated for thirty minutes. In some embodiments, the feedstock slurry may be subjected to raw starch hydrolysis (also known as cold cooking or cold hydrolysis). In the raw starch hydrolysis process, the heating (or cooking) step is eliminated, and the elimination of this step reduces energy consumption and steam load (e.g., water consumption). In some embodiments, liquefaction and/or saccharification may be conducted at fermentation temperatures (e.g., 30°C to 55°C). In some embodiments, liquefaction and/or saccharification may be conducted utilizing raw starch enzymes or low temperature hydrolysis enzymes such as Stargen™ (Genencor International, Palo Alto, CA) and BPX™ (Novozymes, Franklinton, NC). In some embodiments, liquefaction and/or saccharification may be conducted at temperatures less than 50°C.

In one aspect, the feedstock slurry may then be subjected to separation generating wet cake 24, oil 26, and aqueous solution 22 comprising fermentable carbon source, for example, dissolved fermentable sugars. In some embodiments, separation may be a single step process. Separation may be accomplished by a number of means including, but not limited to, decanter bowl centrifugation, three-phase centrifugation, disk stack centrifugation, filtering centrifugation, decanter centrifugation, filtration, micro filtration, vacuum filtration, beltfilter, membrane filtration, crossflow filtration, drum filter, pressure filtration, filtration using a screen, screen separation, rotary screen, grating, porous grating, flotation, hydrocyclone, filter press, screwpress, gravity settler, vortex separator, or combination thereof. This separation step may remove at least 80%, at least 85%, at least 90%, at least 95%, at least 96%, at least 97%, at least 98%, or at least 99% of the undissolved solids from the feedstock slurry. In some embodiments, aqueous solution 22 may comprise at least 0.5%, at least 1%, or at least 2% undissolved solids.

Wet cake 24 may be re-slurried or washed with water and subjected to separation to remove additional fermentable sugars, generating washed wet cake (e.g., 74, 74' as described in Figures 1-10). In some embodiments, 1%, 2%, 3%, 4%, 5%, 10%, or 15% fermentable sugars may be recovered from the washed wet cake. The wash process may be repeated a number of times, for example, one, two, three, four, five, or more times. The water used to re-slurry or wash the wet cake may be recycled water generated during the fermentation process (e.g., backset, cook water, process water, lutter water, evaporation water). In some embodiments, the wet cake may be re-slurried or washed with beer. The wash centrates (e.g., 75, 75' as described in Figures 4A, 4B, 5A, 9B, and 9C) produced by the wash/separation process may be returned to the mix step to form a slurry with the milled grain or used in the liquefaction process. In some embodiments, the wash centrates may be heated or cooled prior to the mix step.

Aqueous solution 22 may be further processed as described herein. For example, aqueous solution 22 may be heated with steam or process-to-process heat exchange. A saccharification enzyme may be added to aqueous solution 22 and the dissolved fermentable sugars of aqueous solution 22 may be partially or completely saccharified. The saccharified aqueous solution 22 may be cooled by a number of means such as process-to-process exchange, exchange with cooling water, or exchange with chilled water.

Aqueous solution 22 and microorganism 32 may be added to fermentation 30 where the fermentable sugars are metabolized by microorganism 32 to produce stream 105 comprising fermentation products (e.g., product alcohol). In some embodiments, microorganism 32 may be a recombinant microorganism capable of producing product alcohol such as 1-butanol, 2-butanol, or isobutanol. In some embodiments, ammonia and recycle streams may also be added to fermentation 30. In some embodiments, the process may include at least one fermentor, at least two fermentors, at least three fermentors, at least four fermentors, at least five fermentors, or more fermentors. In some embodiments, carbon dioxide generated during fermentation may be vented to a scrubber in order to reduce air emissions (e.g., alcohol air emissions) and to increase product yield.

Stream 105 comprising product alcohol may be conducted to beer column 120 to produce alcohol-rich stream 122 and bottoms stream 125. Alcohol-rich stream 122 may be sent to alcohol recovery 160 for recovery of product alcohol. Product alcohol may be recovered from alcohol-rich stream 122 using methods known in the art including, but not limited to, distillation, adsorption (e.g., by resins), separation by molecular sieves, pervaporation, gas stripping, extraction, and the like. Bottoms stream 125 comprising thin stillage, with most of the solids removed prior to fermentation may be concentrated by evaporation via evaporation 130 to form syrup 135. Methods for evaporation are described in U.S. Patent Application Publication No. 2011/0315541. Syrup 135 may be combined with wet cake (24, 74, 74' as described herein) in mixer 140, and the combined stream 145 of wet cake and syrup may then be dried in a dryer 150 to produce DDGS.

In some embodiments, stream 105 may be degassed. In some embodiments, stream 105 may be heated before degassing, for example, by process-to-process exchange with hot mash. In some embodiments, vapors may be vented to a condenser and then, to a scrubber. Degassed stream 105 may be heated further, for example, by process-to-process heat exchange with other streams in the distillation and/or alcohol recovery area.

In another embodiment of Figure 11, aqueous solution 22, microorganism 32, and extractant may be added to fermentation 30 to produce a biphasic stream. In some embodiments, extractant may be added to fermentation 30 via a recycled loop. In some embodiments, extractant may be added downstream of fermentation 30 or external to fermentation 30. A stream comprising fermentation broth and fermentation product (e.g., product alcohol) may be conducted to an external extractor to produce a stream comprising product alcohol and a bottoms stream. In some embodiments, the stream comprising product alcohol may be conducted to alcohol recovery 160 for recovery of the product alcohol. In some embodiments, the bottoms stream may be conducted to a separation device to separate the bottoms stream into thin stillage and extractant. In some embodiments, the recovered extractant may be recycled for extraction of product alcohol. The thin stillage, with most of the solids removed prior to fermentation, may be concentrated by evaporation 130 to form a syrup. The syrup may be combined with wet cake (24, 74, 74' as described herein) in mixer 140, and the combined stream 145 of wet cake and syrup may then be dried in a dryer 150 to produce DDGS.

In some embodiments, aqueous solution 22, microorganism 32, and extractant may be added to fermentation 30 to form a single liquid phase stream. In some embodiments, the biphasic stream or single liquid phase stream may be withdrawn batchwise from fermentation 30 or may be withdraw continually from fermentation 30. In some embodiments, extractant may be added downstream of fermentation 30 or external to fermentation 30 to form a single liquid phase stream. In some embodiments, extractant may be added to an external extractor to form a single liquid phase stream.

An exemplary process for alcohol recovery is described herein, and additional methods for recovering product alcohols from fermentation broth are described in U.S. Patent Application Publication No. 2009/0305370; U.S. Patent Application Publication No. 2010/0221802; U.S. Patent Application Publication No. 2011/0097773; U.S. Patent Application Publication No. 2011/0312044; U.S. Patent Application Publication No. 2011/0312043; U.S. Patent Application Publication No. 2012/0035398; U.S. Patent Application Publication No. 2012/0156738; PCT International Publication No. WO 2011/159998; and PCT International Publication No. WO 2012/030374. For example, vacuum vaporization may be used to recover product alcohol from the fermentation broth. Preheated beer (e.g., aqueous stream 22) and solvent (e.g., extractant) may enter a preflash column. In some embodiments, the preflash column may be a retrofit of a beer column in a conventional dry grind fuel ethanol plant. This column may be operated at sub-atmospheric pressure, driven by water vapor taken from an evaporator train or from the mash cook step. The overheads of the preflash column may be condensed by heat exchange with some combination of cooling water and process-to-process heat exchange including heat exchange with the preflash column feed. The liquid condensate may be directed to an alcohol/water decanter.

The preflash column bottoms may be advanced to a solvent decanter. The preflash column bottoms may be substantially stripped of product alcohol. The decanter may be a still well, a centrifuge, or a hydrocyclone. Water may be separated from the solvent phase in this decanter, generating a water phase. The water phase including suspended and dissolved solids may be centrifuged to produce a wet cake and thin stillage. The wet cake may be combined with other streams and dried to produce DDGS, it may be dried and sold separate from other streams which produce DDGS, or it may be sold as a wet cake. The water phase may be split to provide a backset which is used in part to re-slurry the wet cake described herein. The split also provides thin stillage which may be conducted to evaporators for further processing.

The organic phase produced in the solvent decanter may be an ester of an alcohol. The solvent may be hydrolyzed to regenerate reactive solvent and to recover additional alcohol. Alternatively, the organic phase may be filtered and sold as a product. Hydrolysis may be thermal driven, homogeneously catalyzed, or heterogeneously catalyzed. The heat input to this process may be a fired heater, hot oil, electrical heat input, or high pressure steam. Water added to drive the hydrolysis may be from a recycled water stream, fresh water, or steam.

Cooled hydrolyzed solvent may be pumped into a sub-atmospheric solvent column where it may be substantially stripped of product alcohol with steam. This steam may be water vapor from evaporators, it may be steam from the flash step of the mash process, or it may be steam from a boiler (see, e.g., U.S. Patent Application Publication No. 2009/0171129). A rectifier column from a conventional dry grind ethanol plant may be suitable as a solvent column. The rectifier column may be modified to serve as a solvent column. The bottoms of the solvent column may be cooled, for example, by cooling water or process-to-process heat exchange. The cooled bottoms may be decanted to remove residual water and this water may be recycled to other steps of the process or recycled to liquefaction.

The solvent column overheads may be cooled by exchange with cooling water or by process-to-process heat exchange, and the condensate may be directed to a vented alcohol/ water decanter which may be shared with the preflash column overheads. Other mixed water and product alcohol streams may be added to this decanter including the scrubber bottoms and condensate from the degas step. The vent which comprises carbon dioxide, may be directed to a water scrubber. The aqueous layer of this decanter may also be fed to the solvent column or may be stripped of product alcohol in a small dedicated distillation column. The aqueous layer may be preheated by process-to-process exchange with the preflash column overheads, solvent column overheads, or solvent column bottoms. This dedicated column may be modified from the side stripper of a conventional dry grind fuel ethanol process.

The organic layer of the alcohol/water decanter may be pumped to an alcohol column. This column may be a super-atmospheric column and may be driven by steam condensation within a reboiler. The feed to the column may be heated by process-to-process heat exchange in order to reduce the energy demand to operate the column. This process-to-process heat exchanger may include a partial condenser of the preflash column, a partial condenser of a solvent column, the product of the hydrolyzer, water vapor from the evaporators, or the alcohol column bottoms. The condensate of the alcohol column vapor may be cooled and may be returned to the alcohol/water decanter. The alcohol column bottoms may be cooled by process-to-process heat exchange including exchange with the alcohol column feed and may be further cooled with cooling water, filtered, and sold as product alcohol.

Thin stillage generated from the preflash column bottoms as described herein may be directed to a multiple effect evaporator (see e.g., U.S. Patent Application Publication No. 2011/0315541). This evaporator may have two, three, or more stages. The evaporator may have a configuration of four bodies by two effects similar to the conventional design of a fuel ethanol plant, it may have three bodies by three effects, or it may have other configurations. Thin stillage may enter at any of the effects. At least one of the first effect bodies may be heated with vapor from the super-atmospheric alcohol column. The vapor may be taken from the lowest pressure effect to provide heat in the form of water vapor to the sub-atmospheric preflash column and solvent column. Syrup from the evaporators may be added to the distillers grain dryer.

Carbon dioxide emissions from the fermentor, degasser, alcohol/water decanter, and other sources may be directed to a water scrubber. The water supplied to the top of this scrubber may be fresh water or may be recycled water.

The recycled water may be treated (e.g., biologically digested) to remove volatile organic compounds and may be chilled. Scrubber bottoms may be sent to the alcohol/water decanter, to the solvent column, or may be used with other recycled water to re-slurry the wet cake described herein. Condensate from the evaporators may be treated with anaerobic biological digestion or other processes to purify the water before recycling to re-slurry the wet cakes.

Oil may be separated from the process streams at any of several points. For example, a centrifuge may be operated to produce an oil stream following filtration of cooked mash or the preflash column water phase centrifuge may be operated to produce an oil stream. Intermediate concentration syrup or final syrup may be centrifuged to produce an oil stream.

In another embodiment, the multi-phase material may leave the bottom of the preflash column and may be processed in a separation system as described herein. The concentrated solids may be redispersed in the aqueous stream and this combined stream may be used to re-pulp and pump the low starch solids that were separated and washed from liquefied mash.

In another exemplary process for product alcohol recovery, an extractant may be utilized to remove the product alcohol from the fermentation broth during fermentation to maintain the product alcohol in the fermentation broth below a certain concentration. In some embodiments, product alcohol removal may be achieved by esterification with carboxylic acid in the presence of a catalyst to produce alcohol esters. A description of processes and systems for extracting alcohol by formation of alcohol esters may be found in U.S. Patent Application Publication No. 2012/0156738. For example, oil separated from the feedstock slurry may be hydrolyzed by a catalyst such as an esterase (e.g., lipase) converting the triglycerides in the oil to fatty acids such as carboxylic acids. These fatty acids may be used an extractant for the recovery of the product alcohol. In some embodiments, the hydrolysis of the oil may occur in the fermentor by the addition of a catalyst to the fermentor. In some embodiments, the hydrolysis of the oil may occur in a separate vessel, and the fatty acids may be added to the fermentor. For example, the feedstock slurry may be conducted to a vessel or tank, and an esterase such as lipase may be added to the vessel, converting the oil present in the feedstock slurry to fatty acids. The feedstock slurry comprising fatty acids may be conducted to the fermentor.

The product alcohol produced by fermentation may react with the fatty acids to produce alcohol esters. In some embodiments, these alcohol esters may be extracted from the fermentation broth. For example, the fermentation broth comprising the alcohol esters may be transferred to a separation device such as a three-phase centrifuge to separate the fermentation broth into three streams: undissolved solids (including microorganism), aqueous stream, and organic stream comprising alcohol esters. In some embodiments, the fermentation broth may be separated into two streams: undissolved solids (including microorganism) and a biphasic mixture comprising an aqueous phase and an organic phase. This separation of the fermentation broth may occur continuously during fermentation, for example, by removing a portion of the fermentation broth for separation, or in batch mode, for example, the entire contents of the fermentor may be removed for separation.

In some embodiments, the biphasic mixture may be separated into an alcohol ester-containing organic phase and aqueous phase and this separation may be achieved using any methods known in the art including, but not limited to, siphoning, aspiration, decantation, centrifugation, gravity settler, membrane-assisted phase splitting, hydrocyclone, and the like. The alcohol ester-containing organic phase may be further processed to recover product alcohol. For example, the alcohol ester-containing organic phase may be transferred to a vessel, where the alcohol esters may be hydrolyzed in the presence of a catalyst to form product alcohol and fatty acids, and this mixture of product alcohol and fatty acids may be processed by distillation to separate the product alcohol and fatty acids.

In some embodiments, the fatty acids may be recycled to the fermentor or an extractor column. In some embodiments, the aqueous stream and undissolved solids may be recycled to the fermentor.

In some embodiments, extraction of the product alcohol may occur downstream of the fermentor or external to the fermentor. In some embodiments, the fermentation system may include an external extraction system that includes, for example, a mixing device and a separation system. Fermentation broth may be conducted to the mixing device, and extractant may be added to the mixing device and combined with fermentation broth to produce a biphasic mixture. The biphasic mixture may be introduced to a separation system, in which separation of biphasic mixture produces an alcohol-containing organic phase and an aqueous phase. In some embodiments, the aqueous phase or a portion thereof may be returned to the fermentor. In some embodiments, the alcohol-containing organic phase may be conducted to an extractant column. The biomass processing productivity in these embodiments is substantially improved by the separation of biomass feed stream components after liquefaction but prior to fermentation. In particular, decreasing the amount of undissolved solids and/or oil provides increased efficiency of external alcohol extraction systems.

In some embodiments, oil may be separated from the feedstock or feedstock slurry and may be stored in an oil storage vessel. For example, oil may be separated from the feedstock or feedstock slurry using any suitable means for separation including three-phase centrifugation or mechanical extraction. To improve the removal of oil from the feedstock or feedstock slurry, oil extraction aids such surfactants, anti-emulsifiers, or flocculants as well as enzymes may be utilized. Examples of oil extraction aids include, but are not limited to, non-polymeric, liquid surfactants; talcum powder; microtalcum powder; enzymes such as Pectinex® Ultra SP-L, Celluclast®, and Viscozyme® L (Sigma-Aldrich, St. Louis, MO), and NZ 33095 (Novozymes, Franklinton, NC); salt (NaOH); and calcium carbonate. Another means to improve oil removal may be pH adjustments such as raising or lowering the pH. Additional benefits for oil removal include increased oil yield, improved oil quality, reduced system deposition, and reduced downtime. In addition, oil removal may also result in cleaner, higher-quality oil.

The remaining feedstock or feedstock slurry may then be further treated to remove any residual oil. For example, the feedstock or feedstock slurry after oil separation may be conducted to a vessel or tank and a catalyst such as an esterase (e.g., lipase) may be added to the vessel, converting the oil present in the feedstock or feedstock slurry to fatty acids. Removing oil from the feedstock or feedstock slurry may improve enzyme efficiencies as well as reduce the amount of enzyme needed for the processes described herein. The feedstock or feedstock slurry may then be conducted to a fermentor and microorganisms may also be added to the fermentor for the production of product alcohol. In some embodiments, the catalyst may be deactivated, for example, by heating. In some embodiments, deactivation may be conducted in a separate vessel, for example, a deactivation vessel. The deactivated feedstock or feedstock slurry may be conducted to a fermentor and microorganisms may also be added to the fermentor for production of product alcohol. Removing oil from the feedstock or feedstock slurry by converting the oil to fatty acids can result in energy savings for the production plant due to more efficient fermentation, less fouling due to the removal of the oil, and decreased energy requirements, for example, the energy needed to dry distillers grains. Following fermentation, the fermentation broth comprising product alcohol may be conducted to an external vessel, for example, an external extractor or external extraction loop for the recovery of product alcohol. Removal of oil as presented here differs from known techniques in that embodiments of the present invention separate oil from the feedstock slurry such that stable emulsions are less likely to occur by virtue of the feed stream separation process, and the need for the addition of protic solvents to break emulsions formed with recovery entrapped bio-oil after fermentation is less likely (see e.g., U.S. Patent No. 7,601,858; U.S. Patent No. 8,192,627). In some embodiments of the present invention, an emulsion may form, but is readily broken by mechanical processing or by other conventional means.

If extractants are used to recover product alcohol, removing oil prior to the fermentation process can reduce the amount of oil taken up by the extractant and thus extend the effectiveness of the extractant for recovering product alcohol. Oil taken up by the extractant can reduce the $K_d$ as well as the selectivity of the extractant, and in turn can increase the operating costs of the production process. As the extractant may be recycled in the production process, each fermentation cycle exposes the extractant to more oil which is taken up by the extractant and over time can result in a significant decrease in the $K_d$ and selectivity of the extractant. The processes and systems described herein provide a means to maintain the $K_d$ and selectivity of the extractant by removing oil from the feedstock or feedstock slurry and/or converting the oil to fatty acids by the addition of a catalyst.

The processes and systems described herein can lead to increased extraction activity and/or efficiency in fermentation product (e.g., product alcohol) production as a result of the removal of the undissolved solids. For example, extractive fermentation without the presence of the undissolved solids can lead to increased mass transfer rates of the product alcohol from the fermentation broth to the extractant, better phase separation, and lower hold-up of extractant as a result of increased extractant droplet rise velocities. Also, for example, extractant droplets held up in the fermentation broth during fermentation will disengage from the fermentation broth faster and more completely, thereby resulting in less free extractant in the fermentation broth. In addition, lower hold-up of extractant can decrease the amount of extractant lost in the process. Additional benefits of solids removal include, for example, elimination of agitators in the fermentor and downstream processing equipment such as beer columns and centrifuges resulting in a reduction of capital costs and energy use; increased fermentor volume resulting in increased fermentor productivity; decreased extractant hold-up resulting in increased production efficiency, increased recovery and recycling of extractant, reduced flow rate of extractant which will lower operating costs, and the potential to use continuous fermentation or smaller fermentors. In some embodiments, the volume of the fermentor available for the fermentation may be increased by at least 5%, at least 10%, or more.

Examples of increased extraction efficiency include, for example, stabilization of the partition coefficient, enhanced phase separation, enhanced mass transfer coefficient, operation at a lower titer, increased process stream recyclability, increased fermentation volume efficiency, increased feedstock load feeding, increased product alcohol titer tolerance of the microorganism, water recycling, reduction in energy, increased recycling of extractant, and recycling of the microorganism. For example, because oil in the fermentation broth can be reduced by removing solids from feedstock slurry prior to fermentation, the extractant is exposed to less oil which can combine with the extractant and lower the partition coefficient of the extractant. Therefore, a reduction of oil in the fermentation broth results in a more stable partition coefficient over multiple fermentation cycles. In some embodiments, the partition coefficient may be decreased by less than 10%, less than 5%, or less than 1% over ten or more fermentation cycles. As another example of increased extraction efficiency, a higher mass transfer rate (e.g., in the form of a higher mass transfer coefficient) can result in an increased efficiency of product alcohol production. In some embodiments, the mass transfer coefficient may be increased at least 2-fold, at least 3-fold, at least 4-fold, or at least 5-fold.

An increase in phase separation between fermentation broth and extractant reduces the likelihood of emulsion formation resulting in an increased efficiency of product alcohol production. For example, in the absence of an emulsion, phase separation can occur more quickly and can be more complete. In some embodiments, phase separation may occur where previously no appreciable phase separation was observed. In some embodiments, phase separation may occur in 24 hours. In some embodiments, phase separation may occur at least two times (2x) as quickly, at least five times (5x) as quickly, or at least ten times (10x) as quickly as compared to phase separation where solids have not been removed or emulsions have formed.

As described herein, nutrients such as nitrogen, minerals, trace elements, and/or vitamins may be added to the feedstock slurry or the fermentor. These added nutrients as well as nutrients naturally occurring in the feedstock may be soluble in oil, and thus the presence of oil in the feedstock or feedstock slurry may reduce the concentrations of these nutrients in the fermentation broth. Removing oil from the feedstock or feedstock slurry can minimize the loss of nutrients. In addition, the presence of solids in the fermentation broth may also lead to a reduction in the concentrations of nutrients. Removing the solids and/or oil from the feedstock or feedstock slurry can minimize the loss of nutrients.

For the processes and systems described herein, the presence of oil in the fermentation process may have an effect on the partition coefficient of the extractant over the course of multiple fermentations. Removing oil from the feedstock or feedstock slurry can reduce the variability of the partition coefficient of the extractant over the course of multiple fermentations, and therefore improve the scalability of the processes and systems described herein. Scalability refers to the ability to modify (e.g., expand or condense) a process or system to accommodate, for example, manufacturing demands (e.g., operating volume) without a penalty in functionality.

In addition, removing solids from feedstock or feedstock slurry can also have an effect on scalability. For example, if an external extractor is utilized, reduced solids (e.g., reduced total suspended solids, TSS) can result in improved performance and better scalability. Reduced solids enhance the rate of mass transfer of product alcohol between the aqueous phase and organic phase (e.g., fermentation broth and extractant). Solid particles coat the surface of the extractant droplets effectively reducing the area for mass transfer. Solid particles also inhibit phase separation by increasing viscosity and the tendency for emulsification. Since the presence of solids can impede the operation of the external extractor, removing solids from the feedstock or feedstock slurry provides for improved scalability and reliability of an external extractor.

Therefore, removing solids and/or oil may improve the scalability of the unit operations of processes and systems described herein. For example, removing solids and/or oil may improve unit operations such as, but not limited to, extractor performance, distillation column performance, heat exchanger performance, and/or evaporator performance.

As an example of improved unit operations, referring to Figure 11, stream 105 may be conducted to beer column 120 to produce alcohol-rich stream 122 and bottoms stream 125. Bottoms stream 125 comprising thin stillage, with most of the solids removed prior to fermentation may be concentrated via evaporation 130. By removing solids prior to fermentation, less solids may be sent to the evaporators which can result in a lower feed rate to the evaporators. A lower feed rate requires less energy and therefore, lower costs due to lower energy requirements.

In some embodiments, there may be a need to remove water from the oil recovered from feedstock or feedstock slurry. In some embodiments, water may be removed by a number of methods including gravity separation, coalescing separator, centrifugation (e.g. decanter), adsorption or absorption, distillation, heating, vacuum dehydration, and/or air stripping (e.g., air, nitrogen). Examples of adsorption media include, but are not limited to, activated alumina, bentonite clay, calcium chloride, calcium sulfate, cellulose, magnesium sulfate, molecular sieve, polymers, and/or silica gel. In some embodiments, the adsorption media may be continuously stirred with the oil, or the oil may flow through a packed bed with adsorption media.

From time to time, it may be necessary to clean and/or sterilize the equipment used in the production of the fermentation products such as product alcohols. Examples of equipment include, but are not limited to, fermentors, liquefaction vessels, saccharification vessels, holding tanks, storage tanks, heat exchangers, pipelines, equipment connections, nozzles, fittings, and valves. Cleaning and sterilization can reduce or eliminate microbial contamination as well as minimize the accumulation of residues (e.g., carbohydrates, sugars) on equipment. Residue build-up on equipment can provide a nutritional source for unwanted microorganisms, leading to the proliferation of these microorganisms. There are a number of methods utilized to clean and/or sterilize fermentation equipment including clean-in-place (CIP) and sterilization-in-place (SIP). CIP and SIP may be performed manually or automated systems are also available. A suitable as well as efficient CIP or SIP can maximize the profitability of the production plant by minimizing the need to shut down operations due to, for example, a microbial contamination.

In the processes and systems described herein, removal of solids and oil from feedstock or feedstock slurry, for example, prior to fermentation can improve the efficiency of CIP and SIP. The lack of solids in the fermentation equipment would allow for less rinse water, less cleaning solution, and less time to perform CIP and SIP. Thus, removal of solids may improve the efficiency and reduce the costs of CIP and SIP processes. Also, the caustic agents used for CIP (e.g., sodium hydroxide) may react with the triglycerides in oil forming soap (i.e., saponification) which can have an effect on the efficiency of CIP. Therefore, the removal of oil can reduce the formation of soap during CIP and improve the efficiency of CIP.

During the fermentative process, fouling can have an impact on the productivity and efficiency of the production process. In general, fouling refers to the deposit of extraneous materials or particles, for example, the deposit of materials on the surface of heat exchangers and distillation column reboilers. This deposit of materials on the surface of the heat exchanger can interfere with the transfer of heat and reduce the operational capability of the heat exchanger. For example, the material deposit may interfere with the flow of fluid through the exchanger resulting in an increase in flow resistance. Also, the deposit of material on the surface of heat exchangers or other equipment may require additional cleaning. These additional cleaning requirements may necessitate plant shutdowns which can result in a reduction in plant productivity. In the processes and systems described herein, removal of oil and/or solids from the feedstock or feedstock slurry, for example, prior to fermentation can minimize the deposition of materials and lower the rate of deposition of materials on the surfaces of equipment such as heat exchangers. Thus, solids and oil removal can lower the rate of fouling of the heat exchangers and minimize the effect of fouling on heat transfer and operational capability.

In another example of an embodiment of the processes and systems of the invention, the material discharged from the fermentor may be processed in a separation system that involves devices such as a centrifuge, settler, hydrocyclone, etc., and combinations thereof to effect the recovery of microorganisms in a concentrated form that may be recycled for reuse in a subsequent fermentations either directly or following reconditioning. The ability to recycle microorganisms can increase the overall rate of fermentation product production such as product alcohol production, lower the overall titer requirement, and/or lower the aqueous titer requirement, thereby leading to healthier microorganisms and a higher production rate. This separation system may also produce an organic stream that comprises fermentation product (e.g., product alcohol) and other by-products produced from the fermentation, and an aqueous stream containing only trace levels of immiscible organics. This aqueous stream may be used either before or after it is stripped of product alcohol content to wash the solids that were separated from feedstock slurry. This has the advantage of avoiding what might otherwise be a long belt-driven conveying system to transfer these solids from the liquefaction area to the grain drying and syrup blend area. Furthermore, whole stillage produced after product alcohol has been stripped will need to be separated into thin stillage and wet cake fractions either using existing or new separation devices. The thin stillage may form in part the backset that may be combined with cook water for preparing a new batch of fermentable mash. Another advantage of this embodiment is that any residual fermentable sugars that were retained in the solids separated from feedstock slurry would in part be captured and recovered through this backset. Alternatively, microorganisms contained in the solids stream may be redispersed in the aqueous stream and this combined stream distilled of any product alcohol content remaining from fermentation. If the microorganisms are nonviable, the non-viable microorganisms may further be separated for use as a nutrient, for example, in a propagation process.

In some embodiments of the processes and systems described herein, by-products (or co-products) of the fermentation process may be further processed, for example, undissolved solids may be processed to generate DDGS. Other by-products such as fatty acid esters which may have an inhibitory effect on the microorganisms may be recovered from the fermentation broth and/or by-product streams resulting in an increase in the yield of product alcohol. Recovery of fatty acid esters or other lipids may be accomplished by using a solvent to extract fatty acid esters from the by-product streams. In some embodiments, several by-product streams may be combined and fatty acid esters may be recovered from the combined streams.

In an embodiment of solvent extraction of lipids (e.g., fatty acid esters), solids may be separated from whole stillage ("separated solids") since this stream would contain a large portion of fatty acid esters. These separated solids may then be fed to an extractor and washed with solvent. In some embodiments, the separated solids may be washed at least two or more times. After washing, the resulting mixture of lipid and solvent, known as miscella, may be collected for separation of the extracted lipid from the solvent. For example, the resulting mixture of lipid and solvent may be conducted to a separator or extractor for further processing. During the extraction process, the solvent not only extracts lipid into solution, but it also collects fine particles ("fines"). These fines are generally undesirable impurities in the miscella and in one embodiment, the miscella may be discharged from the separator or extractor and conducted to a separation device that separates or scrubs the fines from the miscella.

In order to separate lipid and solvent contained in the miscella, the miscella may be subjected to a distillation step. In this step, the miscella can, for example, be processed through an evaporator which heats the miscella to a temperature that is high enough to cause vaporization of the solvent, but is not sufficiently high to adversely affect or vaporize the extracted lipid. As the solvent evaporates, it may be collected, for example, in a condenser, and recycled for future use. Separation of the solvent from the miscella results in a stock of crude lipid which may be further processed to separate water, fatty acid esters (e.g., fatty acid isobutyl esters), fatty acids, and triglycerides. A solvent-based extraction system for recovering triglycerides is described in U.S. Patent Application Publication No. 2010/0092603.

After extraction of the lipids, the solids may be conveyed from the extractor and may be conducted to a stripping device (e.g., desolventizer) to remove residual solvent. Recovery of residual solvent can be important to process economics. In some embodiments, the solids may be conveyed to a desolventizer in a vapor tight environment to preserve and collect solvent that may transiently evaporate from the solids. As the solids enter the desolventizer, the solids may be heated to vaporize and remove the residual solvent. In order to heat the solids, the desolventizer may include a mechanism for distributing the solids over one or more trays, and the solids may be heated directly such as through direct contact with heated air or steam, or indirectly such as by heating the tray carrying the solids. In order to facilitate transfer of the solids from one tray to another, the trays carrying the solids may include openings that allow the solids to pass from one tray to the next tray. From the desolventizer, the solids may optionally be conveyed to a mixer where the solids are mixed with other by-products before being conveyed to a dryer. In some embodiments, the solids are conducted to a desolventizer and the solids are contacted by steam. In some embodiments, the flows of steam and solids in the desolventizer may be countercurrent. In some embodiments, vapor exiting the desolventizer may be condensed, optionally mixed with miscella, and then fed to a decanter forming a water-rich phase. This water-rich phase exiting the decanter may be fed to a distillation column where solvent is removed from the water-rich stream. In some embodiments, a solvent-depleted water-rich stream may exit the bottom of the distillation column and may be recycled to the fermentation process, for example, it may be used to process the feedstock. In some embodiments, overhead and bottom products of the distillation column may be recycled to the fermentation process. For example, the lipid-rich bottoms may be added to the feed of a hydrolyzer. The overheads may be, for example, condensed and fed to a decanter forming a solvent-rich stream and a water-rich phase. The solvent-rich stream exiting this decanter may optionally be used as the solvent feed to an extractor, and the water-rich phase exiting this decanter may be fed to a stripping column to strip solvent from water.

In some embodiments, by-products or co-products may be derived from feedstock slurry used in the fermentation process. As described herein, if corn is used as feedstock, corn oil may be separated from the feedstock slurry prior to fermentation. The benefits of removing corn oil prior to the fermentation process are: recovering more corn oil as compared to corn oil removal at the end of the fermentation process (e.g., from the syrup), recovering higher quality and therefore higher value oil as compared to corn oil removal at end of the fermentation process, generating corn oil as a co-product, and conversion of corn oil to other products.

For example, as corn oil contains triglycerides, diglycerides, monoglycerides, fatty acids, phytosterols, vitamin E, carotenoids (e.g., β-carotene, β-cryptoxanthin, lutein zeaxanthin), phospholipids, and antioxidants such as tocopherols, it may be added to other co-products at different concentrations or rates, creating the ability to vary the amount of these components in the resulting co-product. In this manner, the fat content of the resulting co-product may be controlled, for example, to yield a lower fat, high protein animal feed that would better suit the needs of dairy cows compared to a high fat product. In another embodiment where a high fat animal feed may be desired, corn oil may be used as a component of animal feed because its high triglyceride content would provide a source of metabolizable energy. In addition, the natural antioxidants in corn oil provide a source of vitamin E as well as reduce the development of rancidity.

Corn oil separated from feedstock may be further processed to produce refined corn oil or edible oil for consumer use. For example, crude corn oil may be further processed to produce refined corn oil by degumming to remove phosphatides, alkali refining to neutralize free fatty acids, decolorizing for removal of color bodies and trace elements, winterizing to remove waxes, and deodorization (*see, e.g.,* Corn Oil, 5th Edition, Corn Refiners Association, Washington, D.C., 2006). The refined corn oil may be used, for example, by food manufacturers for the production of food products. The free fatty acids removed by alkali refining may be used as soapstock and waxes recovered from the winterizing step may be utilized in animal feeds.

Corn oil may be used in the manufacture of resins, plastics, polymers, lubricants, paints, varnishes printing inks, soap, and textiles; and may also be utilized by the pharmaceutical industry as a component of drug formulations. Corn oil may also be used as feedstock for biodiesel or renewable diesel.

In some embodiments, oils such as corn oil may be used as a feedstock for the generation of extractant for extractive fermentation. For example, oil derived from biomass may be converted into an extractant available for removal of a product alcohol such as butanol from a fermentation broth. The glycerides in the oil may be chemically or enzymatically converted into a reaction product, such as fatty acids, fatty alcohols, fatty amides, fatty acid alkyl esters, fatty acid glycol esters, and hydroxylated triglycerides, or mixtures thereof, which may be used a fermentation product extractant. Using corn oil as an example, corn oil triglycerides may be reacted with a base such as ammonia hydroxide or sodium hydroxide to obtain fatty amides, fatty acids, and glycerol. These fatty amides, fatty acids, or mixtures thereof may be used an extractant. In some embodiments, plant oil such as corn oil may be hydrolyzed by an enzyme such as lipase to form fatty acids (e.g., corn oil fatty acids). Methods for deriving extractants from biomass are described in U.S. Patent Application Publication No. 2011/0312043, U.S. Patent Application Publication No. 2011/0312044, and PCT International Publication No. WO 2011/159998. In some embodiments, extractant may be used, all or in part, as a component of an animal feed or it can be used as feedstock for biodiesel or renewable diesel.

In some embodiments, corn oil can also be used as feedstock for biodiesel or renewable diesel. In some embodiments, oils or a combination of oils can also be used as feedstock for biodiesel or renewable diesel. Examples of oils include canola, castor, corn, jojoba, karanja, mahua, linseed, soybean, palm, peanut, rapeseed, rice, safflower, and sunflower oils. Biodiesel may be derived from either the transesterification or esterification of plant oils with alcohols such as methanol, ethanol, and butanol. For example, biodiesel may be produced by acid-catalyzed, alkali-catalyzed, or enzyme-catalyzed transesterification or esterification (e.g., transesterification of plant oil-derived triglycerides or esterification of plant oil-derived free fatty acids). Inorganic acids such as sulfuric acid, hydrochloric acid, and phosphoric acid; organic acids such as toluenesulfonic acid and naphthalenesulfonic acid; solid acids such as Amberlyst® sulfonated polystyrene resins; or zeolites may be used as a catalyst for acid-catalyzed transesterification or esterification. Bases such as potassium hydroxide, potassium methoxide, sodium hydroxide, sodium methoxide, or calcium hydroxide may be used as a catalyst for alkali-catalyzed transesterification or esterification. In some embodiments, biodiesel may be produced by an integrated process, for example, acid-catalyzed esterification of free fatty acids followed by base-catalyzed transesterification of triglycerides.

Enzymes such as lipases or esterases may be used to catalyze transesterification or esterification reactions. Lipases may be derived from bacteria or fungi, for example, *Pseudomonas, Thermomyces, Burkholderia, Candida,* and *Rhizomucor.* In some embodiments, lipases may be derived *Pseudomonas fluorescens, Pseudomonas cepacia, Rhizomucor miehei, Burkholderia cepacia, Thermomyces lanuginosa,* or *Candida antarctica.* In some embodiments, the enzyme may be immobilized on a soluble or insoluble support. The immobilization of enzymes may be performed using a variety of techniques including 1) binding of the enzyme to a porous or non-porous carrier support, via covalent support, physical adsorption, electrostatic binding, or affinity binding; 2) crosslinking with bifunctional or multifunctional reagents; 3) entrapment in gel matrices, polymers, emulsions, or some form of membrane; and 4) a combination of any of these methods. In some embodiments, lipase may be immobilized, for example, on acrylic resin, silica, or beads (e.g., polymethacrylate beads). In some embodiments, the lipases may be soluble.

In some embodiments, biodiesel described herein may comprise one or more of the following fatty acid alkyl esters (FAAE): fatty acid methyl esters (FAME), fatty acid ethyl esters (FAEE), and fatty acid butyl esters (FABE). In some embodiments, biodiesel described herein may comprise one or more of the following: myristate, palmitate, stearate, oleate, linoleate, linolenate, arachidate, and behenate.

In some embodiments, oil from the fermentation process may be recovered by evaporation forming a non-aqueous stream. This non-aqueous stream may comprise fatty acid esters and fatty acids and this stream may be conducted to a hydrolyzer to recover product alcohol and fatty acids. In some embodiments, this stream may be used as feedstock for biodiesel production.

In some embodiments, the biodiesel described herein meets the specifications of the American Society for Testing and Materials (ASTM) D6751. In some embodiments, the biodiesel described herein meets the specifications of the European standard EN 14214.

In some embodiments, reactor configurations for the production of biodiesel include, for example, batch-stirred tank reactors, continuous-stirred tank reactors, packed bed reactors, fluid bed reactors, expanding bed reactors, and recirculation membrane reactors.

In some embodiments, a composition may comprise at least 2% biodiesel, at least 5% biodiesel, at least 10% biodiesel, at least 20% biodiesel, at least 30% biodiesel, at least 40% biodiesel, at least 50% biodiesel, at least 60% biodiesel, at least 70% biodiesel, at least 80% biodiesel, at least 90% biodiesel, or 100% biodiesel.

In some embodiments, the biodiesel described herein may be blended with a petroleum-based diesel fuel to form a biodiesel blend. In some embodiments, a biodiesel blend may comprise at least 2% by volume biodiesel, at least 3% by volume biodiesel, at least 4% by volume biodiesel, at least 5% by volume biodiesel, at least 6% by volume biodiesel, at least 7% by volume biodiesel, at least 8% by volume biodiesel, at least 9% by volume biodiesel, at least 10% by volume biodiesel, at least 11% by volume biodiesel, at least 12% by volume biodiesel, at least 13% by volume biodiesel, at least 14% by volume biodiesel, at least 15% by volume biodiesel, at least 16% by volume biodiesel, at least 17% by volume biodiesel, at least 18% by volume biodiesel, at least 19% by volume biodiesel, or at least 20% by volume biodiesel. In some embodiments, a biodiesel blend may comprise up to 20% by volume biodiesel.

A by-product of biodiesel production is glycerol. In addition, glycerol may also be a by-product of the generation of extractant from oils and a by-product of the fermentation process. A feedstock for biodiesel may be produced by reacting a fatty acid such as COFA with glycerol. The reaction may be catalyzed by strong inorganic acids such as sulfuric acid or by solid acid catalysts such as Amberlyst™ polymeric catalysts and ion exchange resins. High conversions may be obtained by withdrawing water from the reaction mass. The reaction product may contain monoglycerides, diglycerides, and triglycerides in a proportion determined by the ratio of reactants and the extent of reaction. The glyceride mix may be used in lieu of the triglyceride feed normally used to make biodiesel. In some embodiments, the glycerides may be used as a surfactant or as a feedstock for biodiesel.

Solids are separated from feedstock slurry and may comprise triglycerides and fatty acids. These solids may be used as an animal feed, either recovered as discharge from centrifugation or after drying. The solids may be particularly suited as feed for ruminants (e.g., dairy cows) because of its high content of available lysine and by-pass or rumen undegradable protein. For example, these solids may be of particular value in a high protein, low fat feed. In some embodiments, these solids may be used as a base, that is, other by-products such as syrup may be added to the solids to form a product that may be used as an animal feed. In some embodiments, different amounts of other by-products may be added to the solids to tailor the properties of the resulting product to meet the needs of a certain animal species (e.g., dairy and beef cattle, poultry, swine, livestock, equine, aquaculture, and domestic pets).

In some embodiments where a low fat animal feed is desired, oil may be removed from the feedstock prior to fermentation. By removing the corn oil, the DDGS produced would have a low fat, high protein content. If the corn oil is not removed, the oil present in the wet cake can be oxidized by the drying process. This oxidation causes a darkening effect and produces DDGS with a darker color. If the oil is removed from the feedstock prior to fermentation, the DDGS produced would be lighter in color and this lighter color DDGS may be desirable for some animal feed products.

The composition of solids separated from whole stillage may include, for example, crude protein, fatty acids, and fatty acid esters. In some embodiments, this composition may be used, wet or dry, as an animal feed where, for example, a high protein (e.g., high lysine), low fat, and high fiber content is desired. In some embodiments, fat may be added to this composition, for example, from another by-product stream if a higher fat, low fiber animal feed is desired. In some embodiments, this higher fat, low fiber animal feed may be used for swine or poultry. In some embodiments, a non-aqueous composition of CDS may include, for example, protein, fatty acids, and fatty acid esters as well as other dissolved and suspended solids such as salts and carbohydrates. This CDS composition may be used, for example, as animal feed, either wet or dry, where a high protein, low fat, high mineral salt feed component is desired. In some embodiments, this composition may be used as a component of a dairy cow ration.

In some embodiments, one or more streams generated by the production of a product alcohol via a fermentation process may be combined to generate a composition comprising at least 90% fatty acids which may be used as fuel source such as biodiesel.

The various streams generated by the production of a product alcohol via a fermentation process may be combined in many ways to generate a number of co-products. For example, if crude corn from mash is used to generate fatty acids to be utilized as extractant and lipid is extracted by evaporators, then the remaining streams may be combined and processed to create a co-product composition comprising crude protein, crude fat, triglycerides, fatty acids, and fatty acid esters. In another example, if oil such as corn oil is removed from feedstock slurry, the oil may be added to distillers grains to produce, for example, an animal feed product.

In some embodiments, compositions of the processes and systems described herein may comprise at least 20-35 wt% crude protein, at least 1-20 wt% crude fat, at least 0-5 wt% triglycerides, at least 4-10 wt% fatty acids, and at least 2-6 wt% fatty acid esters. In some embodiments, compositions may comprise 25 wt% crude protein, 10 wt% crude fat, 0.5 wt% triglycerides, 6 wt% fatty acids, and 4 wt% fatty acid esters. In some embodiments, compositions may comprise at least 25-31 wt% crude protein, at least 6-10 wt% crude fat, at least 4-8 wt% triglycerides, at least 0-2 wt% fatty acids, and at least 1-3 wt% fatty acid esters. In some embodiments, compositions may comprise 28 wt% crude protein, 8 wt% crude fat, 6 wt% triglycerides, 0.7 wt% fatty acids, and 1 wt% fatty acid esters. In some embodiments, the fatty acid esters may be fatty acid methyl esters, fatty acid ethyl esters, fatty acid butyl esters, or fatty acid isobutyl ester.

In some embodiments, solids separated from whole stillage and oil extracted from feedstock slurry may be combined and the resulting composition may comprise crude protein, crude fat, triglycerides, fatty acids, fatty acid esters, lysine, neutral detergent fiber (NDF), and acid detergent fiber (ADF). In some embodiments, compositions may comprise at least 26-34 wt% crude protein, at least 15-25 wt% crude fat, at least 12-20 wt% triglycerides, at least 1-2 wt% fatty acids, at least 2-4 wt% fatty acid esters, at least 1-2 wt% lysine, at least 11-23 wt% NDF, and at least 5-11 wt% ADF. In some embodiments, compositions may comprise 29 wt% crude protein, 21 wt% crude fat, 16 wt% triglycerides, 1 wt% fatty acids, 3 wt% fatty acid esters, 1 wt% lysine, 17 wt% NDF, and 8 wt% ADF. In some embodiments, the fatty acid esters may be fatty acid methyl esters, fatty acid ethyl esters, fatty acid butyl esters, or fatty acid isobutyl ester. The high fat, triglyceride, and lysine content and the lower fiber content of this composition may be desirable as feed for swine and poultry.

As described herein, the various streams generated by the production of a product alcohol via a fermentation process may be combined in many ways to generate a composition comprising crude protein, crude fat, triglycerides, fatty acids, and fatty acid esters. For example, a composition comprising at least 6% crude fat and at least 28% crude protein may be utilized as an animal feed product for dairy animals. A composition comprising at least 6% crude fat and at least 26% crude protein may be utilized as an animal feed product for feedlot cattle whereas a composition comprising at least 1% crude fat and at least 27% crude protein may be utilized as an animal feed product for wintering cattle. A composition comprising at least 13% crude fat and at least 27% crude protein may be utilized as an animal feed product for poultry. A composition comprising at least 18% crude fat and at least 22% crude protein may be utilized as an animal feed product for monogastric animals. The various streams may be combined in such a way as to customize a feed product for a specific animal species (e.g., livestock, ruminant, cattle, dairy animal, swine, goat, sheep, poultry, equine, aquaculture, or domestic pet such as dogs, cats, and rabbits).

The DDGS generated by the processes of the present invention may be modified to produce a customized high value feed product by the addition of one or more of the following: protein, fat, fiber, ash, lipid, amino acids, vitamins, and minerals. Amino acids include, for example, essential amino acids such as histidine, isoleucine, leucine, lysine, methionine, phenylalanine, threonine, tryptophan, and valine as well as other amine acids such as alanine, arginine, aspartic acid, asparagine, cysteine, glutamic acid, glutamine, glycine, hydroxylysine, hydroxyproline, ornithine, proline, serine, and tyrosine. Minerals include, for example, calcium, chloride, cobalt, copper, fluoride, iodine, iron, magnesium, manganese, phosphorus, potassium, selenium, sodium, sulfur, and zinc. Vitamins include, for example, vitamins A, C, D, E, K, and B (thiamine, riboflavin, niacin, pantothenic acid, biotin, vitamin B6, vitamin B12 and folate).

As described herein, the processes and systems of the present invention provide a number of benefits that can result in improved production of a product alcohol such as butanol. For example, an improvement in mass transfer enables operation at a lower aqueous titer resulting in a "healthier" microorganism. A better phase separation can lead to improved fermentor volume efficiency as well as the possibility of processing less reactor contents through beer columns, distillation columns, etc. In addition, there is less solvent loss via solids and there is the possibility of cell recycling. The processes and systems of the present invention may also provide a higher quality of DDGS.

The processes and systems described herein also provide for the removal of oil prior to fermentation which would then allow the controlled addition of oil to the fermentation. Furthermore, the removal of oil prior to fermentation would allow flexibility in the amount of oil present in DDGS. That is, oil may be added in different amounts to DDGS resulting in the production of DDGS with different fat contents depending on the nutritional needs of a particular animal species.

The processes and systems described herein may be demonstrated using computational modeling such as Aspen modeling (see, e.g., U.S. Patent No. 7,666,282). For example, the commercial modeling software Aspen Plus® (Aspen Technology, Inc., Burlington, MA) may be used in conjunction with physical property databases such as DIPPR (Design Institute for Physical Property Research), available from American Institute of Chemical Engineers, Inc. (New York, NY) to develop an Aspen model for an integrated alcohol fermentation, purification, and water management process. This process modeling can perform many fundamental engineering calculations, for example, mass and energy balances, vapor/liquid equilibrium, and reaction rate computations. In order to generate an Aspen model, information input may include, for example, experimental data, water content and composition of feedstock, temperature for mash cooking and flashing, saccharification conditions (e.g., enzyme feed, starch conversion, temperature, pressure), fermentation conditions (e.g., microorganism feed, glucose conversion, temperature, pressure), degassing conditions, solvent columns, preflash columns, condensers, evaporators, centrifuges, etc.

## Recombinant microorganisms

While not wishing to be bound by theory, it is believed that the processes described herein are useful in conjunction with any alcohol-producing microorganism, particularly recombinant microorganisms which produce alcohol at titers above their tolerance levels.

Alcohol-producing microorganisms are known in the art. For example, fermentative oxidation of methane by methanotrophic bacteria (e.g., *Methylosinus trichosporium*) produces methanol, and the yeast strain CEN.PK113-7D (CBS 8340, the Centraal Buro voor Schimmelculture; van Dijken, et al., Enzyme Microb. Techno. 26:706-714, 2000) produces ethanol. Recombinant microorganisms which produce alcohol are also known in the art (e.g., Ohta, et al., Appl. Environ. Microbiol. 57:893-900, 1991; Underwood, et al., Appl. Environ. Microbiol. 68:1071-1081, 2002; Shen and Liao, Metab. Eng. 10:312-320, 2008; Hahnai, et al., Appl. Environ. Microbiol. 73:7814-7818, 2007; U.S. Patent No. 5,514,583; U.S. Patent No. 5,712,133; PCT Application Publication No. WO 1995/028476; Feldmann, et al., Appl. Microbiol. Biotechnol. 38: 354-361, 1992; Zhang, et al., Science 267:240-243, 1995; U.S. Patent Application Publication No. 2007/0031918 A1; U.S. Patent No. 7,223,575; U.S. Patent No. 7,741,119; U.S. Patent No. 7,851,188; U.S. Patent Application Publication No. 2009/0203099 A1; U.S. Patent Application Publication No. 2009/0246846 A1; and PCT Application Publication No. WO 2010/075241).

In addition, microorganisms may be modified using recombinant technologies to generate recombinant microorganisms capable of producing product alcohols such as ethanol and butanol. Microorganisms that may be recombinantly modified to produce a product alcohol via a biosynthetic pathway include members of the genera *Clostridium, Zymomonas, Escherichia, Salmonella, Serratia, Erwinia, Klebsiella, Shigella, Rhodococcus, Pseudomonas, Bacillus, Lactobacillus, Enterococcus, Alcaligenes, Klebsiella, Paenibacillus, Arthrobacter, Corynebacterium, Brevibacterium, Schizosaccharomyces, Kluyveromyces, Yarrowia, Pichia, Candida, Hansenula, Issatchenkia,* or *Saccharomyces.* In some embodiments, recombinant microorganisms may be selected from the group consisting of *Escherichia coli, Lactobacillus plantarum, Kluyveromyces lactis, Kluyveromyces marxianus* and *Saccharomyces cerevisiae.* In some embodiments, the recombinant microorganism is yeast. In some embodiments, the recombinant microorganism is crabtree-positive yeast selected from *Saccharomyces, Zygosaccharomyces, Schizosaccharomyces, Dekkera, Torulopsis, Brettanomyces,* and some species of *Candida.* Species of crabtree-positive yeast include, but are not limited to, *Saccharomyces cerevisiae, Saccharomyces kluyveri, Schizosaccharomyces pombe, Saccharomyces bayanus, Saccharomyces mikitae, Saccharomyces paradoxus, Zygosaccharomyces rouxii,* and *Candida glabrata.*

*Saccharomyces cerevisiae* are known in the art and are available from a variety of sources including, but not limited to, American Type Culture Collection (Rockville, MD), Centraalbureau voor Schimmelcultures (CBS) Fungal Biodiversity Centre, LeSaffre, Gert Strand AB, Ferm Solutions, North American Bioproducts, Martrex, and Lallemand. *Saccharomyces cerevisiae* include, but are not limited to, BY4741, CEN.PK 113-7D, Ethanol Red® yeast, Ferm Pro™ yeast, Bio-Ferm® XR yeast, Gert Strand Prestige Batch Turbo alcohol yeast, Gert Strand Pot Distillers yeast, Gert Strand Distillers Turbo yeast, FerMax™ Green yeast, FerMax™ Gold yeast, Thermosacc® yeast, BG-1, PE-2, CAT-1, CBS7959, CBS7960, and CBS7961.

In some embodiments, the microorganism may be immobilized or encapsulated. For example, the microorganism may be immobilized or encapsulated using alginate, calcium alginate, or polyacrylamide gels, or through the induction of biofilm formation onto a variety of high surface area support matrices such as diatomite, celite, diatomaceous earth, silica gels, plastics, or resins. In some embodiments, ISPR may be used in combination with immobilized or encapsulated microorganisms. This combination may improve productivity such as specific volumetric productivity, metabolic rate, product alcohol yields, tolerance to product alcohol. In addition, immobilization and encapsulation may minimize the effects of the process conditions such as shearing on the microorganisms.

The production of butanol utilizing fermentation, as well as microorganisms which produce butanol, is disclosed, for example, in U.S. Patent No. 7,851,188, and U.S. Patent Application Publication Nos. 2007/0092957; 2007/0259410; 2007/0292927; 2008/0182308; 2008/0274525; 2009/0155870; 2009/0305363; and 2009/0305370. In some embodiments, the microorganism is engineered to contain a biosynthetic pathway. In some embodiments, the biosynthetic pathway is an engineered butanol biosynthetic pathway. In some embodiments, the biosynthetic pathway converts pyruvate to a fermentation product. In some embodiments, the biosynthetic pathway converts pyruvate as well as amino acids to a fermentation product. In some embodiments, at least one, at least two, at least three, or at least four polypeptides catalyzing substrate to product conversions of a pathway are encoded by heterologous polynucleotides in the microorganism. In some embodiments, all polypeptides catalyzing substrate to product conversions of a pathway are encoded by heterologous polynucleotides in the microorganism. In some embodiments, the polypeptide catalyzing the substrate to product conversions of acetolactate to 2,3-dihydroxyisovalerate and/or the polypeptide catalyzing the substrate to product conversion of isobutyraldehyde to isobutanol are capable of utilizing reduced nicotinamide adenine dinucleotide (NADH) as a cofactor.

## Biosynthetic Pathways

Biosynthetic pathways for the production of isobutanol that may be used include those described in U.S. Patent No. 7,851,188. In one embodiment, the isobutanol biosynthetic pathway comprises the following substrate to product conversions:

a) pyruvate to acetolactate, which may be catalyzed, for example, by acetolactate synthase;

b) acetolactate to 2,3-dihydroxyisovalerate, which may be catalyzed, for example, by acetohydroxy acid reductoisomerase;

c) 2,3-dihydroxyisovalerate to α-ketoisovalerate, which may be catalyzed, for example, by acetohydroxy acid dehydratase;

d) α-ketoisovalerate to isobutyraldehyde, which may be catalyzed, for example, by a branched-chain α-keto acid decarboxylase; and

e) isobutyraldehyde to isobutanol, which may be catalyzed, for example, by a branched-chain alcohol dehydrogenase.

In another embodiment, the isobutanol biosynthetic pathway comprises the following substrate to product conversions:

a) pyruvate to acetolactate, which may be catalyzed, for example, by acetolactate synthase;

b) acetolactate to 2,3-dihydroxyisovalerate, which may be catalyzed, for example, by ketol-acid reductoisomerase;

c) 2,3-dihydroxyisovalerate to α-ketoisovalerate, which may be catalyzed, for example, by dihydroxyacid dehydratase;

d) α-ketoisovalerate to valine, which may be catalyzed, for example, by transaminase or valine dehydrogenase;

e) valine to isobutylamine, which may be catalyzed, for example, by valine decarboxylase;

f) isobutylamine to isobutyraldehyde, which may be catalyzed by, for example, omega transaminase; and

g) isobutyraldehyde to isobutanol, which may be catalyzed, for example, by a branched-chain alcohol dehydrogenase.

In another embodiment, the isobutanol biosynthetic pathway comprises the following substrate to product conversions:

a) pyruvate to acetolactate, which may be catalyzed, for example, by acetolactate synthase;
b) acetolactate to 2,3-dihydroxyisovalerate, which may be catalyzed, for example, by acetohydroxy acid reductoisomerase;
c) 2,3-dihydroxyisovalerate to α-ketoisovalerate, which may be catalyzed, for example, by acetohydroxy acid dehydratase;
d) α-ketoisovalerate to isobutyryl-CoA, which may be catalyzed, for example, by branched-chain keto acid dehydrogenase;
e) isobutyryl-CoA to isobutyraldehyde, which may be catalyzed, for example, by acylating aldehyde dehydrogenase; and
f) isobutyraldehyde to isobutanol, which may be catalyzed, for example, by a branched-chain alcohol dehydrogenase.

Biosynthetic pathways for the production of 1-butanol that may be used include those described in U.S. Patent Application Publication No. 2008/0182308. In one embodiment, the 1-butanol biosynthetic pathway comprises the following substrate to product conversions:

a) acetyl-CoA to acetoacetyl-CoA, which may be catalyzed, for example, by acetyl-CoA acetyltransferase;

b) acetoacetyl-CoA to 3-hydroxybutyryl-CoA, which may be catalyzed, for example, by 3-hydroxybutyryl-CoA dehydrogenase;

c) 3-hydroxybutyryl-CoA to crotonyl-CoA, which may be catalyzed, for example, by crotonase;

d) crotonyl-CoA to butyryl-CoA, which may be catalyzed, for example, by butyryl-CoA dehydrogenase;

e) butyryl-CoA to butyraldehyde, which may be catalyzed, for example, by butyraldehyde dehydrogenase; and

f) butyraldehyde to 1-butanol, which may be catalyzed, for example, by butanol dehydrogenase.

Biosynthetic pathways for the production of 2-butanol that may be used include those described in U.S. Patent Application Publication No. 2007/0259410 and U.S. Patent Application Publication No. 2009/0155870. In one embodiment, the 2-butanol biosynthetic pathway comprises the following substrate to product conversions:

a) pyruvate to alpha-acetolactate, which may be catalyzed, for example, by acetolactate synthase;

b) alpha-acetolactate to acetoin, which may be catalyzed, for example, by acetolactate decarboxylase;

c) acetoin to 3-amino-2-butanol, which may be catalyzed, for example, acetonin aminase;

d) 3-amino-2-butanol to 3-amino-2-butanol phosphate, which may be catalyzed, for example, by aminobutanol kinase;

e) 3-amino-2-butanol phosphate to 2-butanone, which may be catalyzed, for example, by aminobutanol phosphate phosphorylase; and

f) 2-butanone to 2-butanol, which may be catalyzed, for example, by butanol dehydrogenase.

In another embodiment, the 2-butanol biosynthetic pathway comprises the following substrate to product conversions:

a) pyruvate to alpha-acetolactate, which may be catalyzed, for example, by acetolactate synthase;

b) alpha-acetolactate to acetoin, which may be catalyzed, for example, by acetolactate decarboxylase;

c) acetoin to 2,3-butanediol, which may be catalyzed, for example, by butanediol dehydrogenase;

d) 2,3-butanediol to 2-butanone, which may be catalyzed, for example, by dial dehydratase; and

e) 2-butanone to 2-butanol, which may be catalyzed, for example, by butanol dehydrogenase.

Biosynthetic pathways for the production of 2-butanone that may be used include those described in U.S. Patent Application Publication No. 2007/0259410 and U.S. Patent Application Publication No. 2009/0155870. In one embodiment, the 2-butanone biosynthetic pathway comprises the following substrate to product conversions:

a) pyruvate to alpha-acetolactate, which may be catalyzed, for example, by acetolactate synthase;

b) alpha-acetolactate to acetoin, which may be catalyzed, for example, by acetolactate decarboxylase;

c) acetoin to 3-amino-2-butanol, which may be catalyzed, for example, acetonin aminase;

d) 3-amino-2-butanol to 3-amino-2-butanol phosphate, which may be catalyzed, for example, by aminobutanol kinase; and

e) 3-amino-2-butanol phosphate to 2-butanone, which may be catalyzed, for example, by aminobutanol phosphate phosphorylase.

In another embodiment, the 2-butanone biosynthetic pathway comprises the following substrate to product conversions:

a) pyruvate to alpha-acetolactate, which may be catalyzed, for example, by acetolactate synthase;

b) alpha-acetolactate to acetoin which may be catalyzed, for example, by acetolactate decarboxylase;

c) acetoin to 2,3-butanediol, which may be catalyzed, for example, by butanediol dehydrogenase; and

d) 2,3-butanediol to 2-butanone, which may be catalyzed, for example, by diol dehydratase.

The terms "acetohydroxyacid synthase," "acetolactate synthase," and "acetolactate synthetase" (abbreviated "ALS") are used interchangeably herein to refer to a polypeptide (or polypeptides) having enzyme activity that catalyzes the conversion of pyruvate to acetolactate and $CO_2$. Example acetolactate synthases are known by the EC number 2.2.1.6 (Enzyme Nomenclature 1992, Academic Press, San Diego). These unmodified enzymes are available from a number of sources, including, but not limited to, *Bacillus subtilis* (GenBank Nos: CAB15618 (SEQ ID NO: 1), Z99122 (SEQ ID NO: 2), NCBI (National Center for Biotechnology Information) amino acid sequence, NCBI nucleotide sequence, respectively), *Klebsiella pneumoniae* (GenBank Nos: AAA25079 (SEQ ID NO: 3), M73842 (SEQ ID NO: 4)), and *Lactococcus lactis* (GenBank Nos: AAA25161 (SEQ ID NO: 5), L16975 (SEQ ID NO: 6)).

The term "ketol-acid reductoisomerase" ("KARI"), "acetohydroxy acid isomeroreductase," and "acetohydroxy acid reductoisomerase" will be used interchangeably and refer to a polypeptide (or polypeptides) having enzyme activity that catalyzes the reaction of (S)-acetolactate to 2,3-dihydroxyisovalerate. Example KARI enzymes may be classified as EC number EC 1.1.1.86 (Enzyme Nomenclature 1992, Academic Press, San Diego), and are available from a vast array of microorganisms, including, but not limited to, *Escherichia coli* (GenBank Nos: NP_418222 (SEQ ID NO: 7), NC_000913 (SEQ ID NO: 8)), *Saccharomyces cerevisiae* (GenBank Nos: NP_013459 (SEQ ID NO: 9), NC_001144 (SEQ ID NO: 10)), *Methanococcus maripaludis* (GenBank Nos: CAF30210 (SEQ ID NO: 11), BX957220 (SEQ ID NO: 12)), and *Bacillus subtilis* (GenBank Nos: CAB14789 (SEQ ID NO: 13), Z99118 (SEQ ID NO: 14)). KARIs include *Anaerostipes caccae* KARI variants "K9G9" and "K9D3" (SEQ ID Nos: 15 and 16, respectively). Ketol-acid reductoisomerase (KARI) enzymes are described in U.S. Patent Application Publication Nos. 2008/0261230, 2009/0163376, and 2010/0197519, and PCT Application Publication No. WO/2011/041415. Examples of KARIs disclosed therein are those from *Lactococcus lactis, Vibrio cholera, Pseudomonas aeruginosa* PAO1, and *Pseudomonas fluorescens* PF5 mutants In some embodiments, the KARI utilizes NADH. In some embodiments, the KARI utilizes reduced nicotinamide adenine dinucleotide phosphate (NADPH).

The term "acetohydroxy acid dehydratase" and "dihydroxyacid dehydratase" ("DHAD") refers to a polypeptide (or polypeptides) having enzyme activity that catalyzes the conversion the conversion of 2,3-dihydroxyisovalerate to α-ketoisovalerate. Example acetohydroxy acid dehydratases are known by the EC number 4.2.1.9. Such enzymes are available from a vast array of microorganisms, including, but not limited to, *Escherichia coli* (GenBank Nos: YP_026248 (SEQ ID NO: 17), NC000913 (SEQ ID NO: 18)), *Saccharomyces cerevisiae* (GenBank Nos: NP_012550 (SEQ ID NO: 19), NC 001142 (SEQ ID NO: 20), *M. maripaludis* (GenBank Nos: CAF29874 (SEQ ID NO: 21), BX957219 (SEQ ID NO: 22)), *B. subtilis* (GenBank Nos: CAB14105 (SEQ ID NO: 23), Z99115 (SEQ ID NO: 24)), *L. lactis,* and *N. crassa.* U.S. Patent Application Publication No. 2010/0081154, and U.S. Patent No. 7,851,188, describe dihydroxyacid dehydratases (DHADs), including a DHAD from *Streptococcus mutans.*

The term "branched-chain α-keto acid decarboxylase," "a-ketoacid decarboxylase," "a-ketoisovalerate decarboxylase," or "2-ketoisovalerate decarboxylase" ("KIVD") refers to a polypeptide (or polypeptides) having enzyme activity that catalyzes the conversion of α-ketoisovalerate to isobutyraldehyde and $CO_2$. Example branched-chain α-keto acid decarboxylases are known by the EC number 4.1.1.72 and are available from a number of sources, including, but not limited to, *Lactococcus lactis* (GenBank Nos: AAS49166 (SEQ ID NO: 25), AY548760 (SEQ ID NO: 26); CAG34226 (SEQ ID NO: 27), AJ746364 (SEQ ID NO: 28), *Salmonella typhimurium* (GenBank Nos: NP_461346 (SEQ ID NO: 29), NC_003197 (SEQ ID NO: 30)), *Clostridium acetobutylicum* (GenBank Nos: NP_149189 (SEQ ID NO: 31), NC_001988 (SEQ ID NO: 32)), *M. caseolyticus* (SEQ ID NO: 33), and *L. grayi* (SEQ ID NO: 34).

The term "branched-chain alcohol dehydrogenase" ("ADH") refers to a polypeptide (or polypeptides) having enzyme activity that catalyzes the conversion of isobutyraldehyde to isobutanol. Example branched-chain alcohol dehydrogenases are known by the EC number 1.1.1.265, but may also be classified under other alcohol dehydrogenases (specifically, EC 1.1.1.1 or 1.1.1.2). Alcohol dehydrogenases may be NADPH-dependent or NADH-dependent. Such enzymes are available from a number of sources, including, but not limited to, *Saccharomyces cerevisiae* (GenBank Nos: NP_010656 (SEQ ID NO: 35), NC_001136 (SEQ ID NO: 36), NP_014051 (SEQ ID NO: 37), NC_001145 (SEQ ID NO: 38)), *Escherichia coli* (GenBank Nos: NP_417484 (SEQ ID NO: 39), NC_000913 (SEQ ID NO: 40)), C. *acetobutylicum* (GenBank Nos: NP_349892 (SEQ ID NO: 41), NC_003030 (SEQ ID NO: 42); NP_349891 (SEQ ID NO: 43), NC_003030 (SEQ ID NO: 44)). U.S. Patent Application Publication No. 2009/0269823 describes SadB, an alcohol dehydrogenase (ADH) from *Achromobacter xylosoxidans.* Alcohol dehydrogenases also include horse liver ADH and *Beijerinkia indica* ADH (as described by U.S. Patent Application Publication No. 2011/0269199).

The term "butanol dehydrogenase" refers to a polypeptide (or polypeptides) having enzyme activity that catalyzes the conversion of isobutyraldehyde to isobutanol or the conversion of 2-butanone and 2-butanol. Butanol dehydrogenases are a subset of a broad family of alcohol dehydrogenases. Butanol dehydrogenase may be NAD- or NADP-dependent. The NAD-dependent enzymes are known as EC 1.1.1.1 and are available, for example, from *Rhodococcus ruber* (GenBank Nos: CAD36475, AJ491307). The NADP dependent enzymes are known as EC 1.1.1.2 and are available, for example, from *Pyrococcus furiosus* (GenBank Nos: AAC25556, AF013169). Additionally, a butanol dehydrogenase is available from *Escherichia coli* (GenBank Nos: NP 417484, NC_000913) and a cyclohexanol dehydrogenase is available from *Acinetobacter sp.* (GenBank Nos: AAG10026, AF282240). The term "butanol dehydrogenase" also refers to an enzyme that catalyzes the conversion of butyraldehyde to 1-butanol, using either NADH or NADPH as cofactor. Butanol dehydrogenases are available from, for example, C. acetobutylicum (GenBank Nos: NP_149325, NC_001988; this enzyme possesses both aldehyde and alcohol dehydrogenase activity); NP_349891, NC_003030; and NP_349892, NC_003030) and *Escherichia coli* (GenBank Nos: NP_417-484, NC_000913).

The term "branched-chain keto acid dehydrogenase" refers to a polypeptide (or polypeptides) having enzyme activity that catalyzes the conversion of α-ketoisovalerate to isobutyryl-CoA (isobutyryl-coenzyme A), typically using $NAD^+$ (nicotinamide adenine dinucleotide) as an electron acceptor. Example branched-chain keto acid dehydrogenases are known by the EC number 1.2.4.4. Such branched-chain keto acid dehydrogenases are comprised of four subunits and sequences from all subunits are available from a vast array of microorganisms, including, but not limited to, *Bacillus subtilis* (GenBank Nos: CAB14336 (SEQ ID NO: 45), Z99116 (SEQ ID NO: 46); CAB14335 (SEQ ID NO: 47), Z99116 (SEQ ID NO: 48); CAB14334 (SEQ ID NO: 49), Z99116 (SEQ ID NO: 50); and CAB14337 (SEQ ID NO: 51), Z99116 (SEQ ID NO: 52)) and *Pseudomonas putida* (GenBank Nos: AAA65614 (SEQ ID NO: 53), M57613 (SEQ ID NO: 54); AAA65615 (SEQ ID NO: 55), M57613 (SEQ ID NO: 56); AAA65617 (SEQ ID NO: 57), M57613 (SEQ ID NO: 58); and AAA65618 (SEQ ID NO: 59), M57613 (SEQ ID NO: 60)).

The term "acylating aldehyde dehydrogenase" refers to a polypeptide (or polypeptides) having enzyme activity that catalyzes the conversion of isobutyryl-CoA to isobutyraldehyde, typically using either NADH or NADPH as an electron donor. Example acylating aldehyde dehydrogenases are known by the EC numbers 1.2.1.10 and 1.2.1.57. Such enzymes are available from multiple sources, including, but not limited to, *Clostridium beijerinckii* (GenBank Nos: AAD31841 (SEQ ID NO: 61), AF157306 (SEQ ID NO: 62)), *Clostridium acetobutylicum* (GenBank Nos: NP_149325 (SEQ ID NO: 63), NC_001988 (SEQ ID NO: 64); NP_149199 (SEQ ID NO: 65), NC_001988 (SEQ ID NO: 66)), *Pseudomonas putida* (GenBank Nos: AAA89106 (SEQ ID NO: 67), U13232 (SEQ ID NO: 68)), and *Thermus thermophilus* (GenBank Nos: YP_145486 (SEQ ID NO: 69), NC_006461 (SEQ ID NO: 70)).

The term "transaminase" refers to a polypeptide (or polypeptides) having enzyme activity that catalyzes the conversion of α-ketoisovalerate to L-valine, using either alanine or glutamate as an amine donor. Example transaminases are known by the EC numbers 2.6.1.42 and 2.6.1.66. Such enzymes are available from a number of sources. Examples of sources for alanine-dependent enzymes include, but are not limited to, *Escherichia coli* (GenBank Nos: YP_026231 (SEQ ID NO: 71), NC_000913 (SEQ ID NO: 72)) and *Bacillus licheniformis* (GenBank Nos: YP_093743 (SEQ ID NO: 73), NC_006322 (SEQ ID NO: 74)). Examples of sources for glutamate-dependent enzymes include, but are not limited to, *Escherichia coli* (GenBank Nos: YP_026247 (SEQ ID NO: 75), NC_000913 (SEQ ID NO: 76)), *Saccharomyces cerevisiae* (GenBank Nos: NP_012682 (SEQ ID NO: 77), NC_001142 (SEQ ID NO: 78)) and *Methanobacterium thermoautotrophicum* (GenBank Nos: NP_276546 (SEQ ID NO: 79), NC_000916 (SEQ ID NO: 80)).

The term "valine dehydrogenase" refers to a polypeptide (or polypeptides) having enzyme activity that catalyzes the conversion of α-ketoisovalerate to L-valine, typically using NAD(P)H as an electron donor and ammonia as an amine donor. Example valine dehydrogenases are known by the EC numbers 1.4.1.8 and 1.4.1.9 and such enzymes are available from a number of sources, including, but not limited to, *Streptomyces coelicolor* (GenBank Nos: NP_628270 (SEQ ID NO: 81), NC_003888 (SEQ ID NO: 82)) and *Bacillus subtilis* (GenBank Nos: CAB14339 (SEQ ID NO: 83), Z99116 (SEQ ID NO: 84)).

The term "valine decarboxylase" refers to a polypeptide (or polypeptides) having enzyme activity that catalyzes the conversion of L-valine to isobutylamine and $CO_2$. Example valine decarboxylases are known by the EC number 4.1.1.14. Such enzymes are found in *Streptomyces,* such as for example, *Streptomyces viridifaciens* (GenBank Nos: AAN10242 (SEQ ID NO: 85), AY116644 (SEQ ID NO: 86)).

The term "omega transaminase" refers to a polypeptide (or polypeptides) having enzyme activity that catalyzes the conversion of isobutylamine to isobutyraldehyde using a suitable amino acid as an amine donor. Example omega transaminases are known by the EC number 2.6.1.18 and are available from a number of sources, including, but not limited to, *Alcaligenes denitrificans* (AAP92672 (SEQ ID NO: 87), AY330220 (SEQ ID NO: 88)), *Ralstonia eutropha* (GenBank Nos: YP_294474 (SEQ ID NO: 89), NC_007347 (SEQ ID NO: 90)), *Shewanella oneidensis* (GenBank Nos: NP_719046 (SEQ ID NO: 91), NC_004347 (SEQ ID NO: 92)), and *Pseudomonas putida* (GenBank Nos: AAN66223 (SEQ ID NO: 93), AE016776 (SEQ ID NO: 94)).

The term "acetyl-CoA acetyltransferase" refers to a polypeptide (or polypeptides) having enzyme activity that catalyzes the conversion of two molecules of acetyl-CoA to acetoacetyl-CoA and coenzyme A (CoA). Example acetyl-CoA acetyltransferases are acetyl-CoA acetyltransferases with substrate preferences (reaction in the forward direction) for a short chain acyl-CoA and acetyl-CoA and are classified as E.C. 2.3.1.9 [Enzyme Nomenclature 1992, Academic Press, San Diego]; although, enzymes with a broader substrate range (E.C. 2.3.1.16) will be functional as well. Acetyl-CoA acetyltransferases are available from a number of sources, for example, *Escherichia coli* (GenBank Nos: NP_416728, NC_000913; NCBI (National Center for Biotechnology Information) amino acid sequence, NCBI nucleotide sequence), *Clostridium acetobutylicum* (GenBank Nos: NP_349476.1, NC_003030; NP_149242, NC_001988, *Bacillus subtilis* (GenBank Nos: NP_390297, NC_000964), and *Saccharomyces cerevisiae* (GenBank Nos: NP_015297, NC_001148).

The term "3-hydroxybutyryl-CoA dehydrogenase" refers to a polypeptide (or polypeptides) having enzyme activity that catalyzes the conversion of acetoacetyl-CoA to 3-hydroxybutyryl-CoA. Example 3-hydroxybutyryl-CoA dehydrogenases may be NADH-dependent, with a substrate preference for (S)-3-hydroxybutyryl-CoA or (R)-3-hydroxybutyryl-CoA. Examples may be classified as E.C. 1.1.1.35 and E.C. 1.1.1.30, respectively. Additionally, 3-hydroxybutyryl-CoA dehydrogenases may be NADPH-dependent, with a substrate preference for (S)-3-hydroxybutyryl-CoA or (R)-3-hydroxybutyryl-CoA and are classified as E.C. 1.1.1.157 and E.C. 1.1.1.36, respectively. 3-Hydroxybutyryl-CoA dehydrogenases are available from a number of sources, for example, *Clostridium acetobutylicum* (GenBank Nos: NP_349314, NC_003030), *Bacillus subtilis* (GenBank Nos: AAB09614, U29084), *Ralstonia eutropha* (GenBank Nos: YP_294481, NC_007347), and *Alcaligenes eutrophus* (GenBank Nos: AAA21973, J04987).

The term "crotonase" refers to a polypeptide (or polypeptides) having enzyme activity that catalyzes the conversion of 3-hydroxybutyryl-CoA to crotonyl-CoA and $H_2O$. Example crotonases may have a substrate preference for (S)-3-hydroxybutyryl-CoA or (R)-3-hydroxybutyryl-CoA and may be classified as E.C. 4.2.1.17 and E.C. 4.2.1.55, respectively. Crotonases are available from a number of sources, for example, *Escherichia coli* (GenBank Nos: NP_415911, NC_000913), *Clostridium acetobutylicum* (GenBank Nos: NP_349318, NC_003030), *Bacillus subtilis* (GenBank Nos: CAB13705, Z99113), and *Aeromonas caviae* (GenBank Nos: BAA21816, D88825).

The term "butyryl-CoA dehydrogenase" refers to a polypeptide (or polypeptides) having enzyme activity that catalyzes the conversion of crotonyl-CoA to butyryl-CoA. Example butyryl-CoA dehydrogenases may be NADH-dependent, NADPH-dependent, or flavin-dependent and may be classified as E.C. 1.3.1.44, E.C. 1.3.1.38, and E.C. 1.3.99.2, respectively. Butyryl-CoA dehydrogenases are available from a number of sources, for example, *Clostridium acetobutylicum* (GenBank Nos: NP_347102, NC_003030), *Euglena gracilis* (GenBank Nos: Q5EU90, AY741582), *Streptomyces collinus* (GenBank Nos: AAA92890, U37135), and *Streptomyces coelicolor* (GenBank Nos: CAA22721, AL939127).

The term "butyraldehyde dehydrogenase" refers to a polypeptide (or polypeptides) having enzyme activity that catalyzes the conversion of butyryl-CoA to butyraldehyde, using NADH or NADPH as cofactor. Butyraldehyde dehydrogenases with a preference for NADH are known as E.C. 1.2.1.57 and are available from, for example, *Clostridium beijerinckii* (GenBank Nos: AAD31841, AF157306) and *Clostridium acetobutylicum* (GenBank Nos: NP.sub.--149325, NC.sub.--001988).

The term "isobutyryl-CoA mutase" refers to a polypeptide (or polypeptides) having enzyme activity that catalyzes the conversion of butyryl-CoA to isobutyryl-CoA. This enzyme uses coenzyme $B_{12}$ as cofactor. Example isobutyryl-CoA mutases are known by the EC number 5.4.99.13. These enzymes are found in a number of *Streptomyces,* including, but not limited to, *Streptomyces cinnamonensis* (GenBank Nos: AAC08713 (SEQ ID NO: 95), U67612 (SEQ ID NO: 96); CAB59633 (SEQ ID NO: 97), AJ246005 (SEQ ID NO: 98)), *Streptomyces coelicolor* (GenBank Nos: CAB70645 (SEQ ID NO: 99), AL939123 (SEQ ID NO: 100); CAB92663 (SEQ ID NO: 101), AL939121 (SEQ ID NO: 102)), and *Streptomyces avermitilis* (GenBank Nos: NP_824008 (SEQ ID NO: 103), NC_003155 (SEQ ID NO: 104); NP_824637 (SEQ ID NO: 105), NC_003155 (SEQ ID NO: 106)).

The term "acetolactate decarboxylase" refers to a polypeptide (or polypeptides) having enzyme activity that catalyzes the conversion of alpha-acetolactate to acetoin. Example acetolactate decarboxylases are known as EC 4.1.1.5 and are available, for example, from *Bacillus subtilis* (GenBank Nos: AAA22223, L04470), *Klebsiella terrigena* (GenBank Nos: AAA25054, L04507) and *Klebsiella pneumoniae* (GenBank Nos: AAU43774, AY722056).

The term "acetoin aminase" or "acetoin transaminase" refers to a polypeptide (or polypeptides) having enzyme activity that catalyzes the conversion of acetoin to 3-amino-2-butanol. Acetoin aminase may utilize the cofactor pyridoxal 5'-phosphate or NADH or NADPH. The resulting product may have (R) or (S) stereochemistry at the 3-position. The pyridoxal phosphate-dependent enzyme may use an amino acid such as alanine or glutamate as the amino donor. The NADH- and NADPH-dependent enzymes may use ammonia as a second substrate. A suitable example of an NADH-dependent acetoin aminase, also known as amino alcohol dehydrogenase, is described by Ito, et al. (U.S. Patent No. 6,432,688). An example of a pyridoxal-dependent acetoin aminase is the amine:pyruvate aminotransferase (also called amine:pyruvate transaminase) described by Shin and Kim (J. Org. Chem. 67:2848-2853, 2002).

The term "acetoin kinase" refers to a polypeptide (or polypeptides) having enzyme activity that catalyzes the conversion of acetoin to phosphoacetoin. Acetoin kinase may utilize ATP (adenosine triphosphate) or phosphoenolpyruvate as the phosphate donor in the reaction. Enzymes that catalyze the analogous reaction on the similar substrate dihydroxyacetone, for example, include enzymes known as EC 2.7.1.29 (Garcia-Alles, et al., Biochemistry 43:13037-13046, 2004).

The term "acetoin phosphate aminase" refers to a polypeptide (or polypeptides) having enzyme activity that catalyzes the conversion of phosphoacetoin to 3-amino-2-butanol O-phosphate. Acetoin phosphate aminase may use the cofactor pyridoxal 5'-phosphate, NADH, or NADPH. The resulting product may have (R) or (S) stereochemistry at the 3-position. The pyridoxal phosphate-dependent enzyme may use an amino acid such as alanine or glutamate. The NADH-dependent and NADPH-dependent enzymes may use ammonia as a second substrate. Although there are no reports of enzymes catalyzing this reaction on phosphoacetoin, there is a pyridoxal phosphate-dependent enzyme that is proposed to carry out the analogous reaction on the similar substrate serinol phosphate (Yasuta, et al., Appl. Environ. Microbial. 67:4999-5009, 2001).

The term "aminobutanol phosphate phospholyase," also called "amino alcohol O-phosphate lyase," refers to a polypeptide (or polypeptides) having enzyme activity that catalyzes the conversion of 3-amino-2-butanol O-phosphate to 2-butanone. Amino butanol phosphate phospho-lyase may utilize the cofactor pyridoxal 5'-phosphate. There are reports of enzymes that catalyze the analogous reaction on the similar substrate 1-amino-2-propanol phosphate (Jones, et al., Biochem J. 134:167-182, 1973). U.S. Patent Application Publication No. 2007/0259410 describes an aminobutanol phosphate phospho-lyase from the organism *Erwinia carotovora.*

The term "aminobutanol kinase" refers to a polypeptide (or polypeptides) having enzyme activity that catalyzes the conversion of 3-amino-2-butanol to 3-amino-2-butanol O-phosphate. Amino butanol kinase may utilize ATP as the phosphate donor. Although there are no reports of enzymes catalyzing this reaction on 3-amino-2-butanol, there are reports of enzymes that catalyze the analogous reaction on the similar substrates ethanolamine and 1-amino-2-propanol (Jones, et al., supra). U.S. Patent Application Publication No. 2009/0155870 describes, in Example 14, an amino alcohol kinase of *Erwinia carotovora subsp. Atroseptica.*

The term "butanediol dehydrogenase" also known as "acetoin reductase" refers to a polypeptide (or polypeptides) having enzyme activity that catalyzes the conversion of acetoin to 2,3-butanediol. Butanedial dehydrogenases are a subset of the broad family of alcohol dehydrogenases. Butanediol dehydrogenase enzymes may have specificity for production of (R)- or (S)-stereochemistry in the alcohol product. (S)-specific butanediol dehydrogenases are known as EC 1.1.1.76 and are available, for example, from *Klebsiella pneumoniae* (GenBank Nos: BBA13085, D86412). (R)-specific butanediol dehydrogenases are known as EC 1.1.1.4 and are available, for example, from *Bacillus cereus* (GenBank Nos. NP 830481, NC_004722; AAP07682, AE017000), and *Lactococcus lactis* (GenBank Nos. AAK04995, AE006323).

The term "butanediol dehydratase," also known as "dial dehydratase" or "propanediol dehydratase" refers to a polypeptide (or polypeptides) having enzyme activity that catalyzes the conversion of 2,3-butanediol to 2-butanone. Butanediol dehydratase may utilize the cofactor adenosyl cobalamin (also known as coenzyme Bw or vitamin B12;

although vitamin B12 may refer also to other forms of cobalamin that are not coenzyme B12). Adenosyl cobalamin-dependent enzymes are known as EC 4.2.1.28 and are available, for example, from *Klebsiella oxytoca* (GenBank Nos: AA08099 (alpha subunit), D45071; BAA08100 (beta subunit), D45071; and BBA08101 (gamma subunit), D45071 (Note all three subunits are required for activity), and *Klebsiella pneumonia* (GenBank Nos: AAC98384 (alpha subunit), AF102064; GenBank Nos: AAC98385 (beta subunit), AF102064, GenBank Nos: AAC98386 (gamma subunit), AF102064). Other suitable dial dehydratases include, but are not limited to, B12-dependent dial dehydratases available from *Salmonella typhimurium* (GenBank Nos: AAB84102 (large subunit), AF026270; GenBank Nos: AAB84103 (medium subunit), AF026270; GenBank Nos: AAB84104 (small subunit), AF026270); and *Lactobacillus collinoides* (GenBank Nos: CAC82541 (large subunit), AJ297723 ; GenBank Nos: CAC82542 (medium subunit); AJ297723; GenBank Nos: CAD01091 (small subunit), AJ297723); and enzymes from *Lactobacillus brevis* (particularly strains CNRZ 734 and CNRZ 735, Speranza, et al., J. Agric. Food Chem. 45:3476-3480, 1997), and nucleotide sequences that encode the corresponding enzymes. Methods of dial dehydratase gene isolation are well known in the art (e.g., U.S. Patent No. 5,686,276).

The term "pyruvate decarboxylase" refers to a polypeptide (or polypeptides) having enzyme activity that catalyzes the decarboxylation of pyruvic acid to acetaldehyde and carbon dioxide. Pyruvate dehydrogenases are known by the EC number 4.1.1.1. These enzymes are found in a number of yeast, including *Saccharomyces cerevisiae* (GenBank Nos: CAA97575 (SEQ ID NO: 107), CAA97705 (SEQ ID NO: 109), CAA97091 (SEQ ID NO: 111)).

It will be appreciated that microorganisms comprising an isobutanol biosynthetic pathway as provided herein may further comprise one or more additional modifications. U.S. Patent Application Publication No. 2009/0305363 discloses increased conversion of pyruvate to acetolactate by engineering yeast for expression of a cytosol-localized acetolactate synthase and substantial elimination of pyruvate decarboxylase activity. In some embodiments, the microorganisms may comprise modifications to reduce glycerol-3-phosphate dehydrogenase activity and/or disruption in at least one gene encoding a polypeptide having pyruvate decarboxylase activity or a disruption in at least one gene encoding a regulatory element controlling pyruvate decarboxylase gene expression as described in U.S. Patent Application Publication No. 2009/0305363, and/or modifications that provide for increased carbon flux through an Entner-Doudoroff Pathway or reducing equivalents balance as described in U.S. Patent Application Publication No. 2010/0120105. Other modifications include integration of at least one polynucleotide encoding a polypeptide that catalyzes a step in a pyruvate-utilizing biosynthetic pathway. Other modifications include at least one deletion, mutation, and/or substitution in an endogenous polynucleotide encoding a polypeptide having acetolactate reductase activity. In some embodiments, the polypeptide having acetolactate reductase activity is YMR226C (SEQ ID NOs: 127, 128) of *Saccharomyces cerevisiae* or a homolog thereof. Additional modifications include a deletion, mutation, and/or substitution in an endogenous polynucleotide encoding a polypeptide having aldehyde dehydrogenase and/or aldehyde oxidase activity. In some embodiments, the polypeptide having aldehyde dehydrogenase activity is *ALD6* from *Saccharomyces cerevisiae* or a homolog thereof. A genetic modification which has the effect of reducing glucose repression wherein the yeast production host cell is pdc- is described in U.S. Patent Application Publication No. 2011/0124060. In some embodiments, the pyruvate decarboxylase that is deleted or downregulated is selected from the group consisting of: *PDC1, PDC5, PDC6,* and combinations thereof. In some embodiments, the pyruvate decarboxylase is selected from those enzymes in Table 1. In some embodiments, microorganisms may contain a deletion or down-regulation of a polynucleotide encoding a polypeptide that catalyzes the conversion of glyceraldehyde-3-phosphate to glycerate 1,3, bisphosphate. In some embodiments, the enzyme that catalyzes this reaction is glyceraldehyde-3-phosphate dehydrogenase.

Table 1. SEQ ID Numbers of PDC Target Gene coding regions and Proteins

| Description | SEQ ID NO: (Amino Acid) | SEQ ID NO: (Nucleic Acid) |
|---|---|---|
| PDC1 pyruvate decarboxylase from *Saccharomyces cerevisiae* | 107 | 108 |
| PDC5 pyruvate decarboxylase from *Saccharomyces cerevisiae* | 109 | 110 |
| PDC6 pyruvate decarboxylase *Saccharomyces cerevisiae* | 111 | 112 |
| pyruvate decarboxylase from *Candida glabrata* | 113 | 114 |
| PDC1 pyruvate decarboxylase from *Pichia stipitis* | 115 | 116 |
| PDC2 pyruvate decarboxylase from *Pichia stipitis* | 117 | 118 |
| pyruvate decarboxylase from *Kluyveromyces lactis* | 119 | 120 |
| pyruvate decarboxylase from *Yarrowia lipolytica* | 121 | 122 |

(continued)

| Description | SEQ ID NO: (Amino Acid) | SEQ ID NO: (Nucleic Acid) |
|---|---|---|
| pyruvate decarboxylase from *Schizosaccharomyces pombe* | 123 | 124 |
| pyruvate decarboxylase from *Zygosaccharomyces rouxii* | 125 | 126 |

In some embodiments, any particular nucleic acid molecule or polypeptide may be at least 80%, 85%, 90%, 95%, 96%, 97%, 98%, or 99% identical to a nucleotide sequence or polypeptide sequence described herein. The term "percent identity" as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. Identity and similarity can be readily calculated by known methods, including but not limited to those disclosed in: Computational Molecular Biology (Lesk, A. M., Ed.) Oxford University: NY (1988); Biocomputing: Informatics and Genome Projects (Smith, D. W., Ed.) Academic: NY (1993); Computer Analysis of Sequence Data, Part I (Griffin, A. M., and Griffin, H. G., Eds.) Humania: NJ (1994); Sequence Analysis in Molecular Biology (von Heinje, G., Ed.) Academic (1987); and Sequence Analysis Primer (Gribskov, M. and Devereux, J., Eds.) Stockton: NY (1991).

Methods to determine identity are designed to give the best match between the sequences tested. Methods to determine identity and similarity are codified in publicly available computer programs. Sequence alignments and percent identity calculations may be performed using the MegAlign™ program of the LASERGENE bioinformatics computing suite (DNASTAR Inc., Madison, WI). Multiple alignment of the sequences may be performed using the Clustal method of alignment which encompasses several varieties of the algorithm including the Clustal V method of alignment corresponding to the alignment method labeled Clustal V (disclosed by Higgins and Sharp, CABIOS. 5:151-153, 1989; Higgins, et al., Comput. Appl. Biosci. 8:189-191, 1992) and found in the MegAlign™ program of the LASERGENE bioinformatics computing suite (DNASTAR Inc.). For multiple alignments, the default values correspond to GAP PENALTY=10 and GAP LENGTH PENALTY=10. Default parameters for pairwise alignments and calculation of percent identity of protein sequences using the Clustal method are KTUPLE=1, GAP PENALTY=3, WINDOW=5 and DIAGONALS SAVED=5. For nucleic acids these parameters are KTUPLE=2, GAP PENALTY=5, WINDOW=4 and DIAGONALS SAVED=4. After alignment of the sequences using the Clustal V program, it is possible to obtain a percent identity by viewing the sequence distances table in the same program. Additionally the Clustal W method of alignment is available and corresponds to the alignment method labeled Clustal W (described by Higgins and Sharp, CABIOS. 5:151-153, 1989; Higgins, et al., Comput. Appl. Biosci. 8:189-191, 1992) and found in the MegAlign™ v6.1 program of the LASERGENE bioinformatics computing suite (DNASTAR Inc.). Default parameters for multiple alignment (GAP PENALTY=10, GAP LENGTH PENALTY=0.2, Delay Divergen Seqs(%)=30, DNA Transition Weight=0.5, Protein Weight Matrix=Gonnet Series, DNA Weight Matrix=IUB). After alignment of the sequences using the Clustal W program, it is possible to obtain a percent identity by viewing the sequence distances table in the same program.
Standard recombinant DNA and molecular cloning techniques are well known in the art and are described by Sambrook, et al. (Sambrook, J., Fritsch, E. F. and Maniatis, T. (Molecular Cloning: A Laboratory Manual; Cold Spring Harbor Laboratory Press, Cold Spring Harbor, 1989) and by Ausubel, et al. (Ausubel, et al., Current Protocols in Molecular Biology, pub. by Greene Publishing Assoc. and Wiley-Interscience, 1987). Examples of methods to construct microorganisms that comprise a butanol biosynthetic pathway are disclosed, for example, in U.S. Patent No. 7,851,188, and U.S. Patent Application Publication Nos. 2007/0092957; 2007/0259410; 2007/0292927; 2008/0182308; 2008/0274525; 2009/0155870; 2009/0305363; and 2009/0305370.

Further, while various embodiments of the present invention have been described herein, it should be understood that they have been presented by way of example only, and not limitation. Thus, the breadth and scope of the present invention should not be limited by any of the above-described exemplary embodiments, but should be defined only in accordance with the claims.

All publications, patents, and patent applications mentioned in this specification are indicative of the level of skill of those skilled in the art to which this invention pertains.

## EXAMPLES

The following nonlimiting examples will further illustrate the invention. Test C of Example 20 demonstrates a separation according to the claimed method. It should be understood that, while the following examples involve corn as feedstock, other biomass sources can be used for feedstock without departing from the present invention.

As used herein, the meaning of abbreviations used was as follows: "g" means gram(s), "kg" means kilogram(s), "lbm" means pound mass, "gpm" means gallons per minute, "gal" means gallon(s), "MMGPY" means million gallon per year, "L" means liter(s), "mL" means milliliter(s), "$\mu$L" means microliter(s), "mL/L" means milliliter(s) per liter, "mL/min" means milliliter(s) per min, "min" means minute(s), "hr" means hour(s), "DI" means deionized, "uM" means micrometer(s), "nm" means nanometer(s), "w/v" means weight/volume, "wt%" means weight percent, "OD" means optical density, "$OD_{600}$" means optical density at a wavelength of 600 nM, "dcw" means dry cell weight, "rpm" means revolutions per minute, "°C" means degree(s) Celsius, "°C/min" means degrees Celsius per minute, "slpm" means standard liter(s) per minute, "ppm" means part per million, "cP" means centipoise, "ID" means inner diameter, and "GC" means gas chromatograph.

## Example 1

Effect of Undissolved Solids on the Rate of Mass Transfer

The following experiment was performed to measure the effect of undissolved solids on the rate of mass transfer of *i*-BuOH from an aqueous phase that simulates the composition of a fermentation broth derived from corn mash, which is approximately half way through a simultaneous saccharification and fermentation (SSF) fermentation (i.e., about 50% conversion of the oligosaccharides) in order to mimic the average composition of the liquid phase for an SSF batch.

Approximately 100 kg of liquefied corn mash was prepared in three equivalent batches using a 30 L glass, jacketed resin kettle. The kettle was set up with mechanical agitation, temperature control, and pH control. The protocol used for the three batches was as follows: (a) mixing ground corn with tap water (30 wt% corn on a dry basis), (b) heating the slurry to 55°C while agitating, (c) adjusting pH of the slurry to 5.8 with either NaOH or $H_2SO_4$, (d) adding alpha-amylase (0.02 wt% on a dry corn basis), (e) heating the slurry to 85°C, (f) adjusting pH to 5.8, (g) holding the slurry at 85°C for 2 hr while maintaining pH at 5.8, and (h) cooling the slurry to 25°C.

Pioneer 3335 hybrid corn, whole kernel yellow corn, was used (Pioneer Hi-Bred International, Johnston, IA), and it was ground in a hammer-mill using a 1 mm screen. The moisture content of the ground corn was 12 wt%, and the starch content of the ground corn was 71.4 wt% on a dry corn basis. The alpha-amylase enzyme was Liquozyme® SC DS (Novozymes, Franklinton, NC). The total amounts of the ingredients used for the three batches combined were: 33.9 kg ground corn (12% moisture), 65.4 kg tap water, and 0.006 kg Liquozyme® SC DS. A total of 0.297 kg of NaOH (17 wt%) was added to control pH. No $H_2SO_4$ was required. The total amount of liquefied corn mash recovered from the three 30 L batches was 99.4 kg.

Solids were removed from the mash by centrifugation in a large floor centrifuge which contained six 1 L bottles. Mash (73.4 kg) was centrifuged at 8000 rpm for 20 min at 25°C yielding 44.4 kg of centrate and 26.9 kg of wet cake. It was determined that the centrate contained <1 wt% suspended solids, and that the wet cake contained approximately 18 wt% suspended solids. This implies that the original liquefied mash contained approximately 7 wt% suspended solids. This is consistent with the corn loading and starch content of the corn used assuming most of the starch was liquefied. If all of the starch was liquefied, the 44.4 kg of centrate recovered directly from the centrifuge would have contained approximately 23 wt% dissolved oligosaccharides (liquefied starch). About 0.6 kg of *i*-BuOH was added to 35.4 kg of centrate to preserve it. The resulting 36.0 kg of centrate, which contained 1.6 wt% *i*-BuOH, was used as a stock solution. The centrate mimics the liquid phase composition at the beginning of SSF. Therefore, a portion of it was diluted with an equal amount of $H_2O$ on a mass basis to generate centrate that mimics SSF at about 50% conversion. More i-BuOH was added to bring the final concentration of i-BuOH in the diluted centrate to 3.0 wt% (about 30 g/L).

The diluted centrate was prepared as follows: 18 kg of the centrate stock solution which contained 1.6 wt% *i*-BuOH, was mixed with 18 kg tap water and 0.82 kg i-BuOH was added. The resulting 36.8 kg solution of diluted centrate consisted of approximately 11 wt% oligosaccharides and approximately 30 g/L *i*-BuOH. This solution mimics the liquid phase of a corn mash fermentation (SSF) at approximately 50% conversion of the oligosaccharides and an aqueous titer of 30 g/L *i*-BuOH.

Mass transfer tests were conducted using this solution as the aqueous phase to mimic mass transfer performance in a broth derived from liquefied corn mash after most of the undissolved solids are removed. The objective of the mass transfer tests was to measure the effect of undissolved solids on the overall volumetric mass transfer coefficient ($k_La$) for the transfer of *i*-BuOH from a simulated broth, derived from liquefied corn mash, to a dispersion of solvent (extractant) droplets rising through the simulated broth. Correlations of $k_La$ with key design of operating parameters can be used to scale up mass transfer operations. Examples of parameters that should be held constant as much as possible in order to generate correlations of $k_La$ from smaller scale data which are useful for scale up are the physical properties of both phases and design parameters that determine droplet size (e.g., nozzle diameter, velocity of the phase to be dispersed through the nozzle).

A 15.24 cm (6-inch) diameter, 213.36 cm (7-foot) tall glass, jacketed column was used to measure the $k_La$ for the transfer of i-BuOH from an aqueous solution of oligosaccharides (derived from liquefied corn mash), both with and without suspended mash solids, to a dispersion of oleyl alcohol (OA) droplets rising through the simulated broth. *i*-BuOH was added to the aqueous phase to give an initial concentration of i-BuOH of approximately 30 g/L. A certain amount of the aqueous phase (typically about 35 kg) which contained approximately 11 wt% oligosaccharides and approximately 30 g/L *i*-BuOH, was charged to the column, and the column was heated to 30°C by flowing warm $H_2O$ through the jacket. There was no flow of aqueous phase in or out of the column during the test.

Fresh oleyl alcohol (80/85% grade, Cognis Corporation, Cincinnati, OH) was sparged into the bottom of the column through a single nozzle to create a dispersion of extractant droplets which flowed up through the aqueous phase. After reaching the top of the aqueous phase, the extractant drops formed a separate organic phase which then overflowed from the top of the column and was collected into a receiver. Typically, 11.36 to 18.92 L (3 to 5 gallons) of oleyl alcohol flowed through the column for a single test.

Samples of the aqueous phase were collected from the column at several times throughout the test, and a composite sample of the total "rich" oleyl alcohol collected from the overflow was collected at the end of the test. All samples were analyzed for *i*-BuOH using a HP-6890 GC (Agilent Technologies, Inc., Santa Clara, CA). The concentration profile for i-BuOH in the aqueous phase (i.e., *i*-BuOH concentration versus time) was used to calculate the $k_La$ at the given set of operating conditions. The final composite sample of the total "rich" oleyl alcohol collected during the test was used to check the mass balance for *i*-BuOH.

The nozzle size and nozzle velocity (average velocity of oleyl alcohol through the feed nozzle) were varied to observe the effects on the $k_La$. A series of tests were done using an aqueous solution of oligosaccharides (diluted centrate obtained from liquefied corn mash) with the mash solids removed. A similar series of tests were done using the same aqueous solution of oligosaccharides after adding the mash solids back to simulate liquefied corn mash (including the undissolved solids) at the middle of SSF. It is noted that under some operating conditions (e.g., higher oleyl alcohol flow rates), poor phase separation was obtained at the top of the column which made it difficult to obtain a representative composite sample of the total "rich" oleyl alcohol collected during the test. It is also noted that under some operating conditions, samples of the aqueous phase contained a significant amount of organic phase. Special sample handling and preparation techniques were employed to obtain a sample of the aqueous phase that was as representative as possible of the aqueous phase in the column at the time the sample was collected.

It was determined that the aqueous phase in the column was "well mixed" for all practical purposes because the concentration of *i*-BuOH did not vary much along the length of the column at a given point in time. Assuming the solvent droplet phase is also well mixed, the overall mass transfer of *i*-BuOH from the aqueous phase to the solvent phase in the column can be approximated by the following equation:

$$r_B = k_L a\left(C_{B,broth} - \frac{C_{B,solvent}}{K_B}\right) \qquad (1)$$

where,

$r_B$ = total mass of *i*-BuOH transferred from the aqueous phase to the solvent phase per unit time per unit volume of the aqueous phase, grams *i*-BuOH/liter aqueous phase/hr or g/L/hr.

$k_La$ = overall volumetric mass transfer coefficient describing the mass transfer of i-BuOH from the aqueous phase to the solvent phase, $hr^{-1}$.

$C_{B,broth}$ = average concentration of *i*-BuOH in the simulated broth (aqueous) phase over the entire test, grams *i*-BuOH/Liter aqueous phase or g/L.

$C_{B,solvent}$ = average concentration of i-BuOH in the solvent phase over the entire test, grams *i*-BuOH/Liter solvent phase or g/L.

$K_B$ = average equilibrium distribution coefficient for *i*-BuOH between the solvent and aqueous phase, (grams *i*-BuOH/Liter solvent phase)/(grams *i*-BuOH/Liter aqueous phase).

The parameters $r_B$, $C_{B,broth}$, and $C_{B,solvent}$ were calculated for each test from the concentration data obtained from the samples of the aqueous and solvent phases. The parameter $K_B$ was independently measured by mixing aqueous centrate from liquefied corn mash, oleyl alcohol, and i-BuOH and vigorously mixing the system until the two liquid phases were at equilibrium. The concentration of i-BuOH was measured in both phases to determine $K_B$. After $r_B$, $C_{B,broth}$, $C_{B,solvent}$, and $K_B$ were determined for a given test, the $k_La$ could be calculated by rearranging Equation (1):

$$k_L a = \frac{r_B}{\left(C_{B,broth} - \frac{C_{B,solvent}}{K_B}\right)} \quad (2)$$

Mass transfer tests were conducted with two different size nozzles at nozzle velocities ranging from 1.524 m/s (5 ft/s) to 6.401 m/s (21 ft/s) using the diluted centrate (solids removed) as the aqueous phase. Three tests were done using a nozzle that has an ID of 0.76 mm, and three tests were done using a nozzle that has an ID of 2.03 mm. All tests were conducted at 30°C in the 15.24 cm (6-inch) diameter column described above using oleyl alcohol as the solvent. The equilibrium distribution coefficient for *i*-BuOH between oleyl alcohol and the diluted centrate which was obtained from liquefied corn mash by removing the solids, was measured to be approximately 5. The results of the mass transfer tests using diluted centrate (with the solids removed) are shown in Table 2.

Table 2

| **MASS TRANSFER TEST CONDITIONS:** | 41 | 42 | 43 | 44 | 45 | 46 |
|---|---|---|---|---|---|---|
| Aqueous Phase | **Diluted Centrate from Liq'd Mash, Solids Removed** | **Diluted Centrate from Liq'd Mash, Solids Removed** | **Diluted Centrate from Liq'd Mash, Solids Removed** | **Diluted Centrate from Liq'd Mash, Solids Removed** | **Diluted Centrate from Liq'd Mash, Solids Removed** | **Diluted Centrate from Liq'd Mash, Solids Removed** |
| Volume of Aqueous Phase, L: | 36.0 | 35.0 | 34.3 | 32.0 | 28.0 | 28.6 |
| Solvent Feed Rate, g/min: | 33.2 | 79.5 | 145.3 | 237.7 | 507.7 | 875 |
| Superficial Liq. Velocity (Us), ft/hr: | 0.42 | 1.01 | 1.84 | 3.02 | 6.45 | 11.11 |
| Nozzle I.D., mm: | 0.76 | 0.76 | 0.76 | 2.03 | 2.03 | 2.03 |
| Nozzle Velocity, ft/s | 4.7 | 11.3 | 20.6 | 4.7 | 10.1 | 17.4 |
| **MASS TRANSFER RESULTS:** | | | | | | |
| Initial [i-B] in Aq. Phase, g/L: | 28.2 | 27.0 | 29.1 | 31.3 | 38.7 | 30.1 |
| Final [i-B] in Aq. Phase, g/L: | 25.7 | 14.8 | 14.7 | 24.8 | 11.5 | 5.4 |
| Rich OA collected, kg: | 4.05 | 7.47 | 6.03 | 7.37 | 12.82 | 14.0 |
| [i-B] in OA collected, wt%: | 2.22 | 5.72 | 8.17 | 2.83 | 5.93 | 5.04 |
| Test time, min: | 122 | 94 | 41.5 | 31.0 | 25.3 | 16.0 |

(continued)

| | | | | | | |
|---|---|---|---|---|---|---|
| **MASS TRANSFER RESULTS:** | | | | | | |
| Overall i-BuOH M.T. Rate, g/L/hr | 1.23 | 7.81 | 20.76 | 12.62 | 64.52 | 92.52 |
| kLa, hr^(-1) | 0.05 | 0.70 | 2.58 | 0.54 | 4.29 | 10.06 |
| (kLa/Us) | 0.12 | 0.69 | 1.40 | 0.18 | 0.67 | 0.91 |

An aqueous phase that simulates a fermentation broth from liquefied corn mash (containing undissolved solids) half way through SSF was synthesized by adding some of the wet cake (which was initially obtained by removing the solids from liquefied corn mash) to diluted centrate. Some water was also added to dilute the liquid phase held up in the wet cake because this liquid has the same composition as the concentrated centrate. Diluted supernate (17.8 kg), 13.0 kg wet cake (contains ~18 wt% undissolved mash solids), 5.0 kg $H_2O$, and 0.83 kg *i*-BuOH were mixed together yielding 36.6 kg of a slurry containing approximately 6.3 wt% undissolved solids and a liquid phase consisting of approximately 13 wt% liquefied starch and approximately 2.4 wt% *i*-BuOH (balance $H_2O$). This slurry mimics the composition of a fermentation broth half way through SSF of corn to i-BuOH at approximately 30% corn loadings because the level of undissolved solids and oligosaccharides found in these types of broths is approximately 6-8 wt% and 10-12 wt%, respectively.

Mass transfer tests were conducted with two different size nozzles at nozzle velocities ranging from 1.524 m/s (5 ft/s) to 6.706 m/s (22 ft/s) using the slurry of diluted centrate and undissolved mash solids as the aqueous phase. Three tests were done using a nozzle that has an ID of 0.76 mm, and three tests were done using a nozzle that has an ID of 2.03 mm. All tests were conducted at 30°C in the 15.24 cm (6-inch) diameter column described above using oleyl alcohol as the solvent. The results of the mass transfer tests using the slurry of diluted centrate and undissolved mash solids are shown in Table 3.

Table 3

| | 52 | 53 | 54 | 49 | 50 | 51 |
|---|---|---|---|---|---|---|
| **MASS TRANSFER TEST CONDITIONS:** | | | | | | |
| Aqueous Phase | **Diluted Centrate from Liq'd Mash, +6.3 wt% Solids** | **Diluted Centrate from Liq'd Mash, +6.3 wt% Solids** | **Diluted Centrate from Liq'd Mash, +6.3 wt% Solids** | **Diluted Centrate from Liq'd Mash, +6.3 wt% Solids** | **Diluted Centrate from Liq'd Mash, +6.3 wt% Solids** | **Diluted Centrate from Liq'd Mash, +6.3 wt% Solids** |
| Volume of Aqueous Phase, L: | 35.5 | 35.5 | 32.5 | 31.5 | 30 | 31.6 |
| Solvent Feed Rate, g/min: | 40 | 64 | 157 | 249 | 549 | 853 |
| Superficial Liq. Velocity (Us), ft/hr: | 0.51 | 0.81 | 1.99 | 3.16 | 6.97 | 10.83 |
| Nozzle I.D., mm: | 0.76 | 0.76 | 0.76 | 2.03 | 2.03 | 2.03 |
| Nozzle Velocity, ft/s | 5.7 | 9.1 | 22.3 | 4.9 | 10.9 | 17.0 |

(continued)

| | | | | | | |
|---|---|---|---|---|---|---|
| **MASS TRANSFER RESULTS:** | | | | | | |
| Initial [i-B] in Aq. Phase, g/L: | 28.1 | 26.0 | 26.2 | 27.6 | 26.3 | 36.8 |
| Final [i-B] in Aq. Phase, g/L: | 26.3 | 23.8 | 14.0 | 24.6 | 13.8 | 16.1 |
| Rich OA collected, kg: | 6.02 | 5.75 | 10.23 | 15.05 | 16.58 | 13.22 |
| [i-B] in OA collected, wt%: | 1.05 | 1.35 | 3.86 | 0.68 | 2.30 | 5.00 |
| Test time, min: | 150 | 90 | 65 | 60 | 30 | 15.5 |
| Overall i-BuOH M.T. Rate, g/L/hr | 0.71 | 1.46 | 11.2 | 3.0 | 25.0 | 80.0 |
| kLa, hr^(-1) | 0.03 | 0.06 | 0.83 | 0.12 | 1.55 | 4.45 |
| (kLa/Us) | 0.06 | 0.07 | 0.42 | 0.04 | 0.22 | 0.41 |

Figure 12 illustrates the effect of the presence of undissolved corn mash solids on the overall volumetric mass transfer coefficient, $k_La$, for the transfer of *i*-BuOH from an aqueous solution of liquefied corn starch (i.e., oligosaccharides) to a dispersion of oleyl alcohol droplets flowing up through a bubble column. The oleyl alcohol was fed to the column through a 2.03 mm ID nozzle. It was discovered that the ratio of the $k_La$ for a system where the solids have been removed to the $k_La$ for a system where the solids have not been removed is 2 to 5 depending on the nozzle velocity for a 2.03 mm nozzle.

Figure 13 illustrates the effect of the presence of undissolved corn mash solids on the overall volumetric mass transfer coefficient, $k_La$, for the transfer of *i*-BuOH from an aqueous solution of liquefied corn starch (i.e., oligosaccharides) to a dispersion of oleyl alcohol droplets flowing up through a bubble column. The oleyl alcohol was fed to the column through a 0.76 mm ID nozzle. It was discovered that the ratio of the $k_La$ for a system where the solids have been removed to the $k_La$ for a system where the solids have not been removed is 2 to 4 depending on the nozzle velocity for a 0.76 mm nozzle.

## Example 2

Effect of Removing Undissolved Solids on Phase Separation between an Aqueous Phase and a Solvent Phase

This example illustrates improved phase separation between an aqueous solution of oligosaccharides derived from liquefied corn mash from which undissolved solids have been removed and a solvent phase as compared to an aqueous solution of oligosaccharides derived from liquefied corn mash from which no undissolved solids have been removed and the same solvent. Both systems contained *i*-BuOH. Adequate separation of the solvent phase from the aqueous phase is important for liquid-liquid extraction to be a viable separation method for practicing ISPR.

Approximately 900 g of liquefied corn mash was prepared in a 1 L glass, jacketed resin kettle. The kettle was set up with mechanical agitation, temperature control, and pH control. The following protocol was used: mixed ground corn with tap water (26 wt% corn on a dry basis), heated the slurry to 55°C while agitating, adjusted pH to 5.8 with either NaOH or $H_2SO_4$, added alpha-amylase (0.02 wt% on a dry corn basis), continued heating to 85°C, adjusted pH to 5.8, held at 85°C for 2 hr while maintaining pH at 5.8, cool to 25°C.

Pioneer 3335 hybrid corn, whole kernel yellow corn, was used (Pioneer Hi-Bred International, Johnston, IA), and it was ground in a hammer-mill using a 1 mm screen. The moisture content of the ground corn was 12 wt%, and the starch content of the ground corn was 71.4 wt% on a dry corn basis. The alpha-amylase enzyme was Liquozyme® SC DS from Novozymes (Franklinton, NC). The total amounts of the ingredients used were: 265.9 g ground corn (12% moisture), 634.3 g tap water, and 0.056 g Liquozyme® SC DS. The total amount of liquefied corn mash recovered was 883.5 g.

Part of the liquefied corn mash was used directly, without removing undissolved solids, to prepare the aqueous phase for phase separation tests involving solids. Part of the liquefied corn mash was centrifuged to remove most of the undissolved solids and used to prepare the aqueous phase for phase separation tests involving the absence of solids.

The solids were removed from the mash by centrifugation in a large floor centrifuge. Mash (583.5 g) was centrifuged at 5000 rpm for 20 min at 35°C yielding 394.4 g centrate and 189.0 g wet cake. It was determined that the centrate contained approximately 0.5 wt% suspended solids, and that the wet cake contained approximately 20 wt% suspended solids. This implies that the original liquefied mash contained approximately 7 wt% suspended solids. This is consistent with the corn loading and starch content of the corn used assuming most of the starch was liquefied. If all of the starch was liquefied, the centrate recovered directly from the centrifuge would have contained approximately 20 wt% dissolved oligosaccharides (liquefied starch) on a solids-free basis.

The objective of the phase separation test was to measure the effect of undissolved solids on the degree of phase separation between a solvent phase and an aqueous phase that simulates a broth that is derived from liquefied corn mash. The aqueous liquid phase contained about 20 wt% oligosaccharides, and the organic phase contained oleyl alcohol (OA) in all tests. Furthermore, i-BuOH was added to all tests to give approximately 25 g/L in the aqueous phase when the phases were at equilibrium. Two shake tests were performed. The aqueous phase for the first test (with solids) was prepared by mixing 60.0 g liquefied corn mash with 3.5 g *i*-BuOH. The aqueous phase for the second test (solids removed) was prepared by mixing 60.0 g centrate which was obtained from the liquefied corn mash by removing the solids, with 3.5 g *i*-BuOH. Oleyl alcohol (15.0 g, 80/85% grade, Cognis Corporation, Cincinnati, OH) was added to each of the shake test bottles. The oleyl alcohol formed a separate liquid phase on top of the aqueous phase in both bottles resulting in a mass ratio of phases: Aq Phase/Solvent Phase to be about 1/4. Both bottles were shaken vigorously for 2 min to intimately contact the aqueous and organic phases and enable the *i*-BuOH to approach equilibrium between the two phases. The bottles were allowed to set for 1 hr. Photographs were taken at various times (0, 15, 30, and 60 min) to observe the effect of undissolved solids on phase separation in systems that contain an aqueous phase derived from liquefied corn mash, a solvent phase containing oleyl alcohol, and *i*-BuOH. Time zero (0) corresponds to the time immediately after the two minute shake period was complete.

The degree of separation between the organic (solvent) phase and the aqueous phase as a function of time for the system with solids (from liquefied corn mash) and the system where solids were removed (liquid centrate from liquefied corn mash) appeared about the same in both systems at any point in time. The organic phase was a slightly darker and cloudier, and the interface was a little less distinct (thicker "rag" layer around the interface) for the case with solids. However, for an extractive fermentation where the solvent is operated continuously, the composition of the top of the organic phase is of interest for the process downstream of the extractive fermentation wherein the next step is a distillation.

It may be advantageous to minimize the amount of microorganisms in the top of the organic phase because the microorganisms will be thermally deactivated in the distillation column. It may be advantageous to minimize the amount of undissolved solvents in the top of the organic phase because they could plug the distillation column, foul the reboiler, cause poor phase separation in the solvent/water decanter located at the base of the column, or any combination of the previously mentioned concerns. It may be advantageous to minimize the amount of phase water in the top of the organic phase. Phase water is water that exists as a separate aqueous phase. Additional amounts of aqueous phase will increase the loading and energy requirement in the distillation column. Ten milliliter (10 mL) samples were removed from the top of the organic layers from the "With Solids" and "Solids Removed" bottles, and both samples were centrifuged to reveal and compare the composition of the organic phases in the "With Solids" and "Solids Removed" bottles after 60 min of settling time. The results show that the organic phases at the end of both shake tests contained some undesired phase(s) (both organic phases are cloudy). However, the results also show that the top layer from the phase separation test involving centrate, from which solids were removed, contained essentially no undissolved solids. On the other hand, undissolved solids are clearly seen at the bottom of the 10 mL sample collected from the top of the organic phase of the test involving mash. It was estimated that 3% of the sample collected from the top of the organic layer wash mash solids. If the rich solvent phase exiting the fermentor of an extractive fermentation process contained 3% undissolved solids, one or more of the following problems could occur: loss of significant amount of microorganisms, fouling of solvent column reboiler, plugging of solvent column. The results also show that the top layer from the phase separation test involving centrate contained less phase water. Table 4 shows an estimate of the relative amount of phases that were dispersed throughout the upper "organic" layers in both shake test bottles after 60 min of settling time.

Table 4

| Approximate composition of organic (top) layer from shake tests after 60 min | | |
|---|---|---|
| | Top Layer from "With Solids" Shake Test | Top Layer from "Solids Removed" Shake Test |
| Organic (solvent) Phase: | 82% | 87% |
| Aqueous (water) Phase: | 15% | 13% |

(continued)

| Approximate composition of organic (top) layer from shake tests after 60 min | | |
|---|---|---|
| | Top Layer from "With Solids" Shake Test | Top Layer from "Solids Removed" Shake Test |
| Undissolved Solids: | 3% | 0% |

This example shows that removing most of the undissolved solids from liquefied corn mash results in improved phase separation after the liquid, aqueous phase obtained from the mash is contacted with a solvent, such as oleyl alcohol. This example shows that the upper phase obtained after phase separation will contain significantly less undissolved solids if the solids are removed first before contacting the liquid part of mash with an organic solvent. This demonstrates advantages of minimizing the undissolved solids content of mash in the upper ("organic") layer of the phase separation for an extractive fermentation.

Samples were also allowed to sit for several days after completion of sample preparation before repeating the phase separation shake test described in this example. The sample with solids consisted of liquefied corn mash, *i*-BuOH, and oleyl alcohol, and the sample with solids removed consisted of centrate which was produced by removing most of the undissolved solids from liquefied corn mash, *i*-BuOH, and oleyl alcohol. The liquefied mash contained approximately 7 wt% suspended solids, and the centrate produced from the mash contained approximately 0.5 wt% suspended solids. If all of the starch in the ground corn was liquefied, the liquid phase in the liquefied mash and the centrate produced from the mash would have contained approximately 20 wt% dissolved oligosaccharides (liquefied starch) on a solids-free basis. Both samples contained oleyl alcohol in an amount to give a mass ratio of phases: Solvent Phase/Aq Phase to be about 1/4. Furthermore, *i*-BuOH was added to all tests to give approximately 25 g/L in the aqueous phase when the phases were at equilibrium.

The objective of the phase separation test was to measure the effect of undissolved solids on the degree of phase separation after the multi-phase mixtures sat at room temperature for several days to mimic the potential change in properties of the system throughout an extractive fermentation. Two shake tests were performed. Both bottles were shaken vigorously for 2 min to intimately contact the aqueous and organic phases. The bottles were allowed to sit for 1 hr. Photographs were taken at various times (0, 2, 5, 10, 20, and 60 min) to observe the effect of undissolved solids on phase separation in these systems which had aged for several days. Time zero (0) corresponds to the time immediately after the bottles were placed on the bench.

Phase separation started to occur in the sample where solids were removed after 2 min. It appeared that almost complete phase separation had occurred in the sample where solids had been removed after only 5-10 min based on the fact that the organic phase occupied approximately 25% of the total volume of the two phase mixture. It would be expected that complete separation would be indicated if the organic phase occupied approximately 20% of the total volume, since that corresponds to the initial ratio of phases. No apparent phase separation occurred in the sample where solids were not removed even after 1 hr.

The composition of the upper phase for both samples was also compared. The composition of the upper phase has implications for the process downstream of the extractive fermentation wherein the next step is a distillation. It is advantageous to minimize the amount of microorganisms in the top of the organic phase because the microorganisms will be thermally deactivated in the distillation column. Another component to minimize in the top of the organic phase is the amount of undissolved solids because the solids could plug the distillation column, foul the reboiler, cause poor phase separation in the solvent/water decanter located at the base of the column, or any combination of the previously mentioned concerns. In addition, another component to minimize in the top of the organic phase is the amount of phase water which is water that exists as a separate aqueous phase, because this additional amount of aqueous phase will increase the loading and energy requirement in the subsequent distillation column.

Ten milliliter (10 mL) samples were removed from the top of the organic layers from the "With Solids" and "Solids Removed" bottles, and both samples were centrifuged to reveal and compare the composition of the organic phases in the "With Solids" and "Solids Removed" bottles after 60 min of settling time. The composition of the sample collected from the top of the "With Solids" sample confirms that essentially no phase separation occurred in that sample within 60 min. Specifically, the ratio of the solvent phase to total aqueous phase (aqueous liquid + suspended solids) in the sample collected from the top of the "With Solids" shake test bottle is approximately 1/4 w/w, which is the same ratio used to prepare the sample prior to the test. Also, the amount of undissolved solids in the sample collected from the top of the "With Solids" shake test bottle is approximately the same as what is found in liquefied corn mash, which shows that essentially no solids settled in this shake test bottle within 60 min. On the other hand, the top layer from the phase separation test involving centrate ("Solids Removed") from which solids were removed, contained essentially no undissolved solids. The results also show that the top layer from the phase separation test involving centrate contained less phase water. This is indicated by the fact that the ratio of the solvent phase to aqueous phase in that sample bottle is approximately 1/1 w/w, which shows that the organic phase was enriched with solvent (oleyl alcohol) in the test where solids were removed. Table 5 shows an estimate of the relative amount of phases that were dispersed throughout the upper "organic" layers in both shake test bottles after 60 min of settling time.

Table 5

| Approximate composition of organic (top) layer from shake tests after 60 min | | |
|---|---|---|
| | Top Layer from "With Solids" Shake Test | Top Layer from "Solids Removed" Shake Test |
| Organic (solvent) Phase: | 19% | 50% |
| Aqueous (water) Phase: | 47% | 50% |
| Undissolved Solids: | 34% | 0% |

This example shows that removing undissolved solids from liquefied corn mash that contains *i*-BuOH, contacting it with a solvent phase, letting it set for several days, and mixing the phases again results in improved phase separation when compared to a sample where undissolved solids were not removed from the liquefied mash. In fact, this example shows that essentially no phase separation occurs in the sample where undissolved solids were not removed even after 60 min. This example shows that the upper phase obtained after phase separation contains significantly less undissolved solids if the solids are removed first before contacting the liquid part of mash with an organic solvent. This is important because two of the most important species that should be minimized in the upper ("organic") layer of the phase separation for an extractive fermentation are the level of microorganisms and the level of undissolved solids from mash. The previous example showed that removing solids from liquefied corn mash results in improved phase separation shortly after the aqueous phase is contacted with a solvent phase. This would allow extractive fermentation to be viable at earlier times in the fermentation. This example also shows that removing solids from liquefied corn mash results in improved phase separation in aged samples that contain an aqueous phase (oligosaccharide solution with solids removed) that has been contacted with a solvent phase. This would also allow extractive fermentation to be viable at later times in the fermentation.

## Example 3

Effect of Removing Undissolved Solids on the Loss of ISPR Extraction Solvent - Disk Stack Centrifuge

This example demonstrates the potential for reducing solvent losses via DDGS generated by the extractive fermentation process by removing undissolved solids from the corn mash prior to fermentation using a semi-continuous disk-stack centrifuge.

Approximately 216 kg liquefied corn mash was prepared in a jacketed stainless steel reactor. The reactor was set up with mechanical agitation, temperature control, and pH control. The protocol used was as follows: mixed ground corn with tap water (25 wt% corn on a dry basis), heated the slurry to 55°C while agitating at 400 rpm, adjusted pH to 5.8 with either NaOH or $H_2SO_4$, added alpha-amylase (0.02 wt% on a dry corn basis), continued heating to 85°C, adjusted pH to 5.8, held at 85°C for 30 min while maintaining pH at 5.8, heated to 121°C using live steam injection, held at 121°C for 30 min to simulate a jet cooker, cooled to 85°C, adjusted pH to 5.8, added second charge of alpha-amylase (0.02 wt% on a dry corn basis), held at 85°C for 60 min while maintaining pH at 5.8 to complete liquefaction. The mash was then cooled to 60°C and transferred to the centrifuge feed tank.

Pioneer 3335 hybrid corn, whole kernel yellow corn, was used (Pioneer Hi-Bred International, Johnston, IA), and it was ground in a hammer-mill using a 1 mm screen. The moisture content of the ground corn was 12 wt%, and the starch content of the ground corn was 71.4 wt% on a dry corn basis. The alpha-amylase enzyme was Liquozyme® SC DS from Novozymes (Franklinton, NC). The amounts of the ingredients used were: 61.8 kg ground corn (12% moisture), 147.3 kg tap water, a solution of 0.0109 kg Liquozyme® SC DS in 1 kg water for first alpha-amylase charge, another solution of 0.0109 kg Liquozyme® SC DS in 1 kg water for second alpha-amylase charge (after the cook stage). About 5 kg $H_2O$ was added to the batch via steam condensate during the cook stage. A total of 0.25 kg NaOH (12.5 wt%) and 0.12 kg $H_2SO_4$ (12.5 wt%) were added throughout the run to control pH. The total amount of liquefied corn mash recovered was 216 kg.

The composition of the final liquefied corn mash slurry was estimated to be approximately 7 wt% undissolved solids and 93 wt% liquid. The liquid phase contained about 19 wt% (190 g/L) liquefied starch (soluble oligosaccharides). The rheology of the mash is important regarding the ability to separate the slurry into its components. The liquid phase in the mash was determined to be a Newtonian fluid with a viscosity of about 5.5 cP at 30°C. The mash slurry was determined to be a shear-thinning fluid with a bulk viscosity of about 10 to 70 cP at 85°C depending on shear rate.

The liquefied mash (209 kg, 190 L) was centrifuged using a disk-stack split-bowl centrifuge (Alfa Laval Inc., Richmond, VA). The centrifuge operated in semi-batch mode with continuous feed, continuous centrate outlet, and batch discharge of the wet cake. Liquefied corn mash was continuously fed at a rate of 1 L/min, clarified centrate was removed continuously, and wet cake was periodically discharged every 4 min. To determine an appropriate discharge interval for the solids from the disk stack, a sample of the mash to be fed to the disk stack was centrifuged in a high-speed lab centrifuge. Mash (48.5 g) was spun at 11,000 rpm (about 21,000 g for about 10 min at room temperature. Clarified centrate (36.1 g) and 12.4 g pellet (wet cake) were recovered. It was determined that the clarified centrate contained about 0.3 wt% undissolved solids and that the pellet (wet cake) contained about 27 wt% undissolved solids. Based on this data, a discharge interval of 4 min was chosen for operation of the disk stack centrifuge.

The disk stack centrifuge was operated at 9000 rpm (6100 g) with a liquefied corn mash feed rate of 1 L/min and at about 60°C. Mash (209 kg) was separated into 155 kg clarified centrate and 55 kg wet cake. The split, defined as (amount of centrate)/(amount of mash fed), achieved by the semi-continuous disk stack was similar to the split achieved in the batch centrifuge. The split for the disk stack semi-batch centrifuge operating at 6100 g, 1 L/min feed rate, and 4 min discharge interval was (155 kg/209 kg) = 74%, and the split for the lab batch centrifuge operating at 21,000 g for 10 min was (36.1 g/48.5 g) = 74%.

A 45 mL sample of the clarified centrate recovered from the disk stack centrifuge was spun down in a lab centrifuge at 21,000 g for 10 min to estimate the level of suspended solids in the centrate. About 0.15-0.3 g undissolved solids were recovered from the 45 mL of centrate. This corresponds to 0.3-0.7 wt% undissolved solids in the centrate which is about a ten-fold reduction in undissolved solids from mash fed to the centrifuge. It is reasonable to assume that the ISPR extraction solvent losses via DDGS could be reduced by about an order of magnitude if the level of undissolved solids present in extractive fermentation is reduced by an order of magnitude using some solid/liquid separation device or combination of devices to remove suspended solids from the corn mash before fermentation. Minimizing solvent losses via DDGS is an important factor in the economics and DDGS quality for an extractive fermentation process.

## Example 4

Effect of Removing Undissolved Solids on the Loss of ISPR Extraction Solvent - Bottle Spin Test

This example demonstrates the potential for reducing solvent losses via DDGS generated by the extractive fermentation process by removing undissolved solids from the corn mash prior to fermentation using a centrifuge.

A lab-scale bottle spin test was performed using liquefied corn mash. The test simulates the operating conditions of a typical decanter centrifuge used to remove undissolved solids from whole stillage in a commercial ethanol plant. Decanter centrifuges in commercial ethanol plants typically operate at a relative centrifugal force (RCF) of about 3000 g and a whole stillage residence time of about 30 seconds. These centrifuges typically remove about 90% of the suspended solids in whole stillage which contains about 5% to 6% suspended solids (after the beer column), resulting in thin stillage which contains about 0.5% suspended solids.

Liquefied corn mash was made according to the protocol described in Example 2. About 10 mL mash was placed in a centrifuge tube. The sample was centrifuged at an RCF of about 3000 g (4400 rpm rotor speed) for a total of 1 min. The sample spent about 30-40 seconds at 3000 g and a total of 20-30 seconds at speeds less than 3000 g due to acceleration and deceleration of the centrifuge. The sample temperature was about 60°C.

The mash (10 mL) which contained about 7 wt% suspended solids was separated into about 6.25 mL clarified centrate and 3.75 mL wet cake (pellet at the bottom of the centrifuge tube). The split, defined as (amount of centrate)/(amount of original mash charged), achieved by the bottle spin test was about 62%. It was determined that the clarified centrate contained about 0.5 wt% suspended solids which is more than a ten-fold decrease in suspend solids compared to the level of suspended solids in the original mash. It was also determined that the clarified pellet contained about 18 wt% suspended solids.

Table 6 summarizes the suspended (undissolved) solids mass balance for the bottle spin test at conditions representative of the operation of a decanter centrifuge to convert whole stillage to thin stillage in a commercial ethanol process. All values given in Table 6 are approximate.

Table 6

| | Volume, mL | Suspend Solids, wt% |
|---|---|---|
| Liquefied Corn Mash charge: | 10 | 7% |
| Clarified Centrate: | 6.25 | 0.5% |
| Wet Cake (pellet): | 3.75 | 18% |

(continued)

| | Volume, mL | Suspend Solids, wt% |
|---|---|---|
| | | |
| Performance Summary | | |
| Split: | 62% | |
| Centrate Clarity: | 0.5 wt% suspended solids | |
| Cake (pellet) Dryness: | 18 wt% suspended solids | |
| % Removal of Suspended Solids: | 95% removal from liquefied mash | |

It was also determined that the centrate contained about 190 g/L dissolved oligosaccharides (liquefied starch). This is consistent with the assumption that most of the starch in the ground corn was liquefied (i.e., hydrolyzed to soluble oligosaccharides) in the liquefaction process based on the corn loading used (about 26 wt% on a dry corn basis) and the starch content of the corn used to produce the liquefied mash (about 71.4 wt% starch on a dry corn basis). Hydrolyzing most of the starch in the ground corn at a 26% dry corn loading will result in about 7 wt% suspended (undissolved) solids in the liquefied corn mash charged to the centrifuge used for the bottle spin test.

The fact that the clarified centrate contained only about 0.5 wt% undissolved solids indicates that the conditions used in the bottle spin test resulted in more than a ten-fold reduction in undissolved solids from mash charged. If this same solids removal performance could be achieved by a continuous decanter centrifuge before fermentation, it is reasonable to assume that the ISPR extraction solvent losses in the DDGS could be reduced by about an order of magnitude. Minimizing solvent losses via DDGS is an important factor in the economics and DDGS quality for an extractive fermentation process.

**Example 5**

Removal of Corn Oil by Removing Undissolved Solids

This example demonstrates the potential to remove and recover corn oil from corn mash by removing the undissolved solids prior to fermentation. The effectiveness of the extraction solvent may be improved if corn oil is removed via removal of the undissolved solids. In addition, removal of corn oil via removal of the undissolved solids may also minimize any reduction in solvent partition coefficient and potentially resulting an improved extractive fermentation process.

Approximately 1000 g liquefied corn mash was prepared in a 1 L glass, jacketed resin kettle. The kettle was set up with mechanical agitation, temperature control, and pH control. The following protocol was used: mixed ground corn with tap water (26 wt% corn on a dry basis), heated the slurry to 55°C while agitating, adjusted pH to 5.8 with either NaOH or $H_2SO_4$, added alpha-amylase (0.02 wt% on a dry corn basis), continued heating to 85°C, adjusted pH to 5.8, held at 85°C for 2 hr while maintaining pH at 5.8, cool to 25°C.

Pioneer 3335 hybrid corn, whole kernel yellow corn, was used (Pioneer Hi-Bred International, Johnston, IA), and it was ground in a hammer-mill using a 1 mm screen. The moisture content of the ground corn was about 11.7 wt%, and the starch content of the ground corn was about 71.4 wt% on a dry corn basis. The alpha-amylase enzyme was Liquozyme® SC DS from Novozymes (Franklinton, NC). The total amounts of the ingredients used were: 294.5 g ground corn (11.7% moisture), 705.5 g tap water, and 0.059 g Liquozyme® SC DS. Water (4.3 g) was added to dilute the enzyme, and a total of 2.3 g of 20% NaOH solution was added to control pH. About 952 g of mash was recovered.

The liquefied corn mash was centrifuged at 5000 rpm (7260 g) for 30 min at 40°C to remove the undissolved solids from the aqueous solution of oligosaccharides. Removing the solids by centrifugation also resulted in the removal of free corn oil as a separate organic liquid layer on top of the aqueous phase. Approximately 1.5 g of corn oil was recovered from the organic layer floating on top of the aqueous phase. It was determined by hexane extraction that the ground corn used to produce the liquefied mash contained about 3.5 wt% corn oil on a dry corn basis. This corresponds to about 9 g corn oil fed to the liquefaction process with the ground corn.

After recovering the corn oil from the liquefied mash, the aqueous solution of oligosaccharides was decanted away from the wet cake. About 617 g liquefied starch solution was recovered leaving about 334 g wet cake. The wet cake contained most of the undissolved solids that were in the liquefied mash. The liquefied starch solution contained about 0.2 wt% undissolved solids. The wet cake contained about 21 wt% undissolved solids. The wet cake was washed with 1000 g tap water to remove the oligosaccharides still in the cake. This was done by mixing the cake with the water to form a slurry. The slurry was then centrifuged under the same conditions used to centrifuge the original mash in order to recover the washed solids. Removing the washed solids by centrifuging the wash slurry also resulted in the removal of some additional free corn oil that must have remained with the original wet cake produced from the liquefied mash. This additional corn oil was observed as a separate, thin, organic liquid layer on top of the aqueous phase of the centrifuged wash mixture. Approximately 1 g of additional corn oil was recovered from the wash process.

The wet solids were washed two more times using a 1000 g tap water each time to remove essentially all of the liquefied starch. No visible additional corn oil was removed from the 2$^{nd}$ and 3$^{rd}$ water washes of the mash solids. The final washed solids were dried in a vacuum oven overnight at 80°C and about 67728 Pa (20 inches Hg) vacuum. The amount of corn oil remaining in the dry solids, presumably still in the germ, was determined by hexane extraction. A sample of relatively dry solids (3.60 g, about 2 wt% moisture) contained 0.22 g corn oil. This result corresponds to 0.0624 g corn oil/g dry solids. This was for washed solids which means there are no residual oligosaccharides in the wet solids. After centrifuging the liquefied corn mash to separate the layer of free corn oil and the aqueous solution of oligosaccharides from the wet cake, it was determined that about 334 g wet cake containing about 21 wt% undissolved solids remained. This corresponds to the wet cake comprising about 70.1 g undissolved solids. At 0.0624 g corn oil/g dry solids, the solids in the wet cake should contain about 4.4 g corn oil.

In summary, approximately 1.5 g free corn oil was recovered by centrifuging the liquefied mash. An additional 1 g free corn oil was recovered by centrifuging the first (water) wash slurry which was generated to wash the original wet cake produced from the mash. Finally, it was determined that the washed solids still contained about 4.4 g corn oil. It was also determined that the corn charged to the liquefaction contained about 9 g corn oil. Therefore, a total of 6.9 g corn oil was recovered from the following process steps: liquefaction, removal of solids from liquefied mash, washing of the solids from the mash, and the final washed solids. Consequently, approximately 76% of the total corn oil in the corn fed to liquefaction was recovered during the liquefaction and solids removal process described here.

## Example 6

### Extractive Fermentation Using Mash and Centrate as the Sugar Source

This example describes extractive fermentations performed using corn mash and corn mash centrate as the sugar source. Corn mash centrate was produced by removing undissolved solids from the corn mash prior to fermentation. Four extractive fermentations were conducted side-by side, two with liquefied corn mash as the sugar source (solids not removed) and two with liquefied mash centrate (aqueous solution of oligosaccharides) obtained by removing most of the undissolved solids from liquefied corn mash. Oleyl alcohol (OA) was added to two of the fermentations, one with solids and one with solids removed, to extract the product (*i*-BuOH) from the broth as it was formed. A mixture of corn oil fatty acids (COFA) was added to the other two of the fermentations, one with solids and one with solids removed, to extract the product from the broth as it was formed. The COFA was made by hydrolyzing corn oil. The purpose of these fermentations was to test the effect of removing solids on phase separation between the solvent and broth and to measure the amount of residual solvent trapped in the undissolved solids recovered from fermentation broths where solids were or were not removed.

### Preparation of Liquefied Corn Mash

Approximately 31 kg liquefied corn mash was prepared in a 30 L jacketed glass resin kettle. The reactor was outfitted with mechanical agitation, temperature control, and pH control. The protocol used was as follows: mix ground corn with tap water (40 wt% corn on a dry basis), heat the slurry to 55°C while agitating at 250 rpm, adjust pH to 5.8 with either NaOH or $H_2SO_4$, add a dilute aqueous solution of alpha-amylase (0.16 wt% on a dry corn basis), hold at 55°C for 60 min, heat to 95°C, adjust pH to 5.8, hold at 95°C for 120 min while maintaining pH at 5.8 to complete liquefaction. The mash was transferred into sterile centrifuge bottles to prevent contamination.

The corn used was whole kernel yellow corn (Pioneer Hi-Bred International, Johnston, IA), and it was ground in a pilot-scale hammer-mill using a 1 mm screen. The moisture content of the ground corn was about 12 wt%, and the starch content of the ground corn was about 71.4 wt% on a dry corn basis. The alpha-amylase enzyme used was Spezyme® Fred-L (Genencor®, Palo Alto, CA). The amounts of the ingredients used were: 14.1 kg ground corn (12% moisture), 16.9 kg tap water, a solution of alpha-amylase consisting of 19.5 g Spezyme® Fred-L in 2.0 kg water. The alpha-amylase was sterile filtered. A total of 0.21 kg NaOH (17 wt%) was added throughout the run to control pH.

It was estimated that the liquefied corn mash contained approximately 28 wt% (about 280 g/L) of liquefied starch based on the corn loading used, starch content of the corn, and assuming all the starch was hydrolyzed during liquefaction. The mash was prepared with a higher concentration of oligosaccharides than was desired in the fermentations to allow for dilution when adding the nutrients, inoculum, glucoamylase, and base to the initial fermentation broth. After dilution by addition of nutrients, inoculum, glucoamylase, and base, the expected total initial soluble sugars in the mash (solids not removed) was about 250 g/L.

About 13.9 kg liquefied mash was centrifuged using a bottle centrifuge which contained six 1 L bottles. The centrifuge was operated at 5000 rpm (7260 RCF) for 20 min at room temperature. The mash was separated into about 5.5 kg clarified centrate and about 8.4 kg wet cake (the pellet at the bottom of the centrifuge bottles). The split, defined as (amount of centrate)/(amount of mash fed), was about (5.5 kg/13.9 kg) = 40%.

Solids were not removed from the mash charged to the 2010Y034 and 2010Y036 fermentations described below. The centrate charged to fermentations 2010Y033 and 2010Y035 (also described below) was produced by removing by centrifugation most of the suspended solids from mash according to the protocols above.

General Methods for Fermentation

Seed Flask Growth

A *Saccharomyces cerevisiae* strain (with deletions of *pdc1, pdc5,* and *pdc6*) that was engineered to produce isobutanol from a carbohydrate source was grown to 0.55-1.1 g/L dcw ($OD_{600}$ 1.3-2.6 - Thermo Helios $\alpha$ Thermo Fisher Scientific Inc., Waltham, Massachusetts) in seed flasks from a frozen culture. The *Saccharomyces cerevisiae* strain is described in U.S. Patent Application Publication No. 2012/0164302. The culture was grown at 26°C in an incubator rotating at 300 rpm. The frozen culture was previously stored at - 80°C. The composition of the first seed flask medium was:

3.0 g/L dextrose
3.0 g/L ethanol, anhydrous
3.7 g/L ForMedium™ Synthetic Complete Amino Acid (Kaiser) Drop-Out:
without HIS, without URA (Reference No. DSCK162CK)
6.7 g/L Difco Yeast Nitrogen Base without amino acids (No. 291920).

Twelve milliliters (12 mL) from the first seed flask culture was transferred to a 2 L flask and grown at 30°C in an incubator rotating at 300 rpm. The second seed flask has 220 mL of the following medium:

30.0 g/L dextrose
5.0 g/L ethanol, anhydrous
3.7 g/L ForMedium™ Synthetic Complete Amino Acid (Kaiser) Drop-Out:
without HIS, without URA (Reference No. DSCK162CK)
6.7 g/L Difco Yeast Nitrogen Base without amino acids (No. 291920)
0.2M MES Buffer titrated to pH 5.5-6.0.

The culture was grown to 0.55-1.1 g/L dcw ($OD_{600}$ 1.3-2.6). An addition of 30 mL of a solution containing 200 g/L peptone and 100 g/L yeast extract was added at this cell concentration. Then an addition of 300 mL of 0.2 uM filter sterilized, 90-95% oleyl alcohol (Cognis Corporation, Cincinnati, OH) was added to the flask. The culture continued to grow to > 4 g/L dcw ($OD_{600}$ > 10) before being harvested and added to the fermentation.

Fermentation Preparation

*Initial Fermentor Preparation*

A glass jacked, 2 L fermentor (Sartorius AG, Goettingen, Germany) was charged with liquefied mash either with or without solids (centrate). A pH probe (Hamilton Easyferm Plus K8, part number: 238627, Hamilton Bonaduz AG, Bonaduz, Switzerland) was calibrated through the Sartorius DCU-3 Control Tower Calibration menu. The zero was calibrated at pH=7. The span was calibrated at pH=4. The probe was then placed into the fermentor, through the stainless steel head plate. A dissolved oxygen probe ($pO_2$ probe) was also placed into the fermentor through the head plate. Tubing used for delivering nutrients, seed culture, extracting solvent, and base were attached to the head plate and the ends were foiled. The entire fermentor was placed into an autoclave (Steris Corporation, Mentor, Ohio) and sterilized in a liquid cycle for 30 min.

The fermentor was removed from the autoclave and placed on a load cell. The jacket water supply and return line was connected to the house water and clean drain, respectively. The condenser cooling water in and water out lines were connected to a 6-L recirculating temperature bath running at 7°C. The vent line that transfers the gas from the fermentor was connected to a transfer line that was connected to a Thermo mass spectrometer (Prima dB, Thermo Fisher Scientific Inc., Waltham, Massachusetts). The sparger line was connected to the gas supply line. The tubing for adding nutrients, extract solvent, seed culture, and base was plumbed through pumps or clamped closed. The autoclaved material, 0.9% w/v NaCl was drained prior to the addition of liquefied mash.

*Lipase Treatment Post-Liquefaction*

The fermentor temperature was set to 55°C instead of 30°C after the liquefaction cycle was complete. The pH was manually controlled at pH=5.8 by making bolus additions of acid or base when needed. A lipase enzyme stock solution was added to the fermentor to a final lipase concentration of 10 ppm. The fermentor was held at 55°C, 300 rpm, and 0.3 slpm $N_2$ overlay for >6 hr. After the lipase treatment was complete the fermentor temperature was set to 30°C.

*Nutrient Addition Prior to Inoculation*

Added 7.0 mL/L (post-inoculation volume) of ethanol (200 proof, anhydrous) just prior to inoculation. Added thiamine to 20 mg/L final concentration just prior to inoculation. Added 100 mg/L nicotinic acid just prior to inoculation.

*Fermentor Inoculation*

The fermentor $pO_2$ probe was calibrated to zero while $N_2$ was being added to the fermentor. The fermentor $pO_2$ probe was calibrated to its span with sterile air sparging at 300 rpm. The fermentor was inoculated after the second seed flask was > 4 g/L dcw. The shake flask was removed from the incubator/shaker for 5 min allowing a phase separation of the oleyl alcohol phase and the aqueous phase. The aqueous phase (55 mL) was transferred to a sterile, inoculation bottle. The inoculum was pumped into the fermentor through a peristaltic pump.

*Oleyl Alcohol or Corn Oil Fatty Acids Addition After Inoculation*

Added 1 L/L (post-inoculation volume) of oleyl alcohol or corn oil fatty acids immediately after inoculation

*Fermentor Operating Conditions*

The fermentor was operated at 30°C for the entire growth and production stages. The pH was allowed to decrease from a pH between 5.7-5.9 to a control set-point of 5.2 without adding any acid. The pH was controlled for the remainder of the growth and production stage at a pH =5.2 with ammonium hydroxide. Sterile air was added to the fermentor, through the sparger, at 0.3 slpm for the remainder of the growth and production stages. The $pO_2$ was set to be controlled at 3.0% by the Sartorius DCU-3 Control Box PID control loop, using stir control only, with the stirrer minimum being set to 300 rpm and the maximum being set to 2000 rpm. The glucose was supplied through simultaneous saccharification and fermentation of the liquefied corn mash by adding a $\alpha$-amylase (glucoamylase). The glucose was kept excess (1-50 g/L) for as long as starch was available for saccharification.

**Sample A**

Experiment identifier 2010Y033 included: Seed Flask Growth method, Initial Fermentor Preparation method with corn mash that excludes solids, Lipase Treatment Post-Liquefaction, Nutrient Addition Prior to Inoculation method, Fermentor Inoculation method, Fermentor Operating Conditions method, and all of the Analytical methods. Corn oil fatty acid was added in a single batch between 0.1- 1.0 hr after inoculation.

**Sample B**

Experiment identifier 2010Y034 included: Seed Flask Growth method, Initial Fermentor Preparation method with corn mash that includes solids, Lipase Treatment Post-Liquefaction, Nutrient Addition Prior to Inoculation method, Fermentor Inoculation method, Fermentor Operating Conditions method, and all of the Analytical methods. Corn oil fatty acid was added in a single batch between 0.1- 1.0 hr after inoculation.

**Sample C**

Experiment identifier 2010Y035 included: Seed Flask Growth method, Initial Fermentor Preparation method with corn mash that excludes solids, Nutrient Addition Prior to Inoculation method, Fermentor Inoculation method, Fermentor Operating Conditions method, and all of the Analytical methods. Oleyl alcohol was added in a single batch between 0.1- 1.0 hr after inoculation.

**Sample D**

Experiment identifier 2010Y036 included: Seed Flask Growth method, Initial Fermentor Preparation method with corn mash that includes solids, Nutrient Addition Prior to Inoculation method, Fermentor Inoculation method, Fermentor Operating Conditions method, and all of the Analytical methods. Oleyl alcohol was added in a single batch between 0.1- 1.0 hr after inoculation. Results for Samples A-D are shown in Table 7.

Table 7: Fermentation conditions and results for Samples A-D

| Sample | Experimental ID | Active Lipase | Post - Liquefaction Undissolved Solids Removed | Extracting Solvent | Glucose Equivalents Charged g/kg | g/kg glucose consumed at EOR | Effective isobutan ol g/L |
|---|---|---|---|---|---|---|---|
| A | 2010Y033 | Yes | Yes | Corn oil fatty acids | 257 | 257 | 30.9 |
| B | 2010Y034 | Yes | No | Corn oil fatty acids | 239 | 239 | 17.3 |
| C | 2010Y035 | No | Yes | Oleyl alcohol | 263 | 72 | 15.7 |
| D | 2010Y036 | No | No | Oleyl alcohol | 241 | 101 | 20 |

**Example 7**

Effect of Removing Undissolved Solids from the Fermentor Feed on Improvement in Fermentor Volume Efficiency

This example demonstrates the effect of removing undissolved solids from the mash prior to fermentation on fermentor volume efficiency. Undissolved solids in corn mash occupy at least 5% of the mash volume depending on corn loading and content starch content. Removing solids before fermentation enables at least 5% more sugar to be charged to the fermentor thus increasing batch productivity.

It was estimated that the liquefied corn mash produced in Example 5 contained approximately 28 wt% (280 g/L) liquefied starch based on the corn loading used (40% dry corn basis), starch content of the corn (71.4% dry corn basis), and assuming all the starch was hydrolyzed to soluble oligosaccharides during liquefaction. The mash was prepared with a higher concentration of oligosaccharides than was desired in the fermentations to allow for dilution when adding the nutrients, inoculum, glucoamylase, and base to the initial fermentation broth. The mash was diluted by approximately 10% after adding these ingredients. Therefore, the expected concentration of liquefied starch in the mash (including solids) at the beginning of fermentations 2010Y034 and 2010Y036 was about 250 g/L. The actual glucose equivalents charged to the 2010Y034 and 2010Y036 fermentations was measured to be 239 g/kg and 241 g/kg, respectively. By comparison, the glucose equivalents charged to the 2010Y033 and 2010Y035 fermentations was measured to be 257 g/kg and 263 g/kg, respectively. Note that the feed to these fermentations was centrate (mash from which most of the solids had been removed). Approximately 1.2 L of the sugar source (mash or centrate) was charged to each fermentation. Therefore, based on this data, approximately 8.3% more sugar was charged to the fermentors which used centrate (2010Y033 and 2010Y035) compared to mash (2010Y034 and 2010Y036). These results demonstrate that removing undissolved solids from corn mash prior to fermentation can result in a significant increase in sugar charged per unit volume. This implies that when solids are present, they occupy valuable fermentor volume. If solids are removed from the feed, more sugar may be added ("fit") to the fermentor due to the absence of undissolved solids. This example demonstrates that fermentor volume efficiency can be significantly improved by removing undissolved solids from the mash prior to fermentation.

**Example 8**

Effect of Removing Undissolved Solids on Phase Separation between the Extraction Solvent and the Broth - Extractive Fermentation

This example demonstrates improved separation between the solvent phase and the broth phase during and after an extractive fermentation process by removing undissolved solids from the corn mash prior to fermentation. Two extractive fermentations were conducted side-by side, one with liquefied corn mash as the sugar source (solids not removed) and one with centrate (aqueous solution of oligosaccharides) which was generated by removing most of the undissolved solids from liquefied corn mash. Oleyl alcohol (OA) was added to both fermentations to extract the product (*i*-BuOH) from the broth as it was formed. The fermentation broth referred to in this example where solids were not removed from the feed (used corn mash) was 2010Y036 as described in Example 6. The fermentation broth referred to in this example where solids were removed from the feed (used centrate produced from corn mash) was 2010Y035 as described in Example 6. Oleyl alcohol was the extraction solvent used in both fermentations. The rate and degree of phase separation was measured and compared throughout the fermentations as well as for the final fermentation broths. Adequate phase separation in an extractive fermentation process can lead to minimal loss of the microorganism and minimal solvent losses as well lower capital and operating cost of downstream processing.

Phase Separation Between Solvent and Broth Phases During Fermentation

Approximately 10 mL samples were pulled from each fermentor at 5.3, 29.3, 53.3, and 70.3 hr, and phase separation was compared for the samples from the fermentation where solids were removed (2010Y035) from the samples and where solids were not removed (2010Y036). Phase separation was observed and compared for all samples from all run times by allowing the samples to set for about 2 hr and tracking the position of the liquid-liquid interface. Essentially no phase separation was observed for any of the samples pulled from the fermentation where solids were not removed. Phase separation was observed for all samples from the fermentation where solids were removed from the liquefied corn mash prior to fermentation. Separation started to occur within about 10-15 min of pulling the samples from the run where solids were removed for all fermentation times and continued to improve over a 2 hr period of time. Phase separation started to occur in the sample pulled at 5.3 hr fermentation run time from the centrate fermentation (solids removed) after about 7 min of settling time. Phase separation started to occur in the sample pulled at 53.3 hr from the centrate fermentation (solids removed) after about 17 min of settling time.

Figure 13 is a plot of the position of the liquid-liquid interface in the fermentation sample tubes as a function of (gravity) settling time. The data is for the samples pulled from the extractive fermentation where centrate was fed (solids removed from corn mash) as the sugar source and oleyl alcohol was the ISPR extraction solvent (run 2010Y035 in Example 6). The phase separation data in this plot is for samples pulled at 5.3, 29.3, 53.3, and 70.3 hr run time from fermentation 2010Y035. The interface position is reported as a percentage of the total broth height in the sample tube. For example, the interface position in the sample pulled at 5.3 hr run time from the 2010Y035 fermentation (centrate/ oleyl alcohol) increased from the bottom of the sample tube (no separation) to 3.5 mL after 120 min of settling time. There was about 10 mL of total broth in that particular sample tube. Therefore, the interface position for that sample was reported as 35% in Figure 13. Similarly, the interface position in the sample pulled at 53.3 hr run time from the 2010Y035 fermentation (centrate/ oleyl alcohol) increased from the bottom of the sample tube (no separation) to about 3.9 mL after 125 min of settling time. There was about 10 mL of total broth in that particular sample tube. Therefore, the interface position for that sample was reported as 39% in Figure 13.

Phase Separation Between Solvent and Broth Phases After Completing Fermentation

After 70 hr of run time, the fermentations were stopped, and the two broths from the oleyl alcohol extractive fermentations were transferred to separate 2 L glass graduated cylinders. The separation of the solvent and broth phases were observed and compared. Almost no phase separation was observed after about 3 hr for the broth where solids were not removed prior to fermentation (run 2010Y036). Phase separation was observed for the broth where solids were removed from the liquefied corn mash prior to fermentation (run 2010Y035). Separation started to occur after about 15 min of settling time and continued to improve over a 3 hr period of time. After 15 min, a liquid-liquid interface was established at a level that was about 10% of the total height of the two phase mixture. This indicates that the aqueous phase splits out from the dispersion first and starts to accumulate at the bottom of the mixture. As time proceeded, more aqueous phase accumulated at the bottom of the mixture causing the position of the interface to rise. After about 3 hr of settling time, the interface had increased to a level that was about 40% of the total height of the two phase mixture. This indicates that almost complete phase separation had occurred after about 3 hr of (gravity) settling time for the final two phase broth where solids were removed based on the amounts of centrate and oleyl alcohol initially charged to the fermentation. Approximately equal volumes of initial centrate and solvent were charged to both fermentations. Approximately 1.2 L of liquefied corn mash and approximately 1.1 L of oleyl alcohol were charged to fermentation 2010Y036. Approximately 1.2 L of centrate, which was produced from the same batch of mash, and approximately 1.1 L of oleyl alcohol were charged to fermentation 2010Y035. After accounting for the fact that approximately 100 g/kg of the initial sugar in the aqueous phase was consumed and the fact that about 75% of the i-BuOH produced was in the solvent phase, it would be expected that the relative volumes of the final aqueous and organic phases would be about 1:1 if complete separation occurred. Figure 14 is a plot of the liquid-liquid interface position as a function of (gravity) settling time for the final two phase broth from the extractive fermentation where solids were removed (2010Y035). The interface position is reported as a percentage of the total broth height in the 2 L graduated cylinder used to observe phase separation of the final broth. The interface position of the final broth from the 2010Y035 fermentation increased from the bottom of the graduated cylinder (no separation) to a level that was about 40% of the total height of the two phase mixture after 175 min of settling time. Therefore, almost complete separation of the two phases in the final broth occurred after 3 hr of settling time. An interface position of approximately 50% would correspond to complete separation.

A 10 mL sample was pulled from the top of the organic phase of the final broth (which had settled for about 3 hr) from the fermentation where solids had been removed. The sample was spun in a high-speed lab centrifuge to determine the amount of aqueous phase that was present in the organic phase after allowing the broth to settle for 3 hr. The results showed that about 90% of the top layer of the final broth was solvent phase. About 10% of the top layer of the final broth was aqueous phase, including a relatively small amount of undissolved solids. Some solids were located at the bottom of the aqueous phase (more dense than the aqueous phase) and also a small amount of solids accumulated at the liquid-liquid interface.

A 10 mL sample was also pulled from the bottom phase of the final broth (which had settled for about 3 hr) from the fermentation where solids had been removed. The sample was spun in a high-speed lab centrifuge to determine the amount of organic phase that was present in the aqueous phase after allowing the broth to settle for 3 hr. It was determined that essentially no organic phase was present in the bottom (aqueous) phase of the final broth from the fermentation from which solids had been removed after the broth had settled for 3 hr. These results confirm that almost complete phase separation had occurred for the final broth from the fermentation where solids had been removed. Almost no phase separation was apparent for the final broth from the fermentation where solids had not been removed. This data implies that removing solids from liquefied corn mash before extractive fermentation may enable a significant improvement in phase separation during and after fermentation resulting in less loss of the microorganism, undissolved solids, and water to downstream processing.

A 10 mL sample was pulled from the top of the final broth from the fermentation from which solids had not been removed after the broth had set for about 3 hr. The sample was spun in a high-speed lab centrifuge to determine the relative amount of solvent and aqueous phases at the top of the final broth. This broth contained all solids from the liquefied corn mash solids. About half of the sample was aqueous phase, and about half was organic phase. The aqueous phase contained significantly more undissolved solids (from the liquefied mash) compared to the sample of the top layer from the broth where solids were removed. The amounts of aqueous and solvent phases in this sample are approximately the same indicating that essentially no phase separation occurred in the final broth where solids were not removed (even after 3 hr of settling time). This data implies that if solids are not removed from liquefied corn mash before an extractive fermentation, little to no phase separation is likely to occur during and after fermentation. This could result in a significant loss of the microorganism, undissolved solids, and water to downstream processing.

## Example 9

Effect of Removing Undissolved Solids on the Loss of ISPR Extraction Solvent - Extractive Fermentation

This example demonstrates the potential for reducing solvent losses with the DDGS out the back end of an extractive fermentation process by removing undissolved solids from the corn mash prior to fermentation. Example 6 described two extractive fermentations conducted side-by side, one with liquefied corn mash as the sugar source (2010Y036 - solids not removed) and one with liquefied mash centrate (2010Y035 - aqueous solution of oligosaccharides) obtained by removing most of the undissolved solids from liquefied corn mash. Oleyl alcohol (OA) was added to both fermentations to extract the product isobutanol (*i*-BuOH) from the broth as it was formed. The amount of residual solvent trapped in the undissolved solids recovered from the final fermentation broths was measured and compared.

After completion of the fermentations 2010Y035 and 2010Y036 described in Example 6, the broths were harvested and used to conduct the phase separation tests. Then the undissolved solids (fines from the corn mash that did not get removed prior to fermentation) were recovered from each broth and analyzed for total extractable oils. The oil recovered from each lot of solids was analyzed for oleyl alcohol and corn oil. The following protocol was followed for both broths:

- The broth was centrifuged to separate the organic, aqueous, and solid phases.
- The organic and aqueous phases were decanted away from the solids leaving a wet cake at the bottom of the centrifuge bottle.
- The wet cake was thoroughly washed with water to remove essentially all of the dissolved solids held up in the cake, such as residual oligosaccharides, glucose, salts, enzymes, etc.
- The washed wet cake was dried in a vacuum oven overnight (house-vacuum at 80°C) to remove essentially all of the water in the cake.

- A portion of the dry solids was thoroughly contacted with hexane in a Soxhlet extractor to remove the oil from the solids.
- The oil recovered from the solids was analyzed by GC to determine the relative amount of oleyl alcohol and corn oil present in the oil recovered from the solids.
- A particle size distribution (PSD) was measured for the solids recovered from both fermentation broths.

The data for the recovery and hexane extraction of the undissolved solids from both fermentation broths is shown in Table 8. The data shows that approximately the same amount of oil was absorbed by the solids (per unit mass of solids) in both fermentations.

Table 8

| Fermentation ID: | 2010Y036 | 2010Y035 |
|---|---|---|
| Solids removed from liquefied mash before fermentation | No (mash) | Yes (centrate) |
| Washed wet cake recovered after removing organic phase, aqueous phase, and washing the wet cake with water, g: | 290.6 g | 15.6 g |
| Dry solids content in washed wet cake, wt%: | 23.6% | 25.8% |
| Dry solids recovered from washed wet cake, g: | 68.1 g | 4.02 g |
| Dry solids charged to Soxhlet, g: | 20.11 g | 3.91 g |
| Dry Content of solids charged to Soxhlet via moisture analysis, wt%: | 97.9% | 98.1% |
| Total oil recovered from Soxhlet hexane extraction, g: | 2.30 g | 0.25 g |
| Oil content of solids (dry solids basis), g oil per g of dry solids: | 0.12 g oil/g dry solids | 0.07 g oil/g dry solids |
| Fraction of oil extracted from solids that is OA (approximate value), wt%: | 76% | 74% |

## Example 10

Recovery of Soluble Starch from a Wet Cake Generated from the Removal of Solids from Liquefied Corn Mash by Washing the Wet Cake with Water - Two Stage Process

This example demonstrated the recovery of soluble starch from a wet cake by washing the cake twice with water, where the cake was generated by centrifuging liquefied mash. Liquefied corn mash was fed to a continuous decanter centrifuge to produce a centrate stream (C-1) and a wet cake (WC-1). The centrate was a relatively solids-free, aqueous solution of soluble starch, and the wet cake was concentrated in solids compared to the feed mash. A portion of the wet cake was mixed with hot water to form a slurry (S-1). The slurry was pumped back through the decanter centrifuge to produce a wash water centrate (C-2) and a washed wet cake (WC-2). C-2 was a relatively solids-free, dilute aqueous solution of soluble starch. The concentration of soluble starch in C-2 was less than the concentration of soluble starch in the centrate produced from the separation of mash. The liquid phase held up in WC-2 was more dilute in starch than the liquid in the wet cake produced from the separation of mash. The washed wet cake (WC-2) was mixed with hot water to form a slurry (S-2). The ratio of water charged to the amount of soluble starch in the wet cake charged was the same in both wash steps. The second wash slurry was pumped back through the decanter centrifuge to produce a second wash water centrate (C-3) and a wet cake (WC-3) that had been washed twice. C-3 was a relatively solids-free, dilute aqueous solution of soluble starch. The concentration of soluble starch in C-3 was less than the concentration of soluble starch in the centrate produced in the first wash stage (C-2), and thus the liquid phase held up in WC-3 (second washed wet cake) was more dilute in starch than in WC-2 (first washed wet cake). The total soluble starch in the two wash centrates (C-2 and C-3) is the starch that was recovered and could be recycled back to liquefaction. The soluble starch in the liquid held up in the final washed wet cake is much less that in the wet cake produced in the original separation of the mash.

Production of Liquefied Corn Mash

Approximately 3785 L (1000 gallons) of liquefied corn mash was produced in a continuous dry-grind liquefaction system consisting of a hammer mill, slurry mixer, slurry tank, and liquefaction tank. Ground corn, water, and alpha-amylase were fed continuously. The reactors were outfitted with mechanical agitation, temperature control, and pH control using either ammonia or sulfuric acid. The reaction conditions were as follows:

- Hammer mill screen size: 0.2778 cm (7/64")

- Feed Rates to Slurry Mixer

  - Ground Corn: 254 kg/hr (560 lbm/hr) (14.1 wt% moisture)
  - Process Water: 7.53 kg/min (16.6 lbm/min) (200 F)
  - Alpha-Amylase: 61 g/hr (Genecor: Spezyme® ALPHA)

- Slurry Tank Conditions:

  - Temperature: 185°F (85°C)
  - pH: 5.8
  - Residence Time: 0.5 hr
  - Dry Corn Loading: 31 wt%
  - Enzyme Loading: 0.028 wt% (dry corn basis)

- Liquefaction Tank Conditions:

  - Temperature: 185°F (85°C)
  - pH: 5.8
  - Residence Time: about 3 hr
  - No additional enzyme added.

The production rate of liquefied corn mash was about 0.189 L/s (3 gpm). The starch content of the ground corn was measured to be about 70 wt% on a dry corn basis. The total solids (TS) of the liquefied mash was about 31 wt%, and the total suspended solids (TSS) was approximately 7 wt%. The liquid phase contained about 23-24 wt% liquefied starch as measured by HPLC (soluble oligosaccharides).

The liquefied mash was centrifuged in a continuous decanter centrifuge at the following conditions:

- Bowl Speed: 5000 rpm (about 3600 g's)
- Differential Speed: 15 rpm
- Weir Diameter: 185 mm (weir plate removed)
- Feed Rate: Varied from 0.315-1.262 L/s (5-20 gpm)

Approximately 3218 L (850 gal) of centrate and approximately 635.0 kg (1400 lbm) of wet cake were produced by centrifuging the mash. The total solids in the wet cake were measured to be about 46.3% (suspended + dissolved) by moisture balance. Knowing that the liquid phase contained about 23 wt% soluble starch, it was estimated that the total suspended solids in the wet cake was about 28 wt%. It was estimated that the wet cake contained approximately 12% of the soluble starch that was present in the liquefied mash prior to the centrifuge operation.

Recovery of Soluble Starch from Wet Cake by Washing the Solids with Water - 1st Wash

About 320.7 kg (707 lbm) of the wet cake recovered from separation of the liquefied mash was mixed with 624.6 L (165 gal) of hot (91°C) water in a 1136 L (300 gallon) stainless steel vessel. The resulting slurry was mixed for about 30 min. The slurry was continuously fed to a decanter centrifuge to remove the washed solids from the slurry. The centrifuge used to separate the wash slurry was the same one used to remove solids from the liquefied mash above, and it was rinsed with fresh water before feeding the slurry. The centrifuge was operated at the following conditions to remove solids from the wash slurry:

- Bowl Speed: 5000 rpm (about 3600 g's)
- Differential Speed: 5 rpm
- Weir Diameter: 185 mm (weir plate removed)

(continued)

• Feed Rate: 0.315 L/s (5 gpm).

Approximately 272.2 kg (600 lbm) of washed wet cake was produced by the centrifuge, but only 181.4 kg (400 lbm) were recovered due to loss of material. The total solids in the wet cake were measured to be about 36.7% (suspended + dissolved) by moisture balance. The total soluble starch (sum of glucose, DP2, DP3, and DP4+) in the liquid phase of the slurry and in the wash water centrate (obtained from the slurry) was measured to be about 6.7 wt% and 6.9 wt%, respectively, by HPLC. DP2 refers to a dextrose polymer containing two glucose units (glucose dimer). DP3 refers to a dextrose polymer containing three glucose units (glucose trimer). DP4+ refers to a dextrose polymer containing four or more glucose units (glucose tetramer and higher). This confirmed that a well-mixed dilution wash stage was achieved. Therefore, the concentration of soluble starch in the liquid phase held up in the washed wet cake must have been about 6.8 wt% (by mass balance) for this dilution wash. Based on the total solids and dissolved oligosaccharide data, it was estimated that the total suspended solids in the washed wet cake was about 32 wt%. It was estimated that the washed wet cake contained approximately 2.6% of the soluble starch that was present in the original liquefied mash if all 272.2 kg (600 lbm) of the cake produced by the centrifuge could have been washed. This represents about a 78% reduction in soluble starch in the washed wet cake compared to the mash wet cake prior to washing. If the wet cake produced from the separation of liquefied mash was not washed, about 12% of the total starch in the mash would be lost as soluble (liquefied) starch. If the wet cake produced from the separation of mash is washed with water at the conditions demonstrated in this example, 2.6% of the total starch from the mash would be lost as soluble (liquefied) starch.

About 181.4 kg (400 lbm) of the washed wet cake recovered from the first re-slurry wash of the liquefied mash wet cake was mixed with 416.4 L (110 gal) of hot (90°C) water in a 1136 L (300 gallon) stainless steel vessel. The resulting slurry was mixed for about 30 min. The slurry was continuously fed to a decanter centrifuge to remove the washed solids from the slurry. The centrifuge used to separate the second wash slurry was the same one used in the first wash above, and it was rinsed with fresh water before feeding the second wash slurry. The centrifuge was operated at the following conditions to remove solids from the wash slurry:

• Bowl Speed: 5000 rpm (about 3600 g's)
• Differential Speed: 5 rpm
• Weir Diameter: 185 mm (weir plate removed)
• Feed Rate: 0.252 L/s (4 gpm).

Approximately 146.1 kg (322 lbm) of washed wet cake was produced by the centrifuge. The total solids in the wet cake from the second wash were measured to be about 37.4% (suspended + dissolved) by moisture balance. The total soluble starch (sum of glucose, DP2, DP3, and DP4+) in the liquid phase of the slurry and in the wash water centrate (obtained from the slurry) was measured to be about 1.6 wt% and 1.6 wt%, respectively, by HPLC. This confirmed that a well-mixed dilution wash stage was achieved in the second wash. Therefore, the concentration of soluble starch in the liquid phase held up in the washed wet cake must have been about 1.6 wt% (by mass balance) for this dilution wash. Based on the total solids and dissolved oligosaccharide data, it was estimated that the total suspended solids in the washed wet cake was about 36 wt%. It was estimated that the washed wet cake contained approximately 0.5% of the soluble starch that was present in the original liquefied mash if all 272.2 kg (600 lbm) of the cake produced in the first wash stage could have been washed. This represents an overall reduction in soluble starch in the doubly washed wet cake compared to the mash wet cake prior to washing of about 96%. If the wet cake produced from the separation of liquefied mash was not washed, about 12% of the total starch in the mash would be lost as soluble (liquefied) starch. If the wet cake produced from the separation of mash is washed twice with water at the conditions demonstrated in this example, 0.5% of the total starch from the mash would be lost as soluble (liquefied) starch.

## Example 11

### Effect of High Temperature Stage During Liquefaction on the Conversion of Starch in Corn Solids to Soluble (Liquefied) Starch

This example demonstrates that operating liquefaction with a high temperature (or "cook") stage at some time in the middle of the reaction can result in higher conversion of the starch in corn solids to soluble (liquefied) starch. The "cook" stage demonstrated in this example involves raising the liquefaction temperature at some point after liquefaction starts, holding at the higher temperature for some period of time, and then lowering the temperature back to the original value to complete liquefaction.

A. Procedure to Measure Unhydrolyzed Starch Remaining in Solids after Liquefaction

Liquefied corn mash was prepared in one run according to the protocol in Example 1 (no intermediate high temperature stage). Liquefied corn mash was prepared in another run at the same conditions as in the first run except for the addition of an intermediate high temperature stage. The mash from both runs was worked up according to the following steps. It was centrifuged to separate the aqueous solution of liquefied starch from the undissolved solids. The aqueous solution of liquefied starch was decanted off to recover the wet cake. The wet cake contained most of the undissolved solids from the mash, but the solids were still wet with liquefied starch solution. The wet cake was thoroughly washed with water, and the subsequent slurry was centrifuged to separate the aqueous layer from the undissolved solids. The cake was washed a total of five times with enough water to remove approximately all of the soluble starch that was held up in the original wet cake recovered from liquefaction. Consequently, the liquid phase held up in the final washed wet cake consisted of water containing essentially no soluble starch.

The final washed wet cake was re-slurried in water, and large excesses of both alpha-amylase and glucoamylase were added. The slurry was mixed for at least 24 hr while controlling temperature and pH to enable the alpha-amylase to convert essentially all the unhydrolyzed starch remaining in the undissolved solids to soluble oligosaccharides. The soluble oligosaccharides generated from the residual starch (which was not hydrolyzed during liquefaction at the conditions of interest) were subsequently converted to glucose by the glucoamylase present. Glucose concentration was tracked with time by HPLC to make sure all the oligosaccharides generated from the residual starch were converted to glucose and that the glucose concentration was no longer increasing with time.

B. Production of Liquefied Corn Mash

Two batches of liquefied corn mash were prepared (approximately 1 L each) at 85°C using Liquozyme® SC DS (alpha-amylase from Novozymes, Franklinton, NC). Both batches operated at 85°C for a little more than 2 hr. However, a "cook" period was added in the middle of the second batch ("Batch 2"). The temperature profile for Batch 2 was about 30 min at 85°C, raising the temperature from 85°C to 101°C, holding at 101°C for about 30 min, cooling down to 85°C, and continuing liquefaction for another 120 min. The ground corn used in both batches was the same as in Example 1. A corn loading of 26 wt% (dry corn basis) was used in both batches. The total amount of enzyme used in both runs corresponded to 0.08 wt% (dry corn basis). The pH was controlled at 5.8 during both liquefaction runs. The liquefactions were carried out in a glass, jacketed resin kettle. The kettle was set up with mechanical agitation, temperature control, and pH control.

The following protocol was followed to prepare liquefied corn mash for Batch 1:

- The alpha-amylase was diluted in tap water (0.418 g enzyme in 20.802 g water)
- Charged 704.5 g tap water to the kettle
- Turned on agitator
- Made first charge of ground corn, 198 g
- Heated to 55°C while agitating
- Adjusted pH to 5.8 using $H_2SO_4$ or NaOH
- Made first charge of alpha-amylase solution, 7.111 g
- Heated to 85°C
- Held at 85°C for 30 min
- Made second charge of alpha-amylase solution, 3.501 g
- Made second charge of ground corn, 97.5 g
- Continued to run at 85°C for another 100 min
- After the liquefaction was complete, cooled to 60°C
- Dumped reactor and recovered 998.5 g of liquefied mash.

The following protocol was followed to prepare liquefied corn mash for Batch 2:

- The alpha-amylase was diluted in tap water (0.3366 g enzyme in 16.642 g water)
- Charged 562.6 g tap water to the kettle
- Turned on agitator
- Charged ground corn, 237.5 g
- Heated to 55°C while agitating
- Adjusted pH to 5.8 using dilute $H_2SO_4$ or NaOH
- Made first charge of alpha-amylase solution, 4.25 g
- Heated to 85°C

- Held at 85°C for 30 min
- Heated to 101°C
- Held at 101°C for 30 min
- Lowered temperature of mash back to 85°C
- Adjusted pH to 5.8 using dilute $H_2SO_4$ or NaOH
- Made second charge of alpha-amylase solution, 4.2439 g
- Continued to run at 85°C for another 120 min.
- After the liquefaction was complete, cooled to 60°C.

C. Removal of Undissolved Solids from the Liquefied Mash and Washing of the Wet Cake with Water to Remove Soluble Starch

Most of the solids were removed from both batches of liquefied mash by centrifuging them in a large floor centrifuge at 5000 rpm for 20 min at room temperature. Centrifugation of 500 g of mash from Batch 1 yielded 334.1 g of centrate and 165.9 g of wet cake. Centrifugation of 872 g of mash from Batch 2 yielded 654.7 g of centrate and 217 g of wet cake. The wet cakes recovered from each batch of liquefied mash were washed five times with tap water to remove essentially all of the soluble starch held up in the cakes. The washes were performed in the same bottle used to centrifuge the original mash to avoid transferring the cake between containers. For each wash stage, the cake was mixed with water, and the resulting wash slurry was centrifuged (5000 rpm for 20 min) at room temperature. This was done for all five wash stages performed on the wet cakes recovered from both batches of mash. Approximately 165 g of water was used in each of the five washes of the wet cake from Batch 1 resulting in a total of 828.7 g of water used to wash the wet cake from Batch 1. Approximately 500 g of water was used in each of the five washes of the wet cake from Batch 2 resulting in a total of 2500 g of water used to wash the wet cake from Batch 2. The total wash centrate recovered from all five water washes of the wet cake from Batch 1 was 893.1 g. The total wash centrate recovered from all five water washes of the wet cake from Batch 2 was 2566.3 g. The final washed wet cake recovered from Batch 1 was 101.5 g, and the final washed wet cake recovered from Batch 2 was 151.0 g. The final washed wet cakes obtained from each batch contained essentially no soluble starch; therefore, the liquid held up in each cake was primarily water. The total solids (TS) of the wet cakes was measured using a moisture balance. The total solids of the wet cake from Batch 1 was 21.63 wt%, and the TS for the wet cake from Batch 2 was 23.66 wt%.

D. Liquefaction/Saccharification of Washed Wet Cake to Determine the Level of Unhydrolyzed Starch Remaining in the Undissolved Solids after Liquefaction

The level of unhydrolyzed starch remaining in the solids present in both washed wet cakes was measured by re-slurrying the cakes in water and adding excess alpha-amylase and excess glucoamylase. The alpha-amylase converts residual unhydrolyzed starch in the solids to soluble oligosaccharides which dissolve in the aqueous phase of the slurry. The glucoamylase subsequently converts the soluble oligosaccharides generated by the alpha-amylase to glucose. The reactions were run at 55°C (maximum recommended temperature for the glucoamylase) for at least 24 hr to ensure all of the residual starch in the solids was converted to soluble oligosaccharides and that all the soluble oligosaccharides were converted to glucose. The residual unhydrolyzed starch that was in the solids, which is the starch that did not get hydrolyzed during liquefaction, can be calculated from the amount of glucose generated by this procedure.

The alpha-amylase and glucoamylase enzymes used in the following protocols were Liquozyme® SC DS and Spirizyme® Fuel, respectively (Novozymes, Franklinton, NC). The vessel used to treat the washed wet cakes was a 250 mL jacketed glass resin kettle equipped with mechanical agitation, temperature control, and pH control. The amount of Liquozyme® used corresponds to an enzyme loading of 0.08 wt% on a "dry corn basis." The amount of Spirizyme® used corresponds to an enzyme loading of 0.2 wt% on a "dry corn basis." This basis is defined as the amount of ground corn required to give the amount of undissolved solids held up in the washed cakes assuming all the starch is hydrolyzed to soluble oligosaccharides. The undissolved solids held up in the washed cakes are considered to be mostly the non-starch, non-fermentable part of the corn. These enzyme loadings are at least four times higher than is required to give complete liquefaction and saccharification at 26% corn loading. The enzymes were used in large excess to ensure complete hydrolysis of the residual starch in the solids and complete conversion of the oligosaccharides to glucose.

The following protocol was followed to determine the level of unhydrolyzed starch in the solids present in the washed wet cake from Batch 1 mash:

- The alpha-amylase was diluted in tap water (0.1297 g enzyme in 10.3607 g water)
- The glucoamylase was diluted in tap water (0.3212 g enzyme in 15.6054 g water)
- Charged 132 g tap water to the kettle
- Turned on agitator

- Charged 68 g of the washed wet cake produced from liquefaction Batch 1 (TS=21.63 wt%)
- Heated to 55°C while agitating
- Adjusted pH to 5.5 using dilute $H_2SO_4$ or NaOH
- Charged alpha-amylase solution, 3.4992 g
- Charged glucoamylase solution, 5.319 g
- Run at 55°C for 24 hr while controlling pH at 5.5 and periodically sample the slurry for glucose.

The following protocol was followed to determine the level of unhydrolyzed starch in the solids present in the washed wet cake from Batch 2.

- The alpha-amylase was diluted in tap water (0.2384 g enzyme in 11.709 g water)
- The glucoamylase was diluted in tap water (0.3509 g enzyme in 17.5538 g water)
- Charged 154.3 g tap water to the kettle
- Turned on agitator
- Charged 70.7 g of the washed wet cake produced from liquefaction Batch 1 (TS=23.66 wt%)
- Heated to 55°C while agitating
- Adjusted pH to 5.5 using dilute $H_2SO_4$ or NaOH
- Charged alpha-amylase solution, 2.393 g
- Charged glucoamylase solution, 5.9701 g
- Run at 55°C for 24 hr while controlling pH at 5.5 and periodically sample the slurry for glucose.

Comparison of Results for the Liquefaction/Saccharification of the Washed Wet Cakes

As described above, the washed wet cakes from Batches 1 and 2 were re-slurried in water, and large excesses of both alpha-amylase and glucoamylase were added to the slurries in order to hydrolyze any starch remaining in the solids and convert it to glucose. Figure 15 shows the concentration of glucose in the aqueous phase of the slurries as a function of time.

The glucose concentration increased with time and leveled out at a maximum value at approximately 24 hr for both reactions. The slight decrease in glucose between 24 and 48 hr could have been from microbial contamination; therefore, the maximum level of glucose reached in each system was used to calculate the level of residual unhydrolyzed starch that was in the solids of the washed wet cake. The maximum level of glucose reached by reacting (in the presence of alpha-amylase and glucoamylase) the washed wet cake obtained from the Batch 1 liquefaction was 4.48 g/L. By comparison, the maximum level of glucose reached by reacting (in the presence of alpha-amylase and glucoamylase) the washed wet cake obtained from the Batch 2 liquefaction was 2.39 g/L.

The level of residual unhydrolyzed starch that was in the undissolved solids in the liquefied mash (that did not get hydrolyzed during liquefaction) was calculated based on the glucose data obtained from the washed wet cake obtained from the corresponding batch of mash.

- Liquefaction Batch 1: The residual unhydrolyzed starch in the solids corresponds to 2.1% of the total starch in the corn fed to liquefaction. This implies that 2.1% of the starch in the corn was not hydrolyzed during Batch 1 liquefaction conditions. No intermediate high temperature ("cook") stage occurred during liquefaction Batch 1.
- Liquefaction Batch 2: The residual unhydrolyzed starch in the solids corresponds to 1.1% of the total starch in the corn fed to liquefaction. This implies that 1.1% of the starch in the corn was not hydro lyzed during Batch 2 liquefaction conditions. A high temperature ("cook") stage did occur during liquefaction Batch 2.

This example demonstrates that the addition of a high temperature "cook" stage at some time during the liquefaction could result in higher starch conversion. This will result in less residual unhydrolyzed starch remaining in the undissolved solids in the liquefied corn mash and will lead to less starch loss in a process where undissolved solids are removed from the mash prior to liquefaction.

## Example 12

Effect of High Temperature Stage During Liquefaction on the Conversion of Starch in Corn Solids to Soluble (Liquefied) Starch

Two batches of liquefied corn mash (Batch 3 and Batch 4) were prepared at 85°C using Liquozyme® SC DS (alpha-amylase from Novozymes, Franklinton, NC). Both batches operated at 85°C for a little more than 2 hr. However, a "cook" period was added in the middle of Batch 4. The temperature profile for Batch 4 was about 30 min at 85°C, raising the temperature from 85°C to 121°C, holding at 121°C for about 30 min, cooling down to 85°C, and continuing liquefaction for another 90 min. The ground corn used in both batches was the same as in Example 1. A corn loading of 26 wt% (dry corn basis) was used in both batches. The total amount of enzyme used in both runs corresponded to 0.04 wt% (dry corn basis). The pH was controlled at 5.8 during both liquefaction runs. The liquefaction for Batch 3 was carried out in a 1L glass, jacketed resin kettle, and the liquefaction for Batch 4 was carried out in a 200L stainless steel fermentor. Both reactors were outfitted with mechanical agitation, temperature control, and pH control.

The experimental conditions for this example were similar to those described for Example 9 with the following differences:

For the Production of Liquefied Corn Mash for Batch 3: 0.211 g of alpha-amylase was diluted in 10.403 g tap water. The first charge of alpha-amylase solution was 3.556 g. The second charge of alpha-amylase solution was 1.755 g and the reaction was allowed to continue to run at 85°C for another 90 min.

For the Production of Liquefied Corn Mash for Batch 4: 22 g of alpha-amylase was diluted in 2 kg tap water, 147.9 kg of tap water was charged to the fermentor, and 61.8 kg of ground corn was charged. The first charge of alpha-amylase solution was 1.0 kg, the reaction was heated to 85°C and held at 85°C for 30 min, then the reaction was heated to 121°C and held at 121°C for 30 min. The second charge of alpha-amylase solution was 1 kg and the reaction was allowed to continue to run at 85°C for another 90 min.

Removal of Undissolved Solids from the Liquefied Mash and Washing of the Wet Cake with Water to Remove Soluble Starch

Most of the solids were removed from both batches of liquefied mash by centrifuging them in a large floor centrifuge at 5000 rpm for 15 min at room temperature. Centrifugation of 500.1 g of mash from Batch 3 yielded 337.2 g of centrate and 162.9 g of wet cake. Centrifugation of 509.7 g of mash from Batch 4 yielded 346.3 g of centrate and 163.4 g of wet cake. The wet cakes recovered from each batch of liquefied mash were washed five times with tap water to remove essentially all of the soluble starch held up in the cakes. The washes were performed in the same bottle used to centrifuge the original mash to avoid transferring the cake between containers. For each wash stage, the cake was mixed with water, and the resulting wash slurry was centrifuged (5000 rpm for 15 min) at room temperature. This was done for all five wash stages performed on the wet cakes recovered from both batches of mash. Approximately 164 g of water was used in each of the five washes of the wet cake from Batch 3 resulting in a total of 819.8 g of water used to wash the wet cake from Batch 3. Approximately 400 g of water was used in each of the five washes of the wet cake from Batch 4 resulting in a total of 2000 g of water used to wash the wet cake from Batch 4. The total wash centrate recovered from all five water washes of the wet cake from Batch 3 was 879.5 g. The total wash centrate recovered from all five water washes of the wet cake from Batch 4 was 2048.8 g. The final washed wet cake recovered from Batch 3 was 103.2 g, and the final washed wet cake recovered from Batch 4 was 114.6 g. The final washed wet cakes obtained from each batch contained essentially no soluble starch; therefore, the liquid held up in each cake was primarily water. The total solids (TS) of the wet cakes were measured using a moisture balance. The total solids of the wet cake from Batch 3 was 21.88 wt%, and the TS for the wet cake from Batch 4 was 18.1 wt%.

The experimental conditions for this example were similar to those described for Example 9 with the following differences:

For the Liquefaction/Saccharification of Washed Wet Cake to Determine the Level of Unhydrolyzed Starch Remaining in the Undissolved Solids after Liquefaction for Batch 3: 68 g of the washed wet cake produced from liquefaction of Batch 3 was charged (TS = 21.88 wt%). 3.4984 g of alpha-amylase solution and 5.3042 g of glucoamylase was charged. The reaction was ran at 55°C for 47 hr while controlling pH at 5.5 and periodically sampling the slurry for glucose.

For the Liquefaction/Saccharification of Washed Wet Cake to Determine the Level of Unhydrolyzed Starch Remaining in the Undissolved Solids after Liquefaction for Batch 4: 0.1663 g of alpha-amylase was diluted in 13.8139 g tap water, and 0.213 g of glucoamylase was diluted in 20.8002 g tap water. 117.8 g of tap water was charged to the kettle. 82.24 g of the washed wet cake produced from liquefaction of Batch 4 was charged (TS = 18.1 wt%). 3.4952 g of alpha-amylase solution and 10.510 g of glucoamylase was charged. The reaction was ran at 55°C for 50 hr while controlling pH at 5.5 and periodically sampling the slurry for glucose.

Comparison of Results for the Liquefaction/Saccharification of the Washed Wet Cakes

As described above, the washed wet cakes from Batches 3 and 4 were re-slurried in water, and large excesses of both alpha-amylase and glucoamylase were added to the slurries in order to hydrolyze any starch remaining in the solids and convert it to glucose. Figure 16 shows the concentration of glucose in the aqueous phase of the slurries as a function of time.

The glucose concentration increased with time and leveled out at a maximum value at approximately 26 hr for the washed wet cake from Batch 3. For the Batch 4 washed wet cake, the glucose concentration continued to increase slightly between 24 hr and 47 hr. It is assumed that the glucose concentration measured at 47 hr for the Batch 4 wet cake is approximately equal to the maximum value. The maximum level of glucose reached by reacting (in the presence of alpha-amylase and glucoamylase) the washed wet cake obtained from the Batch 3 liquefaction was 8.33 g/L. By comparison, the maximum level of glucose reached by reacting (in the presence of alpha-amylase and glucoamylase) the washed wet cake obtained from the Batch 4 liquefaction was 4.92 g/L.

The level of residual unhydrolyzed starch that was in the undissolved solids in the liquefied mash (that did not get hydrolyzed during liquefaction) was calculated based on the glucose data obtained from "hydrolyzing" the washed wet cake (in the presence of excess alpha-amylase and glucoamylase) obtained from the corresponding batch of mash.

- Liquefaction Batch 3: The residual unhydrolyzed starch in the solids corresponds to 3.8% of the total starch in the corn fed to liquefaction. This implies that 3.8% of the starch in the corn was not hydrolyzed during Batch 3 liquefaction conditions. No intermediate high temperature ("cook") stage occurred during liquefaction Batch 3.
- Liquefaction Batch 4: The residual unhydrolyzed starch in the solids corresponds to 2.2% of the total starch in the corn fed to liquefaction. This implies that 2.2% of the starch in the corn was not hydrolyzed during Batch 4 liquefaction conditions. A high temperature ("cook") stage did occur during liquefaction Batch 4.

This example demonstrates that the addition of a high temperature "cook" stage at some time during the liquefaction could result in higher starch conversion. This will result in less residual unhydrolyzed starch remaining in the undissolved solids in the liquefied corn mash and will lead to less starch loss in a process where undissolved solids are removed from the mash prior to liquefaction.

Summary and Comparison of Examples 11 and 12

Liquefaction conditions can influence the conversion of starch in the corn solids to soluble (liquefied) starch. Possible liquefaction conditions that could affect the conversion of starch in the ground corn to soluble starch are temperature, enzyme (alpha-amylase) loading, and +/- a high temperature ("cook") stage occurs at some time during liquefaction. Examples 11 and 12 demonstrated that implementing a high temperature ("cook") stage at some time during liquefaction can result in higher conversion of starch in the corn solids to soluble (liquefied) starch. The high temperature stage in the liquefactions described in Examples 11 and 12 involved raising the liquefaction temperature at some point after liquefaction starts, holding at the higher temperature for some period of time, and then lowering the temperature back to the original value to complete liquefaction.

The liquefaction reactions compared in Example 11 were run at a different enzyme loading than the reactions compared in Example 12. These examples demonstrate the effect of two key liquefaction conditions on starch conversion: (1) enzyme loading, and (2) +/- a high temperature stage is applied at some time during liquefaction.

The conditions used to prepare the four batches of liquefied corn mash described in Examples 11 and 12 are summarized below and in Table 9.

Conditions common for all batches:

- Liquefaction temperature - 85°C
- Total time at liquefaction temperature - approximately 2 hr
- Screen size used to grind corn - 1 mm
- pH - 5.8
- Dry corn loading - 26%
- Alpha-amylase - Liquozyme® SC DS (Novozymes, Franklinton, NC).

Table 9

| | Batch 1 | Batch 2 | Batch 3 | Batch 4 |
|---|---|---|---|---|
| Described in Example: | 11 | 11 | 12 | 12 |
| High Temperature Stage Implemented | No | Yes | No | Yes |
| Temperature of High Temperature Stage, °C: | NA | 101°C | NA | 121°C |
| Total Enzyme Loading, wt% (dry corn basis): | 0.08% | 0.08% | 0.04% | 0.04% |
| Residual Unhydrolyzed Starch in Undissolved Solids after Liquefaction (as a percentage of total starch in corn feed): | 2.1% | 1.1% | 3.8% | 2.3% |

The temperature profile for Batches 2 and 4 was (all values are approximate): 85°C for 30 min, High Temperature Stage for 30 min, 85°C for 90 min. Half the enzyme was added before the initial 85°C period, and half was added after the high temperature stage for the final 85°C period.

Figure 17 illustrates the effect of enzyme loading and +/- a high temperature stage was applied at some time during the liquefaction on starch conversion. The level of residual unhydrolyzed starch in the solids is the starch that was not hydrolyzed during the liquefaction conditions of interest. Figure 17 shows that the level of unhydrolyzed starch in the solids was reduced by almost half by applying a high temperature ("cook") stage at some point during the liquefaction. This was demonstrated at two different enzyme loadings. The data in Figure 17 also shows that doubling the enzyme loading resulted in almost half the level of unhydrolyzed starch remaining in the solids whether a high temperature stage was applied during liquefaction or not. These examples demonstrate that operating liquefaction with a higher enzyme (alpha-amylase) loading and/or the addition of a high temperature ("cook") stage at some time during the reaction could result in a significant reduction in residual unhydrolyzed starch in the undissolved solids present in the liquefied corn mash and can reduce the loss of starch in a process where undissolved solids are removed from the mash prior to liquefaction. Any residual starch in the solids after liquefaction will not have the opportunity to hydrolyze during fermentation in a process where solids are removed prior to fermentation.

## Example 13

Screen Separation of Starch and Nonsolubles following 85°C Enzyme Digestion

Mash (301 grams) prepared per the method described in Example 1 were maintained at pH 5.8 using drops of NaOH solution when adjustment was necessary, treated with a vendor-specified dose of approximately 0.064 grams of Liquozyme® alpha-amylase enzyme (Novozyme, Franklinton, NC) and held at 85°C for five hours. The product was refrigerated.

Refrigerated product was warmed to approximately 50°C and 48 g was poured onto a filter assembly containing a 100 mesh screen and connected to a house vacuum source at between -50796 Pa (-15 in Hg) and -67728 Pa (-20 in Hg). The screen dish had an exposed screen surface area of 44 $cm^2$ and was sealed inside a plastic filter housing provided by Nalgene® (Thermo Fisher Scientific, Rochester, NY). The slurry was filtered to form a wet cake on the screen and a yellow cloudy filtrate of 40.4 g in the receiver bottle. The wet cake was immediately washed in place with water and then discontinued while the vacuum source continued to pull any free moisture through the final washed cake. Filtration was ended when dripping ceased from the underside of the filter. An additional 28.5 g of wash filtrate were collected over 3 stages where the final stage of filtrate revealed the least color and turbidity. The final wet cake mass of 7.6 g was air dried to 2.1 g over 24 hours at room temperature. The 2.1 g were determined to contain 7.73% water after drying with a heat lamp. The vacuum filtration of this experiment produced a wet cake containing 25% total dry solids.

A sample of filtrate was combined with oleyl alcohol at room temperature, vigorously mixed and allowed to settle. The interface was restored in approximately 15 min but a hazy rag layer remained.

Lugol's solution (starch indicator) consisting of 1 g of >99.99% (trace metals basis) iodine, 2 g of ReagentPlus® grade (>99%) potassium iodide (both from Sigma-Aldrich, St. Louis, MO), and 17 g of house deionized water in the amount of one drop was added to samples of the filtrate, dried cake solids re-slurried in water and a control sample of water. The filtrate turned dark blue or purple, the solids slurry turned very dark blue and the water became light amber in color. Any color darker than amber indicates presence of oligosaccharides greater than 12 units long.

This experiment illustrated that most suspended solids could be separated from starch solution prepared as described above at a moderate rate on a 100 mesh screen and that starch remains with the filter cake solids. This is an indication of incomplete washing of the cake where a portion of hydrolyzed starch is left behind.

This experiment was repeated with 156 grams of mash on a 63 mm diameter 100 mesh screen. The maximum temperature was 102°C, the enzyme was Spezyme® and the slurry was held above 85°C for three hours. The screening rate was measured and determined to be 0.002716 or less L/s/$m^2$ (0.004 or less gallons per minute per square foot of screen area).

## Example 14

Screen Separation of Starch and Nonsolubles following 115°C Enzyme Digestion

House deionized water (200 g) were charged into an open Parr Model 4635 1 liter pressure vessel (Moline, IL) and heated to a temperature of 85°C. The water was agitated with a magnetic stir bar. Dry ground corn (90 g) prepared as described in Example 1 were added spoon-wise. The pH was raised from 5.2 to near 6.0 with stock aqueous ammonia solution. Approximately 0.064 grams of Liquozyme® solution were added with a small calibrated pipette. The lid of the pressure vessel was sealed and the vessel was pressurized to 344738 Pa (50 psig) with house nitrogen. The agitated mixture was heated to 110°C within 6 min and held between 106 to 116°C for a total of 20 min. The heating was reduced, the pressure was relieved, and the vessel was opened. An additional 0.064 g of Liquozyme® was added and the temperature was held at 63-75°C for an additional 142 min.

A small amount of the slurry was taken from the Parr vessel and gravity screened through a stack of 100, 140, and 170 mesh screens. Solids were retained only on the 100 mesh screen.

A portion, about 40%, of the slurry was transferred while hot onto the top of a dual screen assembly of 100 and 200 mesh dishes of 75 millimeter diameter. Some gravity filtration took place. Vacuum, between -50796 and -67728 Pa (-15 and -20 inches of mercury), was pulled on the filtrate receiver and steady filtration was established. The filtrate was yellow and cloudy but with a stable dispersion. The cake surface was exposed within 5 min. The cake was washed with a spray of deionized water for 2-3 min and repeated with a change of receiver until the turbidity of the filtrate was constant - a total of five sprayings. The screens were examined with the conclusion that all solids were on the 100 mesh screen and none were on the 200 mesh. The wet cake was 5 mm thick. The wet cake mass was determined to be 18.9 g and the combined filtrate masses were 192 g.

The remaining mass of slurry was transferred to the filter assembly with a 100 mesh screen in place at 65°C and filtered over 5-10 min. The cake was washed with a spray of deionized water for 3-4 min and repeated with a change of receiver until the turbidity of the filtrate was constant - a total of eight sprayings. Vacuum was continued until no more drops were observed falling from the underside of the filter. The wet cake was 8 mm thick and 75 mm in diameter with a mass of 36.6 g. The combined filtrates weighed 261 g.

Three vials were tested for starch per the method described above. One vial contained water and the other two contained samples of wet cake slurried in deionized water. All vials turned yellow-amber in color. This was interpreted to mean that the filter cake was washed free of oligosaccharides of starch. These solids were later analyzed rigorously using prolonged liquefaction and subsequent saccharification to confirm that on a glucose basis, the wet cake contained no more than 0.2% of the total starch that was in the original corn.

A sample of filtrate was combined with oleyl alcohol in a vial, vigorously mixed and allowed to settle. A clear oil layer was quickly attained and the interface was well defined with little rag layer. This example illustrated that in a process in which corn mash is heated to hydrothermal conditions of ~110°C for 20 min of cooking and further liquefied for more than two hours at 85°C before being filtered and washed, the total filtrate contains essentially all starch supplied in the grain. Furthermore, no significant interference is observed between the oleyl alcohol and the impurities contained in the filtrate.

This experiment was repeated with 247 grams of mash on a 75 mm diameter 80 mesh screen. The maximum cook temperature was 115°C, the enzyme was Liquozyme® and the slurry was held at or above 85°C for three hours. The screening rate was measured and determined to be more than 0.0679 L/s/m$^2$ (0.1 gallons per minute per square foot of screen area).

## Example 15

This example illustrated the removal of solids from stillage and extraction by desolventizer to recover fatty acids, esters, and triglycerides from the solids. During fermentation, solids are separated from whole stillage and fed to a desolventizer where they are contacted with 0.277 kg/s (1.1 tons/hr) of steam. The flow rates for the whole stillage wet cake (extractor feed), solvent, the extractor miscella, and extractor discharge solids are as shown in Table 10. Table values are kg/s (short tons/hr).

Table 10

| | Solids from whole stillage | Solvent | Miscella | Extractor discharge solids |
|---|---|---|---|---|
| Fatty acids | 0.0249 (0.099) | 0 | 0.0247 (0.0982) | 0.00025 (0.001) |
| Undissolved solids | 4.500 (17.857) | 0 | 0.00023 (0.0009) | 4.500 (17.856) |
| Fatty acid butyl esters | 0.722 (2.866) | 0 | 0.715 (2.837) | 0.00723 (0.0287) |
| Hexane | 0 | 2.78 (11.02) | 2.638 (10.467) | 0.140 (0.555) |
| Triglyceride | 0.250 (0.992) | 0 | 0.247 (0.982) | 0.0025 (0.0099) |
| Water | 7.500 (29.762) | 0 | 7.425 (29.464) | 0.0748 (0.297) |

Solids exiting the desolventizer are fed to a dryer. The vapor exiting the desolventizer contains 0.14 kg/s (0.55 tons/hr) of hexane and 0.278 kg/s (1.102 tons/hr) of water. This stream is condensed and fed to a decanter. The water-rich phase exiting the decanter contains about 360 ppm of hexane. This stream is fed to a distillation column where the hexane is removed from the water-rich stream. The hexane enriched stream exiting the top of the distillation column is condensed and fed to the decanter. The organic-rich stream exiting the decanter is fed to a distillation column. Steam (2.78 kg/s (11.02 tons/hr)) is fed to the bottom of the distillation column. The composition of the overhead and bottom products for this column are shown in Table 11. Table values are kg/s (tons/hr).

Table 11

| | Bottoms | Overheads |
|---|---|---|
| Fatty acids | 0.0247 (0.0981) | 0 |
| Fatty acid butyl esters | 0.7114 (2.8232) | 0 |
| Hexane | 0.00028 (0.0011) | 2.80 (11.12) |
| Triglyceride | 0.247 (0.9812) | 0 |
| Water | 0 | 2.78 (11.02) |

**Example 16**

By-Product Recovery

This example illustrates the recovery of by-products from mash. Corn oil separated from mash under the conditions described in Example 6 with the exception that a three-phase centrifuge (Flottweg Tricanter® Z23-4 bowl diameter, 230 mm, length to diameter ratio 4:1) was used with these conditions:

- Bowl Speed: 5000 rpm
- Differential Speed: 10 rpm
- Feed Rate: 0.189 L/s (3 gpm)
- Phase Separator Disk: 138 mm
- Impeller Setting: 144 mm.

The corn oil separate had 81% triglycerides, 6% free fatty acids, 4% diglyceride, and 5% total of phospholipids and monoglycerides as determined by gas chromatography and thin layer chromatography (see, e.g., U.S. Patent Application Publication No. 2012/0164302).

The solids separated from mash under the conditions described above had a moisture content of 58% as determined by weight loss upon drying and had 1.2% triglycerides and 0.27% free fatty acids as determined by gas chromatography (see, e.g., U.S. Patent Application Publication No. 2012/0164302).

The composition of solids separated from whole stillage, oil extracted between evaporator stages, by-product extractant and Condensed Distillers Solubles (CDS) in Table 14 were calculated assuming the composition of whole stillage shown in Table 12 and the assumptions in Table 13 (separation at three-phase centrifuge). The values of Table 11 were obtained from an Aspen Plus® model (Aspen Technology, Inc., Burlington, MA). This model assumes that corn oil is not extracted from mash. It is estimated that the protein content on a dry basis of cells, dissolved solids, and suspended solids is approximately 50%, 22%, and 35.5%, respectively. The composition of by-product extractant is estimated to be 70.7% fatty acid and 29.3% fatty acid isobutyl ester on a dry basis.

Table 12

| Component | Mass % |
|---|---|
| Water | 57.386% |
| Cells | 0.502% |
| Fatty acids | 6.737% |
| Isobutyl esters of fatty acids | 30.817% |
| Triglyceride | 0.035% |
| Suspended solids | 0.416% |
| Dissolved solids | 4.107% |

Table 13

| | Hydrolyzer feed | Thin stillage | Solids |
|---|---|---|---|
| Organics | 99.175% | 0.75% | 0.08% |
| Water and dissolved solids | 1% | 96% | 3% |
| Suspended solids and cells | 1% | 2% | 97% |

Table 14

| Stream | C. protein | triglyceride | FFA | FABE |
|---|---|---|---|---|
| Whole stillage wet cake | 40% | trace | 0.5% | 2.2% |
| Oil at evaporator | 0% | 0.08% | 16.1% | 73.8% |
| CDS | 22% | trace% | 0.37% | 1.71% |

**Example 17**

Removal of Corn Oil from Liquefied Corn Mash

This example describes the use of a three-phase centrifuge to remove corn oil from liquefied corn mash. Whole corn kernels typically contain about 3-6 wt% corn oil, most of which resides in the germ. Corn oil is released from the germ during dry milling and liquefaction. Consequently, corn mash contains free corn oil.

Liquefied corn mash was generated using a standard continuous liquefaction process as used, for example, in a dry-grind corn-to-ethanol process. The ground corn contained 4.16 wt% corn oil (dry corn basis) and had a moisture content of 14.7 wt%. Ground corn and water were fed to a slurry tank at 0.08 kg/s (10.2 lbm/min) and 0.13 kg/s (17.0 lbm/min), respectively, to give a dry corn loading of 32 wt%. Alpha-amylase was fed to the slurry tank at a rate that corresponded to an enzyme loading of about 0.025 wt% on a dry corn basis. The slurry and liquefaction tanks were both run at 85°C and a pH of 5.8. The total residence time at 85°C was about 2 hr. Mash was produced at a rate of about 0.189 L/s (3 gpm) and contained about 1.3 wt% corn oil on a wet basis. A portion of this oil existed as free oil and a portion was in the undissolved solids. This corresponds to a total corn oil content of the mash to be roughly 25.7 kg/$m^3$ (2.0 lbm of corn oil/bushel of corn). The total solids (TS) in the mash was 32 wt% and the total suspended solids (TSS) was 7.7 wt%.

The liquefied corn mash was fed to a three-phase centrifuge (Model Z23-4/441, Flottweg Tricanter®, Flottweg AG, Vilsibiburg, Germany) at a rate of about 0.189 L/s (3 gpm). The feed temperature was about 80°C. The mash was separated into three streams: (1) corn oil, (2) aqueous solution of oligosaccharides (liquefied starch), and (3) wet cake. The operating conditions of the Tricanter® were as follows:

- Bowl Speed: 5000 rpm
- G-force: approximately 4000 g's
- Differential speed: 10 rpm
- Impeller setting: approximately 145
- Phase separator disk: approximately 138 mm.

Table 15 summarizes data (flow rate, density, solids content, and corn oil content) measured for the feed stream and the three exit streams from the Tricanter®.

Table 15

| | Feed Mash | Aqueous Centrate | Wet Cake | Corn Oil |
|---|---|---|---|---|
| Flow Rate, kg/s (lbm/min): | 0.21 (27.2) | 0.15 (19.5) | 0.06 (7.6) | 0.001 (0.14) |
| Density, g/ml: | 1.1008 | ~1.09 | | 0.875 |
| Total Solids, wt%: | 32.0 | 28.7 | 39.1 | ~0 |
| Total Suspended Solids, wt%: | 7.7 | 4.3 | 16.6 | ~0 |

(continued)

| | Feed Mash | Aqueous Centrate | Wet Cake | Corn Oil |
|---|---|---|---|---|
| Corn Oil Content (wet basis), wt%: | 1.3 | 0.38 | 1.95 | 99.4 * |
| Corn Oil Content, kg/m$^3$ (lbm/bushel): | 25.7 (2.0) | 5.15 (0.4) | 10.3 (0.8) | 10.3 (0.8) |
| % of Corn Oil in feed: | NA | 20 | 41 | 39 |
| * Balance is water | | | | |

The corn oil removed from the mash by the Tricanter® accounted for 39% of the total corn oil in the mash feed. The corn oil removal rate was equal to about 10.3 kg/m$^3$ (0.8 lbm/bushel) of corn. The corn oil separated and recovered from the liquefied corn mash contained about 85 wt% glycerides.

In a process where about 10.3 kg/m$^3$ (0.8 lb corn oil/bushel) of corn is removed, the mash flow rate would decrease by 0.24 L/s (3.9 gallons per minute):

$$37\ \frac{bu\ corn}{min} * 0.8 \frac{lb\ oil}{bu\ corn} / 7.65 \frac{lb\ oil}{gal} = 3.9\ gallons\ oil/minute$$

In a production plant where the total mash flow to fermentation is about 44.1 L/s (700 gpm), the oil that was removed would make about 0.55% of the total mash flow. Assuming that the production plant proportionally raises throughput to take advantage of the extra volume, the yearly production would increase by 0.55%, which means that a 211983060 L/year (56 MMGPY) plant would produce an additional 1173478 L (310,000 gallons) of ethanol.

**Example 18**

Removal of Corn Oil from Liquefied Corn Mash - Feed Rate Adjustment

In this example, liquefied corn mash was fed to a three-phase centrifuge at a feed rate of 0.063 L/s (1 gpm). Liquefied corn mash was generated using a standard continuous liquefaction process as used, for example, in a dry-grind corn-to-ethanol process. The ground corn contained 4.16 wt% corn oil (dry corn basis) and had a moisture content of 14.7 wt%. Ground corn and water were fed to a slurry tank at 0.062 kg/s (8.2 lbm/min) and 0.144 kg/s (19.0 lbm/min), respectively, to give a dry corn loading of approximately 26 wt%. Alpha-amylase was fed to the slurry tank at a rate of 50 g/hr, which corresponded to an enzyme loading of about 0.026 wt% on a dry corn basis. The slurry and liquefaction tanks were both run at 85°C and a pH of 5.8. No jet cooker was used. The total residence time at 85°C was about 2 hr. Mash was produced at a rate of about 0.189 L/s (3 gpm) and inventoried into a 5678 L (1500 gal) tank for centrifuge testing. The mash contained about 1.1 wt% corn oil on a wet basis. A portion of this oil existed as free oil and a portion was in the undissolved solids. This corresponds to a total corn oil content of the mash to be roughly 25.7 kg/m$^3$ (2.0 lbm of corn oil/bushel of corn). The TS in the mash was 25.6 wt% and the TSS was 5.3 wt%.

The liquefied corn mash was fed from a feed tank to a three-phase centrifuge (Model Z23-3, Flottweg Tricanter®, Flottweg AG, Vilsibiburg, Germany) at a rate of about 0.063 L/s (1 gpm). The feed temperature was about 80°C. The mash was separated into three streams: (1) corn oil, (2) aqueous solution of oligosaccharides (liquefied starch), and (3) wet cake. The operating conditions of the Tricanter® were as follows:

- Bowl Speed: 5000 rpm
- G-force: approximately 4000 g's
- Differential speed: 12 rpm
- Impeller setting: approximately 156
- Phase separator disk: approximately 140 mm.

Table 16 summarizes data (flow rate, density, solids content, and corn oil content) measured for the feed stream and the three exit streams from the Tricanter®. The quality of the corn oil mass balance was 102% and the quality of the total solids mass balance was 105%.

Table 16

| | Feed Mash | Aqueous Centrate | Wet Cake | Corn Oil |
|---|---|---|---|---|
| Flow Rate, kg/s (lbm/min): | 0.0696 (9.2) | 0.0469 (6.2) | 0.0227 (3.0) | 0.00012 (0.016) |
| Density, g/ml: | ~1.10 | ~1.09 | | ~0.9 |
| Total Solids, wt%: | 25.6 | 21.6 | 37.4 | ~0 |
| Total Suspended Solids, wt%: | 5.3 | 1.1 | 13.8 | ~0 |
| Corn Oil Content (wet basis), wt%: | 1.1 | 0.28 | 2.2 | >99 * |
| Corn Oil Content, kg/m$^3$ (lbm/bushel): | 25.7 (2.0) | 4.63 (0.36) | 17.25 (1.34) | 4.38 (0.34) |
| % of the Corn Oil in the feed: | NA | 18 | 67 | 17 |
| * Balance is water | | | | |

The corn oil removed from the mash by the Tricanter® accounted for 17% of the total corn oil in the mash feed. This corn oil removal rate was equal to about 4.38 kg/m$^3$ (0.34 lbm/bushel of corn). The corn oil separated and recovered from the liquefied corn mash contained about 81.4 wt% glycerides and 8.3 wt% free fatty acids.

**Example 19**

Removal of Corn Oil from Liquefied Corn Mash - Feed Rate Adjustment

In this example, liquefied corn mash was fed to a three-phase centrifuge at a feed rate of 0.637 L/s (10.1 gpm). Liquefied corn mash was generated using a standard continuous liquefaction process as used, for example, in a dry-grind corn-to-ethanol process. The ground corn contained 4.16 wt% corn oil (dry corn basis) and had a moisture content of 14.7 wt%. Ground corn and water were fed to a slurry tank at 0.062 kg/s (8.2 lbm/min) and 0.144 kg/s (19.0 lbm/min), respectively, to give a dry corn loading of approximately 26 wt%. Alpha-amylase was fed to the slurry tank at a rate of 50 g/hr, which corresponded to an enzyme loading of about 0.026 wt% on a dry corn basis. The slurry and liquefaction tanks were both run at 85°C and a pH of 5.8. No jet cooker was used. The total residence time at 85°C was about 2 hr. Mash was produced at a rate of about 0.189 L/s (3 gpm) and inventoried into a 5678 L (1500 gal) tank for centrifuge testing. The mash contained about 1.1 wt% corn oil on a wet basis. A portion of this oil existed as free oil and a portion was in the undissolved solids. This corresponds to a total corn oil content of the mash to be roughly 25.7 kg/m$^3$ (2.0 lbm of corn oil/bushel of corn). The TS in the mash was 26.2 wt% and the TSS was 6.7 wt%.

The liquefied corn mash was fed from the feed tank to a three-phase centrifuge (Model Z23-4/441, Flottweg Tricanter®, Flottweg AG, Vilsibiburg, Germany) at a rate of about 0.637 L/s (10.1 gpm). The feed temperature was about 80°C. The mash was separated into three streams: (1) corn oil, (2) aqueous solution of oligosaccharides (liquefied starch), and (3) wet cake. The operating conditions of the Tricanter® were as follows:

- Bowl Speed: 5000 rpm
- G-force: approximately 4000 g's
- Differential speed: 20 rpm
- Impeller setting: approximately 148
- Phase separator disk: approximately 138 mm.

Table 17 summarizes data (flow rate, density, solids content, and corn oil content) measured for the feed stream and the three exit streams from the Tricanter®. The quality of the corn oil mass balance was 95%.

Table 17

| | Feed Mash | Aqueous Centrate | Wet Cake | Corn Oil |
|---|---|---|---|---|
| Flow Rate, lbm/min: | 92.2 | 73.1 | 18.9 | 0.177 |
| Density, g/ml: | ~1.10 | ~1.09 | | ~0.9 |
| Total Solids, wt%: | 26.2 | 23.3 | 36.9 | ~0 |
| Total Suspended Solids, wt%: | 6.7 | 1.9 | 25.2 | ~0 |

(continued)

| | Feed Mash | Aqueous Centrate | Wet Cake | Corn Oil |
|---|---|---|---|---|
| Corn Oil Content (wet basis), wt%: | 1.1 | 0.71 | 1.4 | >99 * |
| Corn Oil Content, lbm/bushel: | 2.0 | 1.02 | 0.52 | 0.36 |
| % of the Corn Oil in the feed: | NA | 51 | 26 | 18 |
| * Balance is water | | | | |

The corn oil removed from the mash by the Tricanter® accounted for 18% of the total corn oil in the mash feed. This corn oil removal rate was equal to about 4.63 kg/$m^3$ (0.36 lbm/bushel of corn). The corn oil separated and recovered from the liquefied corn mash contained about 81.4 wt% glycerides and 8.3 wt% free fatty acids.

## Example 20

Effect of Liquefied Corn Mash pH on the Recovery of Corn Oil from Mash

Liquefied corn mash was generated using a standard continuous liquefaction process as typically used in a dry-grind corn-to-ethanol process. The ground corn contained 4.6 wt% corn oil (dry corn basis) and had a moisture content of 12.5 wt%. Ground corn and water were fed to the slurry tank at rates produce corn mash at 0.189 L/s (3 gpm) with a dry corn loading of 25.9 wt%. The slurry tank was operated at 85°C with a 30 min residence time. The slurry was then heated to 105°C using live steam in a jet cooker and held at that temperature for about 30 min. After exiting the hold tube, the slurry was fed into a liquefaction tank which was operated at 85°C with a 90 min residence time. Alpha-amylase (Spezyme® ALPHA, Genencor®, Palo Alto, CA) was continuously fed to the process at a rate that corresponded to an overall enzyme loading of 0.04 wt% enzyme on a dry corn basis. Forty percent (40%) of the total enzyme was added to the slurry tank, and 60% was added to the liquefaction tank. The slurry and liquefaction tanks were both run at a pH of 5.8. Mash was produced at a rate of about 0.189 L/s (3 gpm) and inventoried into a 5678 L (1500 gal) tank for centrifuge testing. The liquefied corn mash contained about 1.12 wt% corn oil on a wet basis. This corresponds to a total corn oil content of the mash to be roughly 28.3 kg/$m^3$ (2.2 lbm of corn oil/bushel of corn). Some of this oil existed as free oil; some still was in the undissolved solids. The ratio of glycerides to free fatty acids in the corn oil in the mash was about 7.6 to 1. The total solids (TS) in the mash were 25.9 wt%, and the total suspended solids (TSS) were 4.7 wt%. The DE (dextrose equivalent) and the pH of the final mash was 15.9 and 5.75, respectively. The density of the mash was 1.08 g/mL.

The liquefied mash was separated using a three-phase centrifuge (Model Z23-4/441, Flottweg Tricanter®, Flottweg AG, Vilsibiburg, Germany) at three different feed flow rates: 0.078 L/s (1.24 gal/min), 0.322 L/s (5.1 gal/min) and 0.631 L/s (10 gal/min). The feed temperature was about 80°C. The mash was separated into three streams: (1) corn oil, (2) aqueous solution of oligosaccharides (liquefied starch), and (3) wet cake. The bowl speed was held constant at about 5000 rpm (approximately 4000 g's). Table 18 compares the corn oil recovery as a function of mash feed rate to the Tricanter® for a mash pH of 5.8. The data shown in Table 18 shows that there is an effect of feed rate to the Tricanter® on the recovery rate of corn oil at pH=5.8.

Table 18

| Test | Mash Feed Rate, L/s (gpm) | Differential Speed, rpm | Impeller Setting, mm | Corn Oil in Mash, g/min | Corn Oil Recovered, g/min | Corn Oil Recovery % |
|---|---|---|---|---|---|---|
| A | 0.076 (1.2) | 5.2 | 144 | 63.3 | 8.3 | 13 |
| B | 0.322 (5.1) | 10.5 | 146 | 248.4 | 73.2 | 29 |
| C | 0.631 (10) | 9.8 | 149 | 487.1 | 100.3 | 21 |

Corn oil recovery is based on the total oil contained in the mash (both free oil and oil in the solids). The mash fed to the Tricanter® contained 1.1-1.2 wt% corn oil (includes free oil and oil in the solids).

The data in Table 18 shows that there is an effect of mash feed rate on corn oil recovery rate (at the conditions tested). Table 19 summarizes the amount of oil phase in the aqueous centrate, aqueous phase in the oil centrate, and solids in the oil centrate for the three conditions tested.

Table 19

| Test | Mash Feed Rate, L/s (gpm) | Corn Oil Recovery % | Corn Oil in Aqueous Centrate, vol%* | Aqueous Phase in Corn Oil, vol%* | Solids in Corn Oil, vol%* | Density of Corn Oil, g/mL |
|---|---|---|---|---|---|---|
| A | 0.076 (1.2) | 13 | 0 | 0 | 1.8 | 0.892 |
| B | 0.322 (5.1) | 29 | 0 | 0 | 1.7 | 0.892 |
| C | 0.631 (10) | 21 | 0 | 3 | 1.5 | 0.906 |
| * Measured using a LuMiSizer® (L.U.M. GmbH, Berlin, Germany) | | | | | | |

The data in Table 19 shows that the corn oil recovered was fairly clean since it contained very little aqueous phase and very little solids. The corn oil separated and recovered from the liquefied corn mash contained about 85.1 wt% glycerides and 8.0 wt% free fatty acids. The balance was solids, aqueous phase, and other extractables (e.g. phospholipids, sterols, etc.).

The pH of the remaining liquefied corn mash in the Tricanter® feed tank was lowered to about 3. The acidic mash was separated using a three-phase centrifuge (Model Z23-4/441, Flottweg Tricanter®, Flottweg AG, Vilsibiburg, Germany) at two different feed flow rates: 0.082 L/s and 0.315 L/s (1.3 gal/min and 5 gal/min). The feed temperature was about 80°C. The mash was separated into three streams: (1) corn oil, (2) aqueous solution of oligosaccharides (liquefied starch), and (3) wet cake. The bowl speed was held constant at about 5000 rpm (approximately 4000 g's). Table 20 compares the corn oil recovery as a function of mash feed rate to the Tricanter® for a mash pH of 3.0.

Table 20

| Test | Mash Feed Rate, L/s (gpm) | Differential Speed, rpm | Impeller Setting, mm | Corn Oil in Mash, g/min | Corn Oil Recovered, g/min | Corn Oil Recovery % |
|---|---|---|---|---|---|---|
| D | 0.082 (1.3) | 5.2 | 144.5 | 47.3 | 20.8 | 44 |
| E | 0.315 (5.0) | 10.3 | 146 | 181.9 | 72.8 | 40 |

Mash was produced at pH=5.8, and the pH of the final mash was then lowered to 3 before feeding the centrifuge. Corn oil recovery is based on the total oil contained in the mash (both free oil and oil in the solids). The mash fed to the Tricanter® contained about 0.9 wt% corn oil (includes free oil and oil in the solids). Total Solids of mash fed to Tricanter® were 27.1 wt%, and Total Suspended Solids of mash were 5.5 wt%.

Table 21 summarizes the amount of oil phase in the aqueous centrate, aqueous phase in the oil centrate, and solids in the oil centrate for the two conditions tested. The data in Table 21 shows that the corn oil recovered was fairly clean since it contained very little aqueous phase and very little solids.

Table 21

| Test | Mash Feed Rate, L/s (gpm) | Corn Oil Recovery % | Corn Oil in Aqueous Centrate, vol%* | Aqueous Phase in Corn Oil, vol%* | Solids in Corn Oil, vol%* | Density of Corn Oil, g/mL |
|---|---|---|---|---|---|---|
| D | 0.082 (1.3) | 44 | 0 | 0.2 | 0 | 0.895 |
| E | 0.315 (5.0) | 40 | 0 | 0 | 0 | 0.895 |
| * Measured using a LuMiSizer® (L.U.M. GmbH, Berlin, Germany) | | | | | | |

Comparing the results of Test A (Table 18) to Test D (Table 20) and comparing the results of Test B (Table 19) to Test E (Table 21) show an effect of mash pH on the corn oil recovery using a Tricanter®. The data suggests that reducing the pH of the mash before separating it with a Tricanter® results in higher corn oil recovery. These comparisons are shown in Table 22.

Table 22

| | pH of Mash fed to Tricanter® | |
|---|---|---|
| Mash Feed Rate L/s (gpm) | pH = 5.8 | pH = 3.0 |
| 0.082 (1.3) | 13% | 44% |
| 0.322 (5.1) | 29% | 40% |

Percentages shown in Table 22 are corn oil recoveries based on the total oil contained in the mash (both free oil and oil in the solids). The composition of corn oil in the mash fed to the Tricanter® ranged from 0.9% to 1.2 wt% corn oil (includes free oil and oil in the solids) for all the tests described in this example. The Tricanter® was operated at 5000 rpm (~4000 G's), and the differential speed and impeller setting were 5-10 rpm and 145 mm, respectively.

## Example 21

Recovery of Corn Oil and Solids from Corn Mash

Liquefied corn mash was generated using a standard continuous liquefaction process as used in a dry-grind corn-to-ethanol process with 30-31 wt% on a dry corn basis. Recycle water consisting of cook water and backset was used, which elevated the total solids (TS) to approximately 33 wt%. Alpha-amylase (Spezyme® RSL, Genencor®, Palo Alto, CA) was added to the slurry tank (85°C, pH approximately 5.8, 30 min residence time) at a rate that corresponded to approximately 0.02 wt% dry corn base enzyme load. A jet cooker was used to elevate the temperature to 105-110°C with 18 min cook time. The liquefaction tank was run at 85°C with a pH of approximately 5.8. Spezyme® RSL (Genencor®, Palo Alto, CA) was also added to the liquefaction tank at a rate that corresponded to approximately 0.005 wt% dry corn base enzyme load, and the total residence time in the liquefaction tank was about 90 min. A side stream of mash was collected from the liquefaction tank and diverted to a small dilution tank, where process condensate was added to achieve the desired dilution. The original mash contained about 1.55 wt% corn oil on a wet basis. A portion of this oil existed as free oil and a portion was in the undissolved solids. This corresponds to a total corn oil content of the original mash to be roughly 38.62 kg/$m^3$ (3.0 lbm of corn oil/bushel of corn). The TS in the original mash was 33.2 wt% and the total suspended solids (TSS) was 6.5 wt%. The dilution with process condensate lowered the TS to approximately 27 wt%, the TSS to approximately 5.5 wt%, and the oil content to approximately 1.3 wt% (wet basis).

The liquefied corn mash was fed from the feed tank to a three-phase centrifuge (Model Z23-4/441, Flottweg Tricanter®, Flottweg AG, Vilsibiburg, Germany) at a rate between 0.57 and 0.69 L/s (9 and 11 gpm). The feed temperature was about 85°C. The mash was separated into three streams: (1) corn oil, (2) aqueous solution of oligosaccharides (liquefied starch), and (3) wet cake. The operating conditions of the three-phase centrifuge were as follows:

- Bowl Speed: 5000 rpm
- G-force: approximately 3200 g
- Differential speed: 25 rpm
- Impeller setting: see table
- Phase separator disk: approximately 138 mm

Table 23 summarizes three-phase centrifuge conditions and properties following separation. Streams at both corn loads 33 wt% and 26 wt% were separated into a very clean corn oil stream and wet cakes at 38-41 wt% total solids. The suspended solids concentration in the heavy phase was strongly affected by the corn load. The 33 wt% sample generated a centrate TSS of approximately 3.5-4 wt% while the 26 wt% TS generate a lower TSS centrate at approximately 1.7-2 wt%.

Table 23

| **Feed Properties** | | | | |
|---|---|---|---|---|
| TS (wt%) | 33 | 33 | 26 | 26 |
| Feed rate (L/s (gpm)) | 0.57 (9) | 0.71 (11.2) | 0.57 (9) | 0.71 (11.3) |
| **Centrifuge Conditions** | | | | |
| Bowl speed (rpm) | 5000 | 5000 (4400-5400) | 5000 | 5000 |

(continued)

| Centrifuge Conditions | | | | |
|---|---|---|---|---|
| Differential speed (rpm) | 25 (25-50) | 25 (25-50) | 25 | 15 (15-25) |
| Impeller Speed (mm) | 155 (145-158) | 155 (155-160) | 155 | 153 (153-155) |
| **Light Centrate Properties** | | | | |
| Water content (ppm) | Very low | Very low | Very low | Very low |
| TSS (wt%) | Very low | Very low | Very low | Very low |
| Flow rate (mL/min) | 230 (150-330) | 300 (195-360) | 280 (170-280) | 364 (364-459) |
| Recovery (on total basis) (%) | 43 (30-60) | 43 (30-54) | 53 (30-53) | 54 (54-68) |
| **Heavy Centrate Properties** | | | | |
| TSS (wt%) | 3.5 (3.5-4) | 4.3 (3.6-4.7) | 1.7 (1.7-3.8) | 2 (2-3.2) |
| **Wet Cake Properties** | | | | |
| TS (wt%) | 41 (36-42) | 39 (37-39) | 38.7 (38.5-38.7) | 39 (30-40) |

Results are also shown in Figures 19A to 19E. Figure 19A shows that at low flow rates of approximately 0.25 L/s (4 gpm), the centrate TSS was about 3.3%, and the centrate TSS increased to about 4.2-4.7% with a flow rate of 0.73 L/s (11.5 gpm).

Figure 19B shows the suspended solids recovery as a function of flow rate. At low flow rates of approximately 0.25 L/s (4 gpm), approximately 60% of the suspended solids were recovered in the wet cake. By increasing the flow rate to about 0.73 L/s (11.5 gpm), the recovery rate decreased to about 40-50%.

Figure 19C shows the wet cake total solids as a function of flow rate. At low flow rates of approximately 0.25 L/s (4 gpm), wet cake total solids were about 41%. By increasing the flow rate to about 0.73 L/s (11.5 gpm), the wet cake total solids decreased to about 39%.

Figure 19D shows the impact of differential rpm on the total wet cake solids. The wet cake solids decreased with increased differential rpm.

Figure 19E shows the effect of feed rate on corn oil recovery. At low flow rates, oil recovery was about 48%. When the flow rate was increased to approx. 0.73 L/s (11.5 gpm), oil recovery decreases to about 35%. It appears that less oil is separated from the feed stream with higher flow rate.

## Example 22

Rheological Characteristics of Corn Mash

The viscosity of corn mash is measured using an AR-G2 rotational rheometer (TA Instruments, New Castle, DE) configured with vane geometry. A slurry of ground corn and water is prepared, mixed, and heated in a resin kettle to 55°C. The pH is adjusted to 5.8, and enzyme (e.g., alpha-amylase and/or glucoamylase) is added to the slurry. The slurry is heated to 65°C at a rate of 2 °C/min. A sample is removed and transferred to the rheometer equipped with a narrow gap concentric cylinder geometry that is preheated to 65°C. A temperature ramp is then performed raising the temperature from 65°C to 85°C at a rate of 2 °C/min. The temperature ramp is conducted at a fixed shear rate (e.g., 75-200 $s^{-1}$). Viscosity is measured as a function of time.

While various embodiments of the present invention have been described above, it should be understood that they have been presented by way of example only, and not limitation. Thus, the breadth and scope of the present invention should not be limited by any of the above-described exemplary embodiments, but should be defined only in accordance with the following claims.

All publications, patents and patent applications mentioned in this specification are indicative of the level of skill of those skilled in the art to which this invention pertains.

**Claims**

1. A method comprising
providing a feedstock slurry comprising a fermentable carbon source, undissolved solids, and oil;
separating the feedstock slurry whereby

(i) a first aqueous solution comprising a fermentable carbon source,
(ii) a first wet cake comprising solids, and
(iii) a stream comprising oil, solids, and an aqueous stream comprising a fermentable carbon source are formed;

adding the first aqueous solution to a fermentation broth comprising microorganisms whereby a fermentation product is produced;
separating the stream (iii) whereby an oil stream, a second wet cake comprising solids, and a second aqueous solution comprising a fermentable carbon source are formed; and
adding the second aqueous solution to liquefaction and/or feedstock preparation.

2. The method of claim1, wherein the second aqueous solution further comprises oil.

3. The method of claim 2, wherein the oil of the second aqueous solution, or a portion thereof, is treated to generate an extractant.

4. The method of claim 3, wherein the oil is treated chemically or enzymatically.

5. The method of claim 1, wherein separation is by decanter bowl centrifugation, three-phase centrifugation, disk stack centrifugation, filtering centrifugation, decanter centrifugation, filtration, microfiltration, vacuum filtration, beltfilter, pressure filtration, crossflow filtration, drum filter, filtration using a screen, screen separation, rotary screen, grating, porous grating, flotation, hydrocyclone, filter press, screwpress, gravity settler, vortex separator, or combinations thereof.

## Patentansprüche

1. Verfahren umfassend
Bereitstellen einer Rohstoffaufschlämmung umfassend eine fermentierbare Kohlenstoffquelle, unaufgelöste Feststoffe und Öl;
Auftrennen der Rohstoffaufschlämmung, wobei

    (i) eine erste wässrige Lösung, die eine fermentierbare Kohlenstoffquelle umfasst,
    (ii) ein erster nasser Kuchen, der Feststoffe umfasst, und
    (iii) ein Strom, der Öl, Feststoffe umfasst, und ein wässriger Strom, der eine fermentierbare Kohlenstoffquelle umfasst, gebildet werden;

    Zugeben der ersten wässrigen Lösung zu einer Fermentationsbouillon, die Mikroorganismen umfasst, wobei ein Fermentationsprodukt hergestellt wird;
    Auftrennen des Stroms (iii), wobei ein Ölstrom, ein zweiter nasser Kuchen, der Feststoffe umfasst, und eine zweite wässrige Lösung, die eine fermentierbare Kohlenstoffquelle umfasst, gebildet werden; und
    Zugeben der zweiten wässrigen Lösung zur Verflüssigung und/oder Rohstoffherstellung.

2. Verfahren nach Anspruch 1, wobei die zweite wässrige Lösung ferner Öl umfasst.

3. Verfahren nach Anspruch 2, wobei das Öl der zweiten wässrigen Lösung, oder ein Teil davon, behandelt wird, um ein Extraktionsmittel zu erzeugen.

4. Verfahren nach Anspruch 3, wobei das Öl chemisch oder enzymatisch behandelt wird.

5. Verfahren nach Anspruch 1, wobei die Auftrennung durch Schüssel-Dekantiergefäß-Zentrifugieren, Dreiphasenzentrifugieren, Scheibenstapelzentrifugieren, Filtrierzentrifugieren, Dekanterzentrifugieren, Filtrieren, Mikrofiltrieren, Vakuumfiltrieren, Bandfilter, Druckfiltrieren, Kreuzflussfiltrieren, Trommelfilter, Filtrieren unter Anwendung eines Siebs, Siebtrennung, Rotationssieb, Gitter, poröses Gitter, Flotation, Hydrozyklon, Filterpresse, Schneckenpresse, Schwerkraftabscheider, Vortextrennvorrichtung oder Kombinationen davon erfolgt.

## Revendications

1. Procédé comprenant
la fourniture d'une suspension de charge d'alimentation comprenant une source de carbone fermentescible, des matières solides non dissoutes, et de l'huile;
la séparation de la suspension de charge d'alimentation moyennant quoi

    (i) une première solution aqueuse comprenant une source de carbone fermentescible,
    (ii) un premier gâteau humide comprenant des matières solides, et
    (iii) un écoulement comprenant de l'huile, des matières solides, et un écoulement aqueux comprenant une source de carbone fermentescible sont formés;

    l'addition de la première solution aqueuse à un bouillon de fermentation comprenant des micro-organismes moyennant quoi un produit de fermentation est produit;
    la séparation de l'écoulement (iii) moyennant quoi un écoulement d'huile, un second gâteau humide comprenant des matières solides, et une seconde solution aqueuse comprenant une source de carbone fermentescible sont formés; et
    l'addition de la seconde solution aqueuse à la préparation de liquéfaction et/ou charge d'alimentation.

2. Procédé selon la revendication 1, la seconde solution aqueuse comprenant en outre de l'huile.

3. Procédé selon la revendication 2, l'huile de la seconde solution aqueuse, ou une partie de celle-ci, étant traitée pour générer un agent d'extraction.

4. Procédé selon la revendication 3, l'huile étant chimiquement ou enzymatiquement traitée.

5. Procédé selon la revendication 1, la séparation s'effectuant par centrifugation du bol de décantation, par centrifugation triphasé, centrifugation par empilement de disques, centrifugation par filtration, centrifugation par décantation, filtration, microfiltration, filtration sous vide, filtre à courroie, filtration sous pression, filtration à écoulements croisés, filtre à tambour, filtration en utilisant un tamis, séparation par tamis, tamis rotatif, grillage, grillage poreux, flottaison, hydrocyclone, presse à filtre, presse à vis, décanteur par gravité, séparateur par vortex, ou leurs combinaisons.

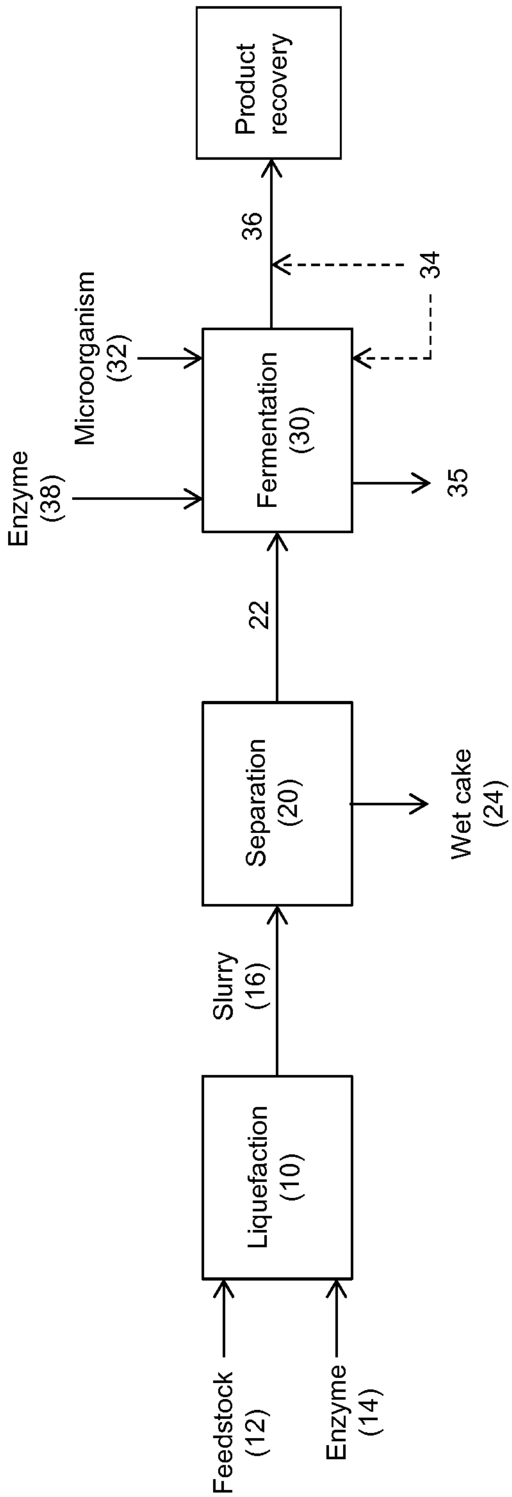
Feedstock
(12)
Enzyme
(14)
Liquefaction
(10)
Slurry
(16)
Separation
(20)
Wet cake
(24)
22
Enzyme
(38)
Microorganism
(32)
Fermentation
(30)
35
34
36
Product
recovery

FIG. 1

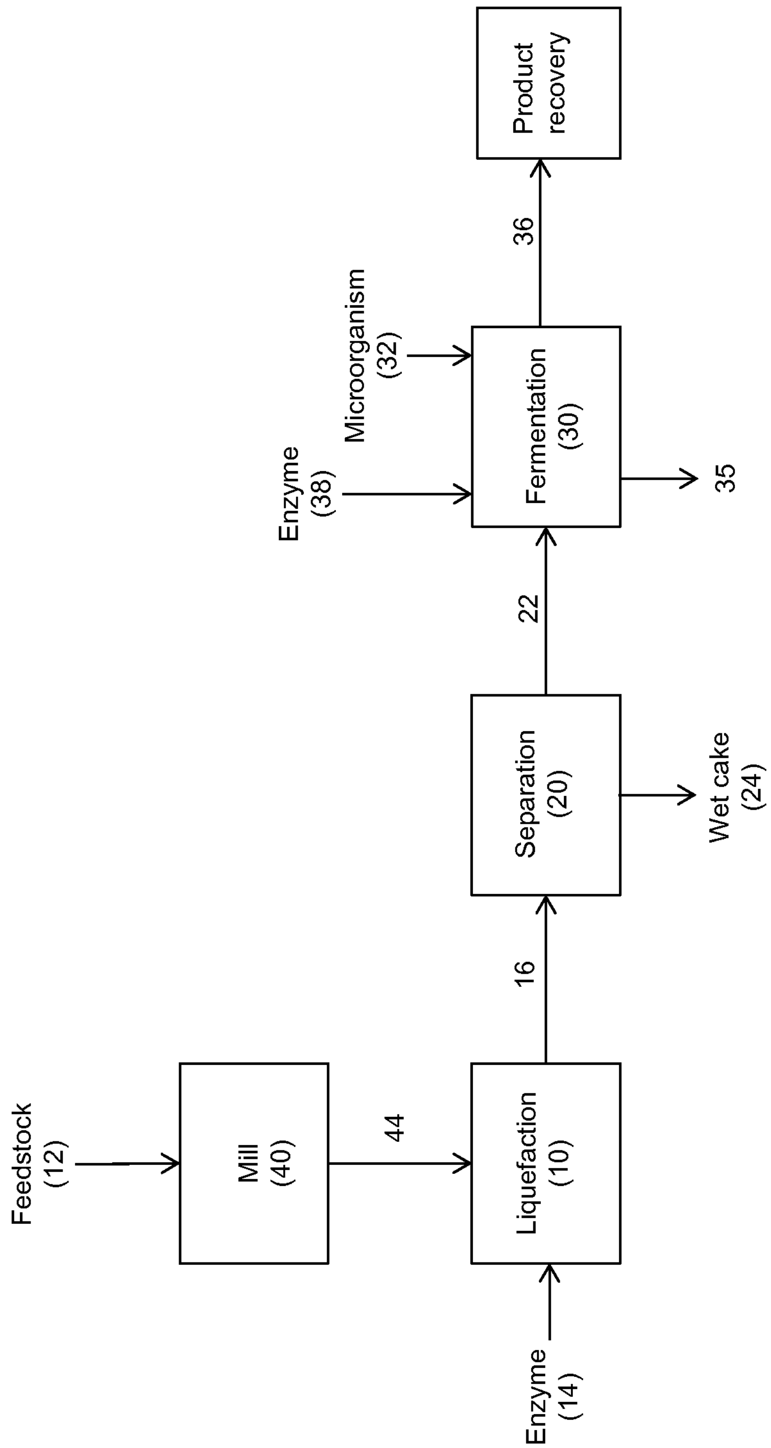
Feedstock
(12)
Mill
(40)
44
Liquefaction
(10)
Enzyme
(14)
16
Separation
(20)
Wet cake
(24)
22
Enzyme
(38)
Microorganism
(32)
Fermentation
(30)
35
36
Product
recovery

FIG. 2

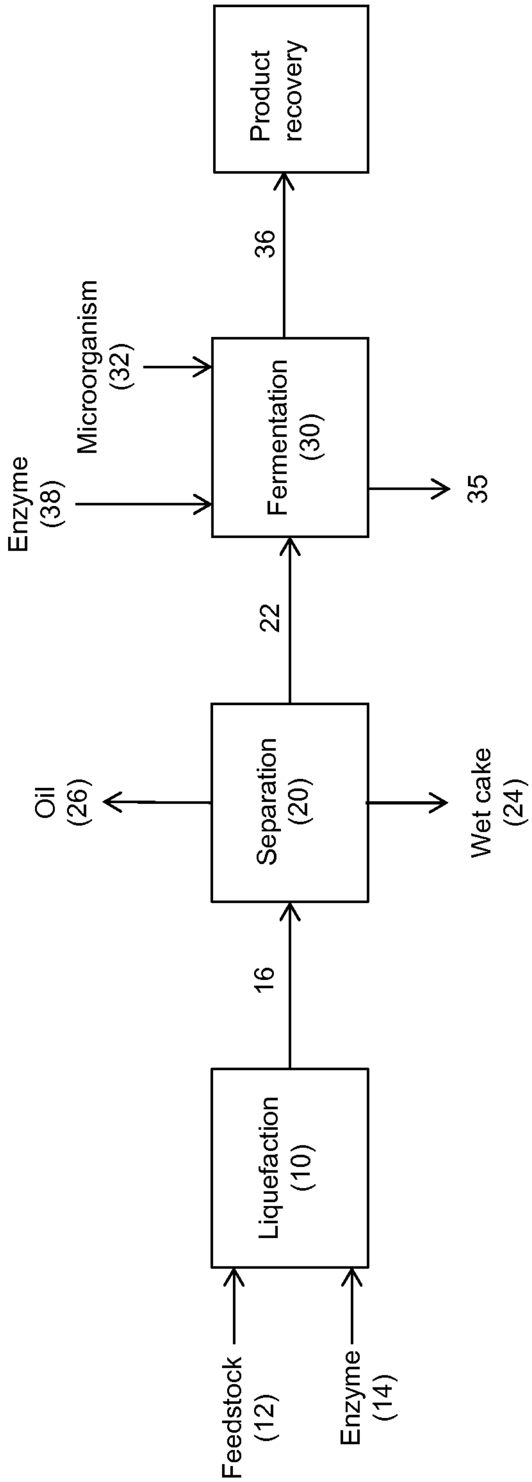
Feedstock
(12)
Enzyme
(14)
Liquefaction
(10)
16
Separation
(20)
Oil
(26)
Wet cake
(24)
22
Fermentation
(30)
Enzyme
(38)
Microorganism
(32)
35
36
Product
recovery

FIG. 3

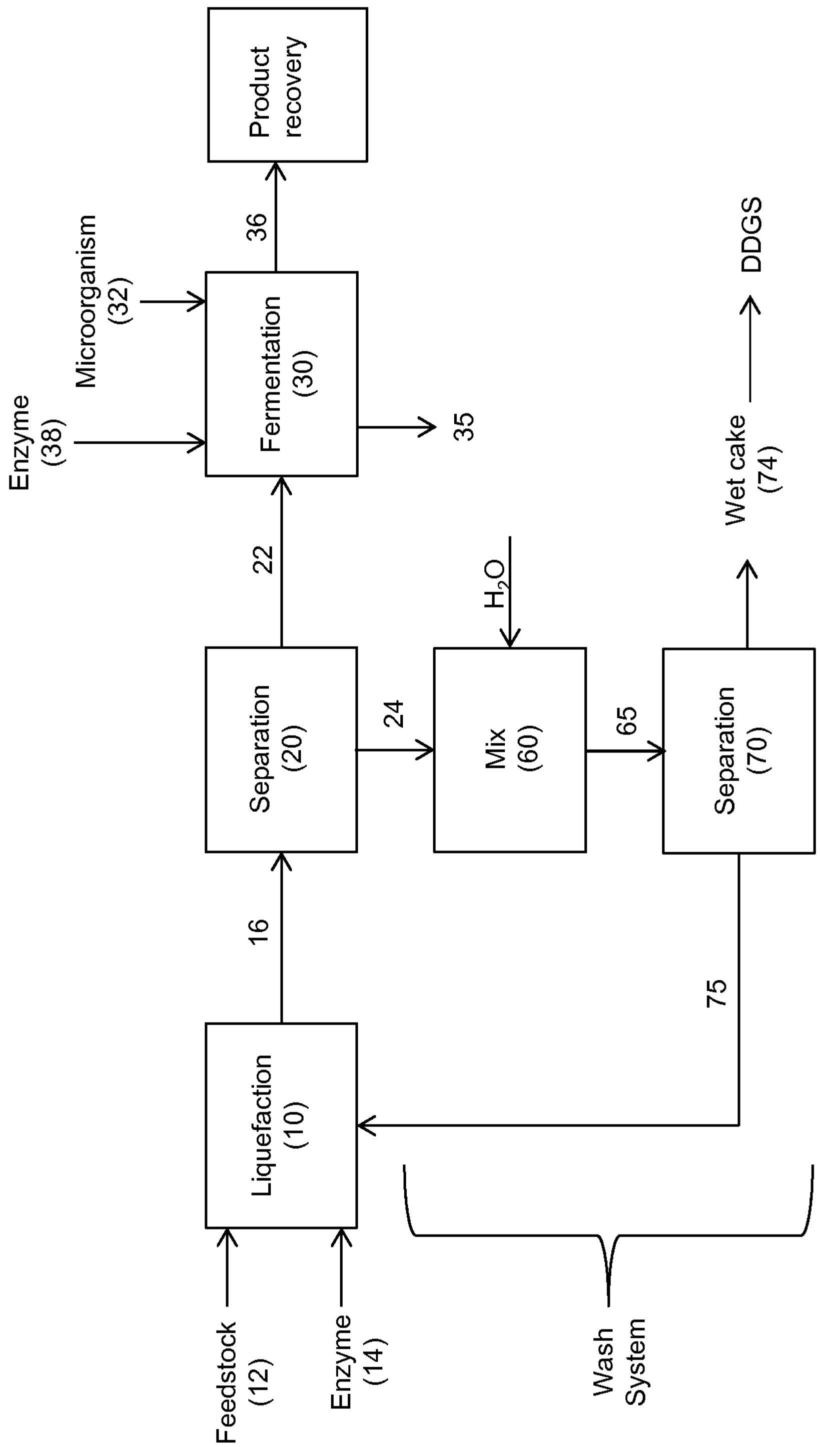
Feedstock (12)
Enzyme (14)
Liquefaction (10)
16
Separation (20)
22
24
Fermentation (30)
Enzyme (38)
Microorganism (32)
35
36
Product recovery
Mix (60)
$H_2O$
65
Separation (70)
Wet cake (74)
DDGS
75
Wash System

FIG. 4A

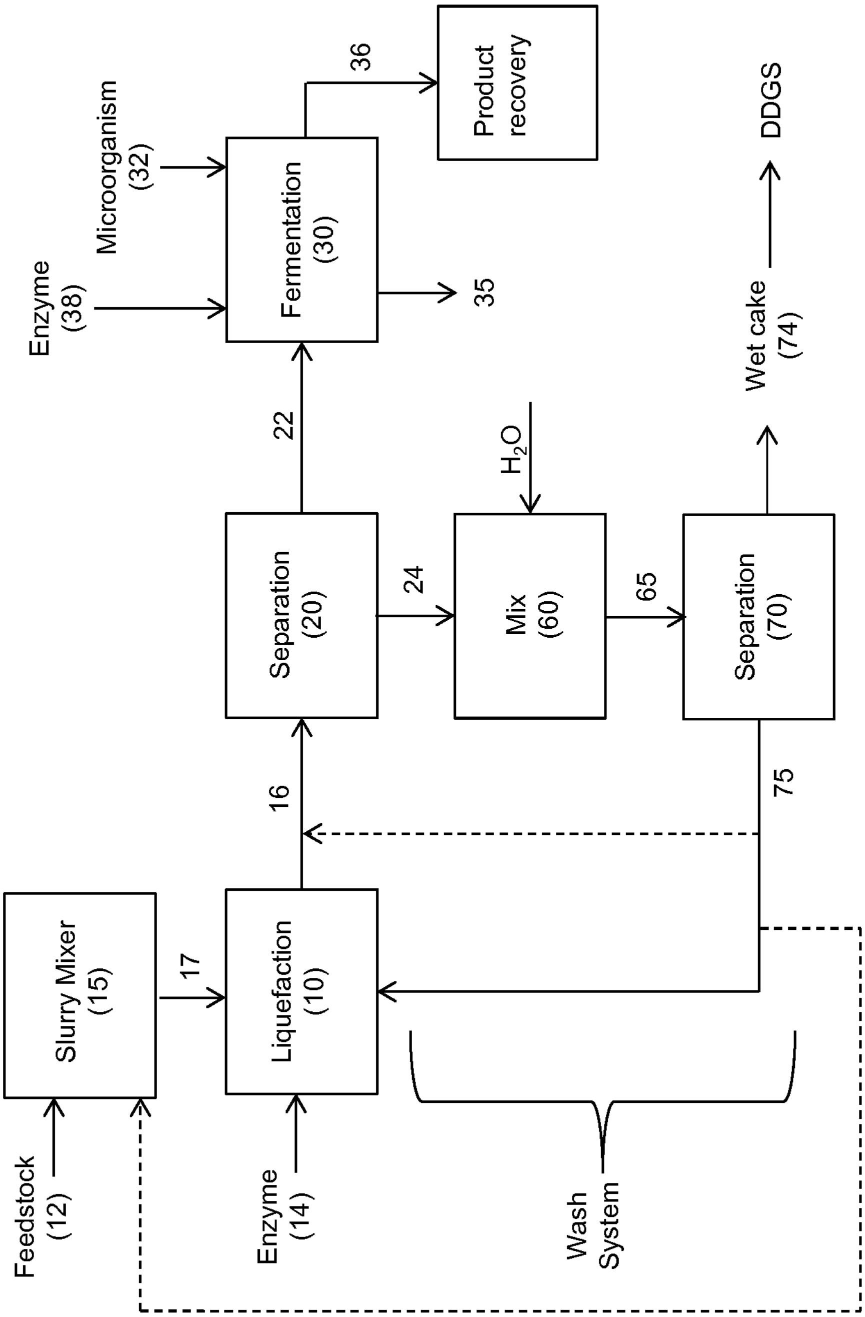
Feedstock (12)
Slurry Mixer (15)
17
Enzyme (14)
Liquefaction (10)
16
Separation (20)
22
24
Enzyme (38)
Microorganism (32)
Fermentation (30)
35
36
Product recovery
$H_2O$
Mix (60)
65
Separation (70)
75
Wet cake (74)
DDGS
Wash System

FIG. 4B

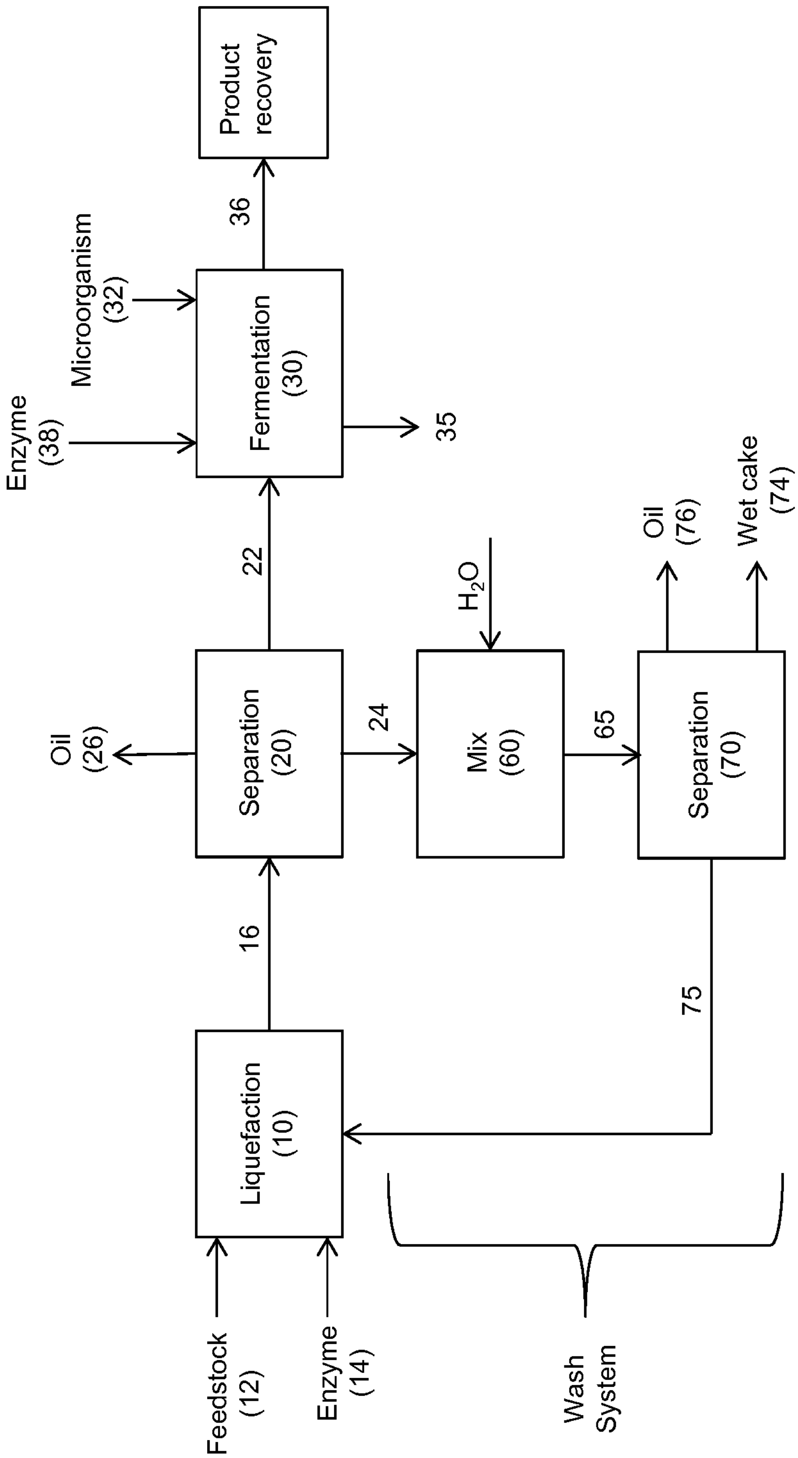
Product recovery
36
Enzyme (38)
Microorganism (32)
Fermentation (30)
35
22
Oil (26)
Separation (20)
24
$H_2O$
Mix (60)
65
Oil (76)
Wet cake (74)
Separation (70)
16
75
Liquefaction (10)
Feedstock (12)
Enzyme (14)
Wash System

FIG. 5A

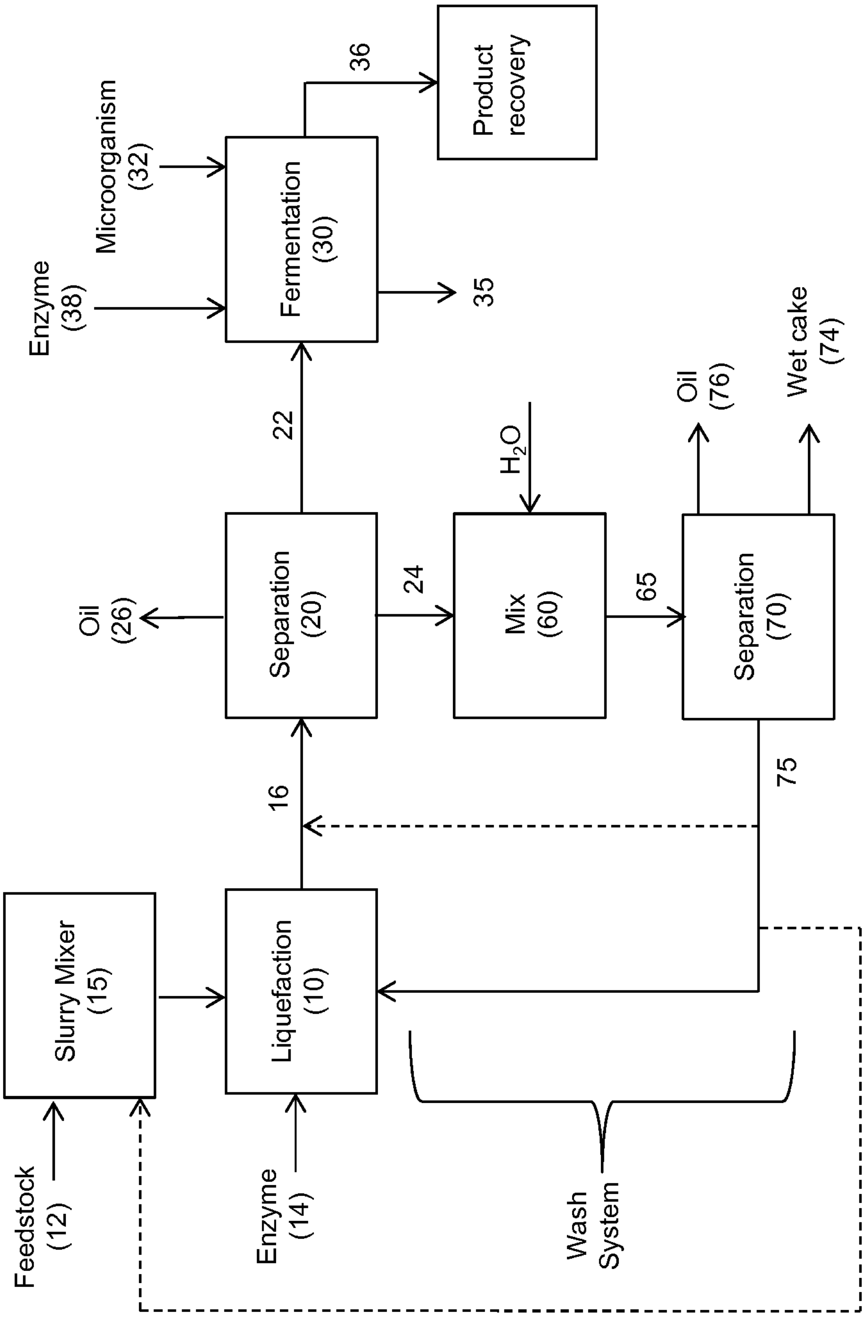

Feedstock (12)
Slurry Mixer (15)
Enzyme (14)
Liquefaction (10)
16
Oil (26)
Separation (20)
22
24
Enzyme (38)
Microorganism (32)
Fermentation (30)
35
36
Product recovery
Mix (60)
$H_2O$
65
Separation (70)
Oil (76)
Wet cake (74)
75
Wash System


FIG. 5B

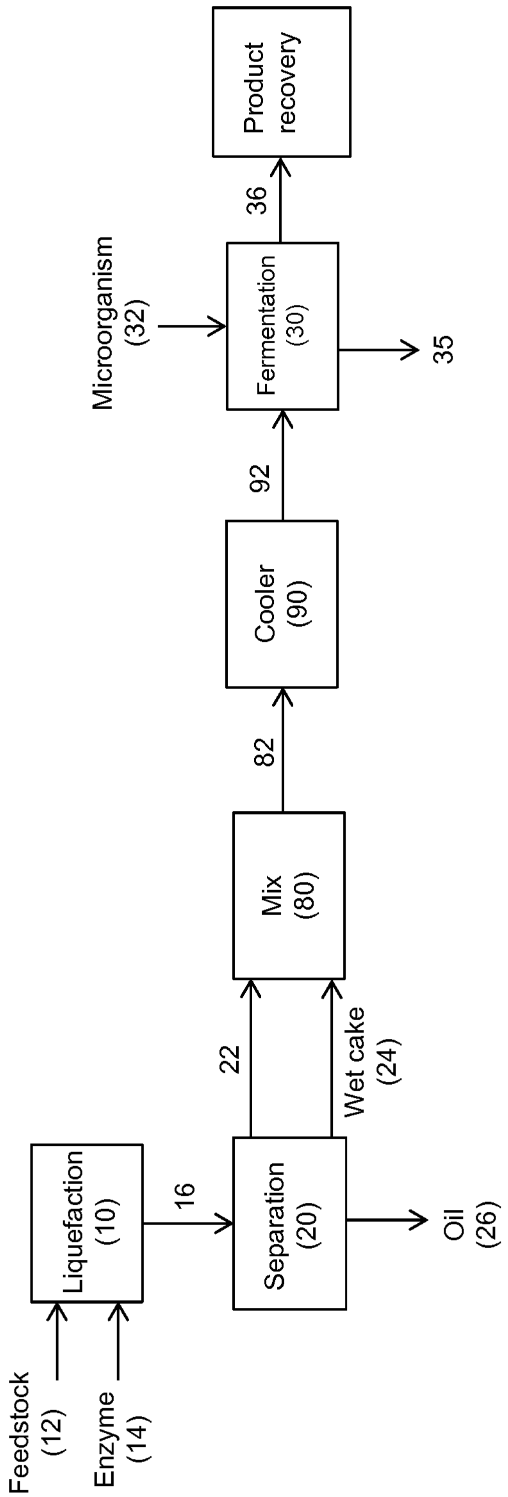
Feedstock (12)
Enzyme (14)
Liquefaction (10)
16
Separation (20)
Oil (26)
22
Wet cake (24)
Mix (80)
82
Cooler (90)
92
Microorganism (32)
Fermentation (30)
35
36
Product recovery

FIG. 6

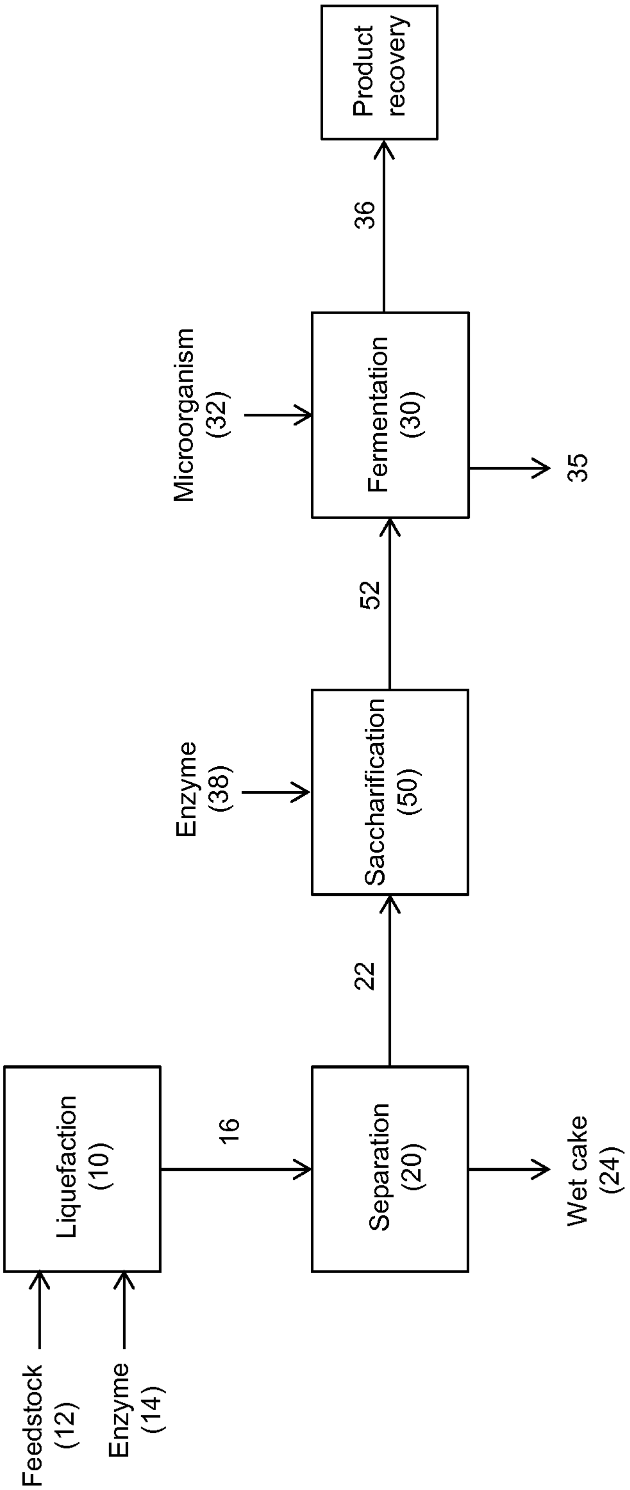
Feedstock (12)
Enzyme (14)
Liquefaction (10)
16
Separation (20)
Wet cake (24)
22
Enzyme (38)
Saccharification (50)
52
Microorganism (32)
Fermentation (30)
35
36
Product recovery

FIG. 7

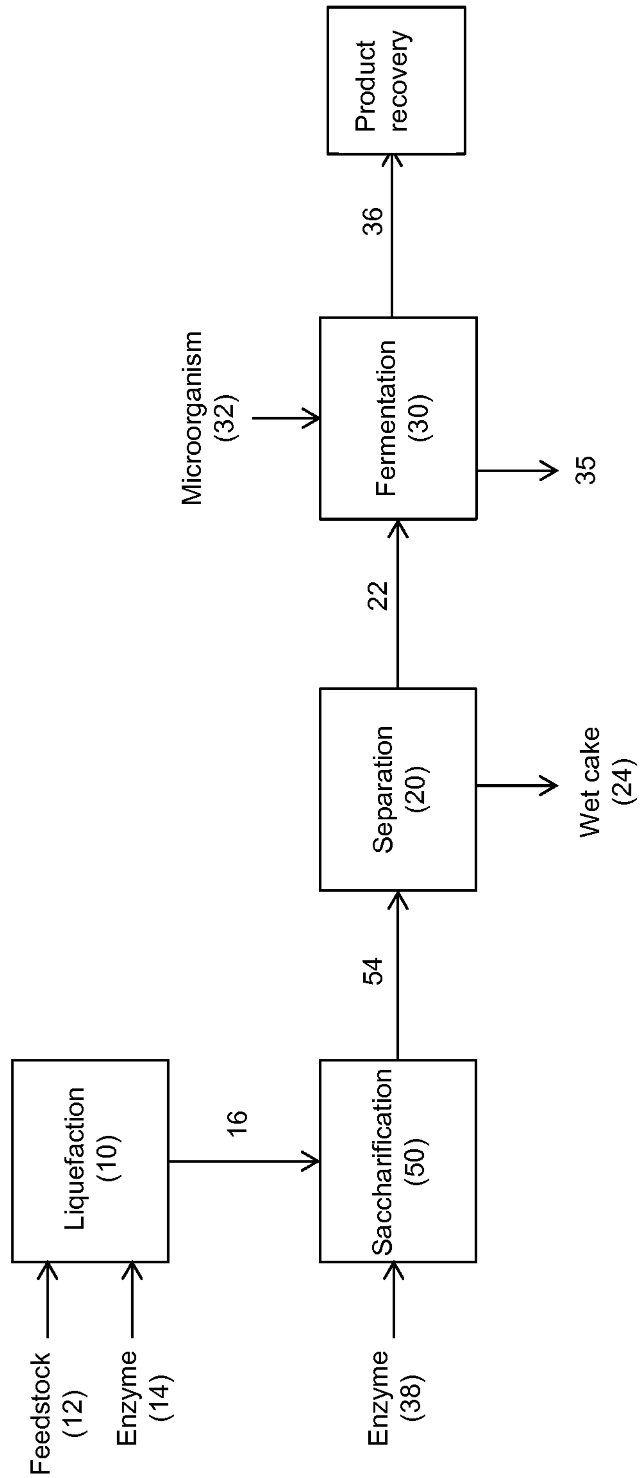
Feedstock (12)
Enzyme (14)
Liquefaction (10)
16
Enzyme (38)
Saccharification (50)
54
Separation (20)
Wet cake (24)
22
Microorganism (32)
Fermentation (30)
35
36
Product recovery

FIG. 8

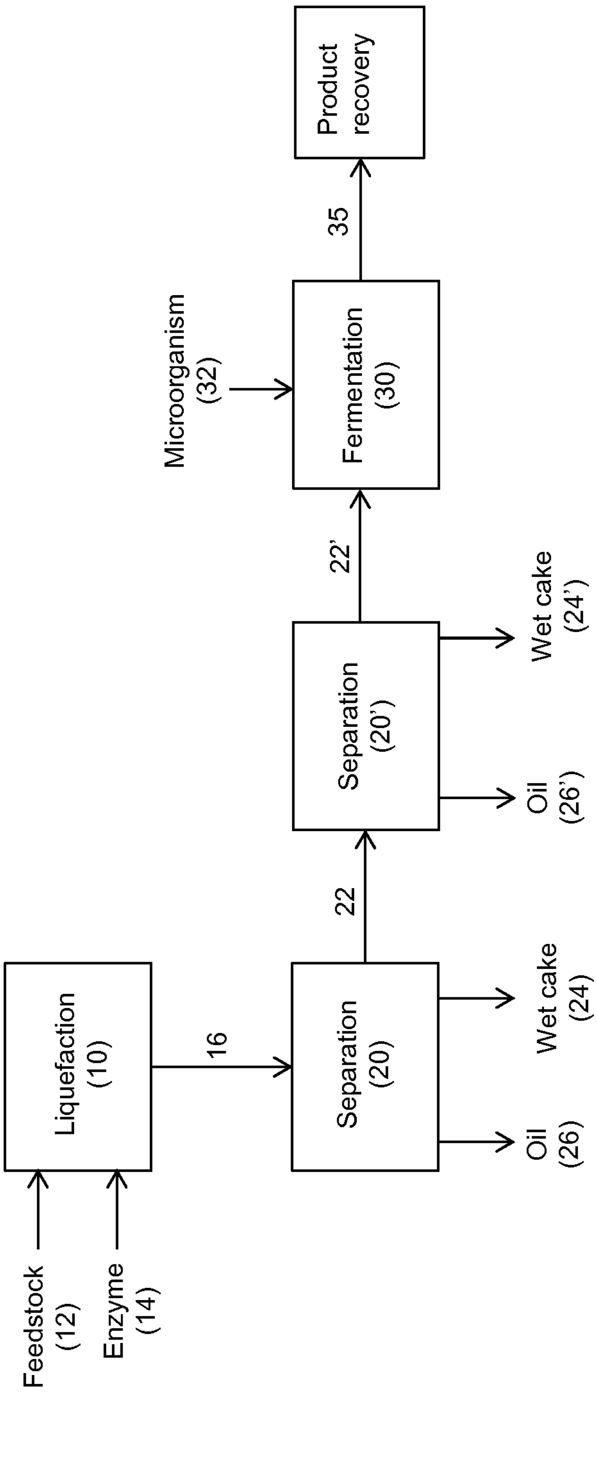
Feedstock (12)
Enzyme (14)
Liquefaction (10)
16
Separation (20)
Oil (26)
Wet cake (24)
22
Separation (20')
Oil (26')
Wet cake (24')
22'
Microorganism (32)
Fermentation (30)
35
Product recovery

FIG. 9A

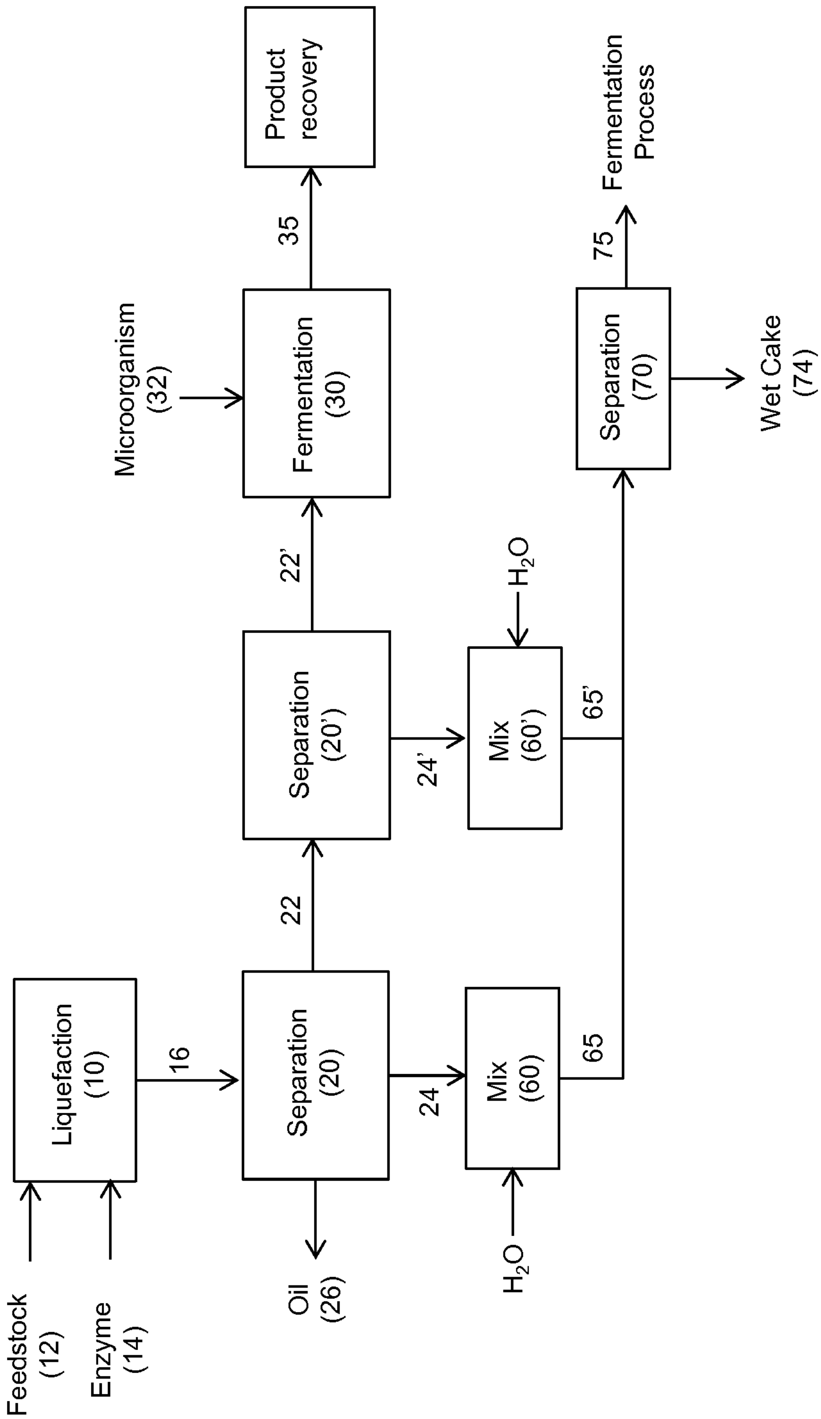
Feedstock (12)
Enzyme (14)
Liquefaction (10)
16
Separation (20)
Oil (26)
22
24
Mix (60)
H2O
65
Separation (20')
22'
24'
Mix (60')
H2O
65'
Microorganism (32)
Fermentation (30)
35
Product recovery
Separation (70)
Wet Cake (74)
75
Fermentation Process

FIG. 9B

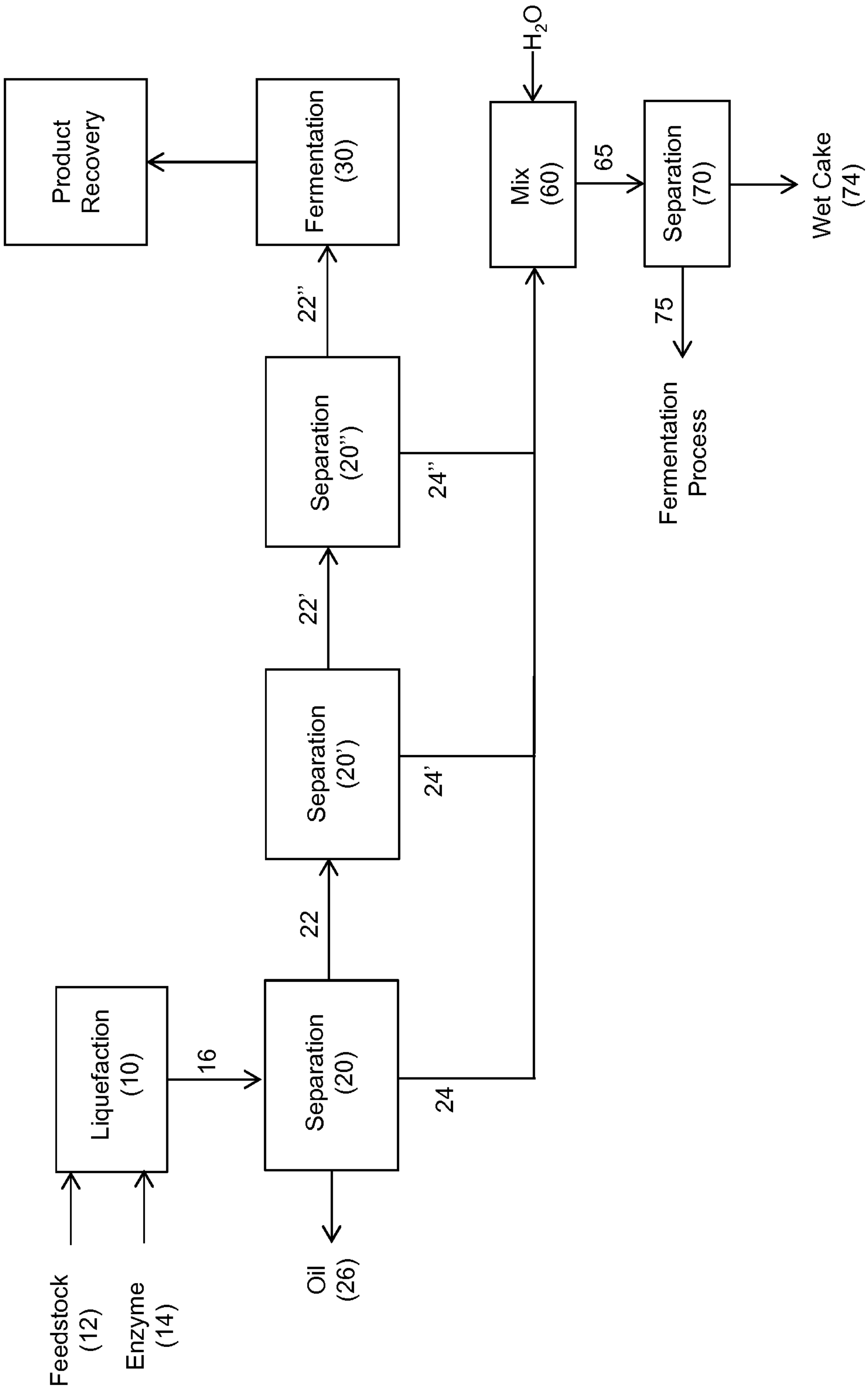
Feedstock (12)
Enzyme (14)
Liquefaction (10)
16
Separation (20)
Oil (26)
22
24
Separation (20')
22'
24'
Separation (20")
22"
24"
Fermentation (30)
Product Recovery
$H_2O$
Mix (60)
65
Separation (70)
Wet Cake (74)
75
Fermentation Process

FIG. 9C

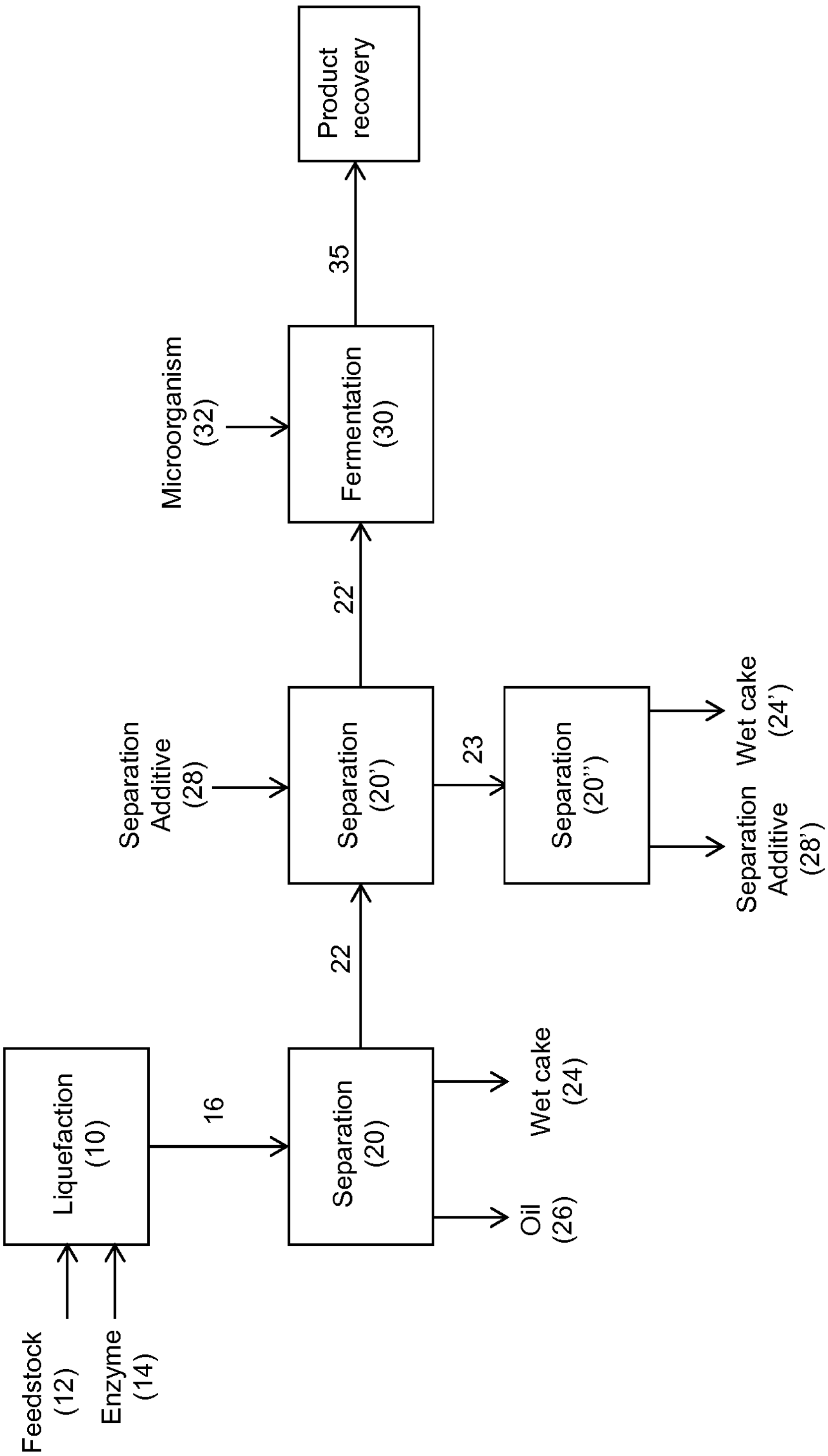
Feedstock (12)
Enzyme (14)
Liquefaction (10)
16
Separation (20)
Oil (26)
Wet cake (24)
22
Separation Additive (28)
Separation (20')
23
Separation (20'')
Separation Additive (28')
Wet cake (24')
22'
Microorganism (32)
Fermentation (30)
35
Product recovery

FIG. 9D

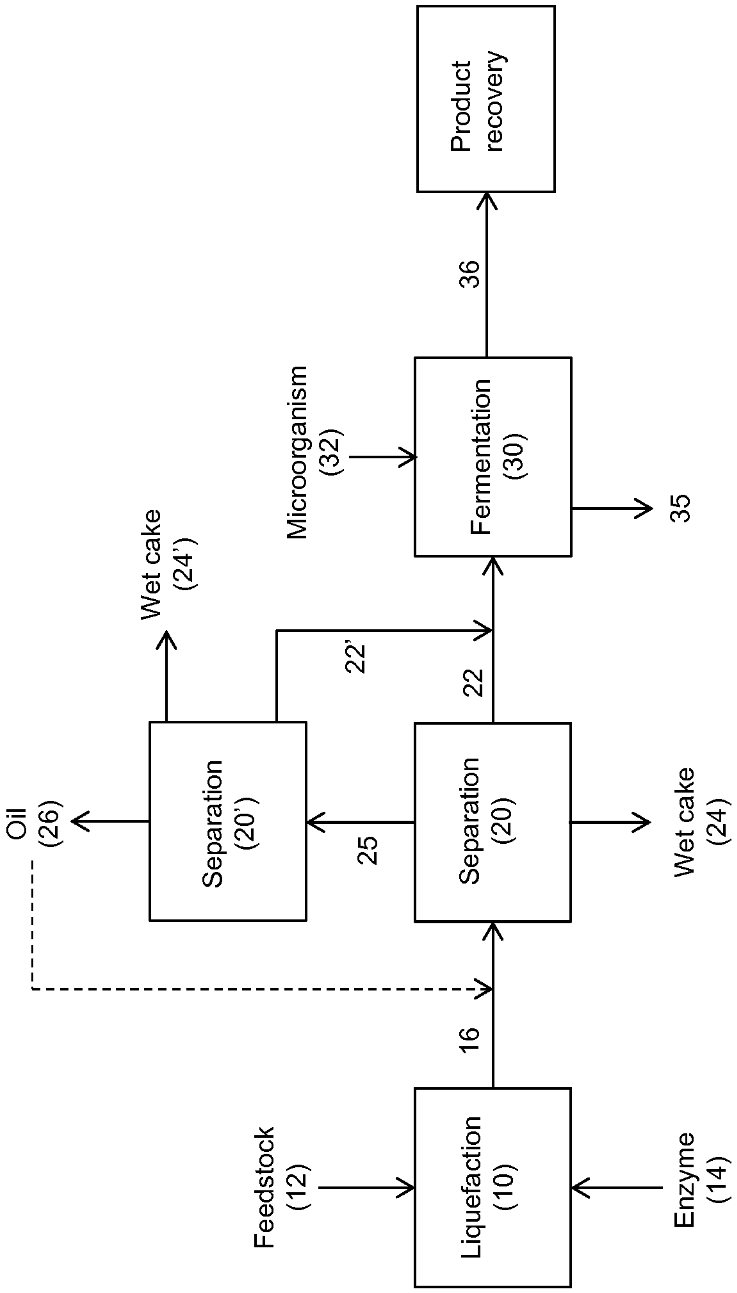
Product recovery
36
Microorganism (32)
Fermentation (30)
35
Wet cake (24')
22'
22
Oil (26)
Separation (20')
25
Separation (20)
Wet cake (24)
16
Feedstock (12)
Liquefaction (10)
Enzyme (14)

FIG. 9E

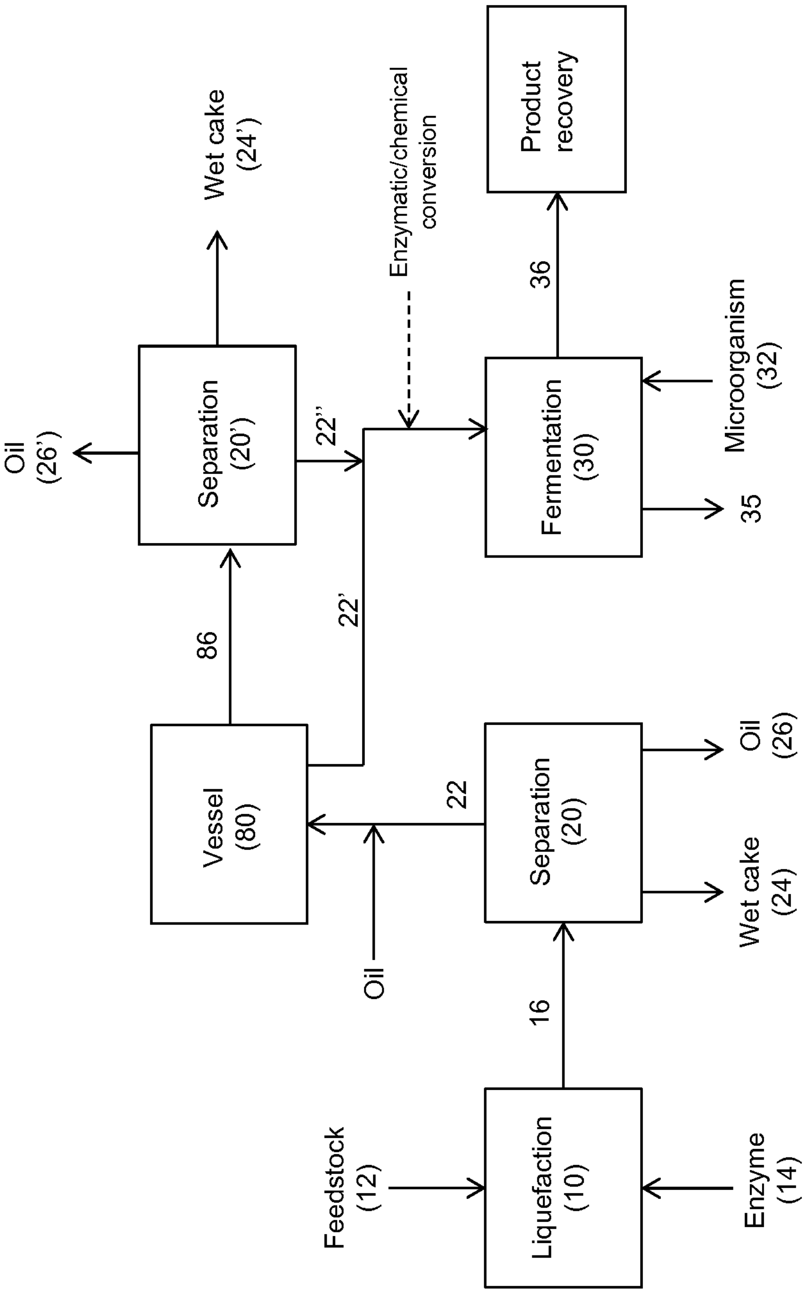
Feedstock (12)
Liquefaction (10)
Enzyme (14)
16
Separation (20)
Wet cake (24)
Oil (26)
Oil
22
Vessel (80)
22'
86
Separation (20')
Oil (26')
Wet cake (24')
22''
Enzymatic/chemical conversion
Fermentation (30)
Microorganism (32)
35
36
Product recovery

FIG. 9F

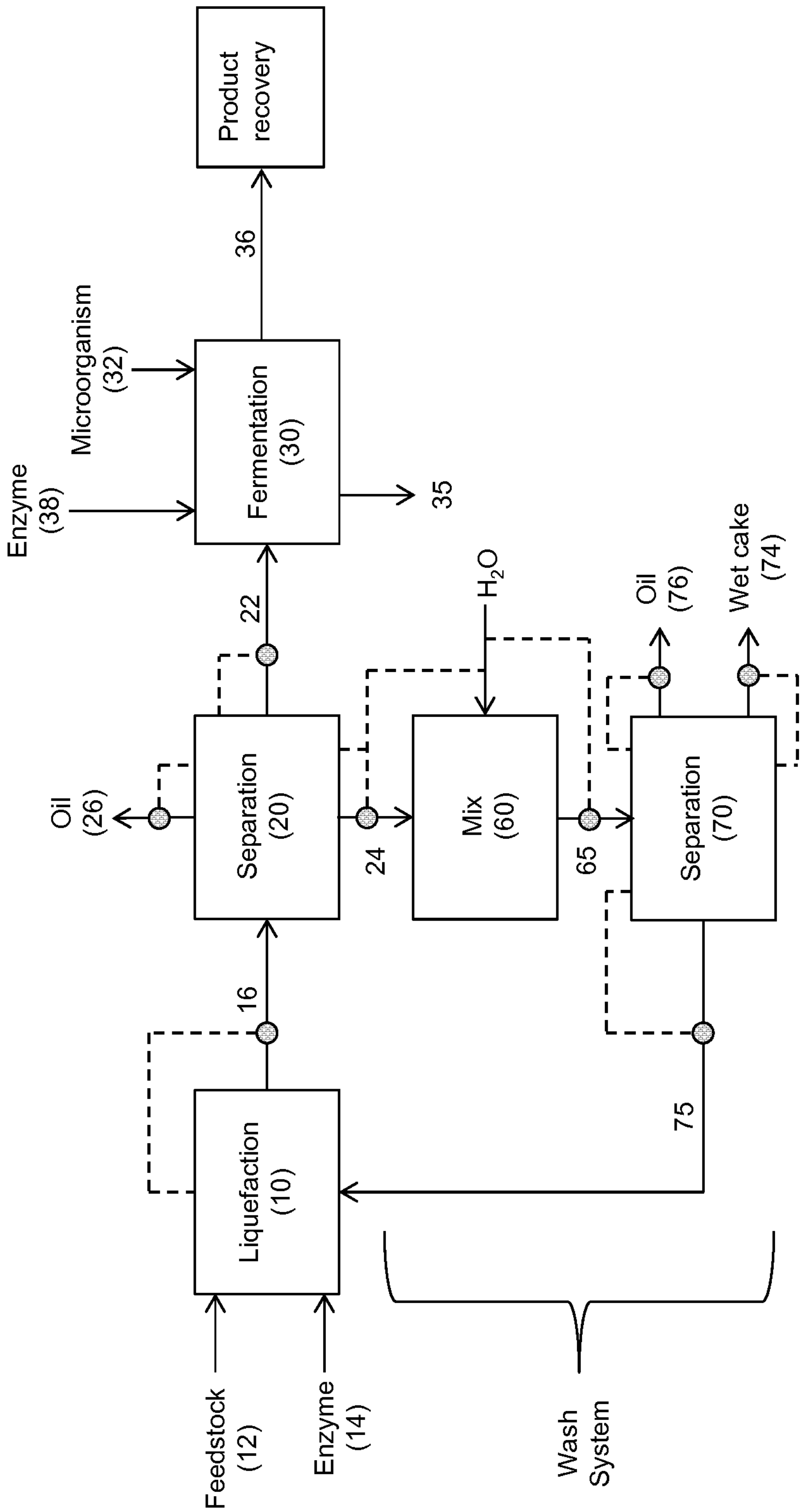
Feedstock (12)
Enzyme (14)
Liquefaction (10)
16
Separation (20)
Oil (26)
22
24
Enzyme (38)
Microorganism (32)
Fermentation (30)
35
36
Product recovery
H$_2$O
Mix (60)
65
Separation (70)
Oil (76)
Wet cake (74)
75
Wash System

FIG. 10

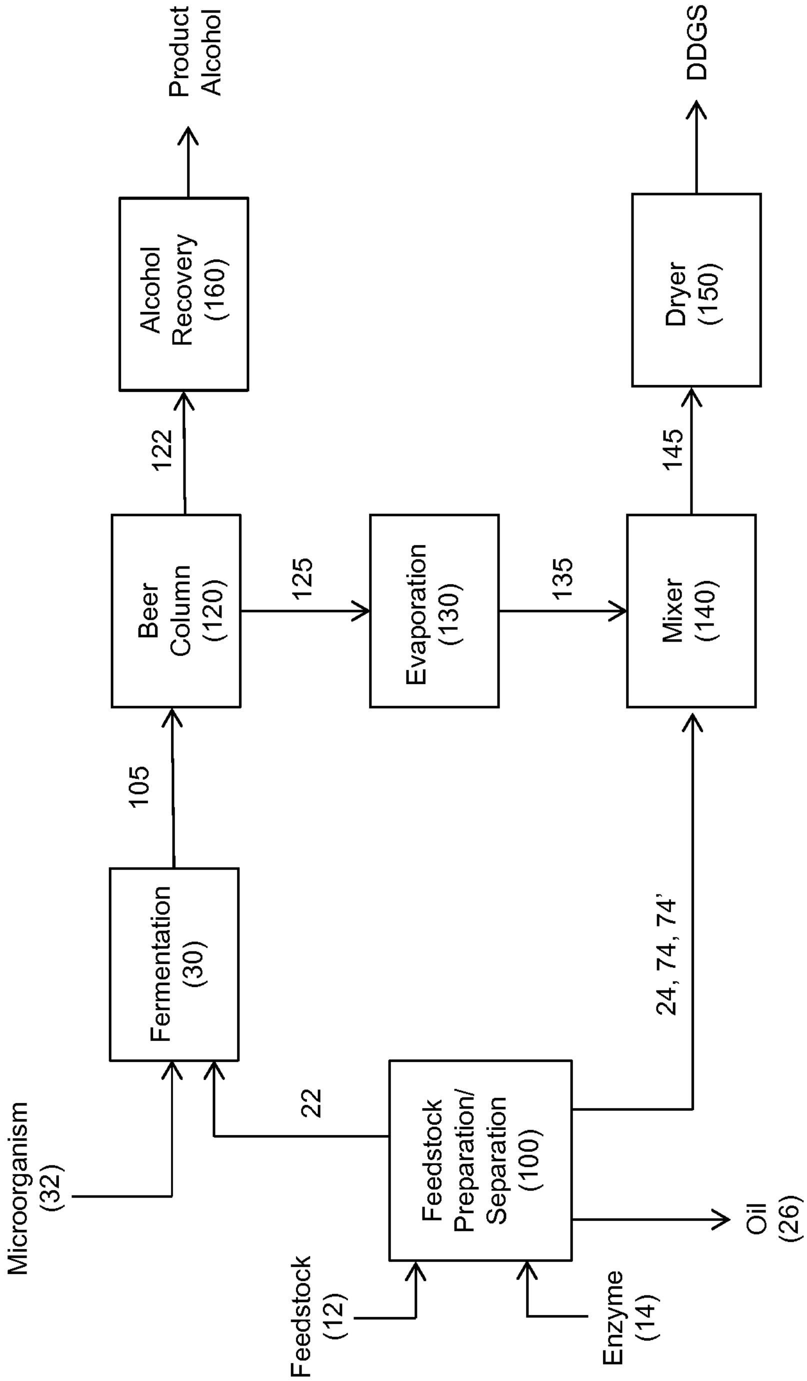
Microorganism (32)
Feedstock (12)
Enzyme (14)
Feedstock Preparation/ Separation (100)
Oil (26)
22
Fermentation (30)
105
Beer Column (120)
122
Alcohol Recovery (160)
Product Alcohol
125
Evaporation (130)
135
Mixer (140)
24, 74, 74'
145
Dryer (150)
DDGS

FIG. 11

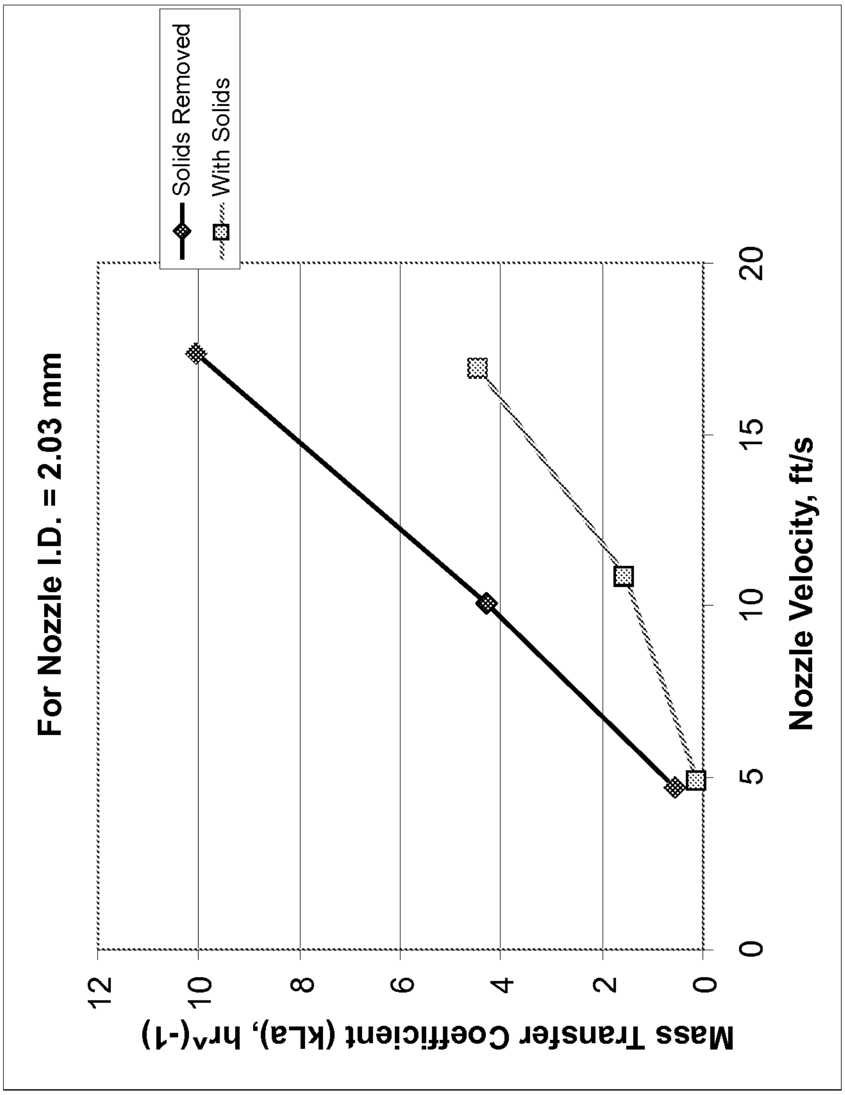
For Nozzle I.D. = 2.03 mm
Mass Transfer Coefficient (kLa), hr^(-1)
Nozzle Velocity, ft/s
Solids Removed
With Solids
0
2
4
6
8
10
12
0
5
10
15
20

FIG. 12

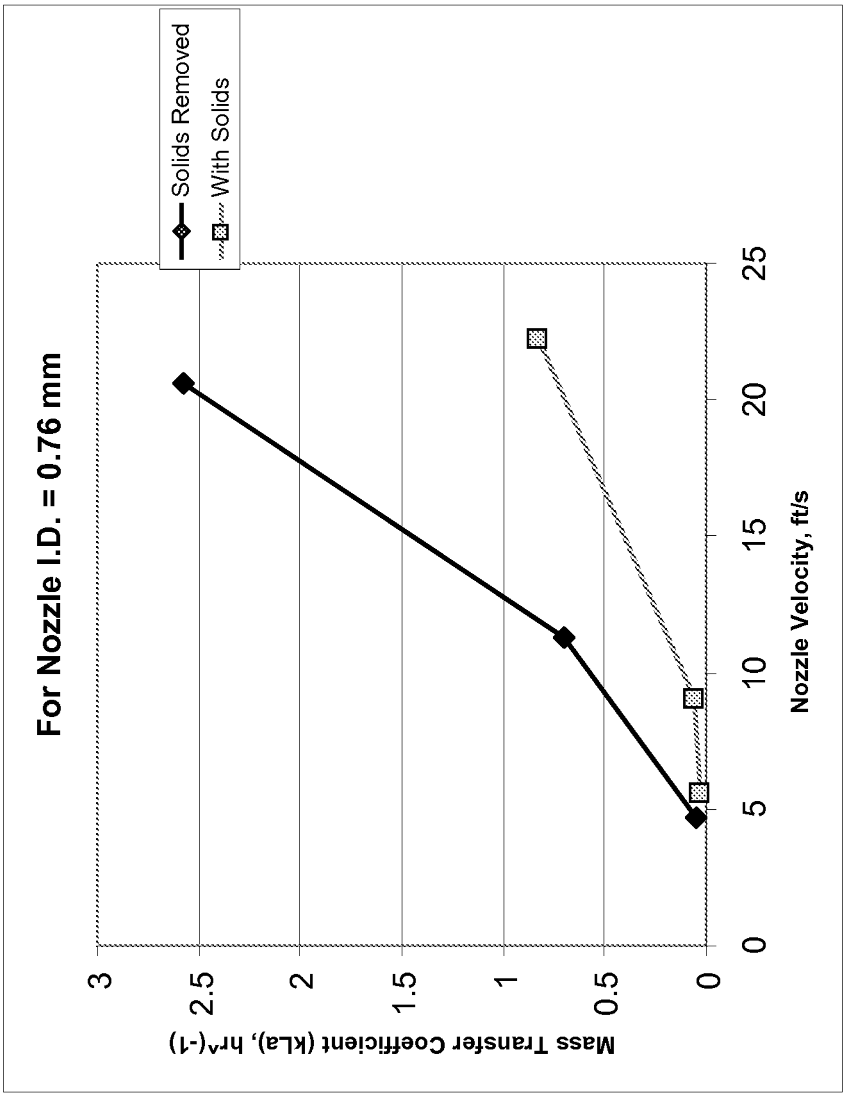
For Nozzle I.D. = 0.76 mm
Solids Removed
With Solids
Mass Transfer Coefficient (kLa), hr^(-1)
0
0.5
1
1.5
2
2.5
3
Nozzle Velocity, ft/s
0
5
10
15
20
25

FIG. 13

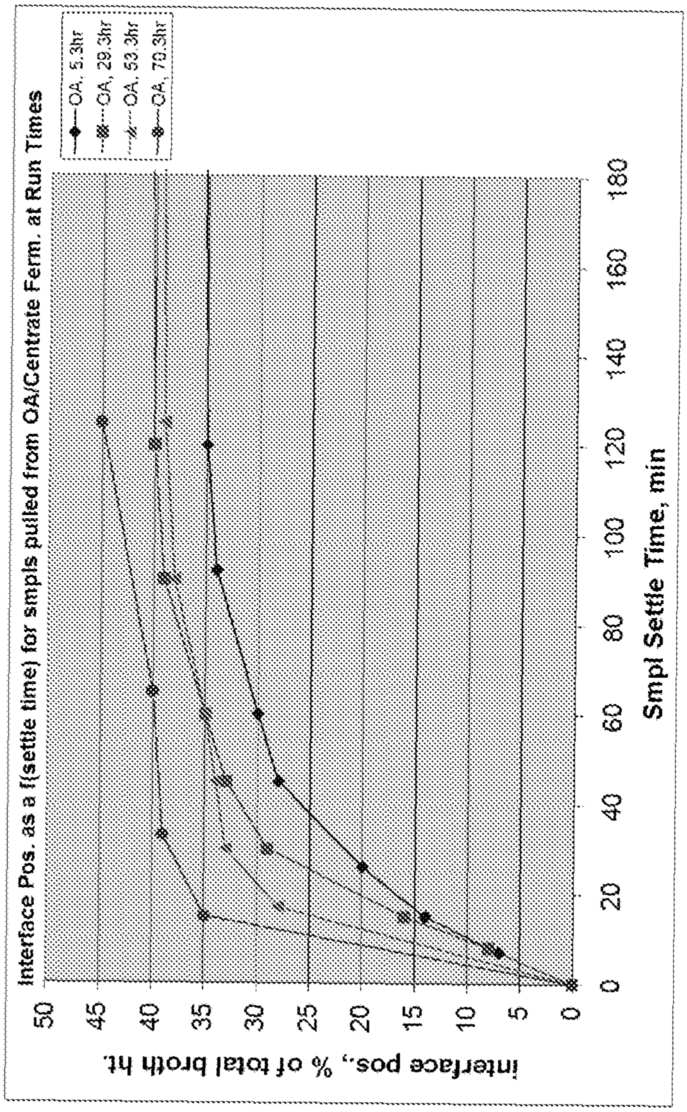
Interface Pos. as a f(settle time) for smpls pulled from OA/Centrate Ferm. at Run Times
OA, 5.3hr
OA, 29.3hr
OA, 53.3hr
OA, 70.3hr
Interface pos., % of total broth ht.
0
5
10
15
20
25
30
35
40
45
50
Smpl Settle Time, min
0
20
40
60
80
100
120
140
160
180

FIG. 14

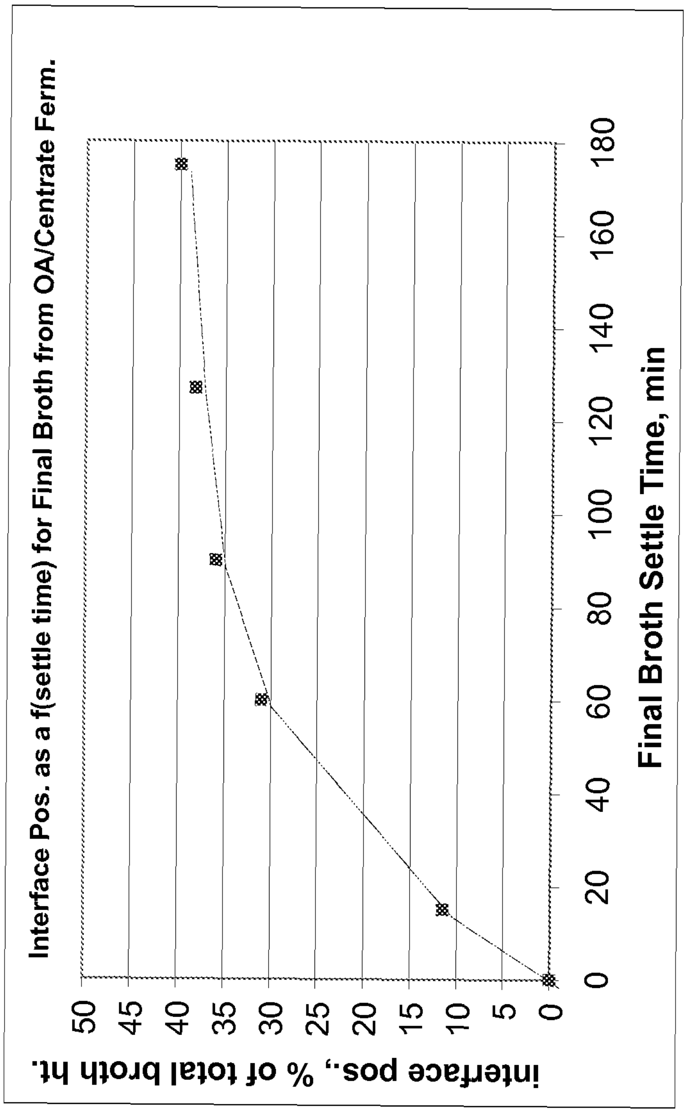
Interface Pos. as a f(settle time) for Final Broth from OA/Centrate Ferm.
interface pos., % of total broth ht.
0
5
10
15
20
25
30
35
40
45
50
0
20
40
60
80
100
120
140
160
180
Final Broth Settle Time, min

FIG. 15

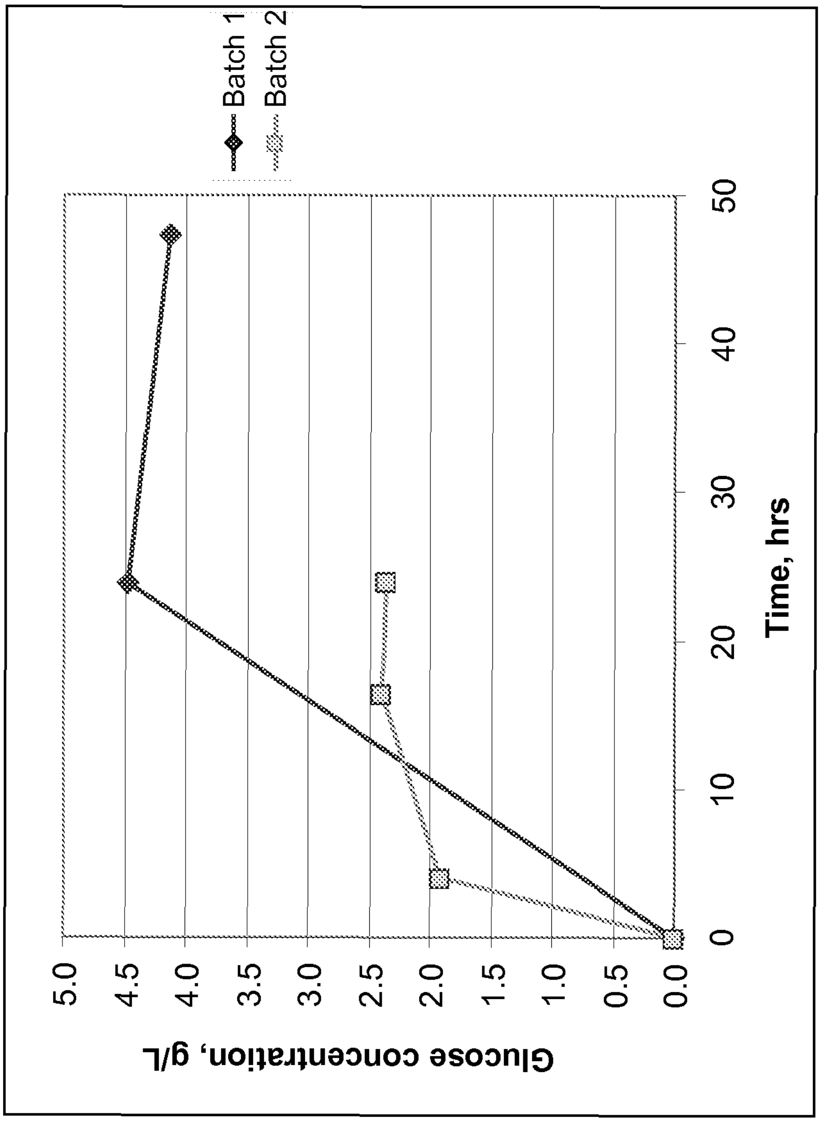
Glucose concentration, g/L
5.0
4.5
4.0
3.5
3.0
2.5
2.0
1.5
1.0
0.5
0.0
0
10
20
30
40
50
Time, hrs
Batch 1
Batch 2

FIG. 16

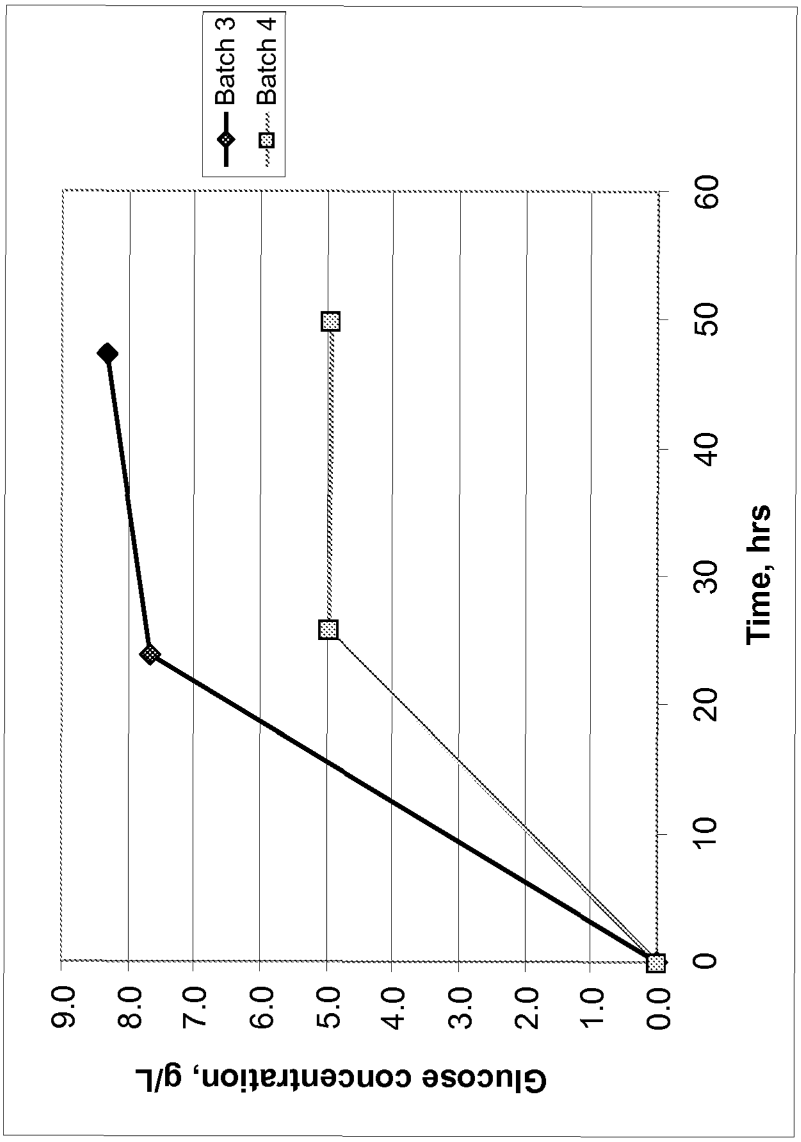
Glucose concentration, g/L
9.0
8.0
7.0
6.0
5.0
4.0
3.0
2.0
1.0
0.0
0
10
20
30
40
50
60
Time, hrs
Batch 3
Batch 4

FIG. 17

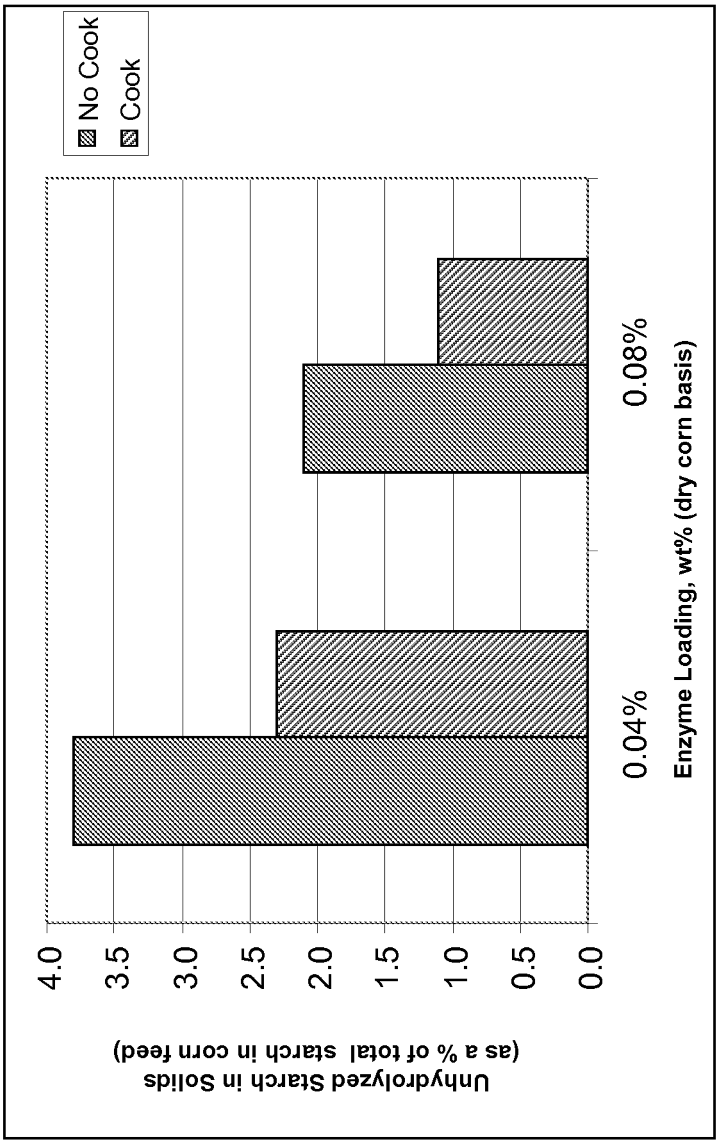
No Cook
Cook
Unhydrolyzed Starch in Solids
(as a % of total starch in corn feed)
4.0
3.5
3.0
2.5
2.0
1.5
1.0
0.5
0.0
0.04%
0.08%
Enzyme Loading, wt% (dry corn basis)

FIG. 18

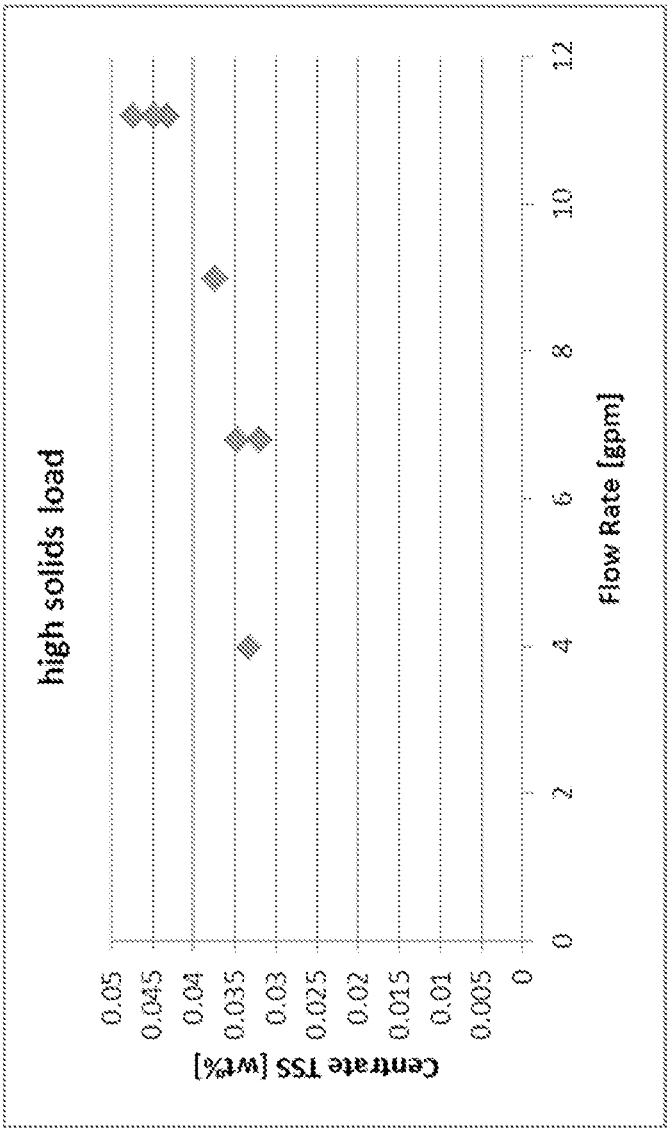
high solids load
Centrate TSS [wt%]
0.05
0.045
0.04
0.035
0.03
0.025
0.02
0.015
0.01
0.005
0
0
2
4
6
8
10
12
Flow Rate [gpm]

FIG. 19A

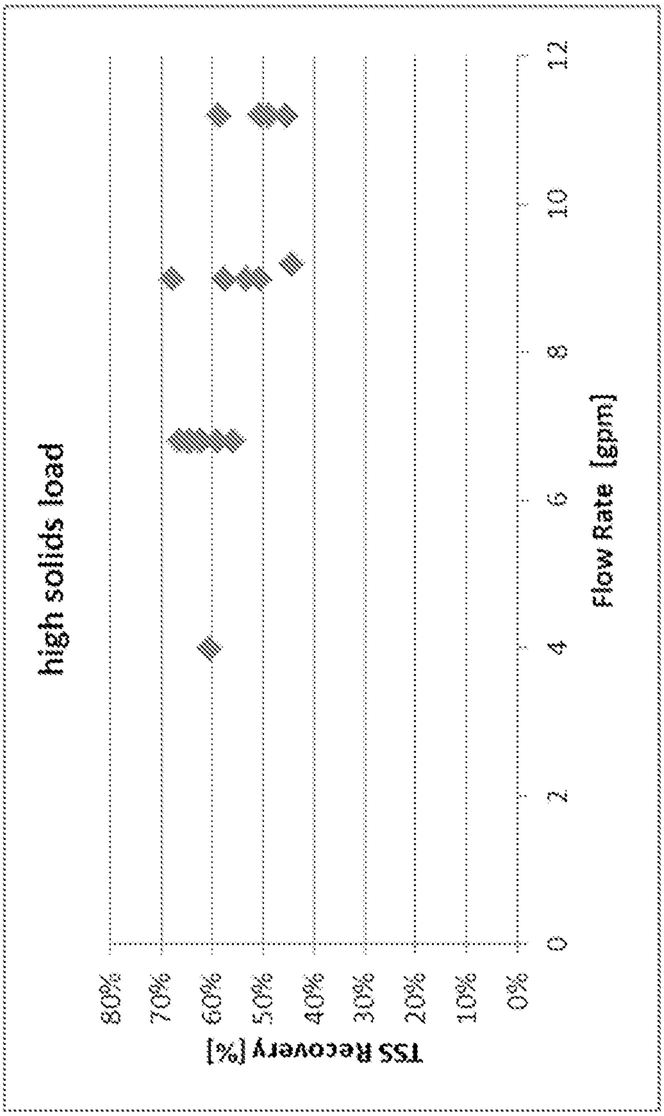
high solids load
TSS Recovery [%]
80%
70%
60%
50%
40%
30%
20%
10%
0%
0
2
4
6
8
10
12
Flow Rate [gpm]

FIG. 19B

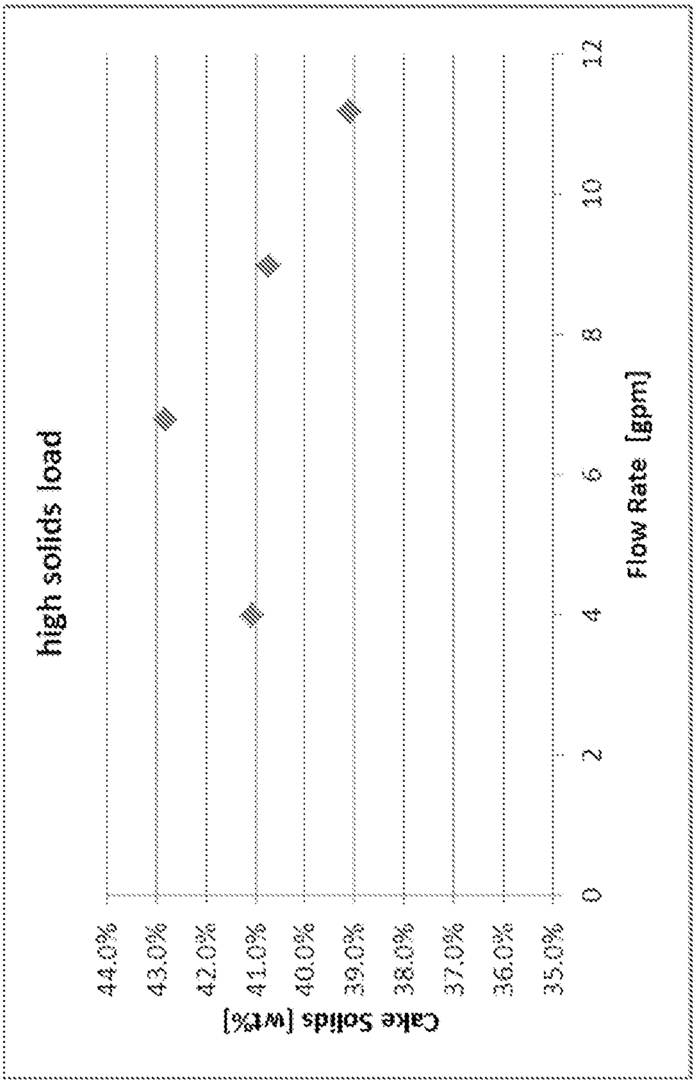
high solids load
Cake Solids [wt%]
44.0%
43.0%
42.0%
41.0%
40.0%
39.0%
38.0%
37.0%
36.0%
35.0%
0
2
4
6
8
10
12
Flow Rate [gpm]

FIG. 19C

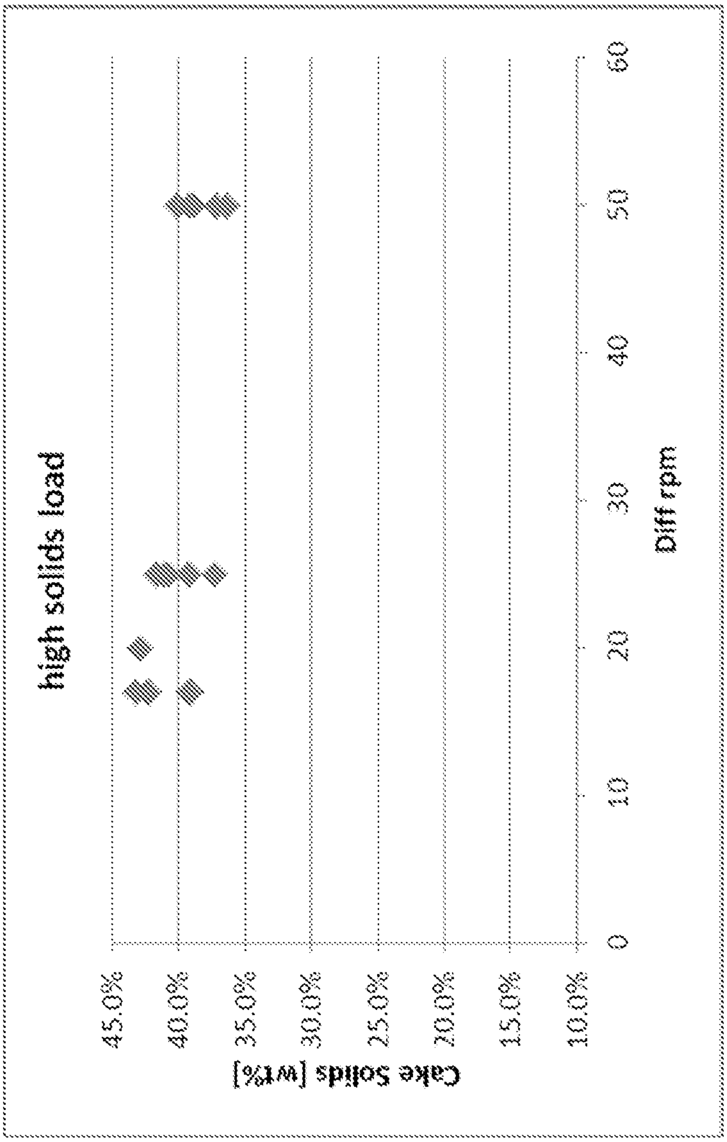
high solids load
Cake Solids [wt%]
45.0%
40.0%
35.0%
30.0%
25.0%
20.0%
15.0%
10.0%
0
10
20
30
40
50
60
Diff rpm

FIG. 19D

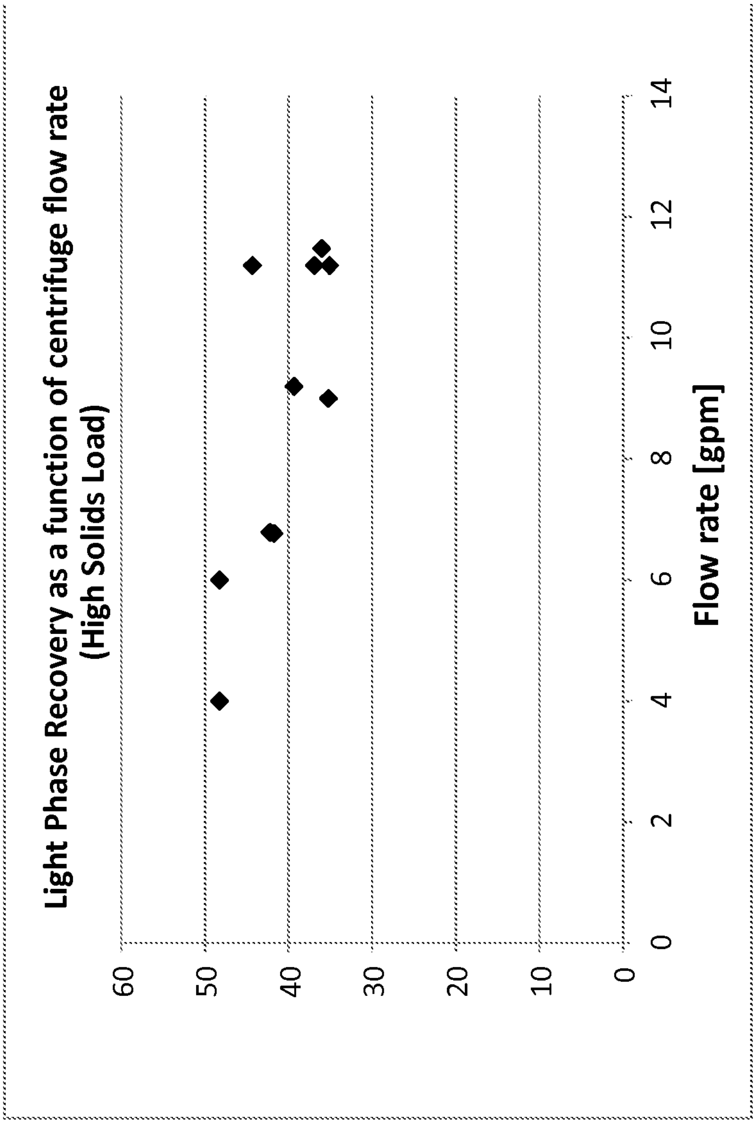
Light Phase Recovery as a function of centrifuge flow rate
(High Solids Load)
60
50
40
30
20
10
0
0
2
4
6
8
10
12
14
Flow rate [gpm]

FIG. 19E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61712385
- US 82835313
- US 83611513
- US 02472213
- US 20090305370
- WO 2011160030 A
- WO 2011159967 A
- US 20070031918 A1
- US 7541026 B
- US 20090209026
- US 20090238923
- US 20090252828
- US 20090314286
- US 201002278970
- US 20100048446
- US 20100021587
- US 7498159 B
- US 20120003701
- US 20120129229
- WO 2010096562 PCT
- WO 2011153516 PCT
- US 7851188 B
- US 20070092957
- US 20070259410 A
- US 20070292927 A
- US 20080182308 A
- US 20080274525 A
- US 20090155870 A
- US 20090305363 A
- US 20090305370 A
- US 20110306116
- US 20100221802
- US 20110097773
- US 20110312044
- US 20110312043
- US 20120156738
- US 7413887 B
- US 7723079 B
- US 20090275080
- US 20100267114
- US 20110014681
- US 20110020899
- WO 2011159998 A
- US 20110315541
- US 20120035398
- WO 2011159998 PCT
- WO 2012030374 PCT
- US 20090171129
- US 7601858 B
- US 8192627 B
- US 20100092603
- US 7666282 B
- US 5514583 A
- US 5712133 A
- WO 1995028476 PCT
- US 7223575 B
- US 7741119 B
- US 20090203099 A1
- US 20090246846 A1
- WO 2010075241 A
- US 20080182308
- US 20070259410
- US 20090155870
- US 20080261230
- US 20090163376 A
- US 20100197519 A
- WO 2011041415 A
- US 20100081154
- US 20090269823
- US 20110269199
- US 6432688 B, Ito
- US 5686276 A
- US 20090305363
- US 20100120105
- US 20110124060
- US 20120164302


### Non-patent literature cited in the description


- **LYND et al.** *Microbiol. Mol. Biol. Rev.,* 2002, vol. 66, 506-577
- Corn Oil. Corn Refiners Association, 2006
- **VAN DIJKEN et al.** *Enzyme Microb. Techno.,* 2000, vol. 26, 706-714
- **OHTA et al.** *Appl. Environ. Microbiol.,* 1991, vol. 57, 893-900
- **UNDERWOOD et al.** *Appl. Environ. Microbiol.,* 2002, vol. 68, 1071-1081

- **SHEN ; LIAO.** *Metab. Eng.,* 2008, vol. 10, 312-320
- **HAHNAI et al.** *Appl. Environ. Microbiol.,* 2007, vol. 73, 7814-7818
- **FELDMANN et al.** *Appl. Microbiol. Biotechnol.,* 1992, vol. 38, 354-361
- **ZHANG et al.** *Science,* 1995, vol. 267, 240-243
- Enzyme Nomenclature. Academic Press, 1992
- **SHIN ; KIM.** *J. Org. Chem.,* 2002, vol. 67, 2848-2853
- **GARCIA-ALLES et al.** *Biochemistry,* 2004, vol. 43, 13037-13046
- **YASUTA et al.** *Appl. Environ. Microbial.,* 2001, vol. 67, 4999-5009
- **JONES et al.** *Biochem J.,* 1973, vol. 134, 167-182
- **SPERANZA et al.** *J. Agric. Food Chem.,* 1997, vol. 45, 3476-3480
- Computational Molecular Biology. Oxford University, 1988
- Biocomputing: Informatics and Genome Projects. Academic, 1993
- Computer Analysis of Sequence Data. Humania, 1994
- Sequence Analysis in Molecular Biology. Academic, 1987
- Sequence Analysis Primer. Stockton, 1991
- **HIGGINS ; SHARP.** *CABIOS,* 1989, vol. 5, 151-153
- **HIGGINS et al.** *Comput. Appl. Biosci.,* 1992, vol. 8, 189-191
- **SAMBROOK, J. ; FRITSCH, E. F. ; MANIATIS, T.** Molecular Cloning: A Laboratory Manual. Cold Spring Harbor Laboratory Press, 1989
- **AUSUBEL et al.** Current Protocols in Molecular Biology. Greene Publishing Assoc. and Wiley-Interscience, 1987